# EUROPEAN PATENT APPLICATION

(11) **EP 4 800 013 A1**
(43) Date of publication of application: **02.09.2026**
(21) Application number: 24881768.6
(22) Date of filing: 25.10.2024
(51) Int. Cl.: C07D 417/04, C07D 417/14, C07D 491/107, C07D 498/04, C07D 413/04, C07D 413/14, A61K 31/433, A61K 31/4439, A61K 31/497, A61K 31/496, A61K 31/454, A61K 31/4184, A61P 35/00

(54) **PARG INHIBITOR**

(30) Priority: 26.10.2023 CN 202311404125; 22.11.2023 CN 202311566766; 05.01.2024 CN 202410020915; 05.03.2024 CN 202410251670; 17.06.2024 CN 202410783604; 13.09.2024 CN 202411292146; 18.10.2024 CN 202411465623
(71) Applicant: Shanghai Qilu Pharmaceutical Research and Development Centre Ltd., Shanghai 201203 (CN)
(72) Inventor: WANG, Jianfei, Shanghai 201203 (CN); BAO, Jinxiao, Shanghai 201203 (CN); SHI, Guqin, Shanghai 201203 (CN); XIA, Tingting, Shanghai 201203 (CN); WEI, Wei, Shanghai 201203 (CN); WANG, Zheng, Shanghai 201203 (CN); SUN, Weimei, Shanghai 201203 (CN); QIAN, Wenyuan, Shanghai 201203 (CN); SUN, Daqing, Shanghai 201203 (CN); TAO, Weikang, Shanghai 201203 (CN)
(74) Representative: Dai, Simin
(86) International application number: PCT/CN2024/127488
(87) International publication number: WO 2025/087409

(57) **Abstract**

Provided in the present disclosure are certain compounds for inhibiting the activity of Poly ADP-Ribose Glycohydrolase (PARG). The present disclosure also relates to a method for preparing these compounds, a pharmaceutical composition comprising same, and the use thereof in the treatment of cancers and other diseases. The compounds have the structure as shown in formula (IA).

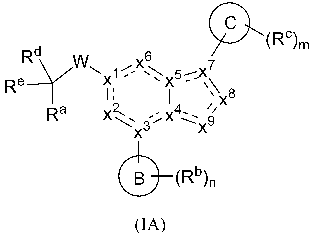

## Description

The present application claims priority to Chinese Patent Application No. 2023114041256, filed on October 26, 2023; Chinese Patent Application No. 2023115667661, filed on November 22, 2023; Chinese Patent Application No. 2024100209152, filed on January 5, 2024; Chinese Patent Application No. 2024102516704, filed on March 5, 2024; Chinese Patent Application No. 2024107836041, filed on June 17, 2024; Chinese Patent Application No. 2024112921468, filed on September 13, 2024; and Chinese Patent Application No. 2024114656236, filed on October 18, 2024. The contents of the above Chinese Patent Applications are incorporated herein by reference in their entireties.

### TECHNICAL FIELD

The present disclosure relates to certain compounds for inhibiting the activity of PARG (poly ADP-ribose glycohydrolase). The present disclosure also relates to methods of preparing these compounds, pharmaceutical compositions comprising the same, and the use thereof in the treatment of cancer and other diseases.

### BACKGROUND

Maintaining genomic stability and integrity is essential for normal cellular function in organisms. Since DNA is highly susceptible to damage from external environmental factors or internal factors, organisms possess a series of complex DNA damage repair (DDR) mechanisms to timely and accurately repair DNA damage. Defects in DNA damage repair mechanisms can easily lead to genomic instability, resulting in cell apoptosis or even carcinogenesis.

The clinical success of PARP (poly ADP-ribose polymerase) inhibitors has sparked intense interest in the development of drugs targeting DNA damage repair pathways based on the mechanism of synthetic lethality. PARP1/2, as poly(ADP-ribose) polymerases, are key proteins involved in DNA damage repair. Inhibition of the enzymatic activity of PARP1/2 results in the accumulation of a large number of single-strand DNA breaks in cells, which subsequently develop into double-strand breaks. In cancer cells with defective DNA homologous recombination repair, these double-strand DNA breaks cannot be properly repaired, ultimately leading to the death of cancer cells. Therefore, PARP1/2 exhibit a synthetic lethal effect with genes involved in DNA double-strand break repair (primarily homologous recombination repair).

PARG (poly ADP-ribose glycohydrolase), as the most important poly(ADP-ribose) hydrolase in cells, is responsible for more than 90% of poly(ADP-ribose) hydrolysis and can hydrolyze the poly(ADP-ribose) chains produced by PARP-catalyzed reactions. Although PARG and PARP have opposite functions with respect to their target proteins, both play crucial regulatory roles in DNA damage repair and under replication stress. When DNA damage occurs, PARP is recruited to the damage site to catalyze the formation of poly(ADP-ribose) chains on itself. These branched chains then serve as a platform to recruit other DNA repair factors, facilitating repair at the DNA damage site. After DNA repair, PARG hydrolyzes these ADP-ribose branched chains, completing the entire repair cycle. On the other hand, the continuous proliferation of cancer cells generates replication stress, which requires stalling of the DNA replication fork to repair DNA damage near the replication fork. During the process, cells also need to recruit PARP to the stalled replication fork. Through rapid synthesis of its own poly(ADP-ribose) branched chains, PARP inhibits the enzymatic activity of RECQ1 that promotes restart of the replication fork while simultaneously recruiting a series of repair factors to complete DNA damage repair. Once repair is completed, PARG rapidly hydrolyzes the poly(ADP-ribose) branched chains, promoting the release of PARP from the stalled replication fork and restoring the enzymatic activity of RECQ1 to restart replication. Since knockout of PARG in cells exacerbates replication stress and DNA damage, PARG has the potential to serve as a synthetic lethal target in tumors with defective DNA damage repair or under replication stress.

Given the similarity in biological functions between PARG and PARP, PARG inhibitors, like PARP inhibitors, have the potential to be effective in tumor patients with defects in BRCA genes, or even those with homologous recombination deficiency (HRD). In addition, PARG exhibits synthetic lethal interactions with genes associated with replication stress. Therefore, PARG inhibitors have the potential to be effective in tumors with defects in replication-related genes.

Currently, few of PARG inhibitors in development have entered clinical stages, with most molecules still in early stages. Therefore, developing a novel class of PARG inhibitors is of significant research importance.

### SUMMARY

The present disclosure aims to provide a class of novel compounds with PARG inhibitory activity, a pharmaceutical composition comprising the compound, a useful intermediate for preparing the compound, and the use of the compound in the manufacture of a medicament for the treatment of cancer.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 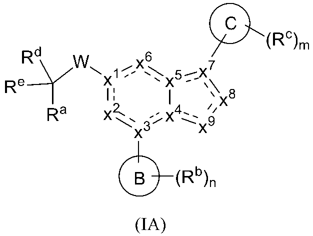 wherein
x¹ is selected from the group consisting of C, CR^{x1}, and N, wherein R^{x1} is selected from the group consisting of H, halogen, hydroxyl, -NRⁿ¹Rⁿ², C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NRⁿ¹Rⁿ², C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NRⁿ¹Rⁿ², C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NRⁿ¹Rⁿ², C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NRⁿ¹Rⁿ², C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, deuterium, halogen, hydroxyl, -NRⁿ¹Rⁿ², cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, -S(O)ₚR^{ba}, -N=S(O)R^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, deuterium, halogen, hydroxyl, -NRⁿ¹Rⁿ², cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, S(O)ₚR^{ba}, S(O)ₚNR^{be}R^{bf}, -C(O)OR^{bb}, and - C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -OR^{f}, - C(O)R^{ba}, S(O)ₚR^{ba}, S(O)ₚNR^{be}R^{bf}, -C(O)OR^{bb}, -C(O)NR^{bc}R^{bd}, C₁₋₆ deuteroalkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NRⁿ¹Rⁿ², nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
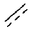 is a single bond or a double bond;
R^{a}, R^{d}, and R^{e} are identical or different, and are each independently selected from the group consisting of hydrogen, deuterium, halogen, C₁₋₆ alkyl, cyano, -NRⁿ¹Rⁿ², hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 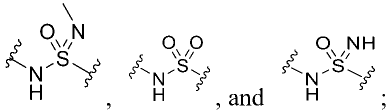
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, deuterium, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3-to 8-membered heterocyclyl, 6- to 10-membered aryl, 5- to 10-membered heteroaryl, 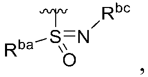 -OR^{f}, -C(O)R^{ba}, -C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
Rⁿ¹, Rⁿ², R^{a1}, and R^{a2} are identical or different, and are each independently selected from the group consisting of hydrogen, deuterium, C₁₋₆ alkyl, C₁₋₆ haloalkoxy, C₁₋₆ hydroxyalkyl, -OR^{f}, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, amino, nitro, hydroxyl, and C₁₋₆ hydroxyalkyl;
R^{ba}, R^{bb}, R^{bc}, R^{bd}, R^{be}, and R^{bf} are identical or different, and are each independently selected from the group consisting of hydrogen, deuterium, C₁₋₆ alkyl, C₁₋₆ haloalkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, C₁₋₆ alkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, -OR^{f}, -C(O)R^{ba1}, S(O)ₚR^{ba1}, S(O)ₚNR^{be1}R^{bf1}, -C(O)OR^{bb1}, -C(O)NR^{bc1}R^{bd1}, cyano, -NR^{a1}R^{a2}, nitro, hydroxyl, and C₁₋₆ hydroxyalkyl;
alternatively, Rⁿ¹ and Rⁿ², R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, deuterium, halogen, C₁₋₆ alkyl, cyano, -NRⁿ¹Rⁿ², hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5-to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{ba1}, R^{bb1}, R^{bc1}, R^{bd1}, R^{be1}, R^{bf1}, R^{ba2}, R^{bb2}, R^{bc2}, R^{bd2}, R^{be2}, and R^{bf2} are identical or different, and are each independently selected from the group consisting of hydrogen, deuterium, C₁₋₆ alkyl, C₁₋₆ haloalkoxy, C₁₋₆ alkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, -NR^{a1}R^{a2}, -OR^{f}, nitro, hydroxyl, and C₁₋₆ hydroxyalkyl;
R^{f} is selected from the group consisting of hydrogen, C₁₋₆ alkyl, C₁₋₆ hydroxyalkyl, 3-to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkyl, C₁₋₆ deuteroalkoxy, cyano, -NR^{a1}R^{a2}, nitro, hydroxyl, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 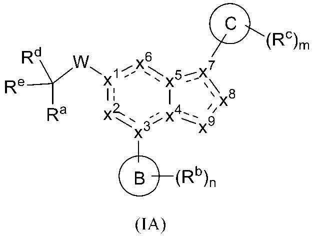 wherein
x¹ is selected from the group consisting of C, CR^{x1}, and N, wherein R^{x1} is selected from the group consisting of H, halogen, hydroxyl, -NR^{b1}R^{b2}, C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NR^{b1}R^{b2}, C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NR^{b1}R^{b2}, C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NR^{b1}R^{b2}, C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NR^{b1}R^{b2}, C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -ORf, -C(O)R^{ba}, -S(O)ₚR^{ba}, -N=S(O)R^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, S(O)ₚR^{ba}, S(O)ₚNR^{be}R^{bf}, -C(O)OR^{bb}, and - C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -OR^{f}, - C(O)R^{ba}, S(O)ₚR^{ba}, S(O)ₚNR^{be}R^{bf}, -C(O)OR^{bb}, -C(O)NR^{bc}R^{bd}, C₁₋₆ deuteroalkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NRⁿ¹Rⁿ², nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
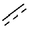 is a single bond or a double bond;
R^{a}, R^{d}, and R^{e} are identical or different, and are each independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 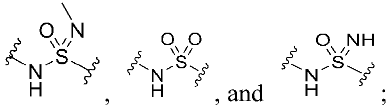
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, deuterium, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3-to 8-membered heterocyclyl, 6- to 10-membered aryl, 5- to 10-membered heteroaryl, 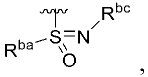 -OR^{f}, -C(O)R^{ba}, -C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a2}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{b1}, R^{b2}, R^{a1}, and R^{a2} are identical or different, and are each independently selected from the group consisting of hydrogen, deuterium, C₁₋₆ alkyl, C₁₋₆ haloalkoxy, C₁₋₆ hydroxyalkyl, -OR^{f}, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, amino, nitro, hydroxyl, and C₁₋₆ hydroxyalkyl;
R^{ba}, R^{bb}, R^{bc}, R^{bd}, R^{be}, and R^{bf} are identical or different, and are each independently selected from the group consisting of hydrogen, deuterium, C₁₋₆ alkyl, C₁₋₆ haloalkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, C₁₋₆ alkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, -OR^{f}, -C(O)R^{ba1}, S(O)ₚR^{ba1}, S(O)ₚNR^{be1}R^{bf1}, -C(O)OR^{bb1}, -C(O)NR^{bc1}R^{bd1}, cyano, -NR^{a1}R^{a2}, nitro, hydroxyl, and C₁₋₆ hydroxyalkyl;
alternatively, R^{b1} and R^{b2}, R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5-to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{ba1}, R^{bb1}, R^{bc1}, R^{bd1}, R^{be1}, R^{bf1}, R^{ba2}, R^{bb2}, R^{bc2}, R^{bd2}, R^{be2}, and R^{bf2} are identical or different, and are each independently selected from the group consisting of hydrogen, deuterium, C₁₋₆ alkyl, C₁₋₆ haloalkoxy, C₁₋₆ alkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, -NR^{a1}R^{a2}, -OR^{f}, nitro, hydroxyl, and C₁₋₆ hydroxyalkyl;
R^{f} is selected from the group consisting of hydrogen, C₁₋₆ alkyl, C₁₋₆ hydroxyalkyl, 3-to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkyl, C₁₋₆ deuteroalkoxy, cyano, -NR^{a1}R^{a2}, nitro, hydroxyl, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 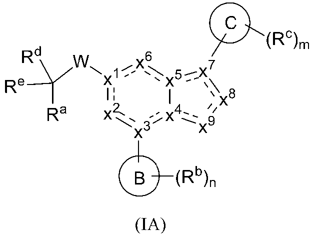 wherein
x¹ is selected from the group consisting of C, CR^{x1}, and N, wherein R^{x1} is selected from the group consisting of H, halogen, hydroxyl, -NR^{b1}R^{b2}, C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NR^{b1}R^{b2}, C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NR^{b1}R^{b2}, C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NR^{b1}R^{b2}, C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NR^{b1}R^{b2}, C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, S(O)ₚR^{ba}, S(O)ₚNR^{be}R^{bf}, -C(O)OR^{bb}, and - C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, S(O)ₚR^{ba}, S(O)ₚNR^{be}R^{bf}, -C(O)OR^{bb}, and - C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -OR^{f}, - C(O)R^{ba}, S(O)ₚR^{ba}, S(O)ₚNR^{be}R^{bf}, -C(O)OR^{bb}, -C(O)NR^{bc}R^{bd}, C₁₋₆ deuteroalkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl;
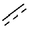 is a single bond or a double bond;
R^{a}, R^{d}, and R^{e} are identical or different, and are each independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 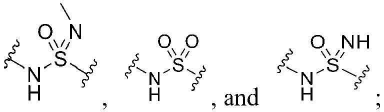
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, deuterium, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3-to 8-membered heterocyclyl, 6- to 10-membered aryl, 5- to 10-membered heteroaryl, 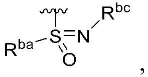 -OR^{f}, -C(O)R^{ba}, -C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{b1}, R^{b2}, R^{a1}, and R^{a2} are identical or different, and are each independently selected from the group consisting of hydrogen, deuterium, C₁₋₆ alkyl, C₁₋₆ haloalkoxy, C₁₋₆ hydroxyalkyl, -OR^{f}, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, amino, nitro, hydroxyl, and C₁₋₆ hydroxyalkyl;
R^{ba}, R^{bb}, R^{bc}, R^{bd}, R^{be}, and R^{bf} are identical or different, and are each independently selected from the group consisting of hydrogen, deuterium, C₁₋₆ alkyl, C₁₋₆ haloalkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, C₁₋₆ alkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, -OR^{f}, -C(O)R^{ba1}, S(O)ₚR^{ba1}, S(O)ₚNR^{be1}R^{bf1}, -C(O)OR^{bb1}, -C(O)NR^{bc1}R^{bd1}, cyano, -NR^{a1}R^{a2}, nitro, hydroxyl, and C₁₋₆ hydroxyalkyl;
alternatively, R^{b1} and R^{b2}, R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5-to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{ba1}, R^{bb1}, R^{bc1}, R^{bd1}, R^{be1}, R^{bf1}, R^{ba2}, R^{bb2}, R^{bc2}, R^{bd2}, R^{be2}, and R^{bf2} are identical or different, and are each independently selected from the group consisting of hydrogen, deuterium, C₁₋₆ alkyl, C₁₋₆ haloalkoxy, C₁₋₆ alkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, -NR^{a1}R^{a2}, -OR^{f}, nitro, hydroxyl, and C₁₋₆ hydroxyalkyl;
R^{f} is selected from the group consisting of hydrogen, C₁₋₆ alkyl, C₁₋₆ hydroxyalkyl, 3-to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkyl, C₁₋₆ deuteroalkoxy, cyano, -NR^{a1}R^{a2}, nitro, hydroxyl, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 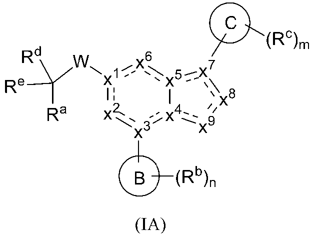 wherein
x¹ is selected from the group consisting of C, CR^{x1}, and N, wherein R^{x1} is selected from the group consisting of H, halogen, hydroxyl, -NR^{b1}R^{b2}, C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NR^{b1}R^{b2}, C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NR^{b1}R^{b2}, C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NR^{b1}R^{b2}, C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NR^{b1}R^{b2}, C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, deuterium, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
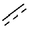 is a single bond or a double bond;
R^{a}, R^{d}, and R^{e} are identical or different, and are each independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 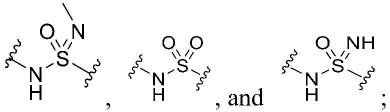
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, deuterium, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3-to 8-membered heterocyclyl, 6- to 10-membered aryl, 5- to 10-membered heteroaryl, -C(O)R^{ba}, -C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl;
R^{b1}, R^{b2}, R^{a1}, R^{a2}, R^{ba}, R^{bb}, R^{bc}, R^{bd}, R^{be}, and R^{bf} are identical or different, and are each independently selected from the group consisting of hydrogen, deuterium, C₁₋₆ alkyl, C₁₋₆ haloalkoxy, C₁₋₆ hydroxyalkyl, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, and C₁₋₆ hydroxyalkyl;
alternatively, R^{b1} and R^{b2}, R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, deuterium, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5-to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl,-C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 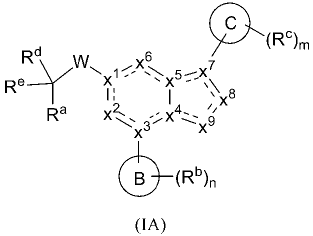 wherein
x¹ is selected from the group consisting of C, CR^{x1}, and N, wherein R^{x1} is selected from the group consisting of H, halogen, hydroxyl, -NR^{b1}R^{b2}, C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NR^{b1}R^{b2}, C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NR^{b1}R^{b2}, C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NR^{b1}R^{b2}, C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NR^{b1}R^{b2}, C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR3^{a1}R^{a2}, nitro, hydroxyl, C₁₋₆ hydroxyalkyl, -C(O)R^{ba}, S(O)ₚR^{ba}, S(O)ₚNR^{be}R^{bf}, - C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
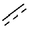 is a single bond or a double bond;
R^{a}, R^{d}, and R^{e} are identical or different, and are each independently selected from the group consisting of hydrogen, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3-to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl,-C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 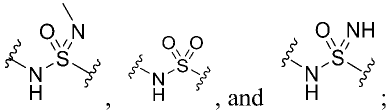
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba},-C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl,-C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl;
R^{b1}, R^{b2}, R^{a1}, R^{a2}, R^{ba}, R^{bb}, R^{bc}, R^{bd}, R^{be}, and R^{bf} are identical or different, and are each independently selected from the group consisting of hydrogen, C₁₋₆ alkyl, C₁₋₆ haloalkoxy, C₁₋₆ hydroxyalkyl, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, and C₁₋₆ hydroxyalkyl;
alternatively, R^{b1} and R^{b2}, R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf},-C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

The present disclosure provides a compound of formula (IA), a pharmaceutically acceptable salt thereof, a stereoisomer thereof, a prodrug thereof, an N-oxide thereof, a solvate thereof, or an isotopic derivative thereof, 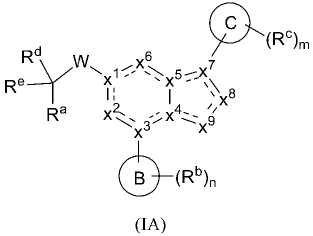 wherein
x¹ is selected from the group consisting of C, CR^{x1}, and N, wherein R^{x1} is selected from the group consisting of H, halogen, hydroxyl, -NR^{b1}R^{b2}, C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3-to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NR^{b1}R^{b2}, C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NR^{b1}R^{b2}, C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NR^{b1}R^{b2}, C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NR^{b1}R^{b2}, C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, halogen, hydroxyl, -NR^{b1}R^{b2}, cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3-to 7-membered ring; wherein the 3- to 7-membered ring is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
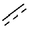 is a single bond or a double bond;
R^{a}, R^{d}, and R^{e} are identical or different, and are each independently selected from the group consisting of hydrogen, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, -C(O)OR^{bb},-C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
W is selected from the group consisting of 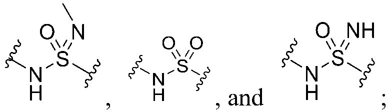
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NR^{b1}R^{b2}, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba},-C(O)OR^{bb}, -C(O)NR^{bc}R^{bd}, and S(O)₂NR^{be}R^{bf}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, and C₁₋₆ hydroxyalkyl;
R^{b1}, R^{b2}, R^{a1}, R^{a2}, R^{ba}, R^{bb}, R^{bc}, R^{bd}, R^{be}, and R^{bf} are identical or different, and are each independently selected from the group consisting of hydrogen, C₁₋₆ alkyl, C₁₋₆ haloalkoxy, C₁₋₆ hydroxyalkyl, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, and C₁₋₆ hydroxyalkyl;
alternatively, R^{b1} and R^{b2}, R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, halogen, C₁₋₆ alkyl, cyano, -NR^{b1}R^{b2}, hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

In some embodiments of the present disclosure, in the compound of formula (IA), the pharmaceutically acceptable salt thereof, the stereoisomer thereof, the prodrug thereof, the N-oxide thereof, the solvate thereof, or the isotopic derivative thereof, R^{d} and R^{e}, together with the atom to which they are attached, form a 3- to 8-membered cycloalkyl or 3- to 8-membered heterocyclyl ring, preferably a 3- to 6-membered cycloalkyl or 3- to 6-membered heterocyclyl ring, more preferably a cyclopropyl ring.

In some embodiments of the present disclosure, in the compound of formula (IA), the pharmaceutically acceptable salt thereof, the stereoisomer thereof, the prodrug thereof, the N-oxide thereof, the solvate thereof, or the isotopic derivative thereof, R^{d} and R^{e}, together with the atom to which they are attached, form an oxetanyl ring.

In some embodiments of the present disclosure, the compound of formula (IA), the pharmaceutically acceptable salt thereof, the stereoisomer thereof, the prodrug thereof, the N-oxide thereof, the solvate thereof, or the isotopic derivative thereof is a compound of formula (I), a pharmaceutically acceptable salt thereof, or a stereoisomer thereof, 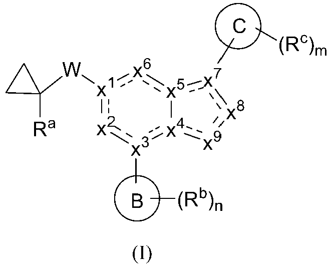 wherein 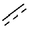 , ring B, ring C, R^{b}, R^{a}, R^{c}, m, n, W, and x¹ to x⁹ are as defined in any embodiment of formula (IA).

The present disclosure provides a compound of formula (I), a pharmaceutically acceptable salt thereof, a stereoisomer thereof, a prodrug thereof, an N-oxide thereof, a solvate thereof, or an isotopic derivative thereof, 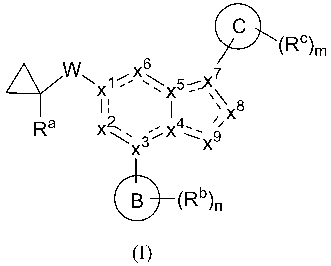 wherein
x¹ is selected from the group consisting of C, CR^{x1}, and N, wherein R^{x1} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x² is selected from the group consisting of CR^{x2}, N, and C(=O), wherein R^{x2} is selected from the group consisting of H, halogen, hydroxyl, amino, cyano, C₂₋₆ alkenyl, C₂₋₆ alkynyl, C₁₋₆ alkyl, C₁₋₆ aminoalkyl, C₂₋₆ haloalkenyl, C₂₋₆ haloalkynyl, C₁₋₆ haloalkyl, C₁₋₆ haloalkoxy, -OC₁₋₆ alkyl, C₁₋₆ hydroxyalkyl, -NHC₁₋₆ alkyl, and -N(C₁₋₆ alkyl)₂;
x³ is selected from the group consisting of C, CR^{x3}, and N, wherein R^{x3} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁴ is selected from the group consisting of C, CR^{x4}, and N, wherein R^{x4} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁵ is selected from the group consisting of C, CR^{x5}, and N, wherein R^{x5} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6}, N, and C(=O), wherein R^{x6} is selected from the group consisting of H, halogen, hydroxyl, amino, cyano, C₂₋₆ alkenyl, C₂₋₆ alkynyl, C₁₋₆ alkyl, C₁₋₆ aminoalkyl, C₂₋₆ haloalkenyl, C₂₋₆ haloalkynyl, C₁₋₆ haloalkyl, C₁₋₆ haloalkoxy, -OC₁₋₆ alkyl, C₁₋₆ hydroxyalkyl, -NHC₁₋₆ alkyl, and -N(C₁₋₆ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, halogen, hydroxyl, amino, cyano, C₂₋₆ alkenyl, C₂₋₆ alkynyl, C₁₋₆ alkyl, C₁₋₆ aminoalkyl, C₂₋₆ haloalkenyl, C₂₋₆ haloalkynyl, C₁₋₆ haloalkyl, C₁₋₆ haloalkoxy,-OC₁₋₆ alkyl, C₁₋₆ hydroxyalkyl, -NHC₁₋₆ alkyl, and -N(C₁₋₆ alkyl)₂;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, hydroxyl, amino, cyano, C₂₋₆ alkenyl, C₂₋₆ alkynyl, C₁₋₆ alkyl, C₁₋₆ aminoalkyl, C₂₋₆ haloalkenyl, C₂₋₆ haloalkynyl, C₁₋₆ haloalkyl, C₁₋₆ haloalkoxy,-OC₁₋₆ alkyl, C₁₋₆ hydroxyalkyl, -NHC₁₋₆ alkyl, and -N(C₁₋₆ alkyl)₂; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
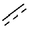 is a single bond or a double bond;
R^{a} is selected from the group consisting of hydrogen, halogen, C₁₋₆ alkyl, cyano, amino, hydroxyl, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ hydroxyalkyl, C₁₋₆ aminoalkyl, -C₁₋₆ alkoxy-C₁₋₆ alkyl, - C(O)R^{ba}, -C(O)OR^{bb}, and -C(O)NR^{bc}R^{bd};
W is selected from the group consisting of 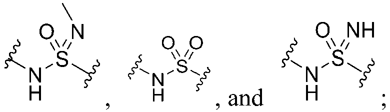
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, halogen, C₁₋₆ alkyl, cyano, amino, hydroxyl, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ hydroxyalkyl, C₁₋₆ aminoalkyl, -C₁₋₆ alkoxy-C₁₋₆ alkyl, -C(O)R^{ba}, -C(O)OR^{bb}, -C(O)NR^{bc}R^{bd}, and S(O)₂NR^{be}R^{bf};
R^{ba}, R^{bb}, R^{bc}, R^{bd}, R^{be}, and R^{bf} are identical or different, and are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, C₁₋₆ alkyl, cyano, amino, hydroxyl, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ hydroxyalkyl, C₁₋₆ aminoalkyl, -C₁₋₆ alkoxy-C₁₋₆ alkyl, -C(O)R^{ba}, -C(O)OR^{bb}, and -C(O)NR^{bc}R^{bd};
n is selected from the group consisting of 0, 1, 2, and 3;
m is selected from the group consisting of 0, 1, 2, and 3;
p is selected from the group consisting of 0, 1, and 2;
   and,
when W is 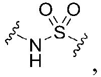 and R^{a} is selected from the group consisting of -CH₃, cyano, - CH₂F, and -CONH₂; the structural moiety 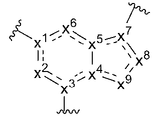 is not 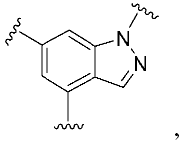 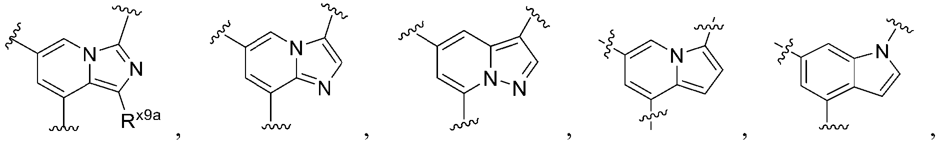 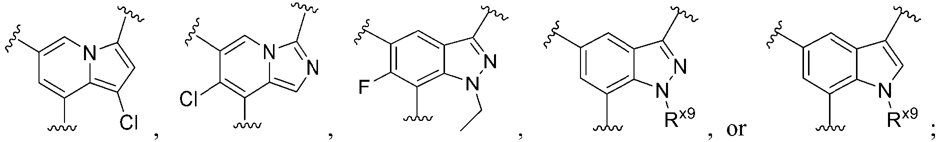 wherein R^{x9} is selected from the group consisting of H and C₁₋₄ alkyl, and R^{x9a} is selected from the group consisting of H, -CH₃, F, and Cl.

The present disclosure provides a compound of formula (I), a pharmaceutically acceptable salt thereof, a stereoisomer thereof, a prodrug thereof, an N-oxide thereof, a solvate thereof, or an isotopic derivative thereof, 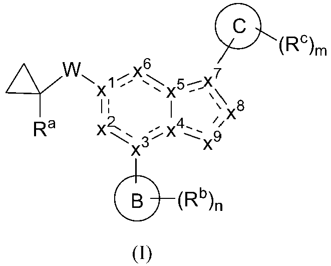 wherein
x¹ is selected from the group consisting of C, CR^{x1}, and N, wherein R^{x1} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x² is selected from the group consisting of CR^{x2}, N, and C(=O), wherein R^{x2} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x³ is selected from the group consisting of C, CR^{x3}, and N, wherein R^{x3} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁴ is selected from the group consisting of C, CR^{x4}, and N, wherein R^{x4} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁵ is selected from the group consisting of C, CR^{x5}, and N, wherein R^{x5} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6}, N, and C(=O), wherein R^{x6} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
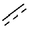 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
W is selected from the group consisting of 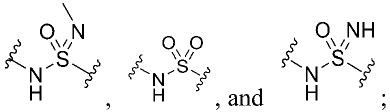
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}, wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2;
   and,
when W is 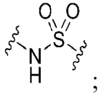 the structural moiety 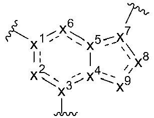 is not 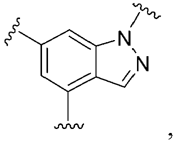 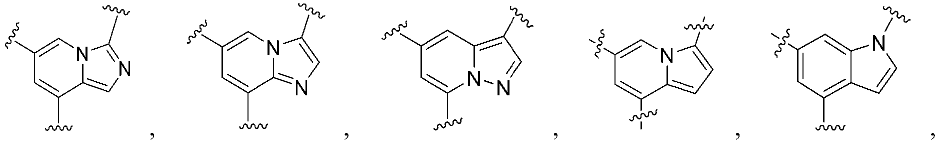 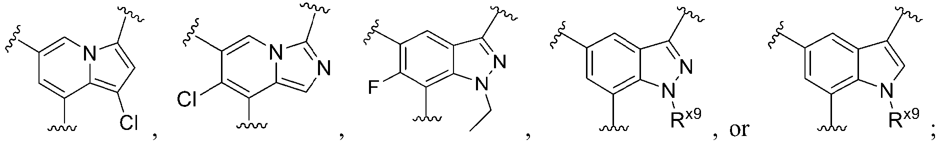 wherein R^{x9} is selected from the group consisting of H and C₁₋₄ alkyl.

The present disclosure provides a compound of formula (I), a pharmaceutically acceptable salt thereof, a stereoisomer thereof, a prodrug thereof, an N-oxide thereof, a solvate thereof, or an isotopic derivative thereof, 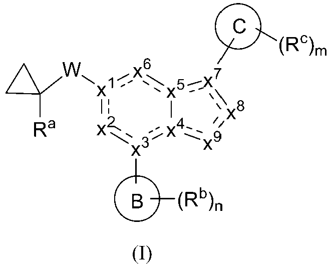 wherein
x¹ is selected from the group consisting of C and N;
x² is selected from the group consisting of CR^{x2}, N, and C(=O), wherein R^{x2} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x³ is selected from the group consisting of C and N;
x⁴ is selected from the group consisting of C and N;
x⁵ is selected from the group consisting of C and N;
x⁶ is selected from the group consisting of CR^{x6}, N, and C(=O), wherein R^{x6} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of C and N;
x⁸ is selected from the group consisting of N, O, CR^{x8}, CR^{x8}R^{x8}, NR^{x8}, S(O)₂, and C(=O), wherein R^{x8} is selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, and CR^{x9}R^{x9}, wherein R^{x9} is selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
W is selected from the group consisting of 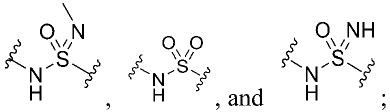
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
R^{b} is selected from the group consisting of hydrogen, hydroxyl, halogen, C₁₋₆ alkyl, C₁₋₆ alkoxy, C₁₋₆ haloalkyl, C₁₋₆ haloalkoxy, -C(O)R^{ba}, -C(O)OR^{bb}, -C(O)NR^{bc}R^{bd}, and S(O)₂NR^{be}R^{bf}, wherein R^{ba} R^{bb}, R^{bc}, R^{bd}, R^{be}, and R^{bf} are each independently selected from the group consisting of hydrogen and C₁₋₄ alkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
R^{c} is selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
   and,
the structural moiety 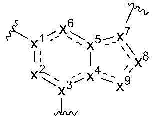 is not

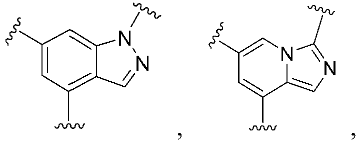

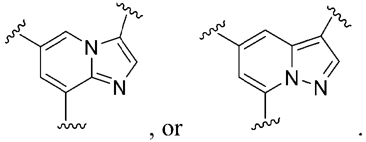

In some embodiments of the present disclosure, the structural moiety 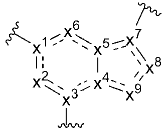 is 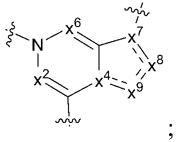 wherein 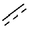 , x², x⁴, x⁶, x⁷, x⁸, and x⁹ are as defined in any embodiment of formula (I) or any embodiment of formula (IA).

The present disclosure provides a compound of formula (I-1) or formula (I-1A), a pharmaceutically acceptable salt thereof, a stereoisomer thereof, a prodrug thereof, an N-oxide thereof, a solvate thereof, or an isotopic derivative thereof: 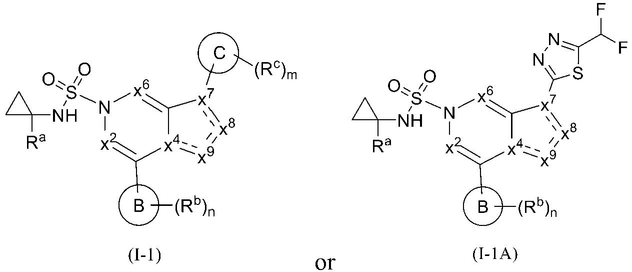 wherein 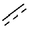 , x², x⁴, x⁶, x⁷, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-1) or formula (I-1A), the pharmaceutically acceptable salt thereof, the stereoisomer thereof, the prodrug thereof, the N-oxide thereof, the solvate thereof, or the isotopic derivative thereof,
x² is selected from the group consisting of CR^{x2} and N, wherein R^{x2} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x⁴ is selected from the group consisting of C, CR^{x4}, and N, wherein R^{x4} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6} and N, wherein R^{x6} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
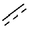 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf},
R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2.

In some embodiments of the present disclosure, the structural moiety 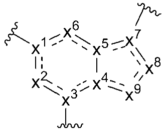 is 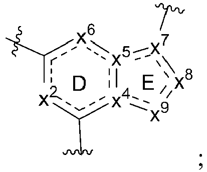 wherein both ring D and ring E are aromatic rings; 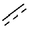 , x², x⁴, x⁵, x⁶, x⁷, x⁸, and x⁹ are as defined in any embodiment of formula (I) or any embodiment of formula (IA).

The present disclosure provides a compound of formula (I-2) or formula (I-2A), a pharmaceutically acceptable salt thereof, a stereoisomer thereof, a prodrug thereof, an N-oxide thereof, a solvate thereof, or an isotopic derivative thereof: 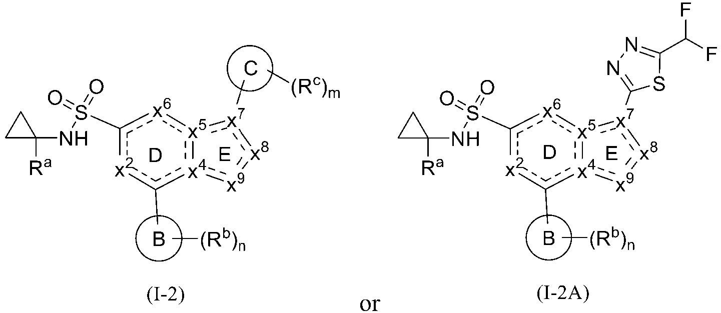 wherein 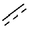 , x², x⁴, x⁵, x⁶, x⁷, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-2) or formula (I-2A), the pharmaceutically acceptable salt thereof, the stereoisomer thereof, the prodrug thereof, the N-oxide thereof, the solvate thereof, or the isotopic derivative thereof,
x² is selected from the group consisting of CR^{x2}, N, and C(=O), wherein R^{x2} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁴ is selected from the group consisting of C, CR^{x4}, and N, wherein R^{x4} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁵ is selected from the group consisting of C, CR^{x5}, and N, wherein R^{x5} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6}, N, and C(=O), wherein R^{x6} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
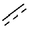 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}; wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2; provided that both ring D and ring E are aromatic rings; x² and x⁶ are not both CH, and 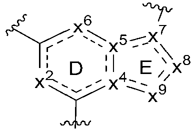 is not

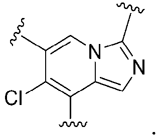

In some embodiments of the present disclosure, the structural moiety 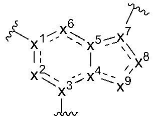 is 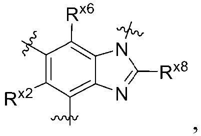 wherein R^{x2}, R^{x6}, and R^{x8} are as defined in any embodiment of formula (IA) or any embodiment of formula (I).

In some embodiments of the present disclosure, the structural moiety 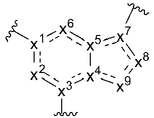 is selected from the group consisting of 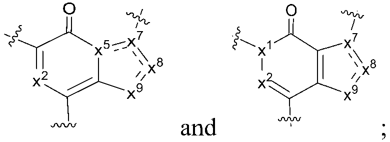 wherein 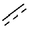 , x¹, x², x³, x⁷, x⁸, and x⁹ are as defined in any embodiment of formula (IA) or any embodiment of formula (I).

The present disclosure provides a compound of formula (I-3), formula (I-3A), formula (I-3'), or formula (I-3'A), a pharmaceutically acceptable salt thereof, a stereoisomer thereof, a prodrug thereof, an N-oxide thereof, a solvate thereof, or an isotopic derivative thereof: 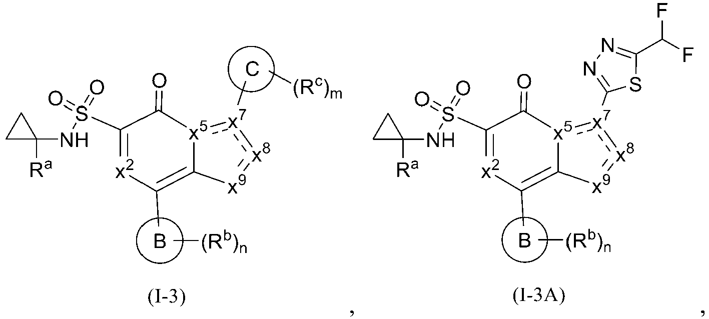 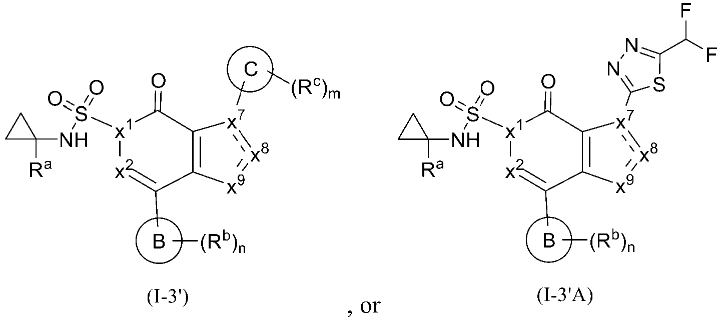 wherein 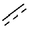 , x¹, x², x⁵, x⁷, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-3), formula (I-3A), formula (I-3'), or formula (I-3'A), the pharmaceutically acceptable salt thereof, the stereoisomer thereof, the prodrug thereof, the N-oxide thereof, the solvate thereof, or the isotopic derivative thereof,
x¹ is selected from the group consisting of CR^{x1} and N, wherein R^{x1} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x² is selected from the group consisting of CR^{x2} and N, wherein R^{x2} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x⁵ is selected from the group consisting of C, CR^{x5}, and N, wherein R^{x5} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
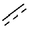 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}, wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2.

In some embodiments of the present disclosure, the structural moiety 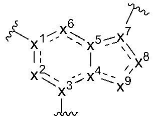 is selected from the group consisting of 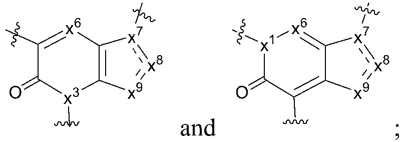 wherein 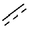 , x¹, x³, x⁶, x⁷, x⁸, and x⁹ are as defined in any embodiment of formula (IA) or any embodiment of formula (I).

The present disclosure provides a compound of formula (I-4), formula (I-4A), formula (I-4'), or formula (I-4'A), a pharmaceutically acceptable salt thereof, a stereoisomer thereof, a prodrug thereof, an N-oxide thereof, a solvate thereof, or an isotopic derivative thereof: 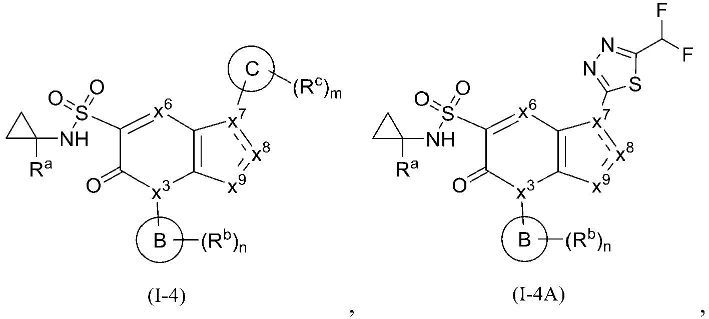 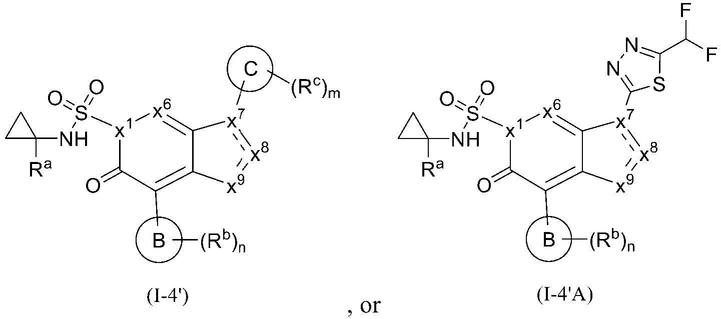 wherein 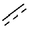 , x¹, x³, x⁶, x⁷, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-4), formula (I-4A), formula (I-4'), or formula (I-4'A), the pharmaceutically acceptable salt thereof, the stereoisomer thereof, the prodrug thereof, the N-oxide thereof, the solvate thereof, or the isotopic derivative thereof,
x¹ is selected from the group consisting of C, CR^{x1}, and N, wherein R^{x1} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x³ is selected from the group consisting of C, CR^{x3}, and N, wherein R^{x3} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6}, N, and C(=O), wherein R^{x6} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of C, CR^{x7}, and N, wherein R^{x7} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁸ is selected from the group consisting of N, O, CR^{x8}, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of CR^{x9}, N, O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
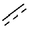 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
W is selected from the group consisting of 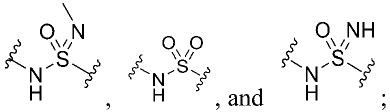
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}, wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2.

In some embodiments of the present disclosure, the structural moiety 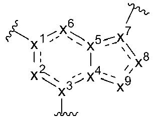 is 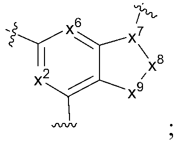 wherein 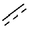 , x², x⁶, x⁷, x⁸, and x⁹ are as defined in any embodiment of formula (IA) or any embodiment of formula (I).

In some embodiments of the present disclosure, the compound of formula (I), the pharmaceutically acceptable salt thereof, the stereoisomer thereof, the prodrug thereof, the N-oxide thereof, the solvate thereof, or the isotopic derivative thereof is a compound of formula (I-5) or formula (I-5A), a pharmaceutically acceptable salt thereof, a stereoisomer thereof, a prodrug thereof, an N-oxide thereof, a solvate thereof, or an isotopic derivative thereof: 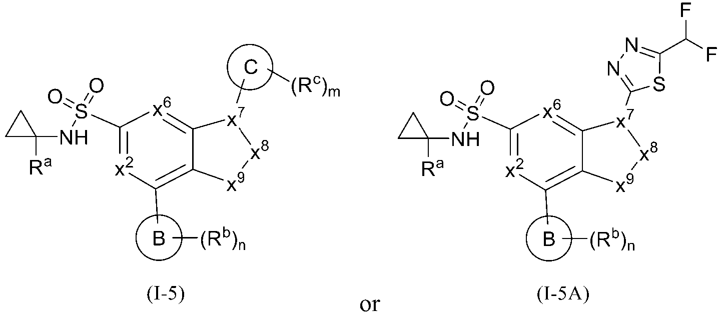 wherein 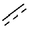 , x², x⁶, x⁷, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-5) or formula (I-5A), the pharmaceutically acceptable salt thereof, the stereoisomer thereof, the prodrug thereof, the N-oxide thereof, the solvate thereof, or the isotopic derivative thereof,
x² is selected from the group consisting of CR^{x2}, N, and C(=O), wherein R^{x2} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6}, N, and C(=O), wherein R^{x6} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁷ is selected from the group consisting of CR^{x7} and N, wherein R^{x7} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x⁸ is selected from the group consisting of O, CR^{x8}R^{x8}, NR^{x8}, S(O)ₚ, and C(=O), wherein R^{x8}, at each occurrence, is identical or different, and is independently selected from the group consisting of H, C≡CH, CN, halogen, and C₁₋₄ alkyl; alternatively, two R^{x8} attached to the same carbon atom together form a C₃₋₆ cycloalkyl ring;
x⁹ is selected from the group consisting of O, NR^{x9}, S(O)ₚ, and CR^{x9}R^{x9}, wherein R^{x9}, at each occurrence, is identical or different, and is independently selected from the group consisting of H, halogen, and C₁₋₄ alkyl; alternatively, two R^{x9} attached to the same carbon atom together form a C₃₋₆ cycloalkyl ring;
alternatively, R^{x8} and R^{x9}, together with the atom to which they are attached, form a 3-to 7-membered ring;
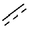 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}, wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2.

In some embodiments of the present disclosure, the structural moiety 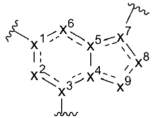 is 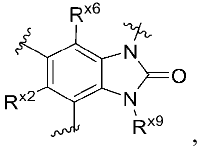 wherein R^{x2}, R^{x6}, and R^{x9} are as defined in any embodiment of formula (IA) or any embodiment of formula (I).

In some embodiments of the present disclosure, the structural moiety 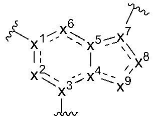 is 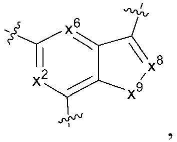 wherein x², x⁶, x⁸, and x⁹ are as defined in any embodiment of formula (IA) or any embodiment of formula (I).

The present disclosure provides a compound of formula (I-6) or formula (I-6A), a pharmaceutically acceptable salt thereof, a stereoisomer thereof, a prodrug thereof, an N-oxide thereof, a solvate thereof, or an isotopic derivative thereof: 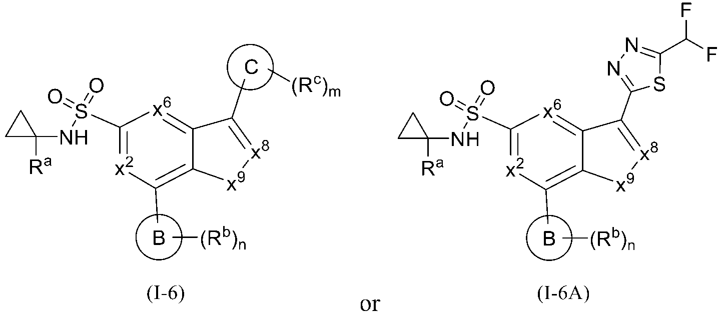 wherein 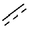 , x², x⁶, x⁸, x⁹, R^{a}, R^{b}, R^{c}, ring B, ring C, n, m, and p are as defined in any embodiment of formula (IA) or any embodiment of formula (I).

In some embodiments of the present disclosure, in the compound of formula (I-6) or formula (I-6A), the pharmaceutically acceptable salt thereof, the stereoisomer thereof, the prodrug thereof, the N-oxide thereof, the solvate thereof, or the isotopic derivative thereof, x² is selected from the group consisting of CR^{x2} and N, wherein R^{x2} is selected from the group consisting of H, halogen, hydroxyl, amino, C₁₋₄ alkyl, -OC₁₋₄ alkyl, -NHC₁₋₄ alkyl, and -N(C₁₋₄ alkyl)₂;
x⁶ is selected from the group consisting of CR^{x6} and N, wherein R^{x6} is selected from the group consisting of H, halogen, hydroxyl, amino, C₁₋₄ alkyl, -OC₁₋₄ alkyl, -NHC₁₋₄ alkyl, and - N(C₁₋₄ alkyl)₂;
x⁸ is selected from the group consisting of N and CR^{x8}, wherein R^{x8} is selected from the group consisting of H, C≡CH, CN, halogen, and C₁₋₄ alkyl;
x⁹ is selected from the group consisting of O and S;
alternatively, R^{x8} and R^{x9}, together with the atom to which they are attached, form a 3-to 7-membered ring;
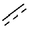 is a single bond or a double bond;
R^{a} is selected from the group consisting of hydrogen, cyano, formyl, -CONH₂, -CH₂OH, -CH₂OC₁₋₄ alkyl, C₁₋₄ alkyl, and C₁₋₄ haloalkyl;
ring B is selected from the group consisting of 5- to 7-membered monocyclic heterocyclyl, 7- to 12-membered spiro heterocyclyl, 6- to 10-membered bridged heterocyclyl, and 6- to 10-membered fused heterocyclyl;
each R^{b} is identical or different, and is independently selected from the group consisting of hydrogen, hydroxyl, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, -C(O)R^{ba}, -C(O)OR^{bb}, -C(O)NR^{bc}R^{bd}, and S(O)₂NR^{be}R^{bf}, wherein R^{ba}, R^{bb}, R^{bc}, R^{bd}, R^{be}, and R^{bf} are each independently selected from the group consisting of hydrogen, C₁₋₆ alkyl, C₁₋₆ haloalkyl, C₁₋₆ hydroxyalkyl, and C₁₋₆ aminoalkyl; alternatively, R^{bc} and R^{bd}, or R^{be} and R^{bf}, together with the nitrogen atom to which they are attached, form a 3- to 7-membered heterocyclyl ring;
ring C is 5- to 6-membered heteroaryl;
each R^{c} is identical or different, and is independently selected from the group consisting of hydrogen, halogen, hydroxyl, C₁₋₆ alkyl, C₁₋₆ alkoxy, C₁₋₆ haloalkyl, and C₁₋₆ haloalkoxy;
n is selected from the group consisting of 0, 1, 2, and 3;
m is selected from the group consisting of 0, 1, 2, and 3;
p is selected from the group consisting of 0, 1, and 2.

In some embodiments of the present disclosure, in the above formula containing ring C, ring C is 5-membered heteroaryl, preferably

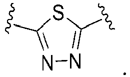

In some embodiments of the present disclosure, in the above formula containing R^{c}, each R^{c} is identical or different, and is independently C₁₋₆ haloalkyl; preferably, R^{c} is -CHF₂.

In some embodiments of the present disclosure, in the above formula containing ring C and R^{c}, ring C is 5-membered heteroaryl; each R^{c} is identical or different, and is independently C₁₋₆ haloalkyl; preferably, ring C is 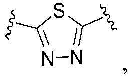 and R^{c} is -CHF₂.

In some embodiments of the present disclosure, in the above formula containing ring C and R^{c}, 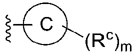 is

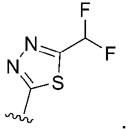

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II), a pharmaceutically acceptable salt thereof, a stereoisomer thereof, a prodrug thereof, an N-oxide thereof, a solvate thereof, or an isotopic derivative thereof: 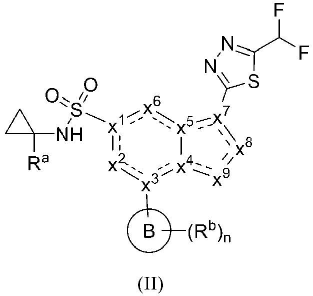 wherein 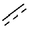 , x¹, x², x³, x⁴, x⁵, x⁶, x⁷, x⁸, x⁹, ring B, R^{b}, R^{a}, and n are as defined in any embodiment of formula (I) or any embodiment of formula (IA).

In some embodiments of the present disclosure, in the above formula containing R^{a}, R^{a} is selected from the group consisting of -CH₃, -CH₂F, -CN, and -CH₂=CHF; preferably, R^{a} is selected from the group consisting of -CH₃, -CH₂F, and -CN.

In some embodiments of the present disclosure, in the above formula containing R^{a}, R^{a} is selected from the group consisting of -CHF₂ and -CHF₃.

In some embodiments of the present disclosure, in the above formula containing R^{a}, R^{a} is -CD₃.

In some embodiments of the present disclosure, in the above formula containing R^{b}, each R^{b} is identical or different, and is independently selected from the group consisting of hydrogen, C₁₋₆ alkyl, -C(O)R^{ba}, -C(O)OR^{bb}, and -C(O)NR^{bc}R^{bd}; wherein R^{ba}, R^{bc}, and R^{bd} are as defined in any embodiment of formula (IA) or any embodiment of formula (I); preferably, each R^{b} is identical or different, and is independently selected from the group consisting of hydrogen, C₁₋₆ alkyl, -C(O)R^{ba}, and -C(O)NR^{bc}R^{bd}; wherein R^{ba}, R^{bc}, and R^{bd} are as defined in any embodiment of formula (IA) or any embodiment of formula (I).

In some embodiments of the present disclosure, in the above formula containing ring B, ring B is

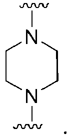

In some embodiments of the present disclosure, ring B is 6-membered heterocyclyl.

In some embodiments of the present disclosure, ring B is

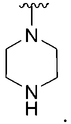

In some embodiments of the present disclosure, ring B is selected from the group consisting of

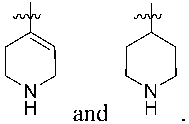

In some embodiments of the present disclosure, ring B is selected from the group consisting of

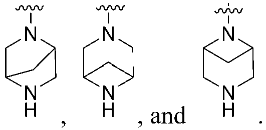

In some embodiments of the present disclosure, in the above formula containing R^{b}, R^{b} is

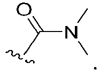

In some embodiments of the present disclosure, 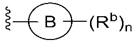 is selected from the group consisting of 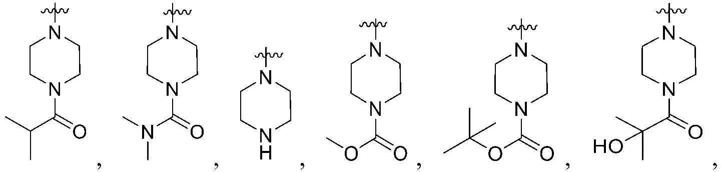 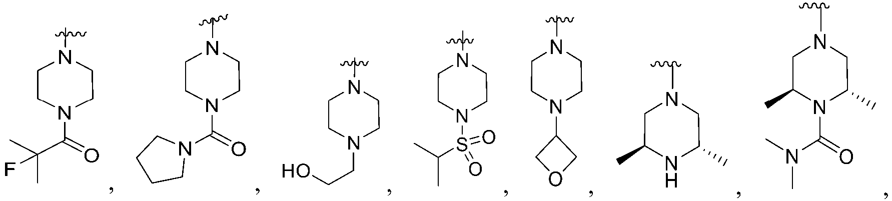 and

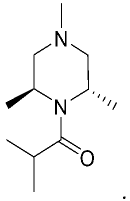

In some embodiments of the present disclosure, 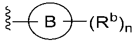 is selected from the group consisting of

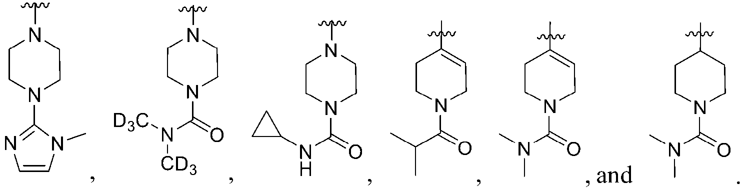

In some embodiments of the present disclosure, 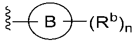 is selected from the group consisting of

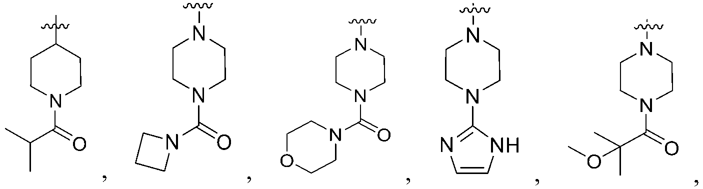

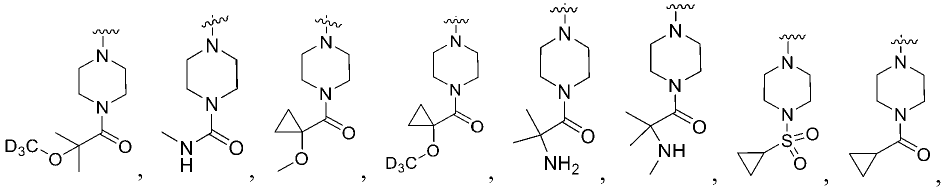

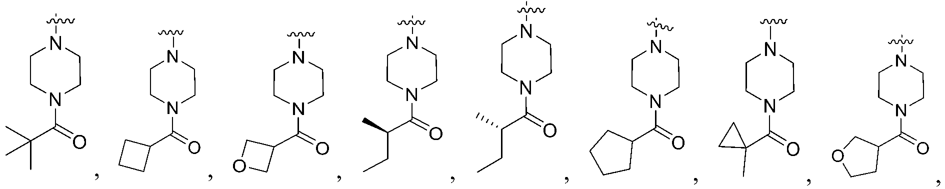

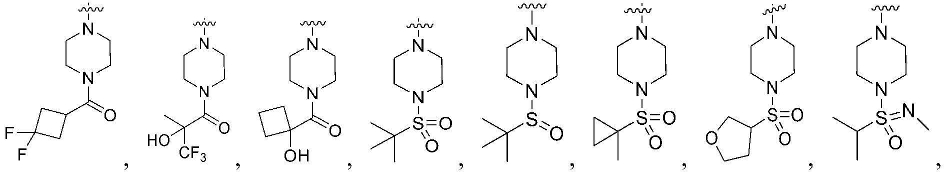

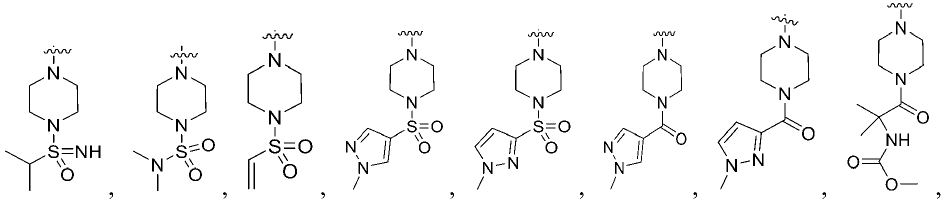

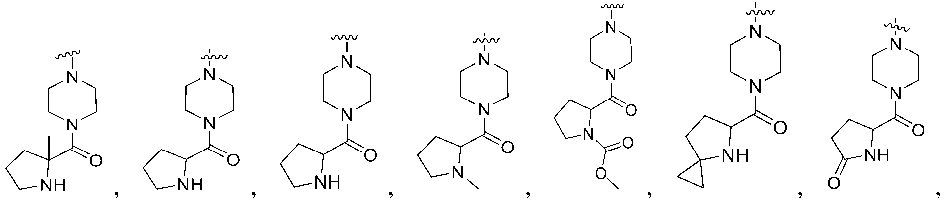

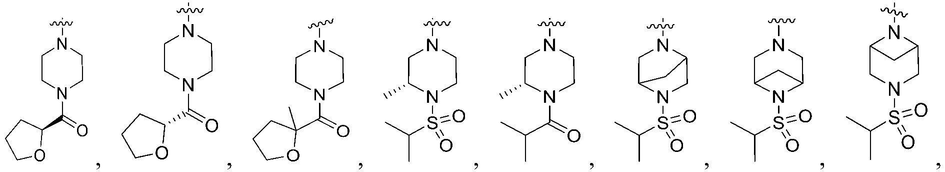

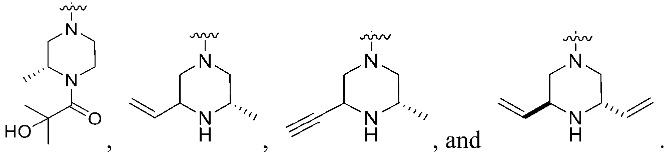

In some embodiments of the present disclosure, 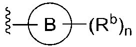 is

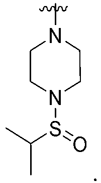

In some embodiments of the present disclosure, in the above formula containing ring B and R^{b}, 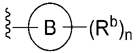 is 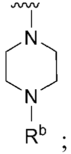 R^{b} is selected from the group consisting of C₁₋₆ hydroxyalkyl, 3- to 8-membered heterocyclyl, and -S(O)₂R^{ba}; R^{ba} is as defined in any embodiment of formula (IA) or any embodiment of formula (I); preferably, 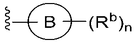 is 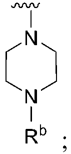 R^{b} is selected from the group consisting of C₁₋₆ hydroxyalkyl, 3- to 8-membered heterocyclyl, and -S(O)₂R^{ba}; R^{ba} is selected from the group consisting of hydrogen, C₁₋₆ alkyl, 3- to 8-membered heterocyclyl, C₁₋₆ haloalkyl, and C₁₋₆ hydroxyalkyl.

In some embodiments of the present disclosure, in the above formula containing ring B and R^{b}, 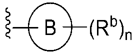 is 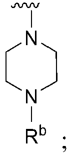 R^{b} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, - C(O)NR^{bc}R^{bd}, and 5- to 6-membered heteroaryl optionally substituted by C₁₋₆ alkyl; R^{ba}, R^{bb}, R^{bc}, and R^{bd} are as defined in claims 1 to 11; preferably, 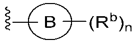 is 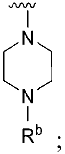 R^{b} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, and 5- to 6-membered heteroaryl optionally substituted by C₁₋₆ alkyl; R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, and 3- to 8-membered cycloalkyl.

In some embodiments of the present disclosure, in the above formula containing ring B and R^{b}, 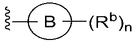 is

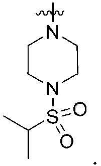

In some embodiments of the present disclosure, in the above formula containing ring B and R^{b}, 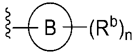 is 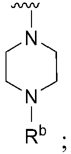 R^{b} is selected from the group consisting of hydrogen, C₁₋₆ alkyl, -C(O)R^{ba}, -C(O)OR^{bb}, and -C(O)NR^{bc}R^{bd}; R^{ba}, R^{bb}, R^{bc}, and R^{bd} are as defined in any embodiment of formula (IA) or any embodiment of formula (I); preferably, 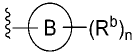 is

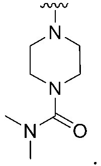

In some embodiments of the present disclosure, in the above formula containing ring B and R^{b}, 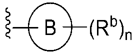 is 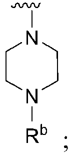 R^{b} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, - C(O)NR^{bc}R^{bd}, 5- to 6-membered heteroaryl optionally substituted by C₁₋₆ alkyl, 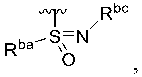 S(O)₂NR^{be}R^{bf}, and -S(O)R^{ba}; R^{ba}, R^{bb}, R^{bc}, R^{bd}, R^{be}, and R^{bf} are as defined in claims 1 to 11; preferably, 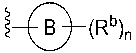 is 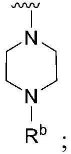 R^{b} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, - C(O)NR^{bc}R^{bd}, 5- to 6-membered heteroaryl optionally substituted by C₁₋₆ alkyl, 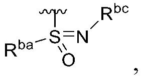 S(O)₂NR^{be}R^{bf}, and -S(O)R^{ba}; R^{ba}, R^{bb}, R^{bc}, R^{bd}, R^{be}, and R^{bf} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, 3- to 8-membered cycloalkyl, C₁₋₆ alkoxy, C₂₋₆ alkenyl, and 5- to 6-membered heteroaryl; wherein the C₁₋₆ alkyl, 3- to 8-membered heterocyclyl, 3- to 8-membered cycloalkyl, and 5- to 6-membered heteroaryl are optionally substituted by one or more substituents selected from the group consisting of C₁₋₆ alkyl, halogen, C₁₋₆ deuteroalkyl, C₁₋₆ haloalkyl, hydroxyl, C₁₋₆ alkoxy, C₁₋₆ deuteroalkoxy, - C(O)OR^{bb1}, and -NR^{a1}R^{a2}; R^{bb1}, R^{a1}, and R^{a2} are as defined in claims 1 to 11.

In some embodiments of the present disclosure, in the above formula containing ring B and R^{b}, 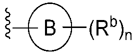 is 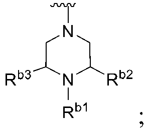 R^{b1} is selected from the group consisting of C₁₋₆ hydroxyalkyl, 3- to 8-membered heterocyclyl, and -S(O)₂R^{ba}; R^{b2} and R^{b3} are identical or different, and are each independently C₁₋₆ alkyl; R^{ba} is as defined in any embodiment of formula (IA) or any embodiment of formula (I); more preferably, 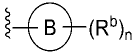 is selected from the group consisting of 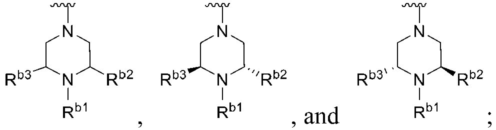 R^{b1} is selected from the group consisting of C₁₋₆ hydroxyalkyl, 3- to 8-membered heterocyclyl, and -S(O)₂R^{ba}; R^{b2} and R^{b3} are identical, and are both C₁₋₆ alkyl; R^{ba} is as defined in any embodiment of formula (IA) or any embodiment of formula (I); further preferably, 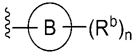 is selected from the group consisting of 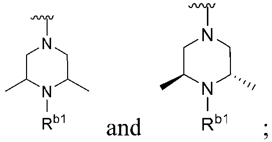 R^{b1} is selected from the group consisting of C₁₋₆ hydroxyalkyl, 3- to 8-membered heterocyclyl, and -S(O)₂R^{ba}; R^{ba} is as defined in any embodiment of formula (IA) or any embodiment of formula (I); most preferably, 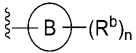 is 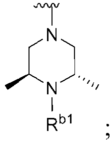 R^{b1} is selected from the group consisting of C₁₋₆ hydroxyalkyl, 3- to 8-membered heterocyclyl, and -S(O)₂R^{ba}; R^{ba} is selected from the group consisting of hydrogen and C₁₋₆ alkyl.

In some embodiments of the present disclosure, in the above formula containing ring B and R^{b}, 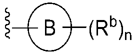 is 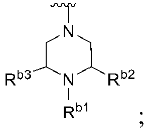 R^{b1}, R^{b2}, and R^{b3} are identical or different, and are each independently R^{b}; R^{b} is as defined in any embodiment of formula (IA) or any embodiment of formula (I); preferably, 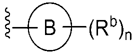 is 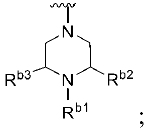 R^{b1} is selected from the group consisting of hydrogen, C₁₋₆ alkyl, -C(O)R^{ba}, -C(O)OR^{bb}, and -C(O)NR^{bc}R^{bd}; R^{b2} and R^{b3} are identical or different, and are each independently C₁₋₆ alkyl; R^{ba}, R^{bb}, R^{bc}, and R^{bd} are as defined in any embodiment of formula (IA) or any embodiment of formula (I); further preferably, 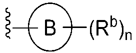 is selected from the group consisting of 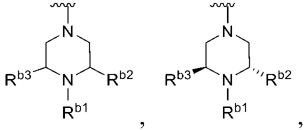 and 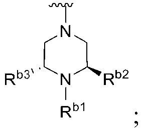 R^{b1} is selected from the group consisting of hydrogen, C₁₋₆ alkyl, -C(O)R^{ba}, - C(O)OR^{bb}, and -C(O)NR^{bc}R^{bd}; R^{b2} and R^{b3} are identical, and are both C₁₋₆ alkyl; R^{ba}, R^{bb}, R^{bc}, and R^{bd} are as defined in any embodiment of formula (IA) or any embodiment of formula (I); most preferably, 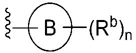 is selected from the group consisting of 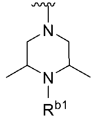 and 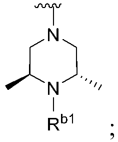 R^{b1} is selected from the group consisting of hydrogen, C₁₋₆ alkyl, -C(O)R^{ba}, - C(O)OR^{bb}, and -C(O)NR^{bc}R^{bd}; R^{ba}, R^{bb}, R^{bc}, and R^{bd} are as defined in any embodiment of formula (IA) or any embodiment of formula (I).

In some embodiments of the present disclosure, in the above formula containing ring B and R^{b}, 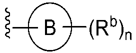 is 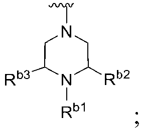 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, and 5- to 6-membered heteroaryl optionally substituted by C₁₋₆ alkyl; R^{b2} and R^{b3} are identical or different, and are each independently selected from the group consisting of hydrogen and C₁₋₆ alkyl; R^{ba}, R^{bb}, R^{bc}, and R^{bd} are as defined in any embodiment of formula (IA) or any embodiment of formula (I); more preferably, 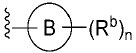 is selected from the group consisting of 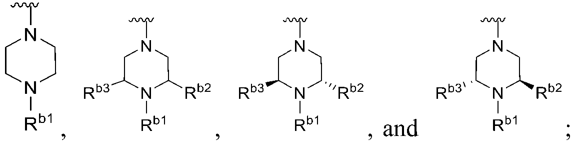 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, and 5- to 6-membered heteroaryl optionally substituted by C₁₋₆ alkyl; R^{b2} and R^{b3} are identical, and are both C₁₋₆ alkyl; R^{ba}, R^{bb}, R^{bc}, and R^{bd} are as defined in any embodiment of formula (IA) or any embodiment of formula (I); further preferably, 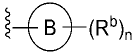 is selected from the group consisting of 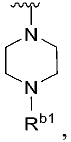 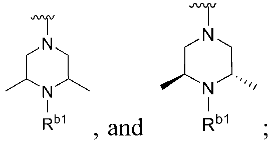 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, - C(O)NR^{bc}R^{bd}, and 5- to 6-membered heteroaryl optionally substituted by C₁₋₆ alkyl; R^{ba}, R^{bb}, R^{bc}, and R^{bd} are as defined in any embodiment of formula (IA) or any embodiment of formula (I); most preferably, 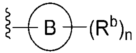 is selected from the group consisting of 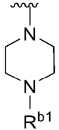 and 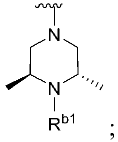 R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, and 5- to 6-membered heteroaryl optionally substituted by C₁₋₆ alkyl; R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, and 3- to 8-membered cycloalkyl.

In some embodiments of the present disclosure, in the above formula containing ring B and R^{b}, 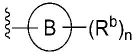 is selected from the group consisting of 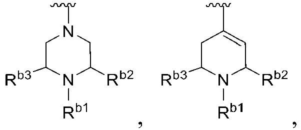 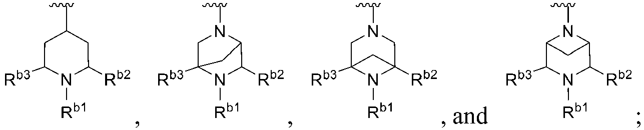 R^{b1}, R^{b2}, and R^{b3} are identical or different, and are each independently R^{b}; R^{b} is as defined in claims 1 to 9; preferably, 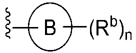 is selected from the group consisting of 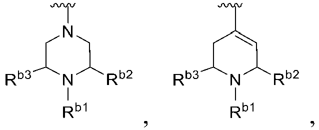 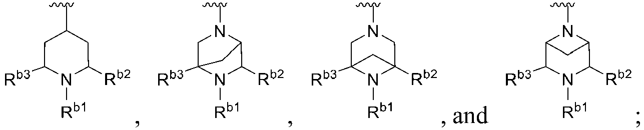 R^{b1} is selected from the group consisting of hydrogen, C₁₋₆ alkyl, C₁₋₆ hydroxyalkyl, 3- to 8-membered heterocyclyl, - S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 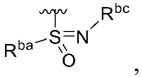 S(O)₂NR^{be}R^{bf}, and -S(O)R^{ba}; R^{b2} and R^{b3} are identical or different, and are each independently selected from the group consisting of hydrogen, C₁₋₆ alkyl, C₂₋₆ alkenyl, and C₂₋₆ alkynyl; R^{ba}, R^{bb}, R^{bc}, R^{bd}, R^{be}, and R^{bf} are as defined in claims 1 to 9; more preferably, 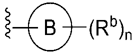 is selected from the group consisting of 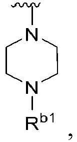 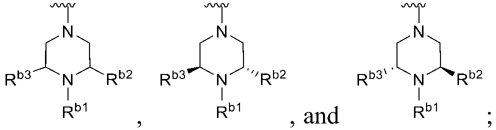 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 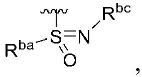 S(O)₂NR^{be}R^{bf}, and -S(O)R^{ba}; R^{b2} and R^{b3} are identical or different, and are each independently selected from the group consisting of hydrogen, C₁₋₆ alkyl, C₂₋₆ alkenyl, and C₂₋₆ alkynyl; R^{ba}, R^{bb}, R^{bc}, R^{bd}, R^{be}, and R^{bf} are as defined in claims 1 to 9; further preferably, 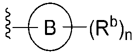 is selected from the group consisting of 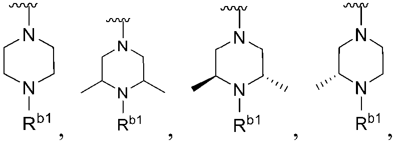 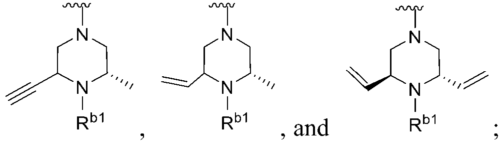 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 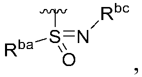 S(O)₂NR^{be}R^{bf}, and -S(O)R^{ba}; R^{ba}, R^{bb}, R^{bc}, R^{bd}, R^{be}, and R^{bf} are as defined in claims 1 to 9; most preferably, 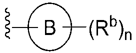 is selected from the group consisting of 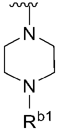 and 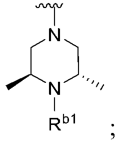 R^{b1} is selected from the group consisting of hydrogen, C₁₋₆ hydroxyalkyl, 3-to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 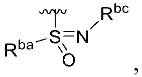 S(O)₂NR^{be}R^{bf}, and - S(O)R^{ba}; R^{ba}, R^{bb}, R^{bc}, R^{bd}, R^{be}, and R^{bf} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, 3- to 8-membered cycloalkyl, C₁₋₆ alkoxy, C₂₋₆ alkenyl, and 5- to 6-membered heteroaryl; wherein the C₁₋₆ alkyl, 3- to 8-membered heterocyclyl, 3- to 8-membered cycloalkyl, and 5- to 6-membered heteroaryl are optionally substituted by one or more substituents selected from the group consisting of C₁₋₆ alkyl, halogen, C₁₋₆ deuteroalkyl, C₁₋₆ haloalkyl, hydroxyl, C₁₋₆ alkoxy, C₁₋₆ deuteroalkoxy, -C(O)OR^{bb1}, and - NR^{a1}R^{a2}; R^{bb1}, R^{a1}, and R^{a2} are as defined in claims 1 to 9.

In some embodiments of the present disclosure, the compound of formula (IA), formula (I), or formula (II), the pharmaceutically acceptable salt thereof, the stereoisomer thereof, the prodrug thereof, the N-oxide thereof, the solvate thereof, or the isotopic derivative thereof is a compound of formula (I-a), a pharmaceutically acceptable salt thereof, a stereoisomer thereof, a prodrug thereof, an N-oxide thereof, a solvate thereof, or an isotopic derivative thereof: 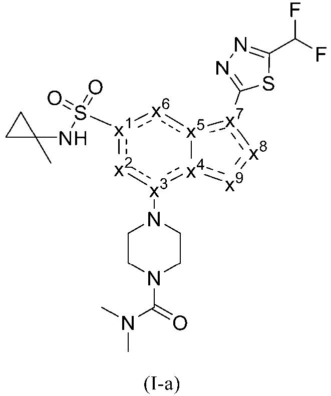 wherein 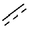 , x¹, x², x³, x⁴, x⁵, x⁶, x⁷, x⁸, and x⁹ are as defined in any embodiment of formula (IA) or any embodiment of formula (I).

In some embodiments of the present disclosure, in the above formula containing the structural moiety 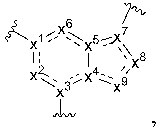 the structural moiety 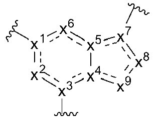 is selected from the group consisting of 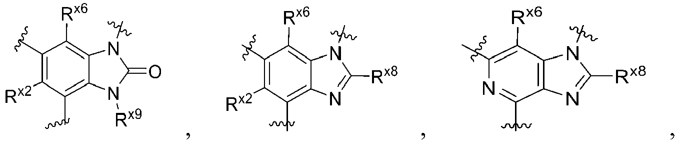 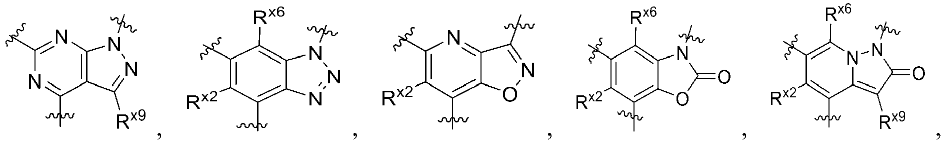 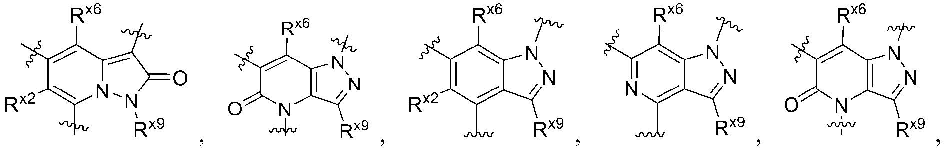 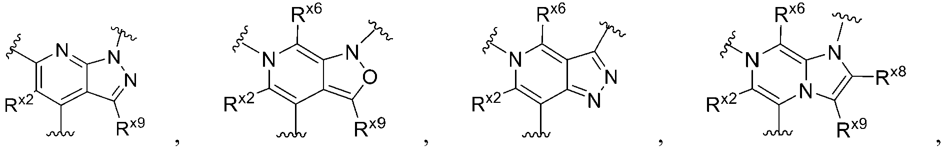 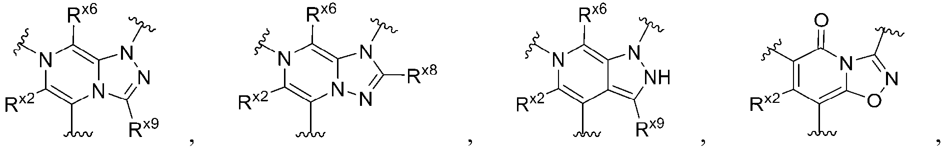 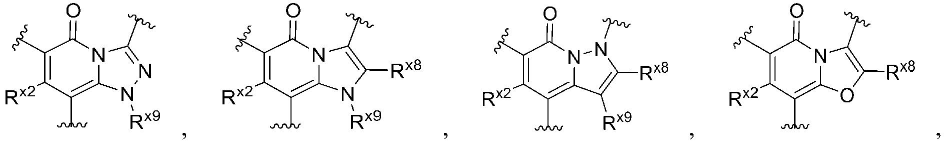 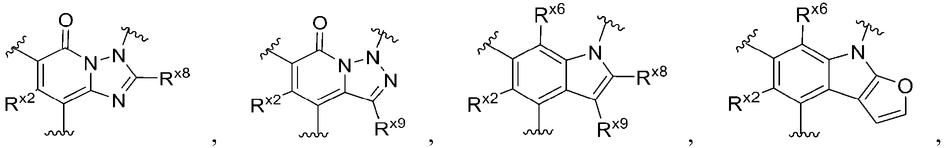 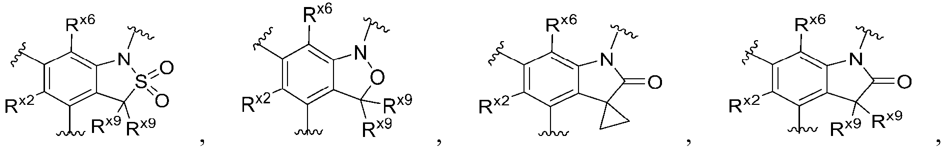 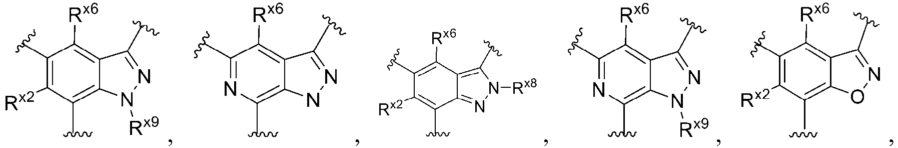 and 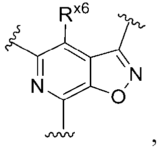 wherein R^{x2}, R^{x6}, R^{x8}, and R^{x9} are as defined in any embodiment of formula (IA) or any embodiment of formula (I); preferably, the structural moiety 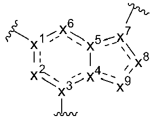 is selected from the group consisting of 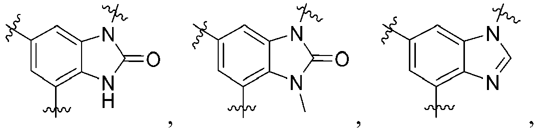 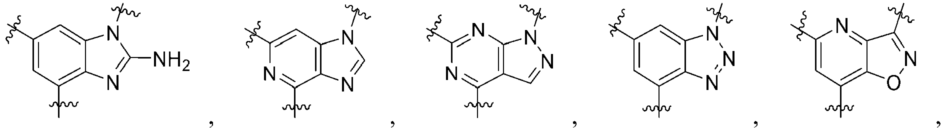 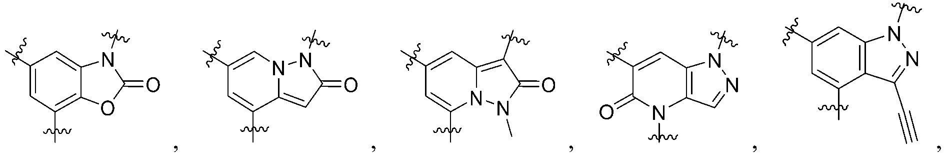 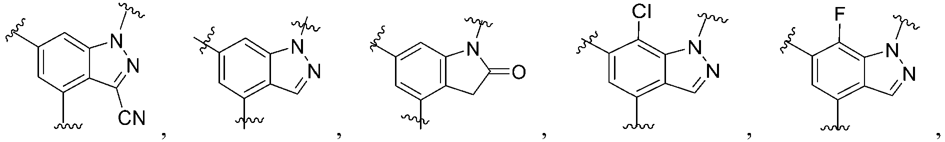 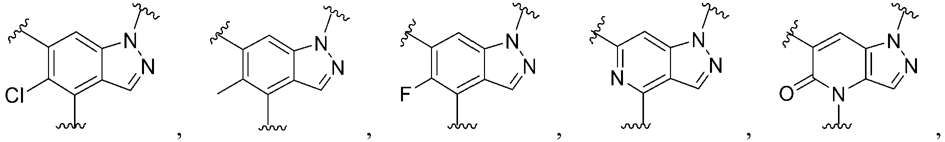 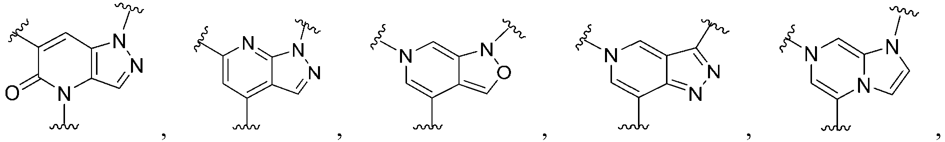 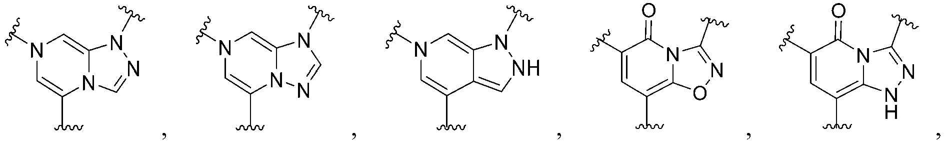 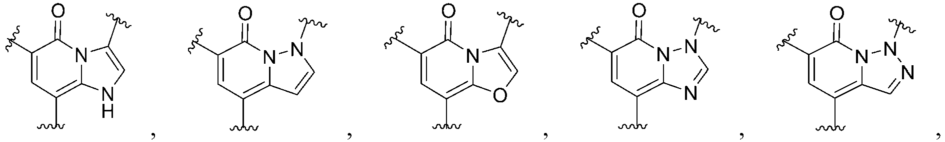 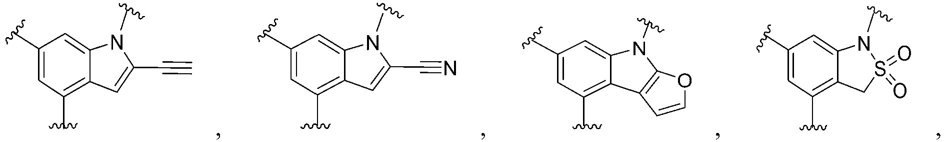 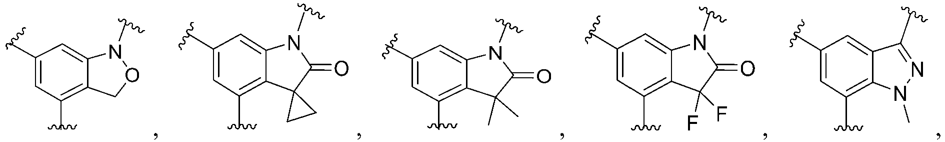 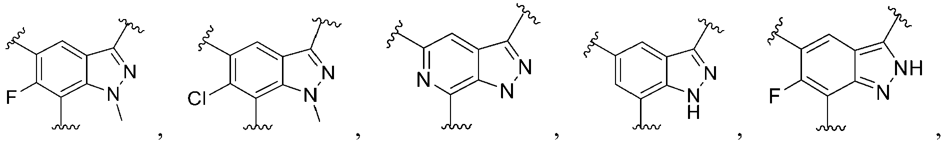 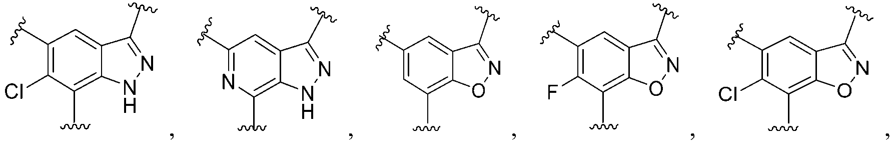 and 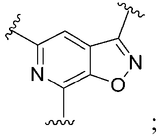 further preferably, the structural moiety 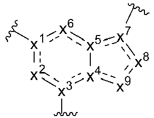 is selected from the group consisting of 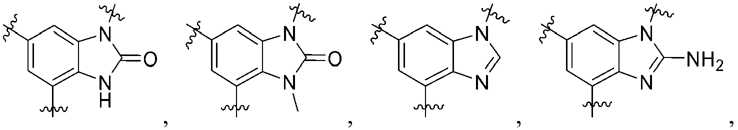 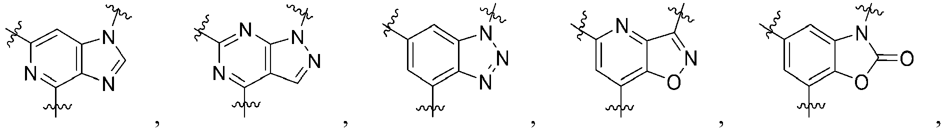 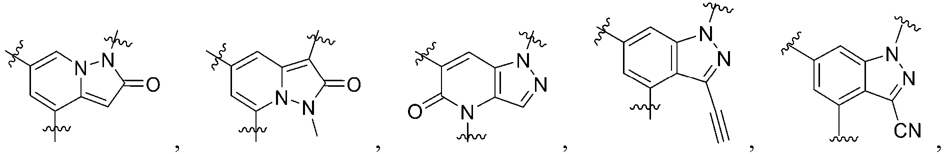 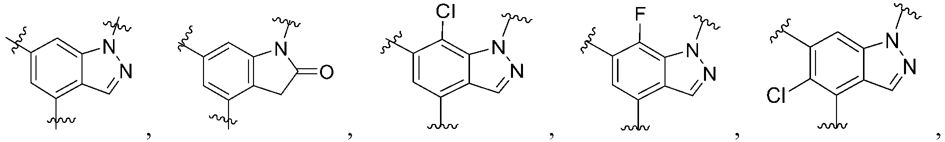 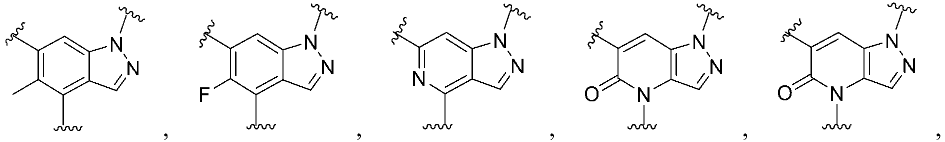 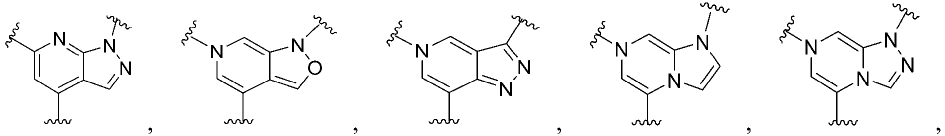 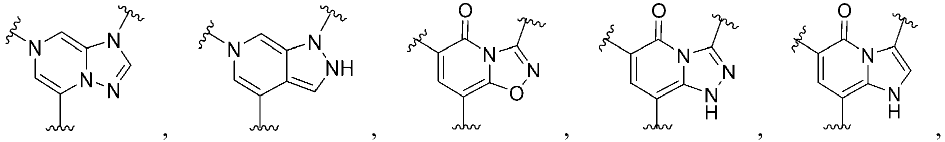 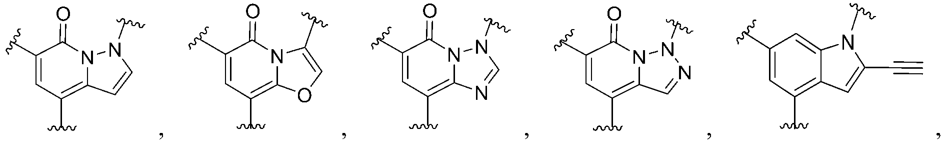 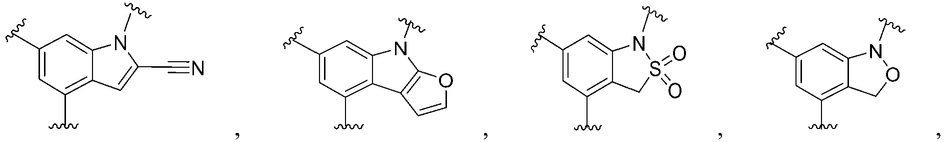 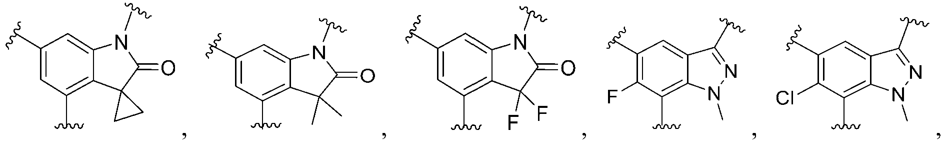 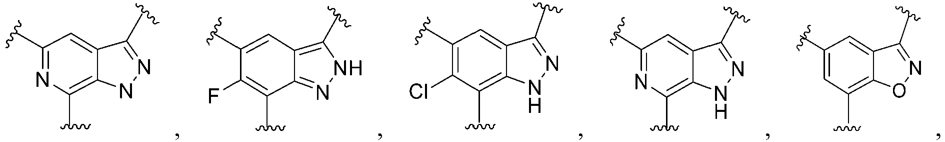 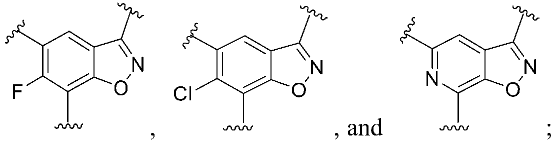 more preferably, the structural moiety 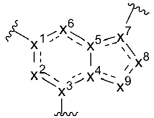 is selected from the group consisting of

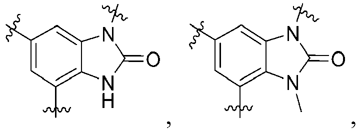

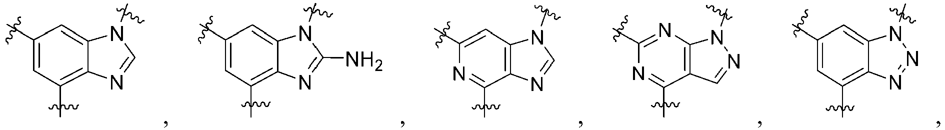

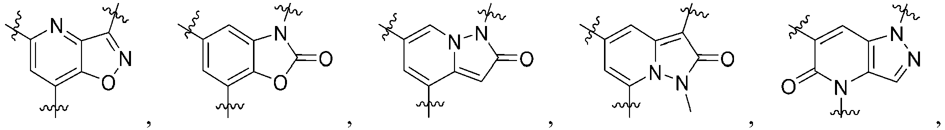

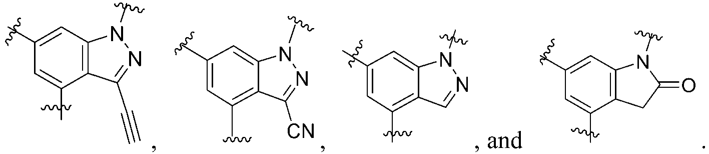

In some embodiments of the present disclosure, in the above formula containing the structural moiety 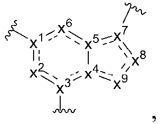 the structural moiety 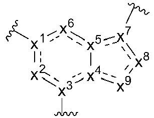 is selected from the group consisting of 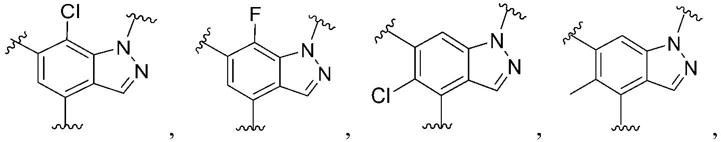 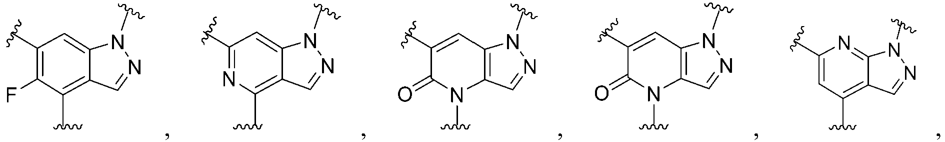 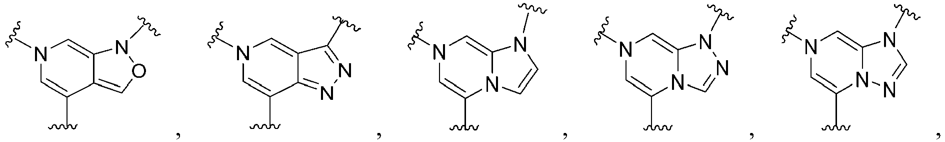 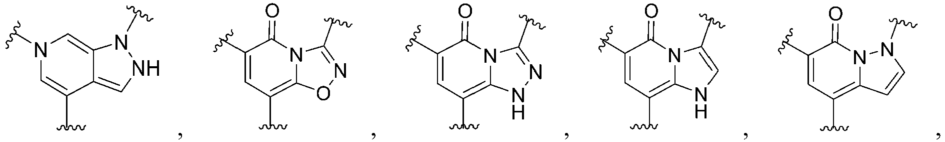 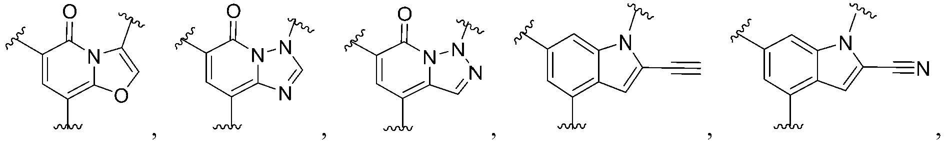 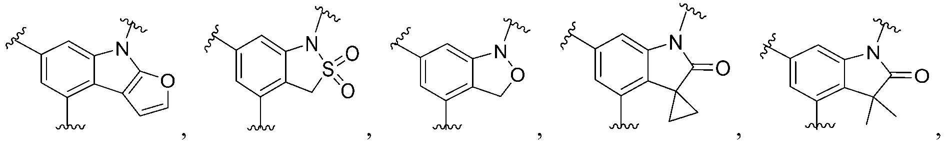 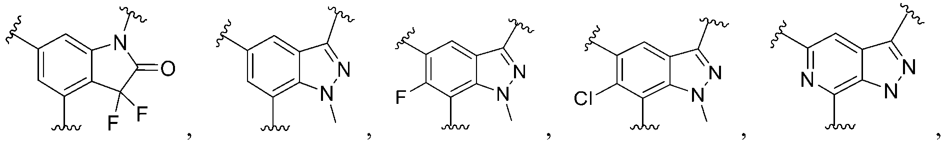 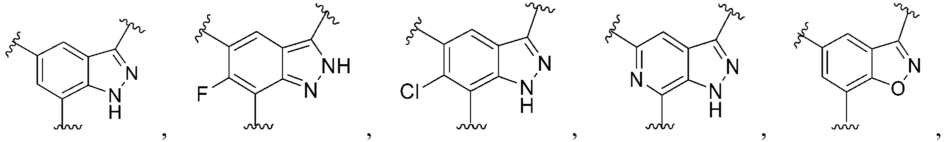 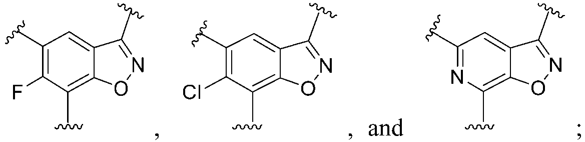 preferably, the structural moiety 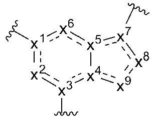 is selected from the group consisting of

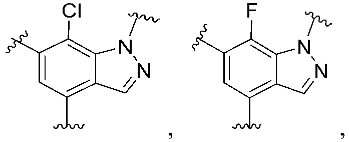

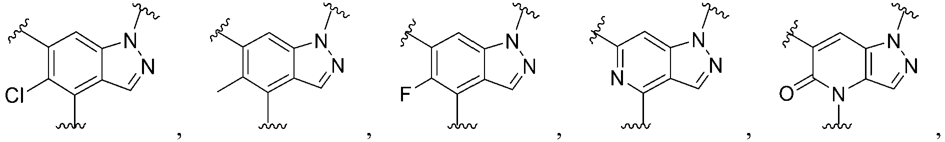

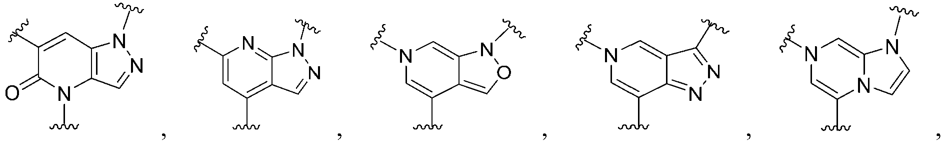

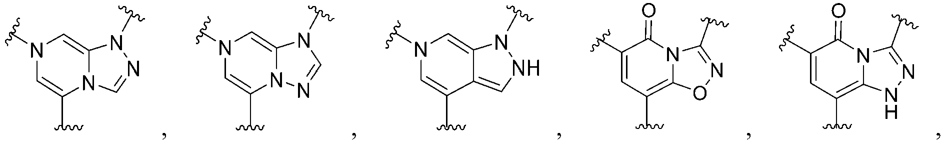

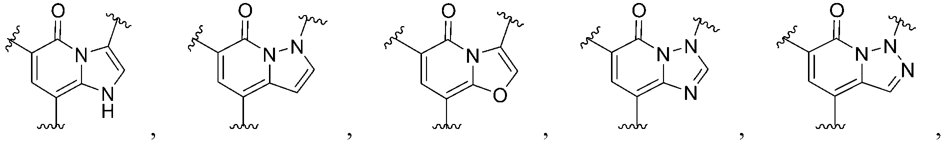

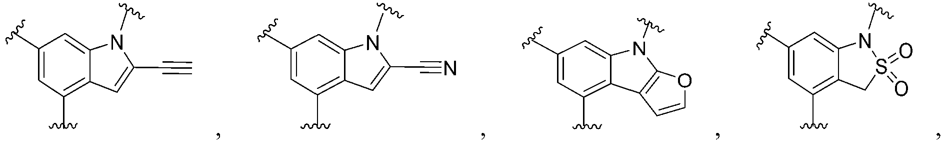

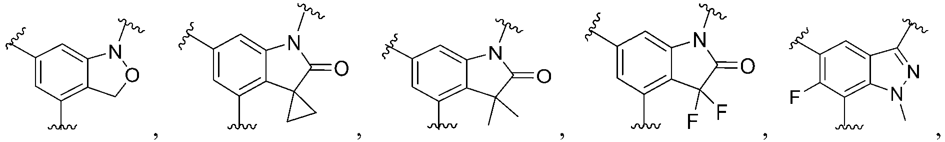

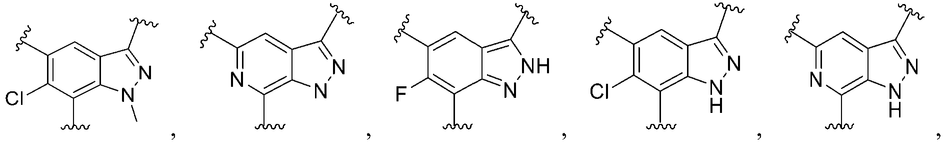

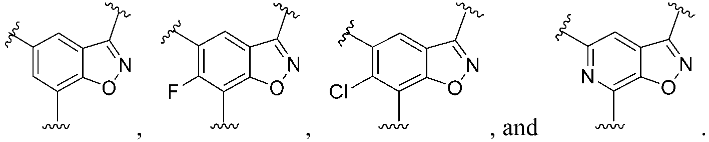

In some embodiments of the present disclosure, the structural moiety 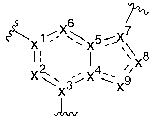 is

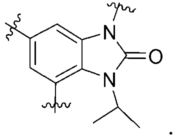

In some embodiments of the present disclosure, the structural moiety 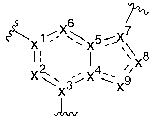 is 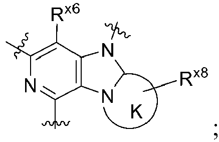 ring K is an optionally substituted 3- to 7-membered ring; R^{x6} and R^{x8} are as defined in any embodiment of formula (IA) or any embodiment of formula (I).

In some embodiments of the present disclosure, ring K is an optionally substituted 3-to 7-membered ring.

In some embodiments of the present disclosure, ring K is 5- to 6-membered heterocyclyl or 5- to 6-membered heteroaryl, wherein the 5- to 6-membered heterocyclyl or 5- to 6-membered heteroaryl is optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl; R^{a1}, R^{a2}, R^{ba}, R^{bb}, R^{bc}, R^{bd}, R^{be}, and R^{bf} are as defined in any embodiment of formula (IA) or any embodiment of formula (I).

In some embodiments of the present disclosure, ring K is

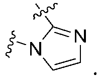

In some embodiments of the present disclosure, the structural moiety 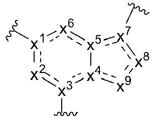 is 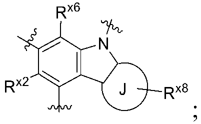 ring J is an optionally substituted 3- to 7-membered ring; R^{x2}, R^{x6}, and R^{x8} are as defined in any embodiment of formula (IA) or any embodiment of formula (I).

In some embodiments of the present disclosure, the structural moiety 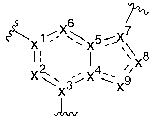 is 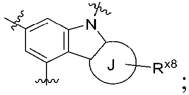 ring J is optionally substituted 5- to 6-membered heterocyclyl or 5- to 6-membered heteroaryl; R^{x8} is as defined in any embodiment of formula (IA) or any embodiment of formula (I).

In some embodiments of the present disclosure, the structural moiety 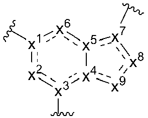 is 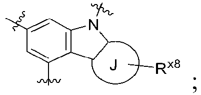 ring J is optionally substituted 6-membered nitrogen-containing heteroaryl; R^{x8} is as defined in any embodiment of formula (IA) or any embodiment of formula (I).

In some embodiments of the present disclosure, the structural moiety 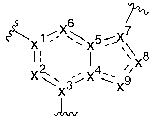 is

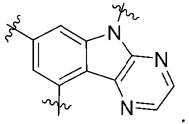

In some embodiments of the present disclosure, ring J is 5- to 6-membered heterocyclyl or 5- to 6-membered heteroaryl, wherein the 5- to 6-membered heterocyclyl or 5- to 6-membered heteroaryl is optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl; R^{a1}, R^{a2}, R^{ba}, R^{bb}, R^{bc}, R^{bd}, R^{be}, and R^{bf} are as defined in any embodiment of formula (IA) or any embodiment of formula (I).

In some embodiments of the present disclosure, ring J is

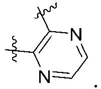

In some embodiments of the present disclosure, the structural moiety 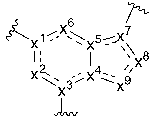 is selected from the group consisting of

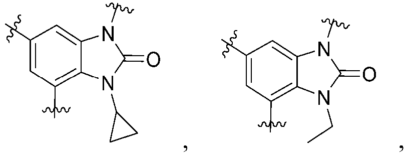

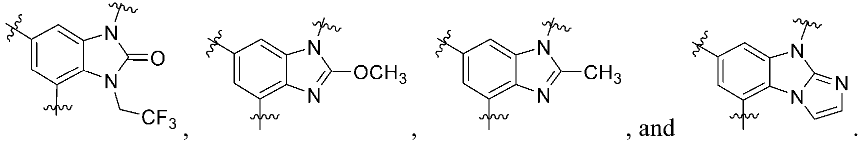

In some embodiments of the present disclosure, in the above formula containing the structural moiety 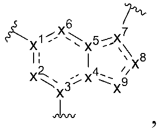 the structural moiety 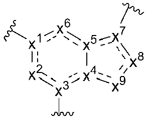 is selected from the group consisting of 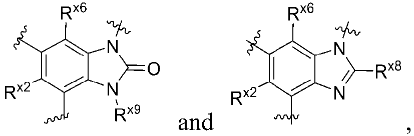 wherein R^{x2}, R^{x6}, R^{x8}, and R^{x9} are as defined in any embodiment of formula (IA) or any embodiment of formula (I); preferably, the structural moiety 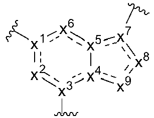 is selected from the group consisting of

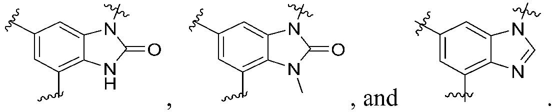

In some embodiments of the present disclosure, in the above formula containing the structural moiety 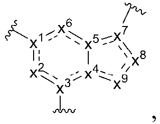 the structural moiety 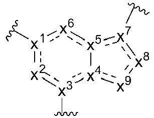 is selected from the group consisting of

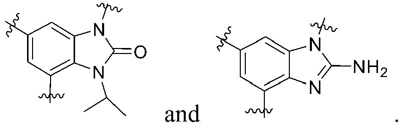

In some embodiments of the present disclosure, in the above formula containing the structural moiety 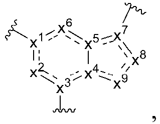 the structural moiety 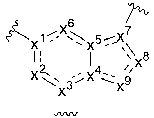 is selected from the group consisting of

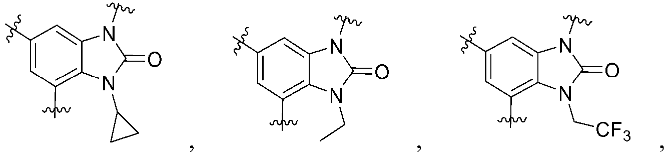

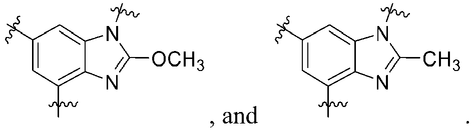

In some embodiments of the present disclosure, the structural moiety 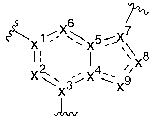 is

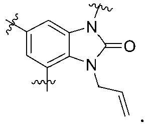

In some embodiments of the present disclosure, the structural moiety 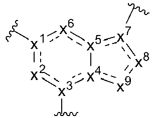 is selected from the group consisting of 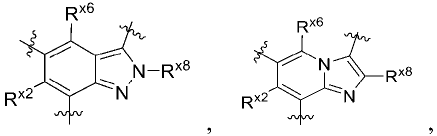 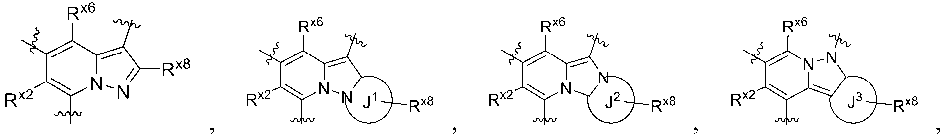 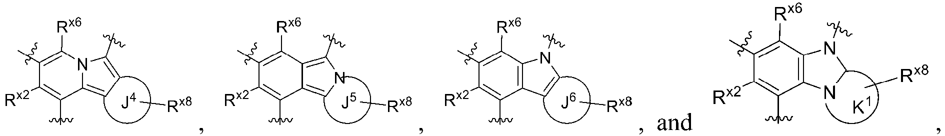 wherein ring J¹, ring J², ring J³, ring J⁴, ring J⁵, ring J⁶, and ring K¹ are identical or different, and are each independently an optionally substituted 3- to 7-membered ring; R^{x2}, R^{x6}, and R^{x8} are as defined in any one of the embodiments of the present disclosure.

In some embodiments of the present disclosure, the structural moiety 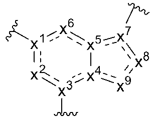 is 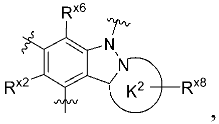 wherein ring K² is an optionally substituted 3- to 7-membered ring; R^{x2}, R^{x6}, and R^{x8} are as defined in any one of the embodiments of the present disclosure.

In some embodiments of the present disclosure, ring J¹, ring J², ring J³, ring J⁴, ring J⁵, ring J⁶, and ring K¹ are identical or different, and are each independently 5- to 6-membered heterocyclyl or 5- to 6-membered heteroaryl, wherein the 5- to 6-membered heterocyclyl or 5-to 6-membered heteroaryl is optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, -C(O)R^{ba}, S(O)ₚR^{ba}, S(O)ₚNR^{be}R^{bf}, -C(O)OR^{bb}, -C(O)NR^{bc}R^{bd}, and C₁₋₆ hydroxyalkyl; R^{a1}, R^{a2}, R^{ba}, R^{bb}, R^{bc}, R^{bd}, R^{be}, and R^{bf} are as defined in any embodiment of formula (IA) or any embodiment of formula (I).

In some embodiments of the present disclosure, the structural moiety 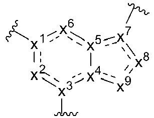 is selected from the group consisting of

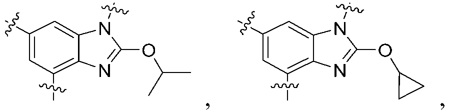

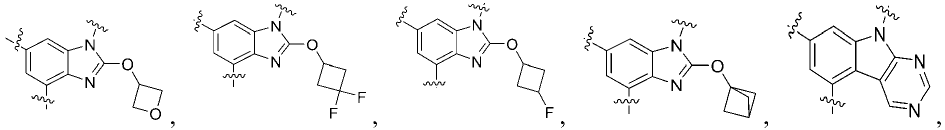

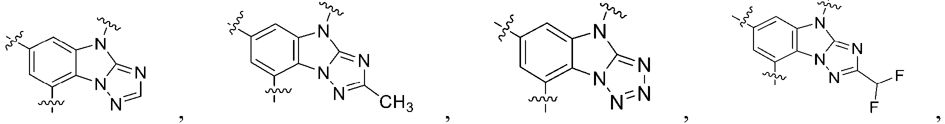

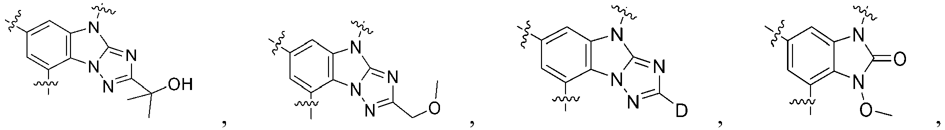

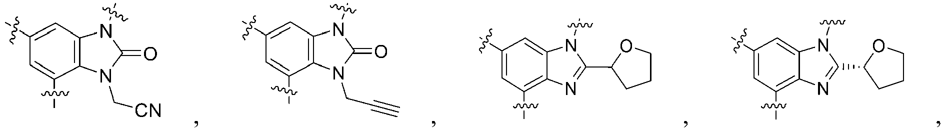

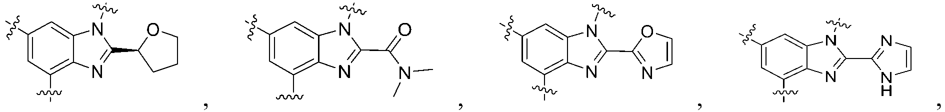

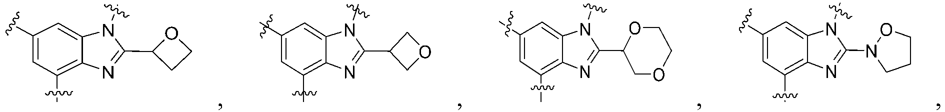

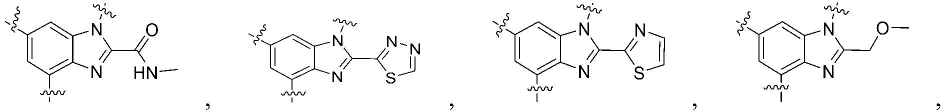

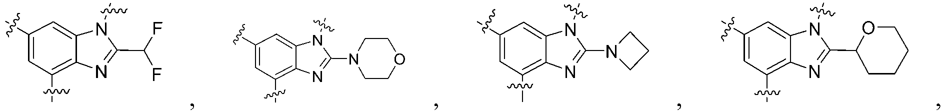

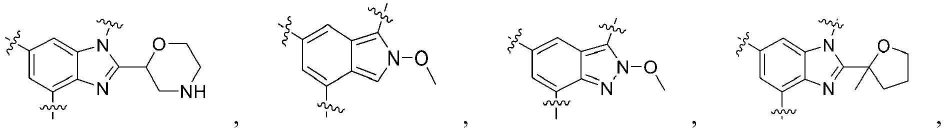

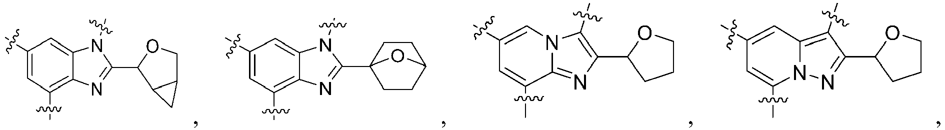

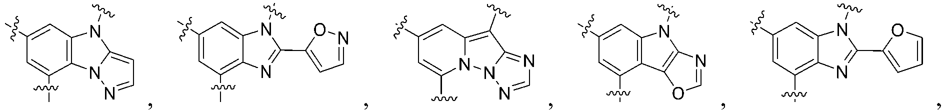

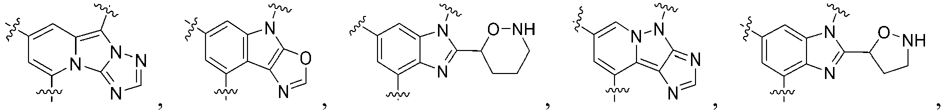

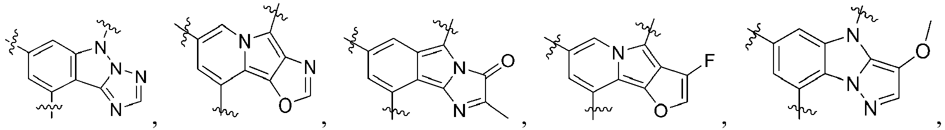

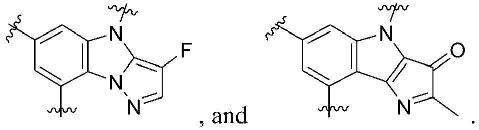

In some embodiments of the present disclosure, the structural moiety 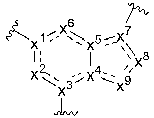 is selected from the group consisting of 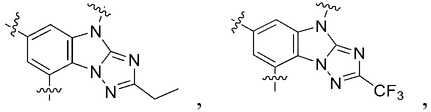 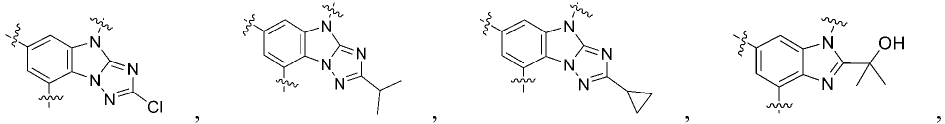 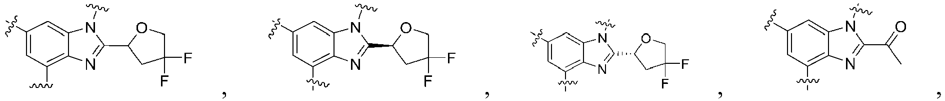 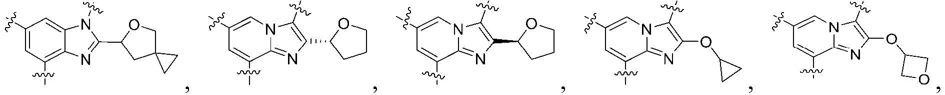 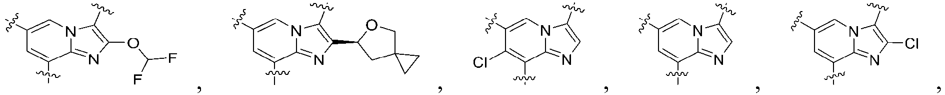 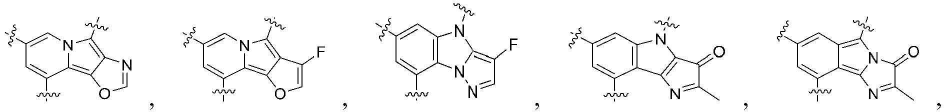 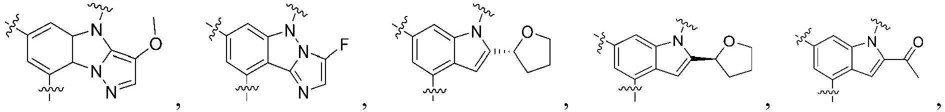 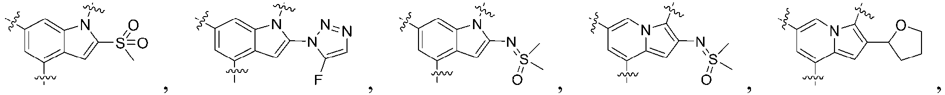 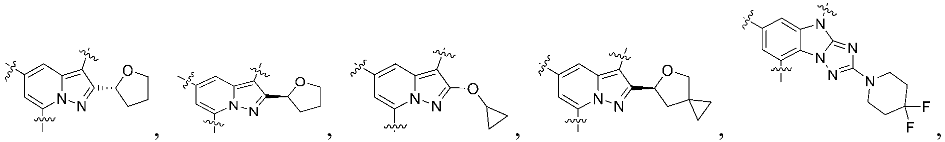 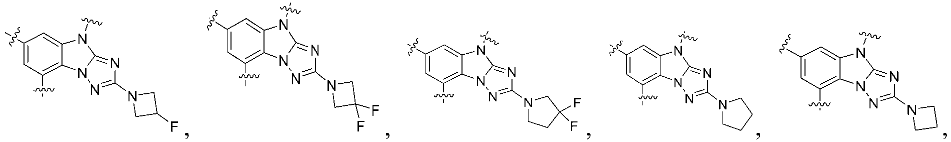 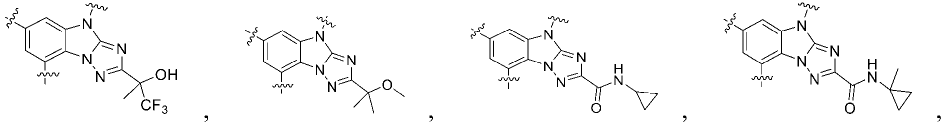 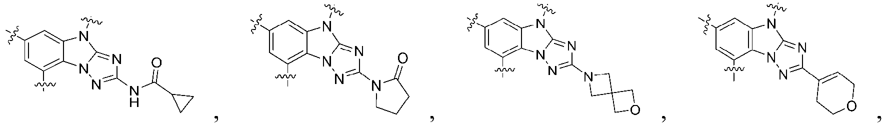 and

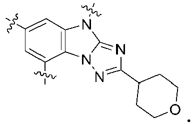

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II-1A) or formula (II-2A), a pharmaceutically acceptable salt thereof, a stereoisomer thereof, a prodrug thereof, an N-oxide thereof, a solvate thereof, or an isotopic derivative thereof: 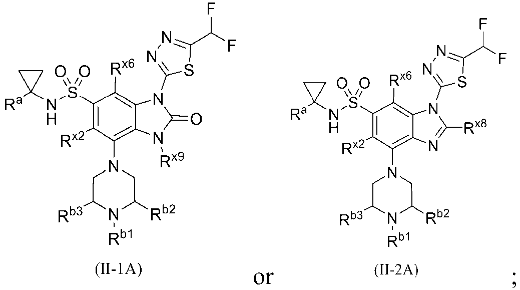 wherein
R^{b1}, R^{b2}, and R^{b3} are identical or different, and are each independently R^{b};
R^{b}, R^{a}, R^{x2}, R^{x6}, R^{x8}, and R^{x9} are as defined in any one of the embodiments of the present disclosure.

In some embodiments of the present disclosure, in the compound of formula (II-1A) or formula (II-2A), the pharmaceutically acceptable salt thereof, the stereoisomer thereof, the prodrug thereof, the N-oxide thereof, the solvate thereof, or the isotopic derivative thereof, R^{b1}, R^{b2}, and R^{b3} are identical or different, and are each independently R^{b}, wherein R^{b} is as defined in any embodiment of formula (IA) or any embodiment of formula (I); preferably, R^{b1}, R^{b2}, and R^{b3} are identical or different, and are each independently selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}, wherein R^{ba}, R^{bb}, R^{bc}, and R^{bd} are as defined in any embodiment of formula (IA) or any embodiment of formula (I); further preferably, R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; R^{b2} and R^{b3} are identical or different, and are each independently C₁₋₆ alkyl, wherein R^{ba}, R^{bb}, R^{bc}, and R^{bd} are as defined in any embodiment of formula (IA) or any embodiment of formula (I); further preferably, R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; R^{b2} and R^{b3} are identical, and are both C₁₋₆ alkyl, wherein R^{ba}, R^{bb}, R^{bc}, and R^{bd} are as defined in any embodiment of formula (IA) or any embodiment of formula (I); further preferably, R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; R^{b2} and R^{b3} are both methyl, wherein R^{ba}, R^{bb}, R^{bc}, and R^{bd} are as defined in any embodiment of formula (IA) or any embodiment of formula (I); most preferably, R^{b1} is selected from the group consisting of hydrogen, C₁₋₆ hydroxyalkyl, 3- to 8-membered heterocyclyl, -S(O)₂R^{ba}, -C(O)R^{ba}, - C(O)OR^{bb}, and -C(O)NR^{bc}R^{bd}; R^{b2} and R^{b3} are both methyl, wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl;

R^{a}, R^{x2}, R^{x6}, R^{x8}, and R^{x9} are as defined in any embodiment of formula (IA) or any embodiment of formula (I).

In some embodiments of the present disclosure, in the compound of formula (II-1A) or formula (II-2A), the pharmaceutically acceptable salt thereof, the stereoisomer thereof, the prodrug thereof, the N-oxide thereof, the solvate thereof, or the isotopic derivative thereof, R^{b1}, R^{b2}, and R^{b3} are identical or different, and are each independently R^{b}, wherein R^{b} is as defined in any embodiment of formula (IA) or any embodiment of formula (I); preferably, R^{b1}, R^{b2}, and R^{b3} are identical or different, and are each independently selected from the group consisting of hydrogen, C₁₋₆ alkyl, -C(O)R^{ba}, -C(O)OR^{bb}, and -C(O)NR^{bc}R^{bd}, wherein R^{ba}, R^{bb}, R^{bc}, and R^{bd} are as defined in any embodiment of formula (IA) or any embodiment of formula (I); further preferably, R^{b1} is selected from the group consisting of hydrogen, C₁₋₆ alkyl, -C(O)R^{ba}, - C(O)OR^{bb}, and -C(O)NR^{bc}R^{bd}; R^{b2} and R^{b3} are identical or different, and are each independently C₁₋₆ alkyl, wherein R^{ba}, R^{bb}, R^{bc}, and R^{bd} are as defined in any embodiment of formula (IA) or any embodiment of formula (I); further preferably, R^{b1} is selected from the group consisting of hydrogen, C₁₋₆ alkyl, -C(O)R^{ba}, -C(O)OR^{bb}, and -C(O)NR^{bc}R^{bd}; R^{b2} and R^{b3} are identical, and are both C₁₋₆ alkyl, wherein R^{ba}, R^{bb}, R^{bc}, and R^{bd} are as defined in any embodiment of formula (IA) or any embodiment of formula (I); most preferably, R^{b1} is selected from the group consisting of hydrogen, C₁₋₆ alkyl, -C(O)R^{ba}, -C(O)OR^{bb}, and -C(O)NR^{bc}R^{bd}; R^{b2} and R^{b3} are both methyl, wherein R^{ba}, R^{bb}, R^{bc}, and R^{bd} are as defined in any embodiment of formula (IA) or any embodiment of formula (I); R^{a}, R^{x2}, R^{x6}, R^{x8}, and R^{x9} are as defined in any embodiment of formula (IA) or any embodiment of formula (I).

In some embodiments of the present disclosure, in the compound of formula (II-1A) or formula (II-2A), the pharmaceutically acceptable salt thereof, the stereoisomer thereof, the prodrug thereof, the N-oxide thereof, the solvate thereof, or the isotopic derivative thereof, R^{b1}, R^{b2}, and R^{b3} are identical or different, and are each independently selected from the group consisting of hydrogen, C₁₋₆ alkyl, C₂₋₆ alkenyl, C₂₋₆ alkynyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 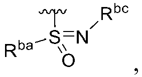 S(O)₂NR^{be}R^{bf}, and -S(O)R^{ba}; wherein R^{ba}, R^{bb}, R^{bc}, R^{bd}, R^{be}, and R^{bf} are as defined in any embodiment of formula (IA) or any embodiment of formula (I).

In some embodiments of the present disclosure, in the compound of formula (II-1A), the pharmaceutically acceptable salt thereof, the stereoisomer thereof, the prodrug thereof, the N-oxide thereof, the solvate thereof, or the isotopic derivative thereof, R^{b2} and R^{b3} are identical or different, and are each independently selected from the group consisting of hydrogen and C₁₋₆ alkyl; preferably, R^{b2} and R^{b3} are both hydrogen.

In some embodiments of the present disclosure, in the compound of formula (II-2A), the pharmaceutically acceptable salt thereof, the stereoisomer thereof, the prodrug thereof, the N-oxide thereof, the solvate thereof, or the isotopic derivative thereof, R^{b2} and R^{b3} are identical or different, and are each independently selected from the group consisting of hydrogen and C₁₋₆ alkyl; preferably, R^{b2} and R^{b3} are identical or different, and are each independently selected from hydrogen and methyl.

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II-1B) or formula (II-2B), a pharmaceutically acceptable salt thereof, a stereoisomer thereof, a prodrug thereof, an N-oxide thereof, a solvate thereof, or an isotopic derivative thereof: 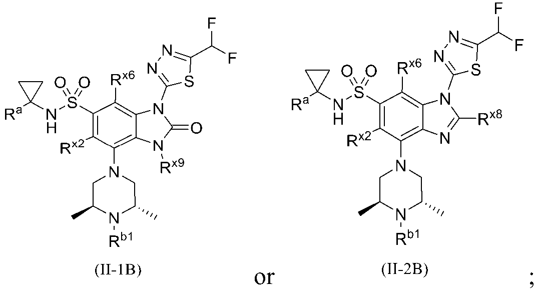 wherein
R^{b1} is R^{b}; R^{b}, R^{a}, R^{x2}, R^{x6}, R^{x8}, and R^{x9} are as defined in any one of the embodiments of the present disclosure.

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II-1C) or formula (II-2C), a pharmaceutically acceptable salt thereof, a stereoisomer thereof, a prodrug thereof, an N-oxide thereof, a solvate thereof, or an isotopic derivative thereof: 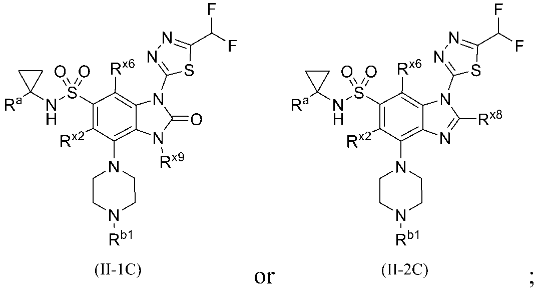 wherein
R^{b1} is R^{b}; R^{b}, R^{a}, R^{x2}, R^{x6}, R^{x8}, and R^{x9} are as defined in any one of the embodiments of the present disclosure.

In some embodiments of the present disclosure, in the compound of formula (II-1B), formula (II-1C), formula (II-2B), or formula (II-2C), the pharmaceutically acceptable salt thereof, the stereoisomer thereof, the prodrug thereof, the N-oxide thereof, the solvate thereof, or the isotopic derivative thereof, R^{b1} is R^{b}, wherein R^{b} is as defined in any embodiment of formula (IA) or any embodiment of formula (I); preferably, R^{b1} is selected from the group consisting of hydrogen, C₁₋₆ alkyl, C₁₋₆ hydroxyalkyl, 3- to 8-membered heterocyclyl, - S(O)₂R^{ba}, -C(O)R^{ba}, -C(O)OR^{bb}, and -C(O)NR^{bc}R^{bd}, wherein R^{ba}, R^{bb}, R^{bc}, and R^{bd} are as defined in any embodiment of formula (IA) or any embodiment of formula (I); more preferably, R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}, wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl;

R^{a}, R^{x2}, R^{x6}, R^{x8}, and R^{x9} are as defined in any embodiment of formula (IA) or any embodiment of formula (I).

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II-2D), formula (II-2E), or formula (II-2F), a pharmaceutically acceptable salt thereof, a stereoisomer thereof, a prodrug thereof, an N-oxide thereof, a solvate thereof, or an isotopic derivative thereof: 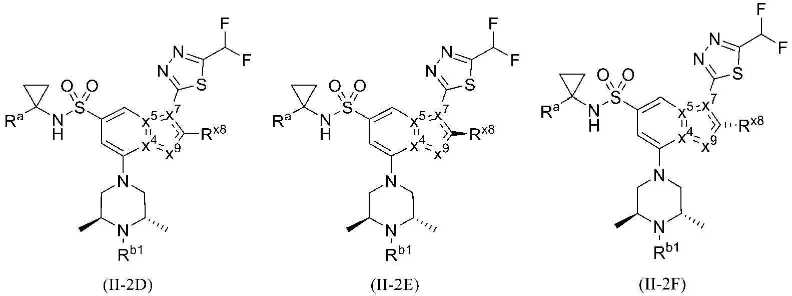 wherein
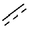 is a single bond or a double bond;
x⁴, x⁵, x⁷, and x⁹ are identical or different, and are each independently selected from the group consisting of N and C; and the ring containing x⁴, x⁵, x⁷, and x⁹ is an aromatic ring;
R^{x8} is optionally substituted 4- to 7-membered heterocyclyl, preferably optionally substituted 5- to 7-membered heterocyclyl, more preferably optionally substituted 5- to 7-membered oxygen-containing heterocyclyl; wherein the optional substituents are selected from the group consisting of halogen, C₁₋₆ alkyl, hydroxyl, -CN, amino, C₁₋₆ haloalkyl, and C₁₋₆ hydroxyalkyl;
R^{b1} is R^{b}; R^{b} and R^{a} are as defined in any one of the embodiments of the present disclosure.

In some embodiments of the present disclosure, the compound of formula (IA), formula (I), formula (II-2D), formula (II-2E), or formula (II-2F), the pharmaceutically acceptable salt thereof, the stereoisomer thereof, the prodrug thereof, the N-oxide thereof, the solvate thereof, or the isotopic derivative thereof is a compound of formula (II-2G), formula (II-2H), or formula (II-2J), a pharmaceutically acceptable salt thereof, a stereoisomer thereof, a prodrug thereof, an N-oxide thereof, a solvate thereof, or an isotopic derivative thereof: 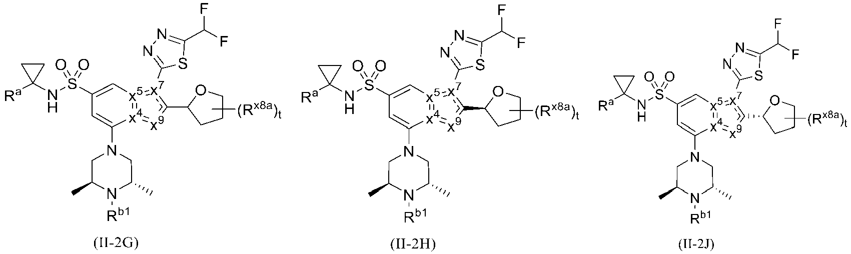 wherein
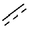 is a single bond or a double bond;
x⁴, x⁵, x⁷, and x⁹ are identical or different, and are each independently selected from the group consisting of N and C; and the ring containing x⁴, x⁵, x⁷, and x⁹ is an aromatic ring;
R^{x8a} is selected from the group consisting of halogen, C₁₋₆ alkyl, hydroxyl, -CN, amino, C₁₋₆ haloalkyl, and C₁₋₆ hydroxyalkyl; alternatively, two R^{x8a} attached to the same carbon atom, together with the carbon atom to which they are attached, form a 3- to 6-membered cycloalkyl ring; alternatively, two adjacent R^{x8a}, together with the carbon atom to which they are attached, form a 3- to 6-membered cycloalkyl ring;
t is selected from the group consisting of 0, 1, 2, 3, and 4;
R^{b1} is R^{b}; R^{b} and R^{a} are as defined in any one of the embodiments of the present disclosure.

In some embodiments of the present disclosure, the compound of formula (IA), formula (I), formula (II-2D), formula (II-2E), formula (II-2F), formula (II-2G), formula (II-2H), or formula (II-2J), the pharmaceutically acceptable salt thereof, the stereoisomer thereof, the prodrug thereof, the N-oxide thereof, the solvate thereof, or the isotopic derivative thereof is a compound of formula (II-2K), formula (II-2L), or formula (II-2M), a pharmaceutically acceptable salt thereof, a stereoisomer thereof, a prodrug thereof, an N-oxide thereof, a solvate thereof, or an isotopic derivative thereof: 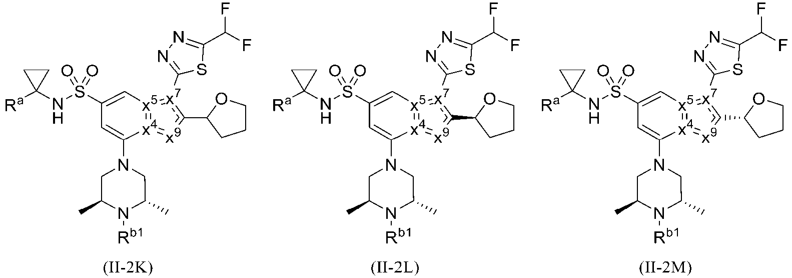 wherein
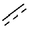 is a single bond or a double bond;
x⁴, x⁵, x⁷, and x⁹ are identical or different, and are each independently selected from the group consisting of N and C; and the ring containing x⁴, x⁵, x⁷, and x⁹ is an aromatic ring;
R^{b1} is R^{b}; R^{b} and R^{a} are as defined in any one of the embodiments of the present disclosure.

In some embodiments of the present disclosure, in the compound of formula (II-2D), formula (II-2G), or formula (II-2K), the pharmaceutically acceptable salt thereof, the stereoisomer thereof, the prodrug thereof, the N-oxide thereof, the solvate thereof, or the isotopic derivative thereof, 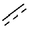 is a single bond or a double bond; x⁴, x⁵, and x⁷ are identical or different, and are each independently selected from the group consisting of N and C; x⁹ is selected from the group consisting of N and CH; and the ring containing x⁴, x⁵, x⁷, and x⁹ is an aromatic ring;
R^{b1} is R^{b}; R^{b} and R^{a} are as defined in any one of the embodiments of the present disclosure.

In some embodiments of the present disclosure, in the compound of formula (II-2D), formula (II-2G), or formula (II-2K), the pharmaceutically acceptable salt thereof, the stereoisomer thereof, the prodrug thereof, the N-oxide thereof, the solvate thereof, or the isotopic derivative thereof, x⁴, x⁵, and x⁷ are identical or different, and are each independently selected from the group consisting of N and C;
x⁹ is selected from the group consisting of N and CH;
preferably, the number of N among x⁴, x⁵, x⁷, and x⁹ is 1 or 2.

In some embodiments of the present disclosure, in the compound of formula (II-2D), formula (II-2G), or formula (II-2K), the pharmaceutically acceptable salt thereof, the stereoisomer thereof, the prodrug thereof, the N-oxide thereof, the solvate thereof, or the isotopic derivative thereof, 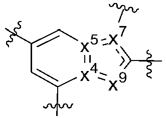 is selected from the group consisting of

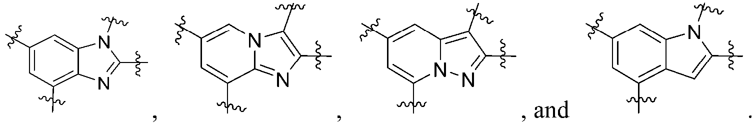

In some embodiments of the present disclosure, in the compound of formula (II-2E), formula (II-2H), or formula (II-2L), the pharmaceutically acceptable salt thereof, the stereoisomer thereof, the prodrug thereof, the N-oxide thereof, the solvate thereof, or the isotopic derivative thereof, 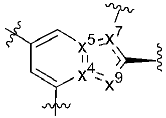 is selected from the group consisting of

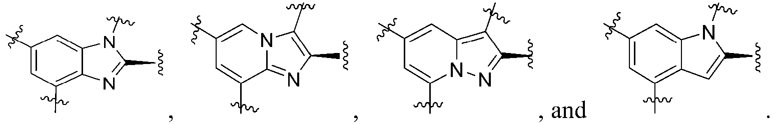

In some embodiments of the present disclosure, in the compound of formula (II-2F), formula (II-2J), or formula (II-2M), the pharmaceutically acceptable salt thereof, the stereoisomer thereof, the prodrug thereof, the N-oxide thereof, the solvate thereof, or the isotopic derivative thereof, 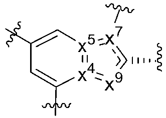 is selected from the group consisting of

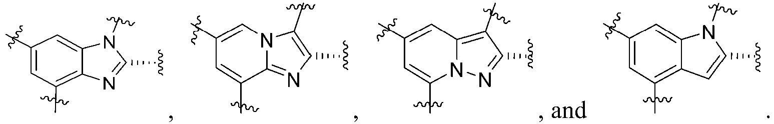

In some embodiments of the present disclosure, in the compound of formula (II-1B), formula (II-1C), formula (II-2B), or formula (II-2C), the pharmaceutically acceptable salt thereof, the stereoisomer thereof, the prodrug thereof, the N-oxide thereof, the solvate thereof, or the isotopic derivative thereof, R^{b1} is R^{b}, wherein R^{b} is as defined in any embodiment of formula (IA) or any embodiment of formula (I); preferably, R^{b1} is selected from the group consisting of hydrogen, C₁₋₆ alkyl, -C(O)R^{ba}, -C(O)OR^{bb}, and -C(O)NR^{bc}R^{bd}, wherein R^{ba}, R^{bb}, R^{bc}, and R^{bd} are as defined in any embodiment of formula (IA) or any embodiment of formula (I); R^{a}, R^{x2}, R^{x6}, R^{x8}, and R^{x9} are as defined in any embodiment of formula (IA) or any embodiment of formula (I).

In some embodiments of the present disclosure, the compound of formula (IA) or formula (I), the pharmaceutically acceptable salt thereof, the stereoisomer thereof, the prodrug thereof, the N-oxide thereof, the solvate thereof, or the isotopic derivative thereof is a compound of formula (II-3A), a pharmaceutically acceptable salt thereof, a stereoisomer thereof, a prodrug thereof, an N-oxide thereof, a solvate thereof, or an isotopic derivative thereof: 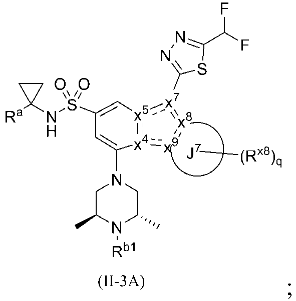 wherein
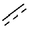 is a single bond or a double bond;
x⁴, x⁵, x⁷, x⁸, and x⁹ are identical or different, and are each independently selected from the group consisting of N and C; and the ring containing x⁴, x⁵, x⁷, x⁸, and x⁹ is an aromatic ring;
ring J⁷ is selected from the group consisting of 5- to 10-membered heteroaryl and 3- to 8-membered heterocyclyl;
q is selected from the group consisting of 0, 1, and 2;
R^{b1} is R^{b}; R^{b}, R^{a}, and R^{x8} are as defined in any one of the embodiments of the present disclosure.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, the number of N among x⁴, x⁵, x⁷, x⁸, and x⁹ is 1 or 2.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, ring J⁷ is selected from the group consisting of 5- to 6-membered heteroaryl and 5- to 6-membered heterocyclyl; more preferably, ring J⁷ is selected from the group consisting of 5- to 6-membered nitrogen-containing heteroaryl and 5- to 6-membered nitrogen-containing heterocyclyl; further preferably, ring J⁷ is 5- to 6-membered nitrogen-containing heteroaryl; most preferably, ring J⁷ is 5-membered nitrogen-containing heteroaryl.

In some embodiments of the present disclosure, ring J, ring J¹, ring J², ring J³, ring J⁴, ring J⁵, ring J⁶, ring J⁷, ring K¹, ring K², and ring K are identical or different, and are each independently a 3- to 7-membered ring, preferably 5- to 10-membered heteroaryl or 3- to 8-membered heterocyclyl, more preferably 5- to 10-membered heteroaryl, further preferably 5-to 6-membered nitrogen-containing heteroaryl, and most preferably 5-membered nitrogen-containing heteroaryl.

In some embodiments of the present disclosure, in the compound of formula (II-1A), formula (II-1B), or formula (II-1C), the pharmaceutically acceptable salt thereof, the stereoisomer thereof, the prodrug thereof, the N-oxide thereof, the solvate thereof, or the isotopic derivative thereof, R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 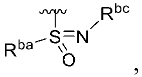 S(O)₂NR^{be}R^{bf}, and -S(O)R^{ba}; preferably, R^{b1} is selected from the group consisting of -S(O)₂R^{ba}, -C(O)R^{ba}, and -C(O)NR^{bc}R^{bd}; wherein R^{ba}, R^{bb}, R^{bc}, R^{bd}, R^{be}, and R^{bf} are as defined in any embodiment of formula (IA) or any embodiment of formula (I).

In some embodiments of the present disclosure, in the compound of formula (II-2A), formula (II-2B), formula (II-2C), formula (II-2D), formula (II-2E), formula (II-2F), formula (II-2G), formula (II-2H), formula (II-2J), or formula (II-3A), the pharmaceutically acceptable salt thereof, the stereoisomer thereof, the prodrug thereof, the N-oxide thereof, the solvate thereof, or the isotopic derivative thereof, R^{b1} is hydrogen.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 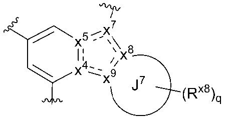 is selected from the group consisting of 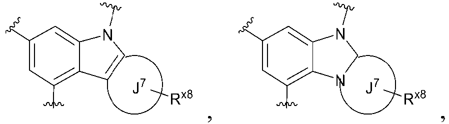 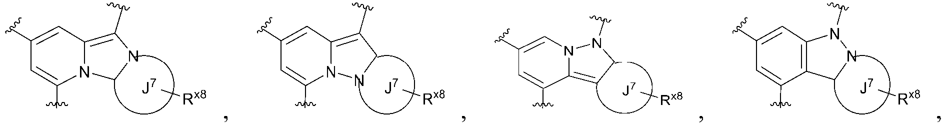 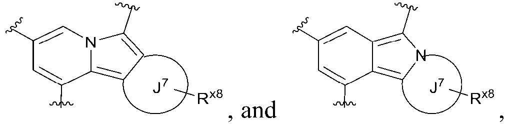 wherein R^{x8} is as defined in any one of the embodiments of the present disclosure.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 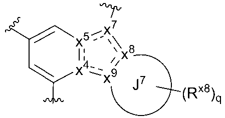 is 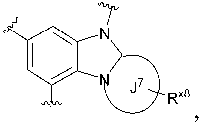 wherein R^{x8} is as defined in any one of the embodiments of the present disclosure; preferably, 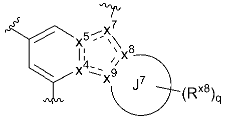 is 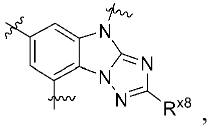 wherein R^{x8} is as defined in any one of the embodiments of the present disclosure; more preferably, 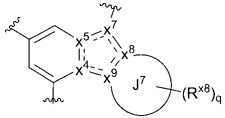 is 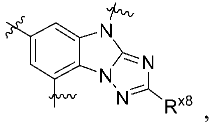 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 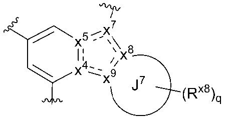 is

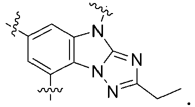

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 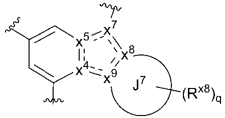 is

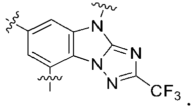

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 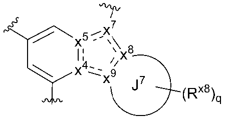 is

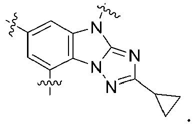

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 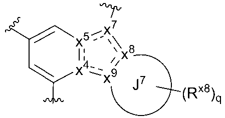 is

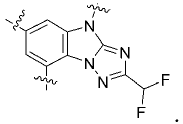

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 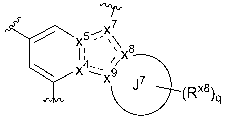 is

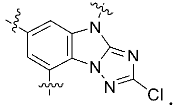

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 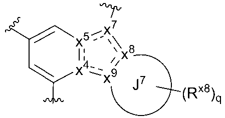 is

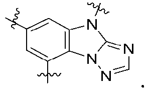

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 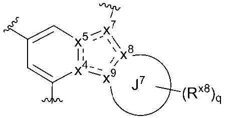 is 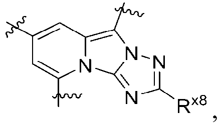 wherein R^{x8} is as defined in any one of the embodiments of the present disclosure; preferably, 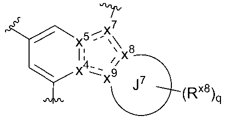 is 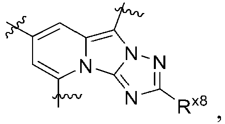 wherein R^{x8} is as defined in any one of the embodiments of the present disclosure; more preferably, 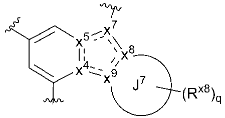 is 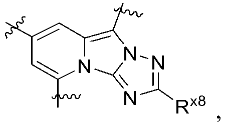 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 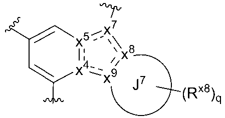 is 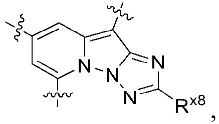 wherein R^{x8} is as defined in any one of the embodiments of the present disclosure; preferably, 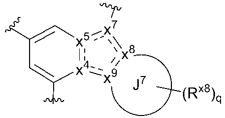 is 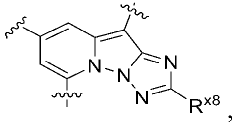 wherein R^{x8} is as defined in any one of the embodiments of the present disclosure; more preferably, 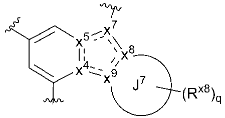 is 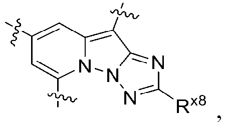 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 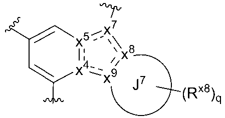 is 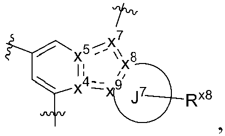 wherein R^{x8} is as defined in any one of the embodiments of the present disclosure.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 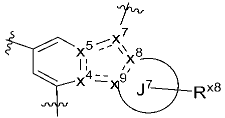 is selected from the group consisting of

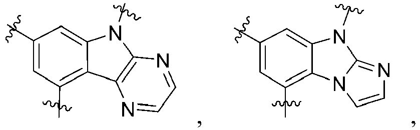

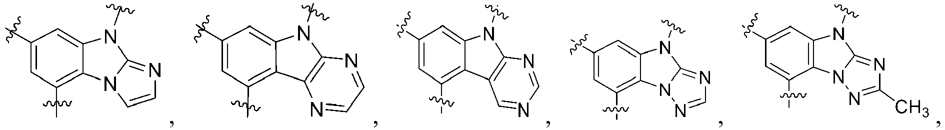

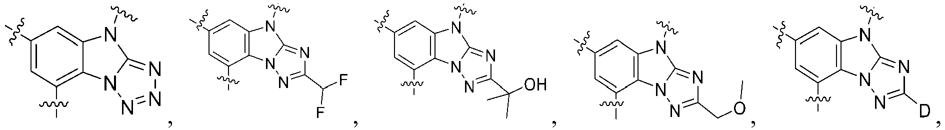

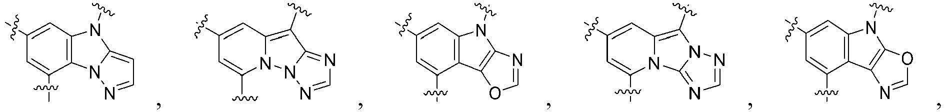

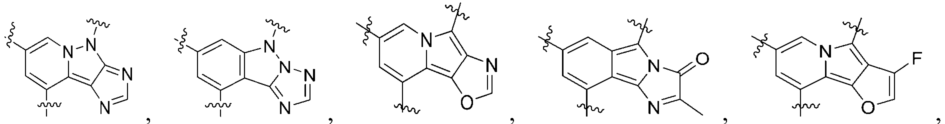

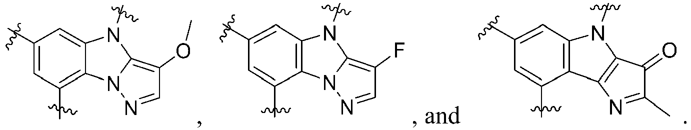

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, 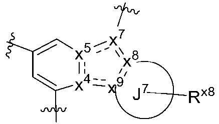 is selected from the group consisting of

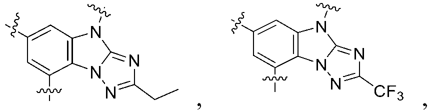

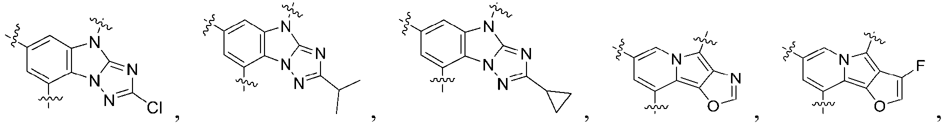

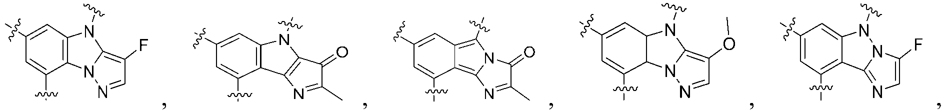

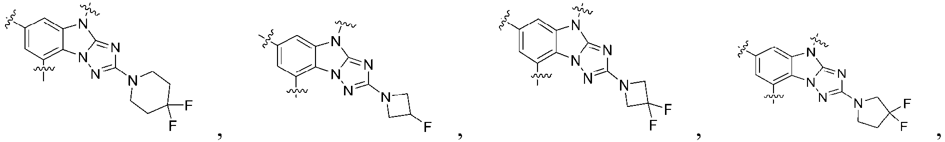

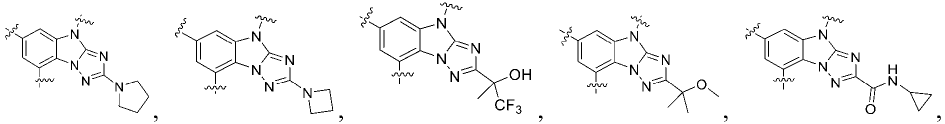

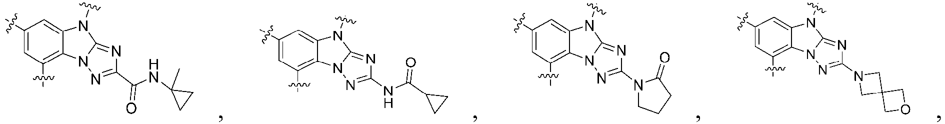

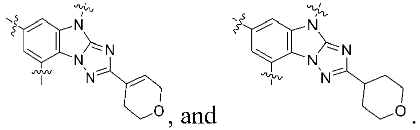

In some embodiments of the present disclosure, in the compound of formula (IA), formula (I), formula (II), formula (II-1A), formula (II-1B), formula (II-2A), formula (II-2B), formula (II-1C), formula (II-2C), formula (II-3A), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{a} is -CD₃.

In some embodiments of the present disclosure, in the compound of formula (IA), formula (I), formula (II), formula (II-1A), formula (II-1B), formula (II-2A), formula (II-2B), formula (II-1C), formula (II-2C), formula (II-3A), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{a} is selected from the group consisting of hydrogen, cyano, formyl, -CONH₂, -CH₂OH, - CH₂OC₁₋₄ alkyl, C₁₋₄ alkyl, C₁₋₄ haloalkyl, and C₂₋₆ haloalkenyl; preferably, R^{a} is selected from the group consisting of -CH₃, -CH₂F, -CN, and -CH₂=CHF.

In some embodiments of the present disclosure, in the compound of formula (IA), formula (I), formula (II), formula (II-1A), formula (II-1B), formula (II-2A), formula (II-2B), formula (II-1C), formula (II-2C), formula (II-3A), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, and -CN.

In some embodiments of the present disclosure, in the compound of formula (IA), formula (I), formula (II), formula (II-1A), formula (II-1B), formula (II-2A), formula (II-2B), formula (II-1C), formula (II-2C), formula (II-3A), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{a} is selected from the group consisting of -CHF₂ and -CHF₃.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, each R^{b} is identical or different, and is independently selected from the group consisting of hydrogen, C₁₋₆ alkyl, -C(O)R^{ba}, -C(O)OR^{bb}, and -C(O)NR^{bc}R^{bd}; wherein R^{ba}, R^{bb}, R^{bc}, and R^{bd} are as defined in any embodiment of formula (IA) or any embodiment of formula (I); preferably, each R^{b} is identical or different, and is independently selected from the group consisting of hydrogen, C₁₋₆ alkyl, -C(O)R^{ba}, and -C(O)NR^{bc}R^{bd}; wherein R^{ba}, R^{bc}, and R^{bd} are as defined in any embodiment of formula (IA) or any embodiment of formula (I).

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of C₁₋₆ hydroxyalkyl, 3- to 8-membered heterocyclyl, and -S(O)₂R^{ba}; wherein R^{ba} is as defined in any embodiment of formula (IA) or any embodiment of formula (I); preferably, R^{b} and R^{b1} are each identical or different, and are each independently selected from the group consisting of hydrogen, C₁₋₆ hydroxyalkyl, 3- to 8-membered heterocyclyl, and -S(O)₂R^{ba}; wherein R^{ba} is selected from the group consisting of hydrogen, C₁₋₆ alkyl, 3- to 8-membered heterocyclyl, C₁₋₆ haloalkyl, and C₁₋₆ hydroxyalkyl.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of hydrogen, C₁₋₆ alkyl, C₁₋₆ hydroxyalkyl, 3- to 8-membered heterocyclyl, - S(O)₂R^{ba}, -C(O)OR^{bb}, -C(O)R^{ba}, -C(O)NR^{bc}R^{bd}, and 5- to 6-membered heteroaryl optionally substituted by C₁₋₆ alkyl; wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, and 3- to 8-membered cycloalkyl.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of hydrogen,

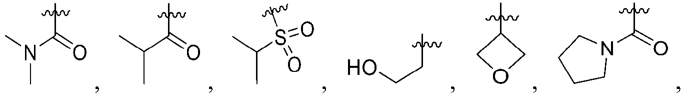

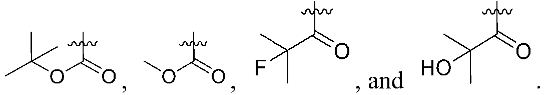

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of

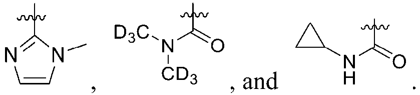

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of hydrogen, C₁₋₆ alkyl, C₁₋₆ hydroxyalkyl, 3- to 8-membered heterocyclyl, - S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 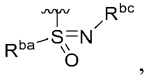 S(O)₂NR^{be}R^{bf}, and -S(O)R^{ba}; wherein R^{ba}, R^{bb}, R^{bc}, R^{bd}, R^{be}, and R^{bf} are as defined in any one of the embodiments of the present disclosure.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b2} and R^{b3} are identical or different, and are each independently selected from the group consisting of hydrogen, C₁₋₆ alkyl, C₂₋₆ alkenyl, and C₂₋₆ alkynyl; preferably, R^{b2} and R^{b3} are identical or different, and are each independently selected from the group consisting of hydrogen, methyl, vinyl, and ethynyl.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently selected from the group consisting of methyl, vinyl, ethynyl,

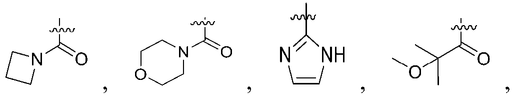

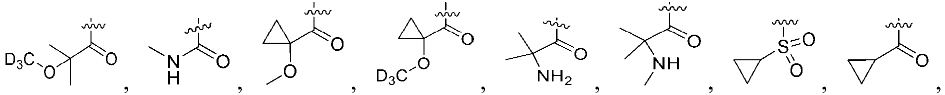

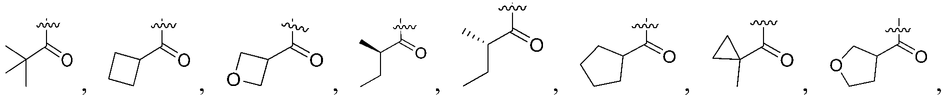

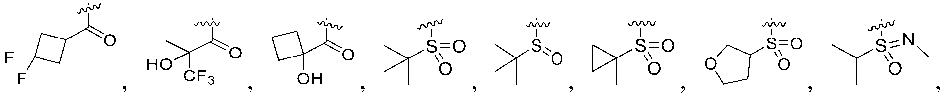

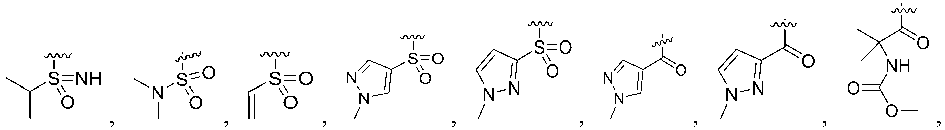

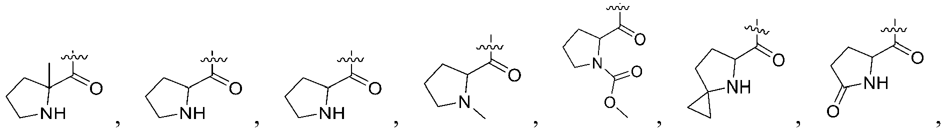

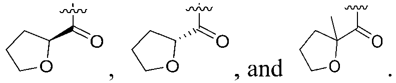

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{b} and R^{b1} are each identical or different, and are each independently

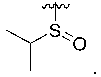

In some embodiments of the present disclosure, in the compound of formula (IA), formula (I), formula (II), formula (II-1A), formula (II-1B), formula (II-2A), formula (II-2B), formula (II-1C), formula (II-2C), formula (II-3A), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, each R^{b} is identical or different, and is independently selected from the group consisting of hydrogen, C₁₋₆ alkyl, -C(O)R^{ba}, and -C(O)NR^{bc}R^{bd}; wherein R^{ba}, R^{bc}, and R^{bd} are as defined in any embodiment of formula (IA) or any embodiment of formula (I).

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, x² is selected from the group consisting of N and CR^{x2}; wherein R^{x2} is selected from the group consisting of H, halogen, and C₁₋₆ alkyl, preferably H, Cl, F, and CH₃.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), formula (I-6A), formula (II-1A), formula (II-1B), formula (II-1C), formula (II-2C), formula (II-2A), or formula (II-2B), the pharmaceutically acceptable salt thereof, the stereoisomer thereof, the prodrug thereof, the N-oxide thereof, the solvate thereof, or the isotopic derivative thereof, R^{x2} is selected from the group consisting of H, halogen, and C₁₋₆ alkyl, preferably H, Cl, F, and CH₃.

In some embodiments of the present disclosure, in the compound of formula (IA), formula (I-1), formula (I-1A), formula (I-2), formula (I-2A), formula (I-3), formula (I-3A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{x2} is selected from the group consisting of H, Cl, F, and CH₃.

In some embodiments of the present disclosure, in the compound of formula (IA), formula (I), formula (II), formula (I-a), formula (I-1), formula (I-1A), formula (I-2), formula (I-2A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, x⁶ is selected from the group consisting of N and CR^{x6}; wherein R^{x6} is selected from the group consisting of H, halogen, and C₁₋₆ alkyl, preferably H, Cl, F, and CH₃.

In some embodiments of the present disclosure, in the compound of formula (IA), formula (I), formula (II), formula (I-a), formula (I-1), formula (I-1A), formula (I-2), formula (I-2A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), or formula (II-2B), the pharmaceutically acceptable salt thereof, the stereoisomer thereof, the prodrug thereof, the N-oxide thereof, the solvate thereof, or the isotopic derivative thereof, R^{x6} is selected from the group consisting of H, halogen, and C₁₋₆ alkyl, preferably H, Cl, F, or CH₃.

In some embodiments of the present disclosure, in the compound of formula (IA), formula (I), formula (II), formula (I-a), formula (I-1), formula (I-1A), formula (I-2), formula (I-2A), formula (I-4), formula (I-4A), formula (I-5), formula (I-5A), formula (I-6), formula (I-6A), formula (II-1A), formula (II-1B), formula (II-2A), or formula (II-2B), the pharmaceutically acceptable salt thereof, the stereoisomer thereof, the prodrug thereof, the N-oxide thereof, the solvate thereof, or the isotopic derivative thereof, R^{x6} is selected from the group consisting of H, Cl, F, and CH₃.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, x⁹ is selected from the group consisting of N, NR^{x9}, CR^{x9}, and CR^{x9}R^{x9}. wherein R^{x9}, at each occurrence, is identical or different, and is independently selected from the group consisting of H, halogen, and C₁₋₆ alkyl, preferably H, Cl, F, and CH₃; alternatively, two R^{x9} attached to the same carbon atom, together with the atom to which they are attached, form a C₃₋₆ cycloalkyl ring, wherein the C₃₋₆ cycloalkyl is preferably cyclopropyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, x⁹ is O.

In some embodiments of the present disclosure, in the compound of formula (IA), formula (I), formula (II), formula (I-1), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (I-a), formula (II-1A), formula (II-1C), or formula (II-1B), the pharmaceutically acceptable salt thereof, the stereoisomer thereof, the prodrug thereof, the N-oxide thereof, the solvate thereof, or the isotopic derivative thereof, R^{x9}, at each occurrence, is identical or different, and is independently selected from the group consisting of H, halogen, and C₁₋₆ alkyl, preferably H, Cl, F, CH₃, and CH(CH₃)₂.

In some embodiments of the present disclosure, in the compound of formula (IA), formula (I), formula (II), formula (I-1), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (I-a), formula (II-1A), formula (II-1C), or formula (II-1B), the pharmaceutically acceptable salt thereof, the stereoisomer thereof, the prodrug thereof, the N-oxide thereof, the solvate thereof, or the isotopic derivative thereof, R^{x9}, at each occurrence, is identical or different, and is independently selected from the group consisting of ethyl, cyclopropyl, and-CH₂CF₃.

In some embodiments of the present disclosure, in the compound of formula (IA), formula (I), formula (II), formula (I-1), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (I-a), formula (II-1A), formula (II-1C), or formula (II-1B), the pharmaceutically acceptable salt thereof, the stereoisomer thereof, the prodrug thereof, the N-oxide thereof, the solvate thereof, or the isotopic derivative thereof, R^{x9}, at each occurrence, is identical or different, and is independently

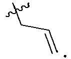

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1C), or formula (II-1B), the pharmaceutically acceptable salt thereof, the stereoisomer thereof, the prodrug thereof, the N-oxide thereof, the solvate thereof, or the isotopic derivative thereof, R^{x9}, at each occurrence, is identical or different, and is independently selected from the group consisting of H, Cl, F, and CH₃.

In some embodiments of the present disclosure, in the above formula containing two R^{x9}, two R^{x9} attached to the same carbon atom, together with the atom to which they are attached, form a 3- to 6-membered cycloalkyl ring, wherein the 3- to 6-membered cycloalkyl is preferably cyclopropyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, x⁸ is selected from the group consisting of N, NR^{x8}, CR^{x8}, and CR^{x8}R^{x8}; wherein R^{x8}, at each occurrence, is identical or different, and is independently selected from the group consisting of H, halogen, amino, C≡CH, CN, and C₁₋₆ alkyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), or formula (I-5A), the pharmaceutically acceptable salt thereof, the stereoisomer thereof, the prodrug thereof, the N-oxide thereof, the solvate thereof, or the isotopic derivative thereof, x⁸ is C(=O).

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of H, halogen, amino, C≡CH, CN, and C₁₋₆ alkyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of methyl, amino, and methoxy.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of deuterium,

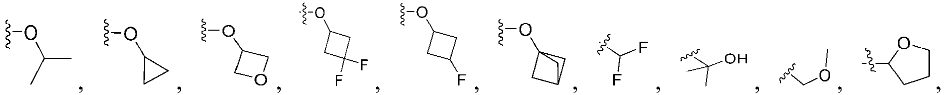

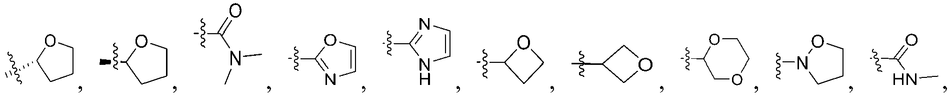

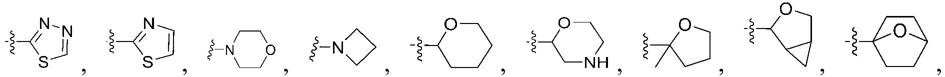

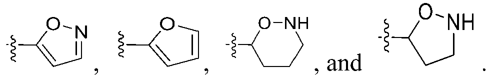

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of oxo, Cl, F, ethyl, trifluoromethyl, isopropyl, cyclopropyl,

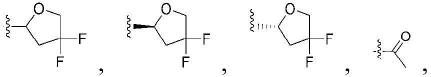

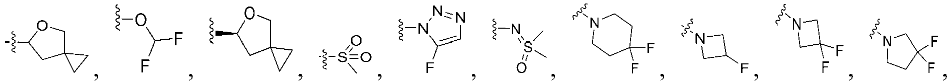

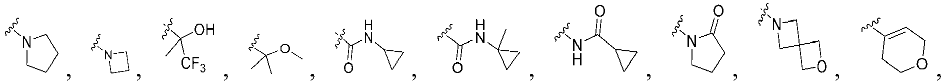

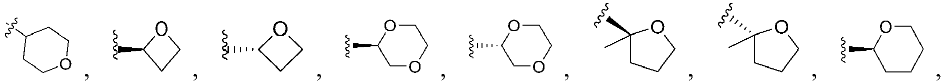

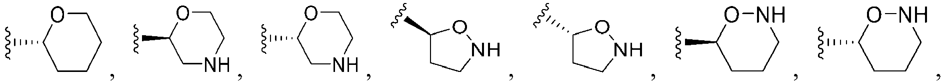

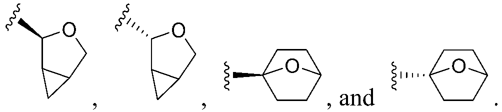

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of hydrogen, deuterium, oxo, halogen, C₁₋₆ alkyl, C₁₋₆ haloalkyl, C₁₋₆ hydroxyalkyl, C₁₋₆ alkoxy-C₁₋₆ alkyl,-NHC(O)R^{ba}, -OR^{f}, -C(O)R^{ba}, -S(O)₂R^{ba}, -N=S(O)R^{ba}, -C(O)NR^{bc}R^{bd}, 3- to 8-membered heterocyclyl, and 5- to 10-membered heteroaryl; wherein the C₁₋₆ alkyl, 3- to 8-membered heterocyclyl, and 5- to 10-membered heteroaryl are optionally substituted by one or more substituents selected from the group consisting of deuterium, halogen, hydroxyl, and C₁₋₆ alkyl; R^{f}, R^{ba}, R^{bc}, and R^{bd} are as defined in any one of the embodiments of the present disclosure.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently 3- to 8-membered heterocyclyl optionally substituted by one or more substituents selected from the group consisting of halogen, C₁₋₆ alkyl, hydroxyl, C₁₋₆ hydroxyalkyl, and C₁₋₆ haloalkyl, preferably 5- to 6-membered heterocyclyl optionally substituted by one or more substituents selected from the group consisting of halogen, C₁₋₆ alkyl, hydroxyl, C₁₋₆ hydroxyalkyl, and C₁₋₆ haloalkyl, more preferably 5- to 6-membered oxygen-containing heterocyclyl optionally substituted by one or more substituents selected from the group consisting of halogen, C₁₋₆ alkyl, hydroxyl, C₁₋₆ hydroxyalkyl, and C₁₋₆ haloalkyl, further preferably 5-membered oxygen-containing heterocyclyl optionally substituted by one or more substituents selected from the group consisting of halogen, C₁₋₆ alkyl, hydroxyl, C₁₋₆ hydroxyalkyl, and C₁₋₆ haloalkyl, further preferably tetrahydrofuranyl optionally substituted by one or more substituents selected from the group consisting of halogen, C₁₋₆ alkyl, hydroxyl, C₁₋₆ hydroxyalkyl, and C₁₋₆ haloalkyl, and most preferably tetrahydrofuranyl. In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{x8}, at each occurrence, is identical or different, and is independently selected from the group consisting of hydrogen, deuterium, oxo, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-2A), formula (II-2C), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{f}, at each occurrence, is identical or different, and is independently selected from the group consisting of hydrogen, C₁₋₆ alkyl, C₁₋₆ haloalkyl, C₁₋₆ hydroxyalkyl; 3- to 6-membered cycloalkyl optionally substituted by one or more substituents selected from the group consisting of halogen and C₁₋₆ alkyl; and 3- to 6-membered heterocyclyl optionally substituted by one or more substituents selected from the group consisting of halogen and C₁₋₆ alkyl.

In some embodiments of the present disclosure, in the compound of formula (IA), formula (I), formula (II), formula (I-1), formula (I-a), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{x8} and R^{x9}, together with the atom to which they are attached, form a 3- to 7-membered ring, wherein the 3- to 7-membered ring is selected from the group consisting of 3- to 7-membered heterocyclyl, C₃₋₇ cycloalkyl, C₃₋₇ carbocyclyl, and 5- to 6-membered heteroaryl; wherein preferably, the 5-to 6-membered heteroaryl is furyl.

In some embodiments of the present disclosure, in the compound of formula (IA), formula (I), formula (II), formula (I-1), formula (I-a), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{x8} and R^{x9}, together with the atom to which they are attached, form a pyrazinyl ring.

In some embodiments of the present disclosure, in the compound of formula (IA), formula (I), formula (II), formula (I-1), formula (I-a), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, R^{x8} and R^{x9}, together with the atom to which they are attached, form a 5-membered nitrogen-containing heteroaryl ring.

In some embodiments of the present disclosure, in the compound of formula (IA), formula (I), formula (II), formula (I-1), formula (I-a), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), or formula (I-6A), the pharmaceutically acceptable salt thereof, the stereoisomer thereof, the prodrug thereof, the N-oxide thereof, the solvate thereof, or the isotopic derivative thereof, Rx8 and Rx9, together with the atom to which they are attached, form a 6-membered nitrogen-containing heteroaryl ring.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{ba} is selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, 3- to 8-membered cycloalkyl, C₂₋₆ alkenyl, and 5- to 6-membered heteroaryl; wherein the C₁₋₆ alkyl, 3- to 8-membered heterocyclyl, 3- to 8-membered cycloalkyl, and 5- to 6-membered heteroaryl are optionally substituted by one or more substituents selected from the group consisting of C₁₋₆ alkyl, halogen, C₁₋₆ deuteroalkyl, C₁₋₆ haloalkyl, hydroxyl, C₁₋₆ alkoxy, C₁₋₆ deuteroalkoxy, -C(O)OR^{bb1}, and -NR^{a1}R^{a2}; R^{bb1}, R^{a1}, and R^{a2} are as defined in any one of the embodiments of the present disclosure.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{bc} and R^{bd} are identical or different, and are each independently selected from the group consisting of hydrogen, C₁₋₆ alkyl, C₁₋₆ deuteroalkyl, and 3- to 8-membered cycloalkyl.

In some embodiments of the present disclosure, in the compound of formula (IA), formula (I), formula (II), formula (I-1), formula (I-1A), formula (I-2), formula (I-2A), formula (I-3), formula (I-3A), formula (I-3'), formula (I-3'A), formula (I-4), formula (I-4A), formula (I-4'), formula (I-4'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{a1}, R^{a2}, R^{bb}, and R^{bb1} are identical or different, and are each independently selected from the group consisting of hydrogen and C₁₋₆ alkyl.

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), formula (I-6A), formula (II-1A), formula (II-1B), formula (II-1C), formula (II-2C), formula (II-2A), or formula (II-2B), the pharmaceutically acceptable salt thereof, the stereoisomer thereof, the prodrug thereof, the N-oxide thereof, the solvate thereof, or the isotopic derivative thereof, R^{ba}, R^{bb}, R^{bc}, and R^{bd} are identical or different, and are each independently selected from the group consisting of hydrogen, methyl, -CD₃, isopropyl, *tert-*butyl,

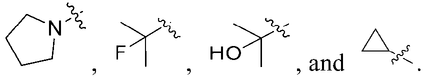

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{ba} is selected from the group consisting of hydrogen, isopropyl, tert-butyl, methoxy, tert-butoxy, 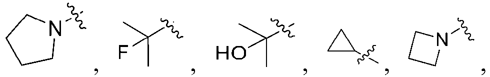 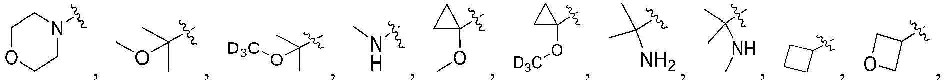 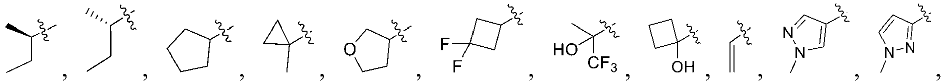 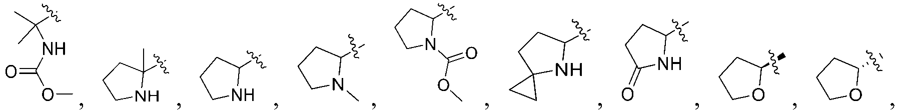 and

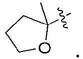

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{bc} and R^{bd} are identical or different, and are each independently selected from the group consisting of hydrogen, methyl, -CD₃, isopropyl, *tert-*butyl, and

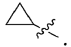

In some embodiments of the present disclosure, in the compound of formula (IA), formula (I), formula (II), formula (I-a), formula (I-1), formula (I-1A), formula (I-2), formula (I-2A), formula (I-3), formula (I-3A), formula (I-3'), formula (I-3'A), formula (I-5), formula (I-5A), formula (I-6), formula (I-6A), formula (II-1A), formula (II-1B), formula (II-1C), formula (II-2C), formula (II-2A), formula (II-2B), or formula (II-3A), the pharmaceutically acceptable salt thereof, the stereoisomer thereof, the prodrug thereof, the N-oxide thereof, the solvate thereof, or the isotopic derivative thereof, R^{be} and R^{bf} are identical or different, and are each independently selected from the group consisting of hydrogen, methyl, and isopropyl.

In some embodiments of the present disclosure, in the compound of formula (II-1A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, -CH₂=CHF, -CHF₂, and -CHF₃; R^{x2} is H; R^{x6} is H; R^{x9}, at each occurrence, is identical or different, and is independently selected from the group consisting of methyl, ethyl, isopropyl, cyclopropyl, and -CH₂CF₃; and R^{b1} is selected from the group consisting of hydrogen, C₁₋₆ hydroxyalkyl, 3- to 8-membered heterocyclyl, -S(O)₂R^{ba},-C(O)R^{ba}, -C(O)OR^{bb}, and -C(O)NR^{bc}R^{bd}; wherein R^{ba}, R^{bb}, R^{bc}, and R^{bd} are identical or different, and are each independently selected from the group consisting of hydrogen, C₁₋₆ alkyl, C₁₋₆ deuteroalkyl, 3- to 8-membered heterocyclyl, 3- to 8-membered cycloalkyl, C₁₋₆ haloalkyl, and C₁₋₆ hydroxyalkyl; R^{b2} and R^{b3} are both H.

In some embodiments of the present disclosure, in the compound of formula (II-1A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is selected from the group consisting of H, halogen, and C₁₋₆ alkyl; R^{x6} is selected from the group consisting of H, halogen, and C₁₋₆ alkyl; R^{x9}, at each occurrence, is identical or different, and is independently selected from the group consisting of H, halogen, and C₁₋₆ alkyl; and R^{b1} is selected from the group consisting of hydrogen, C₁₋₆ alkyl, -C(O)R^{ba}, -C(O)OR^{bb}, and -C(O)NR^{bc}R^{bd}; R^{b2} and R^{b3} are identical, and are both C₁₋₆ alkyl; wherein R^{ba}, R^{bb}, R^{bc}, and R^{bd} are as defined in any embodiment of formula (IA) or any embodiment of formula (I).

In some embodiments of the present disclosure, in the compound of formula (II-1A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x9}, at each occurrence, is identical or different, and is independently selected from the group consisting of H and C₁₋₆ alkyl; and R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl; R^{b2} and R^{b3} are identical or different, and are each independently selected from the group consisting of H and C₁₋₆ alkyl.

In some embodiments of the present disclosure, in the compound of formula (II-1A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x9}, at each occurrence, is identical or different, and is independently selected from the group consisting of H and C₁₋₆ alkyl; and R^{b1} is selected from the group consisting of hydrogen, 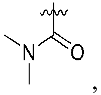 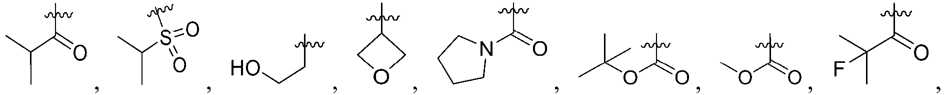 and 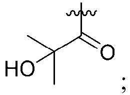 R^{b2} and R^{b3} are identical, and are both selected from the group consisting of H and methyl.

In some embodiments of the present disclosure, in the compound of formula (II-2A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is selected from the group consisting of H, halogen, and C₁₋₆ alkyl; R^{x6} is selected from the group consisting of H, halogen, and C₁₋₆ alkyl; R^{x8}, at each occurrence, is identical or different, and is independently selected from the group consisting of H, halogen, amino, C≡CH, CN, and C₁₋₆ alkyl; and R^{b1} is selected from the group consisting of hydrogen, C₁₋₆ alkyl, -C(O)R^{ba}, -C(O)OR^{bb}, and -C(O)NR^{bc}R^{bd}; R^{b2} and R^{b3} are identical, and are both C₁₋₆ alkyl; wherein R^{ba}, R^{bb}, R^{bc}, and R^{bd} are as defined in any embodiment of formula (IA) or any embodiment of formula (I).

In some embodiments of the present disclosure, in the compound of formula (II-2A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x8}, at each occurrence, is identical or different, and is independently selected from the group consisting of H, halogen, amino, C≡CH, CN, and C₁₋₆ alkyl; and R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl; R^{b2} and R^{b3} are identical or different, and are each independently selected from the group consisting of H and C₁₋₆ alkyl.

In some embodiments of the present disclosure, in the compound of formula (II-2A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x8}, at each occurrence, is identical or different, and is independently selected from the group consisting of H and amino; and R^{b1} is selected from the group consisting of hydrogen, 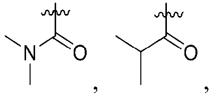 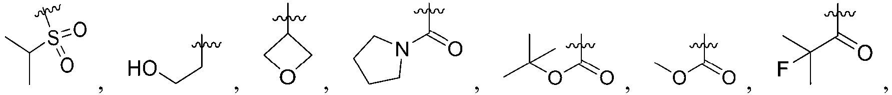 and 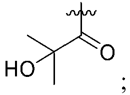 R^{b2} and R^{b3} are identical, and are both selected from the group consisting of H and methyl.

In some embodiments of the present disclosure, in the compound of formula (II-1B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is selected from the group consisting of H, halogen, and C₁₋₆ alkyl; R^{x6} is selected from the group consisting of H, halogen, and C₁₋₆ alkyl; R^{x9}, at each occurrence, is identical or different, and is independently selected from the group consisting of H, halogen, and C₁₋₆ alkyl; and R^{b1} is selected from the group consisting of hydrogen, C₁₋₆ alkyl, -C(O)R^{ba}, -C(O)OR^{bb}, and -C(O)NR^{bc}R^{bd}; wherein R^{ba}, R^{bb}, R^{bc}, and R^{bd} are as defined in any embodiment of formula (IA) or any embodiment of formula (I).

In some embodiments of the present disclosure, in the compound of formula (II-1B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x9}, at each occurrence, is identical or different, and is independently selected from the group consisting of H and C₁₋₆ alkyl; and R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl.

In some embodiments of the present disclosure, in the compound of formula (II-1B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x9}, at each occurrence, is identical or different, and is independently selected from the group consisting of H and C₁₋₆ alkyl; and R^{b1} is selected from the group consisting of hydrogen, 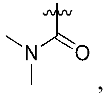 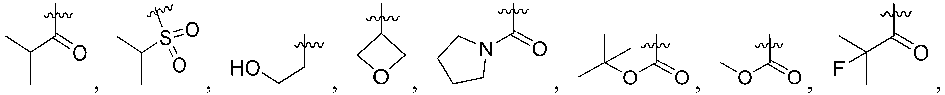 and

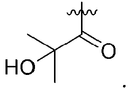

In some embodiments of the present disclosure, in the compound of formula (II-2B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is selected from the group consisting of H, halogen, and C₁₋₆ alkyl; R^{x6} is selected from the group consisting of H, halogen, and C₁₋₆ alkyl; R^{x8}, at each occurrence, is identical or different, and is independently selected from the group consisting of H, halogen, amino, C≡CH, CN, and C₁₋₆ alkyl; and R^{b1} is selected from the group consisting of hydrogen, C₁₋₆ alkyl, -C(O)R^{ba}, -C(O)OR^{bb}, and -C(O)NR^{bc}R^{bd}; wherein R^{ba}, R^{bb}, R^{bc}, and R^{bd} are as defined in any embodiment of formula (IA) or any embodiment of formula (I).

In some embodiments of the present disclosure, in the compound of formula (II-2B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x8}, at each occurrence, is identical or different, and is independently selected from the group consisting of H, halogen, amino, C≡CH, CN, and C₁₋₆ alkyl; and R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, and -C(O)NR^{bc}R^{bd}; wherein R^{ba}, R^{bb}, R^{bc}, and R^{bd} are identical or different, and are each independently selected from the group consisting of hydrogen, C₁₋₆ alkyl, 3- to 8-membered heterocyclyl, C₁₋₆ haloalkyl, and C₁₋₆ hydroxyalkyl.

In some embodiments of the present disclosure, in the compound of formula (II-2B), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃, -CH₂F, -CN, and -CH₂=CHF; R^{x2} is H; R^{x6} is H; R^{x8}, at each occurrence, is identical or different, and is independently selected from the group consisting of H and amino; and R^{b1} is selected from the group consisting of hydrogen, 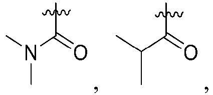 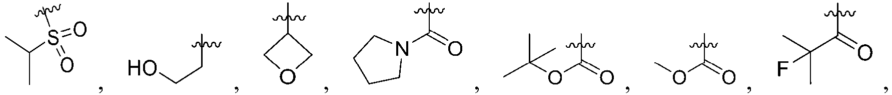 and

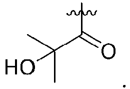

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, R^{a} is selected from the group consisting of -CH₃ and -CN; R^{b1} is hydrogen; 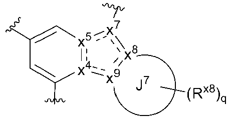 is selected from the group consisting of 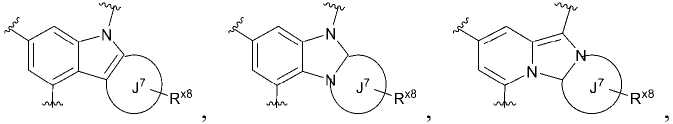 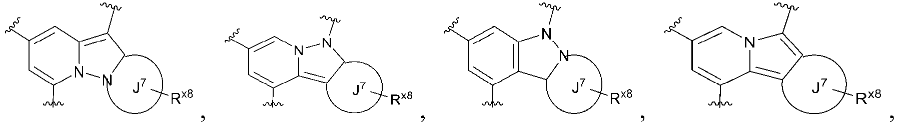 and 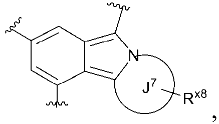 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, R^{a} is -CH₃; R^{b1} is hydrogen; 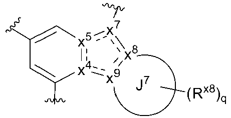 is selected from the group consisting of 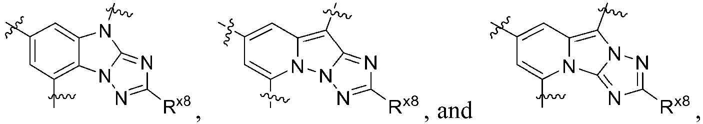 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-3A), the pharmaceutically acceptable salt thereof, the stereoisomer thereof, the prodrug thereof, the N-oxide thereof, the solvate thereof, or the isotopic derivative thereof, R^{a} is -CH₃; R^{b1} is hydrogen; 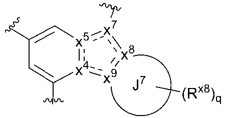 is 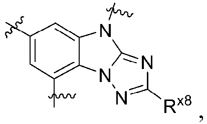 wherein R^{x8} is selected from the group consisting of hydrogen, deuterium, halogen, hydroxyl, amino; C₁₋₆ alkyl optionally substituted by one or more substituents selected from the group consisting of halogen and hydroxyl; C₁₋₆ haloalkyl, C₁₋₆ hydroxyalkyl, C₁₋₆ alkoxy-C₁₋₆ alkyl; and 3- to 8-membered heterocyclyl optionally substituted by one or more substituents selected from the group consisting of halogen and C₁₋₆ alkyl.

In some embodiments of the present disclosure, in the compound of formula (II-2L), the pharmaceutically acceptable salt thereof, the stereoisomer thereof, the prodrug thereof, the N-oxide thereof, the solvate thereof, or the isotopic derivative thereof, R^{a} is -CH₃; R^{b1} is hydrogen; 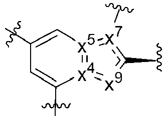 is selected from the group consisting of

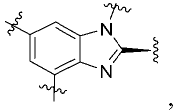

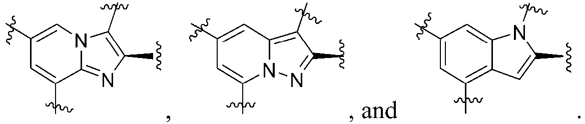

In some embodiments of the present disclosure, in the compound of formula (II-2M), the pharmaceutically acceptable salt thereof, the stereoisomer thereof, the prodrug thereof, the N-oxide thereof, the solvate thereof, or the isotopic derivative thereof, R^{a} is -CH₃; R^{b1} is hydrogen; 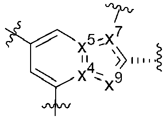 is selected from the group consisting of

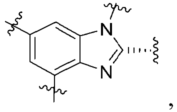

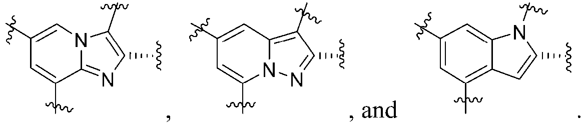

The present disclosure further provides the following compounds, and pharmaceutically acceptable salts thereof, or stereoisomers thereof, prodrugs thereof, N-oxides thereof, solvates thereof, or isotopic derivatives thereof, wherein the compound may be selected from the group consisting of any one of the structures listed in Table 1 below, and pharmaceutically acceptable salts thereof, or stereoisomers thereof, prodrugs thereof, N-oxides thereof, solvates thereof, or isotopic derivatives thereof,

**Table 1**

| | | | | |
|---|---|---|---|---|
| **Example 1** | **Example 2** | **Example 3** | **Example 4** | **Example 5** |
| 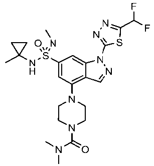 | 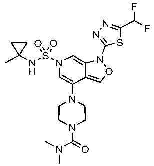 | 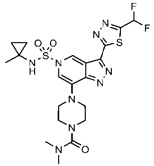 | 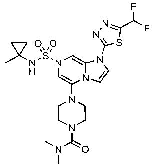 | 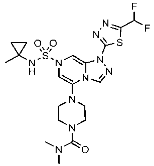 |
| **Example 6** | **Example 7** | **Example 8** | **Example 9** | **Example 10** |
| 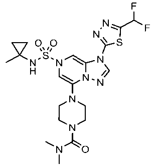 | 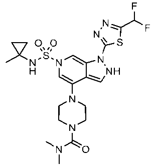 | 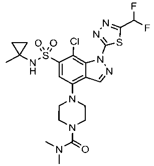 | 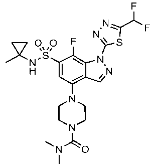 | 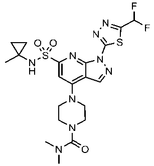 |
| **Example 11** | **Example 12** | **Example 13** | **Example 14** | **Example 15** |
| 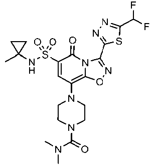 | 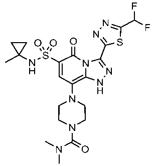 | 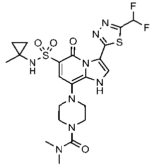 | 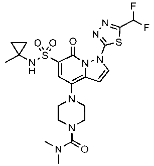 | 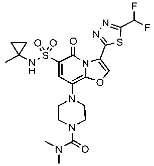 |
| **Example 16** | **Example 17** | **Example 18** | **Example 19** | **Example 20** |
| 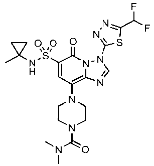 | 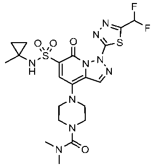 | 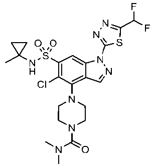 | 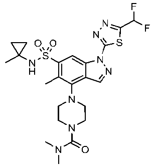 | 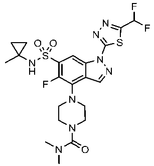 |
| **Example 21** | **Example 22** | **Example 23** | **Example 24** | **Example 25** |
| 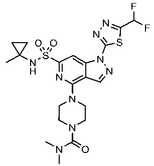 | 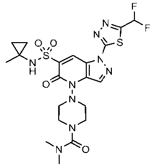 | 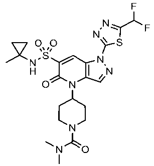 | 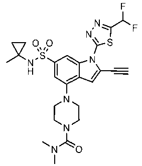 | 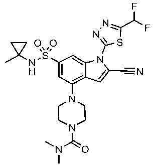 |
| **Example 26** | **Example 27** | **Example 28** | **Example 29** | **Example 30** |
| 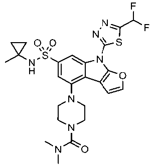 | 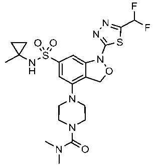 | 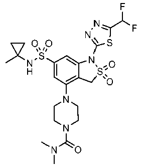 | 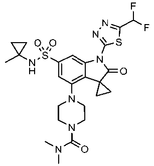 | 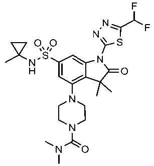 |
| **Example 31** | **Example 32** | **Example 33** | **Example 34** | **Example 35** |
| 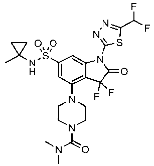 | 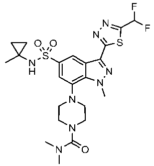 | 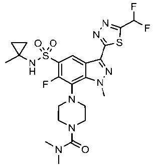 | 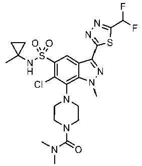 | 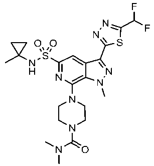 |
| **Example 36** | **Example 37** | **Example 38** | **Example 39** | **Example 40** |
| 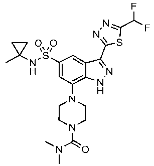 | 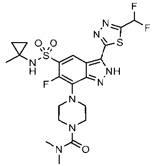 | 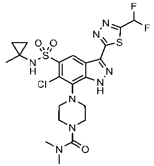 | 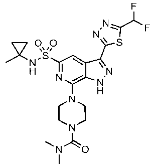 | 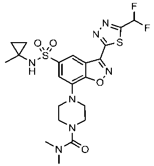 |
| **Example 41** | **Example 42** | **Example 43** | | |
| 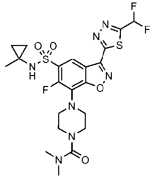 | 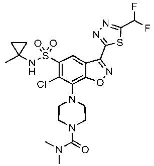 | 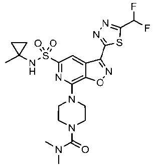 | 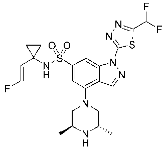 | 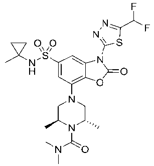 |
| 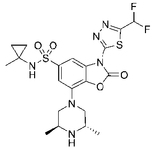 | 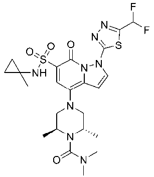 | 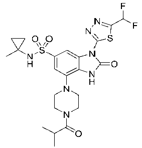 | 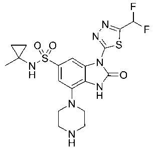 | 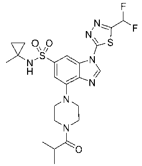 |
| 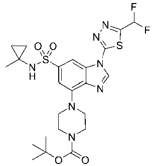 | 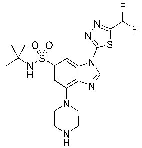 | 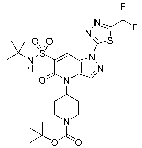 | 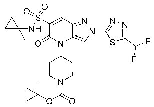 | 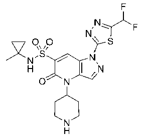 |
| 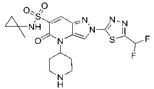 | 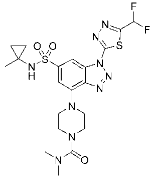 | 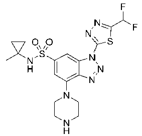 | 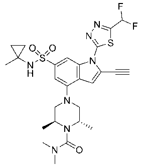 | 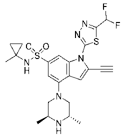 |
| 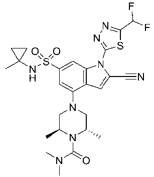 | 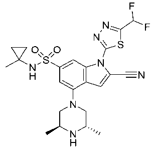 | 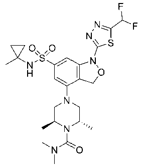 | 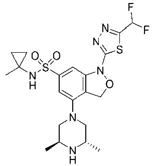 | 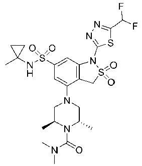 |
| 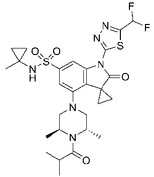 | 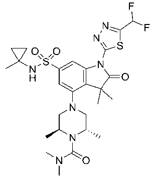 | 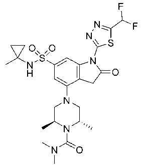 | 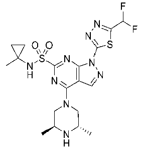 | 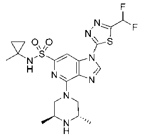 |
| 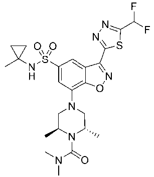 | 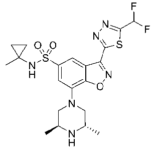 | 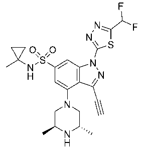 | 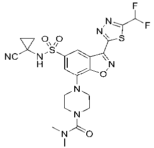 | 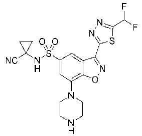 |
| 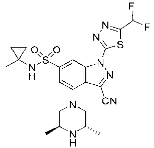 | 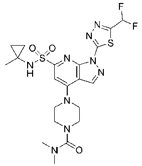 | 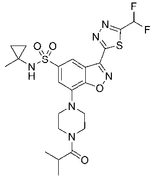 | 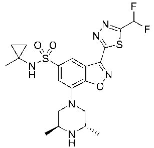 | 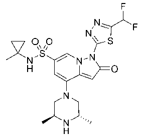 |
| 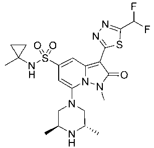 | 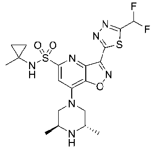 | 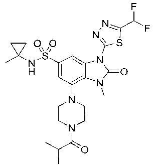 | 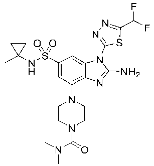 | 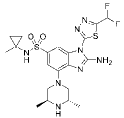 |
| 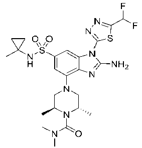 | 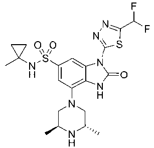 | 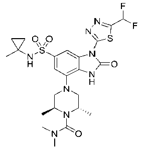 | 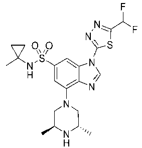 | 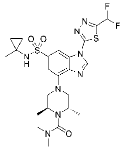 |
| 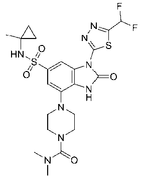 | 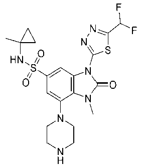 | 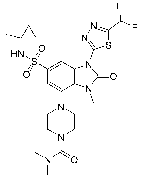 | 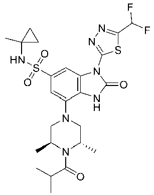 | 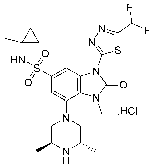 |
| 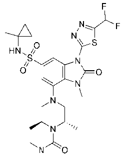 | 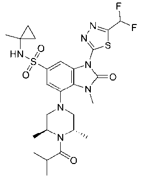 | 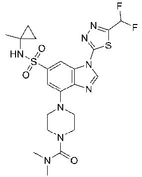 | 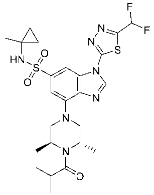 | 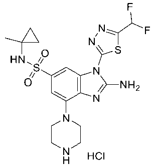 |
| 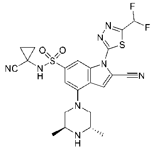 | 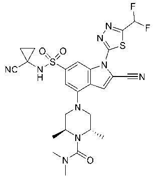 | 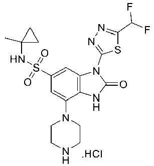 | 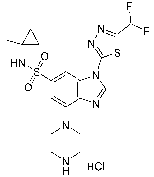 | 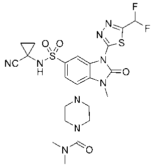 |
| 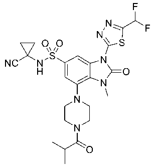 | 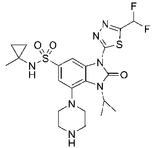 | 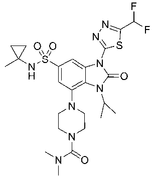 | 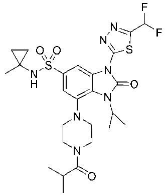 | 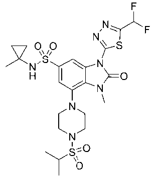 |
| 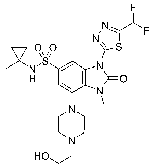 | 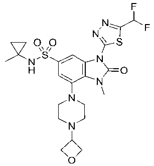 | 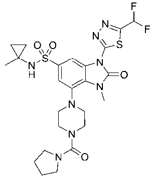 | 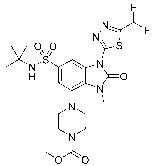 | 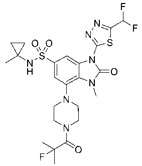 |
| 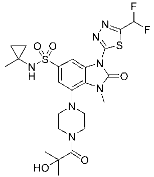 | 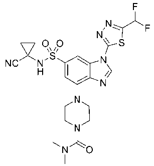 | 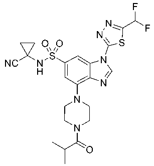 | 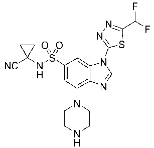 | 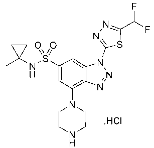 |
| 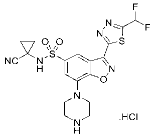 | 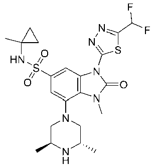 | 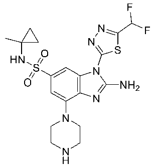 | 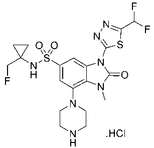 | 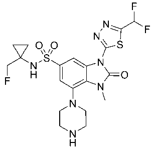 |
| 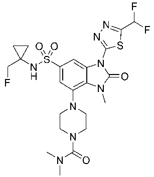 | 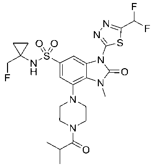 | 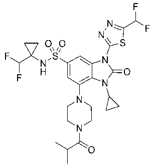 | 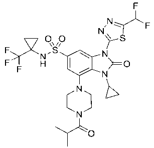 | 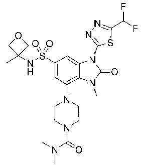 |
| 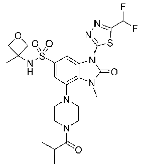 | 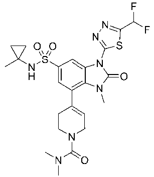 | 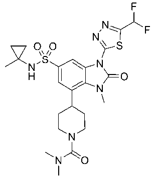 | 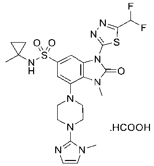 | 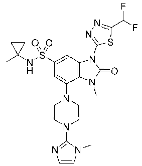 |
| 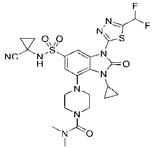 | 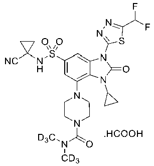 | 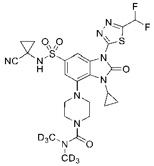 | 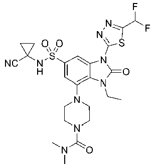 | 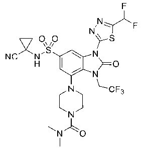 |
| 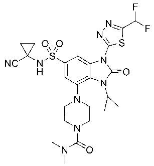 | 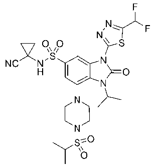 | 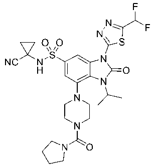 | 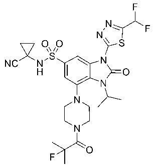 | 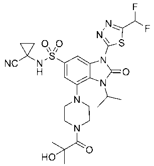 |
| 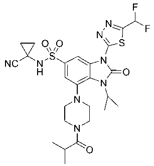 | 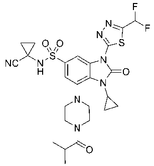 | 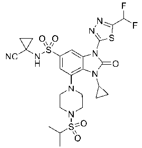 | 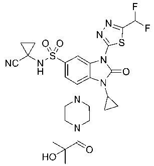 | 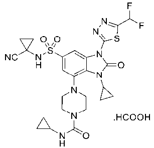 |
| 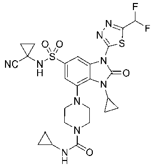 | 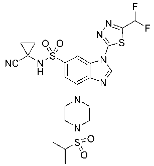 | 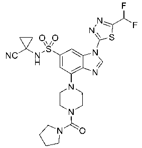 | 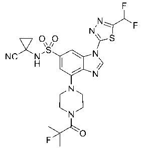 | 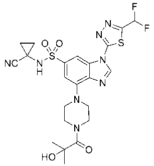 |
| 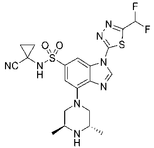 | 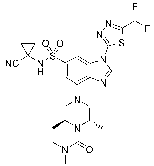 | 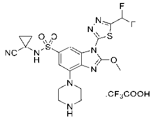 | 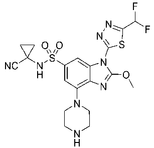 | 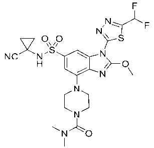 |
| 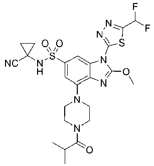 | 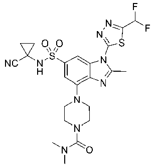 | 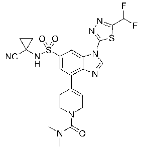 | 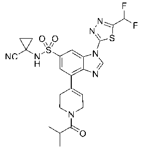 | 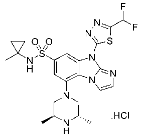 |
| 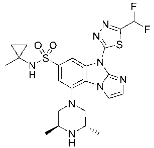 | 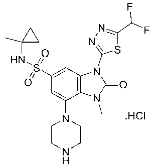 | 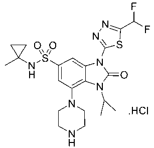 | 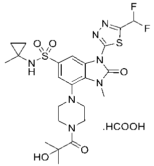 | 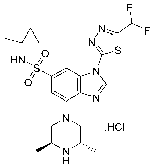 |
| 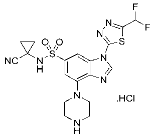 | 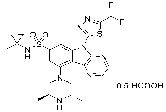 | 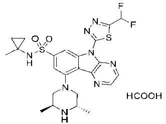 | 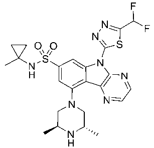 | 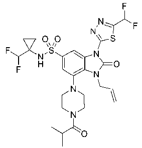 |
| 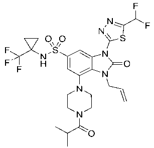 | 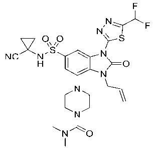 | 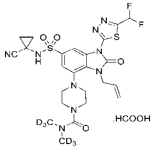 | 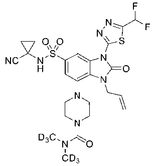 | 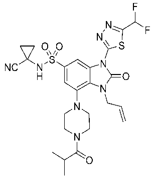 |
| 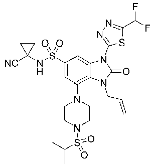 | 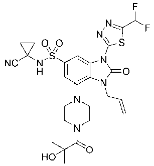 | 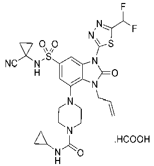 | 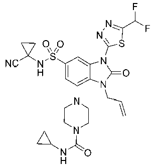 | |

The present disclosure further provides the following compounds, and pharmaceutically acceptable salts thereof, or stereoisomers thereof, prodrugs thereof, N-oxides thereof, solvates thereof, or isotopic derivatives thereof, wherein the compound may be selected from the group consisting of any one of the following structures, and pharmaceutically acceptable salts thereof, or stereoisomers thereof, prodrugs thereof, N-oxides thereof, solvates thereof, or isotopic derivatives thereof,

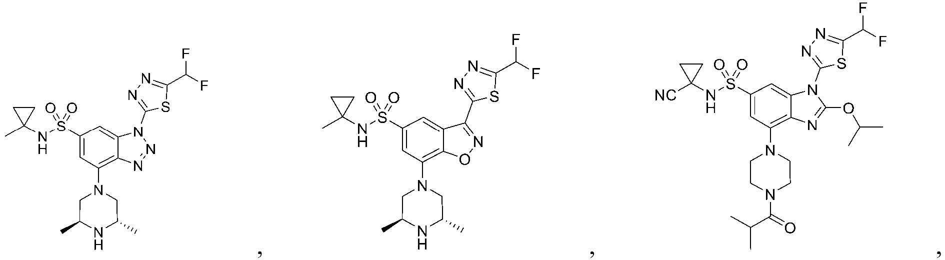

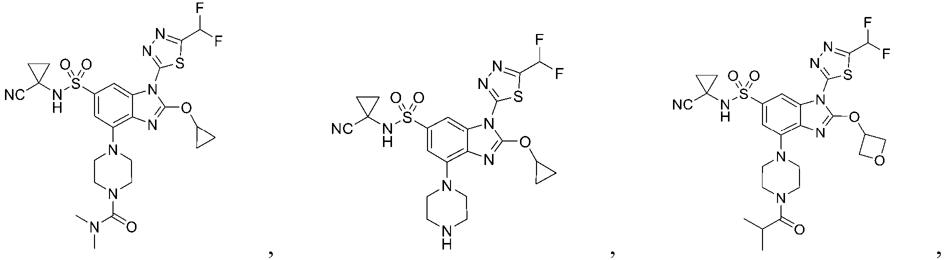

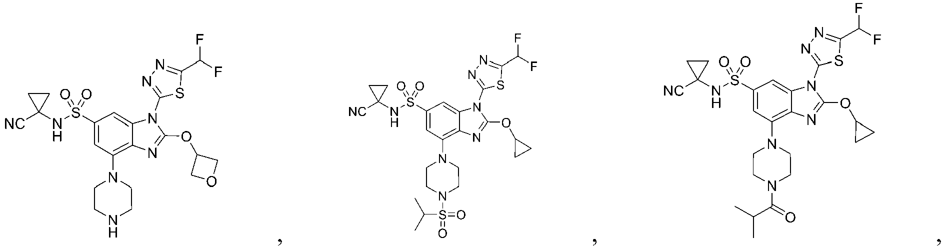

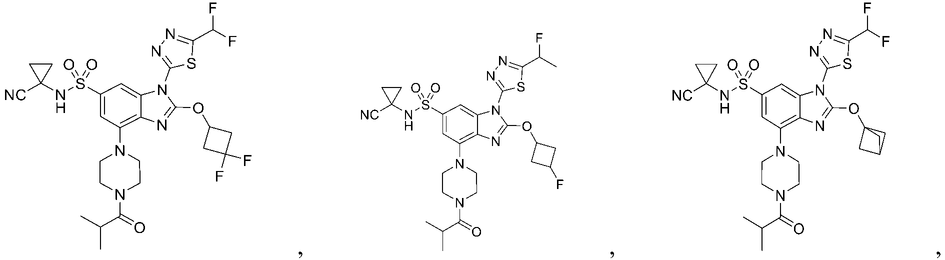

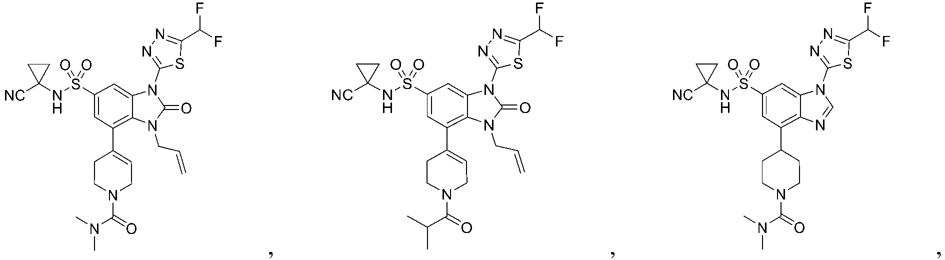

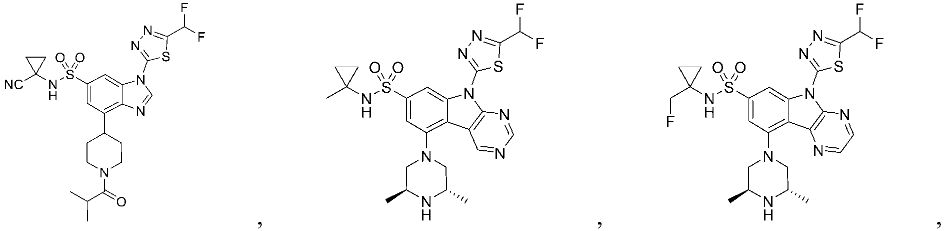

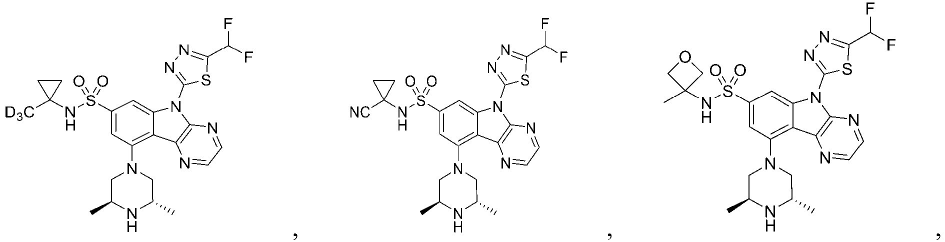

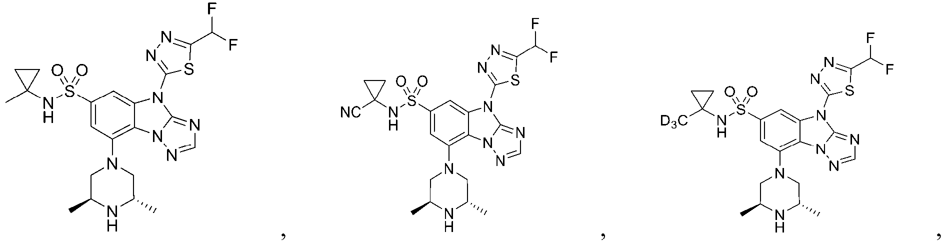

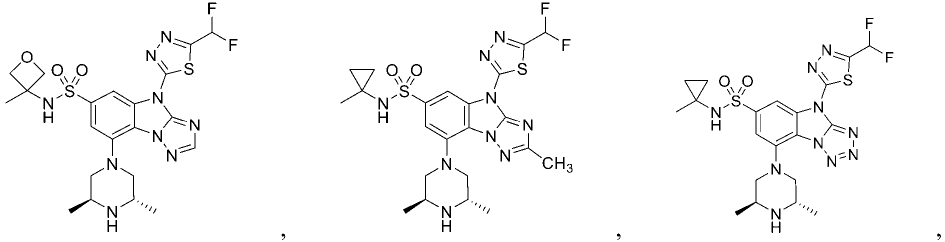

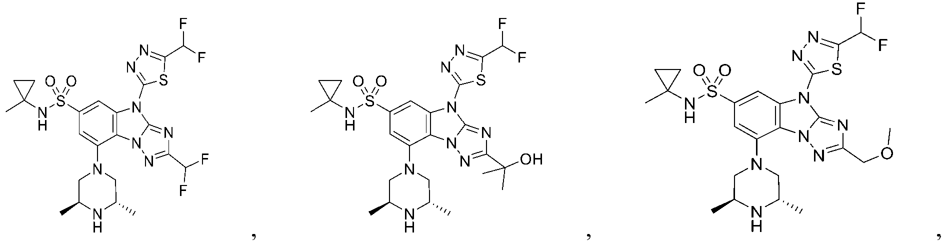

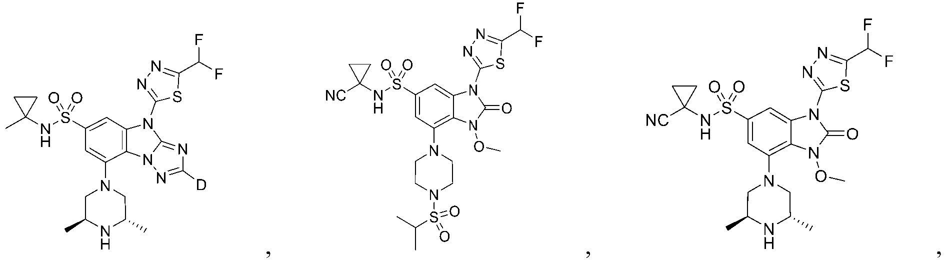

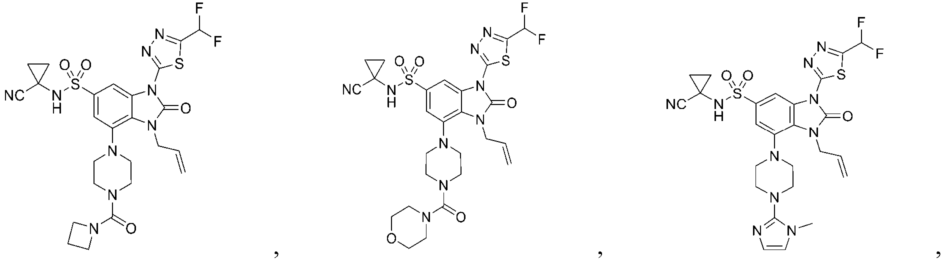

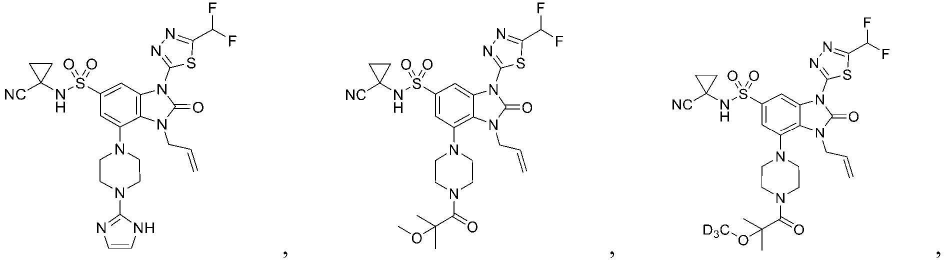

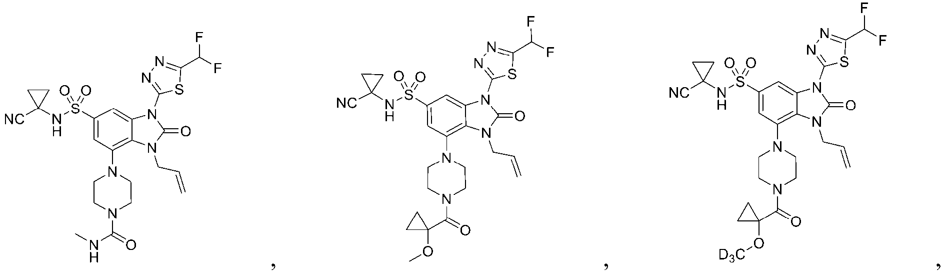

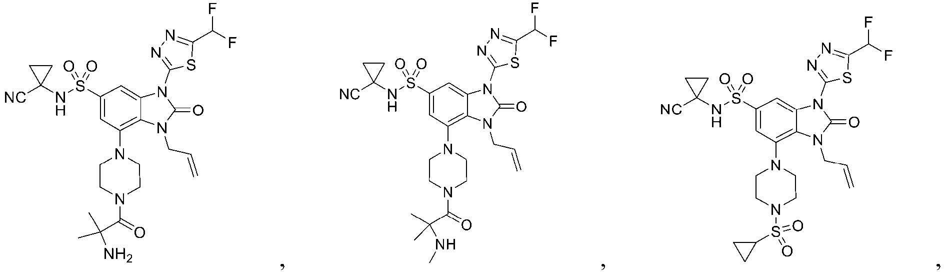

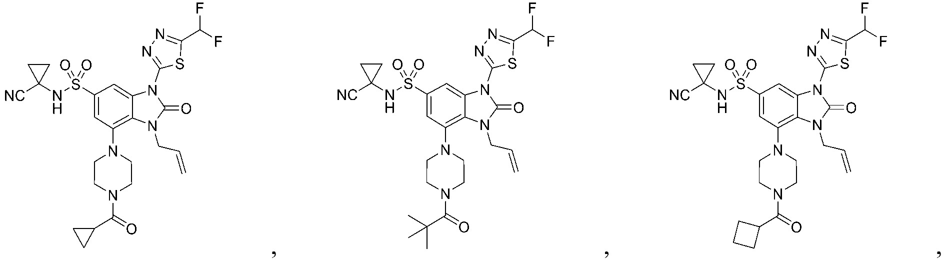

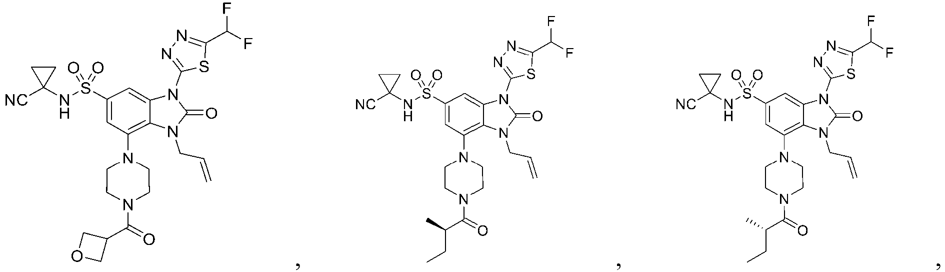

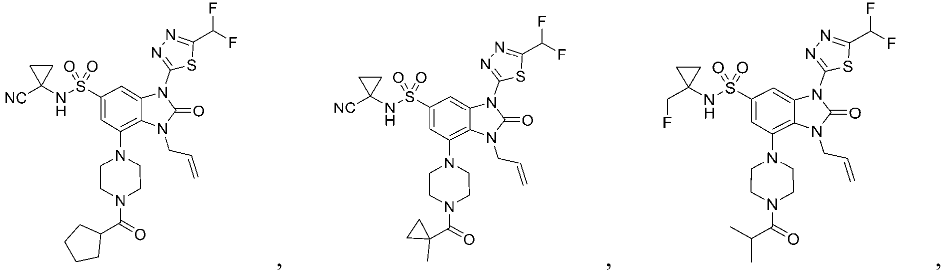

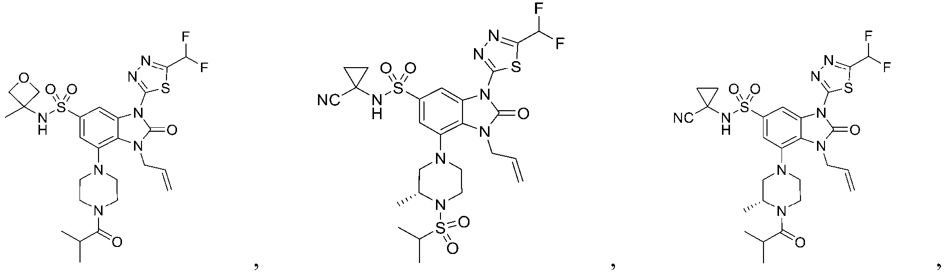

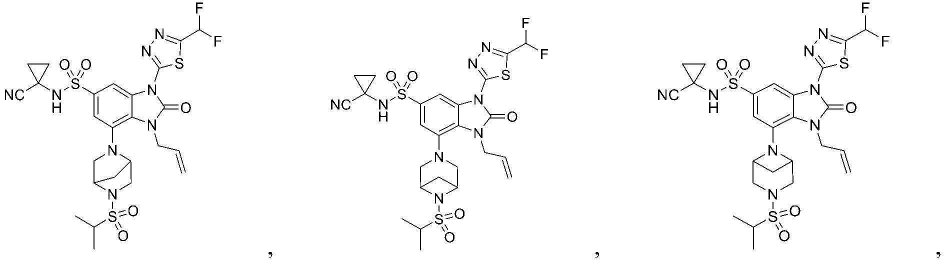

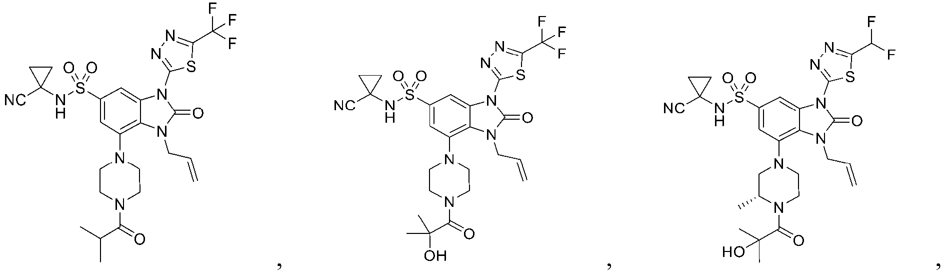

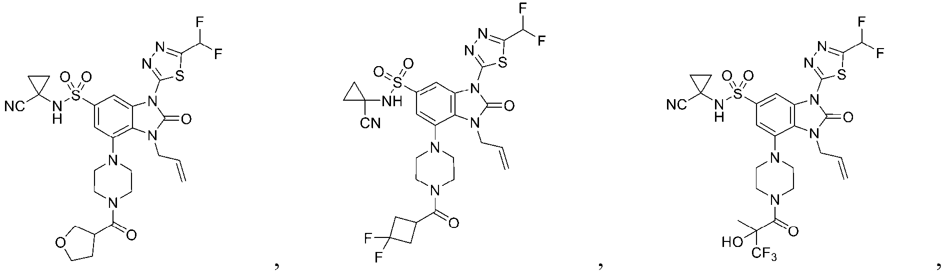

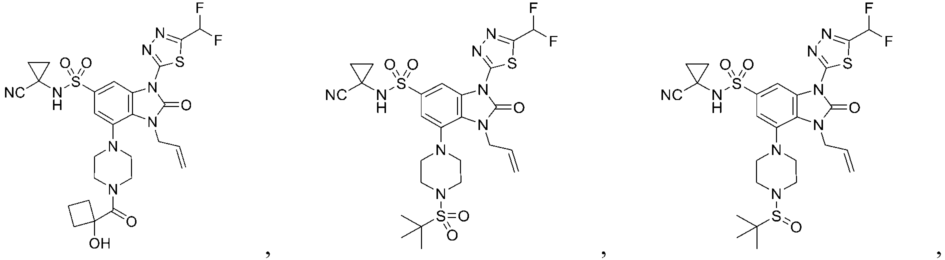

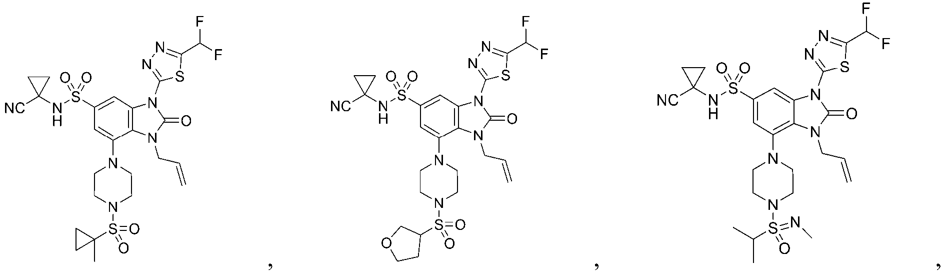

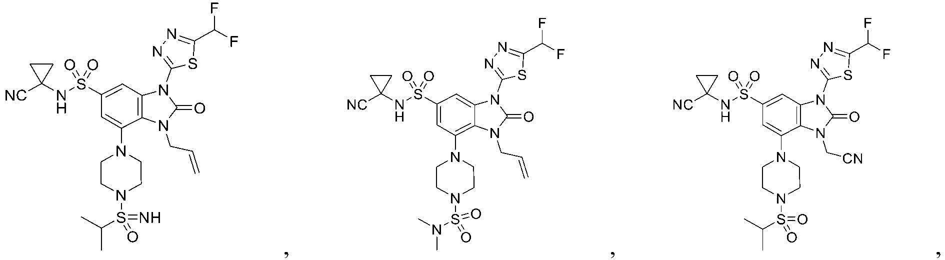

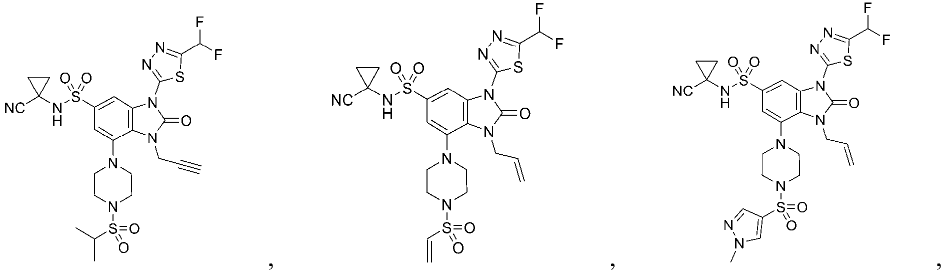

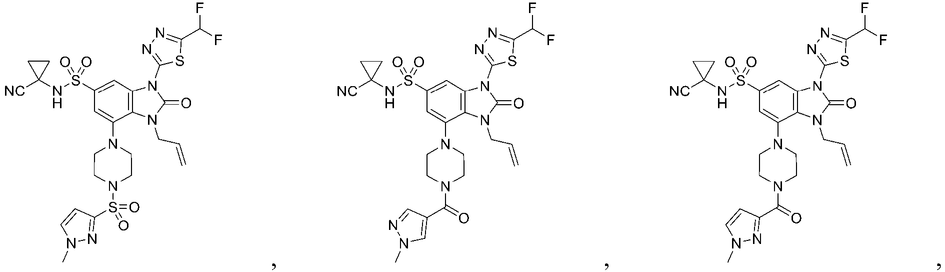

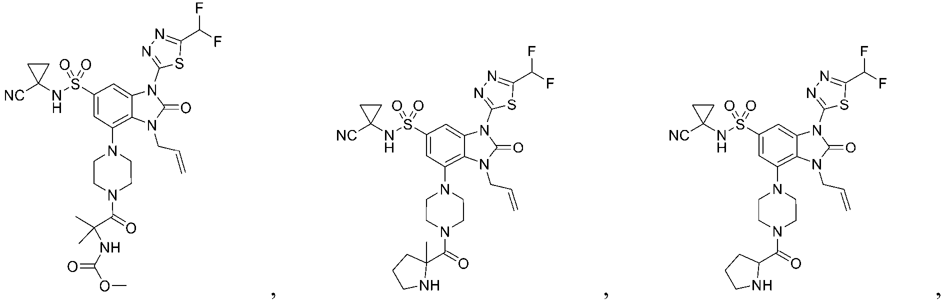

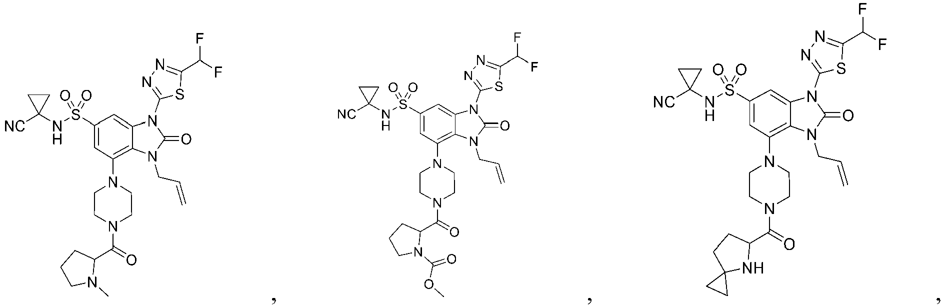

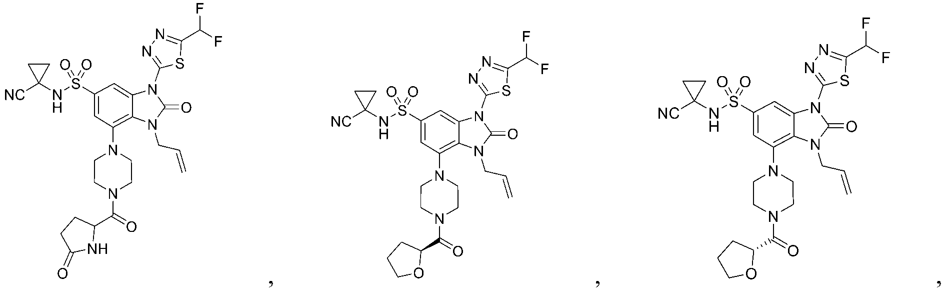

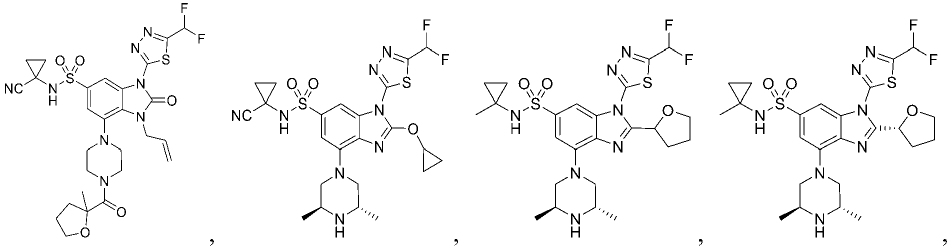

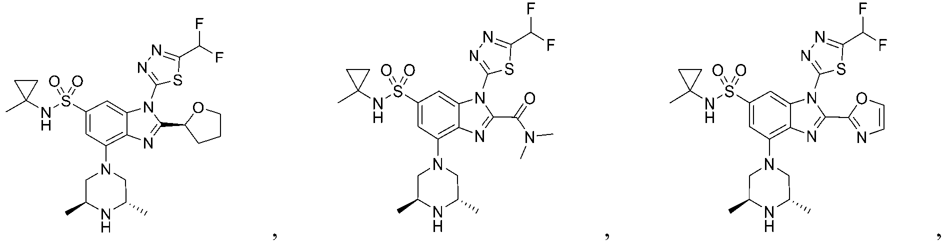

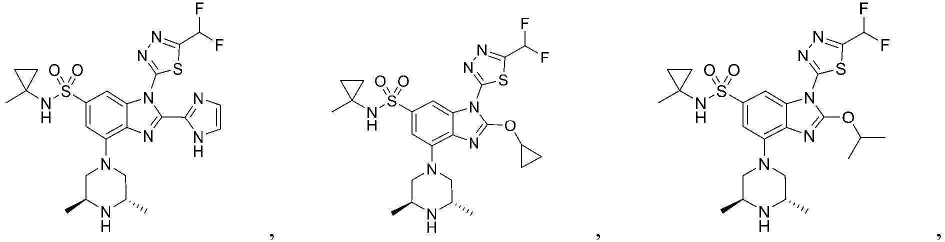

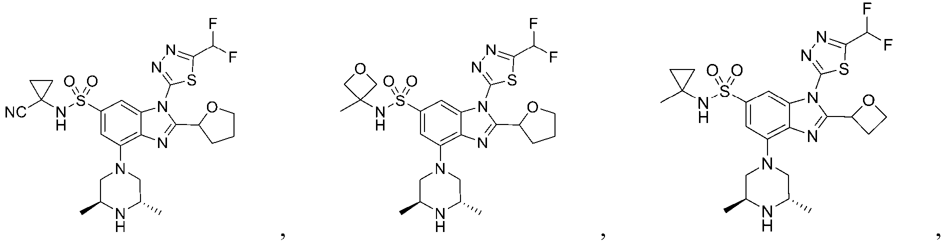

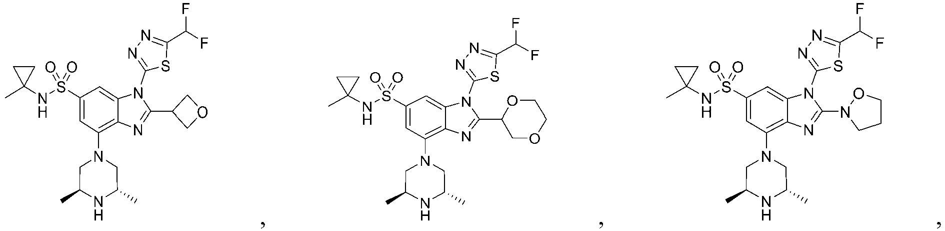

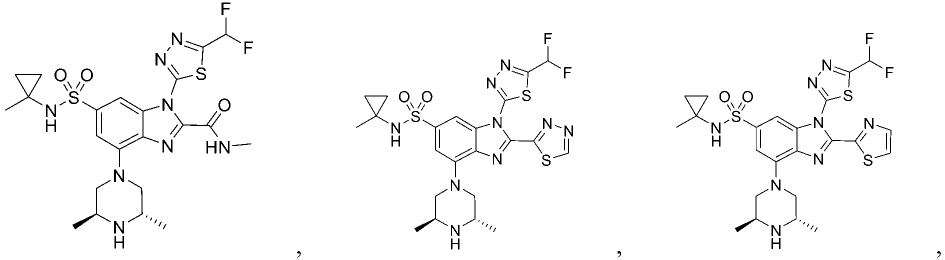

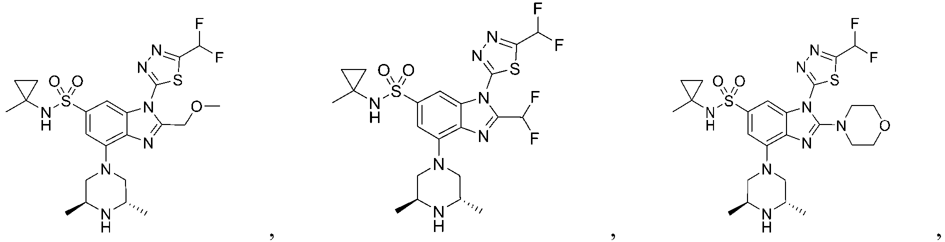

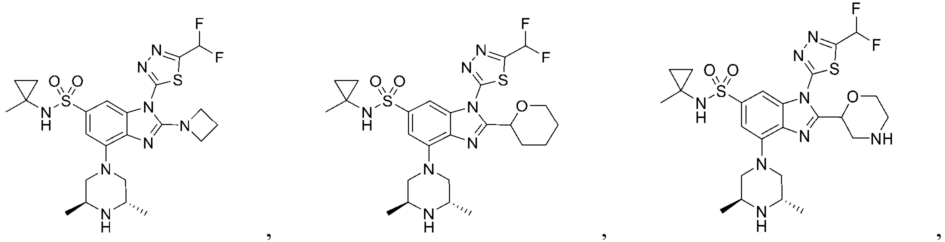

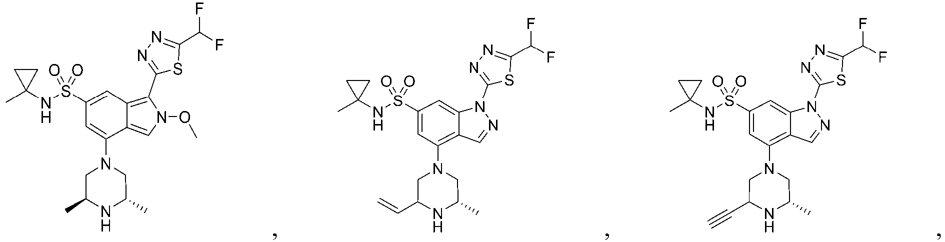

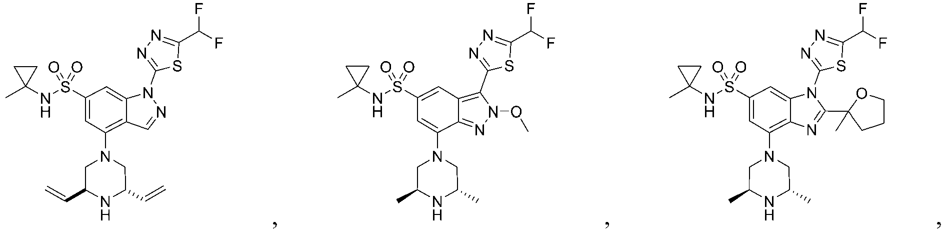

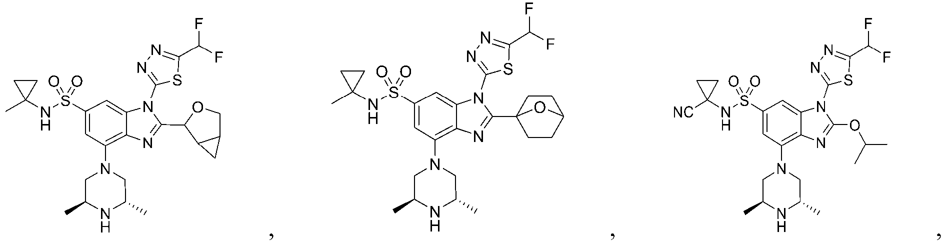

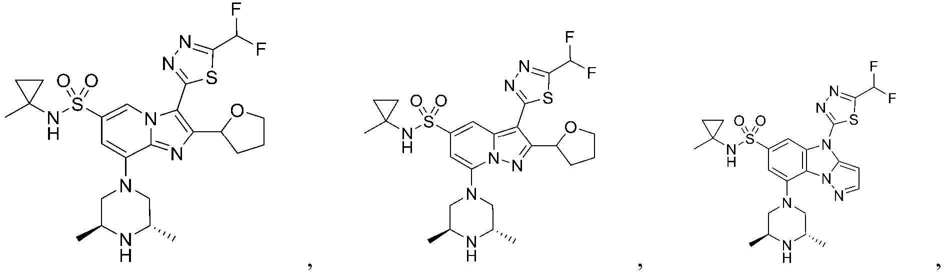

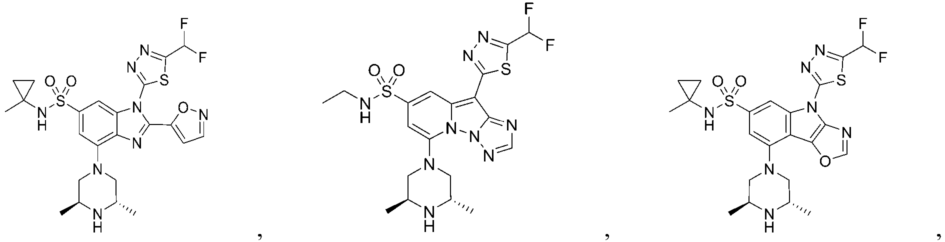

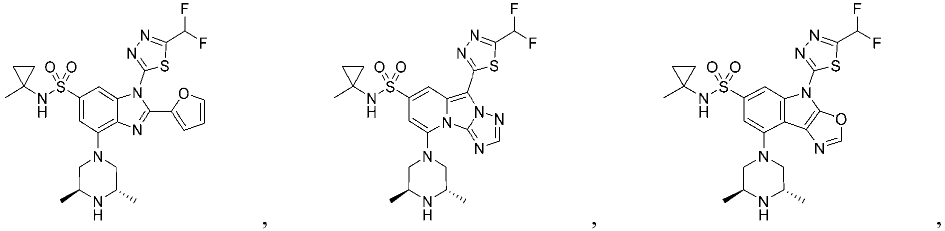

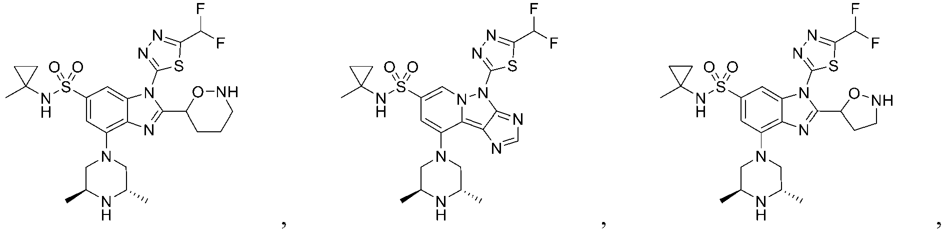

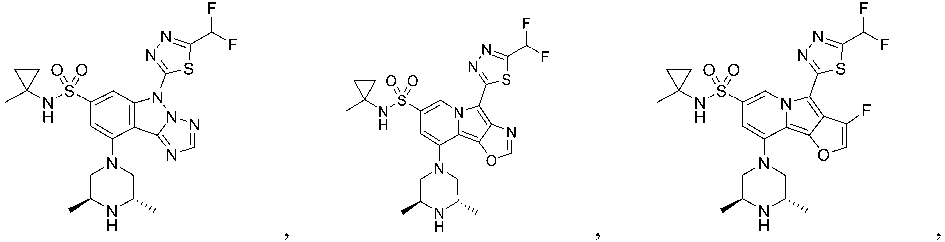

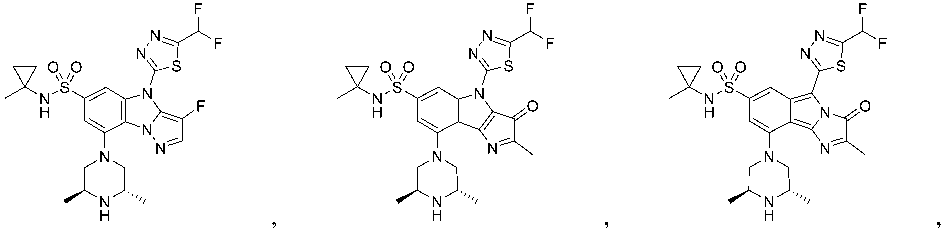

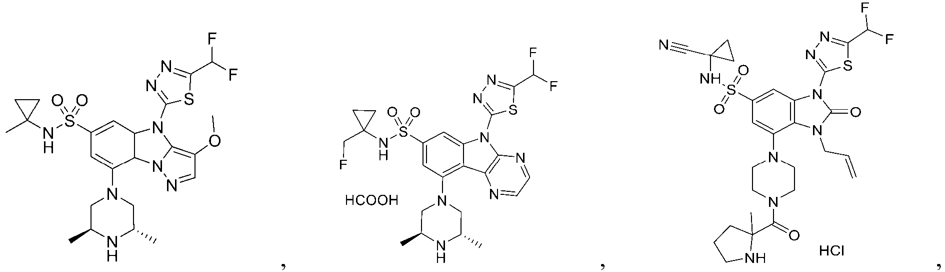

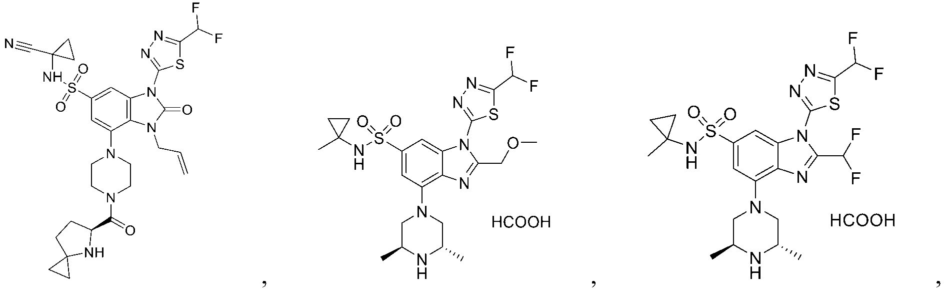

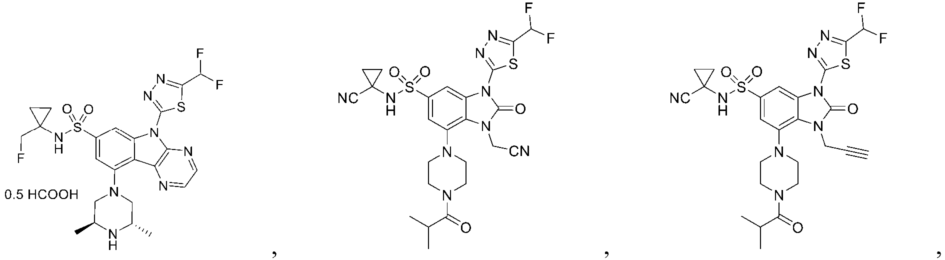

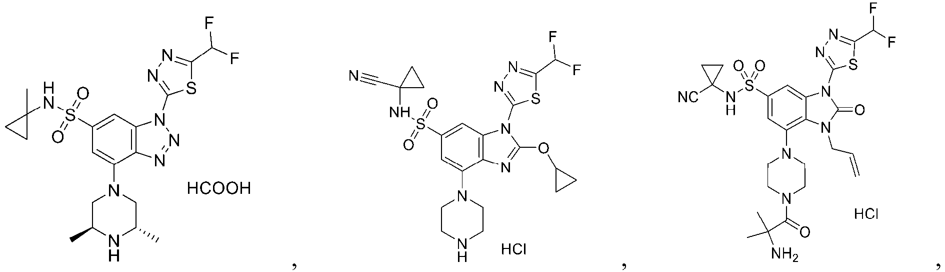

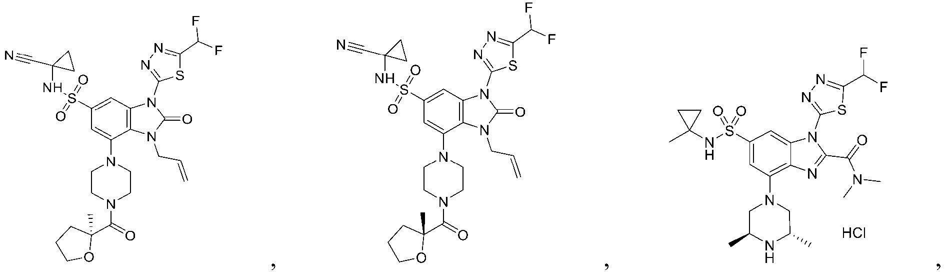

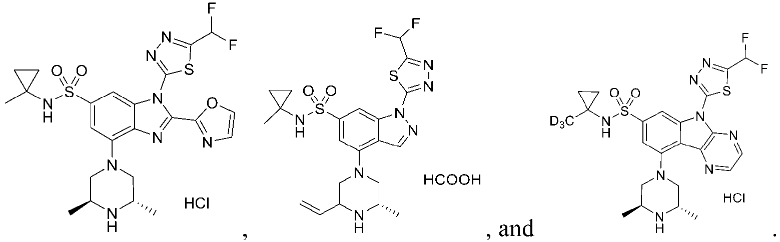

The present disclosure further provides the following compounds, and pharmaceutically acceptable salts thereof, or stereoisomers thereof, prodrugs thereof, N-oxides thereof, solvates thereof, or isotopic derivatives thereof, wherein the compound may be selected from the group consisting of any one of the following structures, and pharmaceutically acceptable salts thereof, or stereoisomers thereof, prodrugs thereof, N-oxides thereof, solvates thereof, or isotopic derivatives thereof,

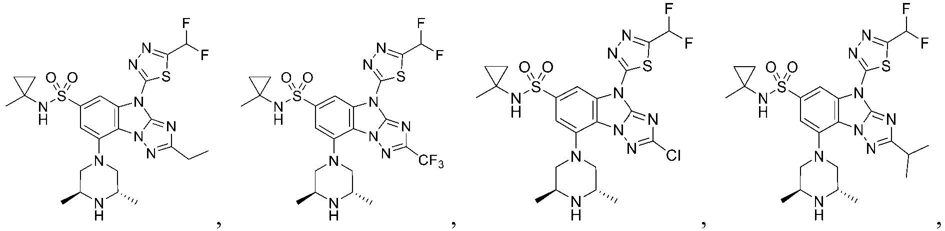

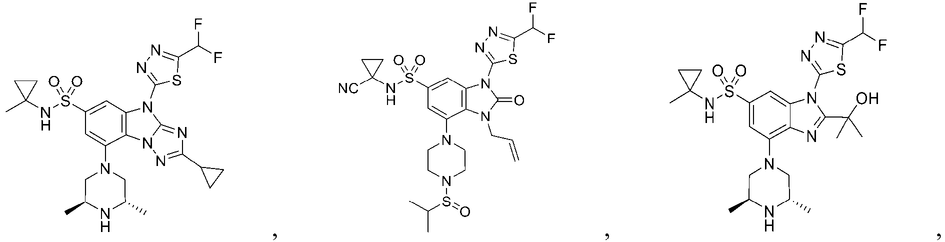

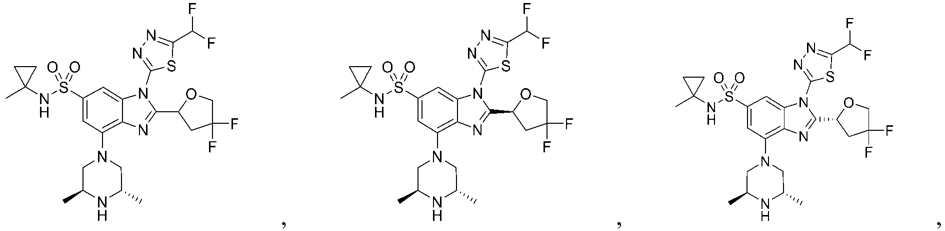

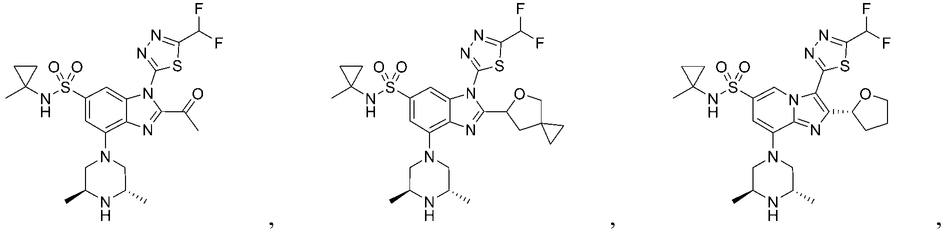

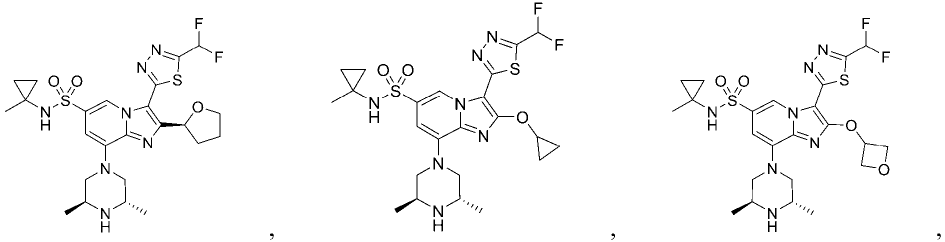

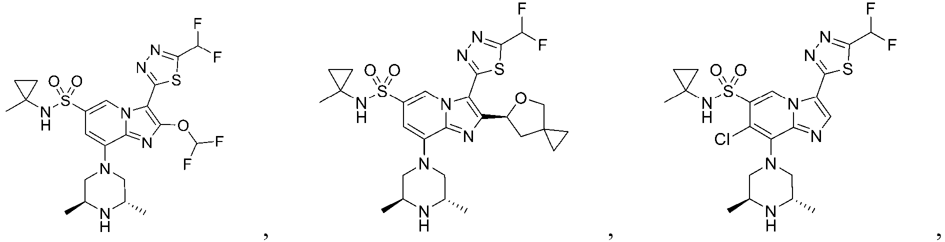

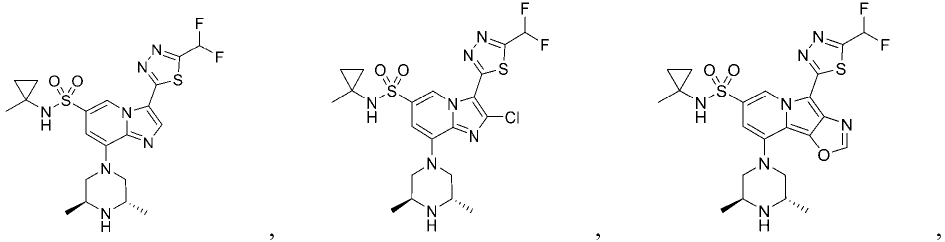

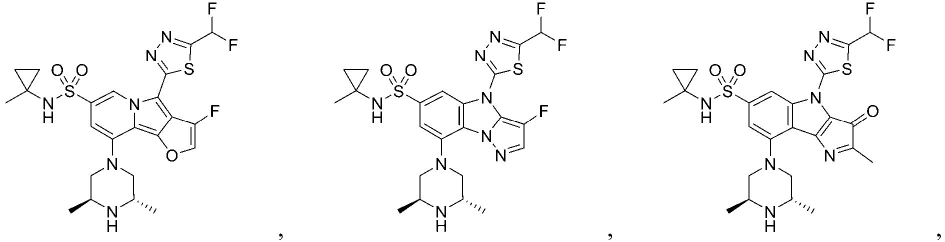

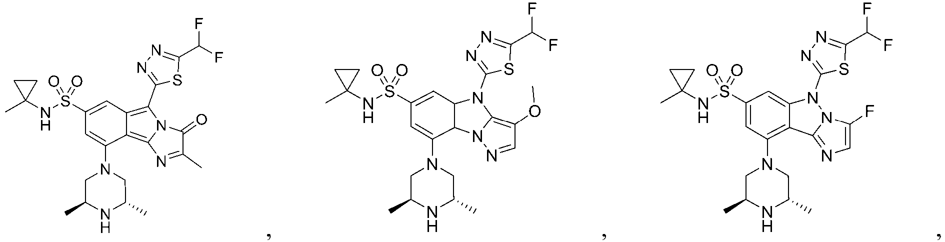

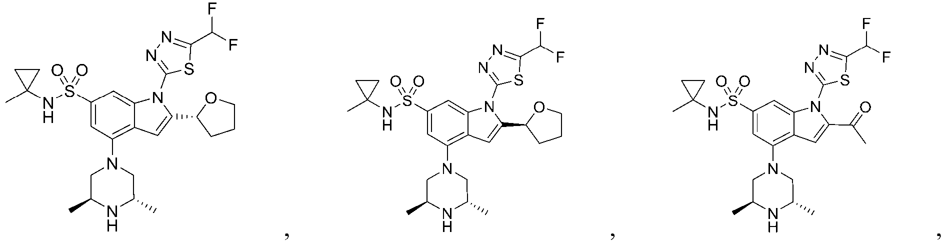

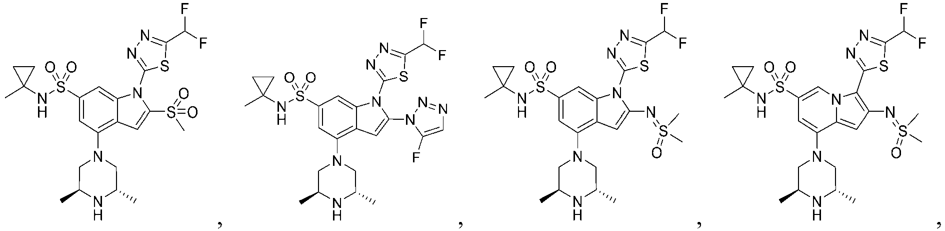

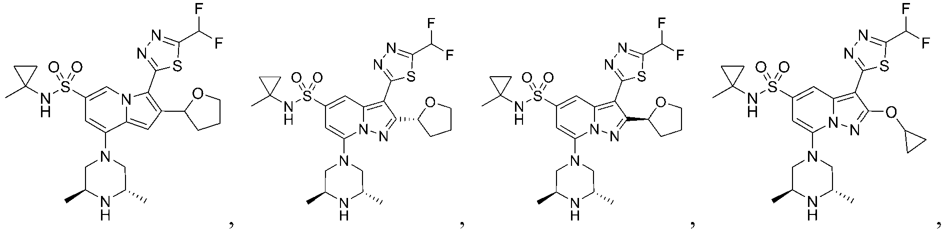

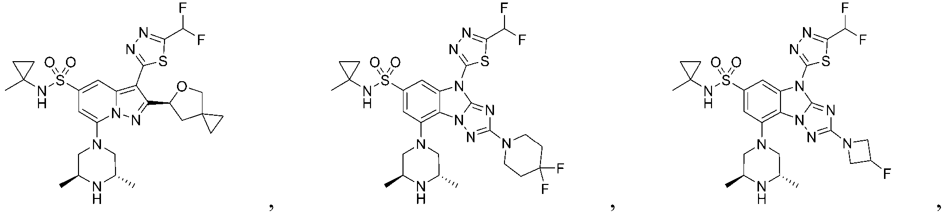

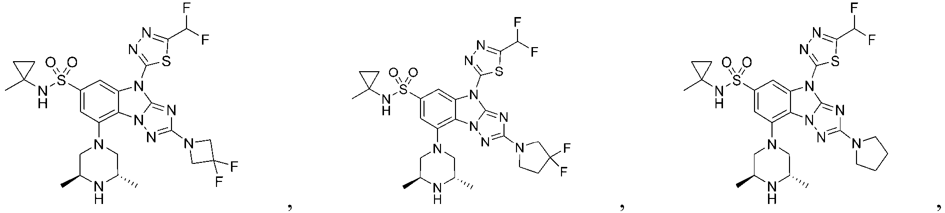

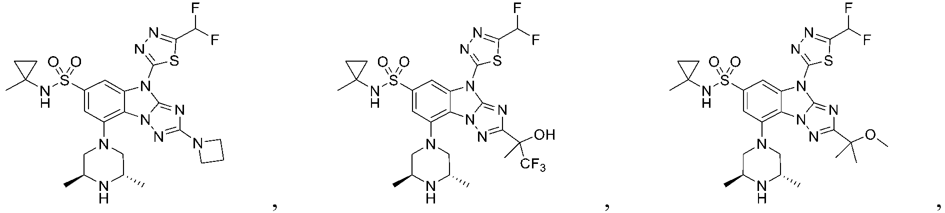

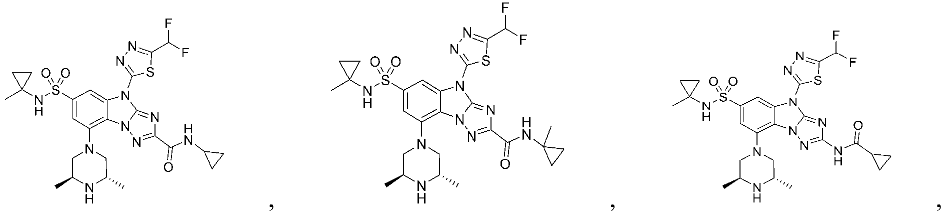

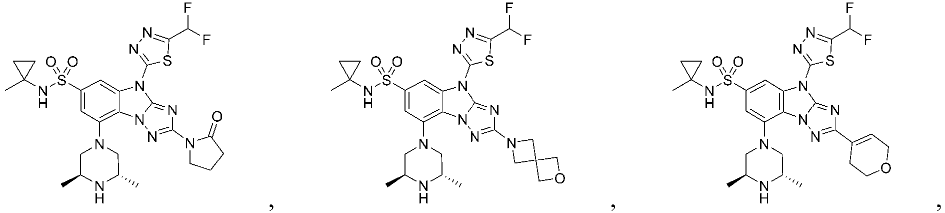

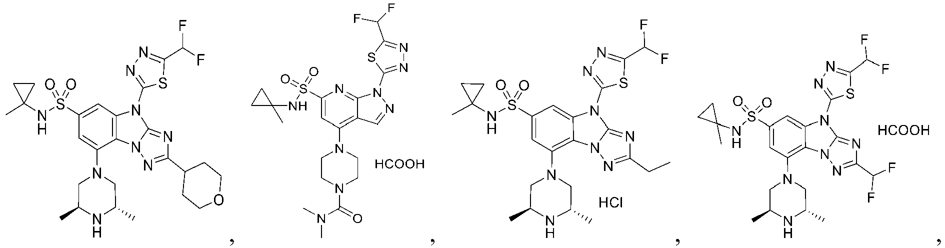

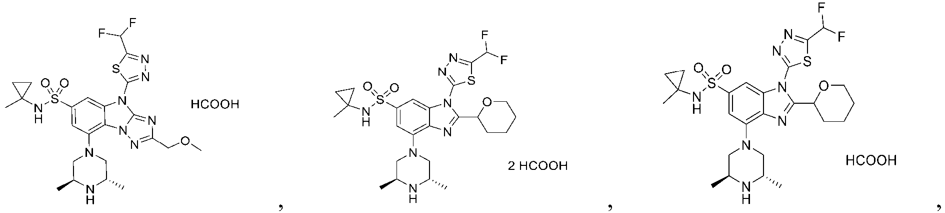

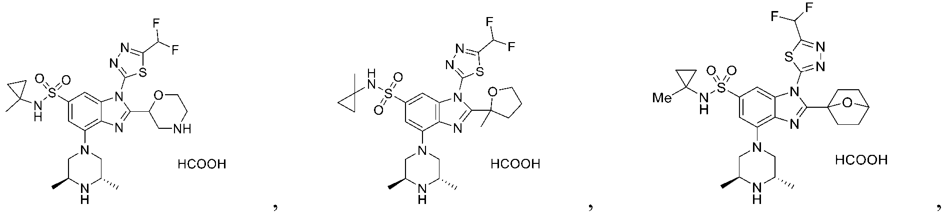

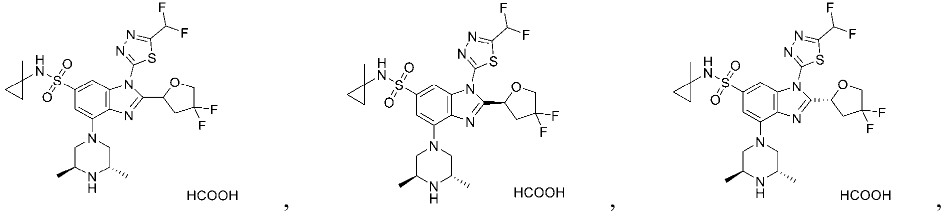

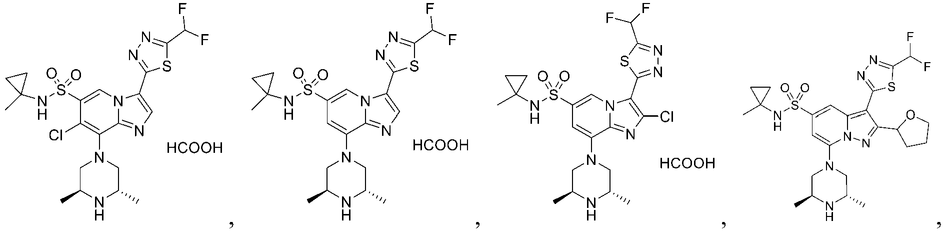

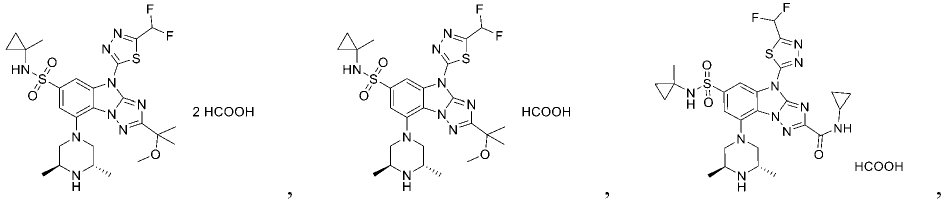

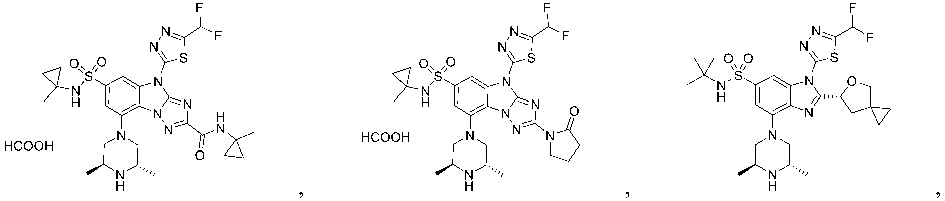

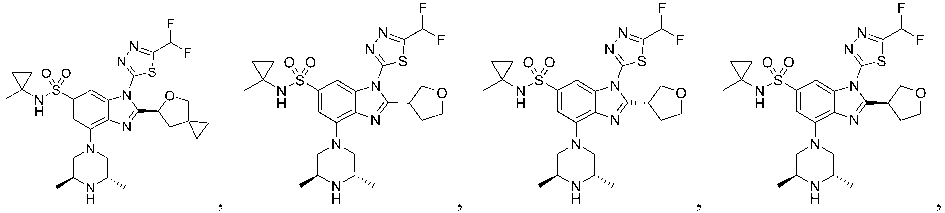

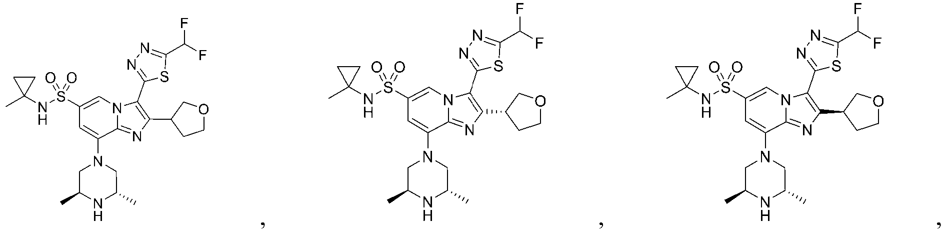

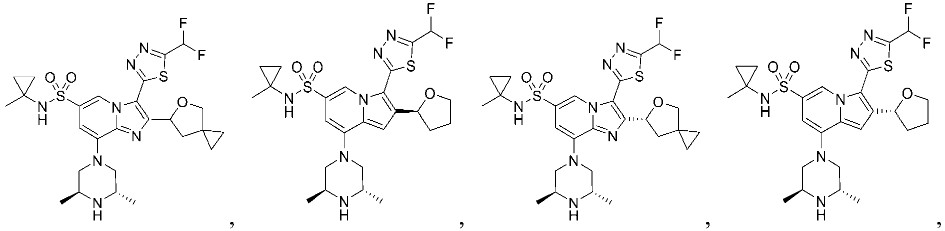

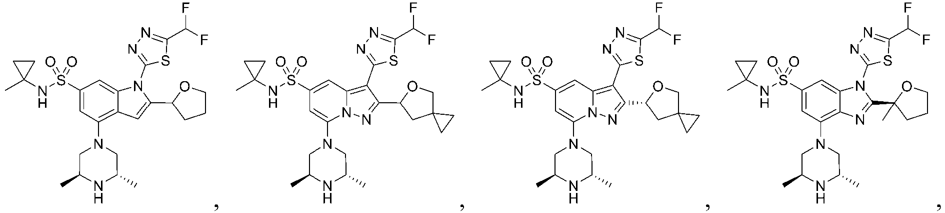

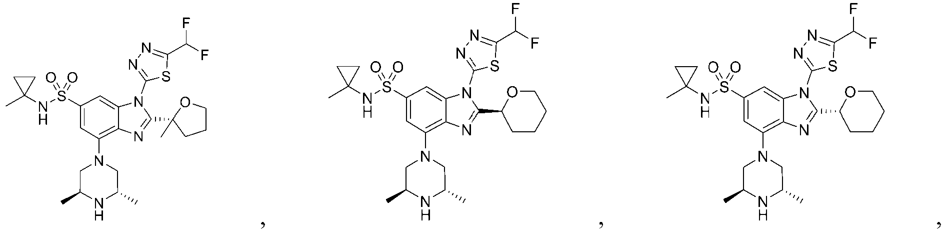

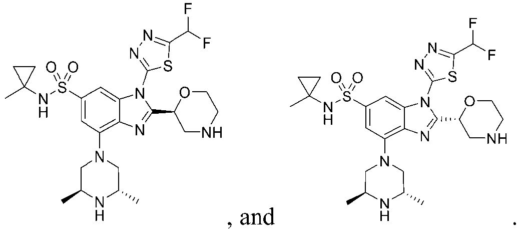

The present disclosure further provides a pharmaceutical composition comprising (preferably a therapeutically effective amount of) the compound described above, or a pharmaceutically acceptable salt thereof, a stereoisomer thereof, a prodrug thereof, an N-oxide thereof, a solvate thereof, or an isotopic derivative thereof, and a pharmaceutically acceptable carrier.

The present disclosure further provides the use of the compound described above, or a pharmaceutically acceptable salt thereof, a stereoisomer thereof, a prodrug thereof, an N-oxide thereof, a solvate thereof, or an isotopic derivative thereof, or the pharmaceutical composition described above, in the manufacture of a medicament for the treatment of PARG-mediated cancer.

The present disclosure further provides the compound described above, or a pharmaceutically acceptable salt thereof, a stereoisomer thereof, a prodrug thereof, an N-oxide thereof, a solvate thereof, or an isotopic derivative thereof, or the pharmaceutical composition described above, for use in the treatment of PARG-mediated cancer.

The present disclosure further provides a method of treating PARG-mediated cancer, comprising administering to a patient a therapeutically effective amount of the compound described above, or a pharmaceutically acceptable salt thereof, a stereoisomer thereof, a prodrug thereof, an N-oxide thereof, a solvate thereof, or an isotopic derivative thereof, or the pharmaceutical composition described above.

Preferably, the cancer according to the present disclosure is selected from the group consisting of ovarian cancer, pancreatic cancer, breast cancer, and prostate cancer.

### Technical effect

1. The binding conformation (active conformation) of the compounds of the present disclosure within the PARG protein structure highly overlaps with their lowest energy conformation (low-energy conformation), and the energy difference between the two conformations is minimal. Therefore, in actual binding to the PARG enzyme, the compounds of the present disclosure are expected to exhibit binding activity similar to or superior to that of the compound in Reference Example 1.
2. The compounds of the present disclosure exhibit strong inhibitory activity against the PARG enzyme.

### Description and definition

Unless otherwise specified, the following terms and phrases used herein have the following meanings. A specific term or phrase should not be considered indefinite or unclear in the absence of a particular definition, but should be understood in the ordinary sense.

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction, or other problems or complications, and is commensurate with a reasonable benefit/risk ratio.

The term "pharmaceutically acceptable salt" refers to a derivative prepared by the compound of the present disclosure with a relatively nontoxic acid or base. These salts may be prepared during the synthesis, separation, and purification of the compound, or the free form of the purified compound may be used alone to react with a suitable acid or base. When the compound contains a relatively acidic functional group, the compound reacts with alkali metal, alkaline earth metal hydroxide, or organic amine to obtain an alkali addition salt, including cations based on alkali metals and alkaline earth metals, as well as non-toxic ammonium, quaternary ammonium, and amine cations. Salts of amino acids are also encompassed. When the compound contains a relatively basic functional group, the compound reacts with an organic acid or an inorganic acid to obtain an acid addition salt, such as a hydrochloride salt and a formate salt.

The term "pharmaceutically acceptable carrier" refers to a medium generally acceptable in the art for delivering a biologically active agent to animals, particularly mammals, including, for example, an adjuvant, an excipient, or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricant, and a dispersant, depending on the route of administration and the nature of dosage forms. The pharmaceutically acceptable carrier is formulated according to a number of factors that are within the purview of those skill in the art. The factors include, but are not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and binders) are well known to those of ordinary skill in the art.

The term "prodrug" refers to certain derivatives of the compound of the present disclosure that themselves possess little or no pharmacological activity, which contain a cleavable group and are converted into the compound of the present disclosure through solvolysis or decomposition under physiological conditions. Types of prodrugs include, but are not limited to, amides, esters, anhydrides, and salts. The term "ester" refers to a derivative formed with a suitable alcohol when the compound of the present disclosure contains an acidic group (e.g., carboxylic acid); or a derivative formed with a suitable acid (including an organic acid or an inorganic acid) when the compound of the present disclosure contains a hydroxyl group. Preparation methods of prodrugs are well known to those skilled in the art.

The term "solvate" refers to an association or complex of one or more solvent molecules with the compound of the present disclosure. The term "hydrate" may be used when the solvent is water. The solvent molecules may be stoichiometric or non-stoichiometric.

The term "nitrogen oxide" or "N-oxide" refers to a derivative formed by further oxidation of the nitrogen atom in a nitrogen-containing group. Common N-oxides include N-oxides of tertiary amines or N-oxides of nitrogen atoms in nitrogen-containing heterocycles. Synthetic methods of N-oxides are well known to those skilled in the art, including oxidation of heterocycles and tertiary amines using peroxyacids such as peracetic acid and *m-*chloroperoxybenzoic acid, hydrogen peroxide, alkyl hydroperoxides such as tert-butyl hydroperoxide, sodium perborate, and dioxiranes such as dimethyldioxirane.

The term "prophylactically or therapeutically effective amount" refers to an amount of the compound of the present disclosure, or a pharmaceutically acceptable salt thereof, a stereoisomer thereof, a prodrug thereof, an N-oxide thereof, a solvate thereof, or an isotopic derivative thereof, that is sufficient to provide a reasonable benefit/risk ratio for the treatment and/or prevention of a disorder in any medical therapy. However, it should be recognized that the total daily dosage of the compound of formula (I) of the present disclosure, or a pharmaceutically acceptable salt thereof, a stereoisomer thereof, a prodrug thereof, an N-oxide thereof, a solvate thereof, or an isotopic derivative thereof, and a composition thereof, should be determined by the attending physician within the scope of sound medical judgment. For any particular patient, the specific therapeutically effective dosage level depends on a variety of factors, including a disorder being treated and the severity of the disorder; activity of a specific compound used; a specific composition used; age, weight, general health status, sex, and diet of the patient; time of administration, route of administration, and excretion rate of the specific compound used; duration of treatment; drugs used in combination with or concurrently with the specific compound used; and similar factors known in the medical field.

The term "optionally" means that an atom may be substituted by a substituent or not. Unless otherwise specified, the type and number of substituents may be arbitrary on the basis of chemical feasibility. For example, the term "optionally substituted by one or more R_{d}" means that an atom may be substituted by one or more R_{d} or may not be substituted by R_{d}.

When any variable (e.g., R_{d}) occurs more than once in the composition or structure of a compound, the definition of the variable at each occurrence is independent. For example, 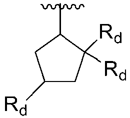 indicates that the cyclopentyl group is substituted by 3 R_{d}, and each R_{d} has an independent option.

When the bond of a substituent may be cross-connected to two atoms on a ring, the substituent may be bonded to any atom on the ring. For example, the structure moiety 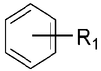 indicates that the substituent R₁ may be substituted at any position on a benzene ring.

When a substituent listed herein does not indicate through which atom it is connected to a compound included but not specifically mentioned in the general formula, such substituent may be bonded through any of its atoms. For example, when pyrazole is used as a substituent, it means that any carbon atom or nitrogen atom on the pyrazole ring is connected to the substituted group. When 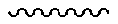 appears in a structure, it indicates that the atom is a bonding atom. For example, 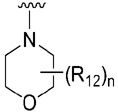 indicates that the nitrogen atom on the morpholine ring is a bonding atom.

Unless otherwise specified, the term "ring" refers to saturated, partially saturated, or unsaturated monocyclic and polycyclic systems, and the term "polycyclic" includes spiro rings, fused rings, or bridged rings. A group derived from a ring by removal of hydrogen atoms is referred to as a "cyclic group", which includes monovalent rings, divalent rings (commonly referred to as ylene rings), trivalent rings, tetravalent rings, *etc*., depending on the number of substituents attached to the ring. In the present disclosure, the term "cyclic group" does not further distinguish the valency of the ring. Representative "rings" include substituted or unsubstituted heterocyclyl, cycloalkyl, heterocycloalkyl, cycloalkenyl, heterocycloalkenyl, cycloalkynyl, heterocycloalkynyl, aryl, or heteroaryl groups. The term "hetero" refers to substituted or unsubstituted heteroatoms and their oxidized forms, also referred to as heteroatom groups. The heteroatoms are generally selected from the group consisting of N, O, S, and P. The oxidized forms generally include NO, P(O), SO, and S(O)₂. The nitrogen atom may be substituted, *i.e*., NR (where R is H or another substituent defined herein). The number of atoms on the ring is typically defined as the number of ring members. For example, "3- to 6-membered heterocycloalkyl" refers to a ring formed by 3 to 6 atoms arranged around, each ring optionally containing 1 to 3 heteroatoms and/or heteroatom groups, *i.e*., N, O, S, NO, SO, S(O)₂, P(O), or NR, and each ring is optionally substituted by an R group as defined herein.

Unless otherwise specified, the term "cycloalkyl" refers to a saturated or unsaturated monocyclic or polycyclic hydrocarbon group. The cycloalkyl group is preferably C₃₋₈ monocyclic cycloalkyl (also referred to as 3- to 8-membered cycloalkyl), more preferably C₃₋₇ monocyclic cycloalkyl (also referred to as 3- to 7-membered cycloalkyl), and even more preferably C₃₋₆ monocyclic cycloalkyl (also referred to as 3- to 6-membered cycloalkyl). Examples of these monocyclic cycloalkyl groups include, but are not limited to, cyclopropyl, cyclobutyl, cyclopentyl, cyclohexyl, cycloheptyl, and cyclooctyl. Unless otherwise specified, the cycloalkyl groups described in the present disclosure include monocyclic cycloalkyl, spiro cycloalkyl, fused cycloalkyl, and bridged cycloalkyl.

Unless otherwise specified, the term "heterocyclyl" refers to a non-aromatic monocyclic or polycyclic ring containing a certain number of heteroatoms and/or heteroatom groups, which may be saturated or partially saturated (e.g., containing one, two, or more double bonds). The heteroatoms and/or heteroatom groups are generally selected from the group consisting of N, O, S, P, NO, SO, S(O)₂, P(O), and NR, wherein the carbon atoms in the heterocycle are optionally oxo-substituted, *i.e*., forming -C(O)-. The "heterocyclyl" group is preferably 3- to 8-membered heterocyclyl or 3- to 8-membered monocyclic heterocyclyl, more preferably 3- to 7-membered heterocyclyl or 3- to 7-membered monocyclic heterocyclyl, and even more preferably 5- to 6-membered heterocyclyl or 5- to 6-membered monocyclic heterocyclyl. Examples of these monocyclic heterocyclyl groups include, but are not limited to, piperidinyl, piperazinyl, morpholinyl, tetrahydropyranyl, 1,4-dioxanyl, pyrrolidinyl, tetrahydrofuranyl, [1,2]oxazolinyl, isoxazolinyl, oxetanyl, azetidinyl, and 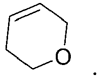 Polycyclic heterocyclyl groups include spiro heterocyclyl, fused heterocyclyl, and bridged heterocyclyl. Unless otherwise specified, the heterocyclyl groups described in the present disclosure include monocyclic heterocyclyl, spiro heterocyclyl, fused heterocyclyl, and bridged heterocyclyl. For example, the 3- to 8-membered heterocyclyl includes 3- to 8-membered spiro heterocyclyl, 3- to 8-membered fused heterocyclyl, and 3- to 8-membered bridged heterocyclyl.

Unless otherwise specified, the term "spiro heterocyclyl" refers to a 5- to 20-membered polycyclic heterocyclic group in which rings share one atom (referred to as a spiro atom), wherein the ring system optionally contains one or more double bonds, and wherein the heteroatoms are selected from the group consisting of N, O, S, P, P(O), NO, SO, and S(O)₂. The spiro heterocyclyl is preferably 5- to 13-membered spiro heterocyclyl, 6- to 12-membered spiro heterocyclyl, 5- to 11-membered spiro heterocycloalkyl, 7- to 11-membered spiro heterocyclyl, or 9- to 11-membered spiro heterocyclyl, such as

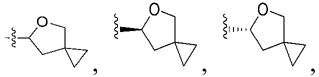

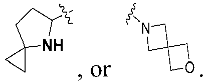

Unless otherwise specified, the term "fused heterocyclyl" refers to a 5- to 20-membered polycyclic heterocyclic group in which rings share a pair of adjacent atoms, wherein one or more rings may contain one or more double bonds, and wherein the heteroatoms are selected from the group consisting of N, O, S, P, P(O), NO, SO, and S(O)₂. The fused heterocyclyl is preferably 6- to 14-membered, more preferably 7- to 10-membered (e.g., 7-membered, 8-membered, 9-membered, or 10-membered), and even more preferably 7- to 10-membered fused heterocyclyl. According to the number of rings involved, it may be classified into bicyclic, tricyclic, tetracyclic, and other polycyclic fused heterocyclyl, which is preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl, such as

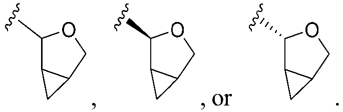

Unless otherwise specified, the term "bridged heterocyclyl" refers to a 5- to 14-membered polycyclic heterocyclic group in which any two rings share two atoms that are not directly connected, wherein the ring system may contain one or more double bonds, and wherein the heteroatom groups are selected from the group consisting of N, O, S, P, P(O), NO, SO, and S(O)₂. The bridged heterocyclyl is preferably 6- to 14-membered, and more preferably 7- to 10-membered (*e.g*., 7-membered, 8-membered, 9-membered, or 10-membered) bridged heterocyclyl. According to the number of rings involved, it may be classified into bicyclic, tricyclic, tetracyclic, and other polycyclic bridged heterocyclyl, which is preferably bicyclic, tricyclic, or tetracyclic bridged heterocyclyl, and more preferably bicyclic bridged heterocyclyl, such as

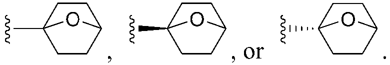

Unless otherwise specified, the term "heteroaryl" refers to a stable monocyclic aromatic hydrocarbon (monocyclic heteroaryl) or polycyclic aromatic hydrocarbon (polycyclic heteroaryl) containing at least one heteroatom or heteroatom group (selected from the group consisting of N, O, S, P, P(O), NO, SO, S(O)₂, and NR). The heteroaryl group is preferably 5- to 10-membered heteroaryl; more preferably 5-membered or 6-membered monocyclic heteroaryl; even more preferably 5-membered or 6-membered monocyclic heteroaryl containing nitrogen or sulfur atoms (also referred to as 5- to 6-membered heteroaryl). Examples of heteroaryl groups include, but are not limited to, 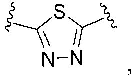 imidazolyl, pyrazolyl, oxazolyl, thiazolyl, isoxazolyl, furyl, and triazolyl. The polycyclic heteroaryl also includes a monocyclic heteroaryl group fused with one or more aryl groups, wherein the point of attachment is on the aromatic ring, and in such cases, the number of ring atoms continues to represent the number of ring atoms in the polycyclic heteroaromatic ring system. The polycyclic heteroaryl also includes a monocyclic heteroaryl group fused with one or more cycloalkyl or heterocyclyl groups, wherein the point of attachment is on the monocyclic heteroaromatic ring, and in such cases, the number of ring atoms continues to represent the number of ring atoms in the polycyclic heteroaromatic ring system.

The term "aryl" refers to a monocyclic all-carbon aromatic ring (*i.e*., monocyclic aryl) or a polycyclic aromatic ring system (*i.e*., polycyclic aryl) having a conjugated π-electron system, which contains 6 to 14 (*e.g*., 6, 7, 8, 9, 10, 11, 12, 13, or 14) ring atoms (*i.e*., 6- to 14-membered aryl). The aryl group is preferably an aryl group having 6 to 10 ring atoms (*i.e*., 6- to 10-membered aryl). The monocyclic aryl is, for example, phenyl. Non-limiting examples of the polycyclic aryl include naphthyl, anthryl, phenanthryl, *etc.* The polycyclic aryl also includes a phenyl group fused with one or more heterocyclyl or cycloalkyl groups, or a naphthyl group fused with one or more heterocyclyl or cycloalkyl groups, wherein the point of attachment is on the phenyl or naphthyl ring, and in such cases, the number of ring atoms continues to represent the number of ring atoms in the polycyclic aromatic ring system. Non-limiting examples include: 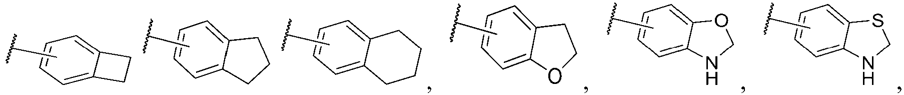 *etc.*

Unless otherwise specified, when a group is not indicated as being substituted by any substituent, it is considered to be unsubstituted.

Unless otherwise specified, the term "alkyl" refers to a linear or branched saturated hydrocarbon group. The alkyl group is preferably C₁₋₆ alkyl, and more preferably C₁₋₄ alkyl. Examples of alkyl groups include, but are not limited to, methyl, ethyl, *n*-propyl, isopropyl, butyl, isobutyl, pentyl, isopentyl, neopentyl, and *n*-hexyl.

Unless otherwise specified, the term "hydroxyalkyl" refers to an alkyl group substituted by one or more hydroxyl groups, preferably an alkyl group substituted by one hydroxyl group (*i.e*., -alkyl-OH). The "hydroxyalkyl" group is preferably C₁₋₆ hydroxyalkyl, and more preferably -C₁₋₆ alkyl-OH.

Unless otherwise specified, the term "alkoxy" refers to an alkyl group connected through an oxygen bridge, *i.e*., a group obtained by replacing the hydrogen atom in a hydroxyl group with an alkyl group. The alkoxy group is preferably C₁₋₆ alkoxy, and more preferably C₁₋₄ alkoxy. Examples of alkoxy groups include, but are not limited to, methoxy, ethoxy, *n-*propoxy, isopropoxy, *n*-butoxy, *sec*-butoxy, *tert*-butoxy, *n*-pentoxy, neopentoxy, and *n-*hexyloxy.

Unless otherwise specified, the term "halogen" refers to a fluorine, chlorine, bromine, or iodine atom.

Unless otherwise specified, the term "haloalkyl" refers to an alkyl group in which one or more hydrogen atoms are substituted by halogen atoms. The haloalkyl is preferably C₁₋₆ haloalkyl, and more preferably C₁₋₄ haloalkyl. Examples of haloalkyl groups include, but are not limited to, monofluoromethyl, difluoromethyl, trifluoromethyl, trichloromethyl, tribromomethyl, 2,2,2-trifluoroethyl, and 2,2,2-trichloroethyl.

Unless otherwise specified, the term "haloalkoxy" refers to an alkoxy group in which one or more hydrogen atoms are substituted by halogen atoms.

Specifically, all combinations of substituents and/or variants thereof described herein are permissible only when such combinations result in stable compounds.

Unless otherwise specified, the term "alkenyl" refers to a linear or branched alkenyl group (or an alkyl group containing at least one carbon-carbon double bond, wherein the alkyl group is as defined above) derived by removal of one hydrogen atom, including "C₂₋₆ alkenyl", "C₂₋₅ alkenyl", "C₂₋₄ alkenyl", and "C₂₋₃ alkenyl". Specific examples include, but are not limited to, -CH=CH₂, -CH=CHCH₃, -C(CH₂)=CH₂, -CH=CHCH₂CH₃, and -CH₂CH=CHCH₃.

Unless otherwise specified, the term "alkynyl" refers to a linear or branched alkynyl group (or an alkyl group containing at least one carbon-carbon triple bond, wherein the alkyl group is as defined above) derived by removal of one hydrogen atom, including "C₂₋₅ alkynyl", "C₂₋₄ alkynyl", and "C₂₋₃ alkynyl". Specific examples include, but are not limited to, -C≡CH, -C≡CHCH₃, CH≡CHCH₂-, and CH=C-C=C-.

The term "hydroxy" refers to -OH.

The term "amino" refers to -NH₂.

The term "cyano" refers to -CN.

The term "oxo" or "oxo group" refers to "=O".

The compounds of the present disclosure may exist in specific stereoisomeric forms. The term "stereoisomer" refers to isomers that have the same structure but differ in the spatial arrangement of their atoms. These include *cis* and *trans* (or Z and *E*) isomers, (-)- and (+)-isomers, (*R*)- and (*S*)-enantiomers, diastereomers, *(D*)- and (*L*)-isomers, tautomers, atropisomers, conformational isomers, and mixtures thereof (*e.g*., racemates and mixtures of diastereomers). Substituents in the compounds of the present disclosure may contain additional asymmetric atoms. All such stereoisomers and mixtures thereof are encompassed within the scope of the present disclosure. Optically active (-)- and (+)-isomers, (*R*)- and (*S*)-enantiomers, and *(D*)- and (*L*)-isomers may be prepared by chiral synthesis, chiral reagents, or other conventional techniques. One isomer of a compound of the present disclosure may be prepared by asymmetric synthesis or using chiral auxiliaries. Alternatively, when the molecule contains a basic functional group (*e.g*., an amino group) or an acidic functional group (*e.g*., a carboxyl group), the compound reacts with an appropriate optically active acid or base to form diastereomeric salt, followed by diastereomeric resolution using conventional methods known in the art to obtain pure isomers. Furthermore, the separation of enantiomers and diastereomers is typically accomplished by chromatography.

In the chemical structures of the compounds of the present disclosure, the bond " 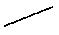 " indicates an unspecified configuration. That is, if chiral isomers are present in the chemical structure, the bond " 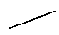 " may be " 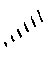 " or " 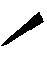 ", or may include both " 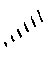 " and " 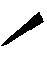 " configurations. For all carbon-carbon double bonds, even if only one configuration is named, both *Z* and *E* configurations are included.

The compounds of the present disclosure include all suitable isotopic derivatives thereof. The term "isotopic derivative" refers to a compound in which at least one atom is replaced by another atom having the same atomic number but a different atomic mass. Examples of isotopes that may be incorporated into the compounds of the present disclosure include stable and radioactive isotopes of hydrogen, carbon, nitrogen, oxygen, phosphorus, sulfur, fluorine, chlorine, bromine, and iodine, such as ²H (deuterium, D), ³H (tritium, T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I, preferably deuterium. Compared to non-deuterated drugs, deuterated drugs offer advantages such as reduced toxic side effects, increased drug stability, enhanced therapeutic efficacy, and prolonged biological half-life. All isotopic variations of the compounds of the present disclosure, whether radioactive or non-radioactive, are encompassed within the scope of the present disclosure. Each available hydrogen atom attached to a carbon atom may be independently replaced by a deuterium atom, and such substitution may be partial or complete. Partial deuterium substitution means that at least one hydrogen atom is replaced by at least one deuterium atom.

When the bond of a substituent may be cross-connected to a ring, it indicates that the substituent may be bonded to any atom on the ring. For example, the structural moiety 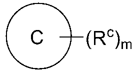 indicates that the substituent R^{c} may be substituted at any position on ring C, and the number of R^{c} is m.

In substituent structures, the dash "-" indicates the point of attachment for the substituent. For example, -CH₃ indicates that the substituent is attached via the carbon atom.

In the examples of the present disclosure, the naming of the title compound is converted from the compound structure by means of ChemDraw. If there is any inconsistency between the compound name and the compound structure, it may be determined by synthesizing relevant information and reaction routes; if it cannot be confirmed by other methods, the given structural formula of the compound shall prevail.

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, and the like may be appropriately adjusted based on the specific reactants employed.

The compounds of the present disclosure may be prepared by a variety of synthetic methods well known to those skilled in the art, including the specific embodiments listed below, the embodiments formed by their combination with other chemical synthesis methods, and equivalent alternatives well known to those skilled in the art. Preferred embodiments include, but are not limited to, the examples of the present disclosure.

### DETAILED DESCRIPTION OF THE PREFERRED EMBODIMENT

The following examples provide a detailed description of the present disclosure, but do not imply any adverse limitation on the present disclosure. The present disclosure has been described in detail herein, and its specific embodiments have also been disclosed. It will be apparent to those skilled in the art that various modifications and improvements may be made to the specific embodiments of the present disclosure without departing from the spirit and scope of the present disclosure.

In the examples of the present disclosure, the naming of the title compound is converted from the compound structure by means of ChemDraw. If there is any inconsistency between the compound name and the compound structure, it may be determined by synthesizing relevant information and reaction routes; if it cannot be confirmed by other methods, the given structural formula of the compound shall prevail. The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, and the like may be appropriately adjusted based on the specific reactants employed. The compounds of the present disclosure may be prepared by a variety of synthetic methods well known to those skilled in the art, including the specific embodiments listed below, the embodiments formed by their combination with other chemical synthesis methods, and equivalent alternatives well known to those skilled in the art. Preferred embodiments include, but are not limited to, the examples of the present disclosure.

### 1. Summary of Experimental Instruments

The structures of the compounds of the present disclosure were determined by nuclear magnetic resonance (NMR) or/and liquid chromatography-mass spectrometry (LC-MS), or ultra-performance liquid chromatography-mass spectrometry (UPLC-MS).

NMR measurements were performed using a Bruker Neo 400M or Bruker Ascend 400 NMR spectrometer with deuterated dimethyl sulfoxide (DMSO-*d*₆), deuterated methanol (CD₃OD), deuterated chloroform (CDCl₃) and deuterium oxide (D₂O) as the solvents and tetramethylsilane (TMS) as the internal standard. NMR chemical shifts (δ) are expressed in parts per million (ppm).

LC-MS analyses were performed using an Agilent 1200 & 6120B single quadrupole mass spectrometer or a Shimadzu LC-20AD XR & MS 2020 single quadrupole mass spectrometer, with electrospray ionization (ESI) as the ion source.

HPLC analyses were performed using a Shimadzu 20AD XR high-performance liquid chromatograph.

Preparative HPLC analyses were performed using Gilson GX281 333/334 Pump or Gilson TRILUTION LC.

Chiral HPLC analyses were performed using Waters Arc with 2998 with QDA.

Supercritical fluid chromatography (SFC) analyses were performed using Waters ACQUITY UPCC with QDA.

The starting materials and intermediates directly used in the present disclosure may be synthesized according to methods known in the art or purchased commercially. Experimental methods for which specific conditions are not indicated in the examples are typically performed under conventional conditions or according to the conditions recommended by the manufacturer of the starting materials or commercial products. Reagents for which specific sources are not indicated are commercially available standard reagents.

The progress of reactions in the examples may be monitored by conventional methods such as thin-layer chromatography (TLC) and LC-MS. The eluent system for column chromatography used in purification and the developing solvent system for TLC may consist of one or more of the following solvents: dichloromethane, methanol, n-hexane, ethyl acetate, petroleum ether, ethyl acetate, acetone, dichloromethane, and the like. The volume ratio of the solvents may be adjusted according to the polarity of the compounds or by adding a small amount of basic or acidic reagents such as triethylamine, acetic acid, or trifluoroacetic acid.

### 2. Synthetic Examples

### Reference Example 1:

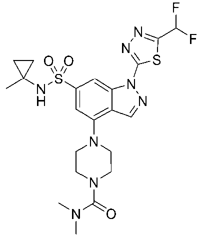

The preparation method refers to patent WO2021055744.

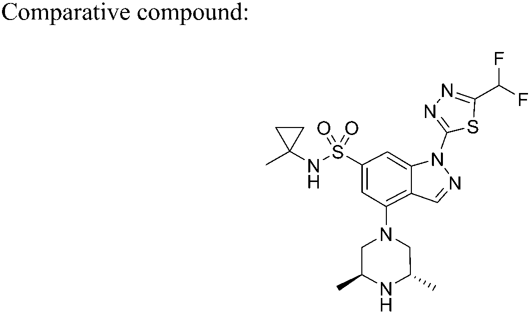

The preparation method refers to the synthesis of compound of formula A in patent WO2023183850A1.

The lowest energy conformation (low-energy conformation) of Reference Example 1 was calculated using Spartan software at the ωB97X-D/6-31G* level of theory. In the low-energy conformation of Reference Example 1, the difluoromethyl-substituted thiazole moiety is coplanar with the benzopyrazole core, and the S atom of the thiazole moiety tends to orient away from the sulfonamide moiety (corresponding to a dihedral angle of 0 degrees in the energy barrier diagram). In the binding conformation of Reference Example 1 with the PARG protein, the dihedral angle between the difluoromethyl-substituted thiazole moiety and the benzopyrazole core is only 1.5 degrees, which is highly consistent with the low-energy conformation of Reference Example 1, fully explaining the high binding activity of Reference Example 1 with the PARG protein as reported in patent WO2021055744. Generally, when the structural difference between the lowest energy conformation (low-energy conformation) of a small molecule and its binding conformation (active conformation) in a protein is minimal, the energy loss during the conversion from the low-energy conformation to the active conformation is also minimal. Consequently, the compound binds more readily to the protein and exhibits higher binding activity.

**The synthetic routes for some examples of the present disclosure are as follows:**

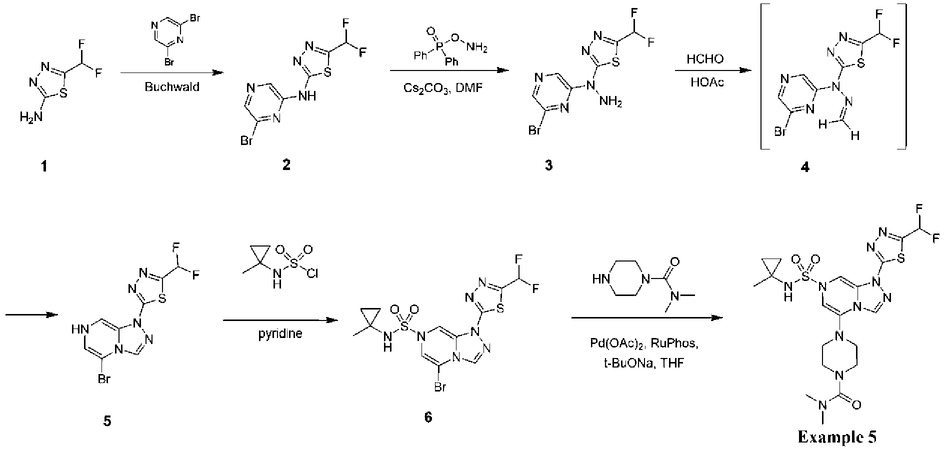

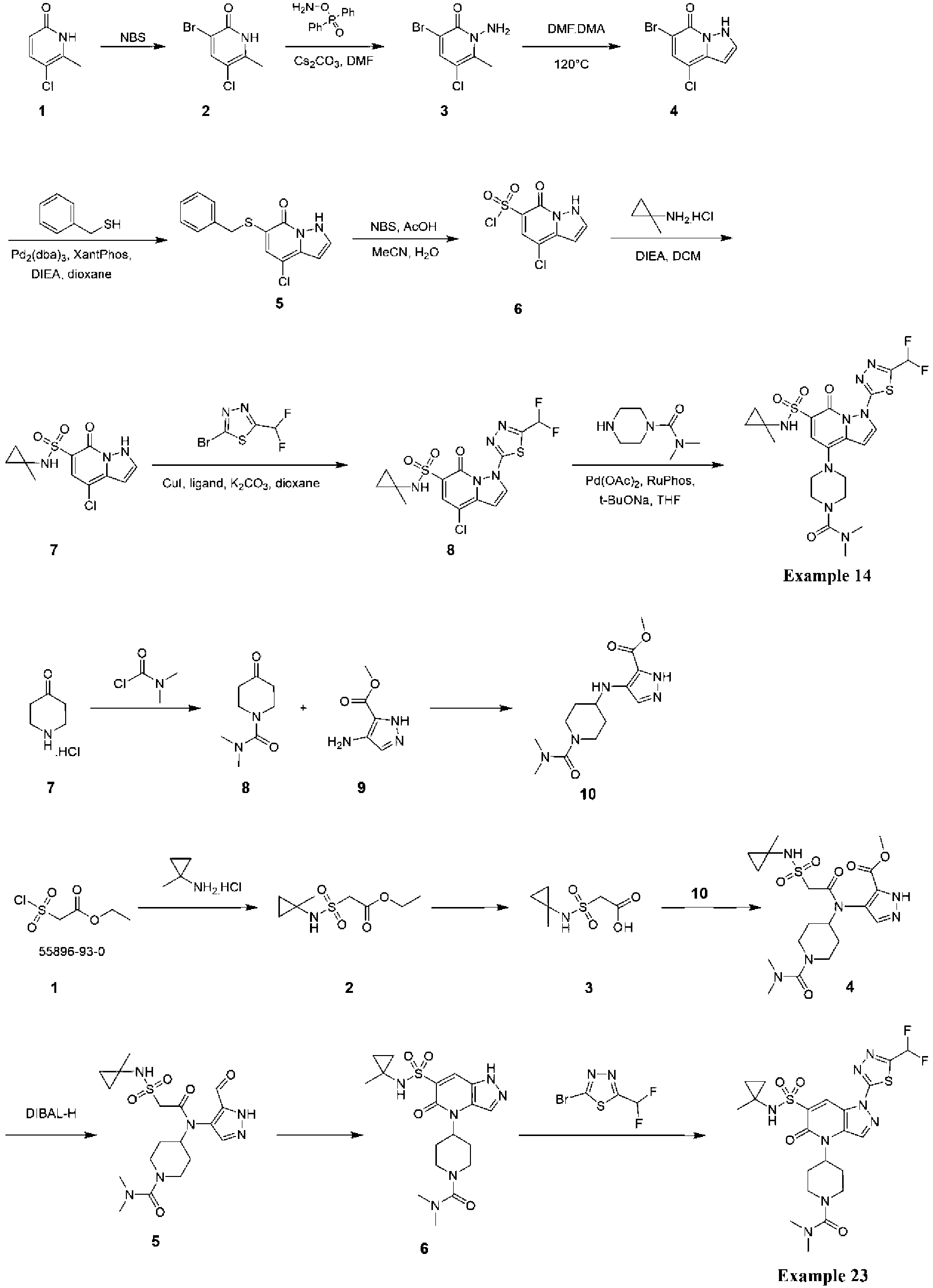

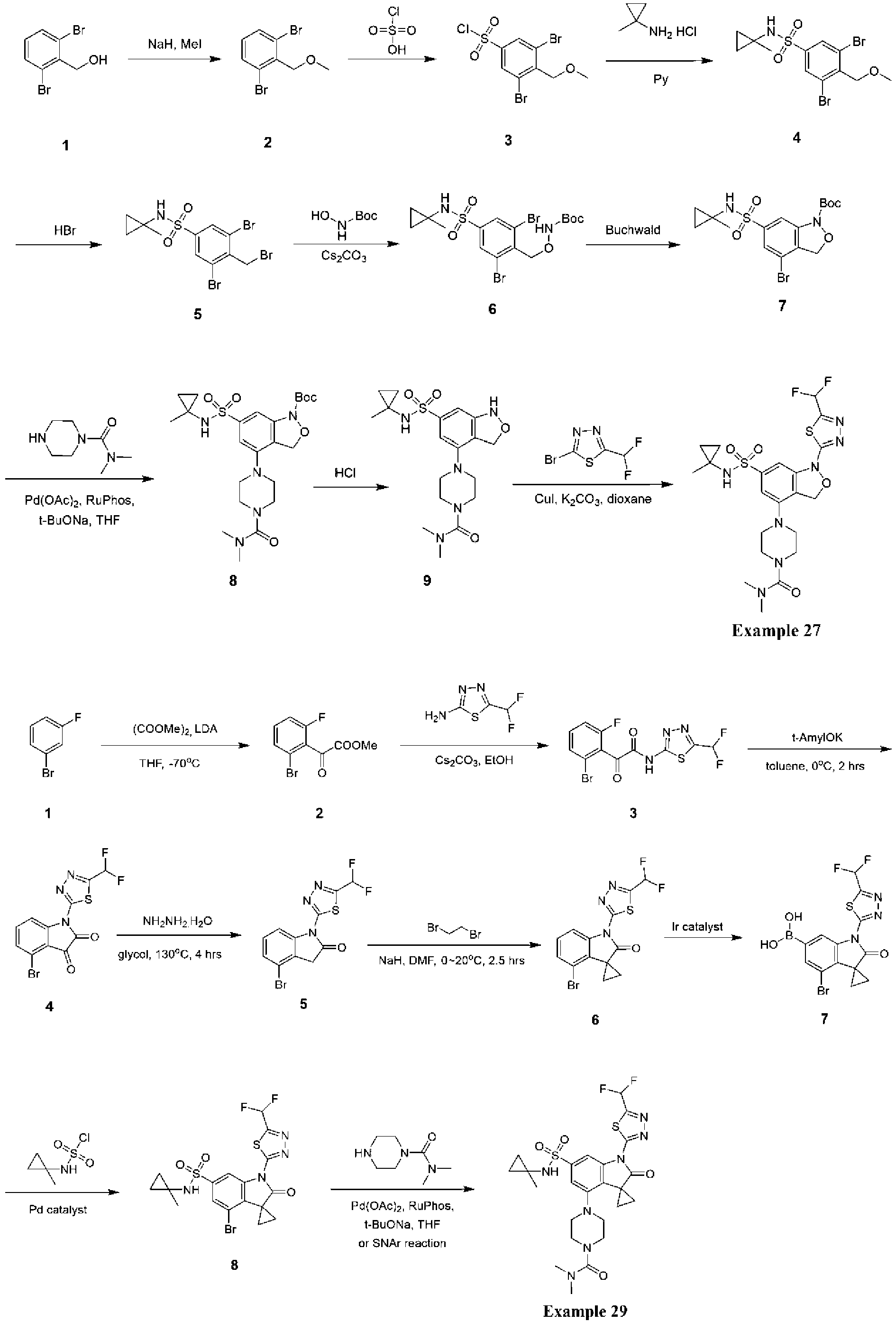

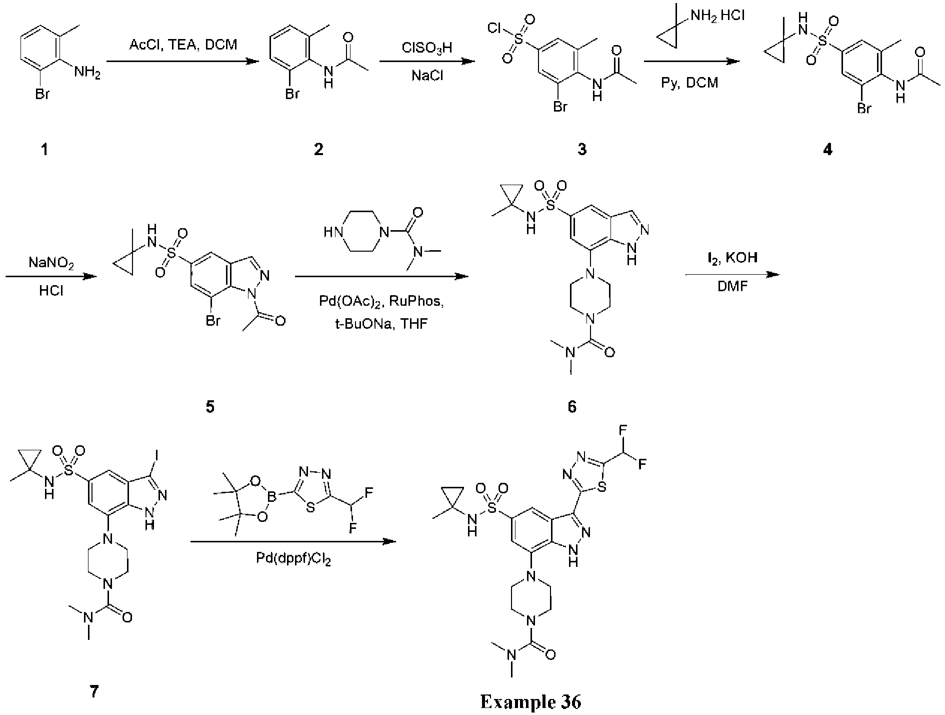

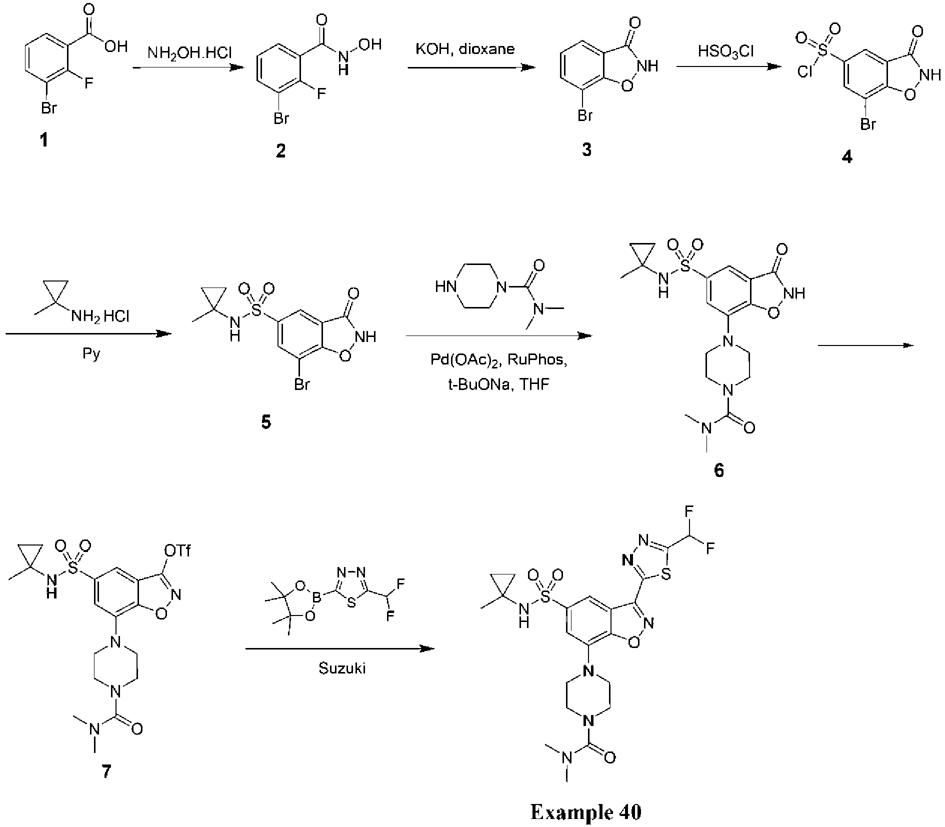

The compounds in other examples listed in Table 1 of the present disclosure, such as Example 1, Example 2, Example 3, Example 4, Example 6, Example 7, Example 8, Example 9, Example 10, Example 11, Example 12, Example 13, Example 15, Example 16, Example 17, Example 18, Example 19, Example 20, Example 21, Example 22, Example 24, Example 25, Example 26, Example 28, Example 30, Example 31, Example 32, Example 33, Example 34, Example 35, Example 37, Example 38, Example 39, Example 41, Example 42, Example 43, and other described compounds, were prepared with reference to any one of the above synthetic procedures.

### Specific Synthetic Examples:

### Example A001 and Example A002

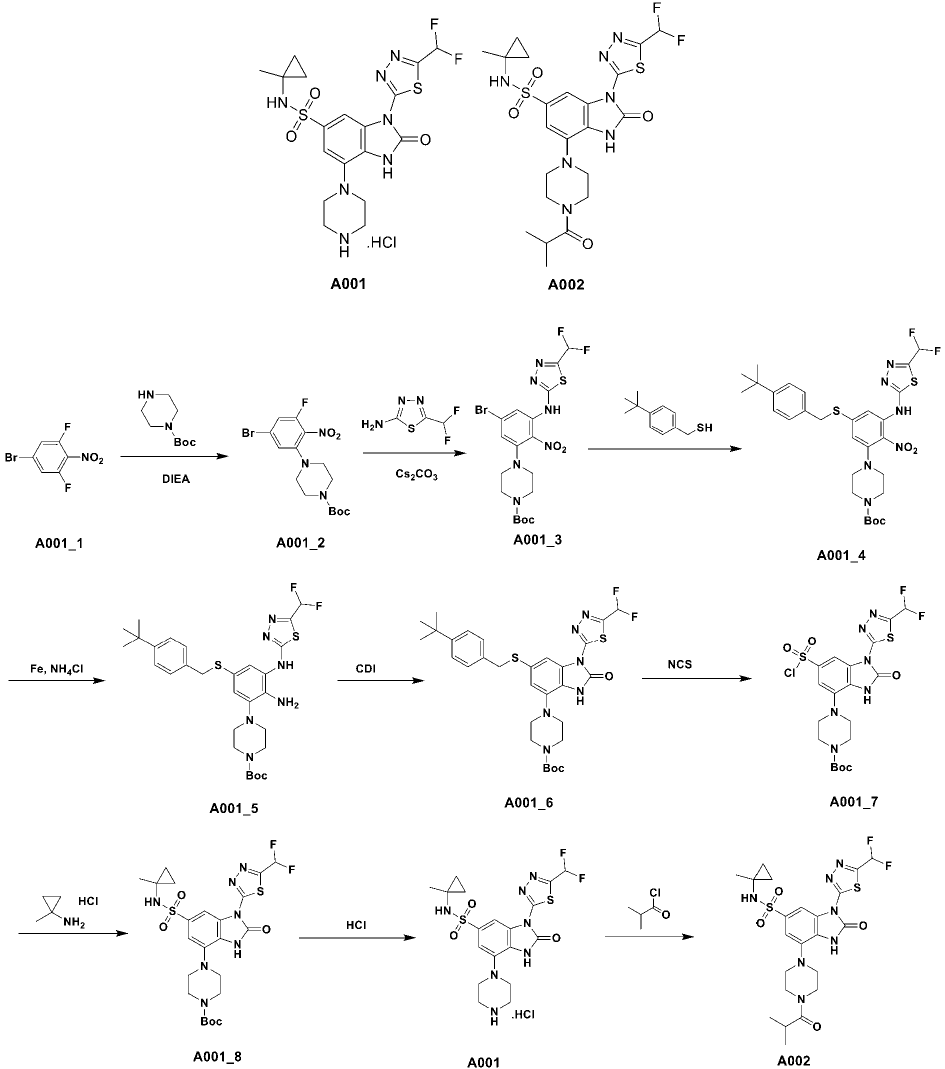

### Step 1: Synthesis of compound A001_2

005_1 (10.0 g, 42.0 mmol, 1.00 eq) and N-Boc piperazine (7.83 g, 42.0 mmol) were dissolved in N,N-dimethylacetamide (100 mL), followed by addition of N,N-diisopropylethylamine (27.1 g, 210 mmol). The reaction mixture was heated to 110°C and stirred for 16 hours. After completion of the reaction, the reaction mixture was cooled to room temperature, quenched with water (600 mL), and extracted with ethyl acetate (100 mL × 3). The organic phases were combined, washed with brine (100 mL × 3), dried over anhydrous sodium sulfate, filtered, and the filtrate was concentrated by rotary evaporation. The residue was purified by column chromatography to obtain A001_2 (16.4 g).

MS (ESI) M/Z: 348.1, 350.2 [M-55]⁺.

¹H NMR (400 MHz, CDCl₃-*d*): δ 7.10 - 7.07 (m, 1H), 7.04 - 7.03 (m, 1H), 3.53 (t, *J* = 4.8 Hz, 4H), 3.01 (t, *J =* 4.8 Hz, 4H), 1.48 (s, 9H).

### Step 2: Synthesis of compound A001_3

Cesium carbonate (19.3 g, 59.3 mmol) and 5-(difluoromethyl)-1,3,4-thiadiazol-2-amine (4.49 g, 29.6 mmol) were added to a solution of A001_2 (8.00 g, 19.8 mmol) in N,N-dimethylacetamide (80 mL). The reaction mixture was heated to 110°C under nitrogen atmosphere and stirred for 16 hours. After completion of the reaction, the reaction mixture was cooled to room temperature, quenched with water (500 mL), and extracted with ethyl acetate (100 mL × 3). The organic phases were combined, washed with brine (100 mL × 3), dried over anhydrous sodium sulfate, filtered, and the filtrate was concentrated by rotary evaporation. The residue was purified by column chromatography to obtain A001_3 (6.7 g).

MS (ESI) M/Z: 533.1, 535.1 [M-H]⁻.

### Step 3: Synthesis of compound A001_4

A001_3 (5.70 g, 10.6 mmol), 4*-tert*-butylbenzyl mercaptan (2.11 g, 11.7 mmol), and N,N-diisopropylethylamine (3.44 g, 26.6 mmol) were dissolved in 1,4-dioxane (60 mL). The system was purged with nitrogen three times, followed by addition of Pd₂(dba)₃ (974 mg, 1.06 mmol) and Xantphos (1.23 g, 2.13 mmol, 0.20 eq), and purged with nitrogen three additional times. The reaction mixture was heated to 70°C and stirred for 1 hour. After completion of the reaction, the reaction mixture was cooled to room temperature, quenched with water (200 mL), and extracted with ethyl acetate (50 mL × 3). The organic phases were combined, washed with brine (100 mL × 1), dried over anhydrous sodium sulfate, filtered, and the filtrate was concentrated by rotary evaporation. The residue was purified by column chromatography to obtain A001_4 (4.75 g).

MS (ESI) M/Z: 633.2 [M-H]⁻.

¹H NMR (400 MHz, CDCl₃-*d*): δ 7.75 (s, 1H), 7.37 - 7.27 (m, 4H), 6.91 (t, *J =* 53.6 Hz, 1H), 6.61 (s, 1H), 4.22 (s, 2H), 3.53 (t, *J =* 4.8 Hz, 4H), 2.94 (t, *J =* 4.8 Hz, 4H), 1.48 (s, 9H), 1.31 (s, 9H).

### Step 4: Synthesis of compound A001_5

A001_4 (4.20 g, 6.62 mmol) was dissolved in ethanol (30 mL) and water (10 mL), followed by addition of iron powder (1.85 g, 33.0 mmol) and ammonium chloride (1.77 g, 33.0 mmol). The reaction mixture was heated to 80°C and stirred for 1.5 hours. After completion of the reaction, the reaction mixture was cooled to room temperature and filtered through diatomite. The filtrate was diluted with water (100 mL) and extracted with ethyl acetate (50 mL × 3). The organic phases were combined, washed with brine (50 mL × 1), dried over anhydrous sodium sulfate, filtered, and the filtrate was concentrated by rotary evaporation. The residue was purified by column chromatography to obtain A001_5 (2.4 g).

MS (ESI) M/Z: 603.3 [M-H]⁻.

¹H NMR (400 MHz, DMSO-*d₆*): δ 9.81 (s, 1H), 7.49 - 7.18 (m, 4H), 7.11 - 7.09 (m 2H), 6.55 (d, *J* = 2.0 Hz, 1H), 4.91 (s, 2H), 3.93 (s, 2H), 3.52 - 3.38 (m, 4H), 2.68 - 2.55 (m, 4H), 1.41 (s, 9H), 1.23 (s, 9H).

### Step 5: Synthesis of compound A001_6

A001_5 (430 mg, 711 µmol) was dissolved in tetrahydrofuran (10 mL), followed by addition of carbonyldiimidazole (173 mg, 1.07 mmol, 1.50 eq). The reaction mixture was heated to 70°C and stirred for 1 hour. After completion of the reaction, the reaction mixture was cooled to room temperature and directly concentrated under reduced pressure to obtain a crude product. The crude product was triturated with methyl *tert*-butyl ether (5 mL) at room temperature, filtered, and the filter cake was collected to obtain A001_6 (360 mg).

MS (ESI) M/Z: 629.2 [M-H]⁻.

¹H NMR (400 MHz, DMSO-*d₆*): δ 12.11 (s, 1H), 8.10 (s, 1H), 7.61 (t, *J =* 53.2 Hz, 1H), 7.29 (d, *J* = 8.4 Hz, 2H), 7.19 (d, *J* = 8.0 Hz, 2H), 7.03 (s, 1H), 6.66 (s, 1H), 4.13 (s, 2H), 3.50 - 3.48 (m, 4H), 2.79 - 2.77 (m, 4H), 1.43 (s, 9H), 1.23 (s, 9H).

### Step 6: Synthesis of compound A001_7

A001_6 (250 mg, 396 µmol) was dissolved in acetic acid (2.5 mL), water (1.25 mL), and dichloromethane (2.5 mL). The mixture was cooled to 0°C in an ice-salt bath, and N-chlorosuccinimide (159 mg, 1.19 mmol, 3.00 eq) was added in portions. After the addition was completed, the reaction mixture was slowly warmed to 20°C and stirred for 4 hours. After completion of the reaction, the reaction mixture was cooled to 0°C, then slowly added with saturated sodium bicarbonate aqueous solution to adjust the pH to approximately 5, and extracted with dichloromethane (5 mL × 3). The organic phases were combined, washed with brine (10 mL × 1), dried over anhydrous sodium sulfate, filtered, and the filtrate was concentrated by rotary evaporation to obtain A001_7 (220 mg) as a crude product, which was directly used in the next step.

MS (ESI) M/Z: 549.2, 551.4 [M-H]⁻.

### Step 7: Synthesis of compound A001_8

1-Methylcyclopropanamine hydrochloride (141 mg, 1.31 mmol) and N,N-diisopropylethylamine (253 mg, 1.96 mmol) were dissolved in dichloromethane (0.9 mL), followed by addition of a solution ofA001_7 (180 mg, 327 µmol) in dichloromethane (0.9 mL). The reaction mixture was stirred at 20°C for 1 hour. After completion of the reaction, the reaction mixture was quenched with water (5 mL) and extracted with ethyl acetate (5 mL × 3). The organic phases were combined, washed with brine (5 mL × 1), dried over anhydrous sodium sulfate, filtered, and the filtrate was concentrated by rotary evaporation. The residue was purified by column chromatography to obtain A001_8 (110 mg).

MS (ESI) M/Z: 584.2 [M-H]⁻.

¹H NMR (400 MHz, DMSO-*d₆*): δ 12.44 (s, 1H), 8.52 (d, *J* = 1.2 Hz, 1H), 8.08 (s, 1H), 7.63 (t, *J* = 53.2 Hz, 1H), 7.37 (d, *J* = 1.2 Hz, 1H), 3.58 - 3.56 (m, 4H), 2.98 - 2.96 (m, 4H), 1.44 (s, 9H), 1.07 (s, 3H), 0.64 - 0.62 (m, 2H), 0.39 - 0.36 (m, 2H).

### Step 8: Synthesis of compound A001

A001_8 (60 mg, 102 µmol) was dissolved in ethyl acetate (1 mL), followed by addition of 4 M HCl in methanol (2 mL). The reaction mixture was stirred at 20°C for 1 hour. After completion of the reaction, the reaction mixture was directly concentrated under reduced pressure to obtain a crude product (50 mg, hydrochloride salt). 20 mg of the crude product was purified by preparative HPLC (acidic conditions, chromatographic column: Phenomenex Luna C18) to obtain A001 (11.81 mg, hydrochloride salt).

MS (ESI) M/Z: 486.0 [M+H]⁺.

¹H NMR (400 MHz, DMSO-*d₆*): δ 12.51 (s, 1H), 9.20 - 8.96 (m, 2H), 8.55 (d, *J =* 1.2 Hz, 1H), 8.17 (s, 1H), 7.64 (t, *J =* 53.2 Hz, 1H), 7.39 (s, 1H), 3.48 - 3.42 (m, 4H), 3.42 - 3.38 (m, 4H), 1.07 (s, 3H), 0.64 - 0.62 (m, 2H), 0.39 - 0.37 (m, 2H).

### Step 9: Synthesis of compound A002

A001 (50 mg, 95.8 µmol, hydrochloride salt) and triethylamine (48.5 mg, 479 µmol) were dissolved in N,N-dimethylformamide (0.5 mL), followed by addition of 2-methylpropanoyl chloride (12.3 mg, 115 µmol). The reaction mixture was stirred at 20°C for 1 hour. After completion of the reaction, the reaction mixture was quenched with water (0.1 mL) and purified by preparative HPLC (neutral conditions, chromatographic column: Waters Xbridge Prep OBD C18) to obtain A002 (27.2 mg).

MS (ESI) M/Z: 556.2 [M+H]⁺.

¹H NMR (400 MHz, DMSO-*d₆*): δ 12.48 (s, 1H), 8.52 (d, *J* = 1.2 Hz, 1H), 8.07 (s, 1H), 7.62 (t, *J* = 53.2 Hz, 1H), 7.35 (d, *J* = 1.2 Hz, 1H), 3.74 - 3.70 (m, 4H), 3.02 - 2.91 (m, 5H), 1.05 - 1.03 (m, 9H), 0.64 - 0.61 (m, 2H), 0.38 - 0.35 (m, 2H).

### Example A003

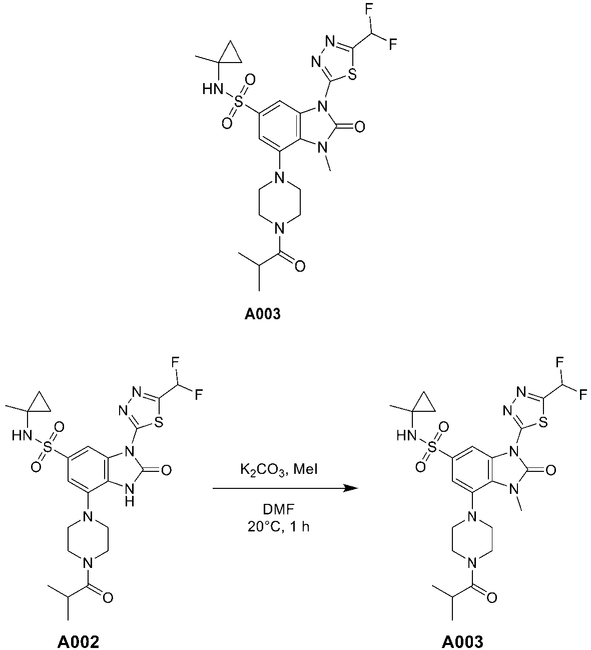

### Step 1: Synthesis of A003

A002 (15 mg, 27.0 µmol) and potassium carbonate (7.46 mg, 54.0 µmol) were dissolved in N,N-dimethylformamide (0.2 mL). Iodomethane (5.75 mg, 40.5 µmol) was added in one portion at 20°C. The reaction mixture was stirred at 20°C for 1 hour. After completion of the reaction, the reaction mixture was quenched with water (2 mL) and extracted with ethyl acetate (2 mL × 3). The organic phases were combined, washed with brine (2 mL × 1), dried over anhydrous sodium sulfate, filtered, and the filtrate was concentrated by rotary evaporation. The crude product was purified by preparative HPLC (neutral conditions, column: Waters Xbridge Prep OBD C18) to obtain A003 (1.2 mg).

MS (ESI) M/Z: 570.2 [M+H]⁺.

¹H NMR (400 MHz, DMSO-*d₆*): δ 8.66 (d, *J* = 1.6 Hz, 1H), 8.13 (s, 1H), 7.77 - 7.50 (m, 2H), 4.52 - 4.50 (m, 1H), 4.07 - 4.06 (m, 1H), 3.80 (s, 3H), 3.45 - 3.42 (m, 1H), 3.18 - 3.16 (m, 2H), 2.98 - 2.80 (m, 3H), 2.71 - 2.68 (m, 1H), 1.08 - 1.05 (m, 9H), 0.64 - 0.62 (m, 2H), 0.40 - 0.37 (m, 2H).

### Example A004

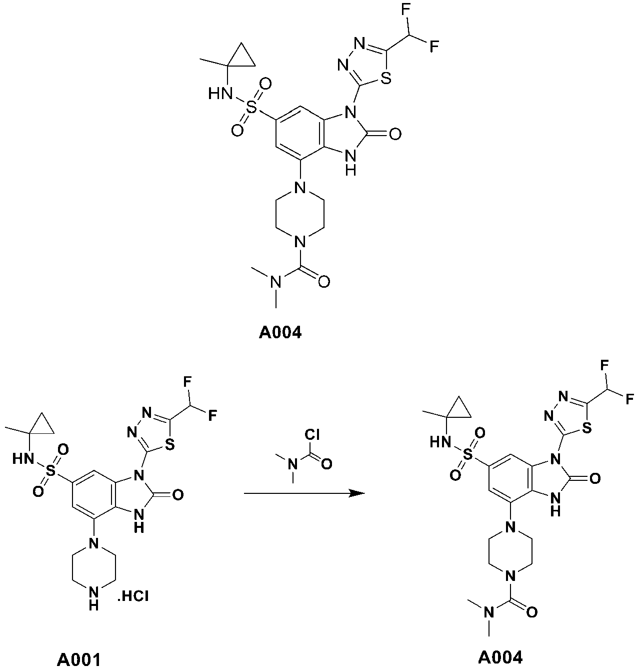

### Step 1: Synthesis of compound A004

A001 (7 mg, 14.4 µmol) and triethylamine (14.6 mg, 144 µmol, 10.00 eq) were dissolved in DCM (2.0 mL), followed by addition of dimethylcarbamoyl chloride (15.5 mg, 144 µmol). The reaction mixture was stirred at 23°C for 20 hours. The reaction mixture was quenched with water (5 mL) and extracted three times with ethyl acetate. The organic phase was washed with saturated brine, dried over anhydrous sodium sulfate, concentrated, and purified by preparative chromatography (acidic conditions, 0.1% FA, chromatographic column: YMC-Actus Triart C18 ExRS) to obtain A004 (4.0 mg).

MS (ESI) M/Z: 557.1 [M+H]⁺.

¹H NMR (400 MHz, DMSO-*d*₆) δ 8.41 (s, 1H), 7.89 (s, 1H), 7.58 (t, *J =* 53.4 Hz, 1H), 7.23 (s, 1H), 3.36 - 3.32 (m, 4H), 3.17 - 3.09 (m, 4H), 2.79 (s, 6H), 1.04 (s, 3H), 0.66 - 0.58 (m, 2H), 0.37 - 0.31 (m, 2H).

### Example A005 and Example A006

**Table 2**

| Example | Structural formula | Structural characterization |
|---|---|---|
| A005 | 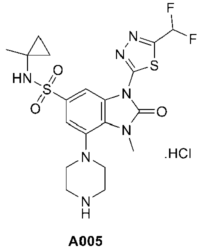 | **¹H NMR** (400 MHz, DMSO-*d₆*): δ 8.94 (s, 2H), 8.69 (d, *J =* 1.6 Hz, 1H), 8.26 (s, 1H), 7.80 - 7.49 (m, 2H), 3.78 (s, 3H), 3.45 - 3.38 (m, 2H), 3.30 - 3.20 (m, 4H), 3.17 - 3.03 (m, 2H), 1.08 (s, 3H), 0.66 - 0.59 (m, 2H), 0.42 - 0.36 (m, 2H). |
| | | **MS (ESI) M/Z:** 500.0 [M+H]⁺. |
| A006 | 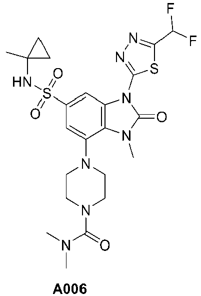 | **¹H NMR** (400 MHz, DMSO-*d₆*): δ 8.65 (d, *J* = 1.6 Hz, 1H), 8.14 (s, 1H), 7.77 - 7.50 (m, 2H), 3.79 (s, 3H), 3.61 - 3.58 (m, 2H), 3.12 - 3.08 (m, 4H), 2.86 - 2.83 (m, 2H), 2.80 (s, 6H), 1.07 (s, 3H), 0.64 - 0.61 (m, 2H), 0.40 - 0.36 (m, 2H). |
| | | **MS (ESI) M/Z:** 571.1 [M+H]⁺. |

### Example A007, Example A008, and Example A009

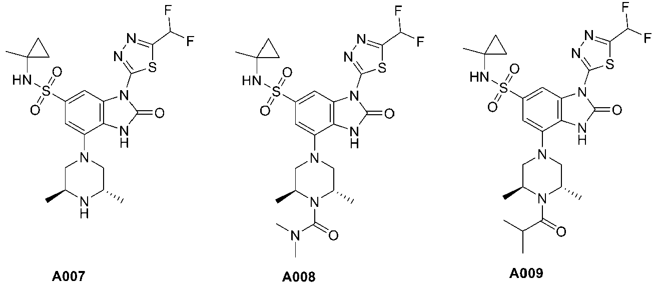

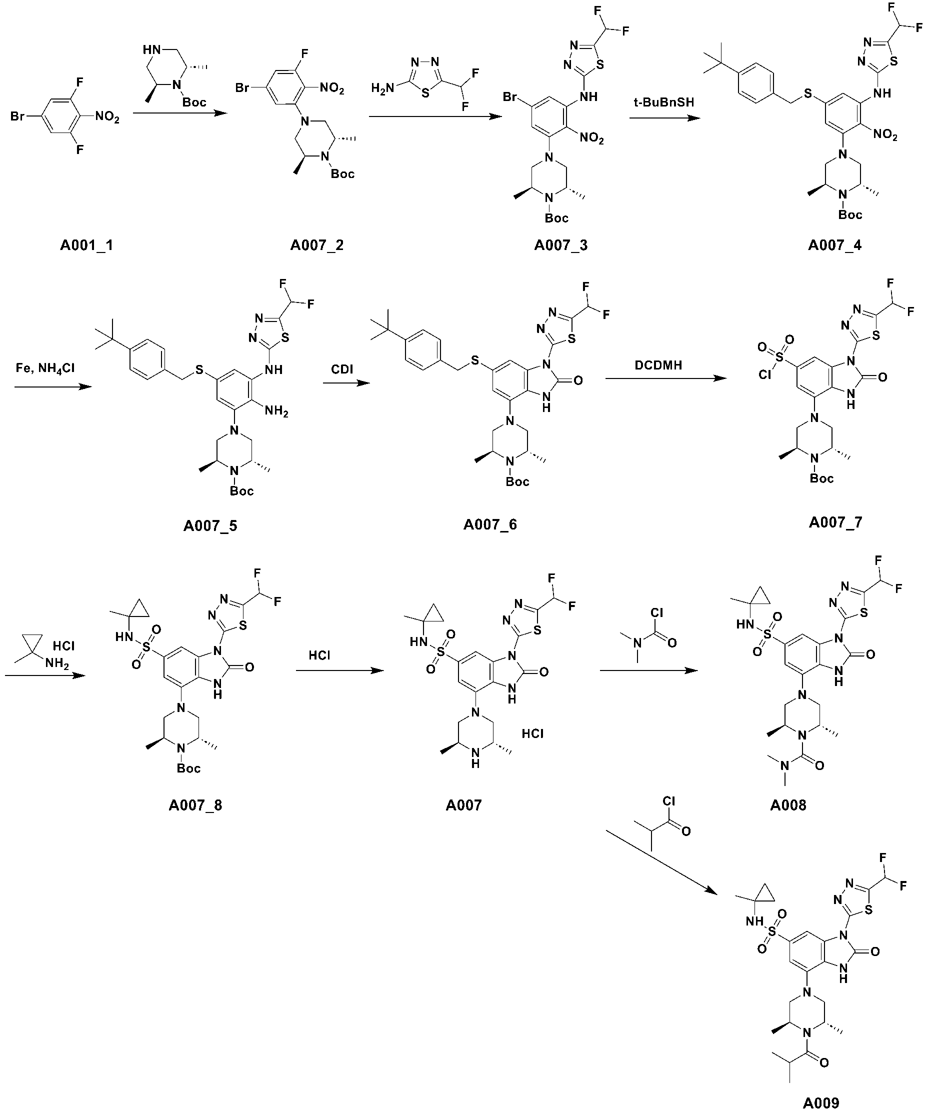

### Step 1: Synthesis of compound A007_2

A001_1 (3.00 g, 12.6 mmol) and N,N-diisopropylethylamine (8.15 g, 63.0 mmol) were dissolved in N,N-dimethylacetamide (30 mL), followed by addition of *tert*-butyl (2S,6S)-2,6-dimethylpiperazine-1-carboxylate (2.70 g, 12.6 mmol). The reaction mixture was heated to 110°C and stirred for 3 hours. The reaction mixture was cooled to room temperature, then quenched with water (100 mL), and extracted with ethyl acetate (30 mL × 3). The organic phase was washed with brine (50 mL × 3), dried over anhydrous sodium sulfate, filtered, and the filtrate was concentrated by rotary evaporation. The residue was purified by column chromatography to obtain A007_2 (5.06 g).

MS (ESI) M/Z: 332.2, 334.2 [M-Boc]⁺.

¹H NMR (400 MHz, DMSO-*d₆*): δ 7.21 - 7.18 (m, 1H), 7.14 (s, 1H), 4.06 - 4.03 (m, 2H), 3.79 - 3.75 (m, 2H), 2.99 - 2.96 (m, 2H), 1.42 (s, 9H), 1.15 (d, *J =* 6.4 Hz, 6H).

### Step 2: Synthesis of compound A007_3

Cesium carbonate (11.4 g, 35.1 mmol) and 5-(difluoromethyl)-1,3,4-thiadiazol-2-amine (2.65 g, 17.6 mmol) were added to a solution of A007_2 (5.06 g, 11.7 mmol) in N,N-dimethylacetamide (50 mL). The reaction mixture was heated to 110°C and stirred for 16 hours. The reaction mixture was cooled to room temperature, then quenched with water (100 mL), and extracted with ethyl acetate (30 mL × 3). The organic phase was washed with brine (50 mL × 3), dried over anhydrous sodium sulfate, filtered, and the filtrate was concentrated by rotary evaporation. The residue was purified by column chromatography to obtain A007_3 (3.65 g).

MS (ESI) M/Z: 561.1, 563.1 [M-H]⁻.

### Step 3: Synthesis of compound A007_4

A007_3 (3.10 g, 5.50 mmol), 4-tert-butylbenzyl mercaptan (1.09 g, 6.05 mmol), and N,N-diisopropylethylamine (1.78 g, 13.8 mmol) were dissolved in 1,4-dioxane (31 mL). The system was purged with nitrogen three times, followed by addition of Pd₂(dba)₃ (504 mg, 550 µmol) and Xantphos (637 mg, 1.10 mmol), and purged with nitrogen three additional times. The reaction mixture was heated to 70°C and stirred for 1 hour. The reaction mixture was cooled to room temperature, then quenched with water (50 mL), and extracted with ethyl acetate (30 mL × 3). The organic phase was washed with brine (50 mL × 1), dried over anhydrous sodium sulfate, filtered, and the filtrate was concentrated by rotary evaporation. The residue was purified by column chromatography to obtain A007_4 (3.65 g).

MS (ESI) M/Z: 663.2 [M+H]⁺.

### Step 4: Synthesis of compound A007_5

A007_4 (3.65 g, 5.51 mmol) was dissolved in ethanol (30 mL) and water (10 mL), followed by addition of iron powder (1.54 g, 27.5 mmol) and ammonium chloride (1.47 g, 27.5 mmol). The reaction mixture was heated to 80°C and stirred for 1.5 hours. The reaction mixture was cooled to room temperature and filtered through diatomite. The filtrate was diluted with water (50 mL) and extracted with ethyl acetate (50 mL × 3). The organic phase was washed with brine (50 mL × 1), dried over anhydrous sodium sulfate, filtered, and the filtrate was concentrated by rotary evaporation. The residue was purified by column chromatography to obtain A007_5 (2.08 g).

MS (ESI) M/Z: 633.2 [M+H]⁺.

¹H NMR (400 MHz, DMSO-*d₆*): δ 9.81 (s, 1H), 7.46 - 7.19 (m, 4H), 7.14 - 7.12 (m, 2H), 6.59 (d, *J* = 2.0 Hz, 1H), 4.82 (s, 2H), 3.96 - 3.92 (m, 4H), 2.99 - 2.96 (m, 2H), 2.48 - 2.44 (m, 2H), 1.42 (s, 9H), 1.37 (d, *J =* 6.4 Hz, 6H), 1.24 (s, 9H).

### Step 5: Synthesis of compound A007_6

A007_5 (600 mg, 948 µmol) was dissolved in tetrahydrofuran (12 mL), followed by addition of carbonyldiimidazole (231 mg, 1.42 mmol). The reaction mixture was heated to 70°C and stirred for 2 hours. The reaction mixture was cooled to room temperature, then quenched with water (10 mL), and extracted with ethyl acetate (10 mL × 3). The organic phase was washed with brine (20 mL × 1), dried over anhydrous sodium sulfate, filtered, and the filtrate was concentrated by rotary evaporation. The residue was purified by column chromatography to obtain A007_6 (720 mg).

MS (ESI) M/Z: 657.3 [M-H]⁻.

¹H NMR (400 MHz, DMSO-*d₆*): δ 11.74 (s, 1H), 7.97 (s, 1H), 7.61 (t, *J* = 53.2 Hz, 1H), 7.31 - 7.24 (m, 4H), 6.58 (s, 1H), 4.16 - 4.14 (m, 2H), 4.07 - 4.02 (m, 2H), 3.76 - 3.73 (m, 2H), 2.88 - 2.86 (m, 2H), 1.43 (s, 9H), 1.23 (s, 9H), 1.19 - 1.17 (m, 6H).

### Step 6: Synthesis of compound A007_7

A007_6 (570 mg, 865 µmol) was dissolved in acetic acid (0.9 mL), water (1.8 mL), and acetonitrile (6.3 mL). The mixture was cooled to 0°C in an ice-salt bath, and 1,3-dichloro-5,5-dimethylhydantoin (341 mg, 1.73 mmol) was added in portions. After the addition was completed, the reaction mixture was slowly warmed to 20°C and stirred for 2 hours. The reaction mixture was cooled again to 0°C, then quenched with water (10 mL), and extracted with dichloromethane (5 mL × 3). The organic phase was washed with brine (10 mL × 1), dried over anhydrous sodium sulfate, filtered, and the filtrate was concentrated by rotary evaporation to obtain A007_7 (500 mg) as a crude product, which was directly used in the next step.

MS (ESI) M/Z: 577.2 [M-H]⁻.

### Step 7: Synthesis of compound A007_8

1-Methylcyclopropanamine hydrochloride (372 mg, 3.45 mmol) and N,N-diisopropylethylamine (670 mg, 5.18 mmol) were dissolved in dichloromethane (5 mL), followed by addition of a solution of A007_7 (500 mg, 864 µmol) in dichloromethane (3 mL). The reaction mixture was stirred at 20°C for 2 hours. The reaction mixture was quenched with water (10 mL) and extracted with ethyl acetate (10 mL × 3). The organic phase was washed with brine (10 mL × 1), dried over anhydrous sodium sulfate, filtered, and the filtrate was concentrated by rotary evaporation. The residue was purified by column chromatography to obtain A007_8 (260 mg).

MS (ESI) M/Z: 612.3 [M-H]⁻.

¹H NMR (400 MHz, DMSO-*d₆*): δ 12.11 (s, 1H), 8.41 (s, 1H), 8.11 (s, 1H), 7.64 (t, *J* = 53.2 Hz, 1H), 7.27 (s, 1H), 4.14 - 4.11 (m, 2H), 3.96 - 3.93 (m, 2H), 3.07 - 3.04 (m, 2H), 1.45 (s. 9H), 1.26 - 1.23 (m, 6H), 1.08 (s. 3H), 0.66 - 0.63 (m, 2H), 0.38 - 0.35 (m, 2H).

### Step 8: Synthesis of compound A007

A007_8 (30 mg, 48.9 µmol) was dissolved in ethyl acetate (0.3 mL), followed by addition of 4 M HCl in ethyl acetate (1 mL). The reaction mixture was stirred at 20°C for 1 hour. The reaction mixture was directly concentrated under reduced pressure to obtain a crude product (25 mg, salt), which was directly used in the next step. 20 mg of the crude product was purified by preparative chromatography (salt, chromatographic column: Phenomenex Luna C18) to obtain A007 (9.91 mg, hydrochloride salt).

MS (ESI) M/Z: 514.3 [M+H]⁺.

¹H NMR (400 MHz, DMSO-*d₆*): δ 12.47 (s, 1H), 9.11 (s, 2H), 8.55 (s, 1H), 8.18 (s, 1H), 7.64 (t, *J* = 53.2 Hz, 1H), 7.38 (s, 1H), 3.93 - 3.91 (m, 2H), 3.41 - 3.38 (m, 2H), 2.82 - 2.78 (m, 2H), 1.35 (d, *J =* 6.4 Hz, 6H), 1.08 (s, 3H), 0.64 - 0.62 (m, 2H), 0.40 - 0.37 (m, 2H).

### Step 9: Synthesis of compound A008

A007 (17 mg, 30.9 µmol) and N,N-diisopropylethylamine (24.0 mg, 185 µmol) were dissolved in N-methylpyrrolidone (0.2 mL), followed by addition of dimethylcarbamoyl chloride (4.99 mg, 46.4 µmol, 1.50 eq). The reaction mixture was heated to 35°C and stirred for 16 hours. The reaction mixture was quenched with water (0.1 mL). The residue was purified by preparative chromatography (neutral conditions, chromatographic column: Waters Xbridge Prep OBD C18) to obtain A008 (9.64 mg).

MS (ESI) M/Z: 585.2 [M+H]⁺.

¹H NMR (400 MHz, DMSO-*d₆*): δ 12.42 (s, 1H), 8.51 (s, 1H), 8.09 (s, 1H), 7.63 (t, *J* = 53.2 Hz, 1H), 7.35 (s, 1H), 3.74 - 3.70 (m, 2H), 3.28 - 3.25 (m, 2H), 2.91 (s, 6H), 2.62 - 2.60 (m, 2H), 1.09 - 1.07 (m, 9H) 0.64 - 0.61 (m, 2H), 0.39 - 0.36 (m, 2H).

### Step 10: Synthesis of compound A009

A007 (20 mg, 36.4 µmol) and triethylamine (22.1 mg, 218 µmol) were dissolved in N,N-dimethylacetamide (0.1 mL), followed by addition of 2-methylpropanoyl chloride (4.26 mg, 40.0 µmol). The reaction mixture was stirred at 20°C for 1 hour. The reaction mixture was quenched with water (0.1 mL). The residue was purified by preparative chromatography (neutral conditions, chromatographic column: Waters Xbridge Prep OBD C18) to obtain A009 (9.05 mg).

MS (ESI) M/Z: 584.2 [M+H]⁺.

¹H NMR (400 MHz, DMSO-*d₆*): δ 12.01 (s, 1H), 8.38 (s, 1H), 8.06 (s, 1H), 7.63 (t, *J* = 53.2 Hz, 1H), 7.25 (s, 1H), 4.32 - 4.30 (m, 2H), 4.13 - 4.10 (m, 2H), 3.22 - 3.19 (m, 2H), 2.82 - 2.79 (m, 1H), 1.26 - 1.25 (m, 6H), 1.07 - 1.05 (m, 9H), 0.68 - 0.63 (m, 2H), 0.37 - 0.35 (m, 2H).

### Example A010, Example A011, and Example A012

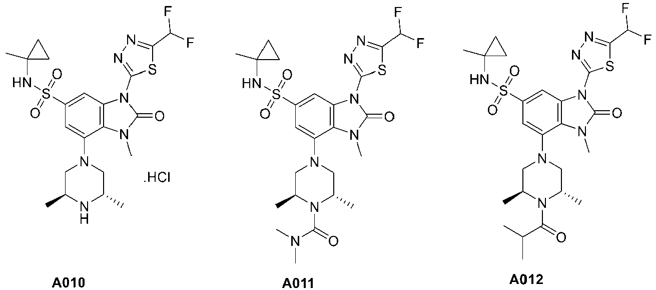

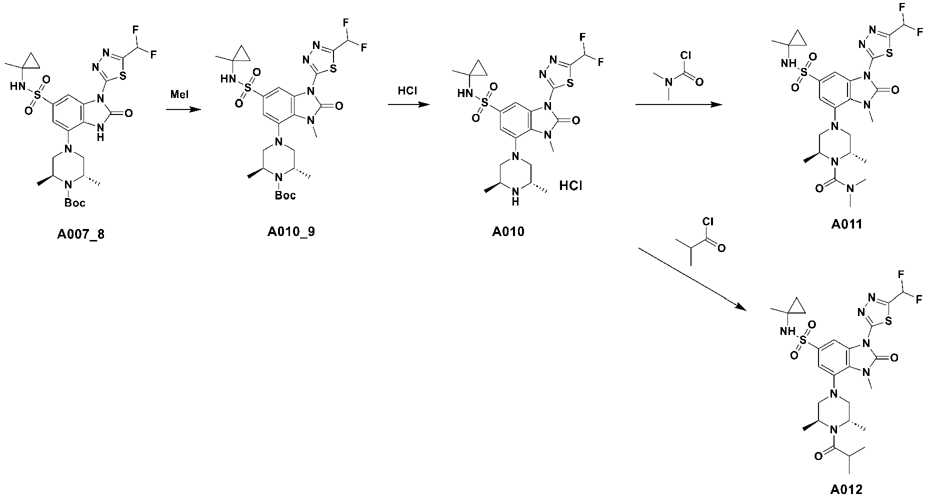

### Step 1: Synthesis of compound A010_9

A007_8 (140 mg, 228 µmol) and potassium carbonate (63.1 mg, 456 µmol) were dissolved in N,N-dimethylformamide (2 mL), followed by addition of iodomethane (48.6 mg, 342 µmol). The reaction mixture was stirred at 20°C for 1 hour. The reaction mixture was quenched with water (2 mL) and extracted with ethyl acetate (2 mL × 3). The organic phase was washed with brine (2 mL × 1), dried over anhydrous sodium sulfate, filtered, and the filtrate was concentrated by rotary evaporation. The residue was purified by preparative chromatography on a silica gel plate to obtain A010_9 (50 mg).

MS (ESI) M/Z: 626.3 [M-H]⁻.

### Step 2: Synthesis of compound A010

A010_9 (50 mg, 79.7 µmol) was dissolved in ethyl acetate (1 mL), followed by addition of 4 M HCl in ethyl acetate (2 mL). The reaction mixture was stirred at 20°C for 1 hour. The reaction mixture was directly concentrated under reduced pressure to obtain A010 (55 mg, hydrochloride salt) as a crude product, which was directly used in the next step. 15 mg of the crude product was purified by preparative chromatography (acidic conditions, chromatographic column: Phenomenex Luna C18) to obtain A010 (7.31 mg, hydrochloride salt).

MS (ESI) M/Z: 528.1 [M+H]⁺.

¹H NMR (400 MHz, DMSO-*d₆*): δ 9.25 (s, 2H), 8.71 (s, 1H), 8.28 (s, 1H), 7.78 - 7.51 (m, 2H), 3.88 - 3.72 (m, 2H), 3.78 (s, 3H), 3.38 - 3.35 (m, 2H), 3.10 - 3.06 (m, 1H), 2.65 - 2.58 (m, 1H), 1.51 (d, *J =* 5.6 Hz, 3H), 1.25 (d, *J =* 4.4 Hz, 3H), 1.09 (s, 3H), 0.64 - 0.62 (m, 2H), 0.41 - 0.38 (m, 2H).

### Step 3: Synthesis of compound A011

A010 (15 mg, 26.6 µmol) and N,N-diisopropylethylamine (20.6 mg, 160 µmol, 6.00 eq) were dissolved in N-methylpyrrolidone (0.2 mL), followed by addition of dimethylcarbamoyl chloride (4.29 mg, 38.9 µmol, 1.50 eq). The reaction mixture was heated to 35°C and stirred for 16 hours. The reaction mixture was quenched with water (0.1 mL). The residue was purified by preparative chromatography (neutral conditions, chromatographic column: Waters Xbridge Prep OBD C18) to obtain A011 (6.65 mg).

MS (ESI) M/Z: 599.2 [M+H]⁺.

¹H NMR (400 MHz, DMSO-*d₆*): δ 8.66 (s, 1H), 8.17 (s, 1H), 7.77 - 7.51 (m, 2H), 3.82 (s, 3H), 3.68 - 3.60 (m, 2H), 3.27 - 3.19 (m, 2H), 2.99 - 2.96 (m, 1H), 2.92 (s, 6H), 2.33 - 2.30 (m, 1H), 1.23 - 1.19 (m, 3H), 1.08 (s, 3H), 1.00 - 0.97 (m, 3H), 0.64 - 0.62 (m, 2H), 0.40 - 0.38 (m, 2H).

### Step 4: Synthesis of compound A012

A010 (15 mg, 26.6 µmol) and triethylamine (16.2 mg, 160 µmol, 6.00 eq) were dissolved in N,N-dimethylacetamide (0.1 mL), followed by addition of 2-methylpropanoyl chloride (4.25 mg, 39.9 µmol, 1.50 eq). The reaction mixture was stirred at 20°C for 1 hour. The reaction mixture was quenched with water (0.1 mL). The residue was purified by preparative chromatography (neutral conditions, chromatographic column: Waters Xbridge Prep OBD C18) to obtain A012 (4.96 mg).

MS (ESI) M/Z: 598.2 [M+H]⁺.

¹H NMR (400 MHz, DMSO-*d₆*): δ 8.60 (s, 1H), 8.15 (s, 1H), 7.77 - 7.51 (m, 2H), 4.27 - 4.23 (m, 2H), 3.79 - 3.75 (m, 5H), 3.04 - 3.02 (m, 2H), 2.89 - 2.82 (m, 1H), 1.32 (d, *J =* 7.0 Hz, 6H), 1.08 - 1.04 (m, 9H), 0.66 - 0.60 (m, 2H), 0.41 - 0.35 (m, 2H).

### Example A013 and Example A014

**Table 3**

| Example | Structural formula | Structural characterization |
|---|---|---|
| A013 | 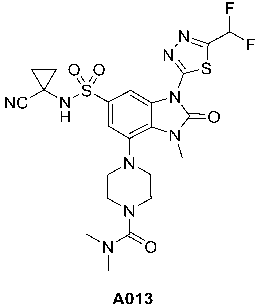 | **¹H NMR** (400 MHz, CDCl₃-*d*): δ 8.84 (s, 1H), 7.81 (s, 1H), 7.05 (t, *J =* 53.6 Hz, 1H), 6.28 (s, 1H), 3.91 (s, 3H), 3.75 - 3.71 (m, 2H), 3.24 - 3.14 (m, 2H), 3.13 - 3.03 (m, 4H), 2.92 (s, 6H), 1.68 - 1.62 (m, 2H), 1.47 - 1.41 (m, 2H). |
| | | **MS (ESI) M/Z:** 582.1 [M+H]⁺. |
| A014 | 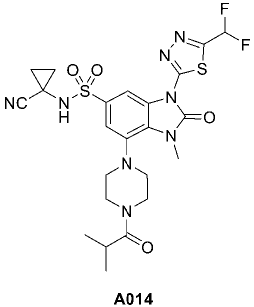 | **¹H NMR** (400 MHz, CDCl₃-*d*): δ 8.85 (d, *J* = 1.6 Hz, 1H), 7.78 (d, *J =* 1.2 Hz, 1H), 7.06 (t, *J =* 53.6 Hz, 1H), 6.24 (s, 1H), 4.84 - 4.71 (m, 1H), 4.11 - 4.00 (m, 1H), 3.92 (s, 3H), 3.52 - 3.39 (m, 1H), 3.19 - 3.16 (m, 2H), 3.06 - 2.91 (m, 3H), 2.89 - 2.81 (m, 1H), 1.67 - 1.65 (m, 2H), 1.46 - 1.42 (m, 2H), 1.23 - 1.18 (m, 6H). |
| | | **MS (ESI) M/Z:** 581.2 [M+H]⁺. |

### Example A017, Example A018, and Example A019

**Table 4**

| Example | Structural formula | Structural characterization |
|---|---|---|
| A017 | 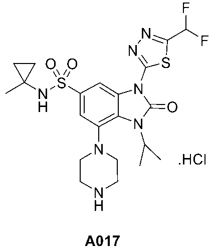 | **¹H NMR** (400 MHz, DMSO-*d₆*): δ 9.11 (s, 2H), 8.73 (d, *J =* 1.6 Hz, 1H), 8.27 (s, 1H), 7.77 - 7.51 (m, 2H), 5.76 - 5.67 (m, 1H), 3.45 - 3.41 (m, 2H), 3.27 - 3.23 (m, 4H), 3.13 - 3.07 (m, 2H), 1.57 (d, *J* = 6.8 Hz, 6H), 1.10 (s, 3H), 0.65 - 0.62 (m, 2H), 0.42 - 0.39 (m, 2H). |
| | | **MS (ESI) M/Z:** 528.2 [M+H]⁺. |
| A018 | 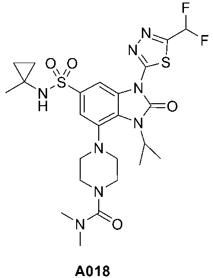 | **¹H NMR** (400 MHz, DMSO-*d₆*): δ 8.70 (s, 1H), 8.16 (s, 1H), 7.77 - 7.50 (m, 2H), 5.91 - 5.84 (m, 1H), 3.64 - 3.61(m, 2H), 3.09 - 3.01 (m, 4H), 2.89 - 2.84 (m, 2H), 2.81 (s, 6H), 1.57 (d, *J =* 6.8 Hz, 6H), 1.08 (s, 3H), 0.65 - 0.62 (m, 2H), 0.41 - 0.38 (m, 2H). |
| | | **MS (ESI) M/Z:** 599.2 [M+H]⁺. |
| A019 | 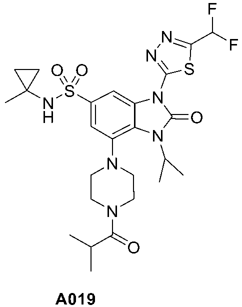 | **¹H NMR** (400 MHz, DMSO-*d₆*): δ 8.70 (s, 1H), 8.14 (s, 1H), 7.77 - 7.51 (m, 2H), 5.94 - 5.83 (m, 1H), 4.54 - 4.51 (m, 1H), 4.13 - 4.09 (m, 1H), 3.38 - 3.36 (m, 1H), 3.16 - 3.13 (m, 2H), 2.97 - 2.92 (m, 1H), 2.87 - 2.83 (m, 2H), 2.69 - 2.67 (m, 1H), 1.57 (d, *J =* 6.4 Hz, 6H), 1.08 - 1.02 (m, 9H), 0.65 - 0.62 (m, 2H), 0.41 - 0.38 (m, 2H). |
| | | **MS (ESI) M/Z:** 598.2 [M+H]⁺. |

### Example A023

**Table 5**

| Example | Structural formula | Structural characterization |
|---|---|---|
| A023 | 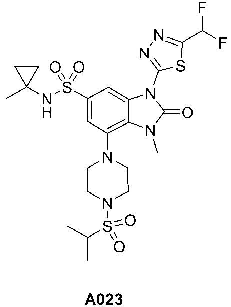 | **¹H NMR** (400 MHz, DMSO-*d₆*): δ 8.67 (d, *J* = 1.6 Hz, 1H), 8.14 (s, 1H), 7.77 - 7.50 (m, 2H), 3.78 (s, 3H), 3.72 - 3.68 (m, 2H), 3.44 - 3.41 (m, 1H), 3.29 - 3.19 (m, 4H), 2.88 - 2.85 (m, 2H), 1.28 (d, *J* = 6.8 Hz, 6H), 1.07 (s, 3H), 0.65 - 0.62 (m, 2H), 0.40 - 0.37 (m, 2H). |
| | | **MS (ESI) M/Z:** 606.1 [M+H]⁺. |

### Example A024

**Table 6**

| Example | Structural formula | Structural characterization |
|---|---|---|
| A024 | 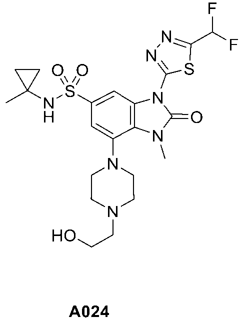 | **¹H NMR** (400 MHz, CDCl₃-*d*) δ 8.89 (d, *J* = 1.2 Hz, 1H), 7.77 (d, *J* = 1.2 Hz, 1H), 7.06 (t, *J* = 53.6 Hz, 1H), 5.00 (s, 1H), 3.92 (s, 3H), 3.72 - 3.70 (m, 2H), 3.12 - 3.09 (m, 4H), 3.04 - 3.01 (m, 2H), 2.68 (t, *J =* 5.2 Hz, 2H), 2.60 - 2.58 (m, 1H), 2.53 - 2.51 (m, 2H), 1.26 (s, 3H), 0.85 - 0.83 (m, 2H), 0.54 - 0.51 (m, 2H). |
| | | **MS (ESI) M/Z:** 544.1 [M+H]⁺. |

### Example A026

**Table 7**

| Example | Structural formula | Structural characterization |
|---|---|---|
| A026 | 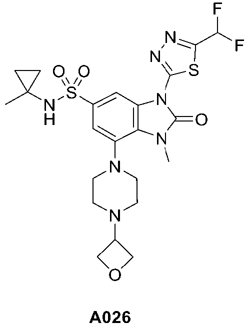 | **¹H NMR** (400 MHz, DMSO-*d*₆) δ 8.65 (d, *J* = 1.6 Hz, 1H), 8.15 (s, 1H), 7.77 - 7.50 (m, 2H), 4.60 - 4.57 (m, 2H), 4.51 - 4.48 (m, 2H), 3.76 (s, 3H), 3.56 - 3.52 (m, 1H), 3.12 - 3.10 (m, 2H), 2.93 - 2.81 (m, 4H), 2.21 - 2.17 (m, 2H), 1.08 (s, 3H), 0.65 - 0.62 (m, 2H), 0.40 - 0.37 (m, 2H). |
| | | **MS (ESI) M/Z:** 556.2 [M+H]⁺. |

### Example A027

**Table 8**

| Example | Structural formula | Structural characterization |
|---|---|---|
| A027 | 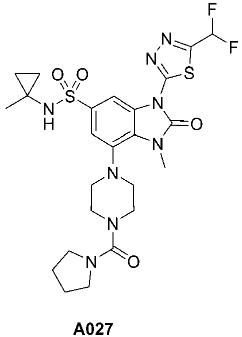 | **¹H NMR** (400 MHz, CDCl₃-*d*): δ 8.89 (d, *J* = 1.2 Hz, 1H), 7.76 (d, *J =* 1.6 Hz, 1H), 7.06 (t, *J =* 53.6 Hz, 1H), 5.09 (s, 1H), 3.92 (s, 3H), 3.84 - 3.79 (m, 2H), 3.44 (t, *J* = 6.8 Hz, 4H), 3.21 - 3.12 (m, 2H), 3.11 - 3.02 (m, 4H), 1.92 - 1.86 (m, 4H), 1.25 (s, 3H), 0.86 - 0.80 (m, 2H), 0.54 - 0.49 (m, 2H). |
| | | **MS (ESI) M/Z:** 597.2 [M+H]⁺. |

### Example A028

**Table 9**

| Example | Structural formula | Structural characterization |
|---|---|---|
| A028 | 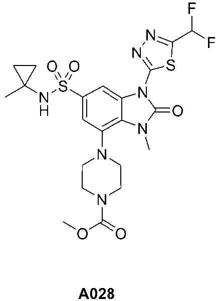 | **¹H NMR** (400 MHz, DMSO-*d*₆) δ 8.66 (s, 1H), 8.13 (s, 1H), 7.77 - 7.50 (m, 2H), 4.06 - 3.99 (m, 2H), 3.79 (s, 3H), 3.65 (s, 3H), 3.25 - 3.10 (m, 4H), 2.81 - 2.71 (m, 2H), 1.07 (s, 3H), 0.64 - 0.61 (m, 2H), 0.40 - 0.37 (m, 2H). |
| | | **MS (ESI) M/Z:** 558.2 [M+H]⁺. |

### Example A029

**Table 10**

| Example | Structural formula | Structural characterization |
|---|---|---|
| A029 | 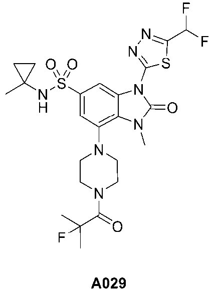 | **¹H NMR** (400 MHz, DMSO-*d₆*): δ 8.67 (d, *J* = 1.2 Hz, 1H), 8.13 (s, 1H), 7.77 - 7.51 (m, 2H), 4.49 - 4.40 (m, 2H), 3.81 (s, 3H), 3.48 - 3.38 (m, 1H), 3.28 - 3.12 (m, 2H), 3.11 - 2.96 (m, 1H), 2.78 - 2.68 (m, 2H), 1.63 (s, 3H), 1.57 (s, 3H), 1.06 (s, 3H), 0.64 - 0.61 (m, 2H), 0.40 - 0.37 (m, 2H). |
| | | **MS (ESI) M/Z:** 588.2 [M+H]⁺. |

### Example A030

**Table 11**

| Example | Structural formula | Structural characterization |
|---|---|---|
| A030 | 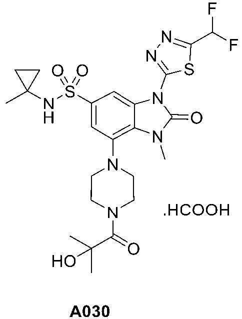 | **¹H NMR** (400 MHz, DMSO-*d*₆) δ 8.66 (s, 1H), 8.15 (s, 1H), 7.77 - 7.50 (m, 2H), 5.50 (s, 1H), 5.02 - 4.52 (m, 2H), 3.81 (s, 3H), 3.17 - 2.77 (m, 6H), 1.36 (s, 6H), 1.07 (s, 3H), 0.64 - 0.61 (m, 2H), 0.40 - 0.37 (m, 2H). |
| | | **MS (ESI) M/Z:** 586.1 [M+H]⁺. |

### Example B001 and Example B002

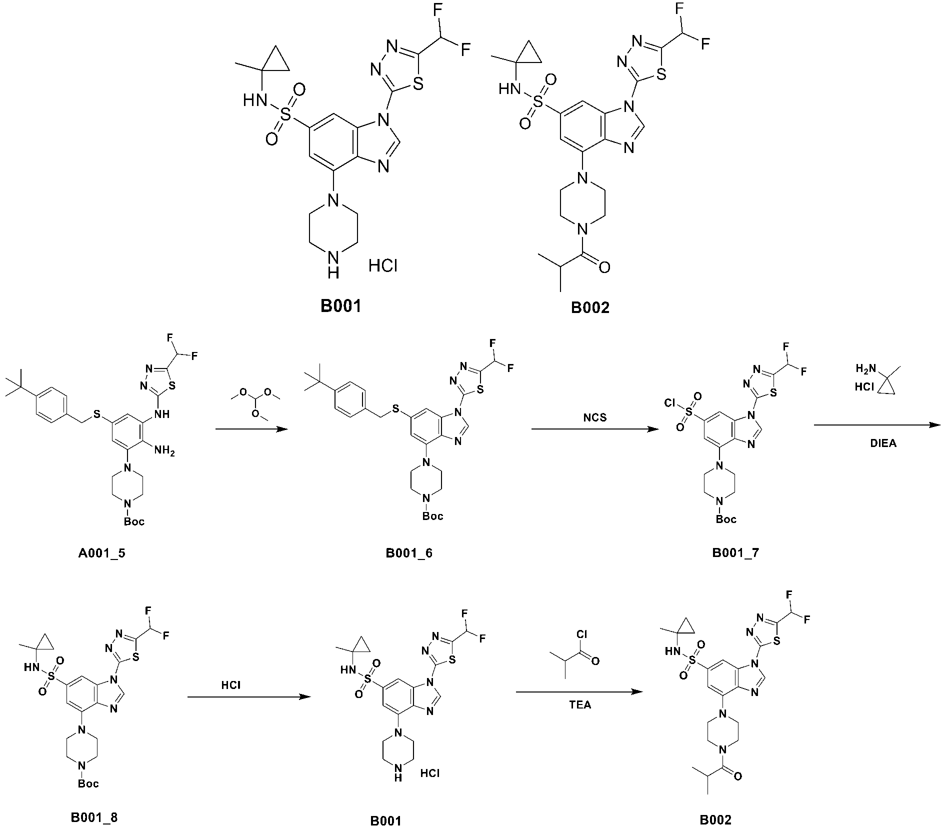

### Step 1: Synthesis of compound B001_6

A001_5 (800 mg, 1.32 mmol) was dissolved in trimethyl orthoformate (8.00 mL), followed by addition of p-toluenesulfonic acid (22.7 mg, 132 µmol, 0.10 eq). The reaction mixture was heated to 100°C and stirred for 1 hour. After completion of the reaction, the reaction mixture was cooled to room temperature, quenched with water (20 mL), and extracted with ethyl acetate (20 mL × 3). The organic phases were combined, washed with brine (30 mL × 3), dried over anhydrous sodium sulfate, filtered, and the filtrate was concentrated by rotary evaporation to obtain B001_6 (700 mg) as a crude product, which was directly used in the next step.

MS (ESI) M/Z: 615.2 [M+H]⁺.

¹H NMR (400 MHz, DMSO-*d₆*): δ 8.80 (s, 1H), 7.80 - 7.53 (m, 2H), 7.33 - 7.27 (m, 4H), 6.60 (s, 1H), 4.23 (s, 2H), 3.51 - 3.48 (m, 4H), 3.37 - 3.35 (m, 4H), 1.43 (s, 9H), 1.24 (s, 9H).

### Step 2: Synthesis of compound B001_7

B001_6 (315 mg, 512 µmol) was dissolved in dichloromethane (4.00 mL), acetic acid (4.00 mL), and water (2.00 mL). The mixture was cooled to 0°C in an ice-salt bath, and N-chlorosuccinimide (205 mg, 1.54 mmol) was added in portions. After the addition was completed, the reaction mixture was slowly warmed to 20°C and stirred for 4 hours. Two parallel reactions were carried out simultaneously and combined for work-up after completion. The reaction mixture was cooled again to 0°C, then slowly added with saturated sodium bicarbonate aqueous solution to adjust the pH to approximately 5, and extracted with dichloromethane (20 mL × 2). The organic phases were combined, washed with brine (30 mL × 1), dried over anhydrous sodium sulfate, filtered, and the filtrate was concentrated by rotary evaporation to obtain B001_7 (548 mg) as a crude product, which was directly used in the next step.

MS (ESI) M/Z: 479.2, 481.1 [M-55]⁺.

### Step 3: Synthesis of compound B001_8

1-Methylcyclopropanamine hydrochloride (440 mg, 4.10 mmol) and N,N-diisopropylethylamine (529 mg, 4.10 mmol) were dissolved in dichloromethane (5.00 mL). The mixture was cooled to 0°C, and a solution of B001_7 (548 mg, 1.02 mmol) in dichloromethane (5 mL) was slowly added dropwise thereto. After the addition was completed, the reaction mixture was slowly warmed to 20°C and stirred for 1 hour. After completion of the reaction, the reaction mixture was quenched with water (20 mL) and extracted with dichloromethane (20 mL × 2). The organic phases were combined, washed with brine (30 mL × 1), dried over anhydrous sodium sulfate, filtered, and the filtrate was concentrated by rotary evaporation. The residue was purified by column chromatography (SiO₂, petroleum ether/ethyl acetate = 1/0 to 1/1) to obtain B001_8 (100 mg).

MS (ESI) M/Z: 514.1 [M-55]⁺.

¹H NMR (400 MHz, DMSO-*d₆*): δ 9.04 (s, 1H), 8.24 (s, 1H), 8.13 (s, 1H), 7.67 (t, *J =* 53.2 Hz, 1H), 7.20 (s, 1H), 3.57 (s, 8H), 1.43 (s, 9H), 1.04 (s, 3H), 0.63 (t, *J* = 5.6 Hz, 2H), 0.37 (t, *J =* 5.6 Hz, 2H).

### Step 4: Synthesis of compound B001

B001_8 (60.0 mg, 105 µmol, 1.00 eq) was dissolved in methanol (0.5 mL), followed by dropwise addition of 4 M HCl in methanol (2 mL). The reaction mixture was stirred at 20°C for 2 hours. After completion of the reaction, the reaction mixture was directly concentrated under reduced pressure to obtain B001 (54.43 mg, hydrochloride salt).

MS (ESI) M/Z: 470.0 [M+H]⁺.

¹H NMR (400 MHz, DMSO-*d₆*): δ 9.24 (s, 1H), 9.09 (s, 1H), 8.30 (s, 1H), 8.20 (s, 1H), 7.68 (t, *J =* 53.0 Hz, 1H), 7.26 (s, 1H), 3.83 (t, *J =* 4.8 Hz, 4H), 3.35 (s, 4H), 1.05 (s, 3H), 0.63 (t, *J =* 5.6 Hz, 2H), 0.39 - 0.36 (m, 2H).

### Step 5: Synthesis of compound B002

B001 (37.0 mg, 73.1 µmol, hydrochloride salt) and triethylamine (29.6 mg, 292 µmol) were dissolved in dichloromethane (1.00 mL). The mixture was cooled to 0°C, followed by addition of 2-methylpropanoyl chloride (15.6 mg, 146 µmol). After the addition was completed, the reaction mixture was slowly warmed to 20°C and stirred for 1 hour. After completion of the reaction, the reaction mixture was quenched with water (5 mL) and extracted with dichloromethane (5 mL × 2). The organic phases were combined, washed with brine (5 mL × 1), dried over anhydrous sodium sulfate, filtered, and the filtrate was concentrated by rotary evaporation. The crude product was purified by preparative chromatography on a silica gel plate to obtain B002 (23.38 mg).

MS (ESI) M/Z: 540.1 [M+H]⁺.

¹H NMR (400 MHz, DMSO-*d₆*): δ 9.05 (s, 1H), 8.25 (s, 1H), 8.14 (s, 1H), 7.67 (t, *J =* 53.2 Hz, 1H), 7.20 (s, 1H), 3.76 - 3.71 (m, 4H), 3.62 - 3.57 (m, 4H), 2.99 - 2.92 (m, 1H), 1.05 - 1.03 (m, 9H), 0.64 (t, *J =* 5.6 Hz, 2H), 0.37 (t, *J =* 5.6 Hz, 2H).

### Example B003

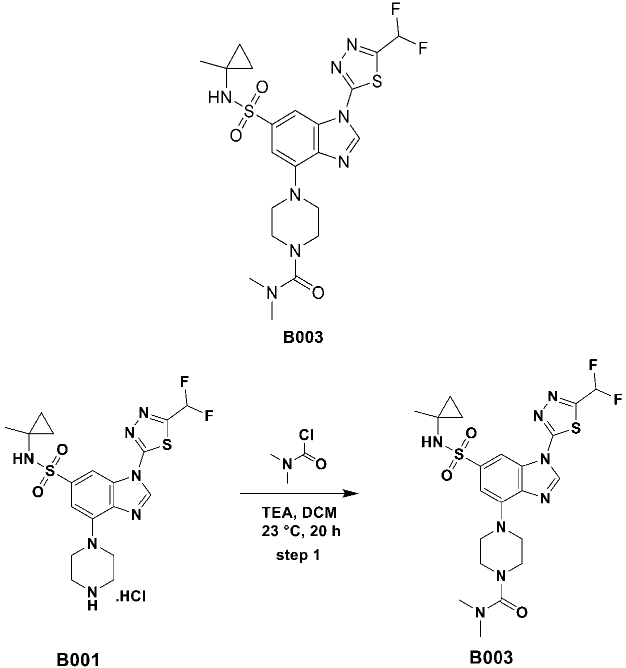

### Step 1: Synthesis of compound B003

B001 (8 mg, 15.8 µmol) and triethylamine (16.0 mg, 158 µmol) were dissolved in DCM (2.0 mL), followed by addition of dimethylcarbamoyl chloride (17.0 mg, 158 µmol, 10 eq). The reaction mixture was stirred at 23°C for 20 hours. The reaction mixture was quenched with water (5 mL) and extracted three times with ethyl acetate. The organic phase was washed with saturated brine, dried over anhydrous sodium sulfate, concentrated, and purified by preparative chromatography (acidic conditions, 0.1% FA, chromatographic column: YMC-Actus Triart C18 ExRS) to obtain B003 (2.1 mg).

MS (ESI) M/Z: 541.1 [M+H]⁺.

¹H NMR (400 MHz, DMSO-*d*₆) 9.04 (s, 1H), 8.25 (d, *J* = 1.4 Hz, 1H), 8.16 (s, 1H), 7.68 (t, *J* = 53.0 Hz, 1H), 7.22 (d, *J* = 1.6 Hz, 1H), 3.66 - 3.56 (m, 4H), 3.40 - 3.30 (m, 4H), 2.81 (s, 6H), 1.05 (s, 3H), 0.67 - 0.61 (m, 2H), 0.40 - 0.35 (m, 2H).

### Example B004, Example B005, and Example B006

**Table 12**

| Example | Structural formula | Structural characterization |
|---|---|---|
| B004 | 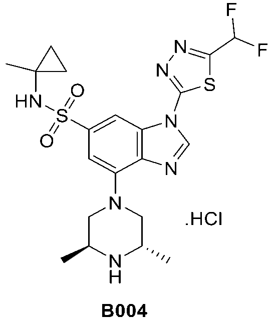 | **¹H NMR** (400 MHz, DMSO-*d₆*): δ 9.22 - 9.09 (m, 2H), 9.08 (s, 1H), 8.29 (s, 1H), 8.20 (s, 1H), 7.69 (t, *J* = 53.2 Hz, 1H), 7.25 (s, 1H), 3.84 - 3.81 (m, 2H), 3.78 - 3.68 (m, 4H), 1.43 (d, *J* = 6.4 Hz, 6H), 1.06 (s, 3H), 0.71 - 0.59 (m, 2H), 0.45 - 0.33 (m, 2H). |
| | | **MS (ESI) M/Z:** 498.1 [M+H]⁺. |
| B005 | 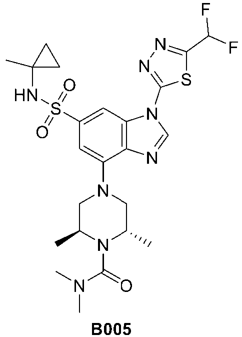 | **¹H NMR** (400 MHz, DMSO-*d₆*): δ 9.03 (s, 1H), 8.20 (d, *J* = 1.2 Hz, 1H), 8.16 (s, 1H), 7.68 (t, *J* = 52.8 Hz, 1H), 7.20 (d, *J* = 1.2 Hz, 1H), 3.66 - 3.51 (m, 6H), 2.90 (s, 6H), 1.13 (d, *J* = 5.6 Hz, 6H), 1.05 (s, 3H), 0.69 - 0.59 (m, 2H), 0.40 - 0.34 (m, 2H). |
| | | **MS (ESI) M/Z:** 569.3 [M+H]⁺. |
| B006 | 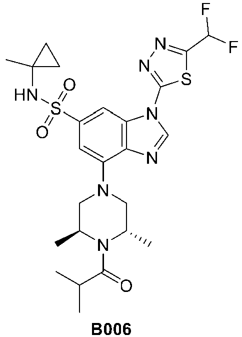 | **¹H NMR** (400 MHz, DMSO-*d₆*): δ 8.97 (s, 1H), 8.11 (s, 1H), 8.06 (s, 1H), 7.68 (t, *J =* 53.2 Hz, 1H), 7.01 (s, 1H), 4.46 - 4.33 (m, 2H), 4.14 - 3.98 (m, 4H), 2.86 - 2.78 (m, 1H), 1.32 - 1.17 (m, 6H), 1.08 - 1.04 (m, 9H), 0.68 - 0.62 (m, 2H), 0.40 - 0.34 (m, 2H). |
| | | **MS (ESI) M/Z:** 568.2 [M+H]⁺. |

### Example B007 and Example B008

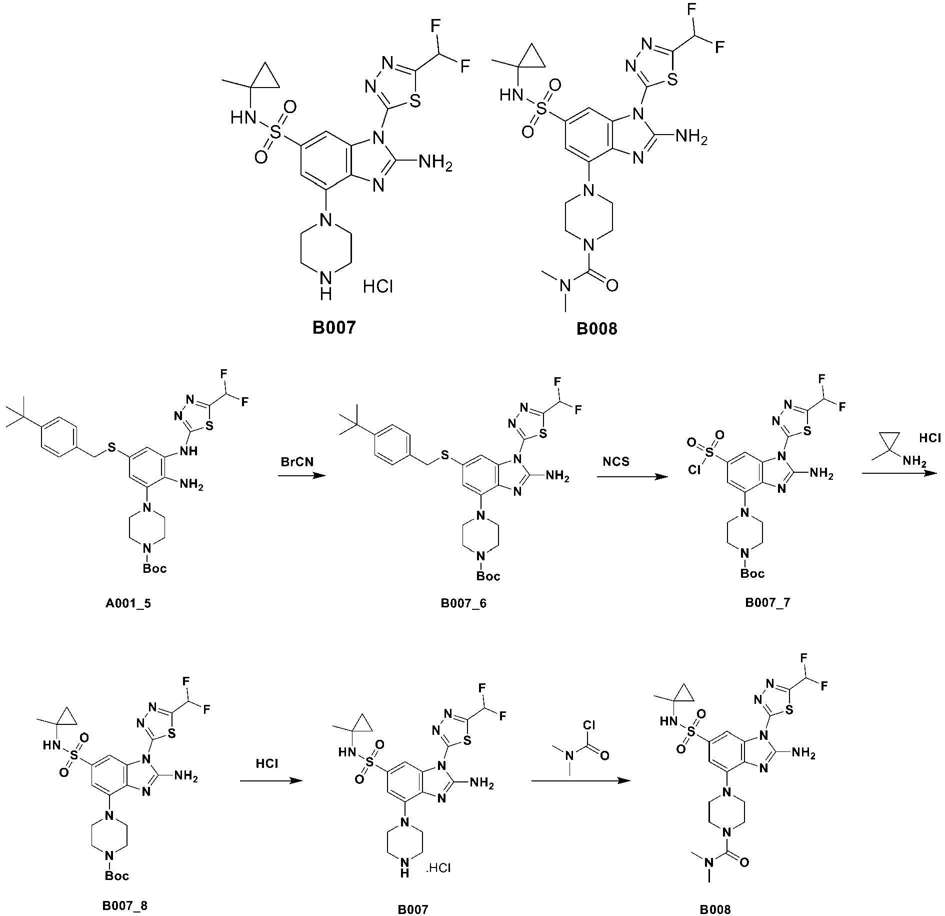

### Step 1: Synthesis of compound B007_6

A001_5 (100 mg, 165 mmol) was dissolved in a mixture of dichloromethane (2 mL) and tert-butanol (0.4 mL), followed by addition of cyanogen bromide (101 mg, 960 mmol, 5.81 eq). The reaction mixture was stirred at 20°C for 12 hours. The reaction mixture was cooled to 0°C, then slowly added with saturated sodium bicarbonate aqueous solution to adjust the pH to approximately 7, and extracted with dichloromethane (10 mL × 3). The organic phase was washed with saturated brine (10 mL × 3), dried over anhydrous sodium sulfate, filtered, and the filtrate was concentrated by rotary evaporation to obtain B007_6 (138 mg) as a crude product, which was directly used in the next step.

MS (ESI) M/Z: 630.2 [M+H]⁺.

¹H NMR (400 MHz, DMSO-*d₆*): δ 7.65 (t, *J* = 52.8 Hz, 1H), 7.29 - 7.26 (m, 3H), 7.25 - 7.22 (m, 2H), 7.18 - 7.16 (m, 2H), 6.46 (s, 1H), 4.08 (s, 2H), 3.47 - 3.45 (m, 4H), 3.19 - 3.16 (m, 4H), 1.43 (s, 9H), 1.23 (s, 9H).

### Step 2: Synthesis of compound B007_7

B007_6 (100 mg, 158.7 mmol) was dissolved in a mixture of dichloromethane (1.5 mL), acetic acid (1.5 mL), and water (0.75 mL). The reaction mixture was cooled to 0°C, and N-chlorosuccinimide (83 mg, 624 mmol) was added in portions. After the addition was completed, the reaction mixture was slowly warmed to 20°C and stirred for 2 hours. The reaction mixture was cooled to 0°C, then slowly added with saturated sodium bicarbonate aqueous solution to adjust the pH to approximately 6, and extracted with dichloromethane (20 mL × 3). The organic phase was washed with brine (20 mL × 3), dried over anhydrous sodium sulfate, filtered, and the filtrate was concentrated by rotary evaporation to obtain B007_7 (87 mg) as a crude product, which was directly used in the next step.

MS (ESI) M/Z: 548.2, 550.1 [M-H]⁻.

### Step 3: Synthesis of compound B007_8

1-Methylcyclopropanamine hydrochloride (68 mg, 632.7 µmol) and N,N-diisopropylethylamine (82 mg, 632.7 µmol) were dissolved in dichloromethane (2 mL). The mixture was cooled to 0°C, and a solution of B007_7 (87 mg, 158 mmol) in dichloromethane (1 mL) was slowly added dropwise thereto. After the addition was completed, the reaction mixture was slowly warmed to 20°C and stirred for 1 hour. The reaction mixture was quenched with water (20 mL) and extracted with dichloromethane (20 mL × 2). The organic phase was washed with brine (30 mL × 1), dried over anhydrous sodium sulfate, filtered, and the filtrate was concentrated by rotary evaporation. The residue was purified by preparative chromatography on a silica gel plate to obtain B007_8 (7 mg) as a crude product, which was directly used in the next step.

MS (ESI) M/Z: 583.3 [M-H]⁻.

### Step 4: Synthesis of compound B007

B007_8 (5 mg, 8.55 µmol) was dissolved in methanol (0.5 mL), followed by dropwise addition of 4 M HCl in methanol (2 mL). The reaction mixture was stirred at 20°C for 2 hours. The reaction was monitored by LCMS until completion. The reaction mixture was evaporated to dryness under a stream of nitrogen and purified by preparative chromatography (acidic conditions, chromatographic column: Phenomenex Luna C18) to obtain B007 (1.36 mg, hydrochloride salt).

MS (ESI) M/Z: 485.1 [M+H]⁺.

¹H NMR (400 MHz, DMSO-*d₆*): δ 9.02 (s, 2H), 7.93 (s, 1H), 7.69 - 7.54 (m, 4H), 7.14 (s, 1H), 3.65 - 3.62 (m, 4H), 3.35 - 3.31 (m, 4H), 1.03 (s, 3H), 0.63 - 0.61 (m, 2H), 0.37 - 0.34 (m, 2H).

### Step 5: Synthesis of compound B008

B007 (9 mg, 17.2 µmol) and triethylamine (20.9 mg, 207 µmol) were dissolved in N,N-dimethylformamide (0.75 mL). The mixture was cooled to 0°C, followed by addition of dimethylcarbamoyl chloride (2.2 mg, 20.7 µmol). After the addition was completed, the reaction mixture was slowly warmed to 20°C and stirred for 1 hour. The reaction was monitored by LCMS until completion. The reaction mixture was quenched with water (5 mL) and extracted with dichloromethane (5 mL × 2). The organic phase was washed with brine (5 mL × 1), dried over anhydrous sodium sulfate, filtered, and the filtrate was concentrated by rotary evaporation. The residue was purified by preparative chromatography (neutral conditions, chromatographic column: Waters Xbridge Prep OBD C18) to obtain B008 (6.53 mg).

MS (ESI) M/Z: 556.2 [M+H]⁺.

¹H NMR (400 MHz, DMSO-*d₆*): δ 7.89 (s, 1H), 7.82 - 7.53 (m, 2H), 7.49 (s, 2H), 7.12 (s, 1H), 3.43 - 3.41 (m, 4H), 3.33 - 3.30 (m, 4H), 2.79 (s, 6H), 1.01 (s, 3H), 0.62 - 0.59 (m, 2H), 0.35 - 0.32 (m, 2H).

### Example B009, Example B010, and Example B011

**Table 13**

| Example | Structural formula | Structural characterization |
|---|---|---|
| B009 | 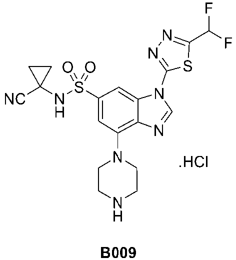 | **¹H NMR** (400 MHz, DMSO-*d*₆) δ 9.29 (s, 1H), 9.24 (s, 2H), 9.13 (s, 1H), 8.36 (s, 1H), 7.69 (t, *J =* 53.2 Hz, 1H), 7.30 (s, 1H), 3.89 - 3.86 (m, 4H), 3.28 - 3.25 (m, 4H), 1.44 - 1.40 (m, 2H), 1.33 - 1.29 (m, 2H). |
| | | **MS (ESI) M/Z:** 481.1 [M+H]⁺. |
| B010 | 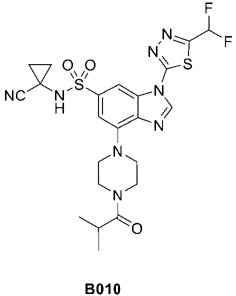 | **¹H NMR** (400 MHz, DMSO-*d₆*): δ 9.17 (s, 1H), 9.09 (s, 1H), 8.30 (d, *J* = 1.6 Hz, 1H), 7.68 (t, *J* = 53.2 Hz, 1H), 7.22 (d, *J =* 1.2 Hz, 1H), 3.81 - 3.69 (m, 4H), 3.67 - 3.54 (m, 4H), 3.01 - 2.89 (m, 1H), 1.44 - 1.37 (m, 2H), 1.31 - 1.24 (m, 2H), 1.04 (d*, J* = 6.4 Hz, 6H). |
| | | **MS (ESI) M/Z:** 551.1 [M+H]⁺. |
| B011 | 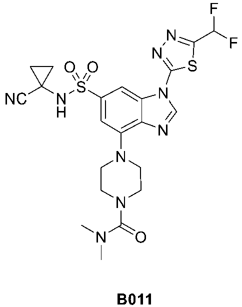 | **¹H NMR** (400 MHz, DMSO-*d₆*): δ 9.20 (s, 1H), 9.07 (s, 1H), 8.29 (d, *J* = 1.2 Hz, 1H), 7.68 (t, *J =* 52.8 Hz, 1H), 7.23 (s, 1H), 3.65 - 3.61 (m, 4H), 3.39 - 3.34 (m, 4H), 2.80 (s, 6H), 1.44 - 1.36 (m, 2H), 1.30 - 1.22 (m, 2H). |
| | | **MS (ESI) M/Z:** 552.0 [M+H]⁺. |

### Example C001 and Example C002

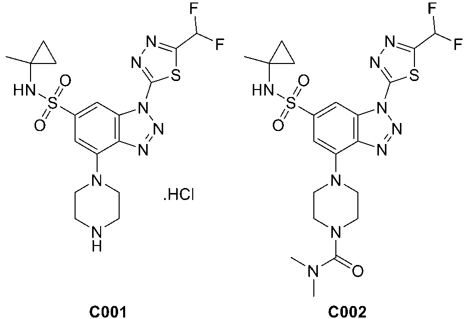

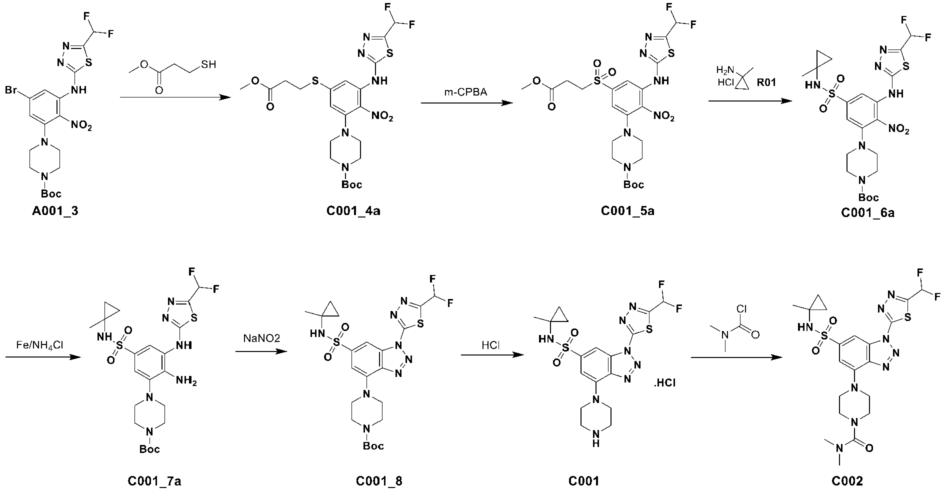

### Step 1: Synthesis of compound C001_4a

A001_3 (2.00 g, 3.74 mmol), methyl 3-mercaptopropionate (493 mg, 4.11 mmol), and N,N-diisopropylethylamine (1.55 g, 11.9 mmol) were dissolved in 1,4-dioxane (20 mL). The system was purged with nitrogen three times, followed by addition of Pd₂(dba)₃ (342 mg, 374 µmol, 0.10 eq) and Xantphos (432 mg, 747 µmol, 0.20 eq), and purged with nitrogen three additional times. The reaction mixture was heated to 85°C and stirred for 2 hours. Three identical parallel reactions were carried out and finally combined for work-up. The reaction mixture was cooled to room temperature, then quenched with water (200 mL), and extracted with ethyl acetate (100 mL × 3). The organic phase was washed with brine (100 mL), dried over anhydrous sodium sulfate, filtered, and the filtrate was concentrated by rotary evaporation. The residue was purified by column chromatography to obtain C001_4a (2.87 g).

MS (ESI) M/Z: 575.1 [M+H]⁺.

### Step 2: Synthesis of compound C001_5a

C001_4a (2.87 g, 4.99 mmol) was dissolved in dichloromethane (30 mL). The reaction mixture was cooled to 0°C, and *m*-chloroperoxybenzoic acid (3.04 g, 14.9 mmol, 85% purity) was added in portions. The reaction mixture was stirred at 0°C for 1 hour. The reaction was monitored by LCMS until completion. The reaction mixture was allowed to warm to room temperature, then quenched with saturated sodium sulfite aqueous solution (30 mL), and extracted with ethyl acetate (30 mL × 3). The organic phase was washed with brine (30 mL × 3), dried over anhydrous sodium sulfate, filtered, and the filtrate was concentrated by rotary evaporation. The residue was purified by column chromatography to obtain C001_5a (1.6 g).

MS (ESI) M/Z: 606.9 [M+H]⁺.

### Step 3: Synthesis of compound C001_6a

C001_5a (1.00 g, 1.65 mmol) was dissolved in methanol (30 mL). The system was purged with nitrogen three times, then cooled to 0°C, followed by addition of sodium methoxide (445 mg, 8.24 mmol). The reaction mixture was stirred at 0°C for 30 minutes, followed by addition of dichloromethane (20 mL), and stirred at room temperature for another 30 minutes. TLC indicated completion of the reaction. 1-Amino-1-cyclopropanecarbonitrile hydrochloride (709 mg, 6.59 mmol) was added at 0°C, and the mixture was stirred until the solid dissolved. The solvent was removed by rotary evaporation at room temperature, and the residue was dried under oil pump vacuum for 15 minutes. 3Å molecular sieves and N,N-dimethylformamide (30 mL) were added. Triethylamine (166 mg, 1.65 mmol) and N-chlorosuccinimide (660 mg, 4.95 mmol) were added in an ice bath. The reaction mixture was stirred at 0°C for 2 hours. The reaction mixture was extracted with ethyl acetate (30 mL × 3), washed once with dilute sodium bisulfite solution and once with water. The organic phase was washed with brine (20 mL × 2), dried over anhydrous sodium sulfate, filtered, and the filtrate was concentrated by rotary evaporation. The residue was purified by column chromatography to obtain C001_6a (550 mg).

MS (ESI) M/Z: 590.2 [M+H]⁺.

### Step 4: Synthesis of compound C001_7a

C001_6a (40.0 mg, 67.8 µmol) was dissolved in ethanol (0.3 mL) and water (0.1 mL), followed by addition of iron powder (18.9 mg, 339 µmol) and ammonium chloride (18.1 mg, 339 µmol). The reaction mixture was heated to 80°C and stirred for 1.5 hours. The reaction mixture was cooled to room temperature and filtered through diatomite. The filtrate was diluted with water (5 mL) and extracted with ethyl acetate (5 mL × 3). The organic phase was washed with brine (5 mL × 1), dried over anhydrous sodium sulfate, filtered, and the filtrate was concentrated by rotary evaporation. The residue was purified by column chromatography to obtain C001_7a (14 mg).

MS (ESI) M/Z: 558.2 [M-H]⁻.

### Step 5: Synthesis of compound C001_8

C001_7a (14.0 mg, 25.0 µmol) was dissolved in acetic acid (0.2 mL), followed by addition of sodium nitrite (2.42 mg, 35.0 µmol). The reaction mixture was stirred at 25°C for 1 hour. The reaction mixture was quenched with water (10 mL) and extracted with ethyl acetate (5 mL × 3). The organic phase was washed with brine (5 mL × 1), dried over anhydrous sodium sulfate, filtered, and the filtrate was concentrated by rotary evaporation to obtain C001_8 (10 mg) as a crude product, which was directly used in the next step.

MS (ESI) M/Z: 569.2 [M-H]⁻.

### Step 6: Synthesis of compound C001

C001_8 (10.0 mg, 17.5 µmol) was dissolved in ethyl acetate (0.5 mL), followed by addition of 4 M HCl in ethyl acetate (1 mL). The reaction mixture was stirred at 25°C for 1 hour. The reaction was monitored by TLC until completion. The reaction mixture was directly concentrated under reduced pressure to obtain C001 (9 mg, hydrochloride salt).

### Step 7: Synthesis of compound C002

C001 (9.00 mg, 17.7 µmol) and triethylamine (2.69 mg, 26.6 µmol) were dissolved in N,N-dimethylformamide (0.2 mL), followed by addition of dimethylcarbamoyl chloride (2.86 mg, 26.6 µmol). The reaction mixture was stirred at 25°C for 1 hour. The reaction was monitored by LCMS until completion. The reaction mixture was quenched with water (0.1 mL). The residue was purified by preparative chromatography (neutral conditions, chromatographic column: Waters Xbridge Prep OBD C18) to obtain C002 (2.08 mg).

MS (ESI) M/Z: 542.2 [M+H]⁺.

¹H NMR (400 MHz, DMSO-*d₆*): δ 8.39 (s, 1H), 8.19 (s, 1H), 7.68 (t, *J =* 52.8 Hz, 1H), 7.21 (s, 1H), 3.89 - 3.86 (m, 4H), 3.42 - 3.39 (m, 4H), 2.82 (s, 6H), 1.09 (s, 3H), 0.70 - 0.64 (m, 2H), 0.44 - 0.38 (m, 2H).

### Example D001 and Example D002

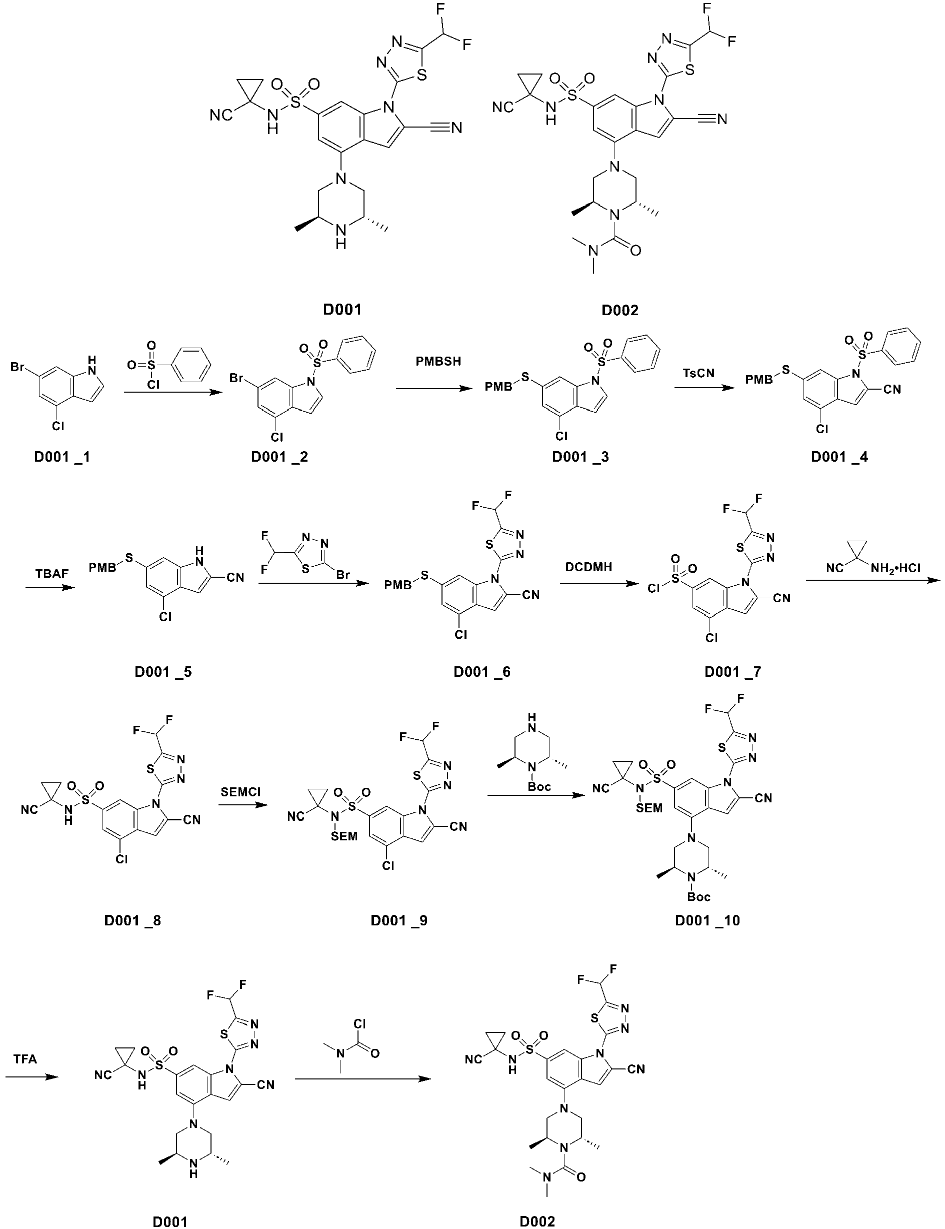

### Step 1: Synthesis of compound D001_2

D001_1 (4.30 g, 18.7 mmol), tetrabutylammonium bromide (601 mg, 1.87 mmol), and sodium hydroxide (1.34 g, 33.6 mmol) were dissolved in dichloromethane (50.0 mL) and H₂O (10 mL). The mixture was cooled to 0°C in an ice-water bath, followed by dropwise addition of benzenesulfonyl chloride (3.95 g, 22.4 mmol), and stirred at 0°C for 0.5 hours. The reaction mixture was quenched with water (50 mL) and extracted with dichloromethane (100 mL × 2). The organic phase was washed with brine (100 mL), dried over anhydrous sodium sulfate, filtered, and the filtrate was concentrated by rotary evaporation. The crude product was triturated in acetonitrile (10 mL), filtered, and concentrated to dryness to obtain D001_2 (6.5 g).

MS (ESI) M/Z: 367.9, 369.9 [M-H]⁻.

¹H NMR (400 MHz, DMSO-*d₆*): δ 8.07 - 8.05 (m, 3H), 8.01 (d, *J =* 4.0 Hz, 1H), 7.75 (t, *J =* 7.6 Hz, 1H), 7.66 - 7.61 (m, 3H), 6.88 (d, *J =* 3.6 Hz, 1H).

### Step 2: Synthesis of compound D001_3

D001_2 (5.00 g, 13.5 mmol), 4-methoxybenzyl mercaptan (2.08 g, 13.5 mmol), and N,N-diisopropylethylamine (3.57 g, 27.6 mmol) were dissolved in 1,4-dioxane (80 mL). The system was purged with nitrogen three times, followed by addition of Pd₂(dba)₃ (371 mg, 405 µmol) and Xantphos (468 mg, 809 µmol), and purged with nitrogen three additional times. The reaction mixture was heated to 110°C and stirred for 1 hour. The reaction mixture was cooled to room temperature, filtered, and the filtrate was concentrated by rotary evaporation. The residue was purified by column chromatography to obtain D001_3 (5.70 g).

MS (ESI) M/Z: 442.1, 444.1 [M-H]⁻.

¹H NMR (400 MHz, CDCl₃-*d*): δ 7.88 (s, 1H), 7.75 (d, *J =* 7.2 Hz, 2H), 7.56 - 7.53 (m, 2H), 7.44 (t, *J =* 8.4 Hz, 2H), 7.27 (d, *J =* 8.8 Hz, 2H), 7.20 (s, 1H), 6.85 (d, *J =* 8.8 Hz, 2H), 6.72 (s, 1H), 4.15 (s, 2H), 3.80 (s, 3H).

### Step 3: Synthesis of compound D001_4

Lithium diisopropylamide (2 M in tetrahydrofuran, 9.29 mL) was added dropwise to a solution of D001_3 (5.50 g, 12.4 mmol) in tetrahydrofuran (60 mL) at -60°C. After the addition was completed, the reaction mixture was stirred at -60°C for 0.5 hours. A solution of 4-toluenesulfonyl cyanide (2.92 g, 16.1 mmol) in tetrahydrofuran (10 mL) was then added dropwise thereto. After the addition was completed, the reaction mixture was warmed to - 40°C and stirred for another 1 hour. The reaction mixture was quenched at room temperature with saturated ammonium chloride aqueous solution (100 mL) and extracted with ethyl acetate (100 mL × 2). The organic phase was washed with brine (100 mL), dried over anhydrous sodium sulfate, filtered, and the filtrate was concentrated by rotary evaporation. The residue was purified by column chromatography to obtain D001_4 (2.10 g).

MS (ESI) M/Z: 466.9, 468.7 [M-H]⁻.

¹H NMR (400 MHz, CDCl₃-*d*): δ 7.99 (s, 1H), 7.82 (d, *J =* 8.4 Hz, 2H), 7.61 (t, *J =* 7.6 Hz, 1H), 7.48 (t, *J =* 8.4 Hz, 2H), 7.39 (s, 1H), 7.37 (d, *J =* 6.4 Hz, 2H), 7.26 (s, 1H), 6.89 (d, *J =* 8.8 Hz, 2H), 4.25 (s, 2H), 3.80 (s, 3H).

### Step 4: Synthesis of compound D001_5

D001_4 (1.00 g, 2.13 mmol) was dissolved in tetrahydrofuran (10 mL), followed by addition of tetrabutylammonium fluoride (1 M in tetrahydrofuran, 10.7 mL). The reaction mixture was heated to 50°C and stirred for 0.5 hours. The reaction mixture was cooled to room temperature, quenched with dilute hydrochloric acid aqueous solution (0.3 N, 30 mL), and extracted with ethyl acetate (20 mL × 2). The organic phase was washed with water (50 mL), dried over anhydrous sodium sulfate, filtered, and concentrated to obtain D001_5 (700 mg).

MS (ESI) M/Z: 326.9, 328.8 [M-H]⁻.

¹H NMR (400 MHz, CDCl₃-*d*): δ 11.26 (s, 1H), 7.43 (s, 1H), 7.18 (d, *J =* 8.8 Hz, 2H), 7.13 (s, 1H), 7.10 (s, 1H), 6.79 (d, *J =* 8.8 Hz, 2H), 4.04 (s, 2H), 3.77 (s, 3H).

### Step 5: Synthesis of compound D001_6

D001_5 (1.40 g, 4.26 mmol), 2-bromo-5-(difluoromethyl)-1,3,4-thiadiazole (1.10 g, 5.11 mmol), and cesium carbonate (1.39 g, 4.26 mmol) were dissolved in N,N-dimethylformamide (15 mL). The reaction mixture was heated to 80°C and stirred for 1 hour. The reaction mixture was cooled to room temperature, quenched with ice water (50 mL), and extracted with ethyl acetate (50 mL × 2). The organic phase was washed with brine (100 mL), dried over anhydrous sodium sulfate, filtered, and the filtrate was concentrated by rotary evaporation. The residue was purified by column chromatography to obtain D001_6 (1.60 g).

MS (ESI) M/Z: 462.9, 464.8 [M+H]⁺.

¹H NMR (400 MHz, CDCl₃-*d*): δ 8.13 (s, 1H), 7.58 (s, 1H), 7.33 (s, 1H), 7.30 (d, *J* = 8.8 Hz, 2H), 7.08 (t, *J =* 53.6 Hz, 1H), 6.84 (d, *J =* 8.8 Hz, 2H), 4.21 (s, 2H), 3.78 (s, 3H).

### Step 6: Synthesis of compound D001_7

D001_6 (1.00 g, 2.16 mmol), acetic acid (1 mL), and water (0.5 mL) were dissolved in acetonitrile (30 mL). The mixture was cooled to -15°C, and 1,3-dichloro-5,5-dimethylhydantoin (638 mg, 3.24 mmol) was added in portions. The resulting mixture was then stirred at the same temperature for 1 hour. The reaction was monitored by LCMS until completion. The reaction mixture was quenched with water (50 mL) and extracted with dichloromethane (50 mL × 2). The organic phase was washed with brine (100 mL), dried over anhydrous sodium sulfate, filtered, and the filtrate was concentrated by rotary evaporation to obtain D001_7 (883 mg).

MS (ESI) M/Z: 389.0, 390.9. (The sulfonyl chloride of D001_7 appears as the corresponding sulfonic acid in LCMS).

### Step 7: Synthesis of compound D001_8

1-Aminocyclopropanecarbonitrile hydrochloride (512 mg, 4.32 mmol) and pyridine (683 mg, 8.63 mmol) were dissolved in N,N-dimethylformamide (10 mL). The reaction mixture was cooled to 0°C in an ice-water bath. A solution of D001_7 (883 mg, 2.16 mmol) in dichloromethane (10 mL) was added dropwise thereto at 0°C. The resulting mixture was then stirred at 0°C for 20 minutes. The reaction mixture was quenched with water (50 mL) and extracted with dichloromethane (50 mL × 2). The organic phase was washed with water (100 mL), dried over anhydrous sodium sulfate, filtered, and the filtrate was concentrated by rotary evaporation. The residue was purified by column chromatography to obtain D001_8 (530 mg).

MS (ESI) M/Z: 455.0, 457.0 [M+H]⁺.

¹H NMR (400 MHz, CDCl₃-*d*): δ 8.94 (s, 1H), 8.00 (s, 1H), 7.75 (s, 1H), 7.09 (t, *J =* 53.2 Hz, 1H), 5.74 (s, 1H), 1.68 - 1.64 (m, 2H), 1.52 - 1.48 (m, 2H).

### Step 8: Synthesis of compound D001_9

D001_8 (200 mg, 440 µmol) and cesium carbonate (683 mg, 8.63 mmol) were dissolved in N,N-dimethylformamide (5 mL). 2-(Trimethylsilyl)ethoxymethyl chloride (88.0 mg, 528 µmol) was added dropwise thereto at 25°C. The reaction mixture was stirred for 1 hour. The reaction mixture was quenched with ice water (10 mL) and extracted with ethyl acetate (10 mL × 2). The organic phase was washed with brine (10 mL), dried over anhydrous sodium sulfate, filtered, and the filtrate was concentrated by rotary evaporation. The residue was purified by column chromatography to obtain D001_9 (250 mg).

MS (ESI) M/Z: 583.1, 585.1 [M-H]⁻.

¹H NMR (400 MHz, CDCl₃-*d*): δ 8.95 (s, 1H), 8.02 (s, 1H), 7.73 (s, 1H), 7.09 (t, *J =* 53.6 Hz, 1H), 4.91 (s, 2H), 3.61 - 3.56 (m, 2H), 1.78 - 1.74 (m, 2H), 1.60 - 1.56 (m, 2H), 0.94 - 0.89 (m, 2H), 0.00 (s, 9H).

### Step 9: Synthesis of compound D001_10

D001_9 (50.0 mg, 85.5 µmol), *tert-butyl* (2S,6S)-2,6-dimethylpiperazine-1-carboxylate (91.6 mg, 427 µmol), and potassium carbonate (35.4 mg, 256 µmol) were dissolved in 1,4-dioxane (4 mL). The system was purged with nitrogen three times, followed by addition of RuPhos Pd G3 (35.7 mg, 42.7 µmol) and RuPhos (19.9 mg, 42.7 µmol), and purged with nitrogen three additional times. The reaction mixture was heated to 90°C and stirred for 16 hours. Six parallel reactions were carried out and finally combined for purification. After cooling to room temperature, the six parallel reactions were combined. The reaction mixture was directly filtered, and the filtrate was concentrated by rotary evaporation. The residue was purified by column chromatography to obtain D001_10 (100 mg) as a crude product.

MS (ESI) M/Z: 761.1 [M-H]⁻.

### Step 10: Synthesis of compound D001

D001_10 (10.0 mg, 13.1 µmol) was dissolved in dichloromethane (1.5 mL). Trifluoroacetic acid (0.5 mL) was added thereto at 25°C. The reaction mixture was stirred at 25°C for 2 hours. The reaction was monitored by LCMS until completion. The reaction mixture was concentrated under reduced pressure at 25°C. The crude product was purified by preparative chromatography (neutral conditions, column: Waters Xbridge Prep OBD C18) to obtain D001 (3.11 mg).

MS (ESI) M/Z: 533.1 [M+H]⁺.

¹H NMR (400 MHz, DMSO-*d₆*): δ 8.16 - 8.12 (m, 2H), 7.69 (t, *J =* 53.2 Hz, 1H), 7.20 (s, 1H), 3.65 - 3.50 (m, 2H), 3.38 - 3.35 (m, 2H), 3.05 - 3.01 (m, 2H), 1.44 - 1.40 (m, 2H), 1.29 - 1.27 (m, 6H), 1.24 - 1.23 (m, 2H).

### Step 11: Synthesis of compound D002

D001 (12.0 mg, 22.5 µmol) and triethylamine (3.42 mg, 33.8 µmol, 1.50 eq) were dissolved in N,N-dimethylformamide (2 mL). Dimethylcarbamoyl chloride (5.75 mg, 40.5 µmol, 1.50 eq) was added in one portion at 20°C. After the addition was completed, the reaction mixture was heated to 40°C and stirred for 36 hours. After completion of the reaction monitored by LCMS, the reaction mixture was quenched with water (0.1 mL). The crude product was purified by preparative chromatography (neutral conditions, column: Waters Xbridge Prep OBD C18) to obtain D002 (4.82 mg).

MS (ESI) M/Z: 604.0 [M+H]⁺.

¹H NMR (400 MHz, DMSO-*d₆*): δ 9.21 (s, 1H), 8.16 (s, 2H), 7.69 (t, *J =* 53.2 Hz, 1H), 7.21 (s, 1H), 3.66 - 3.64 (m, 2H), 3.41 - 3.37 (m, 2H), 3.09 - 3.04 (m, 2H), 2.91 (s, 6H), 1.44 - 1.41 (m, 2H), 1.30 - 1.28 (m, 2H), 1.19 - 1.15 (m, 6H).

### Example F001 and Example F002

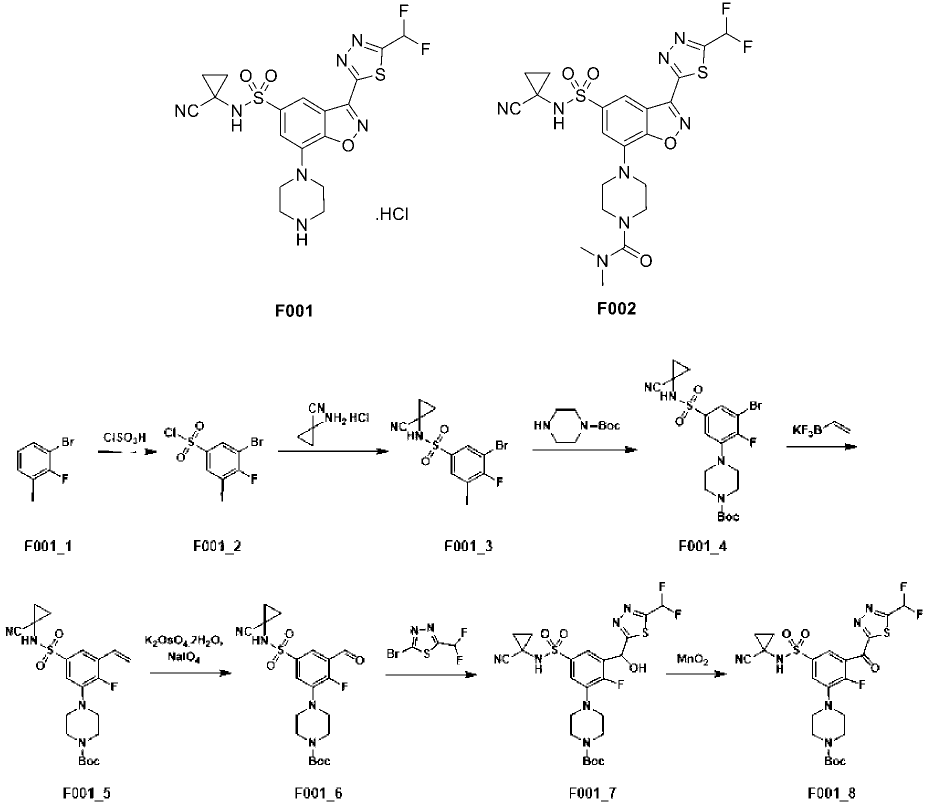

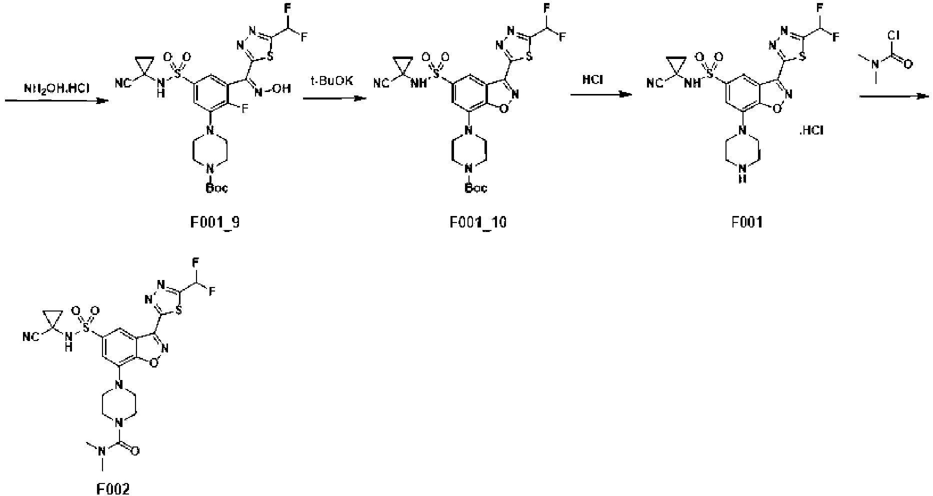

### Step 1: Synthesis of compound F001_2

Chlorosulfonic acid (58.1 g, 499 mmol) was added to a three-necked flask, cooled to 0°C in an ice-salt bath, and F001_1 (30.0 g, 99.7 mmol) was added in portions. The reaction mixture was stirred at 0°C for 0.5 hours. Sodium chloride (5.83 g, 99.7 mmol) was then added in portions. The reaction mixture was slowly warmed to 20°C and stirred for 0.5 hours, then slowly heated to 90°C and stirred for 2 hours. The reaction mixture was cooled to room temperature, then slowly poured into ice water (300 mL) to quench, and extracted with ethyl acetate (200 mL × 3). The organic phase was washed with water (200 mL), dried over anhydrous sodium sulfate, filtered, and the filtrate was concentrated by rotary evaporation. The residue was purified by column chromatography to obtain F001_2 (14.9 g).

MS (ESI) M/Z: 379.0, 381.1 [M-H]⁻. (The sulfonyl chloride of F001_2 appears as the corresponding sulfonic acid in LCMS).

¹H NMR (400 MHz, DMSO-*d₆*): δ 7.93 - 7.91 (m, 1H), 7.77 - 7.75 (m, 1H).

### Step 2: Synthesis of compound F001_3

1-Aminocyclopropanecarbonitrile hydrochloride (6.63 g, 56.0 mmol) was dissolved in dichloromethane (30 mL) and pyridine (41 mL), followed by portionwise addition of 031_2 (14.9 g, 37.3 mmol). The reaction mixture was stirred at 20°C for 1 hour. The reaction mixture was slowly poured into ice water (100 mL) to quench, and extracted with ethyl acetate (100 mL × 3). The organic phase was sequentially washed with 1 N hydrochloric acid aqueous solution (100 mL), brine (100 mL), saturated sodium bicarbonate aqueous solution (100 mL), and brine (100 mL × 2), dried over anhydrous sodium sulfate, filtered, and the filtrate was concentrated by rotary evaporation. The residue was triturated with petroleum ether (100 mL) at room temperature, filtered, and the filter cake was collected to obtain F001_3 (15.4 g).

MS (ESI) M/Z: 443.0, 445.0 [M-H]⁻.

¹H NMR (400 MHz, DMSO-*d₆*): δ 9.33 (s, 1H), 8.22 - 8.20 (m, 1H), 8.11 - 8.09 (m, 1H), 1.50 - 1.47 (m, 2H), 1.34 - 1.32 (m, 2H).

### Step 3: Synthesis of compound F001_4

F001_3 (4.00 g, 8.99 mmol), N-Boc piperazine (3.35 g, 18.0 mmol), and cesium carbonate (7.32 g, 22.5 mmol) were dissolved in 1,4-dioxane (20 mL). The system was purged with nitrogen three times, followed by addition of XPhos Pd G2 (1.41 g, 1.80 mmol), and purged with nitrogen three additional times. The reaction mixture was heated to 110°C and stirred for 5 hours. Five parallel reactions were carried out and finally combined for purification. After cooling to room temperature, the five parallel reactions were combined. The reaction mixture was quenched with water (200 mL) and extracted with ethyl acetate (100 mL × 3). The organic phase was washed with brine (100 mL), dried over anhydrous sodium sulfate, filtered, and the filtrate was concentrated by rotary evaporation. The residue was purified by column chromatography to obtain F001_4 (2.6 g).

MS (ESI) M/Z: 501.0, 503.2 [M-H]⁻.

### Step 4: Synthesis of compound F001_5

F001_4 (2.5 g, 4.97 mmol), potassium vinyltrifluoroborate (4.17 g, 31.1 mmol), and triethylamine (1.51 g, 14.9 mmol) were dissolved in isopropanol (30 mL). The system was purged with nitrogen three times, followed by addition of Pd(dppf)Cl₂·CH₂Cl₂ (811 mg, 993 µmol), and purged with nitrogen three additional times. The reaction mixture was heated to 100°C and stirred for 2 hours. The reaction mixture was cooled to room temperature, then quenched with water (200 mL), and extracted with ethyl acetate (50 mL × 3). The organic phase was washed with brine (100 mL), dried over anhydrous sodium sulfate, filtered, and the filtrate was concentrated by rotary evaporation. The residue was purified by column chromatography to obtain F001_5 (1.4 g).

MS (ESI) M/Z: 449.4 [M-H]⁻.

### Step 5: Synthesis of compound F001_6

Sodium periodate (2.53 g, 11.8 mmol) and potassium osmate dihydrate (229 mg, 622 µmol) were added to a solution of F001_5 (8.00 g, 19.8 mmol) in tetrahydrofuran (50 mL) and water (25 mL). The reaction mixture was stirred at 20°C for 2 hours. The reaction mixture was quenched with water (100 mL) and extracted with ethyl acetate (50 mL × 3). The organic phase was washed with brine (50 mL × 3), dried over anhydrous sodium sulfate, filtered, and the filtrate was concentrated by rotary evaporation. The residue was purified by column chromatography to obtain F001_6 (1.6 g).

MS (ESI) M/Z: 451.3 [M-H]⁻.

¹H NMR (400 MHz, DMSO-*d₆*): δ 10.27 (s, 1H), 9.24 (s, 1H), 7.84 - 7.82 (m, 1H), 7.70 - 7.68 (m, 1H), 3.53 - 3.50 (m, 4H), 3.12 - 3.09 (m, 4H), 1.47 - 1.45 (m, 2H), 1.43 (s, 9H), 1.33 - 1.29 (m, 2H).

### Step 6: Synthesis of compound F001_7

2-Bromo-5-(difluoromethyl)-1,3,4-thiadiazole (7.13 g, 33.2 mmol) was dissolved in tetrahydrofuran (10 mL). The system was purged with nitrogen three times, then cooled to - 60°C, and isopropylmagnesium chloride-lithium chloride complex (1.3 M in tetrahydrofuran, 25.5 mL) was slowly added dropwise thereto. After the addition was completed, the reaction mixture was stirred at -60°C for 2 hours. A solution of F001_6 (1.5 g, 3.31 mmol) in tetrahydrofuran (10 mL) was then added dropwise thereto. After the addition was completed, the reaction mixture was naturally warmed to 20°C and stirred for another 16 hours. The reaction mixture was quenched in an ice bath with saturated ammonium chloride aqueous solution (100 mL) and extracted with ethyl acetate (100 mL × 2). The organic phase was washed with brine (100 mL), dried over anhydrous sodium sulfate, filtered, and the filtrate was concentrated by rotary evaporation. The residue was purified by column chromatography to obtain F001_7 (870 mg).

MS (ESI) M/Z: 587.2 [M-H]⁻.

### Step 7: Synthesis of compound F001_8

F001_7 (870 mg, 1.48 mmol) was dissolved in dichloromethane (30 mL), followed by addition of manganese dioxide (2.57 g, 29.6 mmol). The reaction mixture was stirred at 20°C for 1 hour. The reaction mixture was filtered through diatomite. The filtrate was diluted with water (100 mL) and extracted with ethyl acetate (50 mL × 3). The organic phase was washed with brine (50 mL × 1), dried over anhydrous sodium sulfate, filtered, and the filtrate was concentrated by rotary evaporation. The residue was purified by column chromatography to obtain F001_8 (540 mg).

MS (ESI) M/Z: 585.2 [M-H]⁻.

### Step 8: Synthesis of compound F001_9

F001_8 (150 mg, 256 µmol) was dissolved in ethanol (2 mL), followed by addition of hydroxylamine hydrochloride (88.9 mg, 1.28 mmol). The reaction mixture was heated to 80°C and stirred for 1 hour. The reaction mixture was cooled to room temperature and directly concentrated under reduced pressure to obtain a crude product. The residue was purified by column chromatography to obtain F001_9 (70 mg).

MS (ESI) M/Z: 600.2 [M-H]⁻.

### Step 9: Synthesis of compound F001_10

F001_9 (65 mg, 108 µmol) was dissolved in N-methylpyrrolidone (11 mL), followed by addition of potassium tert-butoxide (18.2 mg, 162 µmol). The reaction mixture was heated to 50°C and stirred for 16 hours. The reaction mixture was cooled to room temperature, then quenched with water (50 mL), and extracted with ethyl acetate (20 mL × 3). The organic phase was washed with brine (30 mL × 3), dried over anhydrous sodium sulfate, filtered, and the filtrate was concentrated by rotary evaporation. The residue was purified by column chromatography to obtain F001_10 (30 mg).

MS (ESI) M/Z: 580.2 [M-H]⁻.

### Step 10: Synthesis of compound F001

F001_10 (15 mg, 25.8 µmol) was dissolved in ethyl acetate (1 mL), followed by addition of 4 M HCl in ethyl acetate (2 mL). The reaction mixture was stirred at 20°C for 1 hour. The reaction mixture was directly concentrated under reduced pressure to obtain F001 (13 mg, hydrochloride salt) as a crude product, which was directly used in the next step.

MS (ESI) M/Z: 480.2 [M-H]⁻.

### Step 11: Synthesis of compound F002

F001 (13 mg, 25.1 µmol, hydrochloride salt) and triethylamine (5.08 mg, 50.2 µmol) were dissolved in N,N-dimethylformamide (1 mL), followed by addition of dimethylcarbamoyl chloride (5.40 mg, 50.2 µmol). The reaction mixture was stirred at 25°C for 1 hour. The reaction mixture was quenched with water (0.1 mL). The residue was purified by preparative chromatography (neutral conditions, chromatographic column: Waters Xbridge Prep OBD C18) to obtain F001 (7.29 mg).

MS (ESI) M/Z: 553.1 [M+H]⁺.

¹H NMR (400 MHz, DMSO-*d₆*): δ 9.28 (s, 1H), 8.37 (d, *J =* 1.6 Hz, 1H), 7.77 (t,*J* = 52.8 Hz, 1H), 7.51 (d, *J =* 1.6 Hz, 1H), 3.50 - 3.48 (m, 4H), 3.40 - 3.37 (m, 4H), 2.81 (s, 6H), 1.46 - 1.43 (m, 2H), 1.34 - 1.30 (m, 2H).

### Example 065M

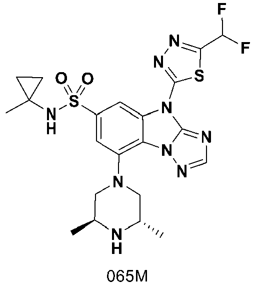

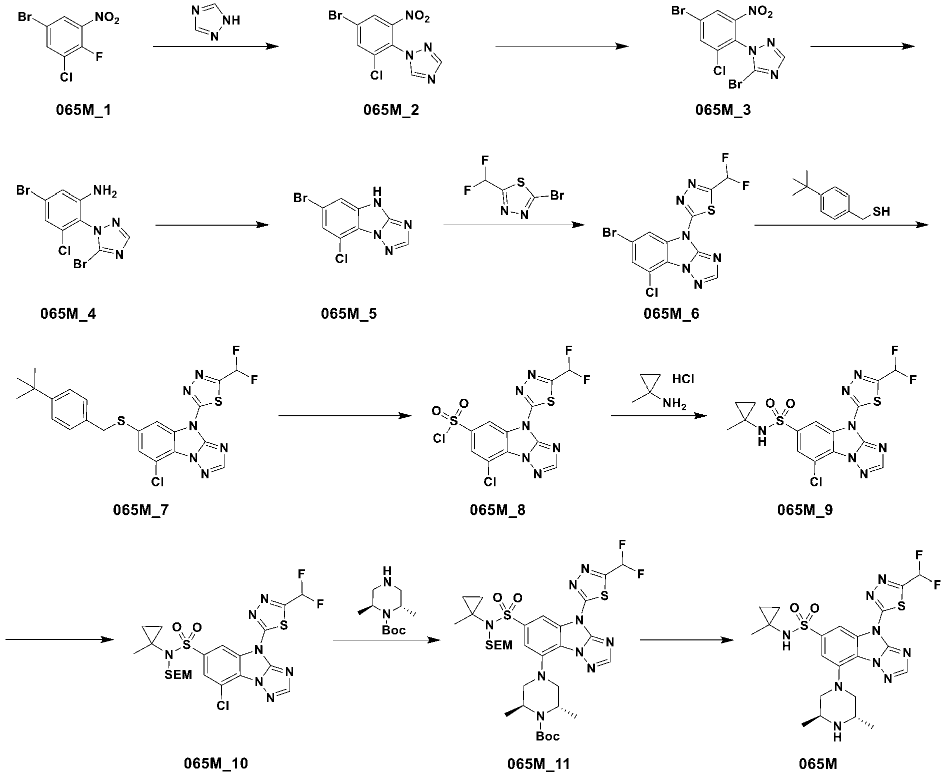

### Step 1: Synthesis of compound 065M_2

065M_1 (5.00 g, 19.7 mmol), 1,2,4-triazole (1.63 g, 23.6 mmol), and potassium carbonate (4.07 g, 29.5 mmol) were dissolved in dimethyl sulfoxide (50 mL). The mixture was heated to 60°C and stirred for 1 hour. The reaction mixture was cooled to room temperature, then quenched with water (150 mL), and extracted with ethyl acetate (100 mL × 2). The combined organic phases were washed with brine (100 mL × 3), dried over anhydrous sodium sulfate, filtered, and concentrated under reduced pressure to obtain 065M_2 (5.60 g).

MS (ESI) M/Z: 302.7, 304.7 [M+H]⁺.

¹H NMR (400 MHz, DMSO-*d₆*): δ 9.07 (s, 1H), 8.56 (s, 1H), 8.53 (s, 1H), 8.35 (s, 1H).

### Step 2: Synthesis of compound 065M_3

065M_2 (5.60 g, 18.5 mmol), N-bromosuccinimide (6.57 g, 36.9 mmol), and benzoyl peroxide (447 mg, 1.85 mmol) were dissolved in chlorobenzene (80 mL). The reaction mixture was heated to 110°C and stirred for 16 hours. The reaction mixture was cooled to room temperature, then quenched with water (100 mL), and extracted with dichloromethane (100 mL × 2). The combined organic phases were washed with saturated brine (100 mL × 1), dried over anhydrous sodium sulfate, filtered, and the filtrate was concentrated by rotary evaporation. The residue was purified by column chromatography to obtain 065M_3 (5.35 g).

¹H NMR (400 MHz, DMSO-*d₆*): δ 8.67 (s, 1H), 8.60 (d, *J =* 2.4 Hz, 1H), 8.44 (s, 1H).

### Step 3: Synthesis of compound 065M_4

065M_3 (5.35 g, 14.0 mmol) and ammonium chloride (3.74 g, 70.0 mmol) were dissolved in ethanol (80 mL) and water (10 mL). The reaction mixture was heated to 75°C, followed by portionwise addition of iron powder (2.34 g, 42.0 mmol). The resulting mixture was stirred at 75°C for 1 hour. The reaction mixture was filtered through diatomite while hot. The filtrate was concentrated by rotary evaporation to remove most of the ethanol, followed by addition of ethyl acetate (300 mL). The resulting mixture was washed with water (200 mL × 1) and saturated brine (200 mL × 1), dried over anhydrous sodium sulfate, filtered, and the filtrate was concentrated by rotary evaporation to obtain 065M_4 (4.70 g) as a crude product, which was directly used in the next step without further purification.

MS (ESI) M/Z: 350.7, 352.7, 354.7 [M+H]⁺.

¹H NMR (400 MHz, DMSO-*d₆*): δ 8.32 (s, 1H), 7.00 (d, *J =* 1.6 Hz, 1H), 6.96 (d, *J =* 1.6 Hz, 1H), 5.96 (s, 2H).

### Step 4: Synthesis of compound 065M_5

065M_4 (4.70 g, 13.3 mmol) and cesium carbonate (13.0 g, 40.0 mmol) were dissolved in N,N-dimethylacetamide (60.0 mL). The reaction mixture was heated to 110°C and stirred for 16 hours. The reaction mixture was cooled to room temperature, quenched with water (200 mL), added with dilute hydrochloric acid (4 N) to adjust the pH to approximately neutral, and extracted with ethyl acetate (100 mL × 2). The combined organic phases were washed with brine (100 mL × 1), dried over anhydrous sodium sulfate, filtered, and concentrated. The crude product was purified by triturating with ethyl acetate (10 mL) to obtain 065M_5 (3.60 g).

MS (ESI) M/Z: 270.7, 272.7 [M+H]⁺.

¹H NMR (400 MHz, DMSO-*d₆*): δ 12.90 (s, 1H), 8.17 (s, 1H), 7.69 (d, *J* = 1.2 Hz, 1H), 7.60 (s, 1H).

### Step 5: Synthesis of compound 065M_6

065M_5 (3.60 g, 13.3 mmol), 2-bromo-5-(difluoromethyl)-1,3,4-thiadiazole (4.28 g, 19.9 mmol), and cesium carbonate (6.48 g, 19.9 mmol) were dissolved in N,N-dimethylformamide (40.0 mL). The reaction mixture was heated to 80°C and stirred for 16 hours. The reaction mixture was cooled to room temperature, quenched with water (80 mL), resulting in precipitation of a large amount of solid, followed by filtration. The filter cake was washed with water and dried under reduced pressure. The residue was triturated with ethyl acetate (50 mL) at room temperature and filtered to obtain 065M_6 (3.50 g).

MS (ESI) M/Z: 405.1, 407.0 [M+H]⁺.

¹H NMR (400 MHz, DMSO-*d₆*): δ 8.73 (s, 1H), 8.50 (s, 1H), 8.03 (d, *J =* 1.2 Hz, 1H), 7.67 (t, *J =* 53.2 Hz, 1H).

### Step 6: Synthesis of compound 065M_7

065M_6 (3.30 g, 8.14 mmol), 4-tert-butylbenzyl mercaptan (2.20 g, 12.2 mmol), and N,N-diisopropylethylamine (3.15 g, 24.4 mmol) were dissolved in 1,4-dioxane (100 mL). The system was purged with nitrogen three times, followed by addition of Pd₂(dba)₃ (373 mg, 407 µmol) and Xantphos (471 mg, 814 µmol), and purged with nitrogen three additional times. The reaction mixture was heated to 90°C and stirred for 1 hour. 200 mg batches were combined for work-up. The reaction mixture was cooled to room temperature and filtered through diatomite. The filtrate was concentrated by rotary evaporation. The crude product was purified by column chromatography to obtain 065M_7 (4.4 g).

MS (ESI) M/Z: 505.0 [M+H]⁺.

¹H NMR (400 MHz, DMSO-*d₆*): δ 8.47 (s, 1H), 8.43 (s, 1H), 7.66 (t, *J =* 53.2 Hz, 1H), 7.65 (d, *J =* 1.2 Hz, 1H), 7.34 - 7.33 (m, 4H), 4.37 (s, 2H), 1.23 (s, 9H).

### Step 7: Synthesis of compound 065M_8

065M_7 (400 mg, 792 µmol) was dissolved in acetic acid (1 mL), water (1 mL), and acetonitrile (10 mL). The mixture was cooled to 0°C, and 1,3-dichloro-5,5-dimethylhydantoin (281 mg, 1.43 mmol) was added in portions. After the addition was completed, the reaction mixture was warmed to 20°C and stirred for 1 hour. The reaction mixture was quenched with water (50 mL) and extracted with dichloromethane (50 mL × 2). The combined organic phases were washed with brine (50 mL × 1), dried over anhydrous sodium sulfate, filtered, and concentrated under reduced pressure at room temperature to obtain 065M_8 (336 mg) as a crude product, which was directly used in the next step without further purification.

MS (ESI) M/Z: 404.9, 406.9 [M-Cl+OH-H]⁻.

### Step 8: Synthesis of compound 065M_9

1-Methylcyclopropanamine hydrochloride (170 mg, 1.58 mmol) and N,N-diisopropylethylamine (306 mg, 2.37 mmol) were dissolved in dichloromethane (10.0 mL). A solution of 065M_8 (336 mg, 790 µmol) in dichloromethane (5 mL) was added dropwise to the reaction mixture. After the addition was completed, the reaction mixture was stirred at 20°C for 20 minutes. The reaction mixture was quenched with water (10 mL) and extracted with dichloromethane (10 mL × 2). The organic phase was washed with saturated brine (20 mL × 1), dried over anhydrous sodium sulfate, filtered, and concentrated. The residue was purified by column chromatography to obtain 065M_9 (320 mg).

MS (ESI) M/Z: 460.2 [M+H]⁺.

¹H NMR (400 MHz, DMSO-*d₆*): δ 9.02 (d, *J* = 1.2 Hz, 1H), 8.55 (s, 1H), 8.52 (s, 1H), 8.02 (d, *J* = 1.2 Hz, 1H), 7.69 (t, *J* = 53.2 Hz, 1H), 1.13 (s, 3H), 0.65 - 0.64 (m, 2H), 0.45 - 0.42 (m, 2H).

### Step 9: Synthesis of compound 065M_10

065M_9 (320 mg, 696 µmol) and N,N-diisopropylethylamine (270 mg, 2.09 mmol) were dissolved in dichloromethane (8.00 mL). 2-(Trimethylsilyl)ethoxymethyl chloride (SEM-Cl) (139 mg, 835 µmol) was added in one portion at 20°C. The reaction mixture was stirred at 20°C for 30 minutes. The reaction mixture was quenched with water (20 mL) and extracted with dichloromethane (20 mL × 2). The organic phase was washed with brine (30 mL × 1), dried over anhydrous sodium sulfate, filtered, and concentrated under reduced pressure. The residue was purified by column chromatography to obtain 065M_10 (230 mg).

MS (ESI) M/Z: 471.9 [M+H-118]⁺.

¹H NMR (400 MHz, DMSO-*d₆*): δ 9.07 (d, *J =* 1.6 Hz, 1H), 8.55 (s, 1H), 8.09 (s, 1H), 7.66 (t, *J* = 53.2 Hz, 1H), 4.91 (s, 2H), 3.55 (t, *J* = 8.0 Hz, 2H), 1.27 (s, 3H), 0.93 - 0.86 (m, 4H), 0.61 - 0.59 (m, 2H), -0.03 (s, 9H).

### Step 10: Synthesis of compound 065M_11

065M_10 (220 mg, 373 µmol), *tert-butyl* (2S,6S)-2,6-dimethylpiperazine-1-carboxylate (240 mg, 1.12 mmol), and potassium carbonate (155 mg, 1.12 mmol) were dissolved in 1,4-dioxane (8 mL). The system was purged with nitrogen three times, followed by addition of RuPhos (69.6 mg, 149 µmol) and RuPhos Pd G3 (125 mg, 149 µmol), and purged with nitrogen three additional times. The reaction mixture was heated to 90°C and stirred for 3 hours. The reaction mixture was cooled to room temperature and filtered through diatomite. The filtrate was concentrated by rotary evaporation. The residue was purified by column chromatography to obtain 065M_11 (130 mg) as a crude product.

MS (ESI) M/Z: 650.1 [M+H-118]⁺.

### Step 11: Synthesis of compound 065M

065M_11 (130 mg, 169 µmol) was dissolved in dichloromethane (3 mL), followed by dropwise addition of trifluoroacetic acid (0.5 mL). The reaction mixture was stirred at 20°C for 1 hour. After concentration at room temperature, the residue was purified by preparative chromatography to obtain 065M (34.27 mg).

MS (ESI) M/Z: 538.3 [M+H]⁺.

¹H NMR (400 MHz, DMSO-*d₆*): δ 8.72 (d, *J =* 1.2 Hz, 1H), 8.48 (s, 1H), 8.33 (s, 1H), 7.68 (t, *J =* 53.2 Hz, 1H), 7.47 (d, *J* = 1.2 Hz, 1H), 3.41 - 3.32 (m, 4H), 2.89 - 2.85 (m, 2H), 1.19 (d, *J =* 6.8 Hz, 6H), 1.09 (s, 3H), 0.67 - 0.64 (m, 2H), 0.42 - 0.39 (m, 2H).

### Example 065R

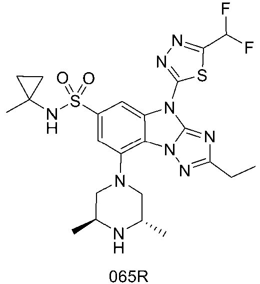

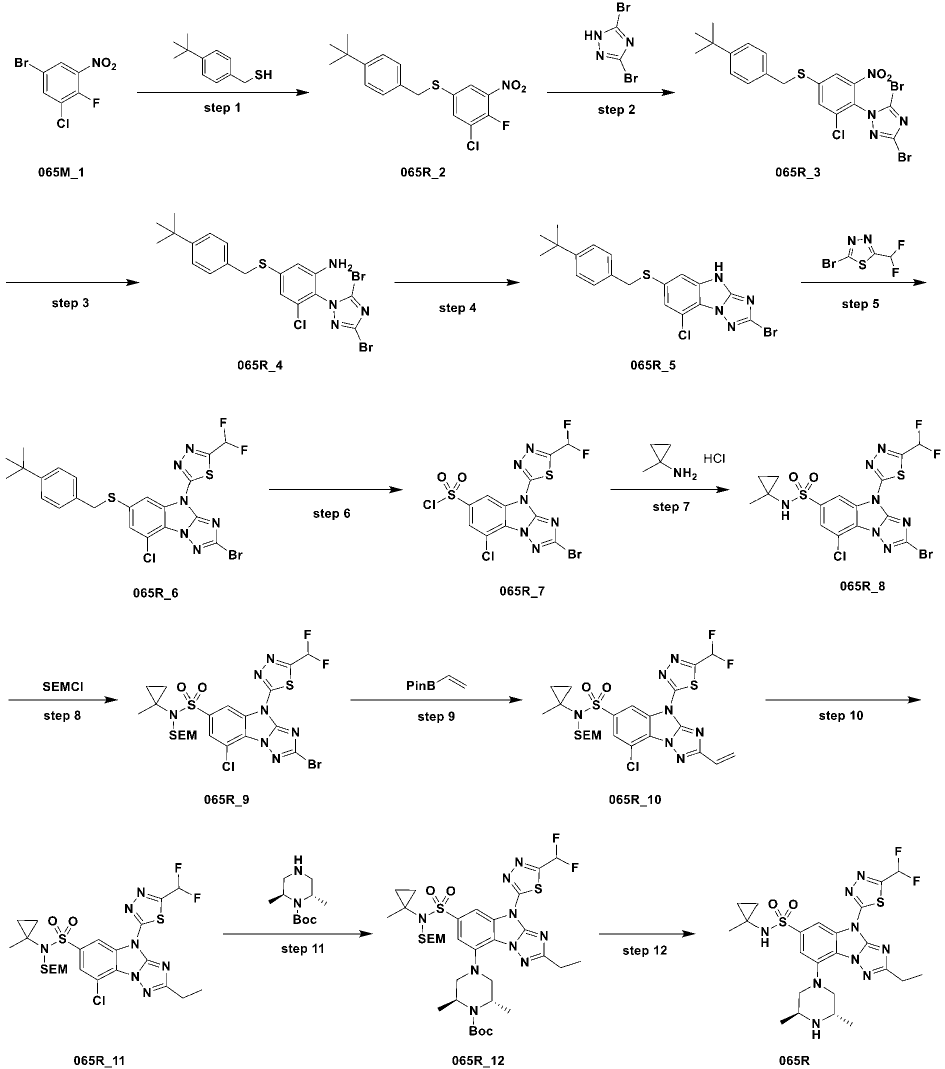

### Step 1: Synthesis of compound 065R_2

065M_1 (5.00 g, 19.7 mmol), 4-tert-butylbenzyl mercaptan (3.54 g, 19.7 mmol), and N,N-diisopropylethylamine (5.08 g, 39.3 mmol) were dissolved in 1,4-dioxane (80 mL). The system was purged with nitrogen three times, followed by addition of Pd₂(dba)₃ (900 mg, 983 µmol) and Xantphos (1.14 g, 1.97 mmol), and purged with nitrogen three additional times. The reaction mixture was heated to 80°C and stirred for 1 hour. Three batches were combined for work-up. The reaction mixture was cooled to room temperature and filtered through diatomite. The filtrate was concentrated by rotary evaporation. The crude product was purified by column chromatography to obtain 065R_2 (13.0 g).

¹H NMR (400 MHz, DMSO-*d₆*): δ 7.99 - 7.96 (m, 1H), 7.79 - 7.77 (m, 1H), 7.35 - 7.23 (m, 4H), 4.35 (s, 2H), 1.25 (s, 9H).

### Step 2: Synthesis of compound 065R_3

065R_2 (12.0 g, 33.9 mmol), 3,5-dibromo-1,2,4-triazole (9.23 g, 40.7 mmol), and potassium carbonate (7.03 g, 50.9 mmol) were dissolved in dimethyl sulfoxide (100 mL). The mixture was heated to 60°C and stirred for 1 hour. The reaction mixture was cooled to room temperature, then quenched with water (200 mL), and extracted with ethyl acetate (200 mL × 2). The combined organic phases were washed with brine (300 mL × 1), dried over anhydrous sodium sulfate, and concentrated under reduced pressure. The residue was purified by column chromatography to obtain 065R_3 (11.0 g).

MS (ESI) M/Z: 560.8, 562.8 [M+H]⁺.

¹H NMR (400 MHz, CDCl₃): δ 7.92 (d, *J* = 2.0 Hz, 1H), 7.65 (d, *J* = 2.0 Hz, 1H), 7.40 (d, *J =* 8.4 Hz, 2H), 7.33 (d, *J =* 8.4 Hz, 2H), 4.28 (s, 2H), 1.33 (s, 9H).

### Step 3: Synthesis of compound 065R_4

065R_3 (11.0 g, 19.6 mmol) and ammonium chloride (5.25 g, 98.1 mmol) were dissolved in ethanol (120 mL) and water (20 mL). The reaction mixture was heated to 70°C, followed by portionwise addition of iron powder (3.29 g, 58.9 mmol). The resulting mixture was stirred at 70°C for 1 hour. The reaction mixture was filtered through diatomite while hot. The filtrate was concentrated under reduced pressure to remove most of the ethanol. The resulting mixture was then diluted with water (100 mL) and extracted with ethyl acetate (100 mL × 2). The combined organic phases were washed with saturated brine (200 mL × 1), dried over anhydrous sodium sulfate, filtered, and the filtrate was concentrated by rotary evaporation to obtain 065R_4 (9.30 g) as a crude product, which was directly used in the next step without further purification.

¹H NMR (400 MHz, CDCl₃): δ 7.37 (d, *J* = 8.4 Hz, 2H), 7.29 (d, *J* = 8.4 Hz, 2H), 6.81 (d, *J =* 1.6 Hz, 1H), 6.62 (d, *J =* 1.6 Hz, 1H), 4.16 (s, 2H), 3.81(s, 2H), 1.33 (s, 9H).

### Step 4: Synthesis of compound 065R_5

065R_4 (9.30 g, 17.5 mmol) and cesium carbonate (11.4 g, 35.0 mmol) were dissolved in N,N-dimethylacetamide (100 mL). The reaction mixture was heated to 110°C and stirred for 2 hours. The reaction mixture was cooled to room temperature, quenched with water (200 mL), added with dilute hydrochloric acid (4 M) to adjust the pH to 6, and extracted with ethyl acetate (200 mL × 2). The combined organic phases were washed with brine (300 mL), dried over anhydrous sodium sulfate, filtered, and concentrated to obtain 065R_5 (8.60 g) as a crude product, which was directly used in the next step without further purification.

MS (ESI) M/Z: 448.9, 450.9 [M+H]⁺.

¹H NMR (400 MHz, DMSO-*d₆*): δ 7.44 (d, *J =* 0.8 Hz, 1H), 7.36 (s, 1H), 7.32 (d,*J* = 8.4 Hz, 2H), 7.27 (d, *J =* 8.4 Hz, 2H), 4.31 (s, 2H), 1.24 (s, 9H).

### Step 5: Synthesis of compound 065R_6

065R_5 (8.60 g, 19.1 mmol) and cesium carbonate (9.34 g, 28.7 mmol) were dissolved in N,N-dimethylacetamide (100 mL), followed by addition of 2-bromo-5-(difluoromethyl)-1,3,4-thiadiazole (6.17 g, 28.7 mmol). The reaction mixture was heated to 80°C and stirred for 16 hours. The reaction mixture was cooled to room temperature, quenched with water (250 mL), resulting in precipitation of a large amount of solid, followed by filtration. The filter cake was washed with water and dried under reduced pressure. The residue was triturated with ethyl acetate/petroleum ether (1/1, 100 mL) at room temperature and filtered to obtain 065R_6 (7.40 g).

MS (ESI) M/Z: 583.2, 585.2 [M+H]⁺.

¹H NMR (400 MHz, DMSO-*d₆*): δ 8.43 (d, *J* = 1.2 Hz, 1H), 7.78 - 7.52 (m, 2H), 7.34 - 7.32 (m, 4H), 4.36 (s, 2H), 1.22 (s, 9H).

### Step 6: Synthesis of compound 065R_7

065R_6 (4.50 g, 7.71 mmol) was dissolved in acetic acid (1 mL), water (1 mL), and acetonitrile (40 mL). The mixture was cooled to 0°C, followed by portionwise addition of 1,3-dichloro-5,5-dimethylhydantoin (2.73 g, 13.8 mmol), and stirred at 20°C for 0.5 hours. The reaction mixture was quenched with H₂O (100 mL) and extracted with dichloromethane (100 mL × 2). The combined organic phases were washed with brine (200 mL × 1), dried over anhydrous sodium sulfate, and concentrated under reduced pressure at room temperature to obtain 065R_7 (4.20 g).

MS (ESI) M/Z: 482.8, 484.8 [M-Cl+OH-H]⁻.

### Step 7: Synthesis of compound 065R_8

1-Methylcyclopropanamine hydrochloride (1.79 g, 16.7 mmol) and N,N-diisopropylethylamine (4.31 g, 33.3 mmol) were dissolved in dichloromethane (50.0 mL). A solution of 065R_7 (4.20 g, 8.33 mmol) in dichloromethane (5 mL) was added dropwise to the reaction mixture. After the addition was completed, the reaction mixture was stirred at 20°C for 20 minutes. The reaction mixture was quenched with water (80 mL) and extracted with dichloromethane (80 mL × 2). The combined organic phases were washed with saturated brine (100 mL × 1), dried, and concentrated under reduced pressure. The residue was purified by column chromatography to obtain 065R_8 (4.00 g).

MS (ESI) M/Z: 537.9, 539.8 [M+H]⁺.

¹H NMR (400 MHz, DMSO-*d₆*): δ 9.01 (s, 1H), 8.55 (s, 1H), 8.05 (d, *J =* 1.2 Hz, 1H), 7.68 (t, *J =* 53.2 Hz, 1H), 1.12 (s, 3H), 0.66 - 0.62 (m, 2H), 0.46 - 0.42 (m, 2H).

### Step 8: Synthesis of compound 065R_9

065R_8 (4.00 g, 7.42 mmol) and N,N-diisopropylethylamine (2.88 g, 22.2 mmol) were dissolved in dichloromethane (40.0 mL) and DMF (5 mL). 2-(Trimethylsilyl)ethoxymethyl chloride (1.86 g, 11.1 mmol) was added in one portion at 20°C. The reaction mixture was stirred at 20°C for 1 hour. The reaction mixture was quenched with water (100 mL) and extracted with dichloromethane (100 mL × 2). The combined organic phases were washed with water (200 mL) and brine (100 mL), dried over anhydrous sodium sulfate, filtered, and concentrated under reduced pressure. The crude product was purified by triturating with petroleum ether (100 mL), followed by purification by column chromatography to obtain 065R_9 (2.90 g).

MS (ESI) M/Z: 550.1, 552.1 [M+H-118]⁺.

¹H NMR (400 MHz, DMSO-*d₆*): δ 9.07 (d, *J* = 1.2 Hz, 1H), 8.12 (d, *J =* 1.6 Hz, 1H), 7.66 (t, *J* = 53.2 Hz, 1H), 4.92 (s, 2H), 3.56 (t, *J* = 8.4 Hz, 2H), 1.28 (s, 3H), 0.93 - 0.86 (m, 4H), 0.62 - 0.59 (m, 2H), -0.01 (s, 9H)

### Step 9: Synthesis of compound 065R_10

065R_9 (0.80 g, 1.20 mmol), vinylboronic acid pinacol ester (920 mg, 5.98 mmol), and cesium carbonate (1.17 g, 3.59 mmol) were dissolved in 1,4-dioxane (10.0 mL) and water (2.00 mL), and the system was purged with nitrogen three times. Pd(dppf)Cl₂·CH₂Cl₂ (292 mg, 358 µmol) was added thereto under nitrogen atmosphere, and the system was purged with nitrogen three times. The reaction mixture was stirred at 80°C for 16 hours. The reaction mixture was cooled to room temperature, then filtered through diatomite, added with water (50.0 mL), and extracted with ethyl acetate (20 mL × 3). The combined organic phases were washed with brine (20 mL × 3), dried over anhydrous sodium sulfate, filtered, and concentrated under reduced pressure. The residue was purified by column chromatography to obtain 065R_10 (0.30 g).

MS (ESI) M/Z: 516.1 [M+H-100]⁺.

¹H NMR (400 MHz, DMSO-*d₆*) δ 9.05 (d, *J =* 1.2 Hz, 1H), 8.08 (d, *J =* 1.6 Hz, 1H), 7.67 (t, *J =* 53.2 Hz, 1H), 6.93 - 6.86 (m, 1H), 6.43 - 6.38 (m, 1H), 5.80 - 5.77 (m, 1H), 4.91 (s, 2H), 3.57 - 3.53 (m, 2H), 1.29 (s, 3H), 0.92 - 0.87 (m, 4H), 0.62 - 0.59 (m, 2H), -0.02 (s, 9H).

### Step 10: Synthesis of compound 065R_11

Platinum dioxide (36.8 mg, 146 µmol) was added to methanol (10.0 mL), followed by addition of 065R_10 (200 mg, 325 µmol). The system was purged with hydrogen five times. The reaction mixture was stirred at 25°C for 0.5 hours. The reaction mixture was filtered through diatomite and concentrated under reduced pressure. The residue was purified by column chromatography to obtain 065R_11 (0.10 g).

MS (ESI) M/Z: 618.3, 620.2 [M+H]⁺.

¹H NMR (400 MHz, DMSO-*d₆*) δ 9.04 (d, *J* = 1.6 Hz, 1H), 8.06 (d, *J* = 1.6 Hz, 1H), 7.66 (t, *J* = 53.2 Hz, 1H), 4.91 (s, 2H), 3.57 - 3.52 (m, 2H), 2.94 - 2.88 (m, 2H), 1.36 (t, *J* = 7.6 Hz, 3H), 1.28 (s, 3H), 0.92 - 0.87 (m, 4H), 0.60 - 0.58 (m, 2H), -0.03 (s, 9H).

### Step 11: Synthesis of compound 065R_12

065R_11 (0.09 g, 145 µmol), *tert*-butyl (2S,6S)-2,6-dimethylpiperazine-1-carboxylate (93.6 mg, 436 µmol), and cesium carbonate (142 mg, 436 µmol) were dissolved in 1,4-dioxane (1.00 mL). RuPhos (13.5 mg, 29.1 µmol) and RuPhos Pd G3 (24.3 mg, 29.1 µmol) were added thereto under nitrogen atmosphere, and the system was purged with nitrogen three times. The reaction mixture was stirred at 90°C for 5 hours. The reaction mixture was cooled to room temperature, added with water (10.0 mL), and extracted with ethyl acetate (10 mL × 3). The combined organic phases were washed with brine (10 mL × 3), dried over anhydrous sodium sulfate, filtered, and concentrated under reduced pressure. The residue was purified by column chromatography to obtain 065R_12 (50 mg).

MS (ESI) M/Z: 796.2 [M+H]⁺.

### Step 4: Synthesis of compound 065R

065R_12 (50.0 mg, 62.8 µmol) was dissolved in dichloromethane (1.00 mL), followed by addition of trifluoroacetic acid (1 mL) at 25°C. The reaction mixture was stirred at 25°C for 1 hour. After concentration at room temperature, the residue was purified by preparative chromatography to obtain 065R (8.89 mg).

MS (ESI) M/Z: 566.3 [M+H]⁺.

¹H NMR (400 MHz, DMSO-*d₆*+D₂O) δ 8.74 (s, 1H), 7.72 - 7.46 (m, 2H), 3.86 - 3.81 (m, 2H), 3.52 - 3.49 (m, 2H), 3.26 - 3.22 (m, 2H), 2.89 (q, *J =* 7.6 Hz, 2H), 1.43 (d, *J =* 6.4 Hz, 6H), 1.35 (t, *J* = 7.6 Hz, 3H), 1.06 (s, 3H), 0.65 - 0.59 (m, 2H), 0.41 - 0.38 (m, 2H).

### Example 065S

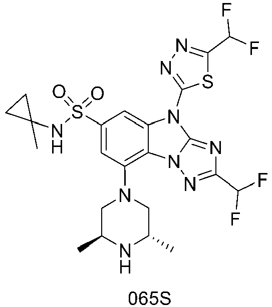

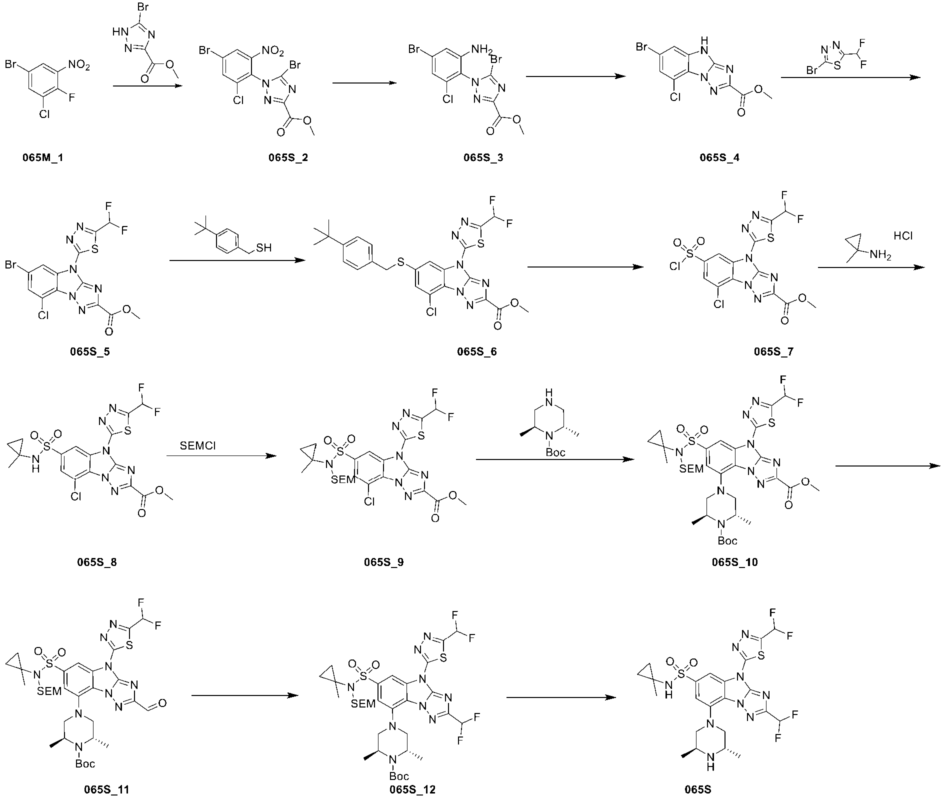

### Step 1: Synthesis of compound 065S_2

Compound 065M_1 (30.0 g, 0.118 mol) and methyl 5-bromo-1,2,4-triazole-3-carboxylate (26.7 g, 0.130 mol) were dissolved in dimethyl sulfoxide (400 mL), followed by addition of potassium carbonate (24.5 g, 0.177 mol). The reaction mixture was stirred at room temperature for 12 hours. The reaction mixture was extracted three times with dichloromethane. The organic phase was washed with saturated brine, dried over anhydrous sodium sulfate, concentrated, and purified by silica gel column chromatography to obtain product 065S_2 (35.6 g).

MS (ESI) M/Z: 438.8 [M+H]⁺.

### Step 2: Synthesis of compound 065S_3

065S_2 (35.6 g, 81.1 mmol), iron powder (45.3 g, 0.811 mol), and acetic acid (500 mL) were added to a flask. The system was purged with nitrogen and stirred at room temperature for 2 hours. The reaction mixture was extracted three times with dichloromethane. The organic phase was washed with saturated brine, dried over anhydrous sodium sulfate, concentrated, and purified by silica gel column chromatography to obtain 065S_3 (29.0 g).

MS (ESI) M/Z: 408.8 [M+H]⁺.

### Step 3: Synthesis of compound 065S_4

Compound 065S_3 (18 g, 43.85 mmol) was dissolved in N,N-dimethylacetamide (200 mL), followed by addition of cesium carbonate (28.57 g, 87.7 mmol). The reaction mixture was stirred at 100°C for 3 hours under nitrogen atmosphere. The reaction mixture was cooled to room temperature and directly used in the next step.

MS (ESI) M/Z: 329.0 [M+H]⁺.

### Step 4: Synthesis of compound 065S_5

Cesium carbonate (14.83 g, 45.52 mmol) and 2-bromo-5-(difluoromethyl)-1,3,4-thiadiazole (7.34 g, 34.14 mmol) were added to the reaction mixture containing 065S_4 (7.5 g, 22.76 mmol) from the previous step. The system was purged with nitrogen. The reaction mixture was stirred at 100°C for 36 hours. The reaction mixture was cooled to room temperature, added with dichloromethane (1000 mL), resulting in precipitation of a solid, followed by filtration to obtain a filter cake. The filtrate was extracted with dichloromethane (1000 mL × 3) and saturated brine (500 mL × 1). The organic phase was dried over anhydrous sodium sulfate, filtered, and the filtrate was concentrated by rotary evaporation. The residue was purified by column chromatography to obtain 065S_5 (9.8 g).

MS (ESI) M/Z: 462.0 [M+H]⁺.

### Step 3: Synthesis of compound 065S_6

065S_5 (9.8 g, 21.57 mmol) was dissolved in 1,4-dioxane (100 mL), followed by addition of 4-tert-butylbenzyl mercaptan (3.89 g, 21.57 mmol), N,N-diisopropylethylamine (6.97 g, 53.92 mmol), Xantphos (0.75 g, 1.29 mmol), and Pd₂(dba)₃ (0.59 g, 0.65 mmol). The system was thoroughly purged with nitrogen. The reaction mixture was stirred at 90°C for 4 hours under nitrogen atmosphere. The reaction mixture was cooled to room temperature, mixed with silica gel, and purified by column chromatography to obtain 065S_6 (10.6 g).

MS (ESI) M/Z: 563.0 [M+H]⁺.

### Step 4: Synthesis of compound 065S_7

065S_6 (10.6 g, 18.83 mmol) was dissolved in a mixture of acetonitrile/acetic acid/water (7/2/1, 100 mL), followed by portionwise addition of 1,3-dichloro-5,5-dimethylhydantoin (7.42 g, 37.66 mmol) in an ice bath. The reaction mixture was then stirred at room temperature for 3 hours under nitrogen atmosphere. The reaction mixture was extracted with dichloromethane (1500 mL × 3) and saturated brine (1000 mL × 1). The organic phase was dried over anhydrous sodium sulfate, filtered, and the filtrate was concentrated by rotary evaporation to obtain 065S_7 (9 g).

MS (ESI) M/Z: 482.9 [M+H]⁺.

### Step 5: Synthesis of compound 065S_8

065S_7 (9 g, 18.62 mmol) was dissolved in dichloromethane (100 mL), followed by addition of 1-methylcyclopropanamine hydrochloride (6.01 g, 55.86 mmol) and triethylamine (5.65 g, 55.86 mmol) in an ice bath. The reaction mixture was then stirred in an ice bath for 2 hours under nitrogen atmosphere. The reaction mixture was directly mixed with silica gel without further work-up, and purified by column chromatography to obtain 065S_8 (8.3 g).

MS (ESI) M/Z: 518.0 [M+H]⁺.

### Step 6: Synthesis of compound 065S_9

065S_8 (9 g, 17.38 mmol) was dissolved in N,N-dimethylformamide (100 mL), followed by addition of cesium carbonate (16.99 g, 52.14 mmol) and 2-(trimethylsilyl)ethoxymethyl chloride (SEMCl) (14.49 g, 86.90 mmol) in an ice bath. The reaction mixture was stirred at room temperature for 5 hours under nitrogen atmosphere. The reaction mixture was extracted with ethyl acetate (1000 mL × 3) and water (500 mL). The organic phase was then washed with saturated sodium chloride solution, dried over anhydrous sodium sulfate, filtered, and the filtrate was concentrated by rotary evaporation. The residue was purified by column chromatography to obtain 065S_9 (8.3 g).

MS (ESI) M/Z: 648.1 [M+H]⁺.

### Step 7: Synthesis of compound 065S_10

065S_9 (5 g, 7.71 mmol) was dissolved in 1,4-dioxane (100 mL), followed by addition of *tert-butyl* (2S,6S)-2,6-dimethylpiperazine-1-carboxylate (3.30 g, 15.42 mmol), cesium carbonate (7.54 g, 23.13 mmol), RuPhos (0.72 g, 1.54 mmol), and RuPhos Pd G3 (0.64 g, 0.77 mmol). The reaction mixture was stirred at 90°C for 3 hours under nitrogen atmosphere. The reaction mixture was cooled to room temperature, and extracted with ethyl acetate (300 mL × 3) and water (500 mL). The organic phase was then washed with saturated sodium chloride solution, dried over anhydrous sodium sulfate, filtered, and the filtrate was concentrated by rotary evaporation. The residue was purified by column chromatography to obtain 065S_10 (3.16 g).

MS (ESI) M/Z: 608.2 [M-118-100+H]⁺.

### Step 8: Synthesis of compound 065S_11

065S_10 (1.6 g, 1.94 mmol) was dissolved in dichloromethane (20 mL). The mixture was then cooled to -78°C in a dry ice-ethanol bath, and diisobutylaluminum hydride (0.83 g, 5.82 mmol, 1 mol/L) was slowly added dropwise thereto. The reaction mixture was stirred at -78°C for 2 hours under nitrogen atmosphere. The reaction mixture was slowly quenched with methanol (10 mL) at -78°C and filtered through diatomite. The filter cake was washed with dichloromethane (100 mL × 3). The filtrate was concentrated under reduced pressure. The residue was purified by column chromatography to obtain 065S_11 (1.1 g).

MS (ESI) M/Z: 578.2 [M-118-100+H]⁺.

### Step 9: Synthesis of compound 065S_12

065S_11 (1.1 g, 1.38 mmol) was dissolved in dichloromethane (30 mL). The mixture was then cooled to -78°C in a dry ice-ethanol bath, and bis(2-methoxyethyl)aminosulfur trifluoride (1.53 g, 6.90 mmol, 1 mol/L) was slowly added dropwise thereto. The reaction mixture was stirred at room temperature for 3 hours under nitrogen atmosphere. The reaction mixture was slowly quenched with saturated sodium bicarbonate solution (10 mL) in an ice bath, and extracted with dichloromethane (100 mL × 3) and water (100 mL). The filtrate was concentrated under reduced pressure. The residue was purified by column chromatography to obtain 065S_12 (0.79 g).

MS (ESI) M/Z: 700.2 [M-118+H]⁺.

### Step 10: Synthesis of compound 065S

065S_12 (1 g, 1.22 mmol) was dissolved in dichloromethane (20 mL), followed by addition of trifluoroacetic acid (2 mL) and water (0.5 mL). The reaction mixture was stirred at room temperature for 2 hours under nitrogen atmosphere. The reaction mixture was concentrated under reduced pressure to remove the solvent. The residue was purified by preparative chromatography to obtain 065S (0.33 g).

MS (ESI) M/Z: 588.2 [M+H]⁺.

### Example 065W

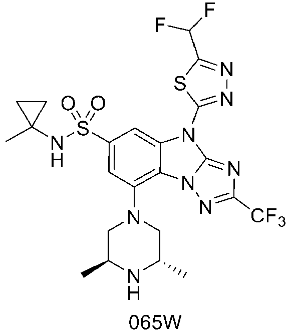

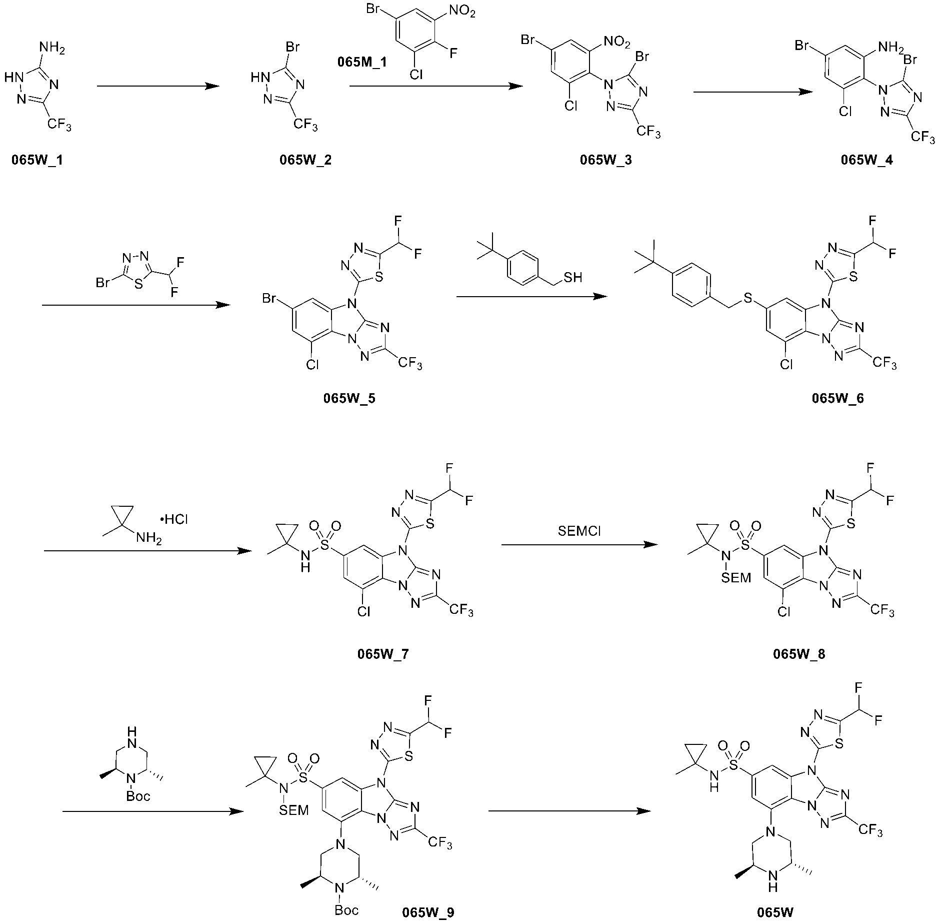

### Step 1: Synthesis of compound 065W_2

065W_1 (5 g, 32.88 mmol) and water (50 mL) were added to a 500 mL single-necked flask, stirred thoroughly, followed by addition of sulfuric acid (2.8 mL, 52.52 mmol). The mixture was then cooled to 0°C, and a solution of sodium nitrite (3.4 g, 49.32 mmol) in water (20 mL) was added dropwise thereto. After the addition was completed, the reaction mixture was stirred for 1 hour, resulting in precipitation of a white solid. Copper(I) bromide (1.41 g, 9.86 mmol) was added thereto at 0°C, followed by dropwise addition of a 48% hydrobromic acid aqueous solution (7.44 mL). The reaction mixture was stirred at room temperature for 3 hours. The reaction mixture was poured into saturated sodium bicarbonate solution and extracted with ethyl acetate (200 mL × 5). The organic phases were combined, washed with saturated brine (100 mL × 2), dried over anhydrous sodium sulfate, and concentrated under reduced pressure to obtain 065W_2 (7.3 g) as a crude product, which was directly used in the next step without further purification.

MS (ESI) M/Z: 216.0, 218.0 [M+H]⁺.

### Step 2: Synthesis of compound 065W_3

The crude product 065W_2 from the previous step was dissolved in dimethyl sulfoxide (50 mL), stirred thoroughly, followed by addition of 065M_1 (8.37 g, 32.88 mmol) and potassium carbonate (5 g, 98.64 mmol). The reaction mixture was stirred at 70°C for 9 hours. The reaction mixture was cooled to room temperature, quenched with water (200 mL), and extracted with ethyl acetate (200 mL × 3). The organic phases were combined, washed with saturated brine (100 mL × 2), dried over anhydrous sodium sulfate, and concentrated under reduced pressure to obtain a crude product, which was purified by flash silica gel column chromatography to obtain 065W_3 (6.60 g).

MS (ESI) M/Z: 448.8, 450.8, 452.8 [M+H]⁺.

### Step 3: Synthesis of compound 065W_4

065W_3 (17 g, 37.75 mmol) and acetic acid (50 mL) were added to a 500 mL single-necked flask, stirred thoroughly, followed by addition of iron powder (21.08 g, 377.5 mmol). The reaction mixture was stirred at room temperature for 1 hour. The reaction mixture was filtered through diatomite and washed with ethyl acetate (200 mL). The filtrate was concentrated under reduced pressure to obtain a crude product. The residue was purified by flash silica gel column chromatography to obtain 065W_4 (15 g).

MS (ESI) M/Z: 418.8, 420.8, 422.8 [M+H]⁺.

### Step 4: Synthesis of compound 065W_5

065W_4 (380 mg, 0.9 mmol) and N,N-dimethylacetamide (5 mL) were added to a 100 mL single-necked flask, stirred thoroughly, followed by addition of cesium carbonate (0.59 g, 1.8 mmol). The reaction mixture was stirred at 100°C for 3 hours. After completion of the reaction, 2-bromo-5-(difluoromethyl)-1,3,4-thiadiazole (0.39 g, 1.8 mmol) and cesium carbonate (0.59 g, 1.8 mmol) were added thereto. The reaction mixture was stirred at 100°C for another 16 hours. The reaction mixture was cooled to room temperature, quenched with water (50 mL), and extracted with ethyl acetate (50 mL × 3). The organic phases were combined, washed with saturated brine (50 mL × 2), dried over anhydrous sodium sulfate, and concentrated under reduced pressure to obtain a crude product, which was purified by flash silica gel column chromatography to obtain 065W_5 (260 mg).

MS (ESI) M/Z: 472.8, 474.8 [M+H]⁺.

### Step 5: Synthesis of compound 065W_6

065W_5 (3 g, 6.33 mmol), Pd₂(dba)₃ (580 mg, 0.63 mmol), Xantphos (732 mg), and 1,4-dioxane (60 mL) were added to a 100 mL single-necked flask. After thorough stirring, the mixture was placed under nitrogen atmosphere. 4-tert-Butylbenzyl mercaptan (1.48 g, 8.23 mmol) and N,N-diisopropylethylamine (2.62 mL, 15.82 mmol) were added thereto. The reaction mixture was stirred at 90°C for 1 hour. The reaction mixture was filtered through diatomite and washed with ethyl acetate (100 mL). The filtrate was concentrated under reduced pressure to obtain a crude product. The residue was purified by flash silica gel column chromatography to obtain 065W_6 (3 g).

MS (ESI) M/Z: 573.0 [M+H]⁺.

### Step 6: Synthesis of compound 065W_7

065W_6 (340 mg, 0.59 mmol) and acetonitrile (5 mL) were added to a 50 mL single-necked flask. After thorough stirring, the mixture was cooled to 0°C. Acetic acid (300 µL) and water (600 µL) were added thereto, followed by portionwise addition of 1,3-dichloro-5,5-dimethylhydantoin (230 mg, 1.18 mmol). The reaction mixture was stirred for 1 hour. After completion of the reaction, the reaction mixture was concentrated under reduced pressure to remove most of the solvent, and extracted with ethyl acetate (30 mL × 3). The organic phases were combined, washed with saturated brine (30 mL × 2), dried over anhydrous sodium sulfate, and concentrated under reduced pressure to obtain a crude product. The crude product was dissolved in dichloromethane (5 mL) and cooled to 0°C, followed by addition of 1-methylcyclopropanamine hydrochloride (190 mg, 1.77 mmol) and triethylamine (330 µL, 2.36 mmol). After stirring for 1 hour, the solvent was removed under reduced pressure. The residue was purified by flash silica gel column chromatography to obtain 065W_7 (200 mg).

MS (ESI) M/Z: 528.0 [M+H]⁺.

### Step 7: Synthesis of compound 065W_8

065W_7 (200 mg, 0.38 mmol) and N,N-dimethylacetamide (5 mL) were added to a 50 mL single-necked flask. After thorough stirring, the mixture was cooled to 0°C. Cesium carbonate (389 mg, 1.14 mmol) was added thereto, followed by dropwise addition of 2-(trimethylsilyl)ethoxymethyl chloride (200 µL). The reaction mixture was warmed to room temperature and stirred for 12 hours. The reaction mixture was quenched with water (20 mL) and extracted with ethyl acetate (30 mL × 3). The organic phases were combined, washed with saturated brine (30 mL × 2), dried over anhydrous sodium sulfate, and concentrated under reduced pressure to obtain a crude product. The residue was purified by flash silica gel column chromatography to obtain 065W_8 (190 mg).

### Step 8: Synthesis of compound 065W_9

065W_8 (190 mg, 0.29 mmol), cesium carbonate (280 mg, 0.87 mmol), RuPhos Pd G3 (24 mg, 0.029 mmol), and RuPhos (20 mg, 0.043 mmol) were added to a 50 mL single-necked flask, followed by addition of 1,4-dioxane (5 mL). After thorough stirring, the mixture was placed under nitrogen atmosphere. *tert*-Butyl (2S,6S)-2,6-dimethylpiperazine-1-carboxylate (87 mg, 0.41 mmol) was then added thereto. The reaction mixture was heated to 90°C and stirred for 3 hours. The reaction mixture was cooled to room temperature, filtered, and concentrated under reduced pressure to obtain a crude product. The residue was purified by flash silica gel column chromatography to obtain 065W_9 (100 mg).

### Step 11: Synthesis of compound 065W

065W_9 (100 mg, 0.12 mmol) and dichloromethane (3 mL) were added to a 50 mL single-necked flask, stirred thoroughly, followed by addition of trifluoroacetic acid (1 mL) and water (0.2 mL). The reaction mixture was stirred at room temperature for 1 hour. The reaction mixture was concentrated under reduced pressure to obtain a crude product, which was purified by preparative chromatography to obtain 065W (41 mg).

### Example 065X

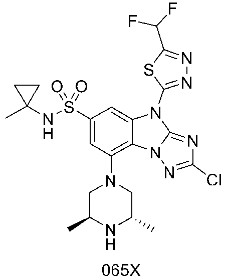

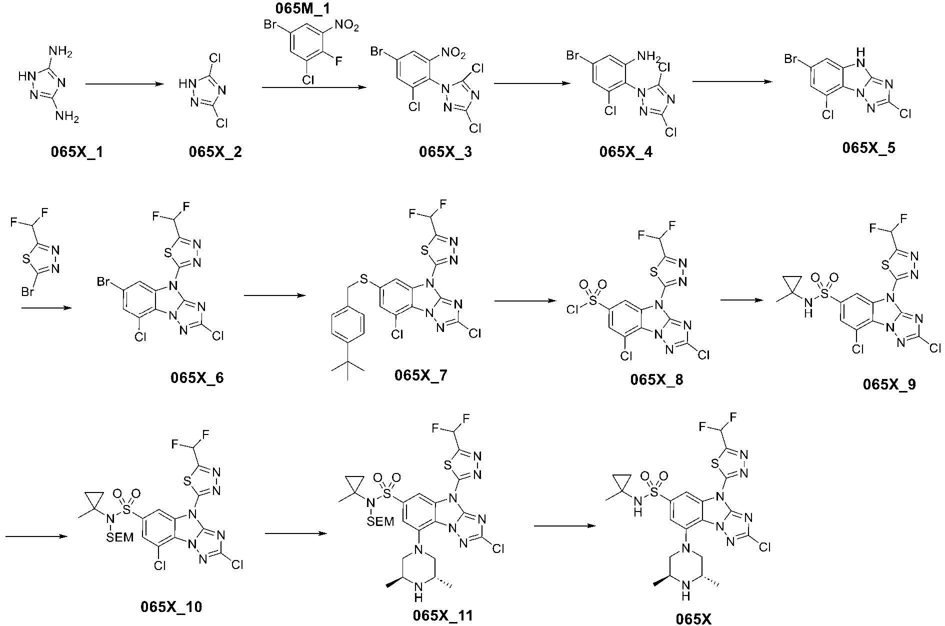

### Step 1: Synthesis of compound 065X_2

065X_1 (12.6 g, 0.127 mol) and hydrochloric acid (100 mL) were added to a two-necked flask. Sodium nitrite (26.4 g, 0.383 mol) was slowly added thereto in an ice bath. After the addition was completed, the reaction mixture was gradually warmed to room temperature and stirred for 60 minutes. The reaction mixture was extracted three times with ethyl acetate. The organic phase was washed with saturated brine, dried over anhydrous sodium sulfate, and concentrated to obtain 065X_2 (11.2 g).

### Step 2: Synthesis of compound 065X_3

065X_2 (4.2 g, 30.6 mmol), 5-bromo-1-chloro-2-fluoro-3-nitrobenzene (065M_1, 5.2 g, 20.4 mmol), potassium carbonate (5.6 g, 40.8 mmol), and dimethyl sulfoxide (50 mL) were sequentially added to a round-bottomed flask. The mixture was stirred at room temperature for 12 hours. The reaction mixture was extracted three times with ethyl acetate. The organic phase was washed with saturated brine, dried over anhydrous sodium sulfate, and concentrated to obtain 065X_3, which was directly used in the next step.

### Step 3: Synthesis of compound 065X_4

Acetic acid (100 mL) was added to 065X_3 from the previous step, followed by addition of iron powder (11.4 g, 0.204 mol). The system was purged with nitrogen and stirred at room temperature for 2 hours. The reaction mixture was extracted three times with ethyl acetate. The organic phase was washed with saturated brine, dried over anhydrous sodium sulfate, concentrated, and purified by silica gel column chromatography to obtain 065X_4 (5.0 g).

MS (ESI) M/Z: 340.8 [M+H]⁺.

### Step 4: Synthesis of compound 065X_5

065X_4 (3.7 g, 10.8 mmol), cesium carbonate (7.0 g, 21.5 mmol), and N,N-dimethylacetamide (50 mL) were sequentially added to a round-bottomed flask. The mixture was stirred in an oil bath at 100°C for 3 hours. The reaction mixture was cooled to room temperature and directly used in the next step.

MS (ESI) M/Z: 305.0 [M+H]⁺.

### Step 5: Synthesis of compound 065X_6

Cesium carbonate (3.5 g, 10.7 mmol) and 2-bromo-5-(difluoromethyl)-1,3,4-thiadiazole (4.6 g, 21.5 mmol) were added to the reaction mixture containing 065X_5 from the previous step. The reaction mixture was stirred in an oil bath at 100°C for 8 hours. The reaction mixture was extracted three times with a large amount of dichloromethane. The organic phase was washed with saturated brine, dried over anhydrous sodium sulfate, and concentrated to obtain a dark brown solid, which was triturated with ethyl acetate and petroleum ether to obtain 065X_6 (4.2 g).

MS (ESI) M/Z: 438.8 [M+H]⁺.

### Step 6: Synthesis of compound 065X_7

065X_6 (0.290 g, 0.66 mmol) was dissolved in 1,4-dioxane (10 mL), followed by addition of 4-tert-butylbenzyl mercaptan (0.119 g, 0.66 mmol), N,N-diisopropylethylamine (0.256 g, 1.98 mmol), Xantphos (38.2 mg, 66.0 µmol), and Pd₂(dba)₃ (30.2 mg, 33.0 µmol). The system was thoroughly purged with nitrogen. The reaction mixture was stirred at 90°C for 3 hours under nitrogen atmosphere. The reaction mixture was concentrated and purified by silica gel column chromatography to obtain 065X_7 (0.259 g).

MS (ESI) M/Z: 539.0 [M+H]⁺.

### Step 7: Synthesis of compound 065X_8

065X_7 (0.259 g, 0.481 mmol) was dissolved in a mixture of acetonitrile/acetic acid/water (7/2/1, 10 mL), followed by portionwise addition of 1,3-dichloro-5,5-dimethylhydantoin (0.189 g, 0.962 mmol) in an ice bath. The reaction mixture was stirred in an ice bath for 3 hours. The reaction mixture was extracted three times with dichloromethane. The organic phase was washed with saturated brine, dried over anhydrous sodium sulfate, and concentrated to obtain 065X_8, which was directly used in the next step without purification.

### Step 8: Synthesis of compound 065X_9

The crude product 065X_8 from the previous step was dissolved in dichloromethane (20 mL), followed by addition of 1-methylcyclopropanamine hydrochloride (0.259 g, 2.41 mmol) and triethylamine (0.244 g, 2.41 mmol) in an ice bath. The reaction mixture was then stirred in an ice bath for 2 hours under nitrogen atmosphere. The reaction mixture was extracted three times with dichloromethane. The organic phase was washed with saturated brine, dried over anhydrous sodium sulfate, concentrated, and purified by silica gel column chromatography to obtain 065X_9 (0.130 g).

MS (ESI) M/Z: 494.0 [M+H]⁺.

### Step 9: Synthesis of compound 065X_10

065X_9 (0.130 g, 0.252 mmol) was dissolved in N,N-dimethylformamide (10 mL), followed by addition of cesium carbonate (0.165 g, 0.505 mmol). 2-(Trimethylsilyl)ethoxymethyl chloride (84.2 mg, 0.505 mmol) was added dropwise thereto in an ice bath. After the addition was completed, the reaction mixture was gradually warmed to room temperature and stirred for 5 hours. The reaction mixture was extracted three times with ethyl acetate. The organic phase was sequentially washed with water and saturated brine, dried over anhydrous sodium sulfate, concentrated, and purified by silica gel column chromatography to obtain 065X_10 (90 mg).

MS (ESI) M/Z: 506.0 [M+H-118]⁺.

### Step 10: Synthesis of compound 065X_11

065X_10 (90.0 mg, 0.140 mmol), RuPhos Pd G3 (11.7 mg, 14.0 µmol), RuPhos (13.1 mg, 28.0 µmol), cesium carbonate (91.2 mg, 0.280 mmol), *tert-butyl* (2S,6S)-2,6-dimethylpiperazine-1-carboxylate (60.0 mg, 0.280 mmol), and 1,4-dioxane (10 mL) were sequentially added to a round-bottomed flask. The reaction mixture was stirred in an oil bath at 90°C for 3 hours. The reaction mixture was extracted three times with ethyl acetate. The organic phase was washed with saturated brine, dried over anhydrous sodium sulfate, concentrated, and purified by silica gel column chromatography to obtain 065X_11 (30.0 mg).

### Step 11: Synthesis of compound 065X

065X_11 (30.0 mg, 42.7 µmol) and dichloromethane (5.0 mL) were added to a reaction flask, followed by addition of trifluoroacetic acid (2.0 mL) in an ice bath. After the addition was completed, the reaction mixture was warmed to room temperature and stirred for 8 hours. The reaction mixture was extracted three times with dichloromethane. The organic phase was washed with saturated brine, dried over anhydrous sodium sulfate, concentrated, and purified by preparative chromatography to obtain compound 065X (10 mg).

¹H NMR (400 MHz, DMSO-*d₆*+D₂O) δ 8.72 (d, *J* = 1.4 Hz, 1H), 7.65 (t, *J =* 53.1 Hz, 1H), 7.50 (d, *J =* 1.6 Hz, 1H), 3.42 - 3.38 (m, 2H), 3.28 (dd, *J =* 11.1, 3.2 Hz, 2H), 2.93 (dd, *J* = 11.1, 6.0 Hz, 2H), 1.22 (d, *J =* 6.5 Hz, 6H), 1.09 (s, 3H), 0.67 - 0.65 (m, 2H), 0.56 - 0.31 (m, 2H).

MS (ESI) M/Z: 572.0 [M+H]⁺.

### Examples 067C, 067D, and 074B

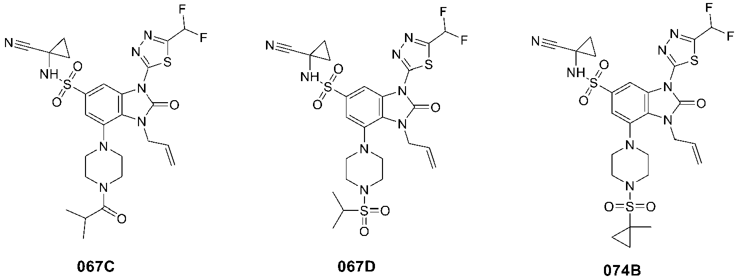

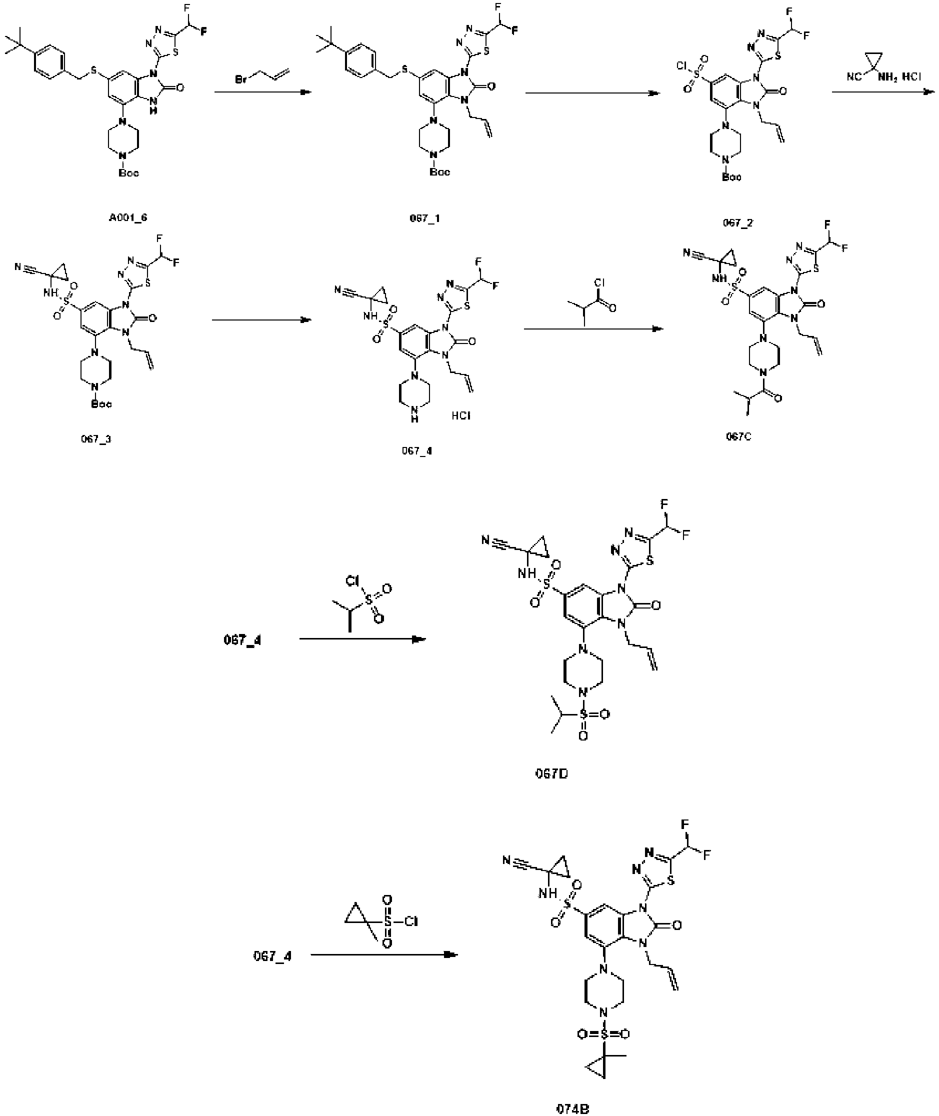

### Step 1: Synthesis of compound 067_1

A001_6 (15.0 g, 23.7 mmol) and potassium carbonate (9.86 g, 71.3 mmol) were dissolved in N,N-dimethylformamide (150 mL), followed by addition of 3-bromopropene (5.75 g, 47.5 mmol) at room temperature. The reaction mixture was heated to 50°C and stirred for 12 hours. The reaction mixture was cooled to room temperature, then quenched with water (500 mL), and extracted with ethyl acetate (200 mL × 3). The combined organic phases were washed with brine (200 mL × 3), dried over anhydrous sodium sulfate, filtered, and the filtrate was concentrated by rotary evaporation. The residue was purified by column chromatography to obtain 067_1 (13.0 g).

MS (ESI) M/Z: 671.4 [M+H]⁺.

¹H NMR (400 MHz, DMSO-*d₆*) δ 8.28 (d, *J =* 1.6 Hz, 1H), 7.62 (t, *J =* 52.8 Hz, 1H), 7.29 - 7.27 (m, 2H), 7.22 - 7.20 (m, 2H), 7.02 (d, *J* = 1.6 Hz, 1H), 6.05 - 5.97 (m, 1H), 5.11 (d, *J = 10.4* Hz, 1H), 5.00 (d, *J =* 17.2 Hz, 1H), 4.83 - 4.82 (m, 2H), 4.17 (s, 2H), 3.97 - 3.85 (m, 2H), 3.08 - 2.96 (m, 2H), 2.86 - 2.77 (m, 2H), 2.66 - 2.59 (m, 2H), 1.43 (s, 9H), 1.23 (s, 9H).

### Step 3: Synthesis of compound 067_2

067_1 (3.00 g, 4.47 mmol) was dissolved in a mixed solvent of acetonitrile (21 mL), acetic acid (3 mL), and water (6 mL), followed by addition of 1,3-dichloro-5,5-dimethylhydantoin (1.76 g, 8.94 mmol) at 0°C. The reaction mixture was slowly warmed to 25°C and stirred for 2 hours. Four parallel batches were performed and combined for work-up. The reaction mixture was quenched at 0°C with water (100 mL), and extracted with dichloromethane (100 mL × 3). The combined organic phases were washed with brine (100 mL × 3), dried over anhydrous sodium sulfate, filtered, and the filtrate was concentrated by rotary evaporation at low temperature to obtain 067_2 (10.0 g) as a crude product, which was directly used in the next step without purification.

MS (ESI) M/Z: 571.1 [M-Cl+OH-H]⁻.

### Step 4: Synthesis of compound 067_3

1-Aminocyclopropanecarbonitrile hydrochloride (4.01 g, 33.8 mmol) was dissolved in pyridine (20 mL), followed by dropwise addition of a solution of 067_2 (5.00 g, 8.46 mmol) in dichloromethane (10 mL). The reaction mixture was stirred at 25°C for 1 hour. Two parallel batches were performed and combined for work-up. The reaction mixture was quenched with water (100 mL) and extracted with dichloromethane (100 mL × 3). The combined organic phases were washed with brine (100 mL × 3), dried over anhydrous sodium sulfate, filtered, and the filtrate was concentrated by rotary evaporation. The residue was purified by column chromatography to obtain 067_3 (4.00 g).

MS (ESI) M/Z: 637.1 [M+H]⁺.

¹H NMR (400 MHz, DMSO-*d₆*) δ 9.21 (s, 1H), 8.76 (d, *J* = 1.6 Hz, 1H), 7.78 - 7.51 (m, 2H), 6.10 - 6.01 (m, 1H), 5.12 (d, *J =* 10.4 Hz, 1H), 5.03 (d, *J =* 17.6 Hz, 1H), 4.94 - 4.91 (m, 2H), 3.99 - 3.93 (m, 1H), 3.08 - 2.96 (m, 3H), 2.90 - 2.88 (m, 2H), 2.83 - 2.78 (m, 2H), 1.45 - 1.41 (m, 11H), 1.30 - 1.27 (m, 2H).

### Step 5: Synthesis of compound 067_4

067_3 (4.00 g, 6.28 mmol) was dissolved in ethyl acetate (20 mL), followed by addition of hydrochloric acid/ethyl acetate (4 M, 20.0 mL). The reaction mixture was stirred at 25°C for 0.5 hours. The reaction mixture was directly concentrated under reduced pressure. The crude product was triturated with methyl *tert-butyl* ether (30 mL) at room temperature and filtered to obtain 067_4 (3.3 g).

MS (ESI) M/Z: 537.4 [M+H]⁺.

¹H NMR (400 MHz, DMSO-*d₆*) δ 9.36 (s, 1H), 9.26 (s, 2H), 8.80 (d, *J* = 1.6 Hz, 1H), 7.78 - 7.52 (m, 2H), 6.13 - 6.04 (m, 1H), 5.13 (d, *J =* 11.6 Hz, 1H), 5.03 (d, *J =* 17.6 Hz, 1H), 4.91 - 4.86 (m, 2H), 3.43 - 3.34 (m, 2H), 3.24 - 3.10 (m, 6H), 1.44 - 1.41 (m, 2H), 1.32 - 1.26 (m, 2H).

### Step 6: Synthesis of compound 074B

067_4 (0.15 g, 261 µmol) was dissolved in N,N-dimethylformamide (4 mL), followed by addition of triethylamine (106 mg, 1.05 mmol) and 1-methylcyclopropane-1-sulfonyl chloride (44.5 mg, 287 µmol). The reaction mixture was stirred at 25°C for 1 hour. After concentration, the residue was purified by preparative chromatography to obtain 074B (53.73 mg).

MS (ESI) M/Z: 655.1 [M+H]⁺.

¹H NMR (400 MHz, DMSO-*d₆*) δ 9.21 (s, 1H), 8.78 (d, *J* = 1.6 Hz, 1H), 7.80 - 7.51 (m, 2H), 6.12 - 6.02 (m, 1H), 5.14 (d, *J =* 10.8 Hz, 1H), 5.03 (d, *J =* 17.6 Hz, 1H), 4.94 - 4.90 (m, 2H), 3.74 - 3.71 (m, 2H), 3.24 - 3.14 (m, 2H), 3.09 - 3.07 (m, 2H), 2.96 - 2.90 (m, 2H), 1.47 (s, 3H), 1.45 - 1.41 (m, 2H), 1.31 - 1.28 (m, 2H), 1.22 - 1.19 (m, 2H), 0.90 - 0.88 (m, 2H).

### Step 7: Synthesis of compound 067C

067_4 (0.28 g, 0.48 mmol) and triethylamine (158 mg, 1.56 mmol) were dissolved in N,N-dimethylformamide (2.00 mL), followed by addition of isobutyryl chloride (45.8 mg, 429 µmol). The reaction mixture was stirred at 25°C for 1 hour. The reaction mixture was quenched with water (0.3 mL). The residue was purified by preparative chromatography to obtain 067C (70 mg).

MS (ESI) M/Z: 607.2 [M+H]⁺.

¹H NMR (400 MHz, DMSO-*d₆*) δ 8.75 (d, *J =* 1.6 Hz, 1H), 8.39 (s, 1H), 7.78 - 7.51 (m, 2H), 6.11 - 6.04 (m, 1H), 5.13 (d, *J =* 10.4 Hz, 1H), 5.04 (d, *J =* 17.2 Hz, 1H), 4.96 - 4.94 (m, 2H), 4.49 - 4.46 (m, 1H), 4.08 - 4.04 (m, 1H), 3.39 - 3.36 (m, 1H), 3.06 -3.03 (m, 2H), 2.96 - 2.93 (m, 1H), 2.89 - 2.83 (m, 3H), 1.44 - 1.41 (m, 2H), 1.28 - 1.25 (m, 2H), 1.05 - 1.02 (m, 6H).

### Step 8: Synthesis of compound 067D

067_4 (2.00 g, 3.49 mmol) was dissolved in a mixed solvent of tetrahydrofuran (20 mL) and water (5 mL), followed by addition of sodium carbonate (1.11 g, 10.5 mmol) and isopropylsulfonyl chloride (995 mg, 6.98 mmol). The reaction mixture was stirred at 20°C for 1 hour. One 800 mg batch was combined for a single work-up. The reaction mixture was diluted with water (30 mL) and extracted with ethyl acetate (30 mL × 3). The combined organic phases were washed with brine (50 mL × 1), dried over anhydrous sodium sulfate, filtered, and the filtrate was concentrated by rotary evaporation. The crude product was purified by column chromatography to obtain 067D (942.64 mg).

MS (ESI) M/Z: 643.3 [M+H]⁺.

¹H **NMR** (400 MHz, DMSO-*d₆*) δ 9.21 (s, 1H), 8.77 (d, *J =* 1.6 Hz, 1H), 7.79 (s, 1H), 7.64 (t, *J =* 53.2 Hz, 1H), 6.11 - 6.02 (m, 1H), 5.13 (d, *J =* 10.8 Hz, 1H), 5.02 (d, *J =* 17.6 Hz, 1H), 4.93 - 4.91 (m, 2H), 3.71 - 3.67 (m, 2H), 3.46 - 3.41 (m, 1H), 3.18 - 3.15 (m, 2H), 3.08 - 3.05 (m, 2H), 2.94 - 2.91 (m, 2H), 1.44 - 1.42 (m, 2H), 1.29 - 1.27 (m, 8H).

### Example 077B2

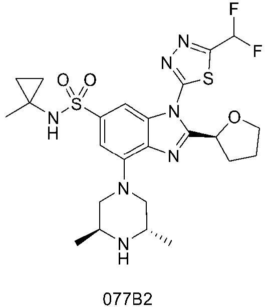

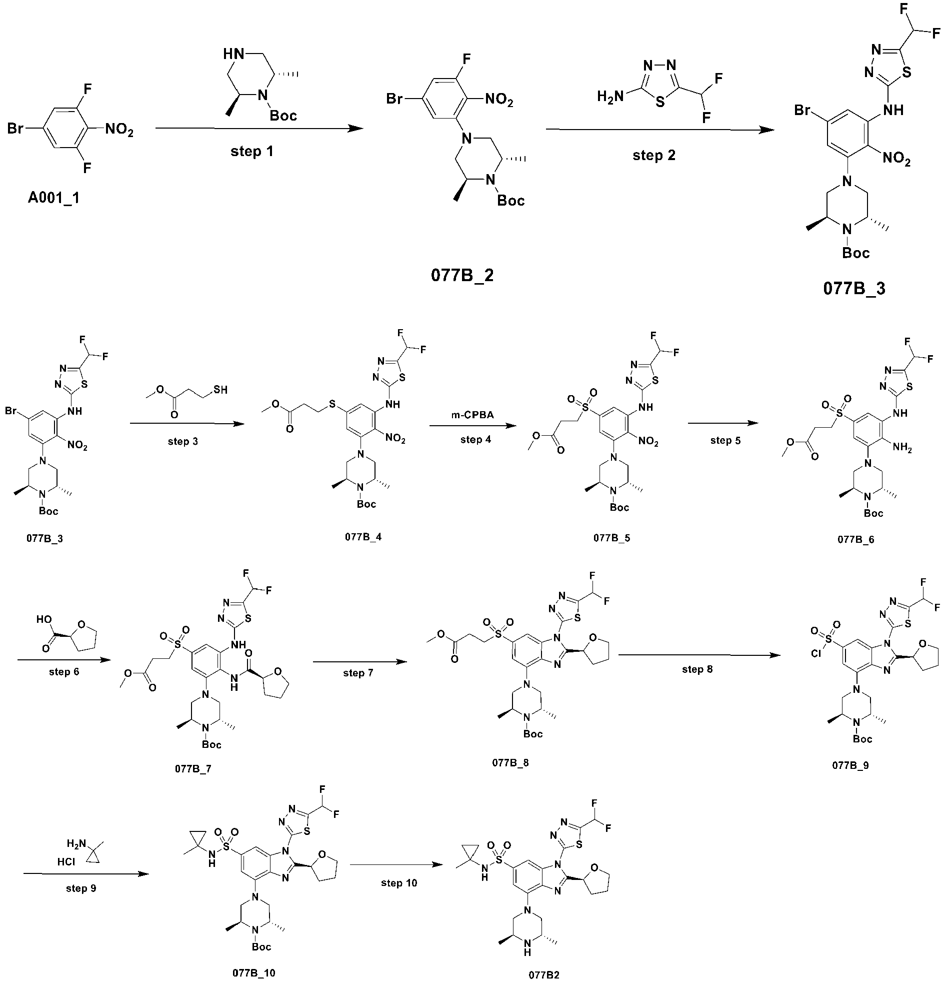

### Step 1: Synthesis of compound 077B_2

A001_1 (3.00 g, 12.6 mmol) and N,N-diisopropylethylamine (8.15 g, 63.0 mmol) were dissolved in N,N-dimethylacetamide (30 mL), followed by addition of *tert-butyl* (2S,6S)-2,6-dimethylpiperazine-1-carboxylate (2.70 g, 12.6 mmol). The reaction mixture was heated to 110°C and stirred for 3 hours. The reaction mixture was cooled to room temperature, then quenched with water (100 mL), and extracted with ethyl acetate (30 mL × 3). The organic phase was washed with brine (50 mL × 3), dried over anhydrous sodium sulfate, filtered, and the filtrate was concentrated by rotary evaporation. The residue was purified by column chromatography to obtain 077B_2 (5.06 g).

MS (ESI) M/Z: 332.2, 334.2 [M-Boc]⁺.

¹H NMR (400 MHz, DMSO-*d₆*): δ 7.21 - 7.18 (m, 1H), 7.14 (s, 1H), 4.06 - 4.03 (m, 2H), 3.79 - 3.75 (m, 2H), 2.99 - 2.96 (m, 2H), 1.42 (s, 9H), 1.15 (d, *J =* 6.4 Hz, 6H).

### Step 2: Synthesis of compound 077B_3

Cesium carbonate (11.4 g, 35.1 mmol) and 5-(difluoromethyl)-1,3,4-thiadiazol-2-amine (2.65 g, 17.6 mmol) were added to a solution of 077B_2 (5.06 g, 11.7 mmol) in N,N-dimethylacetamide (50 mL). The reaction mixture was heated to 110°C and stirred for 16 hours. The reaction mixture was cooled to room temperature, then quenched with water (100 mL), and extracted with ethyl acetate (30 mL × 3). The organic phase was washed with brine (50 mL × 3), dried over anhydrous sodium sulfate, filtered, and the filtrate was concentrated by rotary evaporation. The residue was purified by column chromatography to obtain 077B_3 (3.65 g).

MS (ESI) M/Z: 561.1, 563.1 [M-H]⁻.

### Step 3: Synthesis of compound 077B_4

077B_3 (10 g, 17.7 mmol) and methyl 3-mercaptopropionate (2.35 g, 19.5 mmol) were dissolved in 1,4-dioxane (100 mL), followed by addition of N,N-diisopropylethylamine (7.34 g, 56.8 mmol) and Xantphos (2.05 g, 3.55 mmol). The reaction mixture was purged with nitrogen three times at 25°C. Pd₂(dba)₃ (1.63 g, 1.77 mmol) was then added thereto. The reaction mixture was purged with nitrogen three times at 25°C, then heated to 85°C, and stirred for 2 hours. The reaction mixture was cooled to room temperature, then quenched with water (100 mL), and extracted with ethyl acetate (50 mL × 3). The organic phase was washed with brine (50 mL), dried over anhydrous sodium sulfate, filtered, and the filtrate was concentrated by rotary evaporation. The residue was purified by column chromatography to obtain 077B_4 (8 g).

MS (ESI) M/Z: 603.4 [M+H]⁺.

### Step 4: Synthesis of compound 077B_5

077B_4 (8 g, 13.2 mmol) was dissolved in dichloromethane (80.0 mL). The mixture was cooled to 0°C, and m-chloroperoxybenzoic acid (8.08 g, 39.8 mmol, 85% purity) was added in portions. The reaction mixture was stirred at 0°C for 1 hour. The reaction mixture was quenched with water (100 mL) and extracted with dichloromethane (50 mL × 3). The organic phase was washed with sodium sulfite aqueous solution (30 mL) and brine (30 mL), dried over anhydrous sodium sulfate, filtered, and the filtrate was concentrated by rotary evaporation. The residue was purified by column chromatography to obtain 077B_5 (5.7 g).

MS (ESI) M/Z: 657.1 [M+Na]⁺.

### Step 5: Synthesis of compound 077B_6

077B_5 (5.7 g, 8.98 mmol) was dissolved in ethanol (52.0 mL) and water (13.0 mL). The mixture was heated to 60°C, followed by portionwise addition of iron powder (2.51 g, 44.9 mmol) and ammonium chloride (2.40 g, 44.9 mmol). The reaction mixture was heated to 80°C and stirred for 2 hours. The reaction mixture was filtered through diatomite while hot. The filtrate was diluted with water (30 mL) and extracted with ethyl acetate (50 mL × 3). The organic phase was washed with brine (50 mL), dried over anhydrous sodium sulfate, filtered, and the filtrate was concentrated by rotary evaporation. The residue was purified by column chromatography to obtain 077B_6 (4.1 g).

MS (ESI) M/Z: 603.1 [M-H]⁻.

**¹H NMR** (400 MHz, DMSO-*d₆*) δ 9.95 (s, 1H), 7.78 (s, 1H), 7.48 - 7.21 (m, 2H), 5.65 (s, 2H), 4.03 - 4.00 (m, 2H), 3.54 (s, 3H), 3.45 (t, *J* = 7.2 Hz, 2H), 3.20 - 3.17 (m, 2H), 2.64 - 2.58 (m, 4H), 1.43 (s, 9H), 1.32 (d, *J =* 6.4 Hz, 6H).

### Step 6: Synthesis of compound 077B_7S

077B_6 (4.00 g, 6.61 mmol), S-(-)-tetrahydrofuran-2-carboxylic acid (921 mg, 7.94 mmol), HATU (3.02 g, 7.94 mmol), and N,N-diisopropylethylamine (2.56 g, 19.8 mmol) were dissolved in N,N-dimethylformamide (40.0 mL). The reaction mixture was stirred at 25°C for 1 hour. The reaction mixture was added with water (200 mL) and extracted with ethyl acetate (100 mL × 3). The combined organic phases were washed with brine (100 mL × 6), dried over anhydrous sodium sulfate, filtered, and concentrated under reduced pressure to obtain 077B_7 (5.00 g) as a crude product, which was directly used in the next step without further purification.

MS (ESI) M/Z: 703.4 [M+H]⁺.

### Step 7: Synthesis of compound 077B_8

077B_7S (5.00 g, 7.11 mmol) was dissolved in toluene (80.0 mL) and acetic acid (40.0 mL). The reaction mixture was stirred at 50°C for 12 hours. The reaction mixture was cooled to room temperature, added with water (200 mL), and extracted with ethyl acetate (100 mL × 3). The combined organic phases were washed with brine (100 mL × 6), dried over anhydrous sodium sulfate, filtered, and concentrated under reduced pressure. The residue was purified by column chromatography to obtain 077B_8 (3.20 g).

MS (ESI) M/Z: 685.1 [M+H]⁺.

¹H NMR (400 MHz, DMSO-*d₆*) δ 7.67 (t, *J =* 53.2 Hz, 1H), 7.52 (d, *J =* 1.2 Hz, 1H), 6.89 (d, *J =* 1.2 Hz, 1H), 5.40 - 5.37 (m, 1H), 4.19 - 4.14 (m, 2H), 4.05 - 4.01 (m, 4H), 3.82 - 3.76 (m, 1H), 3.72 - 3.66 (m, 1H), 3.57 (t, *J =* 7.2 Hz, 2H), 3.52 (s, 3H), 2.76 - 2.68 (m, 1H), 2.63 (t, *J* = 7.2 Hz, 2H), 2.31 - 2.22 (m, 1H), 2.09 - 2.00 (m, 1H), 1.96 - 1.92 (m, 1H), 1.45 (s, 9H), 1.25 (d, *J =* 6.4 Hz, 6H).

### Step 8: Synthesis of compound 077B_9

077B_8 (1.60 g, 2.34 mmol) was dissolved in tetrahydrofuran (16.0 mL), followed by addition of sodium methoxide (378 mg, 7.01 mmol) at 0°C. The reaction mixture was stirred at 0°C for 30 minutes. The solvent was removed by rotary evaporation at room temperature, and the residue was dried under oil pump vacuum for 15 minutes. The remaining solid was dissolved in dichloromethane (16.0 mL), followed by addition of N-chlorosuccinimide (312 mg, 2.34 mmol) in an ice bath. Two parallel batches were performed. The reaction mixture was warmed to 25°C and stirred for 1 hour. The reaction mixture was quenched with water (100 mL) and extracted with dichloromethane (100 mL × 3). The organic phase was washed with brine (100 mL × 3), dried over anhydrous sodium sulfate, filtered, and concentrated under reduced pressure to obtain 077B_9 (2.90 g) as a crude product, which was directly used in the next step without further purification.

MS (ESI) M/Z: 633.2 [M+H]⁺.

### Step 9: Synthesis of compound 077B_10

1-Methylcyclopropanamine hydrochloride (1.97 g, 18.3 mmol) and N,N-diisopropylethylamine (4.74 g, 36.6 mmol) were dissolved in dichloromethane (30.0 mL), followed by addition of 077B_9 (2.90 g, 4.58 mmol). The reaction mixture was stirred at 25°C for 1 hour. The reaction mixture was quenched with water (100 mL) and extracted with dichloromethane (50 mL × 3). The organic phase was washed with brine (100 mL × 3), dried over anhydrous sodium sulfate, filtered, and concentrated under reduced pressure. The residue was purified by column chromatography to obtain 077B_10S (1.47 g).

MS (ESI) M/Z: 668.4 [M+H]⁺.

¹H NMR (400 MHz, DMSO-*d₆*) δ 8.02 (s, 1H), 7.67 (t, *J =* 53.2 Hz, 1H), 7.50 (d, *J =* 1.2 Hz, 1H), 6.93 (d, *J* = 1.2 Hz, 1H), 5.38 - 5.35 (m, 1H), 4.19 - 4.15 (m, 2H), 3.99 - 3.95 (m, 4H), 3.82 - 3.77 (m, 1H), 3.74 - 3.68 (m, 1H), 2.77 - 2.69 (m, 1H), 2.29 - 2.22 (m, 1H), 2.09 - 2.01 (m, 1H), 1.96 - 1.90 (m, 1H), 1.45 (s, 9H), 1.24 (d, *J =* 6.4 Hz, 6H), 1.04 (s, 3H), 0.64 - 0.61 (m, 2H), 0.36 - 0.34 (m, 2H)

### Step 10: Synthesis of compound 077B2

077B_10 (1.47 g, 2.20 mmol) was dissolved in 1,4-dioxane (10.0 mL), followed by addition of 4 M HCl in dioxane (20 mL) at 25°C. The reaction mixture was stirred at 25°C for 0.5 hours. The reaction mixture was concentrated under reduced pressure. The residue was purified by preparative chromatography to obtain 077B2 (900 mg).

MS (ESI) M/Z: 568.1 [M+H]⁺.

¹H NMR (400 MHz, DMSO-*d₆*) δ 8.06 (s, 1H), 7.80 - 7.54 (m, 2H), 7.09 (d, *J* = 1.2 Hz, 1H), 5.40 - 5.37 (m, 1H), 3.83 - 3.74 (m, 2H), 3.45 - 3.39 (m, 4H), 3.28 - 3.22 (m, 2H), 2.75 - 2.67 (m, 1H), 2.33 - 2.23 (m, 1H), 2.12 - 2.03 (m, 1H), 2.01 - 1.93 (m, 1H), 1.17 (d, *J* = 6.4 Hz, 6H), 1.03 (s, 3H), 0.65 - 0.58 (m, 2H), 0.37 - 0.33 (m, 2H).

### Example 080B2

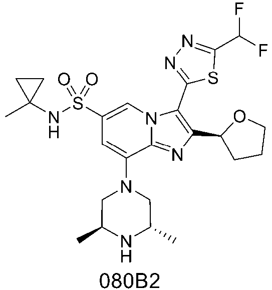

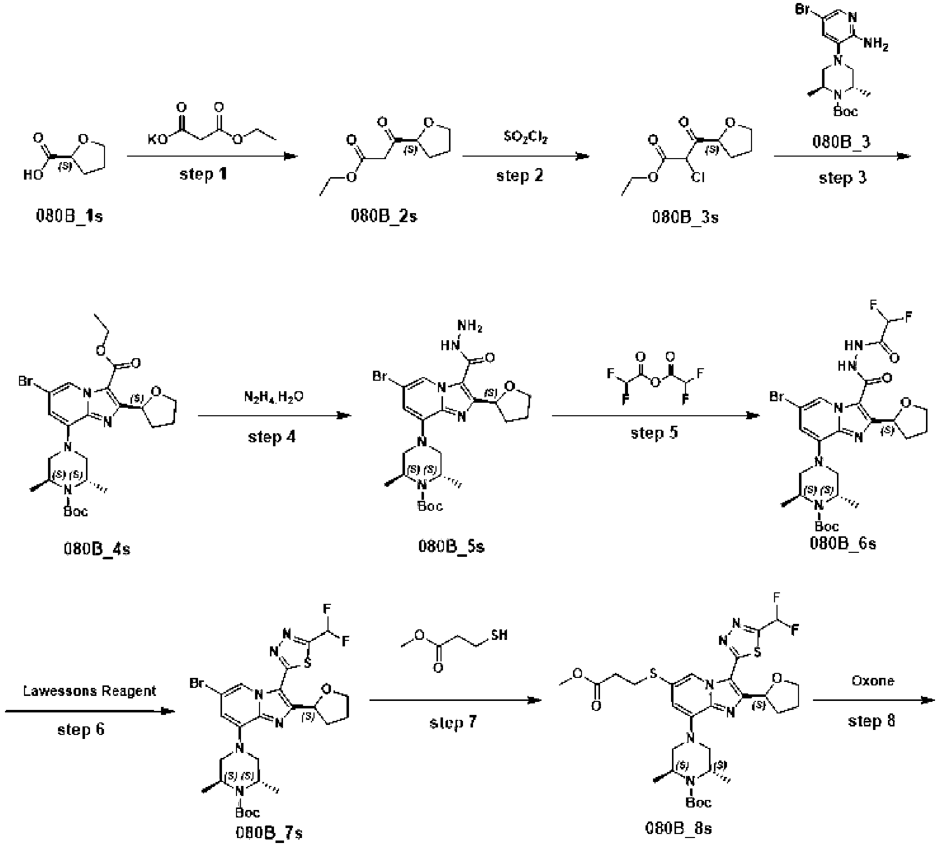

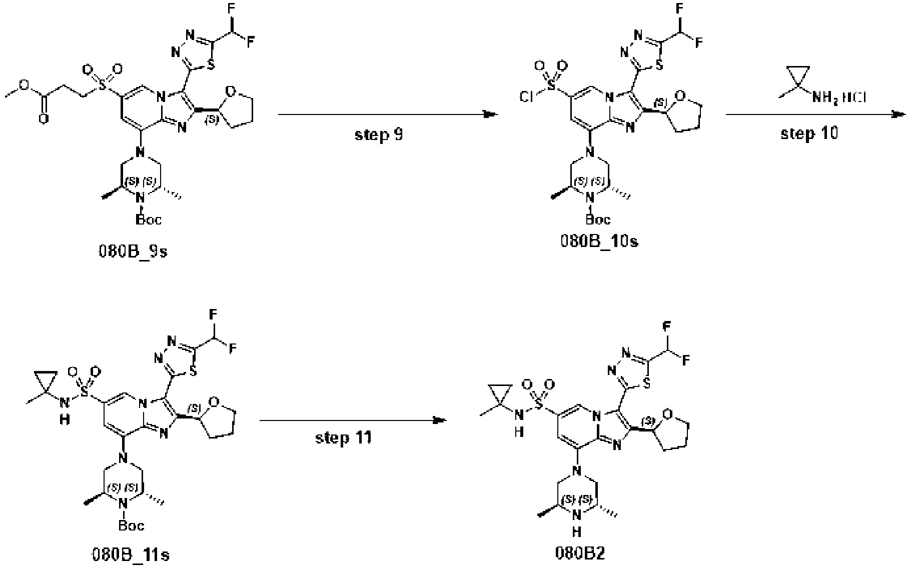

### Step 1: Synthesis of compound 080B_2s

080B_1s (60.0 g, 517 mmol) was dissolved in tetrahydrofuran (600 mL), followed by portionwise addition of 1,1-carbonyldiimidazole (CDI, 101 g, 620 mmol). After the addition was completed, the reaction mixture was stirred at 20°C for 1.5 hours. Magnesium chloride (48.2 g, 506 mmol) and ethyl potassium malonate (132 g, 775 mmol) were added in portions. The reaction mixture was stirred at 20°C for another 3 hours. The reaction mixture was quenched with water (600 mL), added with dilute hydrochloric acid (4 M) to adjust the pH to approximately 5, and extracted with ethyl acetate (500 mL × 2). The extract was washed with saturated brine (500 mL × 1), dried over anhydrous sodium sulfate, filtered, and the filtrate was concentrated by rotary evaporation. The crude product was purified by column chromatography to obtain 080B_2s (86.8 g).

MS (ESI) M/Z: 186.9 [M+H]⁺.

¹H NMR (400 MHz, CDCl₃): δ 4.40 - 4.36 (m, 1H), 4.19 (q, *J =* 7.2 Hz, 2H), 3.93 - 3.89 (m, 2H), 3.57 (s, 2H), 2.23 - 2.19 (m, 1H), 2.04 - 2.01 (m, 1H), 1.94 - 1.88 (m, 2H), 1.27 (t, *J =* 7.2 Hz, 3H).

### Step 2: Synthesis of compound 080B_3s

080B_2s (28.5 g, 153 mmol) was dissolved in dichloromethane (500 mL). The mixture was cooled to 0°C, followed by dropwise addition of sulfonyl chloride (20.6 g, 153 mmol). After the addition was completed, the reaction mixture was slowly warmed to 25°C and stirred for 2 hours. The reaction mixture was quenched with water (500 mL) and extracted with dichloromethane (400 mL × 2). The combined organic phases were washed with brine (400 mL × 1), dried over anhydrous sodium sulfate, and concentrated under reduced pressure at room temperature to obtain 080B_3s (100 g) as a crude product, which was directly used in the next step without further purification.

MS (ESI) M/Z: 218.9, 220.9 [M-H]⁻.

¹H NMR (400 MHz, CDCl₃): δ 5.19 - 5.15 (m, 1H), 4.63 - 4.60 (m, 1H), 4.28 - 4.25 (m, 2H), 3.96 - 3.82 (m, 2H), 2.25 - 2.12 (m, 2H), 1.95 - 1.85 (m, 2H), 1.33 - 1.28 (m, 3H).

### Step 3: Synthesis of compound 080B_4s

080B_3s (23.7 g, 107 mmol), 080B_3 (13.8 g, 35.8 mmol), and 4Å molecular sieves (14 g) were added to isopropanol (150 mL). The reaction mixture was heated to 100°C and stirred for 36 hours. The reaction mixture was cooled to room temperature and filtered. The filtrate was concentrated under reduced pressure at room temperature. The residue was dissolved in ethyl acetate (300 mL), washed with water (200 mL × 1) and saturated brine (200 mL × 1), dried over anhydrous sodium sulfate, filtered, and the filtrate was concentrated by rotary evaporation. The crude product was purified by column chromatography to obtain 080B_4s (9.8 g).

MS (ESI) M/Z: 551.4, 553.3 [M+H]⁺.

¹H NMR (400 MHz, CDCl₃): δ 8.96 (s, 1H), 6.38 (s, 1H), 5.76 - 5.73 (m, 1H), 4.47 - 4.41 (m, 2H), 4.24 - 4.20 (m, 3H), 4.02 - 3.99 (m, 5H), 2.31 - 2.19 (m, 4H), 1.51 (s, 9H), 1.43 (t, *J =* 7.2 Hz, 3H), 1.30 (d, *J =* 7.2 Hz, 6H).

### Step 4: Synthesis of compound 080B_5s

080B_4s (9.80 g, 17.8 mmol) was dissolved in ethanol (90 mL), followed by addition of hydrazine hydrate (37.0 g, 591 mmol, 80%). The reaction mixture was heated to 80°C and stirred for 16 hours. The reaction mixture was cooled to room temperature, quenched with water (150 mL), and extracted with ethyl acetate (100 mL × 3). The extract was washed with brine (100 mL × 2), dried over anhydrous sodium sulfate, filtered, and the filtrate was concentrated by rotary evaporation to obtain 080B_5s (7 g) as a crude product, which was directly used in the next step without further purification.

MS (ESI) M/Z: 537.3, 539.3 [M+H]⁺.

¹H NMR (400 MHz, CDCl₃): δ 9.58 (s, 1H), 9.05 (s, 1H), 6.32 (d, *J* = 1.2 Hz, 1H), 5.17 (t, *J =* 7.2 Hz, 1H), 4.23 - 4.21 (m, 2H), 4.14 - 4.11 (m, 2H), 4.02 - 3.95 (m, 6H), 2.86 - 2.81 (m, 1H), 2.27 - 2.24 (m, 1H), 2.16 - 2.07 (m, 2H), 1.50 (s, 9H), 1.30 (d, *J* = 6.4 Hz, 6H).

### Step 5: Synthesis of compound 080B_6s

080B_5s (7.00 g, 12.7 mmol) and triethylamine (3.84 g, 37.9 mmol) were dissolved in a mixed solvent of tetrahydrofuran (50.0 mL) and dichloromethane (50.0 mL). The mixture was cooled to 0°C, followed by dropwise addition of difluoroacetic anhydride (2.64 g, 15.2 mmol). The reaction mixture was naturally warmed to 25°C and stirred for 1 hour. The reaction mixture was quenched with water (100 mL) and extracted with dichloromethane (100 mL × 3). The extract was washed with brine (100 mL × 1), dried over anhydrous sodium sulfate, filtered, and the filtrate was concentrated by rotary evaporation. The crude product was purified by column chromatography to obtain 080B_6s (6.90 g).

MS (ESI) M/Z: 615.3, 617.3 [M+H]⁺.

¹H NMR (400 MHz, CDCl₃): δ 11.82 (s, 1H), 9.26 (s, 1H), 9.12 (d, *J* = 1.6 Hz, 1H), 6.39 (d, *J* = 1.2 Hz, 1H), 6.10 (t, *J* = 53.6 Hz, 1H), 5.26 (t, *J* = 7.2 Hz, 1H), 4.25 - 4.23 (m, 3H), 4.06 - 3.96 (m, 5H), 2.71 - 2.68 (m, 1H), 2.35 - 2.34 (m, 1H), 2.17 - 2.08 (m, 2H), 1.51 (s, 9H), 1.31 (d, *J* = 6.4 Hz, 6H).

### Step 6: Synthesis of compound 080B_7s

080B_6s (6.90 g, 10.9 mmol) and pyridine (4.32 g, 54.6 mmol) were dissolved in dioxane (70.0 mL). Lawesson's reagent (8.84 g, 21.9 mmol) was added in one portion at 25°C. The reaction mixture was heated to 80°C and stirred for 2 hours. The reaction mixture was cooled to room temperature, quenched with water (100 mL), and filtered. The filtrate was extracted with ethyl acetate (100 mL × 2), washed with brine (100 mL × 1), dried over anhydrous sodium sulfate, filtered, and concentrated under reduced pressure. The crude product was purified by column chromatography to obtain 080B_7s (4.6 g).

MS (ESI) M/Z: 613.3, 615.3 [M+H]⁺.

¹H NMR (400 MHz, CDCl₃): δ 9.40 (d, *J* = 1.2 Hz, 1H), 7.09 (t, *J* = 53.6 Hz, 1H), 6.43 (d, *J* = 1.2 Hz, 1H), 5.26 (t, *J* = 6.4 Hz, 1H), 4.27 - 4.25 (m, 2H), 4.05 - 3.97 (m, 6H), 2.78 - 2.73 (m, 1H), 2.33 - 2.29 (m, 2H), 2.24 - 2.22 (m, 1H), 1.51 (s, 9H), 1.33 (d, *J =* 6.4 Hz, 1H).

### Step 7: Synthesis of compound 080B_8s

080B_7s (4.6 g, 7.50 mmol), methyl 3-mercaptopropionate (991 mg, 8.25 mmol), and N,N-diisopropylethylamine (DIEA, 2.91 g, 22.5 mmol) were dissolved in dioxane (50.0 mL), and the system was purged with nitrogen three times. Xantphos (868 mg, 1.50 mmol) and Pd₂(dba)₃ (687 mg, 750 µmol) were added to the reaction mixture in one portion, and the system was purged with nitrogen three times. The reaction mixture was heated to 85°C and stirred for 2 hours. The reaction mixture was cooled to room temperature and filtered. The filtrate was concentrated under reduced pressure. The residue was purified by column chromatography to obtain 080B_8s (4.6 g).

MS (ESI) M/Z: 653.4 [M+H]⁺.

¹H NMR (400 MHz, CDCl₃): δ 9.34 (s, 1H), 7.09 (t, *J =* 54.0 Hz, 1H), 6.41 (d, *J =* 0.8 Hz, 1H), 5.28 (t, *J =* 6.8 Hz, 1H), 4.26 - 4.24 (m, 2H), 4.07 - 3.97 (m, 6H), 3.70 (s, 3H), 3.16 (t, *J* = 3.2 Hz, 2H), 2.75 - 2.74 (m, 1H), 2.66 (t, *J =* 7.2 Hz, 2H), 2.31 - 2.27 (m, 2H), 2.13 - 2.07 (m, 1H), 1.52 (s, 9H), 1.33 (d, *J =* 6.8 Hz, 6H).

### Step 8: Synthesis of compound 080B_9s

080B_8s (4.60 g, 7.05 mmol) was dissolved in methanol (150 mL), followed by portionwise addition of potassium peroxymonosulfate (oxone, 8.66 g, 14.1 mmol) at 25°C. The reaction mixture was stirred at 25°C for 1 hour. The reaction mixture was quenched with saturated sodium sulfite aqueous solution (100 mL) and extracted with ethyl acetate (100 mL × 3). The organic phase was washed with brine (200 mL), dried over anhydrous sodium sulfate, filtered, and concentrated under reduced pressure. The crude product was purified by column chromatography to obtain 080B_9s (2.9 g).

MS (ESI) M/Z: 685.2 [M+H]⁺.

¹H NMR (400 MHz, CDCl₃): δ 9.76 (d, *J =* 1.6 Hz, 1H), 7.11 (t, *J =* 53.6 Hz, 1H), 6.58 (s, 1H), 5.30 (t, *J* = 6.4 Hz, 1H), 4.30 - 4.28 (m, 2H), 4.12 - 4.00 (m, 6H), 3.67 (s, 3H), 3.55 (t, *J =* 7.6 Hz, 2H), 2.86 (t, *J =* 6.4 Hz, 2H), 2.73 - 2.72 (m, 1H), 2.37 - 2.34 (m, 1H), 2.22 - 2.21 (m, 1H), 2.15 - 2.14 (m, 1H), 1.52 (s, 9H), 1.34 (d, *J =* 6.8 Hz, 6H).

### Step 9: Synthesis of compound 080B_10s

080B_9s (2.90 g, 4.23 mmol) was dissolved in tetrahydrofuran (100 mL). The mixture was cooled to 0°C, and sodium methoxide (1.14 g, 21.2 mmol) was added in one portion. The reaction mixture was stirred at 0°C for 30 minutes. The reaction mixture was concentrated under reduced pressure at room temperature. The crude product was dissolved in dichloromethane (30 mL). The mixture was cooled to 0°C, and N-chlorosuccinimide (NCS, 566 mg, 4.24 mmol) was added in one portion. The reaction mixture was warmed to 25°C and stirred for 30 minutes. The reaction mixture was quenched with water (100 mL) and extracted with dichloromethane (50 mL × 3). The combined organic phases were washed with brine (100 mL × 1), dried over anhydrous sodium sulfate, filtered, and concentrated under reduced pressure at room temperature to obtain 080B_10s (2.68 g) as a crude product, which was directly used in the next step without further purification.

MS (ESI) M/Z: 633.2 [M+H]⁺.

### Step 10: Synthesis of compound 080B_11s

1-Methylcyclopropanamine hydrochloride (911 mg, 8.47 mmol) and N,N-diisopropylethylamine (DIEA, 1.64 g, 12.7 mmol) were dissolved in dichloromethane (30.0 mL), followed by addition of 080B_10s (2.68 g, 4.23 mmol). After the addition was completed, the reaction mixture was stirred at 25°C for 30 minutes. The reaction mixture was quenched with water (50 mL) and extracted with dichloromethane (50 mL × 2). The organic phase was washed with saturated brine (100 mL × 1), dried, and concentrated under reduced pressure. The residue was purified by column chromatography to obtain 080B_11s (1.20 g).

MS (ESI) M/Z: 668.5 [M+H]⁺.

¹H NMR (400 MHz, CDCl₃): δ 9.76 (d, *J* = 1.2 Hz, 1H), 7.10 (t, *J =* 53.6 Hz, 1H), 6.64 (d, *J* = 1.6 Hz, 1H), 5.29 (t, *J* = 6.8 Hz, 1H), 5.15 (s, 1H), 4.28 - 4.26 (m, 2H), 4.12 - 4.00 (m, 6H), 2.77 - 2.72 (m, 1H), 2.35 - 2.12 (m, 3H), 1.52 (s, 9H), 1.34 - 1.33 (m, 9H), 0.94 - 0.92 (m, 2H), 0.59 - 0.56 (m, 2H).

### Step 11: Synthesis of compound 080B2

080B_11s (1.20 g, 1.80 mmol) was dissolved in dioxane (5 mL), followed by addition of 4 M HCl in dioxane (5 mL). The reaction mixture was stirred at 20°C for 0.5 hours. After concentration, the crude product was purified by preparative chromatography (neutral conditions, column: Waters Xbridge Prep OBD C18) to obtain compound 080B2 (784.6 mg).

MS (ESI) M/Z: 568.2 [M+H]⁺.

¹H NMR (400 MHz, DMSO-*d₆*): δ 9.65 (s, 1H), 8.36 (s, 1H), 7.70 (t, *J =* 53.2 Hz, 1H), 6.94 (d, *J* = 1.2 Hz, 1H), 5.30 (t, *J* = 6.8 Hz, 1H), 3.91 - 3.88 (m, 2H), 3.50 - 3.46 (m, 2H), 3.37 - 3.36 (m, 2H), 3.30 - 3.27 (m, 2H), 2.59 - 2.53 (m, 1H), 2.30 - 2.25 (m, 1H), 2.12 - 2.06 (m, 2H), 1.19 (d, *J =* 6.4 Hz, 6H), 1.12 (s, 3H), 0.73 - 0.65 (m, 2H), 0.46 - 0.43 (m, 2H).

### Examples 080B, 080B1, and 080B2

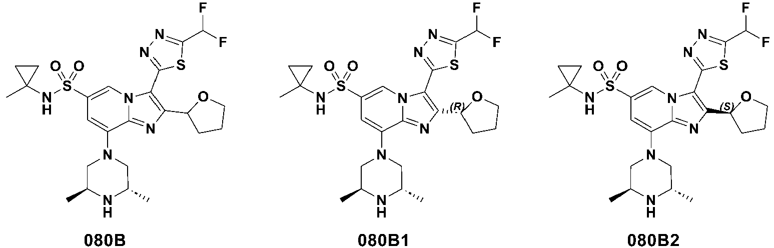

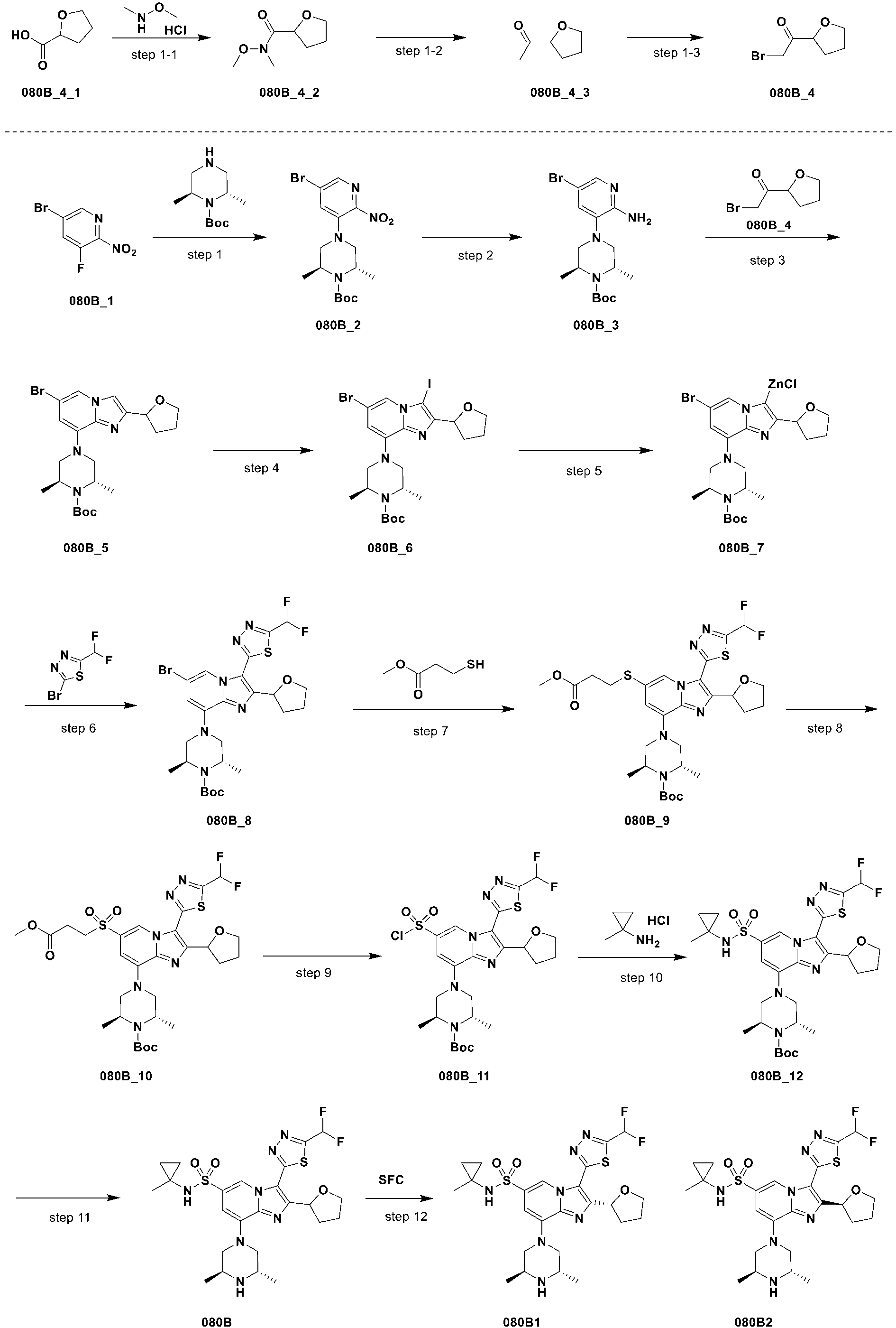

### Step 1-1: Synthesis of compound 080B_4_2

080B_4_1 (30.0 g, 258 mmol) and triethylamine (TEA, 131 g, 1.29 mol) were dissolved in tetrahydrofuran (500 mL), followed by addition of methoxymethylamine (15.7 g, 258 mmol) and butyl phosphate anhydride (50% solution in ethyl acetate, 372 g, 516 mmol). After the addition was completed, the reaction mixture was stirred at 20°C for 1 hour. The reaction mixture was quenched with water (1 L) and extracted with dichloromethane (500 mL × 6). The combined organic phases were washed with saturated brine (400 mL × 2), dried over anhydrous sodium sulfate, filtered, and concentrated under reduced pressure. The residue was purified by column chromatography to obtain 080B_4_2 (17.0 g).

MS (ESI) M/Z: 160.4 [M+H]⁺.

¹H NMR (400 MHz, CDCl₃-*d*): δ 4.77 (s, 1H), 4.05 - 4.00 (m, 1H), 3.96 - 3.88 (m, 1H), 3.72 (s, 3H), 3.20 (s, 3H), 2.23 - 2.15 (m, 1H), 2.02 - 1.84 (m, 3H).

### Step 1-2: Synthesis of compound 080B_4_3

080B_4_2 (5 g, 31.4 mmol) was dissolved in tetrahydrofuran (50 mL), and the system was purged with nitrogen three times. The mixture was cooled to 0°C, followed by dropwise addition of methylmagnesium bromide (3 M in diethyl ether, 15.71 mL). After the addition was completed, the reaction mixture was stirred at 0°C for 1 hour. After completion of the reaction, the reaction mixture was quenched with ammonium chloride aqueous solution (30 mL) and extracted with dichloromethane (15 mL × 6). The combined organic phases were washed with saturated brine (25 mL), dried over anhydrous sodium sulfate, filtered, and concentrated under reduced pressure to obtain 080B_4_3 (4.80 g) as a crude product, which was directly used in the next step without further purification.

### Step 1-3: Synthesis of compound 080B_4

080B_4_3 (3.60 g, 31.5 mmol) was dissolved in dichloromethane (40 mL), and the system was purged with nitrogen three times. The mixture was cooled to 0°C, followed by dropwise addition of triethylamine (3.83 g, 37.8 mmol) and trimethylsilyl trifluoromethanesulfonate (7.71 g, 34.7 mmol). After the addition was completed, the reaction mixture was stirred at 0°C for 0.5 hours. N-bromosuccinimide (6.17 g, 34.6 mmol) was then added thereto. The reaction mixture was stirred at 0°C for another 0.5 hours. The reaction mixture was quenched with water (30 mL) and extracted with dichloromethane (25 mL × 3). The combined organic phases were washed with saturated brine (25 mL), dried over anhydrous sodium sulfate, filtered, and concentrated under reduced pressure to obtain 080B_4 (7.00 g) as a crude product, which was directly used in the next step without further purification.

### Step 1: Synthesis of compound 080B_2

080B_1 (10 g, 45.2 mmol), *tert*-butyl (2S,6S)-2,6-dimethylpiperazine-1-carboxylate (9.70 g, 45.2 mmol), and N,N-diisopropylethylamine (29.2 g, 226 mmol) were dissolved in N,N-dimethylacetamide (100 mL). The mixture was heated to 110°C and stirred for 6 hours. The reaction mixture was cooled to room temperature, then quenched with water (150 mL), and extracted with ethyl acetate (100 mL × 2). The combined organic phases were washed with brine (100 mL × 1), dried over anhydrous sodium sulfate, and concentrated under reduced pressure at room temperature. The residue was purified by column chromatography to obtain 080B_2 (18 g).

MS (ESI) M/Z: 315.2, 317.2 [M-Boc+H]⁺.

¹H NMR (400 MHz, DMSO-*d₆*): δ 8.02 (d, *J* = 1.6 Hz, 1H), 7.93 (d, *J* = 2.0 Hz, 1H), 4.13 - 4.03 (m, 2H), 3.82 - 3.78 (m, 2H), 2.97 - 2.94 (m, 2H), 1.42 (s, 9H), 1.16 (d, *J* = 6.4 Hz, 6H).

### Step 2: Synthesis of compound 080B_3

080B_2 (18.0 g, 43.3 mmol) and ammonium chloride (11.6 g, 216 mmol) were dissolved in ethanol (200 mL) and water (50 mL). The reaction mixture was heated to 60°C, followed by portionwise addition of iron powder (12.1 g, 216 mmol). The resulting mixture was stirred at 85°C for 1 hour. The reaction mixture was filtered through diatomite while hot. The filtrate was concentrated under reduced pressure to remove most of the ethanol. The resulting mixture was diluted with water (100 mL) and extracted with ethyl acetate (150 mL × 3). The combined organic phases were washed with saturated brine (200 mL × 1), dried over anhydrous sodium sulfate, filtered, and the filtrate was concentrated by rotary evaporation. The residue was purified by column chromatography to obtain 080B_3 (15.2 g).

MS (ESI) M/Z: 385.2, 387.2 [M+H]⁺.

¹H NMR (400 MHz, DMSO-*d₆*): δ 7.70 (d, *J* = 2.0 Hz, 1H), 7.21 (d, *J* = 2.0 Hz, 1H), 5.78 (s, 2H), 4.00 - 3.95 (m, 2H), 3.28 - 3.24 (m, 2H), 2.63 - 2.59 (m, 2H), 1.42 (s, 9H), 1.26 (d, *J* = 6.4 Hz, 6H).

### Step 3: Synthesis of compound 080B_5

080B_3 (6 g, 15.6 mmol) and 080B_4 (6.01 g, 31.1 mmol) were dissolved in tetrahydrofuran (60 mL), followed by addition of sodium carbonate (908 mg, 8.56 mmol). The mixture was heated to 70°C and stirred for 16 hours. The reaction mixture was cooled to room temperature, then quenched with water (100 mL), and extracted with ethyl acetate (50 mL × 3). The combined organic phases were washed with saturated brine (50 mL), dried over anhydrous sodium sulfate, filtered, and the filtrate was concentrated by rotary evaporation. The residue was purified by column chromatography to obtain 080B_5 (3.40 g).

MS (ESI) M/Z: 479.3, 481.3 [M+H]⁺.

¹H NMR (400 MHz, DMSO-*d₆*): δ 8.16 (d, *J* = 1.2 Hz, 1H), 7.71 (d, *J =* 1.6 Hz, 1H), 6.17 (s, 1H), 4.95 (t, *J* = 7.2 Hz, 1H), 4.13 - 4.00 (m, 4H), 3.94 - 3.85 (m, 3H), 3.78 - 3.73 (m, 1H), 2.24 - 2.19 (m, 1H), 2.05 - 1.89 (m, 3H), 1.43 (s, 9H), 1.20 (d, *J* = 6.4 Hz, 6H).

### Step 4: Synthesis of compound 080B_6

080B_5 (2.90 g, 6.05 mmol) was dissolved in N,N-dimethylformamide (30.0 mL), followed by addition of N-iodosuccinimide (1.63 g, 7.26 mmol). The reaction mixture was stirred at 25°C for 16 hours. The reaction mixture was quenched with water (100 mL) and extracted with ethyl acetate (20 mL × 3). The combined organic phases were washed with saturated brine (50 mL × 3), dried, and concentrated. The residue was purified by column chromatography to obtain 080B_6 (2.00 g).

MS (ESI) M/Z: 605.2, 607.2 [M+H]⁺.

¹H NMR (400 MHz, DMSO-*d₆*): δ 7.81 (s, 1H), 6.33 (s, 1H), 4.96 - 4.92 (m, 1H), 4.10 - 4.07 (m, 2H), 4.04 - 4.01 (m, 1H), 3.93 - 3.88 (m, 4H), 3.80 - 3.74 (m, 1H), 2.26 - 2.20 (m, 1H), 2.18 - 2.09 (m, 2H), 1.98 - 1.93 (m, 1H), 1.43 (s, 9H), 1.20 (d, *J* = 6.8 Hz, 6H).

### Steps 5 and 6: Synthesis of compound 080B_8

080B_6 (200 mg, 330 µmol) was dissolved in tetrahydrofuran (2.00 mL), and the system was purged with nitrogen three times. The mixture was cooled to -65°C, followed by dropwise addition of isopropylmagnesium chloride (2.0 M in tetrahydrofuran, 185 µL). The reaction mixture was stirred at 0°C for 0.5 hours. A solution of dichloro(N,N,N,N-tetramethylethylenediamine)zinc (91.7 mg, 363 µmol) in tetrahydrofuran (1 mL) was added dropwise thereto at the same temperature. The reaction mixture was slowly warmed to room temperature and stirred for 0.5 hours. The reaction mixture was cooled again to 0°C, followed by dropwise addition of a solution of 2-bromo-5-(difluoromethyl)-1,3,4-thiadiazole (355 mg, 1.65 mmol) and tetrakis(triphenylphosphine)palladium (190 mg, 165 µmol) in tetrahydrofuran (1 mL). The reaction mixture was slowly warmed to 25°C and stirred for 1 hour. The reaction mixture was quenched with ammonium chloride aqueous solution (3 mL) and extracted with ethyl acetate (5 mL × 3). The combined organic phases were washed with brine (5 mL × 2), dried over anhydrous sodium sulfate, filtered, and the filtrate was concentrated by rotary evaporation. The residue was purified by thin-layer chromatography to obtain 080B_8 (50 mg).

MS (ESI) M/Z: 613.2, 615.2 [M+H]⁺.

¹H NMR (400 MHz, DMSO-*d₆*): δ 9.22 - 9.20 (m, 1H), 7.68 (t, *J* = 53.6 Hz, 1H), 6.63 (s, 1H), 5.26 - 5.22 (m, 1H), 4.15 - 4.10 (m, 3H), 4.02 - 3.98 (m, 2H), 3.90 - 3.85 (m, 3H), 2.64 - 2.57 (m, 1H), 2.29 - 2.20 (m, 1H), 2.17 - 2.00 (m, 2H), 1.44 (s, 9H), 1.24 (d, *J* = 6.8 Hz, 6H).

### Step 7: Synthesis of compound 080B_9

080B_8 (260 mg, 424 µmol), methyl 3-mercaptopropionate (56.0 mg, 466 µmol), and N,N-diisopropylethylamine (175 mg, 1.36 mmol) were dissolved in 1,4-dioxane (3 mL). The system was purged with nitrogen three times, followed by addition of Pd₂(dba)₃ (38.8 mg, 42.3 µmol) and Xantphos (49.0 mg, 84.7 µmol), and purged with nitrogen three additional times. The reaction mixture was heated to 85°C and stirred for 2 hours. The reaction mixture was cooled to room temperature, then quenched with water (10 mL), and extracted with ethyl acetate (15 mL × 3). The combined organic phases were washed with brine (20 mL), dried over anhydrous sodium sulfate, filtered, and the filtrate was concentrated by rotary evaporation. The crude product was purified by column chromatography to obtain 080B_9 (167 mg).

MS (ESI) M/Z: 653.2 [M+H]⁺.

### Step 8: Synthesis of compound 080B_10

080B_9 (100 mg, 153 µmol) was dissolved in methanol (4 mL), followed by addition of potassium peroxymonosulfate complex (188 mg, 306 µmol). The reaction mixture was stirred at 25°C for 2 hours. Two parallel batches were performed and combined for work-up. The reaction mixture was quenched with sodium sulfite aqueous solution (5 mL) and extracted with ethyl acetate (5 mL × 3). The combined organic phases were washed with brine (5 mL), dried over anhydrous sodium sulfate, and concentrated under reduced pressure at room temperature. The residue was purified by thin-layer chromatography to obtain 080B_10 (103 mg).

MS (ESI) M/Z: 685.2 [M+H]⁺.

¹H NMR (400 MHz, DMSO-*d₆*): δ 9.54 - 9.52 (m, 1H), 7.70 (t, *J* = 53.2 Hz, 1H), 6.72 - 6.70 (m, 1H), 5.32 - 5.28 (m, 1H), 4.17 - 4.12 (m, 2H), 4.08 - 4.03 (m, 3H), 3.92 - 3.85 (m, 3H), 3.70 (t, *J =* 7.2 Hz, 2H), 3.52 (s, 3H), 2.71 (t, *J* = 7.2 Hz, 2H), 2.30 - 2.23 (m, 1H), 2.18 - 2.00 (m, 3H), 1.44 (s, 9H), 1.26 (d, *J =* 6.8 Hz, 6H).

### Step 9: Synthesis of compound 080B_11

080B_10 (80.0 mg, 116 µmol) was dissolved in tetrahydrofuran (2 mL), and the system was purged with nitrogen three times. The reaction mixture was then cooled to 0°C, followed by addition of sodium methoxide (18.9 mg, 350 µmol), and stirred at room temperature for 30 minutes. The solvent was removed by rotary evaporation at room temperature, and the residue was dried under oil pump vacuum for 15 minutes. The solid was dissolved in dichloromethane (2 mL). The mixture was cooled to 0°C, followed by addition of N-chlorosuccinimide (15.6 mg, 116 µmol). The reaction mixture was slowly warmed to room temperature and stirred for 1 hour. The reaction mixture was quenched with water (10 mL) and extracted with dichloromethane (5 mL × 3). The combined organic phases were washed with brine (5 mL), dried over anhydrous sodium sulfate, filtered, and the filtrate was concentrated by rotary evaporation to obtain 080B_11 (74 mg) as a crude product, which was directly used in the next step without further purification.

MS (ESI) M/Z: 633.1 [M+H]⁺.

### Step 10: Synthesis of compound 080B_12

1-Methylcyclopropanamine hydrochloride (50.3 mg, 467 µmol) and N,N-diisopropylethylamine (121 mg, 934 µmol) were dissolved in dichloromethane (2.00 mL). A solution of 080B_11 (74.0 mg, 117 µmol) in dichloromethane (1 mL) was added dropwise to the reaction mixture. After the addition was completed, the reaction mixture was stirred at 20°C for 20 minutes. After completion of the reaction, the reaction mixture was quenched with water (10 mL) and extracted with dichloromethane (10 mL × 2). The combined organic phases were washed with saturated brine (10 mL × 1), dried over anhydrous sodium sulfate, filtered, and concentrated under reduced pressure. The residue was purified by column chromatography to obtain 080B_12 (46.0 mg).

MS (ESI) M/Z: 668.2 [M+H]⁺.

### Step 11: Synthesis of compound 080B

080B_12 (45.0 mg, 67.4 µmol) was dissolved in 1,4-dioxane (0.5 mL), followed by addition of HCl in dioxane (0.5 mL). The reaction mixture was stirred at 25°C for 1 hour. After concentration, the residue was purified by preparative chromatography to obtain 080B (14.67 mg).

MS (ESI) M/Z: 568.4 [M+H]⁺.

¹H NMR (400 MHz, DMSO-*d₆*) δ 9.65 (s, 1H), 8.35 (s, 1H), 7.70 (t, *J* = 53.2 Hz, 1H), 6.94 (s, 1H), 5.34 - 5.29 (m, 1H), 3.93 - 3.86 (m, 2H), 3.51 - 3.47 (m, 2H), 3.36 - 3.35 (m, 2H), 3.28 -3.26 (m, 2H), 2.65 - 2.56 (m, 1H), 2.30 - 2.23 (m, 1H), 2.17 - 2.01 (m, 2H), 1.18 (d, *J* = 6.4 Hz, 6H), 1.12 (s, 3H), 0.74 - 0.66 (m, 2H), 0.46 - 0.39 (m, 2H).

### Step 12: Synthesis of compounds 080B1 and 080B2

080B (60.0 mg, 89.8 µmol) was separated by preparative chromatography (SFC, column: DAICEL CHIRALCEL OD (250 mm × 30 mm, 10 µm); mobile phase), and purified by column chromatography (neutral conditions, column: WePure Biotech XP tC18) to obtain 080B1 (6.50 mg, peak 1, t = 1.647 min) and 080B2 (8.47 mg, peak 2, t = 1.896 min).

### 080B1:

MS (ESI) M/Z: 568.0 [M+H]⁺.

¹H NMR (400 MHz, DMSO-*d₆*): δ 9.64 (d, *J* = 1.6 Hz, 1H), 8.34 (s, 1H), 7.70 (t, *J =* 53.2 Hz, 1H), 6.94 (d, *J* = 1.2 Hz, 1H), 5.34 - 5.31 (m, 1H), 3.88 (t, *J* = 6.8 Hz, 2H), 3.50 - 3.46 (m, 2H), 3.37 - 3.34 (m, 2H), 3.28 - 3.24 (m, 2H), 2.67 - 2.61 (m, 1H), 2.30 - 2.24 (m, 1H), 2.15 - 2.03 (m, 2H), 1.19 (d, *J* = 6.4 Hz, 6H), 1.12 (s, 3H), 0.74 - 0.66 (m, 2H), 0.46 - 0.40 (m, 2H).

### 080B2:

MS (ESI) M/Z: 568.1 [M+H]⁺.

¹H NMR (400 MHz, DMSO-*d₆*): δ 9.65 (s, 1H), 8.35 (s, 1H), 7.70 (t, *J* = 53.6 Hz, 1H), 6.94 (s, 1H), 5.30 (t, *J* = 6.4 Hz, 1H), 3.95 - 3.85 (m, 2H), 3.50 - 3.46 (m, 2H), 3.36 - 3.32 (m, 2H), 3.28 - 3.26 (m, 2H), 2.65 - 2.56 (m, 1H), 2.32 - 2.23 (m, 1H), 2.17 - 2.02 (m, 2H), 1.19 (d, *J* = 6.4 Hz, 6H), 1.12 (s, 3H), 0.74 - 0.66 (m, 2H), 0.47 - 0.40 (m, 2H).

The retention time, PARG enzyme activity, or KURAMOCHI cell activity of compound 080B2 prepared in this example were essentially identical to those of example 080B2 prepared by chiral synthesis, confirming that the configuration of compound 080B2 prepared in this example is identical to that of example 080B2 prepared by chiral synthesis, both being the S configuration.

Other chiral compounds disclosed herein, such as 079J1 and 079J2, 083A1 and 083A2, were confirmed for their chiral structures by referring to the synthesis of 077B2 or 080B2 and PARG enzyme activity or KURAMOCHI cell activity data. The currently recorded structures correspond one-to-one with their retention times.

### Example 080J

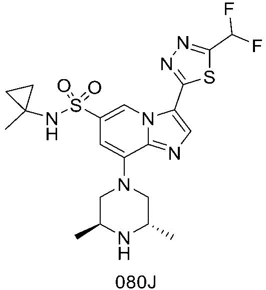

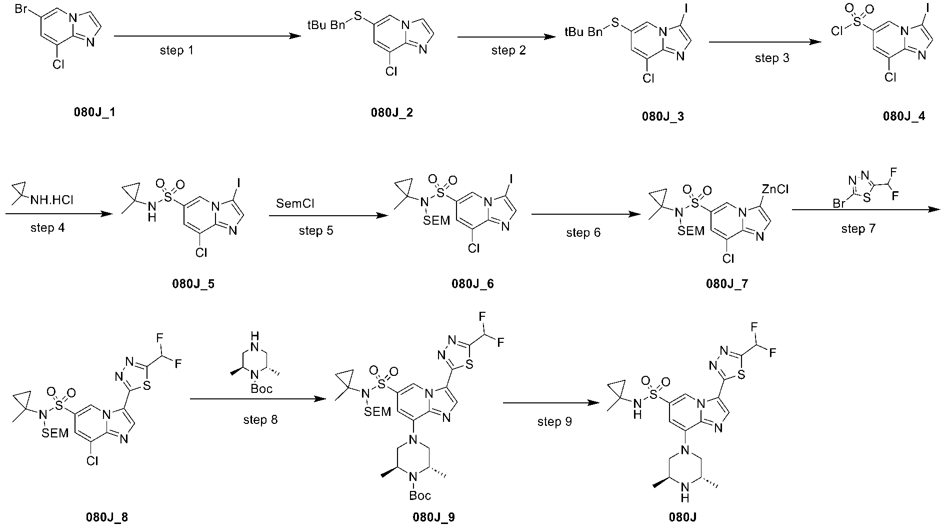

### Step 1: Synthesis of compound 080J_2

Compound 080J_1 (3 g, 12.96 mmol) was dissolved in 1,4-dioxane (30 mL), followed by addition of 4-*tert*-butylbenzyl mercaptan (3.51 g, 19.44 mmol), N,N-diisopropylethylamine (5.02 g, 38.88 mmol), Xantphos (1.50 g, 2.59 mmol), and Pd₂(dba)₃ (1.19 g, 1.30 mmol). The system was thoroughly purged with nitrogen. The reaction mixture was stirred at 100°C for 5 hours under nitrogen atmosphere. The reaction mixture was cooled to room temperature, then quenched with water, and extracted with ethyl acetate (200 mL × 3). The combined organic phases were washed with saturated brine (100 mL × 2), dried over anhydrous sodium sulfate, filtered, and the filtrate was concentrated by rotary evaporation. The residue was purified by column chromatography to obtain 080J_2 (3 g).

MS (ESI) M/Z: 331.1 [M+H]⁺.

### Step 2: Synthesis of compound 080J_3

080J_2 (1 g, 3.02 mmol) was dissolved in acetonitrile (10 mL), followed by addition of N-iodosuccinimide (0.75 g, 3.32 mmol) and trifluoroacetic acid (0.69 g, 6.04 mmol). The system was purged with nitrogen. The reaction mixture was stirred at room temperature for 1 hour. LCMS indicated complete conversion of the starting material, and the target product was detected by MS. The reaction mixture was quenched with water and extracted with ethyl acetate (100 mL × 3). The organic phase was washed with saturated brine (100 mL × 1), dried over anhydrous sodium sulfate, filtered, and the filtrate was concentrated by rotary evaporation. The residue was purified by column chromatography to obtain 080J_3 (1.2 g).

MS (ESI) M/Z: 457.0 [M+H]⁺.

### Step 3: Synthesis of compound 080J_4

080J_3 (1.1 g, 2.41 mmol) was dissolved in a mixture of acetonitrile/acetic acid/water (7/2/1, 10 mL), followed by portionwise addition of 1,3-dichloro-5,5-dimethylhydantoin (0.95 g, 4.82 mmol) in an ice bath. The system was thoroughly purged with nitrogen. The reaction mixture was stirred at room temperature for 2 hours under nitrogen atmosphere. The reaction mixture was quenched with water and extracted with ethyl acetate (100 mL × 3). The organic phase was washed with saturated brine (30 mL × 1), dried over anhydrous sodium sulfate, filtered, and the filtrate was concentrated by rotary evaporation. The residue was purified by column chromatography to obtain 080J_4 (0.9 g) as a crude product.

MS (ESI) M/Z: 376.9 [M+H]⁺.

### Step 4: Synthesis of compound 080J_5

080J_4 (0.9 g, 2.39 mmol) was dissolved in dichloromethane (10 mL), followed by addition of 1-methylcyclopropanamine hydrochloride (0.77 g, 7.17 mmol) in an ice bath, and triethylamine (0.73 g, 2.39 mmol) was slowly added thereto. The system was thoroughly purged with nitrogen. The reaction mixture was stirred in an ice bath for 2 hours. The reaction mixture was quenched with water and extracted with dichloromethane (100 mL × 3). The organic phase was washed with saturated brine (50 mL × 1), dried over anhydrous sodium sulfate, filtered, and the filtrate was concentrated by rotary evaporation. The residue was purified by column chromatography to obtain 080J_5 (0.65 g) as a crude product.

MS (ESI) M/Z: 412.0 [M+H]⁺.

### Step 5: Synthesis of compound 080J_6

080J_5 (1.8 g, 4.37 mmol) was dissolved in N,N-dimethylformamide (20 mL), followed by addition of cesium carbonate (2.85 g, 8.74 mmol) in an ice bath, and 2-(trimethylsilyl)ethoxymethyl chloride (0.87 g, 5.24 mmol) was slowly added thereto. The system was thoroughly purged with nitrogen. The reaction mixture was stirred at room temperature for 16 hours. The reaction mixture was quenched with water and extracted with ethyl acetate (100 mL × 3). The organic phase was washed with saturated brine (100 mL × 1), dried over anhydrous sodium sulfate, filtered, and the filtrate was concentrated by rotary evaporation. The residue was purified by column chromatography to obtain 080J_6 (2.1 g) as a crude product.

MS (ESI) M/Z: 542.0 [M+H]⁺.

### Steps 6 and 7: Synthesis of compound 080J_8

080J_6 (2.1 g, 3.88 mmol) was dissolved in tetrahydrofuran (20 mL). The system was thoroughly purged with nitrogen. The reaction mixture was then cooled to -78°C in a dry ice-ethanol bath. Isopropylmagnesium chloride-lithium chloride complex (0.85 g, 5.82 mmol, 1.3 mol/L) was slowly added dropwise thereto, and the mixture was stirred for 30 minutes. Dichloro(N,N,N',N'-tetramethylethylenediamine)zinc (1.47 g, 5.82 mmol) was added thereto, and the mixture was stirred at room temperature for 30 minutes. The reaction mixture was then cooled in an ice bath, followed by addition of 2-bromo-5-(difluoromethyl)-1,3,4-thiadiazole (1.25 g, 5.82 mmol) and tetrakis(triphenylphosphine)palladium (1.35 g, 1.16 mmol), and stirred at room temperature for 1 hour. The reaction mixture was added with saturated ammonium chloride solution (10 mL) in an ice bath and extracted with ethyl acetate (150 mL × 3). The organic phase was washed with saturated brine (100 mL × 1), dried over anhydrous sodium sulfate, filtered, and the filtrate was concentrated by rotary evaporation. The residue was purified by column chromatography to obtain 080J_8 (0.45 g).

MS (ESI) M/Z: 550.1 [M+H]⁺.

### Step 8: Synthesis of compound 080J_9

080J_8 (2.1 g, 3.88 mmol) was dissolved in 1,4-dioxane (10 mL), followed by addition of *tert*-butyl (2S,6S)-2,6-dimethylpiperazine-1-carboxylate (0.35 g, 1.64 mmol), cesium carbonate (0.8 g, 2.46 mmol), RuPhos (0.077 g, 0.16 mmol), and RuPhos Pd G3 (0.069 g, 0.082 mmol). The system was thoroughly purged with nitrogen. The reaction mixture was then stirred at 90°C for 3 hours. The reaction mixture was then cooled to room temperature, quenched with water, and extracted with ethyl acetate (150 mL × 3). The organic phase was washed with saturated brine (100 mL × 1), dried over anhydrous sodium sulfate, filtered, and the filtrate was concentrated by rotary evaporation. The residue was purified by column chromatography to obtain 080J_9 (0.35 g).

MS (ESI) M/Z: 728.2 [M+H]⁺.

### Step 9: Synthesis of compound 080J

080J_9 (87 mg, 0.12 mmol) was dissolved in dichloromethane (10 mL), followed by addition of trifluoroacetic acid (1 mL) and water (0.5 mL). The system was thoroughly purged with nitrogen. The reaction mixture was then stirred at room temperature for 3 hours. The reaction mixture was concentrated under reduced pressure to remove the solvent. The residue was purified by preparative chromatography to obtain 080J (0.043 g).

MS (ESI) M/Z: 498.2 [M+H]⁺.

### Examples 083A, 083A1, and 083A2

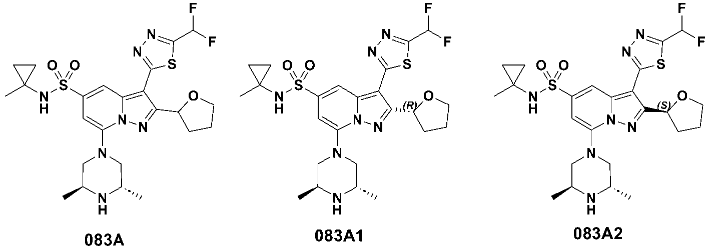

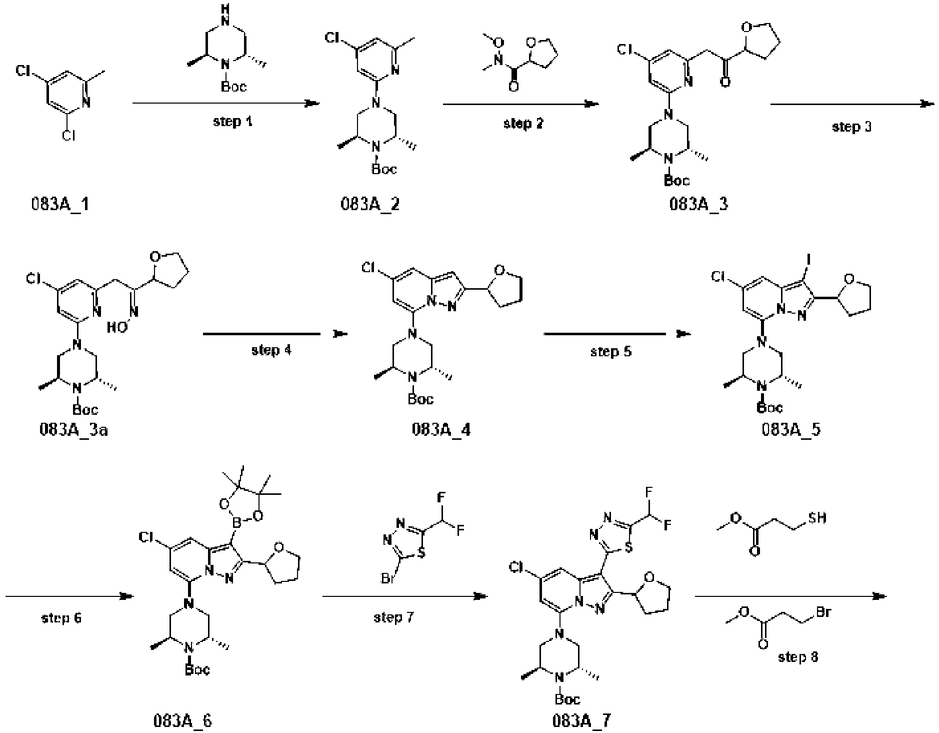

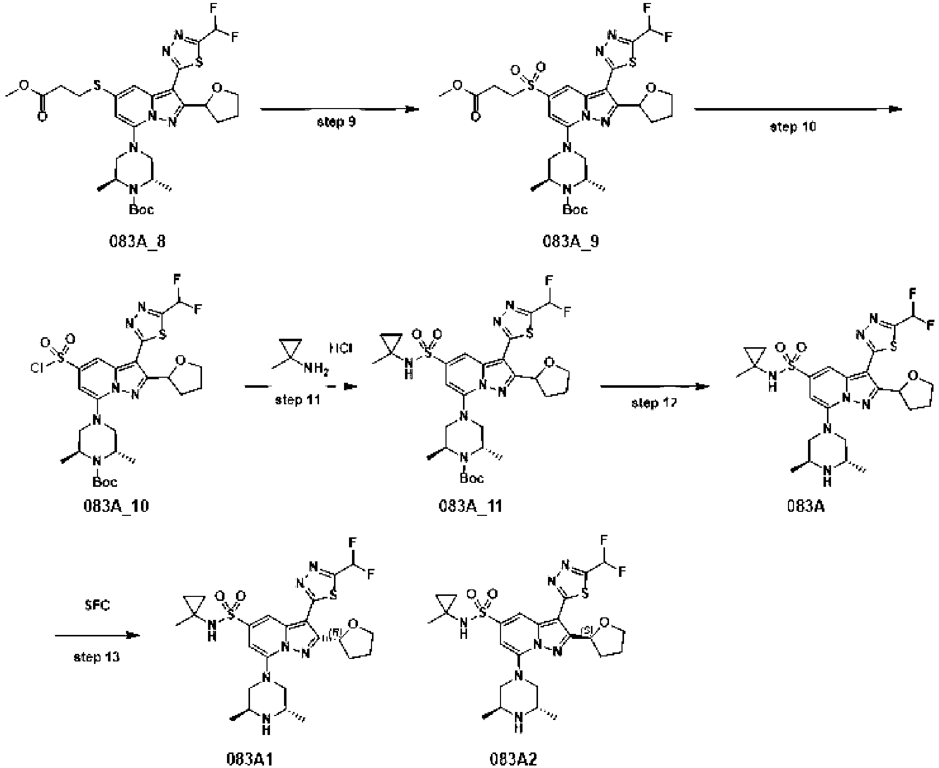

### Step 1: Synthesis of compound 083A_2

083A_1 (20.0 g, 123 mmol), *tert*-butyl (2S,6S)-2,6-dimethylpiperazine-1-carboxylate (26.5 g, 123 mmol), and anhydrous potassium phosphate (78.6 g, 370 mmol) were dissolved in 1,4-dioxane (500 mL). The system was purged with nitrogen three times, followed by addition of Pd(OAc)₂ (2.77 g, 12.3 mmol) and 1,1'-bis(diphenylphosphino)ferrocene (DPPF, 27.4 g, 49.4 mmol), and purged with nitrogen three additional times. The reaction mixture was heated to 85°C and stirred for 16 hours. The reaction mixture was cooled to room temperature and filtered through diatomite. The filtrate was concentrated by rotary evaporation. The crude product was purified by column chromatography to obtain 083A_2 (15.8 g).

MS (ESI) M/Z: 340.3 [M+H]⁺.

¹H NMR (400 MHz, DMSO-*d₆*): δ 6.57 (s, 1H), 6.42 (s, 1H), 4.10 - 4.08 (m, 2H), 3.82 - 3.79 (m, 2H), 3.56 - 3.52 (m, 2H), 2.30 (s, 3H), 1.43 (s, 9H), 1.14 (d, *J* = 6.4 Hz, 6H).

### Step 2: Synthesis of compound 083A_3

083A_2 (6.00 g, 17.7 mmol) was dissolved in tetrahydrofuran (60 mL). The system was purged with nitrogen three times, then cooled to -70°C, and LDA (2 M in tetrahydrofuran, 17.7 mL, 35.4 mmol) was added dropwise thereto. After the addition was completed, the reaction mixture was stirred at -70°C for 2 hours. A solution of N-methoxy-N-methyltetrahydrofuran-2-carboxamide (4.22 g, 26.5 mmol) in tetrahydrofuran (5 mL) was then added dropwise thereto. The reaction mixture was stirred at -70°C for another 1 hour. The reaction mixture was warmed to 0°C, quenched with saturated ammonium chloride aqueous solution (100 mL), and extracted with ethyl acetate (60 mL × 3). The combined organic phases were washed with brine (100 mL × 1), dried over anhydrous sodium sulfate, filtered, and concentrated under reduced pressure. The residue was purified by column chromatography to obtain 083A_3 (6.1 g).

MS (ESI) M/Z: 438.3 [M+H]⁺.

### Step 3: Synthesis of compound 083A_3a

083A_3 (4.00 g, 9.13 mmol), potassium acetate (1.79 g, 18.3 mmol), and hydroxylamine hydrochloride (1.27 g, 18.3 mmol) were dissolved in ethanol (40 mL) and water (5 mL). The reaction mixture was stirred at 25°C for 16 hours. The reaction mixture was quenched with water (70 mL) and extracted with ethyl acetate (50 mL × 2). The combined organic phases were washed with saturated brine (100 mL), dried over anhydrous sodium sulfate, filtered, and the filtrate was concentrated by rotary evaporation to obtain 083A_3a (4.00 g).

MS (ESI) M/Z: 453.3 [M+H]⁺.

¹H NMR (400 MHz, CDCl₃): δ 9.34 (s, 1H), 6.59 (s, 1H), 6.32 (d, *J* = 1.2 Hz, 1H), 4.55 (t, *J* = 7.2 Hz, 1H), 4.25 (s, 2H), 3.94 - 3.90 (m, 2H), 3.72 - 3.62 (m, 6H), 2.05 - 1.94 (m, 4H), 1.50 (s, 9H), 1.25 - 1.21 (m, 6H).

### Step 4: Synthesis of compound 083A_4

083A_3a (3.00 g, 6.62 mmol), triphenylphosphine (2.61 g, 9.93 mmol), imidazole (902 mg, 13.3 mmol), and elemental iodine (2.52 g, 9.93 mmol) were dissolved in tetrahydrofuran (100 mL). The reaction mixture was heated to 35°C and stirred for 0.5 hours under air atmosphere. The reaction mixture was cooled to room temperature, quenched with saturated sodium sulfite aqueous solution (100 mL), and extracted with ethyl acetate (100 mL × 2). The combined organic phases were washed with brine (100 mL × 2), dried over anhydrous sodium sulfate, filtered, and concentrated. The crude product was purified by reverse-phase chromatography to obtain 083A_4 (540 mg).

MS (ESI) M/Z: 435.1 [M+H]⁺.

¹H NMR (400 MHz, CDCl₃): δ 7.02 (d, *J* = 1.6 Hz, 1H), 6.38 (d, *J* = 2.4 Hz, 1H), 5.90 (d, *J* = 1.6 Hz, 1H), 5.14 (t, *J* = 6.8 Hz, 1H), 4.22 - 4.20 (m, 2H), 4.01 - 3.93 (m, 4H), 3.57 - 3.53 (m, 2H), 2.34 - 2.32 (m, 1H), 2.25 - 2.23 (m, 1H), 2.09 - 2.01 (m, 2H), 1.51 (s, 9H), 1.38 - 1.35 (m, 6H).

### Step 5: Synthesis of compound 083A_5

083A_4 (720 mg, 1.66 mmol) was dissolved in N,N-dimethylformamide (10.0 mL). N-Iodosuccinimide (372 mg, 1.66 mmol) was added in one portion at 25°C. The reaction mixture was stirred at 25°C for 1 hour. The reaction mixture was quenched with water (20 mL) and extracted with ethyl acetate (15 mL × 2). The extract was washed with saturated sodium sulfite aqueous solution (20 mL) and brine (10 mL × 1), dried over anhydrous sodium sulfate, filtered, and concentrated. The crude product was purified by column chromatography to obtain 083A_5 (800 mg).

MS (ESI) M/Z: 561.2 [M+H]⁺.

¹H NMR (400 MHz, CDCl₃): δ 7.01 (d, *J* = 1.6 Hz, 1H), 5.96 (d, *J* = 2.0 Hz, 1H), 5.20 - 5.15 (m, 1H), 4.22 - 4.21 (m, 2H), 4.10 - 4.08 (m, 1H), 4.05 - 3.97 (m, 3H), 3.56 - 3.52 (m, 2H), 2.40 - 2.37 (m, 1H), 2.30 - 2.19 (m, 2H), 2.09 - 2.05 (m, 1H), 1.51 (s, 9H), 1.36 - 1.34 (m, 6H).

### Step 6: Synthesis of compound 083A_6

083A_5 (900 mg, 1.60 mmol) and 2-isopropoxy-4,4,5,5-tetramethyl-1,3,2-dioxaborolane (896 mg, 4.81 mmol) were dissolved in tetrahydrofuran (10.0 mL). The system was purged with nitrogen three times. The reaction mixture was cooled to 0°C, followed by dropwise addition of isopropylmagnesium chloride (2 M, 2.41 mL), then warmed to 25°C, and stirred for 0.5 hours. The reaction mixture was quenched with saturated ammonium chloride aqueous solution (10 mL) and extracted with ethyl acetate (10 mL × 2). The combined organic phases were washed with brine (10 mL × 1), dried over anhydrous sodium sulfate, filtered, and concentrated under reduced pressure. The crude product was purified by column chromatography to obtain 083A_6 (600 mg).

MS (ESI) M/Z: 561.2 [M+H]⁺.

¹H NMR (400 MHz, CDCl₃): δ 7.49 (t, *J* = 2.0 Hz, 1H), 5.97 (t, *J* = 2.0 Hz, 1H), 5.58 - 5.53 (m, 1H), 4.21 - 4.18 (m, 2H), 4.01 - 3.97 (m, 4H), 3.57 - 3.53 (m, 2H), 2.32 - 2.23 (m, 3H), 2.21 - 2.04 (m, 1H), 1.51 (s, 9H), 1.38 - 1.34 (m, 6H), 1.29 (s, 12H).

### Step 7: Synthesis of compound 083A_7

083A_6 (600 mg, 1.07 mmol), 2-bromo-5-(difluoromethyl)-1,3,4-thiadiazole (690 mg, 3.21 mmol), and sodium carbonate (340 mg, 3.21 mmol) were dissolved in 1,4-dioxane (5.00 mL) and water (1.00 mL). The system was purged with nitrogen three times, followed by addition of XPhos (51.0 mg, 107 µmol) and XPhos Pd G2 (42.1 mg, 53.5 µmol), and purged with nitrogen three additional times. The reaction mixture was heated to 80°C and stirred for 4 hours. The reaction mixture was cooled to room temperature, then quenched with water (15 mL), and extracted with ethyl acetate (15 mL × 2). The combined organic phases were washed with saturated brine (20 mL × 1), dried over anhydrous sodium sulfate, filtered, and concentrated under reduced pressure. The crude product was purified by column chromatography to obtain 083A_7 (200 mg).

MS (ESI) M/Z: 569.3 [M+H]⁺.

¹H NMR (400 MHz, CDCl₃): δ 8.10 - 8.09 (m, 1H), 7.07 (t, *J* = 54.0 Hz, 1H), 6.17 (d, *J* = 1.6 Hz, 1H), 5.39 - 5.34 (m, 1H), 4.26 - 4.25 (m, 2H), 4.14 - 4.07 (m, 4H), 3.59 - 3.55 (m, 2H), 2.63 - 2.62 (m, 1H), 2.37 - 2.35 (m, 1H), 2.19 - 2.13 (m, 2H), 1.51 (s, 9H), 1.38 (d, *J* = 6.4 Hz, 6H).

### Step 8: Synthesis of compound 083A_8

Methyl 3-mercaptopropionate (127 mg, 1.05 mmol) was dissolved in N,N-dimethylacetamide (5.00 mL). The system was purged with nitrogen three times. The reaction mixture was cooled to 0°C in an ice-water bath, followed by addition of sodium hydride (56.2 mg, 1.41 mmol, 60% purity), and stirred at 0°C for 10 minutes. 083A_7 (200 mg, 351 µmol) was then added thereto. After the addition was completed, the reaction mixture was slowly warmed to 25°C and stirred for another 1 hour. Methyl 3-bromopropionate (294 mg, 1.76 mmol) was added thereto, and the mixture was stirred for 30 minutes. The reaction mixture was quenched with saturated ammonium chloride aqueous solution (10 mL) and extracted with ethyl acetate (10 mL × 2). The combined organic phases were washed with brine (20 mL × 1), dried over anhydrous sodium sulfate, filtered, and concentrated under reduced pressure. The residue was purified by column chromatography to obtain 083A_8 (200 mg).

MS (ESI) M/Z: 653.4 [M+H]⁺.

¹H NMR (400 MHz, CDCl₃): δ 7.93 (d, *J* = 2.0 Hz, 1H), 7.06 (t, *J* = 54.0 Hz, 1H), 6.09 (d, *J* = 1.6 Hz, 1H), 5.40 - 5.35 (m, 1H), 4.24 - 4.23 (m, 2H), 4.07 - 4.03 (m, 4H), 3.73 (s, 3H), 3.55 - 3.51 (m, 2H), 3.36 (t, *J* = 7.2 Hz, 2H), 2.79 (t, *J* = 7.2 Hz, 2H), 2.64 - 2.62 (m, 1H), 2.37 - 2.35 (m, 1H), 2.17 - 2.12 (m, 2H), 1.52 (s, 9H), 1.38 (d, *J* = 6.4 Hz, 6H).

### Step 9: Synthesis of compound 083A_9

083A_8 (200 mg, 306 µmol) was dissolved in methanol (10.0 mL), followed by addition of potassium peroxymonosulfate (oxone, 377 mg, 613 µmol) at room temperature. The reaction mixture was stirred at 25°C for 0.5 hours. The reaction mixture was quenched with sodium sulfite aqueous solution (15 mL) and extracted with ethyl acetate (15 mL × 2). The combined organic phases were washed with brine (30 mL), dried over anhydrous sodium sulfate, filtered, and concentrated under reduced pressure. The crude product was purified by column chromatography to obtain 083A_9 (160 mg).

MS (ESI) M/Z: 685.4 [M+H]⁺.

¹H NMR (400 MHz, CDCl₃): δ 8.55 (d, *J* = 1.6 Hz, 1H), 7.09 (t, *J* = 54.0 Hz, 1H), 6.56 (s, 1H), 5.43 - 5.38 (m, 1H), 4.30 - 4.20 (m, 4H), 4.10 - 3.95 (m, 2H), 3.67 - 3.62 (m, 5H), 3.56 - 3.52 (m, 2H), 2.88 - 2.83 (m, 2H), 2.75 - 2.55 (m, 1H), 2.45 - 2.35 (m, 1H), 2.20 - 2.10 (m, 2H), 1.53 (s, 9H), 1.39 (d, *J* = 6.4 Hz, 6H).

### Step 10: Synthesis of compound 083A_10

083A_9 (150 mg, 219 µmol) was dissolved in tetrahydrofuran (10 mL). The reaction mixture was cooled to 0°C, and sodium methoxide (59.2 mg, 1.10 mmol) was added in one portion. The reaction mixture was stirred at 0°C for 30 minutes. After concentration under reduced pressure at room temperature, the crude product was dissolved in dichloromethane (5 mL). The reaction mixture was cooled to 0°C, followed by addition of N-chlorosuccinimide (48.6 mg, 364 µmol), then slowly warmed to 25°C, and stirred for another 30 minutes. The reaction mixture was quenched with H₂O (10 mL) and extracted with dichloromethane (10 mL × 2). The combined organic phases were washed with saturated brine (20 mL × 1), dried over anhydrous sodium sulfate, filtered, and concentrated under reduced pressure at room temperature to obtain 083A_10 (230 mg) as a crude product, which was directly used in the next step without further purification.

MS (ESI) M/Z: 633.3 [M+H]⁺.

### Step 11: Synthesis of compound 083A_11

1-Methylcyclopropanamine hydrochloride (78.2 mg, 727 µmol) and N,N-diisopropylethylamine (141 mg, 1.09 mmol) were dissolved in dichloromethane (5.00 mL). A solution of 083A_10 (230 mg, 363 mmol) in dichloromethane (1 mL) was added dropwise to the reaction mixture. After the addition was completed, the reaction mixture was stirred at 25°C for 10 minutes. The reaction mixture was quenched with water (10 mL) and extracted with dichloromethane (10 mL × 2). The combined organic phases were washed with saturated brine (10 mL × 1), dried over anhydrous sodium sulfate, filtered, and concentrated under reduced pressure. The residue was purified by column chromatography to obtain 083A_11 (125 mg).

MS (ESI) M/Z: 668.1 [M+H]⁺.

¹H NMR (400 MHz, CDCl₃): δ 8.53 - 8.52 (m, 1H), 7.08 (t, *J* = 53.6 Hz, 1H), 6.61 (d, *J* = 1.6 Hz, 1H), 5.40 (q, *J* = 6.8 Hz, 1H), 5.12 (s, 1H), 4.29 - 4.26 (m, 2H), 4.19 - 4.15 (m, 2H), 4.13 - 3.95 (m, 2H), 3.63 - 3.59 (m, 2H), 2.66 - 2.63 (m, 1H), 2.40 - 2.38 (m, 1H), 2.19 - 2.15 (m, 2H), 1.52 (s, 9H), 1.38 (d, *J* = 6.8 Hz, 6H), 1.33 (s, 3H), 0.93 - 0.90 (m, 2H), 0.59 - 0.55 (m, 2H).

### Step 12: Synthesis of compound 083A

083A_11 (120 mg, 180 µmol) was dissolved in dioxane (1 mL), followed by addition of 4 M HCl in dioxane (1 mL). The reaction mixture was stirred at 20°C for 0.5 hours. After concentration, the residue was purified by preparative chromatography to obtain 083A (80.0 mg).

MS (ESI) M/Z: 568.1 [M+H]⁺.

¹H NMR (400 MHz, DMSO-*d₆*): δ 8.48 (d, *J* = 1.6 Hz, 1H), 8.45 (s, 1H), 7.65 (t, *J* = 53.2 Hz, 1H), 6.80 (s, 1H), 5.43 - 5.38 (m, 1H), 3.92 - 3.85 (m, 2H), 3.37 - 3.23 (m, 6H), 2.62 - 2.60 (m, 1H), 2.33 - 2.31 (m, 1H), 2.11 - 2.05 (m, 2H), 1.26 (d, *J* = 4.2 Hz, 6H), 1.11 (s, 3H), 0.74 - 0.65 (m, 2H), 0.48 - 0.44 (m, 2H).

### Step 13: Synthesis of compounds 083A1 and 083A2

083A (65 mg, 115 µmol) was separated by SFC (column: DAICEL CHIRALPAK IG) to obtain 083A1 (25.50 mg, peak 1, t = 1.590 min) and 083A2 (24.22 mg, peak 2, t = 1.898 min).

### 083A2:

MS (ESI) M/Z: 568.1 [M+H]⁺.

¹H NMR (400 MHz, DMSO-*d₆*): δ 8.48 (d, *J* = 1.6 Hz, 1H), 8.45 (s, 1H), 7.65 (t, *J* = 53.2 Hz, 1H), 6.80 (s, 1H), 5.42 (t, *J* = 6.4 Hz, 1H), 3.93 - 3.85 (m, 2H), 3.42 - 3.40 (m, 4H), 3.28 - 3.25 (m, 2H), 2.64 - 2.62 (m, 1H), 2.35 - 2.29 (m, 1H), 2.09 - 2.05 (m, 2H), 1.27 (d, *J*= 5.6 Hz, 6H), 1.11 (s, 3H), 0.74 - 0.65 (m, 2H), 0.48 - 0.44 (m, 2H).

### 083A1:

MS (ESI) M/Z: 568.1 [M+H]⁺.

¹H NMR (400 MHz, DMSO-*d₆*): δ 8.49 (d, *J* = 1.6 Hz, 1H), 8.45 (s, 1H), 7.66 (t, *J* = 53.2 Hz, 1H), 6.81 (s, 1H), 5.40 (t, *J* = 6.4 Hz, 1H), 3.93 - 3.89 (m, 2H), 3.46 - 3.40 (m, 4H), 3.28 - 3.25 (m, 2H), 2.59 - 2.50 (m, 1H), 2.33 - 2.30 (m, 1H), 2.11 - 2.04 (m, 2H), 1.29 (d, *J*= 5.2 Hz, 6H), 1.11 (s, 3H), 0.75 - 0.66 (m, 2H), 0.48 - 0.44 (m, 2H).

The compounds listed in Table 14 below can be synthesized according to the synthetic methods described in the above examples, or according to the synthetic schemes of Example 5, Example 14, Example 23, Example 27, Example 29, Example 36, Example 40, Reference Example 1, and comparative compound, or according to methods reported in the prior literature.

**Table 14**

| Example | Structural formula | Structural characterization |
|---|---|---|
| A031 | 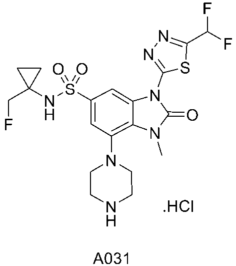 | ¹H **NMR** (400 MHz, DMSO-*d₆*) δ 9.25 (s, br. 1H), 8.93 (s, br. 1H), 8.68 (d, *J =* 1.6 Hz, 1H), 8.65 (s, 1H), 7.77 - 7.51 (m, 2H), 4.25 (s, 1H), 4.13 (s, 1H), 3.78 (s, 3H), 3.41 - 3.38 (m, 2H), 3.31 - 3.26 (m, 4H), 3.12 - 3.09 (m, 2H), 0.73 - 0.71 (m, 4H). |
| | | **MS (ESI) M/Z:** 518.1 [M+H]⁺. |
| A032 | 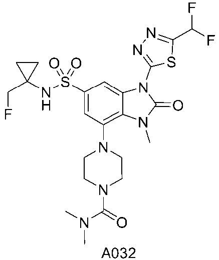 | **¹H NMR** (400 MHz, DMSO-*d₆*) δ 8.64 (d, *J =* 1.6 Hz, 1H), 8.51 (s, 1H), 7.77 - 7.50 (m, 2H), 4.24 (s, 1H), 4.12 (s, 1H), 3.79 (s, 3H), 3.65 - 3.56 (m, 2H), 3.15 - 3.05 (m, 4H), 2.92 - 2.84 (m, 2H), 2.80 (s, 6H), 0.72 - 0.69 (m, 4H). |
| | | **MS (ESI) M/Z:** 589.2 [M+H]⁺. |
| A033 | 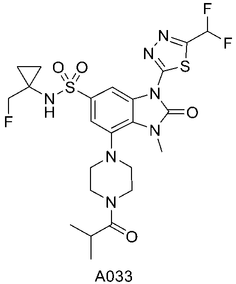 | **¹H NMR** (400 MHz, DMSO-*d₆*) δ 8.65 (d, *J* = 1.6 Hz, 1H), 8.51 (s, 1H), 7.77 - 7.50 (m, 2H), 4.55 - 4.48 (m, 1H), 4.24 (s, 1H), 4.12 (s, 1H), 4.10 - 4.05 (m, 1H), 3.80 (s, 3H), 3.42 - 3.35 (m, 1H), 3.18 - 3.15 (m, 2H), 3.00 - 2.90 (m, 2H), 2.86 - 2.76 (m, 1H), 2.68 - 2.66 (m, 1H), 1.08 - 1.03 (m, 6H), 0.72 - 0.70 (m, 4H). |
| | | **MS (ESI) M/Z:** 588.1 [M+H]⁺. |
| A034 | 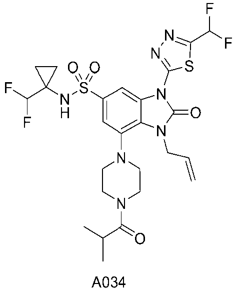 | **¹H NMR** (400 MHz, DMSO-*d₆*) δ 8.75 (s, 1H), 8.70 (d, *J* = 1.6 Hz, 1H), 7.79 - 7.50 (m, 2H), 6.12 - 6.05 (m, 1H), 5.83 (t, *J =* 56.8 Hz, 1H), 5.17 - 5.08 (m, 2H), 4.95 - 4.92 (m, 2H), 4.52 - 4.42 (m, 1H), 4.10 - 4.00 (m, 1H), 3.37 - 3.35 (m, 1H), 3.08 - 2.99 (m, 2H), 2.98 - 2.90 (m, 1H), 2.88 - 2.77 (m, 2H), 2.75 - 2.64 (m, 1H), 1.10 - 0.99 (m, 6H), 0.94 - 0.88 (m, 2H), 0.78 - 0.75 (m, 2H). |
| | | **MS (ESI) M/Z:** 632.2 [M+H]⁺. |
| A035 | 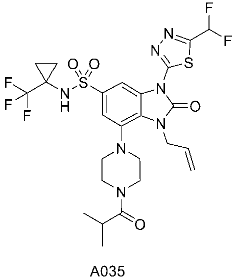 | **¹H NMR** (400 MHz, DMSO-*d₆*) δ 9.16 (s, 1H), 8.70 (d, *J* = 1.6 Hz, 1H), 7.78 - 7.51 (m, 2H), 6.12 - 6.05 (m, 1H), 5.17 - 5.08 (m, 2H), 4.94 - 4.92 (m, 2H), 4.53 - 4.41 (m, 1H), 4.11 - 3.98 (m, 1H), 3.37 - 3.35 (m, 1H), 3.09 - 2.99 (m, 2H), 2.98 - 2.90 (m, 1H), 2.87 - 2.77 (m, 2H), 2.74 - 2.64 (m, 1H), 1.21 - 1.17 (m, 2H), 1.10 - 0.99 (m, 8H). |
| | | **MS (ESI) M/Z:** 650.3 [M+H]⁺. |
| A036 | 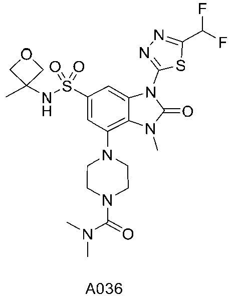 | **¹H NMR** (400 MHz, DMSO-*d₆*) δ 8.66 (d, *J* = 1.6 Hz, 1H), 8.42 (s, 1H), 7.64 - 7.50 (m, 2H), 4.53 (d, *J* = 6.0 Hz, 2H), 4.09 (d, *J* = 6.4 Hz, 2H), 3.79 (s, 3H), 3.70 - 3.51 (m, 2H), 3.18 - 3.01 (m, 4H), 2.95 - 2.83 (m, 2H), 2.80 (s, 6H), 1.43 (s, 3H). |
| | | **MS (ESI) M/Z:** 587.1 [M+H]⁺. |
| A037 | 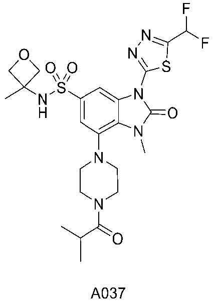 | **¹H NMR** (400 MHz, DMSO-*d₆*) δ 8.67 (d, J= 1.6 Hz, 1H), 8.40 (s, 1H), 7.77 - 7.50 (m, 2H), 4.53 (d, *J* = 6.0 Hz, 2H), 4.51 - 4.41 (m, 1H), 4.09 (d, *J* = 6.0 Hz, 2H), 4.07 - 3.95 (m, 1H), 3.80 (s, 3H), 3.47 - 3.36 (m, 1H), 3.24 - 3.09 (m, 2H), 3.03 - 2.88 (m, 2H), 2.88 - 2.77 (m, 1H), 2.76 - 2.62 (m, 1H), 1.43 (s, 3H), 1.04 (s, 6H). |
| | | **MS (ESI) M/Z:** 586.1 [M+H]⁺. |
| A038 | 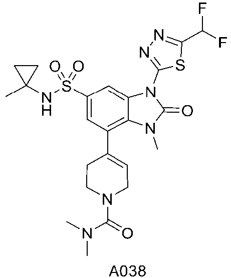 | **¹H NMR** (400 MHz, DMSO-*d₆*) δ 8.78 (s, 1H), 8.16 (s, 1H), 7.64 (t, *J* = 53.2 Hz, 1H), 7.53 (s, 1H), 5.84 (s, 1H), 3.89 - 3.87 (m, 2H), 3.47 (s, 3H), 3.44 - 3.41 (m, 2H), 2.80 (s, 6H), 2.48 - 2.46 (m, 2H), 1.09 (s, 3H), 0.64 - 0.61 (m, 2H), 0.40 - 0.36 (m, 2H). |
| | | **MS (ESI) M/Z:** 568.2 [M+H]⁺. |
| A039 | 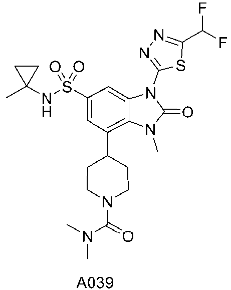 | **¹H NMR** (400 MHz, DMSO-*d₆*) δ 8.78 (d, *J* = 1.6 Hz, 1H), 8.19 (s, 1H), 7.72 (d, *J* = 1.6 Hz, 1H), 7.64 (t, *J* = 53.2 Hz, 1H), 3.78 (s, 3H), 3.73 - 3.69 (m, 2H), 3.56 (t, *J* = 12.0 Hz, 1H), 2.95 (t, *J =* 12.0 Hz, 2H), 2.78 (s, 6H), 1.88 - 1.85 (m, 2H), 1.76 - 1.68 (m, 2H), 1.05 (s, 3H), 0.63 - 0.60 (m, 2H), 0.39 - 0.35 (m, 2H). |
| | | **MS (ESI) M/Z:** 570.2 [M+H]⁺. |
| A040 | 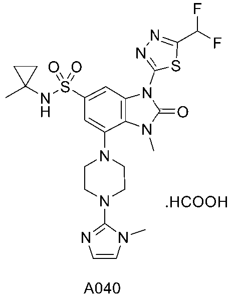 | **¹H NMR** (400 MHz, DMSO-*d₆*) δ 8.67 (d, *J* = 1.6 Hz, 1H), 8.17 (s, 1H), 7.77 - 7.51 (m, 2H) 6.91 (d, *J* = 1.2 Hz, 1H), 6.64 (d, *J* = 1.6 Hz, 1H), 3.82 (s, 3H), 3.51 (s, 3H), 3.21 - 3.12 (m, 8H), 1.09 (s, 3H), 0.66 - 0.63 (m, 2H), 0.41 - 0.38 (m, 2H). |
| | | **MS (ESI) M/Z:** 580.1 [M+H]⁺. |
| A041 | 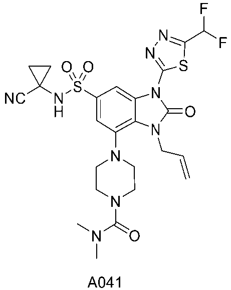 | **¹H NMR** (400 MHz, DMSO-*d₆*) δ 9.14 (s, 1H), 8.76 (d, *J* = 0.8 Hz, 1H), 7.78 - 7.52 (m, 2H), 6.09 - 6.02 (m, 1H), 5.15 - 5.12 (m, 1H), 5.07 - 5.02 (m, 1H), 4.94 - 4.93 (m, 2H), 3.59 - 3.56 (m, 2H), 3.04 - 2.91 (m, 6H), 2.80 (s, 6H), 1.44 - 1.40 (m, 2H), 1.30 - 1.26 (m, 2H). |
| | | **MS (ESI) M/Z:** 608.1 [M+H]⁺. |
| A042 | 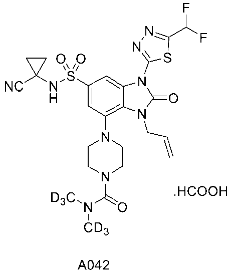 | **¹H NMR** (400 MHz, CDCl₃-*d*) δ 8.99 (s, 1H), 7.88 (s, 1H), 7.06 (t*, J =* 53.6 Hz, 1H), 6.00 - 5.95 (m, 1H), 5.90 (s, 1H), 5.24 - 5.22 (m, 1H), 5.15 - 5.11 (m, 1H), 5.05 - 5.04 (m, 2H), 3.71 - 3.69 (m, 2H), 3.15 - 3.05 (m, 6H), 1.69 - 1.65 (m, 2H), 1.46 - 1.43 (m, 2H). |
| | | **MS (ESI) M/Z:** 614.2 [M+H]⁺. |
| A043 | 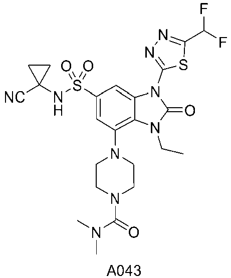 | **¹H NMR** (400 MHz, DMSO-*d₆*) δ 8.73 (s, 1H), 7.77 - 7.51 (m, 2H), 4.31 - 4.29 (m, 2H), 3.64 - 3.61 (m, 2H), 3.07 - 2.95 (m, 6H), 2.81 (s, 6H), 1.43 - 1.41 (m, 2H), 1.34 - 1.30 (m, 3H), 1.27 - 1.23 (m, 2H). |
| | | **MS (ESI) M/Z:** 596.1 [M+H]⁺. |
| A044 | 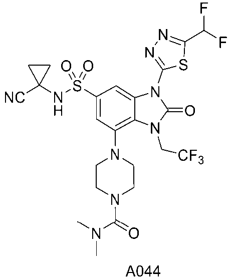 | **¹H NMR** (400 MHz, DMSO-*d₆*) δ 9.28 (s, 1H), 8.80 (d, *J* = 1.2 Hz, 1H), 7.92 (d, *J* = 1.6 Hz, 1H), 7.66 (t, *J =* 52.8 Hz, 1H), 5.22 - 5.15 (m, 2H), 3.63 - 3.60 (m, 2H), 3.00 - 2.93 (m, 6H), 2.81 (s, 6H), 1.46 - 1.42 (m, 2H), 1.32 - 1.28 (m, 2H). |
| | | **MS (ESI) M/Z:** 650.2 [M+H]⁺. |
| A045 | 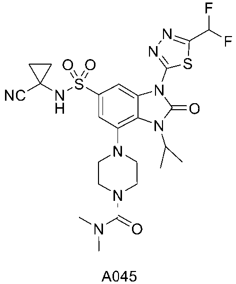 | **¹H NMR** (400 MHz, DMSO-*d₆*) δ 9.18 (s, 1H), 8.73 (d, *J* = 1.6 Hz, 1H), 7.80 - 7.50 (m, 2H), 5.92 - 5.82 (m, 1H), 3.65 - 3.61 (m, 2H), 3.13 - 3.00 (m, 4H), 2.97 - 2.86 (m, 2H), 2.81 (s, 6H), 1.56 (d, *J =* 6.8 Hz, 6H), 1.45 - 1.39 (m, 2H), 1.31 - 1.24 (m, 2H). |
| | | **MS (ESI) M/Z:** 610.3 [M+H]⁺. |
| A046 | 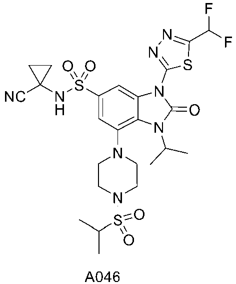 | **¹H NMR** (400 MHz, DMSO-*d₆*) δ 9.16 (s, 1H), 8.76 - 8.71 (m, 1H), 7.78 - 7.50 (m, 2H), 5.88 - 5.76 (m, 1H), 3.76 - 3.73 (m, 2H), 3.48 -3.39 (m, 1H), 3.27 - 3.12 (m, 4H), 2.95 - 2.85 (m, 2H), 1.56 (d, *J* = 6.8 Hz, 6H), 1.46 - 1.40 (m, 2H), 1.30 - 1.27 (m, 8H) |
| | | **MS (ESI) M/Z:** 645.1 [M+H]⁺. |
| A047 | 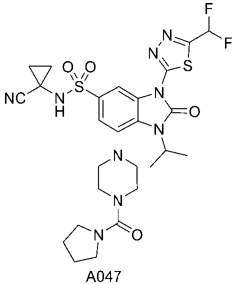 | **¹H NMR** (400 MHz, DMSO-*d₆*) δ 9.14 (s, 1H), 8.73 (d, *J* = 1.6 Hz, 1H), 7.80 - 7.49 (m, 2H), 5.93 - 5.82 (m, 1H), 3.77 - 3.73 (m, 2H), 3.35 - 3.33 (m, 3H), 3.31 - 3.30 (m, 1H), 3.12 - 2.98 (m, 4H), 2.95 - 2.84 (m, 2H), 1.81 - 1.74 (m, 4H), 1.57 (d, *J* = 6.8 Hz, 6H), 1.44 - 1.39 (m, 2H), 1.30 - 1.24 (m, 2H). |
| | | **MS (ESI) M/Z:** 636.1 [M+H]⁺. |
| A048 | 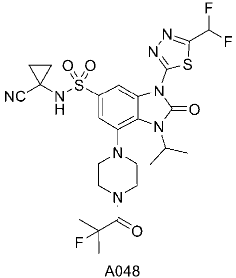 | **¹H NMR** (400 MHz, DMSO-*d₆*) δ 9.13 (s, 1H), 8.74 (d, *J* = 1.6 Hz, 1H), 7.79 - 7.47 (m, 2H), 5.94 - 5.82 (m, 1H), 4.48 - 4.44 (m, 2H), 3.56 - 3.39 (m, 1H), 3.20 - 3.17 (m, 2H), 3.09 - 2.96 (m, 1H), 2.93 - 2.78 (m, 2H), 1.63 (s, 3H), 1.60 - 1.55 (m, 9H), 1.46 - 1.39 (m, 2H), 1.30 - 1.25 (m, 2H). |
| | | **MS (ESI) M/Z:** 627.1 [M+H]⁺. |
| A049 | 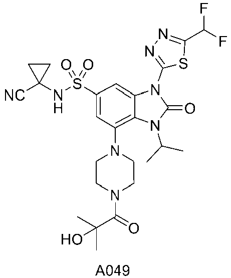 | **¹H NMR** (400 MHz, DMSO-*d₆*) δ 9.14 (s, 1H), 8.73 (d, *J* = 1.6 Hz, 1H), 7.77 - 7.51 (m, 2H), 5.93 - 5.86 (m, 1H), 5.51 - 3.61 (m, 2H), 5.20 - 4.35 (m, 2H), 3.23 - 3.07 (m, 2H), 3.01 (s, 6H), 1.58 (d, *J =* 6.8 Hz, 6H), 1.45 - 1.39 (m, 2H), 1.29 - 1.24 (m, 2H). |
| | | **MS (ESI) M/Z:** 625.2 [M+H]⁺. |
| A050 | 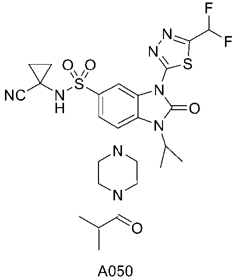 | **¹H NMR** (400 MHz, DMSO-*d₆*) δ 9.18 (s, 1H), 8.74 (d, *J* = 1.6 Hz, 1H), 7.80 - 7.48 (m, 2H), 5.97 - 5.80 (m, 1H), 4.55 - 4.50 (m, 1H), 4.14 - 4.08 (m, 1H), 3.44 - 3.34 (m, 1H), 3.18 - 3.13 (m, 2H), 3.00 - 2.93 (m, 1H), 2.92 - 2.82 (m, 2H), 2.79 - 2.69 (m, 1H), 1.57 (d, *J =* 6.8 Hz, 6H), 1.48 - 1.39 (m, 2H), 1.30 - 1.25 (m, 2H), 1.11 - 0.96 (m, 6H). |
| | | **MS (ESI) M/Z:** 609.2 [M+H]⁺. |
| A051 | 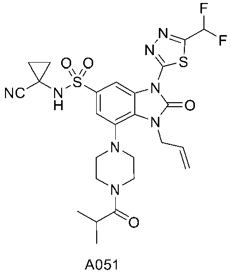 | **¹H NMR** (400 MHz, DMSO-*d₆*) δ 8.76 (d, *J =* 1.6 Hz, 1H), 8.56 (s, 1H), 7.78 - 7.52 (m, 2H), 6.11 - 6.04 (m, 1H), 5.15 - 5.02 (m, 2H), 4.95 - 4.92 (m, 2H), 4.49 - 4.45 (m, 1H), 4.08 - 4.04 (m, 1H), 3.06 - 3.03 (m, 2H), 2.96 - 2.83 (m, 5H), 1.42 - 1.40 (m, 2H), 1.28 - 1.26 (m, 2H), 1.07 - 1.02 (m, 6H). |
| | | **MS (ESI) M/Z:** 607.1 [M+H]⁺. |
| A052 | 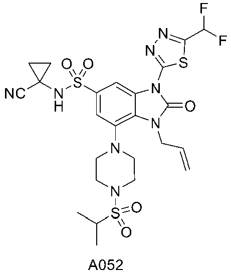 | **¹H NMR** (400 MHz, DMSO-*d₆*) δ 9.21 (s, 1H), 8.77 (s, 1H), 7.80 (s, 1H), 7.65 (t*, J =* 52.8 Hz, 1H), 6.11 - 6.03 (m, 1H), 5.14 - 5.12 (m, 1H), 5.05 - 5.00 (m, 1H), 4.93 - 4.91 (m, 2H), 3.71 - 3.68 (m, 2H), 3.45 - 3.41 (m, 1H), 3.25 - 3.15 (m, 2H), 3.08 - 3.05 (m, 2H), 2.94 - 2.85 (m, 2H), 1.44 - 1.41 (m, 2H), 1.31 - 1.27 (m, 8H). |
| | | **MS (ESI) M/Z:** 643.1 [M+H]⁺. |
| A053 | 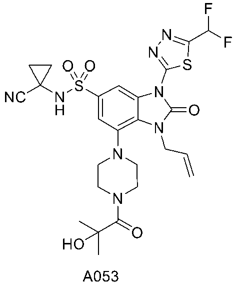 | **¹H NMR** (400 MHz, CDCl₃-*d*) δ 8.98 (s, 1H), 7.85 (s, 1H), 7.06 (t*, J =* 53.6 Hz, 1H), 6.08 - 5.93 (m, 2H), 5.27 - 5.25 (m, 1H), 5.15 - 5.11 (m, 1H), 5.06 - 5.05 (m, 2H), 4.63 - 4.60 (m, 2H), 3.87 - 3.77 (m, 1H), 3.19 - 3.15 (m, 1H), 3.14 - 3.10 (m, 2H), 3.04 - 2.98 (m, 2H), 1.69 - 1.65 (m, 2H), 1.57 (s, 6H), 1.46 - 1.43 (m, 2H). |
| | | **MS (ESI) M/Z:** 623.1 [M+H]⁺. |
| A054 | 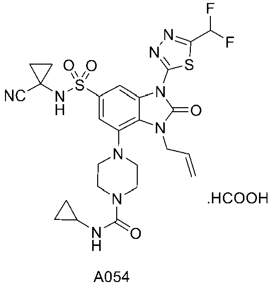 | **¹H NMR** (400 MHz, DMSO-*d₆*) δ 9.20 (s, 1H), 8.76 (d, *J* = 1.6 Hz, 1H), 7.78 - 7.51 (m, 2H), 6.70 (d, *J* = 2.4 Hz, 1H), 6.08 - 6.01 (m, 1H), 5.14 - 5.02 (m, 2H), 4.94 - 4.92 (m, 2H), 4.00 - 3.96 (m, 2H), 3.00 - 2.93 (m, 4H), 2.81 - 2.77 (m, 2H), 2.55 - 2.52 (m, 1H), 1.44 - 1.41 (m, 2H), 1.31 - 1.29 (m, 2H), 0.57 - 0.55 (m, 2H), 0.41 - 0.39 (m, 2H). |
| | | **MS (ESI) M/Z:** 620.1 [M+H]⁺. |
| A055 | 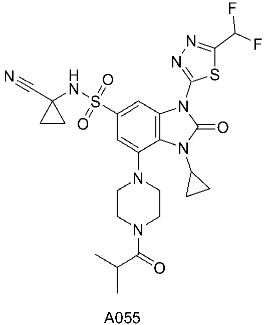 | **¹H NMR** (400 MHz, CDCl₃-*d*) δ 8.87 *(d, J* = 1.2 Hz, 1H), 7.73 *(d, J* = 1.2 Hz, 1H), 7.03 (t, *J* = 53.6 Hz, 1H), 6.02 (s, 1H), 4.68 - 4.64 (m, 1H), 3.98 - 3.96 (m, 1H), 3.85 - 3.84 (m, 1H), 3.48 - 3.47 (m, 1H), 3.32 - 3.27 (m, 1H), 3.20 - 2.85 (m, 4H), 2.87 - 2.82 (m, 1H), 1.64 - 1.62 (m, 2H), 1.42 - 1.41 (m, 2H), 1.24 - 1.20 (m, 4H), 1.18 - 1.17 (m, 6H). |
| | | **MS (ESI) M/Z:** 607.2 [M+H]⁺. |
| A056 | 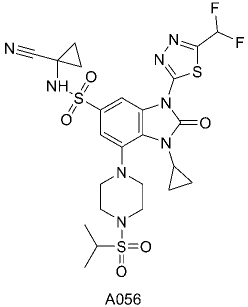 | **¹H NMR** (400 MHz, DMSO-*d₆*) δ 9.16 (s, 1H), 8.67 (d, *J* = 1.6 Hz, 1H), 7.77 - 7.50 (m, 2H), 3.67 - 3.52 (m, 2H), 3.45 - 3.34 (m, 4H), 3.28 - 3.19 (m, 2H), 3.00 - 2.78 (m, 2H), 1.44 - 1.41 (m, 2H), 1.28 - 1.26 (m, 8H), 1.17 - 1.15 (m, 2H), 1.03 - 1.01 (m, 2H). |
| | | **MS (ESI) M/Z:** 643.2 [M+H]⁺. |
| B012 | 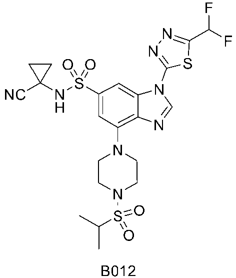 | **¹H NMR** (400 MHz, DMSO-*d₆*) δ 9.18 (s, 1H), 9.10 (s, 1H), 8.32 (d, *J =* 1.6 Hz, 1H), 7.68 (t, *J =* 53.2 Hz, 1H), 7.24 (d, *J =* 1.6 Hz, 1H), 3.73 - 3.64 (m, 4H), 3.55 - 3.47 (m, 4H), 3.46 - 3.37 (m, 1H), 1.47 - 1.39 (m, 2H), 1.31 - 1.28 (m, 2H), 1.27 *(d, J* = 6.8 Hz, 6H). |
| | | **MS (ESI) M/Z:** 587.3 [M+H]⁺. |
| B013 | 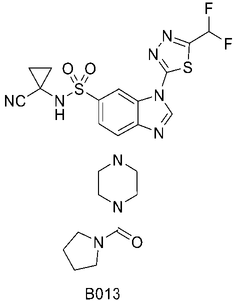 | **¹H NMR** (400 MHz, DMSO-*d₆*) δ 9.17 (s, 1H), 9.08 (s, 1H), 8.30 (d, *J* = 1.2 Hz, 1H), 7.69 (t, *J* = 53.2 Hz, 1H), 7.22 (d, *J* = 0.8 Hz, 1H), 3.68 - 3.58 (m, 4H), 3.42 - 3.41 (m, 4H), 3.34 - 3.33 (m, 2H), 3.31 - 3.29 (m, 2H), 1.81 - 1.73 (m, 4H), 1.46 - 1.38 (m, 2H), 1.32 - 1.24 (m, 2H). |
| | | **MS (ESI) M/Z:** 578.2 [M+H]⁺. |
| B014 | 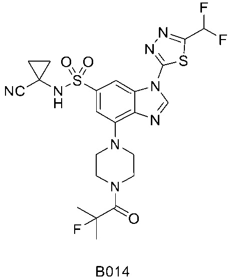 | **¹H NMR** (400 MHz, DMSO-*d₆*) δ 9.17 (s, 1H), 9.10 (s, 1H), 8.31 (d, *J* = 1.2 Hz, 1H), 7.69 (t, *J* = 53.2 Hz, 1H), 7.23 (d, *J* = 1.2 Hz, 1H), 4.03 - 3.72 (m, 4H), 3.67 - 3.65 (m, 4H), 1.62 (s, 3H), 1.57 (s, 3H), 1.45 - 1.38 (m, 2H), 1.31 - 1.24 (m, 2H). |
| | | **MS (ESI) M/Z:** 569.2 [M+H]⁺. |
| B015 | 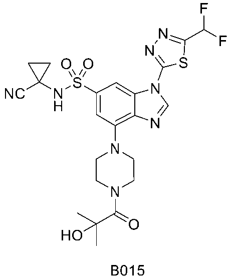 | **¹H NMR** (400 MHz, DMSO-*d₆*) δ 9.16 (s, 1H), 9.09 (s, 1H), 8.30 (d, *J* = 1.6 Hz, 1H), 7.68 (t, *J* = 53.2 Hz, 1H), 7.23 (d, *J =* 1.2 Hz, 1H), 5.51 (s, 1H), 4.31 - 3.71 (m, 4H), 3.64 - 3.62 (m, 4H), 1.45 - 1.39 (m, 2H), 1.36 (s, 6H), 1.31 - 1.23 (m, 2H). |
| | | **MS (ESI) M/Z:** 567.2 [M+H]⁺. |
| B016 | 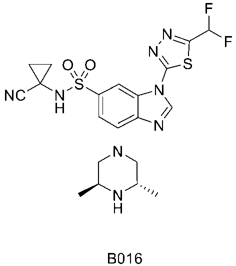 | **¹H NMR** (400 MHz, DMSO-*d₆*) δ 9.04 (s, 1H), 8.21 (d, *J* = 1.2 Hz, 1H), 7.68 (t, *J* = 52.8 Hz, 1H), 7.16 (d, *J* = 1.6 Hz, 1H), 3.56 - 3.53 (m, 2H), 3.38 - 3.34 (m, 2H), 3.33 - 3.29 (m, 2H), 1.40 - 1.38 (m, 2H), 1.28 - 1.24 (m, 2H), 1.16 (d, *J* = 6.4 Hz, 6H). |
| | | **MS (ESI) M/Z:** 509.1 [M+H]⁺. |
| B017 | 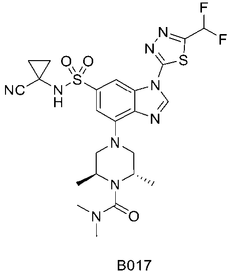 | **¹H NMR** (400 MHz, DMSO-*d₆*) δ 9.07 (s, 1H), 8.25 (s, 1H), 7.68 (t, *J =* 53.2 Hz, 1H), 7.21 (s, 1H), 3.65 - 3.63 (m, 2H), 3.60 - 3.52 (m, 4H), 2.90 (s, 6H), 1.42 - 1.40 (m, 2H), 1.29 - 1.26 (m, 2H), 1.23 (d, *J =* 6.0 Hz, 6H). |
| | | **MS (ESI) M/Z:** 580.2 [M+H]⁺. |
| B018 | 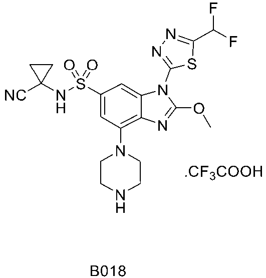 | **¹H NMR** (400 MHz, DMSO-*d₆*) δ 9.16 (s, 1H), 8.80 (s, 1H), 8.51 (d, *J* = 1.6 Hz, 1H), 7.66 (t, *J* = 53.2 Hz, 1H), 7.29 *(d, J* = 1.6 Hz, 1H), 4.37 (s, 3H), 3.72 - 3.70 (m, 4H), 3.36 - 3.35 (m, 4H), 1.44 - 1.38 (m, 2H), 1.32 - 1.27 (m, 2H). |
| | | **MS (ESI) M/Z:** 511.1 [M+H]⁺. |
| B019 | 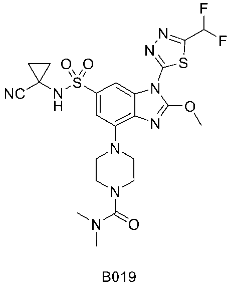 | **¹H NMR** (400 MHz, DMSO-*d₆*) δ 8.97 (s, 1H), 8.45 (d, *J* = 1.6 Hz, 1H), 7.65 (t, *J* = 53.2 Hz, 1H), 7.25 (d, *J* = 1.2 Hz, 1H), 4.36 (s, 3H), 3.53 - 3.49 (m, 4H), 3.35 - 3.32 (m, 4H), 2.80 (s, 6H), 1.42 - 1.38 (m, 2H), 1.27 - 1.22 (m, 2H). |
| | | **MS (ESI) M/Z:** 582.1 [M+H]⁺. |
| B020 | 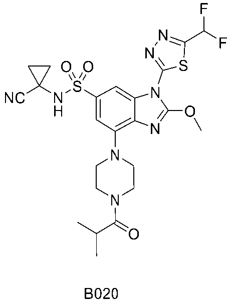 | **¹H NMR** (400 MHz, DMSO-*d₆*) δ 9.09 (s, 1H), 8.46 (d, *J* = 1.2 Hz, 1H), 7.66 (t, *J* = 53.6 Hz, 1H), 7.25 (d, *J =* 1.6 Hz, 1H), 4.37 (s, 3H), 3.77 - 3.68 (m, 4H), 3.56 - 3.51 (m, 2H), 3.49 - 3.43 (m, 2H), 2.98 - 2.94 (m, 1H), 1.44 - 1.35 (m, 2H), 1.29 - 1.23 (m, 2H), 1.04 (d, *J =* 6.4 Hz, 6H). |
| | | **MS (ESI) M/Z:** 581.1 [M+H]⁺. |
| B021 | 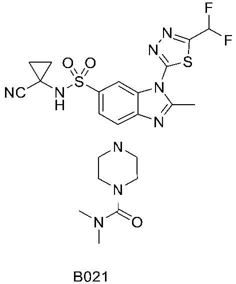 | **¹H NMR** (400 MHz, DMSO-*d₆*) δ 9.05 (s, 1H), 7.88 (d, *J* = 1.2 Hz, 1H), 7.69 (t, *J* = 52.8 Hz, 1H), 7.18 (d, *J* = 1.2 Hz, 1H), 3.65 - 3.55 (m, 4H), 3.42 - 3.32 (m, 4H), 2.80 (s, 6H), 2.78 (s, 3H), 1.42 - 1.37 (m, 2H), 1.28 - 1.22 (m, 2H). |
| | | **MS (ESI) M/Z:** 566.1 [M+H]⁺. |
| B022 | 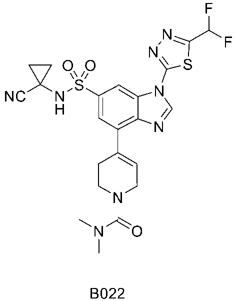 | **¹H NMR** (400 MHz, DMSO-*d₆*) δ 9.23 (s, 1H), 8.98 (s, 1H), 8.71 (s, 1H), 7.84 (s, 1H), 7.69 (t, *J* = 53.2 Hz, 1H), 6.96 (s, 1H), 3.97 (d, *J* = 2.4 Hz, 2H), 3.44 (t, *J* = 5.6 Hz, 2H), 2.81 - 2.79 (m, 8H), 1.44 - 1.40 (m, 2H), 1.30 - 1.26 (m, 2H). |
| | | **MS (ESI) M/Z:** 549.0 [M+H]⁺. |
| B023 | 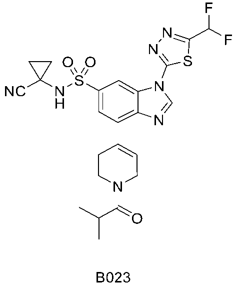 | **¹H NMR** (400 MHz, DMSO-*d₆*) δ 9.26 (s, 1H), 9.24 (s, 1H), 8.72 (s, 1H), 7.84 (s, 1H), 7.70 (t, *J* = 52.8 Hz, 1H), 6.95 (s, 1H), 4.37 - 4.23 (m, 2H), 3.80 - 3.77 (m, 2H), 3.03 - 2.91 (m, 1H), 2.84 - 2.72 (m, 2H), 1.43 - 1.41 (m, 2H), 1.30 - 1.27 (m, 2H), 1.07 - 1.03 (m, 6H). |
| | | **MS (ESI) M/Z:** 548.1 [M+H]⁺. |
| B024 | 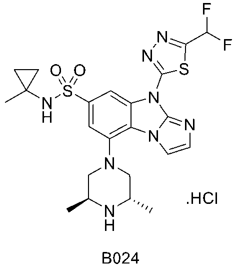 | **¹H NMR** (400 MHz, CDCl₃-*d*) δ 10.25 (s, 2H), 9.12 (s, 1H), 7.82 (s, 1H), 7.54 - 7.53 (m, 1H), 7.42 - 7.41 (m, 1H), 7.09 (t*, J =* 53.6 Hz, 1H), 5.34 (s, 1H), 4.12 - 4.02 (m, 2H), 3.68 - 3.66 (m, 2H), 3.23 - 3.18 (m, 2H), 1.78 - 1.75 (m, 6H), 1.29 (s, 3H), 0.85 - 0.83 (m, 2H), 0.57 - 0.54 (m, 2H). |
| | | **MS (ESI) M/Z:** 537.2 [M+H]⁺. |
| H001 | 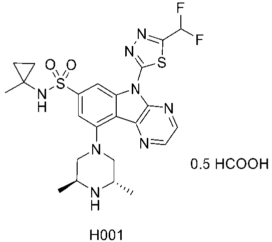 | **¹H NMR** (400 MHz, CD₃CN-*d₃*) δ 9.17 (s, 1H), 8.84 (d, *J* = 2.4 Hz, 1H), 8.58 (d, *J* = 2.4 Hz, 1H), 8.23 (s, 0.5H, HCOOH), 7.48 (s, 1H), 7.28 (t, *J* = 53.2 Hz, 1H), 6.37 (s, 1H), 3.75 - 3.74 (m, 2H), 3.65 (d, *J =* 10.4 Hz, 2H), 3.15 - 3.12 (m, 2H), 1.34 (d, *J* = 6.0 Hz, 6H), 1.15 (s, 3H), 0.78 - 0.77 (m, 2H), 0.48 - 0.47 (m, 2H). |
| | | **MS (ESI) M/Z:** 549.4 [M+H]⁺. |
| 057L | 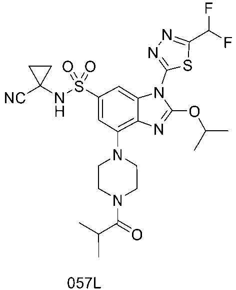 | **¹H NMR** (400 MHz, DMSO-*d₆*) δ 9.03 (s, 1H), 8.48 (d, *J* = 1.6 Hz, 1H), 7.65 (t, *J* = 53.2 Hz, 1H), 7.24 (d, *J =* 1.2 Hz, 1H), 5.51 - 5.45 (m, 1H), 3.74 - 3.70 (m, 4H), 3.53 - 3.44 (m, 4H), 2.96 - 2.90 (m, 1H), 1.58 (d, *J =* 6.4 Hz, 6H), 1.41 - 1.38 (m, 2H), 1.27 - 1.23 (m, 2H), 1.04 (d, *J =* 6.8 Hz, 6H). |
| | | **MS (ESI) M/Z:** 609.1 [M+H]⁺. |
| 057M | 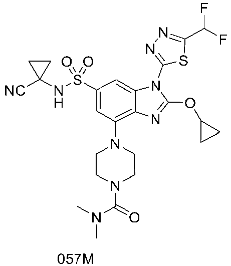 | **¹H NMR** (400 MHz, DMSO-*d₆*) δ 9.06 (s, 1H), 8.46 (d, *J* = 1.6 Hz, 1H), 7.64 (t, *J* = 52.8 Hz, 1H), 7.26 (d, *J* = 1.2 Hz, 1H), 4.80 - 4.75 (m, 1H), 3.54 - 3.52 (m, 4H), 3.36 - 3.33 (m, 4H), 2.80 (s, 6H), 1.42 - 1.38 (m, 2H), 1.27 - 1.24 (m, 2H), 1.13 - 1.08 (m, 2H), 0.97 - 0.94 (m, 2H). |
| | | **MS (ESI) M/Z:** 608.4 [M+H]⁺. |
| 065I | 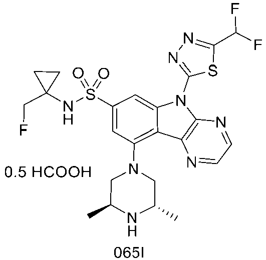 | **¹H NMR** (400 MHz, DMSO-*d₆*) δ 9.08 (s, 1H), 8.92 (d, *J* = 2.8 Hz, 1H), 8.81 (s, 1H), 8.69 (d, *J =* 2.4 Hz, 1H), 8.27 (s, 0.5H), 7.70 (t, *J =* 53.2 Hz, 1H), 7.47 (s, 1H), 4.21 (d, *J* = 50.0 Hz, 2H), 3.64 - 3.62 (m, 2H), 3.54 - 3.52 (m, 2H), 3.10 - 3.06 (m, 2H), 1.27 (d, *J =* 6.0 Hz, 6H), 0.77 - 0.75 (m, 4H). |
| | | **MS (ESI) M/Z:** 567.4 [M+H]⁺. |
| 067L | 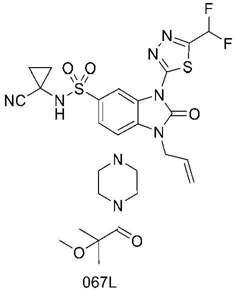 | **¹H NMR** (400 MHz, DMSO-*d₆*) δ 9.16 (s, 1H), 8.77 (s, 1H), 7.76 (s, 1H), 7.65 (t, *J =* 53.2 Hz, 1H), 6.12 - 6.05 (m, 1H), 5.16 - 5.13 (m, 1H), 5.07 - 5.03 (m, 1H), 4.96 - 4.94 (m, 2H), 4.83 - 4.79 (m, 1H), 4.51 - 4.49 (m, 1H), 3.20 (s, 3H), 3.09 - 3.06 (m, 2H), 3.07 - 2.70 (m, 4H), 1.44 - 1.41 (m, 2H), 1.37 (s, 6H), 1.30 - 1.27 (m, 2H). |
| | | **MS (ESI) M/Z:** 637.2 [M+H]⁺. |
| 067LD | 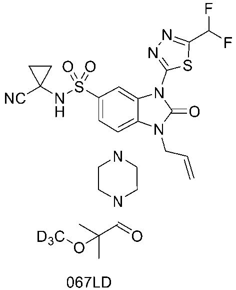 | **¹H NMR** (400 MHz, DMSO-*d₆*) δ 8.77 (d, *J* = 1.6 Hz, 1H), 7.78 - 7.51 (m, 2H), 6.12 - 6.05 (m, 1H), 5.15 - 5.03 (m, 2H), 4.96 - 4.95 (m, 2H), 4.87 - 4.70 (m, 1H), 4.58 - 4.40 (m, 1H), 3.36 - 3.33 (m, 1H), 3.11 - 3.06 (m, 2H), 2.92 - 2.84 (m, 3H), 1.45 - 1.41 (m, 2H), 1.37 (s, 6H), 1.30 - 1.27 (m, 2H). |
| | | **MS (ESI) M/Z:** 640.4 [M+H]⁺. |
| 067N | 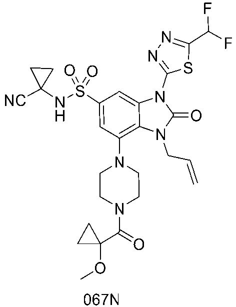 | **¹H NMR** (400 MHz, CDCl₃) δ 8.90 (s, 1H), 7.85 (d, *J =* 2.0 Hz, 1H), 7.05 (t, *J* = 53.6 Hz, 1H), 6.41 (s, 1H), 6.05 - 5.97 (m, 1H), 5.27 - 5.24 (m, 1H), 5.16 - 5.12 (m, 1H), 5.07 - 5.06 (m, 2H), 4.67 - 4.64 (m, 2H), 3.39 (s, 3H), 3.15 - 2.97 (m, 6H), 1.66 - 1.63 (m, 2H), 1.43 - 1.42 (m, 2H), 1.18 - 1.16 (m, 2H), 1.01 - 0.99 (m, 2H). |
| | | **MS (ESI) M/Z:** 635.2 [M+H]⁺. |
| 067R | 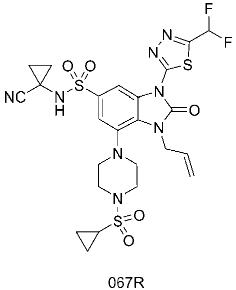 | **¹H NMR** (400 MHz, DMSO-*d₆*) δ 9.22 (s, 1H), 8.78 (d, *J* = 2.0 Hz, 1H), 7.80 (d, *J* = 1.6 Hz, 1H), 7.65 (t, *J* = 53.2 Hz, 1H), 6.10 - 6.03 (m, 1H), 5.16 - 5.13 (m, 1H), 5.08 - 5.03 (m, 1H), 4.92 - 4.91 (m, 2H), 3.67 - 3.65 (m, 2H), 3.16 - 3.10 (m, 4H), 3.01 - 2.98 (m, 2H), 2.76 - 2.72 (m, 1H), 1.46 - 1.43 (m, 2H), 1.32 - 1.30 (m, 2H), 1.06 - 1.03 (m, 2H), 0.99 - 0.97 (m, 2H). |
| | | **MS (ESI) M/Z:** 641.2 [M+H]⁺. |
| 068A | 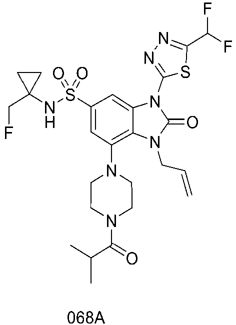 | **¹H NMR** (400 MHz, DMSO-*d₆*) δ 8.71 (d, *J* = 1.2 Hz, 1H), 8.51 (s, 1H), 7.78 - 7.51 (m, 2H), 6.13 - 6.05 (m, 1H), 5.16 - 5.07 (m, 2H), 4.94 - 4.93 (m, 2H), 4.51 - 4.46 (m, 1H), 4.25 (s, 1H), 4.13 (s, 1H), 4.07 - 4.04 (m, 1H), 3.34 - 3.33 (m, 1H), 3.04 - 3.01 (m, 2H), 2.96 - 2.92 (m, 1H), 2.85 - 2.79 (m, 2H), 2.69 - 2.67 (m, 1H), 1.07 - 1.01 (m, 6H), 0.72 - 0.70 (m, 4H). |
| | | **MS (ESI) M/Z:** 614.2 [M+H]⁺. |
| 068B | 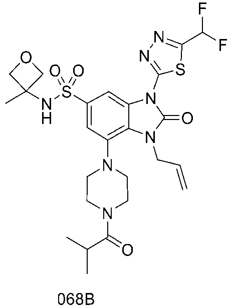 | **¹H NMR** (400 MHz, DMSO-*d₆*) δ 8.74 (d, *J =* 1.6 Hz, 1H), 8.42 (s, 1H), 7.72 *(d, J* = 1.6 Hz, 1H), 7.64 (t, *J* = 53.2 Hz, 1H), 6.12 - 6.05 (m, 1H), 5.17 - 5.08 (m, 2H), 4.94 - 4.93 (m, 2H), 4.54 - 4.46 (m, 3H), 4.10 - 4.04 (m, 3H), 3.36 - 3.33 (m, 1H), 3.04 - 2.96 (m, 2H), 2.94 - 2.91 (m, 1H), 2.87 |
| | | - 2.82 (m, 2H), 2.73 - 2.67 (m, 1H), 1.44 (s, 3H), 1.04 (d, *J* = 5.6 Hz, 6H). |
| | | **MS (ESI) M/Z:** 612.2 [M+H]⁺. |
| 069B | 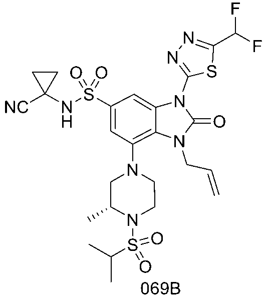 | **¹H NMR** (400 MHz, DMSO-*d₆*) δ 8.95 (d, *J* = 1.6 Hz, 1H), 7.91 (d, *J =* 1.6 Hz, 1H), 7.64 (t, *J =* 53.2 Hz, 1H), 6.08 - 5.94 (m, 1H), 5.13 (d, *J =* 10.4 Hz, 1H), 5.05 (d, *J =* 17.6 Hz, 1H), 5.01 - 4.87 (m, 2H), 4.13 - 4.07 (m, 1H), 2.98 - 2.83 (m, 5H), 2.81 - 2.72 (m, 1H), 2.42 - 2.37 (m, 1H), 2.05 - 1.93 (m, 1H), 1.91 - 1.83 (m, 2H), 1.66 - 1.54 (m, 1H), 1.51 - 1.30 (m, 6H), 0.98 *(d, J* = 6.0 Hz, 3H). |
| | | **MS (ESI) M/Z:** 657.1 [M+H]⁺. |
| 076A | 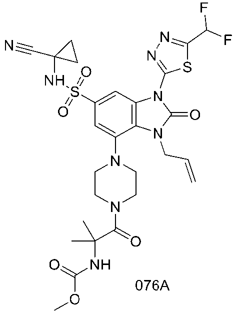 | **¹H NMR** (400 MHz, DMSO-*d₆*) δ 9.24 (s, 1H), 8.76 (s, 1H), 7.77 - 7.51 (m, 3H), 6.00 - 5.99 (m, 1H), 5.13 (d, *J =* 10.8 Hz, 1H), 5.04 (d, *J* = 17.6 Hz, 1H), 4.95 - 4.94 (m, 2H), 4.48 - 4.45 (m, 2H), 3.57 (s, 3H), 3.15 - 2.98 (m, 4H), 2.74 - 2.68 (m, 2H), 1.40 - 1.37 (m, 8H), 1.29 - 1.25 (m, 2H). |
| | | **MS (ESI) M/Z:** 680.1 [M+H]⁺. |
| 076B | 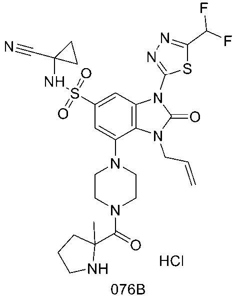 | **¹H NMR** (400 MHz, DMSO-*d₆*) δ 9.44 - 9.41 (m, 1H), 9.28 (s, 1H), 8.92 - 8.88 (m, 1H), 8.78 (d, *J =* 1.6 Hz, 1H), 7.84 (d, *J* = 1.6 Hz, 1H), 7.65 (t, *J* = 53.2 Hz, 1H), 6.12 - 6.05 (m, 1H), 5.14 (d, *J =* 10.8 Hz, 1H), 5.06 (d, *J =* 17.2 Hz, 1H), 4.96 - 4.95 (m, 2H), 4.52 - 4.38 (m, 1H), 4.03 - 3.95 (m, 1H), 3.50 - 3.43 (m, 1H), 3.24 - 2.87 (m, 7H), 2.39 - 2.19 (m, 2H), 2.13 - 1.88 (m, 2H), 1.65 (s, 3H), 1.44 - 1.40 (m, 2H), 1.31 - 1.28 (m, 2H). |
| | | **MS (ESI) M/Z:** 648.3 [M+H]⁺. |
| 076D | 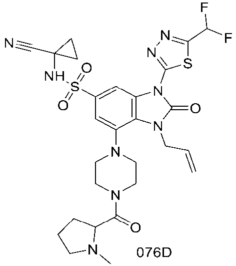 | **¹H NMR** (400 MHz, DMSO-*d₆*) δ 9.19 (s, 1H), 8.76 (s, 1H), 7.78 - 7.51 (m, 2H), 6.10 - 6.03 (m, 1H), 5.13 (d, *J =* 10.8 Hz, 1H), 5.04 (d, *J* = 16.8 Hz, 1H), 4.95 - 4.94 (m, 2H), 4.47 - 4.31 (m, 2H), 3.26 - 3.11 (m, 2H), 3.05 - 2.96 (m, 3H), 2.90 - 2.72 (m, 3H), 2.25 (s, 3H), 2.21 - 2.06 (m, 2H), 1.77 - 1.71 (m, 3H), 1.42 - 1.40 (m, 2H), 1.26 - 1.23 (m, 2H). |
| | | **MS (ESI) M/Z:** 648.2 [M+H]⁺. |
| 076F1 | 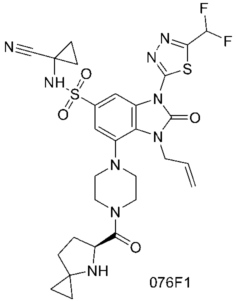 | **¹H NMR** (400 MHz, DMSO-*d₆*) δ 8.76 (s, 1H), 7.78 (s, 1H), 7.65 (t, *J* = 53.2 Hz, 1H), 6.12 - 6.03 (m, 1H), 5.13 (d, *J =* 10.0 Hz, 1H), 5.04 (d, *J =* 17.6 Hz, 1H), 4.96 - 4.95 (m, 2H), 4.46 - 4.43 (m, 1H), 4.13 - 3.96 (m, 2H), 3.05 - 2.99 (m, 2H), 2.95 - 2.90 (m, 2H), 2.82 - 2.76 (m, 1H), 2.46 - 2.36 (m, 1H), 2.28 - 2.17 (m, 1H), 1.94 - 1.70 (m, 2H), 1.57 - 1.54 (m, 1H), 1.43 - 1.40 (m, 2H), 1.29 - 1.26 (m, 2H), 0.73 - 0.68 (m, 1H), 0.59 - 0.54 (m, 1H), 0.49 - 0.44 (m, 1H), 0.37 - 0.32 (m, 1H). |
| | | **MS (ESI) M/Z:** 660.2 [M+H]⁺. |
| 076H1 | 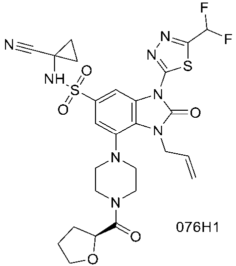 | **¹H NMR** (400 MHz, DMSO-*d₆*) δ 8.76 (d, *J =* 1.6 Hz, 1H), 8.40 (s, 1H), 7.78 - 7.51 (m, 2H), 6.12 - 6.03 (m, 1H), 5.13 (d, *J =* 10.4 Hz, 1H), 5.04 (d, *J =* 17.6 Hz, 1H), 4.96 - 4.94 (m, 2H), 4.75 - 4.72 (m, 1H), 4.43 - 4.39 (m, 1H), 4.12 - 4.09 (m, 1H), 3.84 - 3.72 (m, 2H), 3.31 - 3.26 (m, 1H), 3.04 - 3.02 (m, 2H), 2.91 - 2.82 (m, 2H), 2.80 - 2.73 (m, 1H), 2.12 - 2.01 (m, 2H), 1.87 - 1.82 (m, 2H), 1.41 - 1.38 (m, 2H), 1.27 - 1.25 (m, 2H). |
| | | **MS (ESI) M/Z:** 635.2 [M+H]⁺. |
| 076J | 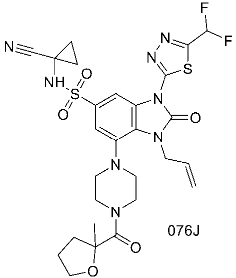 | **¹H NMR** (400 MHz, DMSO-*d₆*) δ 9.18 (s, 1H), 8.77 (d, *J* = 1.6 Hz, 1H), 7.78 - 7.51 (m, 2H), 6.12 - 6.03 (m, 1H), 5.13 (d, *J =* 10.4 Hz, 1H), 5.05 (d, *J =* 17.2 Hz, 1H), 4.96 - 4.95 (m, 2H), 4.81 - 4.60 (m, 1H), 4.56 - 4.35 (m, 1H), 3.92 - 3.84 (m, 1H), 3.82 - 3.72 (m, 1H), 3.31 - 3.18 (m, 1H), 3.17 - 3.04 (m, 2H), 2.94 - 2.76 (m, 3H), 2.70 - 2.66 (m, 1H), 1.92 - 1.82 (m, 1H), 1.81 - 1.72 (m, 1H), 1.62 - 1.57 (m, 1H), 1.42 - 1.40 (m, 5H), 1.30 - 1.26 (m, 2H). |
| | | **MS (ESI) M/Z:** 649.2 [M+H]⁺. |
| 077E | 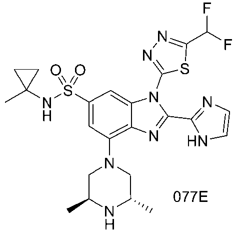 | **¹H NMR** (400 MHz, DMSO-*d₆*) δ 8.03 (s, 1H), 7.67 (t, *J* = 53.2 Hz, 1H), 7.49 - 7.26 (m, 2H), 7.09 - 7.05 (m, 2H), 3.64 - 3.61 (m, 2H), 3.46 - 3.42 (m, 2H), 3.38 - 3.34 (m, 2H), 1.20 *(d, J* = 6.4 Hz, 6H), 1.04 (s, 3H), 0.62 - 0.59 (m, 2H), 0.37 - 0.34 (m, 2H). |
| | | **MS (ESI) M/Z:** 564.3 [M+H]⁺. |
| 074J | 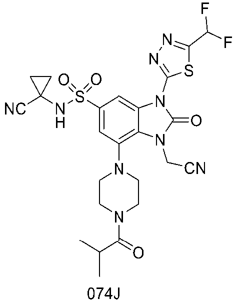 | **¹H NMR** (400 MHz, DMSO-*d₆*) δ 8.73 (d, *J* = 1.6 Hz, 1H), 7.85 (d, *J =* 1.7 Hz, 1H), 7.65 (t, *J* = 53.2 Hz, 1H), 5.40 (s, 2H), 4.56 - 4.42 (m, 1H), 4.15 - 4.10 (m, 1H), 3.55 - 3.40 (m, 1H), 3.14 - 3.07 (m, 2H), 3.01 - 2.91 (m, 3H), 2.86 - 2.78 (m, 1H), 1.39 (q, *J* = 5.3, 4.6 Hz, 2H), 1.30 - 1.25 (m, 2H), 1.06 (s, 6H). |
| | | **MS (ESI) M/Z:** 606.2 [M+H]⁺. |
| 074K | 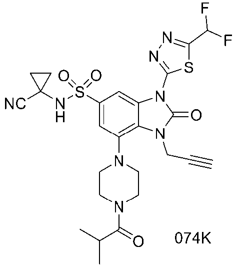 | **¹H NMR** (400 MHz, DMSO-*d₆*) δ 9.25 (s, 1H), 8.75 (d, *J* = 1.7 Hz, 1H), 7.80 (d, *J* = 1.7 Hz, 1H), 7.64 (t, *J* = 53.2 Hz, 1H), 5.17 (d, *J* = 6.7 Hz, 2H), 4.56 - 4.39 (m, 1H), 4.16 - 4.02 (m, 1H), 3.50 - 3.38 (m, 2H), 3.20 - 3.10 (m, 2H), 3.02 - 2.90 (m, 3H), 2.84 - 2.74 (m, 1H), 1.47 - 1.39 (m, 2H), 1.31 - 1.26 (m, 2H), 1.05 (s, 6H). |
| | | **MS (ESI) M/Z:** 605.2 [M+H]⁺. |
| 077F | 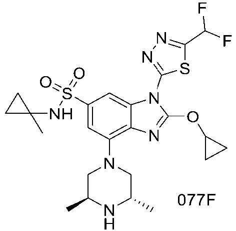 | **¹H NMR** (400 MHz, DMSO-*d₆*) δ 8.42 (d, *J* = 1.5 Hz, 1H), 8.08 (s, 1H), 7.64 (t, *J* = 53.0 Hz, 1H), 7.24 (d, *J* = 1.7 Hz, 1H), 4.72 (tt, *J* = 6.1, 2.8 Hz, 1H), 3.78 - 3.70 (m, 2H), 3.66 - 3.58 (m, 2H), 3.55 - 3.47 (m, 2H), 1.42 (d, *J* = 6.4, 6H), 1.12 - 1.07 (m, 2H), 1.05 (s, 3H), 0.96 - 0.92 (m, 2H), 0.68 - 0.58 (m, 2H), 0.42 - 0.32 (m, 2H). |
| | | **MS (ESI) M/Z:** 554.2 [M+H]⁺. |
| 077S | 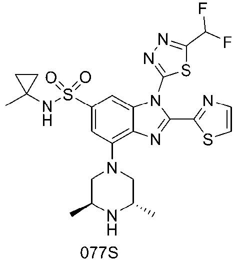 | **¹H NMR** (400 MHz, DMSO-*d₆*) δ 8.10 (s, 1H), 8.06 (d, *J* = 3.2 Hz, 1H), 7.94 (d, *J =* 3.2 Hz, 1H), 7.70 (t, *J =* 53.1 Hz, 1H), 7.32 (d, *J =* 1.4 Hz, 1H), 7.19 (d,J = 1.5 Hz, 1H), 3.88 - 3.62 (m, 6H), 1.24 (d, *J =* 6.1 Hz, 6H), 1.04 (s, 3H), 0.68 - 0.58 (m, 2H), 0.42 - 0.33 (m, 2H). |
| | | **MS (ESI) M/Z:** 581.2 [M+H]⁺. |
| 077T | 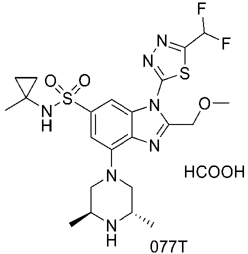 | **¹H NMR** (400 MHz, DMSO-*d₆*+D₂O) δ 8.39 (s, 1H), 7.88 (d, *J* = 1.4 Hz, 1H), 7.61 (t*, J =* 53.1 Hz, 1H), 7.19 (d, *J =* 1.6 Hz, 1H), 4.86 (s, 2H), 3.77 - 3.51 (m, 6H), 3.35 (s, 3H), 1.36 (d, *J* = 6.1 Hz, 6H), 1.05 (s, 3H), 0.68 - 0.58 (m, 2H), 0.51 - 0.32 (m, 2H). |
| | | **MS (ESI) M/Z:** 542.2 [M+H]⁺. |
| 077U | 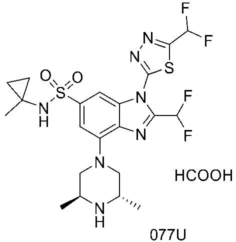 | **¹H NMR** (400 MHz, DMSO-*d₆*+D₂O) δ 8.38 (s, 1H), 7.69 - 7.25 (m, 3H), 7.25 (s, 1H), 7.40 (d, *J* = 1.4 Hz, 1H), 7.25 (d, *J* = 1.5 Hz, 1H), 3.90 - 3.65 (m, 6H), 1.42 (d, *J* = 6.2 Hz, 6H), 1.06 (s, 3H), 0.68 - 0.59 (m, 2H), 0.50 - 0.39 (m, 2H). |
| | | **MS (ESI) M/Z:** 548.2 [M+H]⁺. |
| 077V | 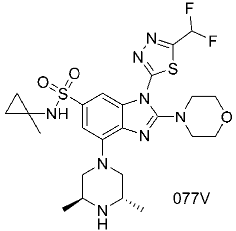 | **¹H NMR** (400 MHz, DMSO-*d₆*) δ 8.43 (s, 1H), 8.25 (d, *J* = 1.5 Hz, 1H), 7.59 (t, *J* = 53.2 Hz, 1H), 7.18 (d, *J =* 1.6 Hz, 1H), 3.90 - 3.80 (m, 4H), 3.57 - 3.40 (m, 6H), 3.33 - 3.21 (m, 4H), 1.33 (d, *J* = 5.4 Hz, 6H), 1.05 (s, 3H), 0.69 - 0.57 (m, 2H), 0.42 - 0.33 (m, 2H). |
| | | **MS (ESI) M/Z:** 583.2 [M+H]⁺. |
| 077W | 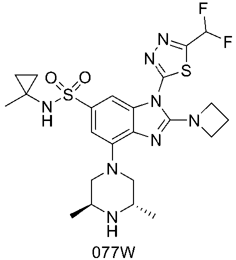 | **¹H NMR** (400 MHz, DMSO-*d₆*) δ 8.41 (s, 1H), 7.78 (d, *J* = 1.5 Hz, 1H), 7.59 (t, *J* = 53.2 Hz, 1H), 7.13 (d, *J* = 1.6 Hz, 1H), 4.12 *(t, J =* 7.7 Hz, 4H), 3.60 - 3.53 (m, 2H), 3.50 - 3.43 (m, 4H), 2.43 - 2.34 (m, 2H), 1.34 (d, *J* = 6.5 Hz, 6H), 1.03 (s, 3H), 0.66 - 0.56 (m, 2H), 0.42 - 0.32 (m, 2H). |
| | | **MS (ESI) M/Z:** 553.2 [M+H]⁺. |
| 008B | 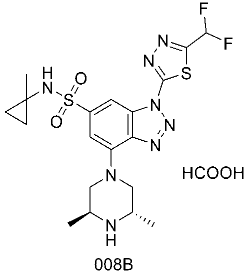 | **¹H NMR** (400 MHz, DMSO-*d₆*) δ 8.42 - 8.31 (m, 1H), 8.07 (d, *J* = 6.0 Hz, 1H), 7.84 - 7.51 (m, 1H), 7.14 (d, *J* = 5.2 Hz, 1H), 3.88 - 3.79 (m, 2H), 3.66 - 3.56 (m, 2H), 2.61 - 2.54 (m, 2H), 1.18 - 1.02 (m, 9H), 0.72 - 0.61 (m, 2H), 0.45 - 0.35 (m, 2H). |
| | | **¹⁹FNMR** (376 MHz, DMSO-*d₆*) δ -110.28. |
| | | **MS (ESI) M/Z:** 499.1 [M+H]⁺. |
| 057M0 | 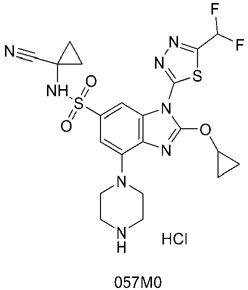 | **¹H NMR** (400 MHz, MeOD-*d₄*) δ 8.74 - 8.73 (m, 1H), 7.43 - 7.16 (m, 2H), 4.95 - 4.85 (m, 1H), 3.86 - 3.84 (m, 4H), 3.51 - 3.48 (m, 4H), 1.51 - 1.47 (m, 2H), 1.41 - 1.37 (m, 2H), 1.16 - 1.11 (m, 2H), 1.03 - 1.00 (m, 2H). |
| | | **MS (ESI) M/Z:** 537.4 [M+H]⁺. |
| 057N | 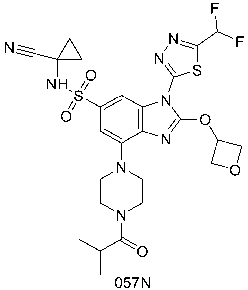 | **¹H NMR** (400 MHz, DMSO-*d₆*) δ 9.08 (s, 1H), 8.46 (d, *J* = 1.2 Hz, 1H), 7.67 (t, *J* = 53.2 Hz, 1H), 7.23 (d, *J =* 1.2 Hz, 1H), 5.99 - 5.93 (m, 1H), 5.04 - 5.00 (m, 2H), 4.91 - 4.87 (m, 2H), 3.73 - 3.68 (m, 4H), 3.52 - 3.38 (m, 4H), 2.98 - 2.91 (m, 1H), 1.41 - 1.38 (m, 2H), 1.27 - 1.23 (m, 2H), 1.04 *(d, J* = 6.8 Hz, 6H). |
| | | **MS (ESI) M/Z:** 623.3 [M+H]⁺. |
| 057N0 | 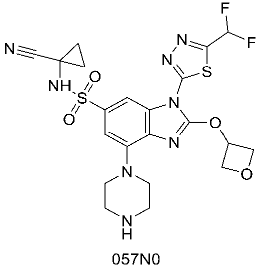 | **¹H NMR** (400 MHz, DMSO-*d₆*) δ 8.40 (s, 1H), 7.66 (t, *J* = 53.2 Hz, 1H), 7.18 (s, 1H), 6.11 - 6.07 (m, 1H), 5.96 - 5.91 (m, 1H), 5.00 - 4.97 (m, 2H), 4.89 - 4.86 (m, 2H), 3.53 - 3.47 (m, 5H), 2.91 - 2.89 (m, 3H), 1.33 - 1.29 (m, 2H), 1.23 (s, 1H), 1.19 - 1.15 (m, 2H). |
| | | **MS (ESI) M/Z:** 553.3 [M+H]⁺. |
| 057P | 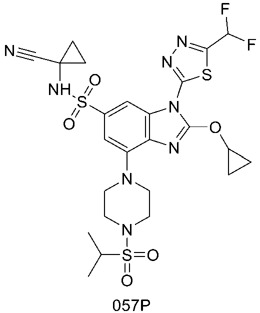 | **¹H NMR** (400 MHz, DMSO-*d₆*) δ 9.07 (s, 1H), 8.47 (d, *J* = 0.8 Hz, 1H), 7.63 (t, *J* = 52.8 Hz, 1H), 7.27 (s, 1H), 4.79 - 4.75 (m, 1H), 3.62 - 3.55 (m, 4H), 3.54 - 3.47 (m, 4H), 3.44 - 3.38 (m, 1H), 1.42 - 1.38 (m, 2H), 1.30 - 1.23 (m, 8H), 1.11 - 1.07 (m, 2H), 0.97 - 0.92 (m, 2H). |
| | | **MS (ESI) M/Z:** 643.3 [M+H]⁺. |
| 057Q | 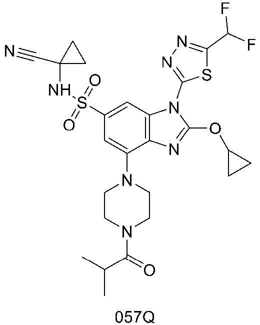 | **¹H NMR** (400 MHz, DMSO-*d₆*) δ 9.06 (s, 1H), 8.46 (s, 1H), 7.63 (t*, J* = 53.6 Hz, 1H), 7.25 (s, 1H), 4.83 - 4.78 (m, 1H), 3.80 - 3.65 (m, 4H), 3.55 - 3.48 (m, 4H), 2.98 - 2.94 (m, 1H), 1.39 - 1.35 (m, 2H), 1.26 - 1.22 (m, 2H), 1.10 - 0.94 (m, 10H). |
| | | **MS (ESI) M/Z:** 607.4 [M+H]⁺. |
| 058B | 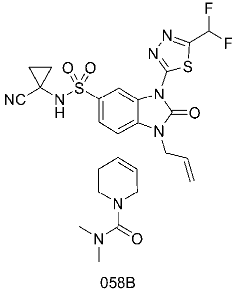 | **¹H NMR** (400 MHz, DMSO-*d₆*) δ 12.51 (s, 1H), 8.73 (d, *J* = 1.6 Hz, 1H), 7.77 - 7.50 (m, 2H), 6.08 - 6.06 (m, 1H), 5.86 - 5.79 (m, 1H), 5.37 - 5.33 (m, 1H), 5.25 - 5.23 (m, 1H), 3.99 - 3.97 (m, 2H), 3.90 - 3.89 (m, 2H), 3.41 - 3.38 (m, 2H), 2.79 (s, 6H), 2.55 - 2.51 (m, 2H), 1.59 - 1.57 (m, 4H). |
| | | **MS (ESI) M/Z:** 605.2 [M+H]⁺. |
| 058E | 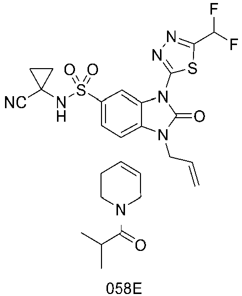 | **¹H NMR** (400 MHz, DMSO-*d₆*) δ 12.53 (s, 1H), 8.74 (s, 1H), 7.77 - 7.50 (m, 2H), 6.10 - 6.08 (m, 1H), 5.85 - 5.77 (m, 1H), 5.37 - 5.33 (m, 1H), 5.25 - 5.23 (m, 1H), 4.26 - 4.16 (m, 2H), 3.99 - 3.97 (m, 2H), 3.75 - 3.74 (m, 2H), 2.99 - 2.96 (m, 1H), 2.60 - 2.58 (m, 2H), 1.59 - 1.57 (m, 4H), 1.05 (d, *J* = 6.8 Hz, 6H). |
| | | **MS (ESI) M/Z:** 604.2 [M+H]⁺. |
| 059C | 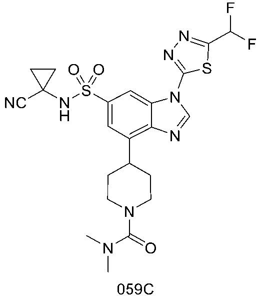 | **¹H NMR** (400 MHz, DMSO-*d₆*) δ 9.20 (s, 1H), 8.66 - 8.65 (m, 2H), 7.78 (s, 1H), 7.69 (t, *J* = 53.2 Hz, 1H), 3.75 - 3.72 (m, 2H), 3.58 - 3.50 (m, 1H), 2.95 - 2.92 (m, 2H), 2.78 (s, 6H), 1.90 - 1.78 (m, 4H), 1.42 - 1.38 (m, 2H), 1.29 - 1.25 (m, 2H). |
| | | **MS (ESI) M/Z:** 551.1 [M+H]⁺. |
| 059D | 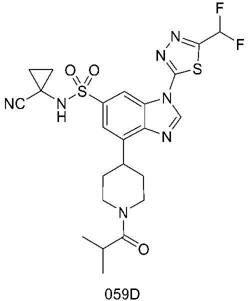 | **¹H NMR** (400 MHz, DMSO-*d₆*) δ 9.21 (s, 1H), 8.66 (s, 1H), 8.43 (s, 1H), 7.77 (s, 1H), 7.69 (t, *J* = 53.2 Hz, 1H), 4.66 - 4.62 (m, 1H), 4.17 - 4.13 (m, 1H), 3.63 (t, *J* = 12.0 Hz, 1H), 3.25 (t, *J* = 12.8 Hz, 1H), 2.97 - 2.92 (m, 1H), 2.74 - 2.70 (m, 1H), 2.02 - 1.97 (m, 2H), 1.80 - 1.65 (m, 2H), 1.41 - 1.39 (m, 2H), 1.30 - 1.27 (m, 2H), 1.07 - 1.01 (m, 6H). |
| | | **MS (ESI) M/Z:** 550.1 [M+H]⁺. |
| 065J | 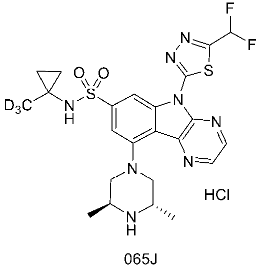 | **¹H NMR** (400 MHz, DMSO-*d₆*) δ 9.29 (s, 2H), 9.17 (s, 1H), 8.95 (d, *J* = 2.8 Hz, 1H), 8.74 (d, *J* = 2.4 Hz, 1H), 8.45 (s, 1H), 7.70 (t, *J* = 53.2 Hz, 1H), 7.53 (s, 1H), 4.09 - 3.90 (m, 2H), 3.80 - 3.65 (m, 2H), 3.37 - 3.33 (m, 2H), 1.46 (d, *J* = 6.8 Hz, 6H), 0.72 - 0.65 (m, 2H), 0.45 - 0.41 (m, 2H). |
| | | **MS (ESI) M/Z:** 552.1 [M+H]⁺. |
| 065K | 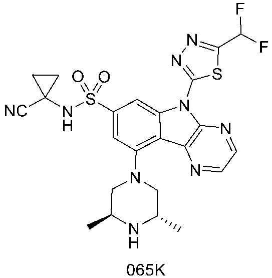 | **¹H NMR** (400 MHz, DMSO-*d₆*) δ 9.12 (s, 1H), 8.93 (d, *J* = 2.4 Hz, 1H), 8.70 (d, *J* = 2.8 Hz, 1H), 7.70 (t, *J* = 53.2 Hz, 1H), 7.49 (s, 1H), 3.54 - 3.49 (m, 4H), 3.07 - 3.02 (m, 2H), 1.44 - 1.42 (m, 2H), 1.33 - 1.29 (m, 2H), 1.23 (d, *J* = 6.4 Hz, 6H). |
| | | **MS (ESI) M/Z:** 560.0 [M+H]⁺. |
| 065L | 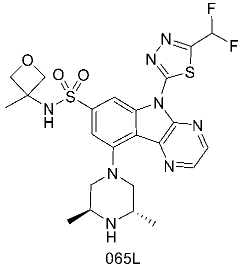 | **¹H NMR** (400 MHz, DMSO-*d₆*) δ 9.08 (d, *J* = 1.2 Hz, 1H), 8.90 (d, *J* = 2.8 Hz, 1H), 8.68 (d, *J* = 2.8 Hz, 1H), 7.69 (t, *J* = 53.2 Hz, 1H), 7.46 (s, 1H), 4.60 (d, *J* = 4.8 Hz, 2H), 4.14 (d, *J =* 6.0 Hz, 2H), 3.49 - 3.46 (m, 4H), 3.02 - 2.97 (m, 2H), 1.48 (s, 3H), 1.21 (d, *J =* 6.4 Hz, 6H). |
| | | **MS (ESI) M/Z:** 565.4 [M+H]⁺. |
| 065M | 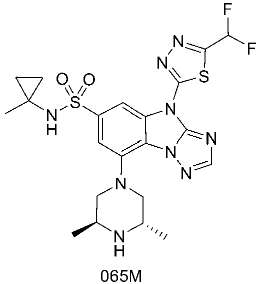 | **¹H NMR** (400 MHz, DMSO-*d₆*) δ 8.72 (d, *J* = 1.2 Hz, 1H), 8.48 (s, 1H), 8.33 (s, 1H), 7.68 (t, *J* = 53.2 Hz, 1H), 7.47 (d, *J* = 1.2 Hz, 1H), 3.41 - 3.32 (m, 4H), 2.89 - 2.85 (m, 2H), 1.19 (d, *J =* 6.8 Hz, 6H), 1.09 (s, 3H), 0.67 - 0.64 (m, 2H), 0.42 - 0.39 (m, 2H). |
| | | **MS (ESI) M/Z:** 538.3 [M+H]⁺. |
| 065N | 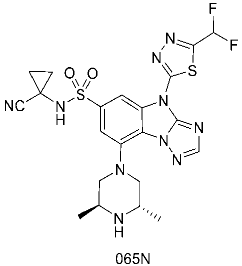 | **¹H NMR** (400 MHz, DMSO-*d₆*) δ 8.76 (s, 1H), 8.51 (s, 1H), 7.68 (t, *J =* 53.2 Hz, 1H), 7.51 (s, 1H), 3.43 - 3.35 (m, 4H), 2.93 - 2.89 (m, 2H), 1.44 - 1.41 (m, 2H), 1.29 - 1.27 (m, 2H), 1.20 (d, *J* = 6.4 Hz, 6H). |
| | | **MS (ESI) M/Z:** 549.0 [M+H]⁺. |
| 065P | 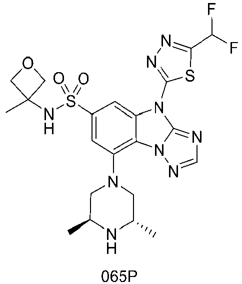 | **¹H NMR** (400 MHz, DMSO-*d₆*) δ 8.72 (d, *J* = 1.2 Hz, 1H), 8.64 (s, 1H), 8.49 (s, 1H), 7.67 (t, *J* = 53.2 Hz, 1H), 7.46 (d, *J* = 1.2 Hz, 1H), 4.57 - 4.55 (m, 2H), 4.12 (d, *J* = 6.4 Hz, 2H), 3.41 - 3.32 (m, 4H), 2.89 - 2.84 (m, 2H), 1.46 (s, 3H), 1.18 (d, *J* = 6.4 Hz, 6H). |
| | | **MS (ESI) M/Z:** 554.0 [M+H]⁺. |
| 067B | 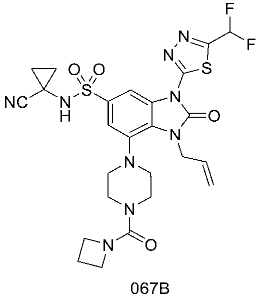 | **¹H NMR** (400 MHz, DMSO-*d₆*) δ 9.06 (s, 1H), 8.75 (d, *J* = 1.6 Hz, 1H), 7.78 - 7.51 (m, 2H), 6.09 - 6.02 (m, 1H), 5.14 - 5.02 (m, 2H), 4.93 - 4.89 (m, 2H), 3.94 (t, *J* = 7.6 Hz, 4H), 3.79 - 3.76 (m, 2H), 3.02 - 2.96 (m, 4H), 2.85 - 2.80 (m, 2H), 2.19 - 2.15 (m, 2H), 1.42 - 1.39 (m, 2H), 1.28 - 1.25 (m, 2H). |
| | | **MS (ESI) M/Z:** 620.1 [M+H]⁺. |
| 067G | 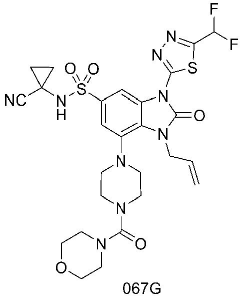 | **¹H NMR** (400 MHz, DMSO-*d₆*) δ 9.17 (s, 1H), 8.76 (d, *J* = 1.6 Hz, 1H), 7.78 - 7.51 (m, 2H), 6.09 - 6.02 (m, 1H), 5.15 - 5.12 (m, 1H), 5.07 - 5.02 (m, 1H), 4.94 - 4.93 (m, 2H), 3.65 - 3.58 (m, 6H), 3.22 - 3.20 (m, 4H), 3.06 - 2.91 (m, 6H), 1.43 - 1.40 (m, 2H), 1.29 - 1.26 (m, 2H). |
| | | **MS (ESI) M/Z:** 650.2 [M+H]⁺. |
| 067J | 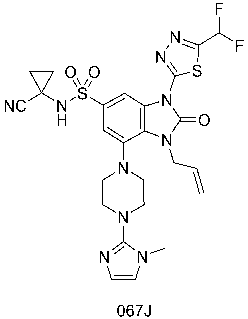 | **¹H NMR** (400 MHz, DMSO-*d₆*) δ 9.14 (s, 1H), 8.77 (d, *J* = 1.6 Hz, 1H), 7.83 (d, *J* = 2.0 Hz, 1H), 7.66 (t, *J* = 53.2 Hz, 1H), 6.91 (d, *J* = 1.6 Hz, 1H), 6.63 (d, *J* = 1.2 Hz, 1H), 6.11 - 6.02 (m, 1H), 5.16 - 5.14 (m, 1H), 5.09 - 5.05 (m, 1H), 4.98 - 4.97 (m, 2H), 3.50 (s, 3H), 3.26 - 3.23 (m, 2H), 3.17 - 3.02 (m, 6H), 1.45 - 1.38 (m, 2H), 1.31 - 1.28 (m, 2H). |
| | | **MS (ESI) M/Z:** 617.2 [M+H]⁺. |
| 067JD | 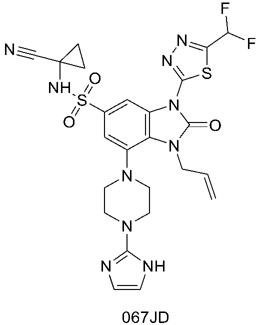 | **¹H NMR** (400 MHz, DMSO-*d₆*) δ 8.77 (d, *J* = 1.6 Hz, 1H), 8.20 (s, 1H), 7.79 - 7.52 (m, 2H), 6.65 (s, 2H), 6.10 - 6.01 (m, 1H), 5.14 - 5.12 (m, 1H), 5.07 - 5.02 (m, 1H), 4.96 - 4.95 (m, 2H), 3.84 - 3.81 (m, 2H), 3.09 - 3.05 (m, 2H), 3.02 - 2.97 (m, 4H), 1.45 - 1.42 (m, 2H), 1.31 - 1.28 (m, 2H). |
| | | **MS (ESI) M/Z:** 603.2 [M+H]⁺. |
| 067M | 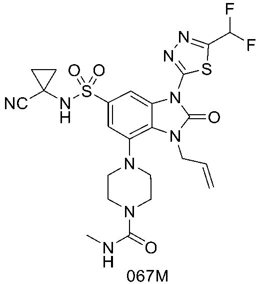 | **¹H NMR** (400 MHz, DMSO-*d₆*) δ 9.18 (s, 1H), 8.75 (d, *J* = 1.2 Hz, 1H), 7.78 - 7.52 (m, 2H), 6.58 - 6.56 (m, 1H), 6.09 - 6.02 (m, 1H), 5.14 - 5.11 (m, 1H), 5.06 - 5.02 (m, 1H), 4.93 - 4.92 (m, 2H), 4.00 - 3.96 (m, 2H), 3.00 - 2.92 (m, 4H), 2.81 - 2.78 (m, 2H), 2.60 (d, *J* = 4.0 Hz, 3H), 1.42 - 1.40 (m, 2H), 1.28 - 1.26 (m, 2H). |
| | | **MS (ESI) M/Z:** 594.1 [M+H]⁺. |
| 067ND | 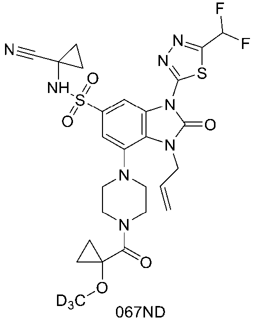 | **¹H NMR** (400 MHz, DMSO-*d₆*) δ 9.17 (s, 1H), 8.77 (s, 1H), 7.78 - 7.51 (m, 2H), 6.13 - 6.05 (m, 1H), 5.14 (d,*J* = 10.8 Hz, 1H), 5.05 (d, *J* = 17.2 Hz, 1H), 4.96 - 4.95 (m, 2H), 4.45 - 4.39 (m, 2H), 3.30 - 3.07 (m, 4H), 2.90 - 2.85 (m, 2H), 1.44 - 1.41 (m, 2H), 1.30 - 1.26 (m, 2H), 1.04 - 1.02 (m, 2H), 0.93 - 0.92 (m, 2H). |
| | | **MS (ESI) M/Z:** 638.2 [M+H]⁺. |
| 067P | 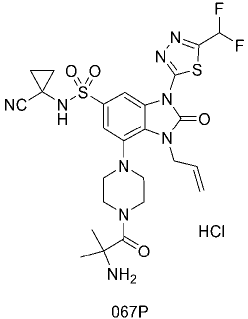 | **¹H NMR** (400 MHz, DMSO-*d₆*) δ 9.29 (s, 1H), 8.78 (s, 1H), 8.27 (s, 3H), 7.83 (s, 1H), 7.65 (t, *J* = 53.2 Hz, 1H), 6.13 - 6.05 (m, 1H), 5.15 - 5.12 (m, 1H), 5.07 - 5.03 (m, 1H), 4.95 - 4.94 (m, 2H), 4.34 - 4.31 (m, 2H), 3.30 - 3.19 (m, 2H), 3.08 - 3.06 (m, 2H), 2.91 - 2.89 (m, 2H), 1.62 (s, 6H), 1.44 - 1.40 (m, 2H), 1.31 - 1.29 (m, 2H). |
| | | **MS (ESI) M/Z:** 622.2 [M+H]⁺. |
| 067Q | 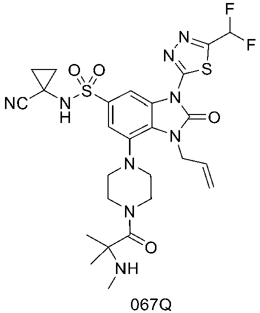 | **¹H NMR** (400 MHz, DMSO-*d₆*) δ 9.17 (s, 1H), 8.76 (d, *J* = 1.6 Hz, 1H), 7.78 - 7.52 (m, 2H), 6.11 - 6.03 (m, 1H), 5.15 - 5.13 (m, 1H), 5.07 - 5.03 (m, 1H), 4.96 - 4.95 (m, 2H), 3.35 - 3.33 (m, 2H), 3.06 - 2.98 (m, 4H), 2.86 - 2.82 (m, 2H), 2.22 (s, 3H), 1.43 - 1.40 (m, 2H), 1.30 - 1.27 (m, 8H). |
| | | **MS (ESI) M/Z:** 636.4 [M+H]⁺. |
| 067S | 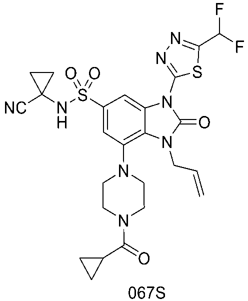 | **¹H NMR** (400 MHz, DMSO-*d₆*) δ 8.83 (s, 1H), 8.77 (d, *J* = 1.6 Hz, 1H), 7.78 - 7.52 (m, 2H), 6.13 - 6.04 (m, 1H), 5.15 - 5.03 (m, 2H), 4.96 - 4.95 (m, 2H), 4.42 - 4.39 (m, 2H), 3.38 - 3.36 (m, 1H), 3.07 - 3.05 (m, 2H), 2.90 - 2.79 (m, 3H), 2.07 - 2.04 (m, 1H), 1.44 - 1.41 (m, 2H), 1.30 - 1.27 (m, 2H), 0.76 - 0.73 (m, 4H). |
| | | **MS (ESI) M/Z:** 605.1 [M+H]⁺. |
| 067T | 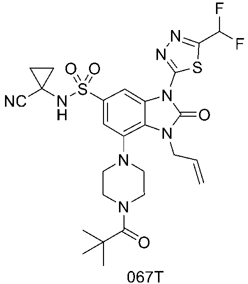 | **¹H NMR** (400 MHz, DMSO-*d₆*) δ 9.18 (s, 1H), 8.76 (d, *J* = 1.2 Hz, 1H), 7.78 - 7.52 (m, 2H), 6.11 - 6.04 (m, 1H), 5.15 - 5.02 (m, 2H), 4.95 - 4.94 (m, 2H), 4.40 - 4.37 (m, 2H), 3.12 - 3.03 (m, 4H), 2.85 - 2.79 (m, 2H), 1.44 - 1.40 (m, 2H), 1.30 - 1.27 (m, 2H), 1.25 (s, 9H). |
| | | **MS (ESI) M/Z:** 621.2 [M+H]⁺. |
| 067U | 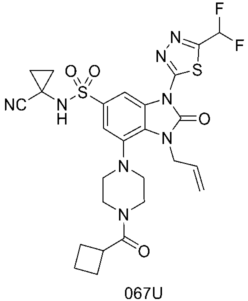 | **¹H NMR** (400 MHz, DMSO-*d₆*) δ 9.17 (s, 1H), 8.76 (d, *J* = 1.6 Hz, 1H), 7.78 - 7.51 (m, 2H), 6.10 - 6.02 (m, 1H), 5.14 - 5.12 (m, 1H), 5.06 - 5.02 (m, 1H), 4.94 - 4.92 (m, 2H), 4.44 - 4.41 (m, 1H), 3.79 - 3.76 (m, 1H), 3.43 - 3.37 (m, 1H), 3.24 (t, *J* = 11.6 Hz, 1H), 3.01 - 2.99 (m, 2H), 2.84 - 2.72 (m, 3H), 2.27 - 2.07 (m, 4H), 1.95 - 1.88 (m, 1H), 1.80 - 1.75 (m, 1H), 1.44 - 1.40 (m, 2H), 1.30 - 1.26 (m, 2H). |
| | | **MS (ESI) M/Z:** 619.1 [M+H]⁺. |
| 067V | 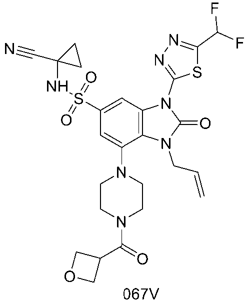 | **¹H NMR** (400 MHz, DMSO-*d₆*) δ 9.16 (s, 1H), 8.75 (d, *J* = 1.6 Hz, 1H), 7.78 - 7.52 (m, 2H), 6.11 - 6.05 (m, 1H), 5.14 - 5.02 (m, 2H), 4.94 - 4.93 (m, 2H), 4.76 - 4.66 (m, 3H), 4.46 - 4.44 (m, 1H), 4.20 - 4.16 (m, 1H), 3.47 - 3.44 (m, 1H), 3.27 - 3.21 (m, 1H), 3.05 - 2.79 (m, 6H), 1.39 - 1.37 (m, 2H), 1.25 - 1.23 (m, 2H). |
| | | **MS (ESI) M/Z:** 621.1 [M+H]⁺. |
| 067W1 | 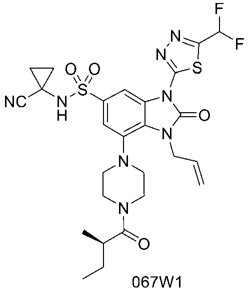 | **¹H NMR** (400 MHz, DMSO-*d₆*) δ 9.18 (s, 1H), 8.77 (s, 1H), 7.78 - 7.52 (m, 2H), 6.12 - 6.03 (m, 1H), 5.15 - 5.12 (m, 1H), 5.07 - 5.02 (m, 1H), 4.95 - 4.94 (m, 2H), 4.53 - 4.49 (m, 1H), 4.11 - 4.08 (m, 1H), 3.36 - 3.34 (m, 1H), 3.07 - 3.04 (m, 2H), 2.90 - 2.74 (m, 4H), 1.62 - 1.59 (m, 1H), 1.43 - 1.40 (m, 2H), 1.34 - 1.26 (m, 3H), 1.05 - 1.00 (m, 3H), 0.87 - 0.83 (m, 3H). |
| | | **MS (ESI) M/Z:** 621.1 [M+H]⁺. |
| 067W2 | 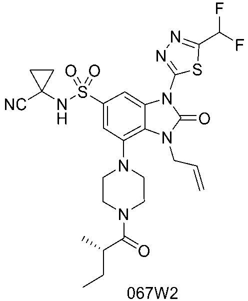 | **¹H NMR** (400 MHz, DMSO-*d₆*) δ 9.18 (s, 1H), 8.76 (d, *J* = 1.2 Hz, 1H), 7.78 - 7.52 (m, 2H), 6.11 - 6.04 (m, 1H), 5.15 - 5.02 (m, 2H), 4.95 - 4.94 (m, 2H), 4.52 - 4.49 (m, 1H), 4.11 - 4.08 (m, 1H), 3.36 - 3.34 (m, 1H), 3.06 - 3.03 (m, 2H), 2.87 - 2.74 (m, 4H), 1.62 - 1.59 (m, 1H), 1.39 - 1.26 (m, 5H), 1.06 - 0.99 (m, 3H), 0.87 - 0.83 (m, 3H). |
| | | **MS (ESI) M/Z:** 621.2 [M+H]⁺. |
| 067X | 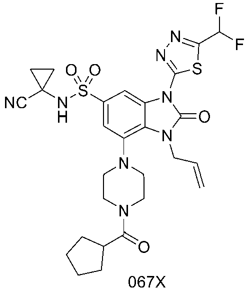 | **¹H NMR** (400 MHz, DMSO-*d₆*) δ 9.18 (s, 1H), 8.77 (d, *J* = 1.6 Hz, 1H), 7.78 - 7.51 (m, 2H), 6.11 - 6.04 (m, 1H), 5.15 - 5.03 (m, 2H), 4.95 - 4.94 (m, 2H), 4.49 - 4.46 (m, 1H), 4.10 - 4.07 (m, 1H), 3.34 - 3.31 (m 1H), 3.07 - 3.03 (m, 3H), 2.90 - 2.75 (m, 3H), 1.80 - 1.54 (m, 8H), 1.43 - 1.41 (m, 2H), 1.30 - 1.28 (m, 2H). |
| | | **MS (ESI) M/Z:** 633.1 [M+H]⁺. |
| 067Y | 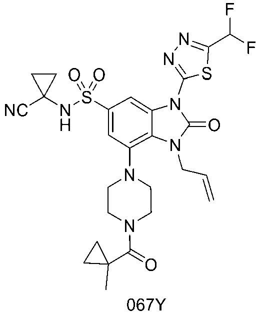 | **¹H NMR** (400 MHz, DMSO-*d₆*) δ 9.18 (s, 1H), 8.77 (s, 1H), 7.79 (d, *J* = 0.8 Hz, 1H), 7.65 (t, *J* = 53.2 Hz, 1H), 6.12 - 6.04 (m, 1H), 5.15 - 5.13 (m, 1H), 5.07 - 5.03 (m, 1H), 4.96 - 4.95 (m, 2H), 4.34 - 4.31 (m, 2H), 3.15 - 3.03 (m, 4H), 2.89 - 2.84 (m, 2H), 1.44 - 1.41 (m, 2H), 1.30 - 1.26 (m, 5H), 0.87 - 0.85 (m, 2H), 0.59 - 0.56 (m, 2H). |
| | | **MS (ESI) M/Z:** 619.2 [M+H]⁺. |
| 069D | 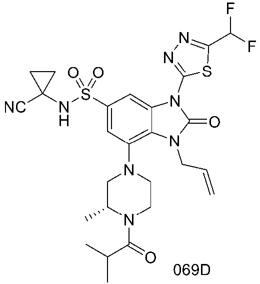 | **¹H NMR** (400 MHz, DMSO-*d₆*) δ 9.13 (s, 1H), 8.77 (s, 1H), 7.76 (s, 1H), 7.65 (t, *J* = 53.2 Hz, 1H), 6.12 - 6.05 (m, 1H), 5.14 (d, *J =* 9.6 Hz, 1H), 5.10 (m, 0.5H), 5.02 (d, *J =* 17.2 Hz, 1H), 4.94 - 4.92 (m, 1H), 4.79 - 4.78 (m, 0.5H), 4.43 - 4.42 (m, 0.5H), 4.33 - 4.32 (m, 0.5H), 3.88 - 3.87 (m, 0.5H), 3.51 - 3.49 (m, 0.5H), 3.21 - 2.88 (m, 5H), 2.67 - 2.66 (m, 0.5H), 2.51 - 2.50 (m, 0.5H), 1.38 (s, 3H), 1.30 - 1.22 (m, 4H), 1.08 - 0.98 (m, 6H). |
| | | **MS (ESI) M/Z:** 621.4 [M+H]⁺. |
| 069E | 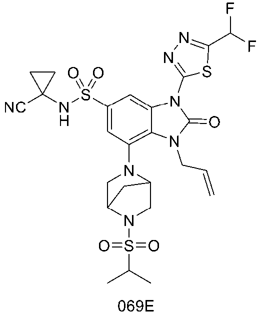 | **¹H NMR** (400 MHz, DMSO-*d₆*) δ 9.25 (s, 1H), 8.73 (d, *J* = 1.6 Hz, 1H), 7.78 - 7.51 (m, 2H), 6.03 - 5.94 (m, 1H), 5.14 (d, *J* = 10.0 Hz, 1H), 5.09 - 5.00 (m, 2H), 4.79 - 4.74 (m, 1H), 4.47 - 4.46 (m, 1H), 4.05 - 4.04 (m, 1H), 3.59 - 3.56 (m, 1H), 3.41 - 3.35 (m, 3H), 3.26 - 3.22 (m, 1H), 2.21 - 2.18 (m, 1H), 1.97 - 1.94 (m, 1H), 1.46 - 1.39 (m, 2H), 1.35 - 1.26 (m, 8H). |
| | | **MS (ESI) M/Z:** 655.1 [M+H]⁺. |
| 070A | 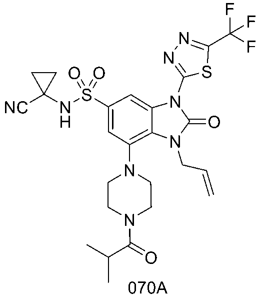 | **¹H NMR** (400 MHz, DMSO-*d₆*) δ 9.12 (s, 1H), 8.75 (d, *J* = 1.6 Hz, 1H), 7.79 (d, *J* = 1.6 Hz, 1H), 6.11 - 6.04 (m, 1H), 5.15 - 5.13 (m, 1H), 5.08 - 5.03 (m, 1H), 4.96 - 4.95 (m, 2H), 4.49 - 4.46 (m, 1H), 4.07 - 4.05 (m, 1H), 3.34 - 3.31 (m, 1H), 3.06 - 3.03 (m, 2H), 2.96 - 2.83 (m, 4H), 1.44 - 1.41 (m, 2H), 1.30 - 1.27 (m, 2H), 1.05 - 1.03 (m, 6H). |
| | | **MS (ESI) M/Z:** 625.2 [M+H]⁺. |
| 070B | 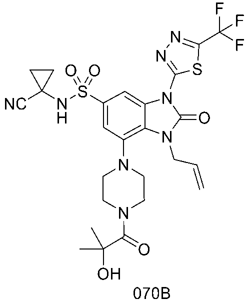 | **¹H NMR** (400 MHz, DMSO-*d₆*) δ 9.20 (s, 1H), 8.75 (d, *J* = 1.6 Hz, 1H), 7.77 (d, *J* = 1.6 Hz, 1H), 6.12 - 6.05 (m, 1H), 5.50 (s, 1H), 5.16 - 5.14 (m, 1H), 5.08 - 5.04 (m, 1H), 4.96 - 4.95 (m, 2H), 4.89 - 4.25 (m, 2H), 3.14 - 2.96 (m, 3H), 2.95 - 2.71 (m, 3H), 1.44 - 1.40 (m, 2H), 1.36 (s, 6H), 1.29 - 1.26 (m, 2H). |
| | | **MS (ESI) M/Z:** 641.2 [M+H]⁺. |
| 071C2 | 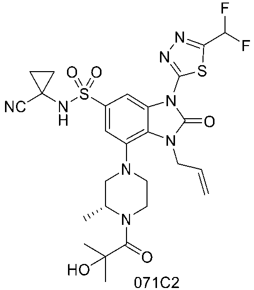 | **¹H NMR** (400 MHz, DMSO-*d₆*) δ 8.78 (s, 1H), 8.60 (s, 1H), 7.76 (s, 1H), 7.65 (t, *J* = 53.2 Hz, 1H), 6.12 - 6.05 (m, 1H), 5.51 - 5.48 (m, 0.5H), 5.30 - 5.29 (m, 0.5H), 5.16 - 5.14 (m, 1H), 5.11 - 5.10 (m, 0.5H), 5.05 - 5.00 (m, 1H), 4.95 - 4.90 (m, 1H), 4.81 - 4.80 (m, 0.5H), 4.76 - 4.75 (m, 0.5H), 4.30 - 4.29 (m, 0.5H), 3.47 - 3.45 (m, 0.5H), 3.25 - 3.23 (m, 0.5H), 3.10 - 2.96 (m, 2H), 2.95 - 2.85 (m, 1H), 2.75 - 2.73 (m, 0.5H), 2.52 - 2.51 (m, 0.5H), 1.50 - 1.48 (m, 3H), 1.40 - 1.36 (m, 6H), 1.30 - 1.27 (m, 4H). |
| | | **MS (ESI) M/Z:** 637.4 [M+H]⁺. |
| 072B | 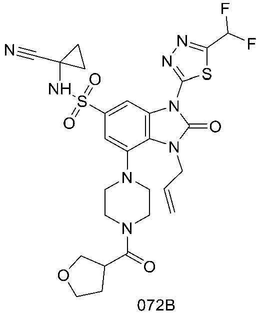 | **¹H NMR** (400 MHz, DMSO-*d₆*) δ 9.18 (s, 1H), 8.77 (d, *J* = 1.2 Hz, 1H), 7.78 - 7.51 (m, 2H), 6.11 - 6.04 (m, 1H), 5.13 (d, *J* = 10.4 Hz, 1H), 5.05 (d, *J* = 17.2 Hz, 1H), 4.95 - 4.90 (m, 2H), 4.53 - 4.45 (m, 1H), 4.18 - 4.05 (m, 1H), 3.96 - 3.84 (m, 1H), 3.82 - 3.68 (m, 3H), 3.45 - 3.39 (m, 1H), 3.05 - 2.99 (m, 2H), 2.90 - 2.78 (m, 4H), 2.08 - 1.98 (m, 2H), 1.41 - 1.39 (m, 2H), 1.30 - 1.26 (m, 2H). |
| | | **MS (ESI) M/Z:** 635.2 [M+H]⁺. |
| 072D | 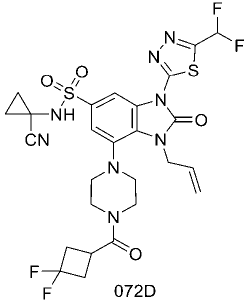 | **¹H NMR** (400 MHz, DMSO-*d₆*) δ 9.21 (s, 1H), 8.76 (d, *J* = 1.6 Hz, 1H), 7.79 - 7.52 (m, 2H), 6.10 - 6.03 (m, 1H), 5.14 - 5.02 (m, 2H), 4.94 - 4.93 (m, 2H), 4.45 - 4.42 (m, 1H), 3.87 - 3.84 (m, 1H), 3.30 - 3.26 (m, 2H), 3.04 - 2.98 (m, 2H), 2.93 - 2.77 (m, 7H), 1.44 - 1.41 (m, 2H), 1.30 - 1.26 (m, 2H). |
| | | **MS (ESI) M/Z:** 655.0 [M+H]⁺. |
| 073A | 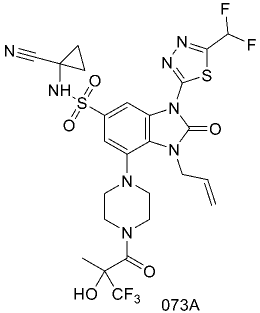 | **¹H NMR** (400 MHz, DMSO-*d₆*) δ 8.93 (s, 1H), 8.77 (d, *J* = 1.6 Hz, 1H), 7.78 - 7.52 (m, 2H), 7.20 (s, 1H), 6.12 - 6.04 (m, 1H), 5.14 (d, *J* = 11.2 Hz, 1H), 5.05 (d, *J* = 17.6 Hz, 1H), 4.96 - 4.95 (m, 2H), 4.86 - 4.83 (m, 1H), 4.47 - 4.45 (m, 1H), 3.05 - 2.87 (m, 6H), 1.58 (s, 3H), 1.43 - 1.40 (m, 2H), 1.30 - 1.26 (m, 2H). |
| | | **MS (ESI) M/Z:** 677.3 [M+H]⁺. |
| 073B | 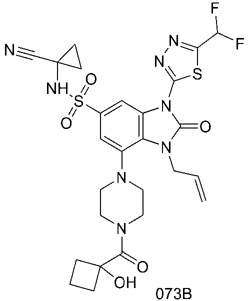 | **¹H NMR** (400 MHz, DMSO-*d₆*) δ 9.02 (s, 1H), 8.76 (d, *J* = 1.6 Hz, 1H), 7.78 - 7.51 (m, 2H), 6.11 - 6.02 (m, 2H), 5.14 (d, *J* = 10.0 Hz, 1H), 5.05 (d, *J* = 18.0 Hz, 1H), 4.96 - 4.95 (m, 2H), 4.45 - 4.42 (m, 1H), 4.18 - 4.15 (m, 1H), 3.30 - 3.25 (m, 1H), 3.06 - 2.87 (m, 5H), 2.65 - 2.55 (m, 2H), 2.09 - 2.07 (m, 2H), 1.77 - 1.75 (m, 1H), 1.45 - 1.40 (m, 3H), 1.30 - 1.26 (m, 2H). |
| | | **MS (ESI) M/Z:** 635.4 [M+H]⁺. |
| 074A-11 | 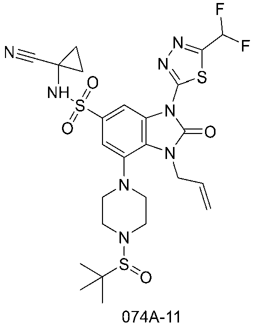 | **¹H NMR** (400 MHz, DMSO-*d₆*) δ 9.20 (s, 1H), 8.76 (d, *J* = 1.6 Hz, 1H), 7.79 - 7.51 (m, 2H), 6.10 - 6.00 (m, 1H), 5.12 (d, *J* = 11.2 Hz, 1H), 5.02 (d, *J* = 18.4 Hz, 1H), 4.93 - 4.91 (m, 2H), 3.38 - 3.34 (m, 2H), 3.20 - 3.15 (m, 1H), 3.06 - 2.91 (m, 5H), 1.44 - 1.40 (m, 2H), 1.30 - 1.27 (m, 2H), 1.15 (s, 9H). |
| | | **MS (ESI) M/Z:** 641.1 [M+H]⁺. |
| 074A | 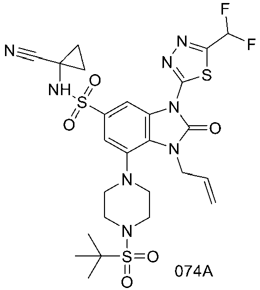 | **¹H NMR** (400 MHz, DMSO-*d₆*) δ 9.12 (s, 1H), 8.77 (s, 1H), 7.81 - 7.51 (m, 2H), 6.14 - 6.01 (m, 1H), 5.12 (d,*J* = 9.2 Hz, 1H), 5.02 (d, *J* = 17.2 Hz, 1H), 4.93 - 4.91 (m, 2H), 3.71 - 3.65 (m, 2H), 3.32 - 3.30 (m, 2H), 3.05 - 2.92 (m, 4H), 1.44 - 1.40 (m, 2H), 1.36 - 1.27 (m, 11H). |
| | | **MS (ESI) M/Z:** 657.0 [M+H]⁺. |
| 074B | 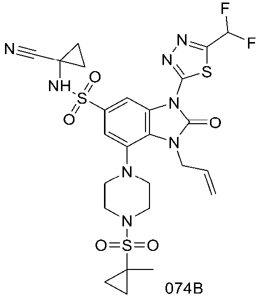 | **¹H NMR** (400 MHz, DMSO-*d₆*) δ 9.20 (s, 1H), 8.78 (d, *J* = 1.6 Hz, 1H), 7.80 - 7.51 (m, 2H), 6.12 - 6.04 (m, 1H), 5.14 (d, *J* = 10.4 Hz, 1H), 5.03 (d, *J* = 18.0 Hz, 1H), 4.93 - 4.91 (m, 2H), 3.74 - 3.71 (m, 2H), 3.23 - 3.14 (m, 2H), 3.10 - 3.06 (m, 2H), 2.96 - 2.91 (m, 2H), 1.48 (s, 3H), 1.44 - 1.38 (m, 2H), 1.31 - 1.27 (m, 2H), 1.22 - 1.19 (m, 2H), 0.91 - 0.89 (m, 2H). |
| | | **MS (ESI) M/Z:** 655.0 [M+H]⁺. |
| 074C | 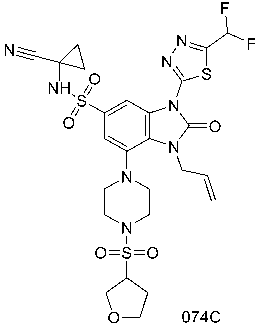 | **¹H NMR** (400 MHz, DMSO-*d₆*) δ 9.14 (s, 1H), 8.78 (d, *J* = 1.2 Hz, 1H), 7.79 - 7.51 (m, 2H), 6.12 - 6.04 (m, 1H), 5.13 (*J* = 10.8 Hz, 1H), 5.03 (d, *J* = 17.2 Hz, 1H), 4.92 - 4.90 (m, 2H), 4.19 - 4.10 (m, 1H), 4.03 - 3.97 (m, 1H), 3.94 - 3.83 (m, 2H), 3.72 - 3.64 (m, 3H), 3.20 - 3.03 (m, 4H), 2.99 - 2.86 (m, 2H), 2.36 - 2.23 (m, 1H), 2.19 - 2.09 (m, 1H), 1.45 - 1.41 (m, 2H), 1.31 - 1.27 (m, 2H). |
| | | **MS (ESI) M/Z:** 671.2 [M+H]⁺. |
| 074H | 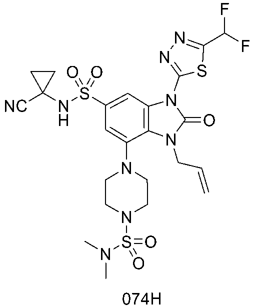 | **¹H NMR** (400 MHz, DMSO-*d₆*) δ 9.24 (s, 1H), 8.78 (d, *J* = 2.0 Hz, 1H), 7.81 (d, *J =* 1.6 Hz, 1H), 7.65 (t, *J =* 53.2 Hz, 1H), 6.12 - 6.01 (m, 1H), 5.14 (dd, *J =* 10.4 Hz, 0.8 Hz, 1H), 5.03 (dd, *J =* 17.6 Hz, 1.2 Hz, 1H), 4.94 - 4.89 (m, 2H), 3.63 - 3.54 (m, 2H), 3.14 - 3.03 (m, 4H), 3.02 - 2.91 (m, 2H), 2.82 (s, 6H), 1.45 - 1.42 (m, 2H), 1.32 - 1.28 (m, 2H). |
| | | **¹⁹FNMR** (376 MHz, DMSO-*d₆*) δ -109.76. |
| | | **MS (ESI) M/Z:** 643.9 [M+H]⁺. |
| 075A | 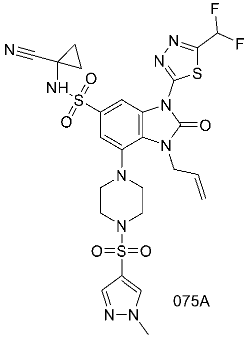 | **¹H NMR** (400 MHz, DMSO-*d₆*) δ 9.20 (s, 1H), 8.76 (s, 1H), 8.41 (s, 1H), 7.86 (s, 1H), 7.78 - 7.51 (m, 2H), 5.88 - 5.80 (m, 1H), 5.04 (d, *J* = 10.4 Hz, 1H), 4.89 (d, *J =* 17.2 Hz, 1H), 4.77 - 4.75 (m, 2H), 3.94 (s, 3H), 3.63 - 3.60 (m, 2H), 3.08 - 3.01 (m, 4H), 2.56 - 2.52 (m, 2H), 1.45 - 1.42 (m, 2H), 1.32 - 1.27 (m, 2H). |
| | | **MS (ESI) M/Z:** 681.1 [M+H]⁺. |
| 075B | 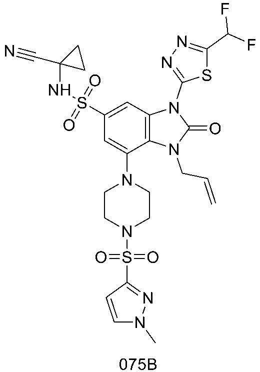 | **¹H NMR** (400 MHz, DMSO-*d₆*) δ 9.19 (s, 1H), 8.76 (d, *J* = 1.2 Hz, 1H), 8.01 (d, *J* = 2.0 Hz, 1H), 7.79 - 7.51 (m, 2H), 6.74 (d, *J* = 2.0 Hz, 1H), 5.93 - 5.81 (m, 1H), 5.07 (d, *J* = 10.8 Hz, 1H), 4.94 (d, *J* = 17.6 Hz, 1H), 4.78 - 4.77 (m, 2H), 4.00 (s, 3H), 3.70 - 3.66 (m, 2H), 3.12 - 2.96 (m, 4H), 2.79 - 2.74 (m, 2H), 1.47 - 1.38 (m, 2H), 1.33 - 1.25 (m, 2H). |
| | | **MS (ESI) M/Z:** 681.2 [M+H]⁺. |
| 075C | 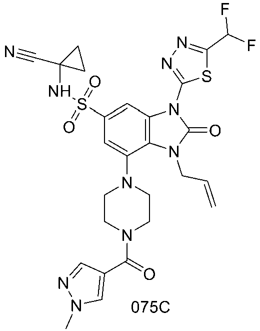 | **¹H NMR** (400 MHz, DMSO-*d₆*) δ 9.17 (s, 1H), 8.77 (d, *J* = 1.6 Hz, 1H), 8.12 (s, 1H), 7.79 - 7.51 (m, 3H), 6.12 - 6.05 (m, 1H), 5.14 (d, *J* = 10.4 Hz, 1H), 5.06 (d, *J* = 18.0 Hz, 1H), 4.97 - 4.95 (m, 2H), 4.37 - 4.33 (m, 2H), 3.87 (s, 3H), 3.26 - 3.15 (m, 2H), 3.07 - 3.05 (m, 2H), 2.93 - 2.88 (m, 2H), 1.43 - 1.40 (m, 2H), 1.29 - 1.25 (m, 2H). |
| | | **MS (ESI) M/Z:** 645.4 [M+H]⁺. |
| 075D | 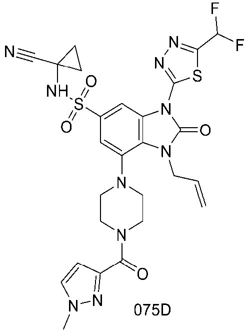 | **¹H NMR** (400 MHz, DMSO-*d₆*) δ 9.17 (s, 1H), 8.76 (d, *J* = 1.6 Hz, 1H), 7.79 - 7.51 (m, 3H), 6.60 (d, *J* = 2.4 Hz, 1H), 6.14 - 6.05 (m, 1H), 5.14 (d, *J* = 10.4 Hz, 1H), 5.06 (d, *J* = 17.6 Hz, 1H), 4.97 - 4.95 (m, 2H), 4.92 - 4.87 (m, 1H), 4.62 - 4.53 (m, 1H), 3.90 (s, 3H), 3.42 - 3.37 (m, 1H), 3.12 - 3.05 (m, 3H), 2.92 - 2.86 (m, 2H), 1.44 - 1.40 (m, 2H), 1.30 - 1.26 (m, 2H). |
| | | **MS (ESI) M/Z:** 645.1 [M+H]⁺. |
| 076C | 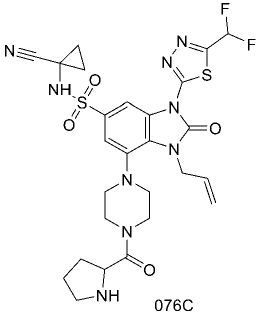 | **¹H NMR** (400 MHz, DMSO-*d₆*) δ 8.76 (d, *J* = 1.2 Hz, 1H), 7.78 - 7.51 (m, 2H), 6.12 - 6.03 (m, 1H), 5.13 (d, *J =* 10.8 Hz, 1H), 5.05 (d, *J* = 17.6 Hz, 1H), 4.95 - 4.93 (m, 2H), 4.46 - 4.43 (m, 1H), 4.09 - 4.01 (m, 1H), 3.95 - 3.86 (m, 1H), 3.05 - 2.99 (m, 3H), 2.93 - 2.87 (m, 2H), 2.81 - 2.72 (m, 1H), 2.69 - 2.63 (m, 1H), 2.10 - 1.93 (m, 2H), 1.75 - 1.54 (m, 3H), 1.41 - 1.38 (m, 2H), 1.27 - 1.24 (m, 2H). |
| | | **MS (ESI) M/Z:** 634.3 [M+H]⁺. |
| 076E | 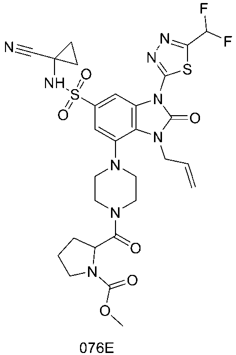 | **¹H NMR** (400 MHz, DMSO-*d₆*) δ 9.13 (s, 1H), 8.77 (s, 1H), 7.79 - 7.51 (m, 2H), 6.13 - 6.05 (m, 1H), 5.14 (d,*J* = 10.8 Hz, 1H), 5.07 - 5.01 (m, 1H), 4.96 - 4.94 (m, 2H), 4.80 - 4.68 (m, 1H), 4.42 - 4.37 (m, 1H), 4.15 - 4.03 (m, 1H), 3.59 - 3.51 (m, 3H), 3.40 - 3.36 (m, 3H), 3.11 - 2.97 (m, 2H), 2.90 - 2.71 (m, 3H), 2.30 - 2.08 (m, 1H), 1.93 - 1.75 (m, 3H), 1.47 - 1.37 (m, 2H), 1.28 - 1.23 (m, 2H). |
| | | **MS (ESI) M/Z:** 692.3 [M+H]⁺. |
| 076G | 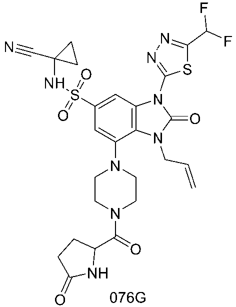 | **¹H NMR** (400 MHz, DMSO-*d₆*) δ 9.20 (s, 1H), 8.76 (d, *J* = 1.6 Hz, 1H), 7.80 - 7.52 (m, 3H), 6.11 - 6.04 (m, 1H), 5.13 (d, *J* = 10.8 Hz, 1H), 5.05 (d, *J* = 16.8 Hz, 1H), 4.96 - 4.94 (m, 2H), 4.66 - 4.56 (m, 1H), 4.45 - 4.41 (m, 1H), 4.02 - 3.95 (m, 1H), 3.39 - 3.35 (m, 1H), 3.08 - 3.03 (m, 2H), 2.93 - 2.77 (m, 3H), 2.37 - 2.32 (m, 1H), 2.15 - 2.09 (m, 2H), 2.04 - 1.90 (m, 1H), 1.44 - 1.40 (m, 2H), 1.30 - 1.26 (m, 2H). |
| | | **MS (ESI) M/Z:** 648.2 [M+H]⁺. |
| 076H2 | 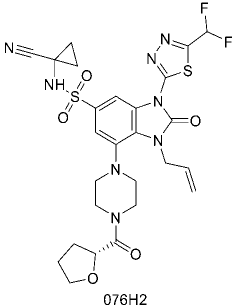 | **¹H NMR** (400 MHz, DMSO-*d₆*) δ 9.18 (s, 1H), 8.76 (s, 1H), 7.78 - 7.51 (m, 2H), 6.12 - 6.03 (m, 1H), 5.13 (d,*J* = 10.8 Hz, 1H), 5.04 (d, *J* = 16.8 Hz, 1H), 4.95 - 4.93 (m, 2H), 4.75 - 4.71 (m, 1H), 4.43 - 4.39 (m, 1H), 4.13 - 4.08 (m, 1H), 3.84 - 3.76 (m, 2H), 3.30 - 3.27 (m, 1H), 3.05 - 3.02 (m, 2H), 2.91 - 2.76 (m, 3H), 2.14 - 2.00 (m, 2H), 1.88 - 1.82 (m, 2H), 1.44 - 1.40 (m, 2H), 1.29 - 1.27 (m, 2H). |
| | | **MS (ESI) M/Z:** 635.0 [M+H]⁺. |
| 076J1 | 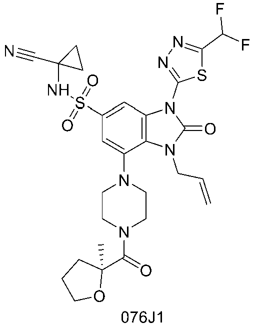 | **¹H NMR** (400 MHz, DMSO-*d₆*) δ 9.13 (s, 1H), 8.77 (d, *J* = 1.2 Hz, 1H), 7.78 - 7.51 (m, 2H), 6.12 - 6.03 (m, 1H), 5.13 (d, *J* = 10.8 Hz, 1H), 5.05 (d, *J* = 17.2 Hz, 1H), 4.96 - 4.95 (m, 2H), 4.81 - 4.60 (m, 1H), 4.56 - 4.35 (m, 1H), 3.92 - 3.84 (m, 1H), 3.82 - 3.72 (m, 1H), 3.25 - 3.22 (m, 1H), 3.07 - 3.04 (m, 2H), 2.94 - 2.76 (m, 3H), 2.70 - 2.66 (m, 1H), 1.92 - 1.82 (m, 1H), 1.81 - 1.75 (m, 1H), 1.62 - 1.57 (m, 1H), 1.43 (s, 3H), 1.41 - 1.40 (m, 2H), 1.30 - 1.26 (m, 2H). |
| | | **MS (ESI) M/Z:** 649.3 [M+H]⁺. |
| 076J2 | 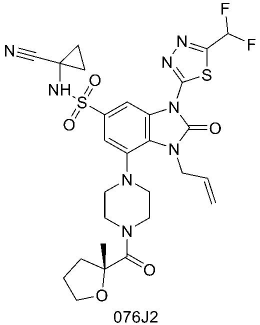 | **¹H NMR** (400 MHz, DMSO-*d₆*) δ 9.13 (s, 1H), 8.75 (d, *J* = 1.2 Hz, 1H), 7.78 - 7.51 (m, 2H), 6.12 - 6.03 (m, 1H), 5.13 (d, *J* = 10.8 Hz, 1H), 5.05 (d, *J* = 17.2 Hz, 1H), 4.96 - 4.95 (m, 2H), 4.81 - 4.60 (m, 1H), 4.56 - 4.35 (m, 1H), 3.92 - 3.84 (m, 1H), 3.82 - 3.72 (m, 1H), 3.25 - 3.22 (m, 1H), 3.07 - 3.04 (m, 2H), 2.94 - 2.80 (m, 3H), 2.70 - 2.66 (m, 1H), 1.87 - 1.85 (m, 1H), 1.77 - 1.75 (m, 1H), 1.62 - 1.57 (m, 1H), 1.43 (s, 3H), 1.42 - 1.40 (m, 2H), 1.30 - 1.26 (m, 2H). |
| | | **MS (ESI) M/Z:** 649.3 [M+H]⁺. |
| 077A | 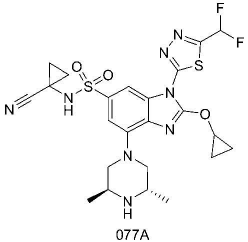 | **¹H NMR(400** MHz, DMSO-*d₆*) δ 8.38 (d, *J* = 1.6 Hz, 1H), 7.63 (t, *J =* 52.8 Hz, 1H), 7.18 (d, *J =* 1.6 Hz, 1H), 4.73 - 4.69 (m, 1H), 3.29 - 3.26 (m, 6H), 1.39 - 1.37 (m, 2H), 1.26 - 1.22 (m, 2H), 1.20 (d, *J* = 6.4 Hz, 6H), 1.10 - 1.08 (m, 2H), 0.96 - 0.90 (m, 2H). |
| | | **MS (ESI) M/Z:** 565.3 [M+H]⁺. |
| 077B | 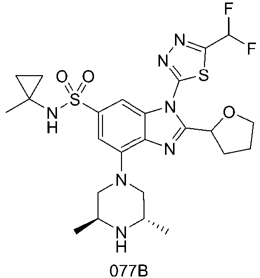 | **¹H NMR** (400 MHz, DMSO-*d₆*) δ 8.05 (s, 1H), 7.80 - 7.53 (m, 2H), 7.08 (s, 1H), 5.42 - 5.37 (m, 1H), 3.84 - 3.79 (m, 1H), 3.78 - 3.72 (m, 1H), 3.45 - 3.38 (m, 4H), 3.29 - 3.25 (m, 2H), 2.77 - 2.66 (m, 1H), 2.32 - 2.23 (m, 1H), 2.13 - 2.05 (m, 1H), 2.03 - 1.93 (m, 1H), 1.17 (d, *J* = 6.4 Hz, 6H), 1.03 (s, 3H), 0.63 - 0.58 (m, 2H), 0.38 - 0.33 (m, 2H). |
| | | **MS (ESI) M/Z:** 568.4 [M+H]⁺. |
| 077C | 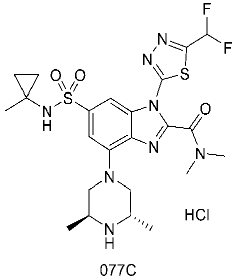 | **¹H NMR** (400 MHz, DMSO-*d₆*) δ 9.21 (s, 2H), 8.19 (s, 1H), 7.77 (s, 1H), 7.65 (t, *J* = 53.2 Hz, 1H), 7.24 (s, 1H), 3.78 - 3.67 (m, 6H), 3.35 (s, 3H), 3.08 (s, 3H), 1.43 (d, *J* = 6.0 Hz, 6H), 1.06 (s, 3H), 0.71 - 0.58 (m, 2H), 0.45 - 0.35 (m, 2H). |
| | | **MS (ESI) M/Z:** 569.1 [M+H]⁺. |
| 077D | 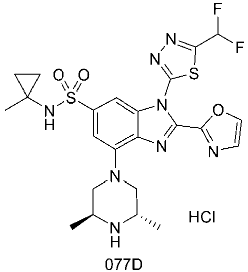 | **¹H NMR** (400 MHz, DMSO-*d₆*) δ 9.29 (s, 2H), 8.47 (s, 1H), 8.15 (s, 1H), 7.70 (t, *J* = 53.2 Hz, 1H), 7.50 (s, 1H), 7.34 (s, 1H), 7.23 (s, 1H), 3.83 - 3.79 (m, 6H), 1.45 (d, *J* = 5.6 Hz, 6H), 1.04 (s, 3H), 0.64 - 0.62 (m, 2H), 0.40 - 0.37 (m, 2H). |
| | | **MS (ESI) M/Z:** 565.2 [M+H]⁺. |
| 077G | 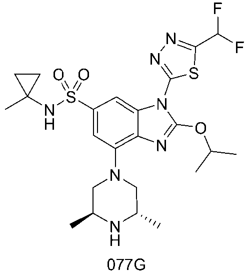 | **¹H NMR(400** MHz, DMSO-*d₆*) δ 8.36 (d, *J* = 1.2 Hz, 1H), 8.02 (s, 1H), 7.64 (t, *J* = 53.2 Hz, 1H), 7.15 (d, *J* = 1.6 Hz, 1H), 5.42 - 5.35 (m, 1H), 3.28 - 3.22 (m, 6H), 2.01 (s, 1H), 1.58 - 1.56 (m, 6H), 1.18 (d, *J* = 5.6 Hz, 6H), 1.04 (s, 3H), 0.66 - 0.59 (m, 2H), 0.38 - 0.33 (m, 2H). |
| | | **MS (ESI) M/Z:** 556.1 [M+H]⁺. |
| 077H | 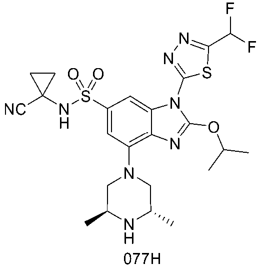 | **¹H NMR** (400 MHz, DMSO-*d₆*) δ 8.40 (d, *J* = 1.2 Hz, 1H), 7.64 (t, *J =* 53.2 Hz, 1H), 7.16 (d, *J* = 1.2 Hz, 1H), 5.43 - 5.37 (m, 1H), 3.27 - 3.24 (m, 6H), 1.59 - 1.56 (m, 6H), 1.39 - 1.37 (m, 2H), 1.24 - 1.22 (m, 2H), 1.19 (d, *J* = 5.6 Hz, 6H). |
| | | **MS (ESI) M/Z:** 567.3 [M+H]⁺. |
| 077J | 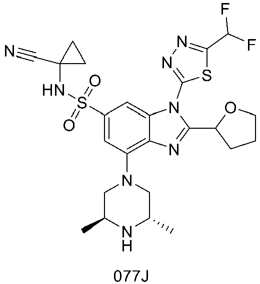 | **¹H NMR** (400 MHz, DMSO-*d₆*) δ 7.80 - 7.54 (m, 2H), 7.10 (s, 1H), 5.44 - 5.39 (m, 1H), 3.85 - 3.80 (m, 1H), 3.78 - 3.69 (m, 1H), 3.47 - 3.40 (m, 4H), 3.28 - 3.26 (m, 2H), 2.78 - 2.66 (m, 1H), 2.33 - 2.24 (m, 1H), 2.14 - 1.94 (m, 2H), 1.40 - 1.35 (m, 2H), 1.26 - 1.22 (m, 2H), 1.18 (d, *J* = 6.0 Hz, 6H). |
| | | **MS (ESI) M/Z:** 579.0 [M+H]⁺. |
| 077K | 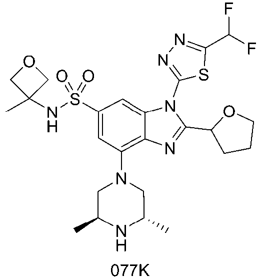 | **¹H NMR** (400 MHz, DMSO-*d₆*) δ 8.33 (s, 1H), 7.80 - 7.53 (m, 2H), 7.09 (s, 1H), 5.42 - 5.37 (m, 1H), 4.53 - 4.50 (m, 2H), 4.08 - 4.06 (m, 2H), 3.84 - 3.79 (m, 1H), 3.78 - 3.68 (m, 1H), 3.45 - 3.40 (m, 4H), 3.29 - 3.25 (m, 2H), 2.76 - 2.66 (m, 1H), 2.32 - 2.23 (m, 1H), 2.09 - 1.97 (m, 2H), 1.40 (s, 3H), 1.17 (d, *J* = 6.0 Hz, 6H). |
| | | **MS (ESI) M/Z:** 584.1 [M+H]⁺. |
| 077N | 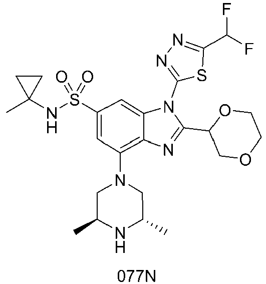 | **¹H NMR** (400 MHz, DMSO-*d₆*) δ 8.07 (s, 1H), 7.79 (s, 1H), 7.68 (t, *J* = 53.2 Hz, 1H), 7.10 (s, 1H), 5.07 - 5.04 (m, 1H), 4.19 - 4.14 (m, 2H), 3.78 - 3.75 (m, 3H), 3.66 - 3.65 (m, 1H), 3.42 - 3.39 (m, 4H), 3.28 - 3.25 (m, 2H), 2.17 (s, 1H), 1.17 (d, *J* = 6.4 Hz, 6H), 1.02 (s, 3H), 0.65 - 0.58 (m, 2H), 0.36 - 0.35 (m, 2H). |
| | | **MS (ESI) M/Z:** 584.4 [M+H]⁺. |
| 078A | 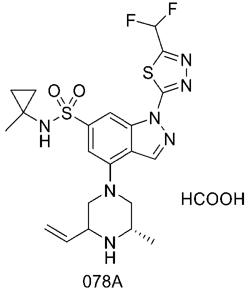 | **¹H NMR** (400 MHz, DMSO-*d₆*) δ 8.92 (s, 1H), 8.45 (s, 1H), 8.33 (s, 1H), 7.74 - 7.47 (m, 1H), 7.15 (d, *J* = 0.8 Hz, 1H), 5.95 - 5.87 (m, 1H), 5.39 (d, *J* = 17.6 Hz, 1H), 5.19 (d, *J* = 10.8 Hz, 1H), 3.72 - 3.69 (m, 2H), 3.62 - 3.58 (m, 1H), 3.14 - 3.10 (m, 1H), 2.69 - 2.55 (m, 2H), 1.13 (d,*J* = 6.0 Hz, 3H), 1.07 (s, 3H), 0.67 - 0.64 (m, 2H), 0.41 - 0.39 (m, 2H). |
| | | **MS (ESI) M/Z:** 510.1 [M+H]⁺. |
| 078B | 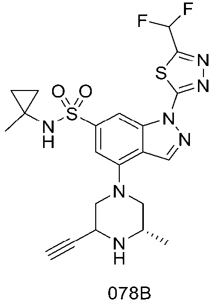 | **¹H NMR** (400 MHz, DMSO-*d₆*) δ 8.92 (s, 1H), 8.47 (s, 1H), 8.34 (s, 1H), 7.60 (t, *J* = 53.2 Hz, 1H), 7.15 (s, 1H), 3.83 - 3.74 (m, 2H), 3.66 (d, *J* = 11.2 Hz, 1H), 3.31 (d,*J* = 2.4 Hz, 1H), 2.99 - 2.96 (m, 1H), 2.87 (t, *J* = 10.8 Hz, 1H), 2.57 (t, *J* = 11.2 Hz, 1H), 1.07 (d, *J* = 6.0 Hz, 3H), 1.06 (s, 3H), 0.69 - 0.63 (m, 2H), 0.42 - 0.40 (m, 2H). |
| | | **MS (ESI) M/Z:** 508.1 [M+H]⁺. |
| 078C | 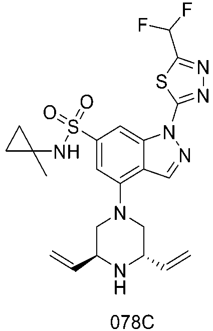 | **¹H NMR** (400 MHz, CD₃OD) δ 8.65 (s, 1H), 8.51 - 8.47 (m, 2H), 7.37 - 7.10 (m, 2H), 6.24 - 6.18 (m, 2H), 5.42 - 5.38 (m, 2H), 5.30 - 5.27 (m, 2H), 3.89 - 3.87 (m, 2H), 3.54 - 3.50 (m, 2H), 3.38 - 3.35 (m, 2H), 1.15 (s, 3H), 0.77 - 0.75 (m, 2H), 0.46 - 0.43 (m, 2H). |
| | | **¹⁹F NMR** (377 MHz, CD₃OD) δ -110.87. |
| | | **MS (ESI) M/Z:** 522.6 [M+H]⁺. |
| 021B | 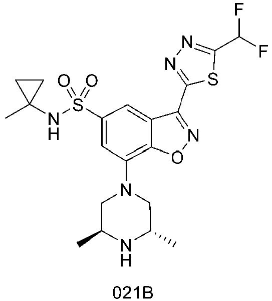 | **¹H NMR** (400 MHz, DMSO-*d₆*+D₂O) δ 8.22 (s, 1H), 7.69 (t, *J* = 53.2 Hz, 1H), 7.40 (s, 1H), 3.38 - 3.35 (m, 2H), 3.29 - 3.25 (m, 2H), 3.16 - 3.11 (m, 2H), 1.16 (d, *J* = 6.4 Hz, 6H), 1.03 (s, 3H), 0.66 - 0.59 (m, 2H), 0.42 - 0.37 (m, 2H). |
| | | **MS (ESI) M/Z:** 499.3 [M+H]⁺. |
| 023 | 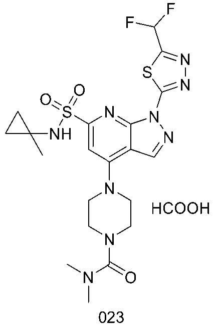 | **¹H NMR** (400 MHz, DMSO-*d₆*) δ 9.72 (s, 1H), 8.47 (s, 1H), 8.31 (s, 1H), 7.63 (t, *J* = 53.0 Hz, 1H), 6.78 (s, 1H), 3.89 - 3.79 (m, 4H), 3.59 - 3.39 (m, 4H), 2.80 (s, 6H), 1.16 (s, 3H), 0.78 - 0.70 (m, 2H), 0.47 - 0.36 (m, 2H). |
| | | **MS (ESI) M/Z:** 542.2 [M+H]⁺. |
| 0650 | 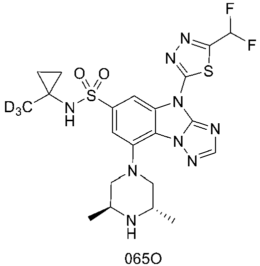 | **¹H NMR** (400 MHz, DMSO-*d₆*) δ 8.72 (s, 1H), 8.48 (s, 1H), 8.32 (s,1H), 7.68 (t, *J* = 52.8 Hz, 1H), 7.47 (s, 1H), 3.41 - 3.37 (m, 2H), 3.30 - 3.31 (m, 2H), 2.89 - 2.86 (m, 2H), 1.18 (d, *J* = 6.4 Hz, 6H), 0.66 - 0.64 (m, 2H), 0.41 - 0.39 (m, 2H). |
| | | **MS (ESI) M/Z:** 541.3 [M+H]⁺. |
| 065R | 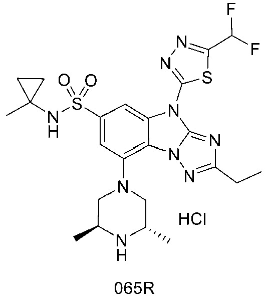 | **¹H NMR** (400 MHz, DMSO-*d₆*+D₂O) δ 8.74 (s, 1H), 7.72 - 7.46 (m, 2H), 3.86 - 3.81 (m, 2H), 3.52 - 3.49 (m, 2H), 3.26 - 3.22 (m, 2H), 2.89 (q, *J =* 7.6 Hz, 2H), 1.43 (d, *J* = 6.4 Hz, 6H), 1.35 (t, *J =* 7.6 Hz, 3H), 1.06 (s, 3H), 0.65 - 0.59 (m, 2H), 0.41 - 0.38 (m, 2H). |
| | | **MS (ESI) M/Z:** 566.3 [M+H]⁺. |
| 065S | 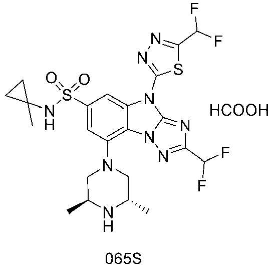 | **¹H NMR** (400 MHz, DMSO-*d₆*) δ 8.73 (d, *J* = 1.4 Hz, 1H), 8.38 (s, 1H), 8.31 (s, 1H), 7.68 (t, *J* = 53.0 Hz, 1H), 7.51 (d, *J* = 1.6 Hz, 1H), 7.34 (t, *J* = 52.6 Hz, 1H), 3.41 - 3.25 (m, 4H), 3.01 - 2.92 (m, 2H), 1.24 (d, *J* = 6.4 Hz, 6H), 1.10 (s, 3H), 0.66 (q, *J* = 4.2 Hz, 2H), 0.46 - 0.38 (m, 2H). |
| | | **MS (ESI) M/Z:** 588.1 [M+H]⁺. |
| 065T | 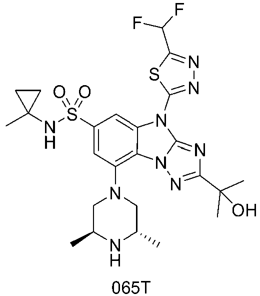 | **¹H NMR** (400 MHz, DMSO-*d₆*) δ 8.76 (d, *J* = 1.4 Hz, 1H), 8.42 (s, 1H), 7.68 (t, *J* = 53.1 Hz, 1H), 7.54 (d, *J* = 1.6 Hz, 1H), 3.65 - 3.04 (m, 6H), 1.26 - 1.21 (m, 12H), 1.11 (s, 3H), 0.67 (q, *J* = 3.3 Hz, 2H), 0.54 - 0.30 (m, 2H). |
| | | **MS (ESI) M/Z:** 596.2 [M+H]⁺. |
| 065U | 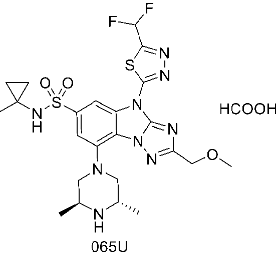 | **¹H NMR** (400 MHz, DMSO-*d₆*+D₂O) δ 8.73 (d, *J* = 1.5 Hz, 1H), 8.30 (s, 1H), 7.66 (t, *J* = 53.1 Hz, 1H), 7.49 (d, *J* = 1.6 Hz, 1H), 4.62 (d, *J* = 1.3 Hz, 2H), 3.54 - 3.46 (m, 2H), 3.41 (s, 3H), 3.37 - 3.30 (m, 2H), 3.01 (dd, *J* = 11.2, 6.0 Hz, 2H), 1.27 (d, *J* = 6.5 Hz, 6H), 1.09 (s, 3H), 0.66 (d, *J* = 4.8 Hz, 2H), 0.49 - 0.37 (m, 2H). |
| | | **MS (ESI) M/Z:** 582.2 [M+H]⁺. |
| 065W | 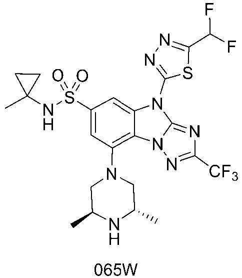 | **¹H NMR** (400 MHz, DMSO-*d₆*) δ 8.76 (s, 1H), 8.29 (s, 1H), 7.97 - 7.36 (m, 2H), 3.61 - 3.55 (m, 2H), 3.35 - 3.33 (m, 2H), 3.12 - 3.05 (m, 2H), 1.31 (s, 6H), 1.10 (s, 3H), 0.69 - 0.63 (m, 2H), 0.46 - 0.39 (m, 2H). |
| | | **MS (ESI) M/Z:** 606.0 [M+H]⁺. |
| 065X | 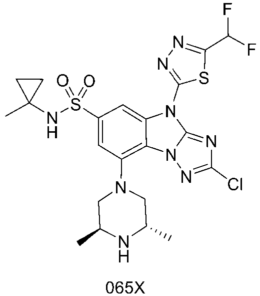 | **¹H NMR** (400 MHz, DMSO-*d₆*+D₂O) δ 8.72 (d, *J* = 1.4 Hz, 1H), 7.65 (t, *J* = 53.1 Hz, 1H), 7.50 (d, *J* = 1.6 Hz, 1H), 3.42 - 3.38 (m, 2H), 3.28 (dd, *J* = 11.1, 3.2 Hz, 2H), 2.93 (dd, *J* = 11.1, 6.0 Hz, 2H), 1.22 (d, *J* = 6.5 Hz, 6H), 1.09 (s, 3H), 0.67 - 0.65 (m, 2H), 0.56 - 0.31 (m, 2H). |
| | | **MS (ESI) M/Z:** 572.0 [M+H]⁺. |
| 065Z | 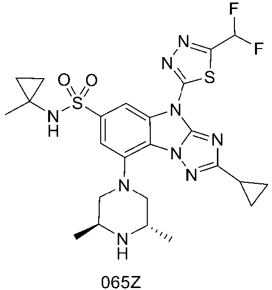 | **¹H NMR** (400 MHz, DMSO-*d₆*) δ 8.67 (d, *J* = 1.6 Hz, 1H), 8.29 (s, 1H), 7.66 (t, *J* = 53.2 Hz, 1H), 7.42 (d, *J* = 1.6 Hz, 1H), 3.39 - 3.37 (m, 2H), 3.26 - 3.23 (m, 2H), 2.92 - 2.88 (m, 2H), 2.20 - 2.18 (m, 1H), 1.20 (d, *J* = 6.4 Hz, 6H), 1.12 - 1.09 (m, 2H), 1.07 (s, 3H), 1.03 - 0.99 (m, 2H), 0.66 - 0.63 (m, 2H), 0.41 - 0.39 (m, 2H). |
| | | **MS (ESI) M/Z:** 578.4 [M+H]⁺. |
| 074G | 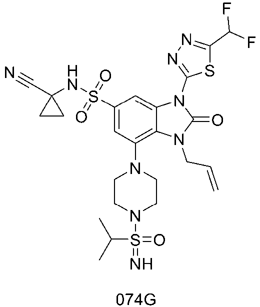 | **¹H NMR** (400 MHz, CHLOROFORM-d) δ ppm 8.98 (s, 1H), 7.89 (s, 1H), 7.06 (t, *J* = 54.00 Hz, 1H), 6.07 (br s, 1H), 5.91 - 6.03 (m, 1H), 5.24 (d, *J* = 10.61 Hz, 1H), 5.12 (d, *J* = 17.05 Hz, 1H), 5.02 (br d, *J =* 4.77 Hz, 2H), 3.94 - 4.06 (m, 2H), 3.28 - 3.39 (m, 1H), 3.00 - 3.21 (m, 6H), 1.67 - 1.70 (m, 2H), 1.40 - 1.47 (m, 8H). |
| | | **MS (ESI) M/Z:** 642.3 [M+H]⁺. |
| 074M | 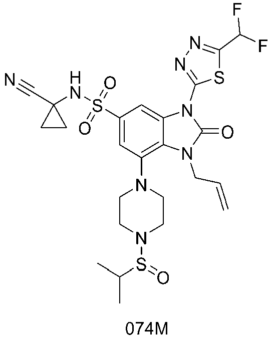 | **¹H NMR** (400 MHz, CHLOROFORM-d) δ ppm 8.97 (d, *J* = 1.67 Hz, 1H), 7.89 (d, *J* = 1.67 Hz, 1H), 7.05 (t, *J =* 53.60 Hz, 1H), 6.17 (s, 1H), 5.92 - 6.04 (m, 1H), 5.22 (d, *J =* 10.25 Hz, 1H), 5.08 (d, *J* = 16.81 Hz, 1H), 5.00 - 5.05 (m, 2H), 3.33 - 3.51 (m, 3H), 3.08 - 3.24 (m, 5H), 2.94 - 3.04 (m, 1H), 1.63 - 1.68 (m, 2H), 1.42 - 1.46 (m, 2H), 1.38 (d, *J* = 6.91 Hz, 3H), 1.23 (d, *J* = 6.91 Hz, 3H). |
| | | **MS (ESI) M/Z:** 627.3 [M+H]⁺. |
| 077B1 | 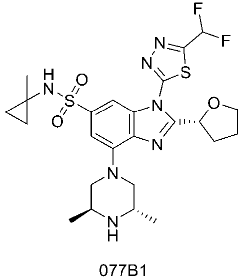 | **¹H NMR** (400 MHz, DMSO-*d₆*+D₂O) δ 7.62 - 7.35 (m, 2H), 7.07 (s, 1H), 5.34 - 5.31 (m, 1H), 3.78 - 3.76 (m, 1H), 3.68 - 3.65 (m, 1H), 3.50 - 3.48 (m, 4H), 3.41 - 3.38 (m, 2H), 2.67 - 2.65 (m, 1H), 2.29 - 2.26 (m, 1H), 2.01 - 1.95 (m, 2H), 1.21 (d, *J* = 6.4 Hz, 6H), 0.98 (s, 3H), 0.60 - 0.54 (m, 2H), 0.36 - 0.34 (m, 2H). |
| | | **MS (ESI) M/Z:** 568.4 [M+H]⁺. |
| 077B2 | 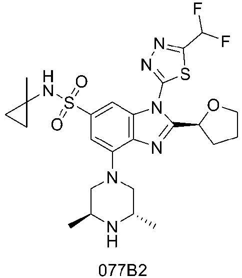 | **¹H NMR** (400 MHz, DMSO-*d₆*+D₂O) δ 7.63 (s, 1H), 7.47 (t, *J* = 52.8 Hz, 1H), 7.09 (s, 1H), 5.33 - 5.29 (m, 1H), 3.78 - 3.75 (m, 1H), 3.72 - 3.68 (m, 1H), 3.54 - 3.50 (m, 6H), 2.62 - 2.59 (m, 1H), 2.28 - 2.26 (m, 1H), 2.02 - 1.95 (m, 2H), 1.26 (d, *J* = 6.4 Hz, 6H), 0.97 (s, 3H), 0.59 - 0.53 (m, 2H), 0.36 - 0.32 (m, 2H). |
| | | **MS (ESI) M/Z:** 568.4 [M+H]⁺. |
| 077X | 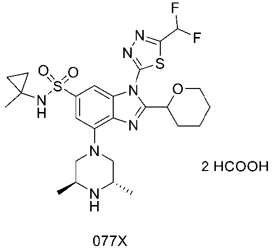 | **¹H NMR** (400 MHz, DMSO-*d₆*+D₂O) δ 8.38 (s, 2H), 7.85 (d, *J* = 3.8 Hz, 1H), 7.64 (t, *J* = 53.1 Hz, 2H), 7.15 (s, 1H), 4.87 (d, *J* = 9.9 Hz, 1H), 3.90 - 3.79 (m, 1H), 3.60 - 3.47 (m, 7H), 2.22 - 1.90 (m, 3H), 1.73 - 1.50 (m, 3H), 1.30 (d, *J* = 6.2 Hz, 6H), 1.03 (s, 3H), 0.70 - 0.56 (m, 2H), 0.38 (s, 2H). |
| | | **MS (ESI) M/Z:** 582.2 [M+H]⁺. |
| 077Y | 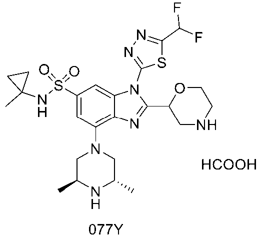 | **¹H NMR** (400 MHz, DMSO-*d₆*) δ 8.22 (s, 1H), 8.10 (s, 1H), 7.86 (d, *J* = 2.2 Hz, 1H), 7.69 (t, *J* = 53.0 Hz, 1H), 7.18 - 7.14 (m, 1H), 4.92 - 4.85 (m, 1H), 3.80 - 3.51 (m, 8H), 3.35 - 3.21 (m, 2H), 2.83 - 2.71 (m, 2H), 1.33 (d,*J* = 5.4 Hz, 6H), 1.03 (s, 3H), 0.69 - 0.56 (m, 2H), 0.42 - 0.31 (m, 2H). |
| | | **MS (ESI) M/Z:** 583.2 [M+H]⁺. |
| 079A | 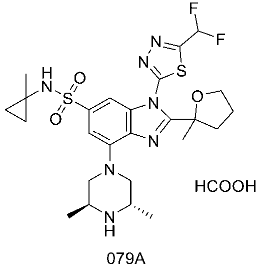 | **¹H NMR** (400 MHz, DMSO-*d₆*) δ 8.01 (s, 1H), 7.80 - 7.53 (m, 1H), 7.09 - 7.04 (m, 2H), 3.76 - 3.71 (m, 1H), 3.60 - 3.47 (m, 4H), 3.45 - 3.35 (m, 3H), 2.93 - 2.81 (m, 1H), 2.04 - 1.91 (m, 2H), 1.89 - 1.76 (m, 1H), 1.64 (d, *J* = 6.0 Hz, 3H), 1.26 (d, *J* = 6.4 Hz, 6H), 1.03 (s, 3H), 0.64 - 0.57 (m, 2H), 0.39 - 0.31 (m, 2H). |
| | | **¹⁹F NMR** (376 MHz, DMSO-*d₆*) δ -110.85. |
| | | **MS (ESI) M/Z:** 581.8 [M+H]⁺. |
| 079C | 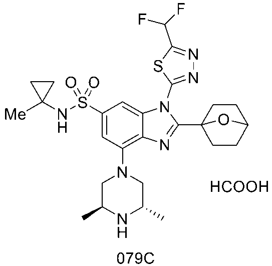 | **¹H NMR** (400 MHz, DMSO-*d₆*) δ 8.85 (s, 1H), 8.05 (s, 1H), 7.67 (t, *J* = 56.0 Hz, 1H), 7.40 (d, *J* = 1.4 Hz, 1H), 7.17 (d, *J* = 1.5 Hz, 1H), 4.68 (t, *J* = 4.6 Hz, 1H), 3.80 - 3.76 (m, 4H), 3.60 - 3.66 (d, *J* = 11.9 Hz, 2H), 2.14 - 2.05 (m, 4H), 1.72 - 1.66 (m, 4H), 1.42 (d, *J* = 6.1 Hz, 6H), 1.03 (s, 3H), 0.65 - 0.58 (m, 2H), 0.42 - 0.35 (m, 2H). |
| | | **MS (ESI) M/Z:** 594.2 [M+H]⁺. |
| 079F | 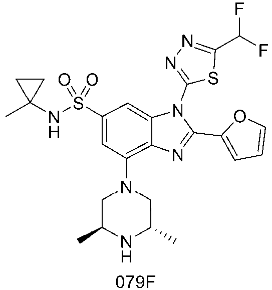 | **¹H NMR** (400 MHz, DMSO+D₂O-*d₆*) δ 7.85 (s, 1H), 7.59 (t, *J* = 53.6 Hz, 1H), 7.32 (d, *J* = 2.0 Hz, 1H), 7.08 (s, 1H), 7.02 (d, *J* = 3.2 Hz, 1H), 6.72 - 6.70 (m, 1H), 3.56 - 3.52 (m, 2H), 3.42 - 3.37 (m, 2H), 3.28 - 3.23 (m, 2H), 1.16 (d, *J* = 6.4 Hz, 6H), 1.01 (s, 3H), 0.62 - 0.56 (m, 2H), 0.36 - 0.33 (m, 2H). |
| | | **MS (ESI) M/Z:** 564.1 [M+H]⁺. |
| 079J | 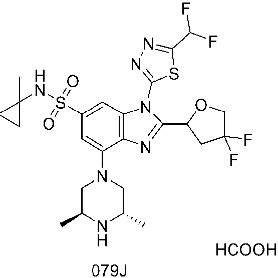 | **¹H NMR** (400 MHz, DMSO-*d₆*) δ 8.10 (s, 1H), 7.82 -7.55 (m, 2H), 7.15 (s, 1H), 5.86 - 5.81 (m, 1H), 4.19 - 3.92 (m, 2H), 3.69 - 3.50 (m, 6H), 3.07 - 2.88 (m, 2H), 1.38 - 1.20 (m, 6H), 1.04 (s, 3H), 0.70 - 0.56 (m, 2H), 0.43 - 0.31 (m, 2H). |
| | | **¹⁹F NMR** (376 MHz, DMSO-*d₆*) δ -96.95 (dd, *J =* 228.61 Hz, 20.68 Hz, 1 F), -101.50 (dd, *J* = 228.61 Hz, 34.59 Hz, 1 F), -110.58. |
| | | **MS (ESI) M/Z:** 604.1 [M+H]⁺. |
| 079J1 | 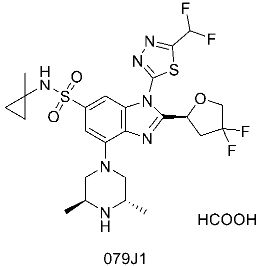 | **¹H NMR** (400 MHz, DMSO-*d₆*) δ 8.26 (s, 1H), 8.10 (s, 1H), 7.82 - 7.55 (m, 2H), 7.67 (d, *J* = 1.6 Hz, 1H), 7.14 (d, *J* = 1.6 Hz, 1H), 5.84 - 5.80 (m, 1H), 4.17 - 3.98 (m, 2H), 3.54 - 3.47 (m, 6H), 3.05 - 2.89 (m, 2H), 1.24 (d, *J* = 6.4 Hz, 6H), 1.04 (s, 3H), 0.66 - 0.59 (m, 2H), 0.42 - 0.31 (m, 2H). |
| | | **¹⁹F NMR** (376 MHz, DMSO-*d₆*) δ -96.68, -97.29, -101.19, -101.80, -110.59. |
| | | **MS (ESI) M/Z:** 604.1 [M+H]⁺. |
| 079J2 | 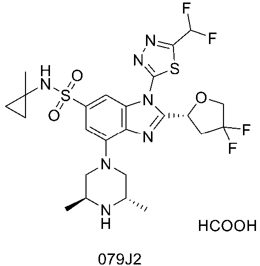 | **¹H NMR** (400 MHz, DMSO-*d₆*) δ 8.32 (s, 1H), 8.10 (s, 1H), 7.82 - 7.55 (m, 2H), 7.67 (s, 1H), 7.14 (s, 1H), 5.86 - 5.83 (m, 1H), 4.14 - 3.92 (m, 2H), 3.55 - 3.48 (m, 6H), 3.06 - 2.88 (m, 2H), 1.24 (d, *J* = 6.4 Hz, 6H), 1.04 (s, 3H), 0.68 - 0.57 (m, 2H), 0.42 - 0.31 (m, 2H). |
| | | **¹⁹F NMR** (376 MHz, DMSO-*d₆*) δ -96.61, -97.22, -101.29, -101.90, -110.59. |
| | | **MS (ESI) M/Z:** 604.1 [M+H]⁺. |
| 080B | 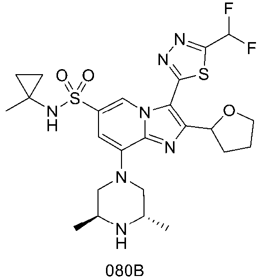 | **¹H NMR** (400 MHz, DMSO-*d₆*) δ 9.65 (s, 1H), 8.35 (s, 1H), 7.70 (t, *J* = 53.2 Hz, 1H), 6.94 (s, 1H), 5.34 - 5.29 (m, 1H), 3.93 - 3.86 (m, 2H), 3.51 - 3.47 (m, 2H), 3.36 - 3.35 (m, 2H), 3.28 - 3.26 (m, 2H), 2.65 - 2.56 (m, 1H), 2.30 - 2.23 (m, 1H), 2.17 - 2.01 (m, 2H), 1.18 (d, *J* = 6.4 Hz, 6H), 1.12 (s, 3H), 0.74 - 0.66 (m, 2H), 0.46 - 0.39 (m, 2H). |
| | | **MS (ESI) M/Z:** 568.4 [M+H]⁺. |
| 080B2 | 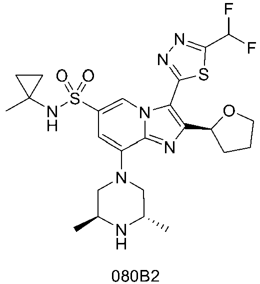 | **¹H NMR** (400 MHz, DMSO-*d₆*) δ 9.65 (d, *J* = 1.2 Hz, 1H), 8.35 (s, 1H), 7.70 (t, *J* = 53.6 Hz, 1H), 6.94 (s, 1H), 5.30 (t, *J* = 6.4 Hz, 1H), 3.95 - 3.85 (m, 2H), 3.50 - 3.46 (m, 2H), 3.36 - 3.32 (m, 2H), 3.28 - 3.26 (m, 2H), 2.65 - 2.56 (m, 1H), 2.32 - 2.23 (m, 1H), 2.17 - 2.02 (m, 2H), 1.19 (d, *J* = 6.4 Hz, 6H), 1.12 (s, 3H), 0.74 - 0.66 (m, 2H), 0.47 - 0.40 (m, 2H). |
| | | **MS (ESI) M/Z:** 568.1 [M+H]⁺. |
| 080B1 | 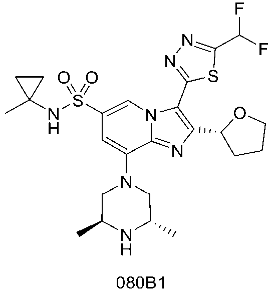 | **¹H NMR** (400 MHz, DMSO-*d₆*) δ 9.64 (d, *J* = 1.6 Hz, 1H), 8.34 (s, 1H), 7.70 (t, *J* = 53.2 Hz, 1H), 6.94 (d, *J* = 1.2 Hz, 1H), 5.34 - 5.31 (m, 1H), 3.88 (t, *J* = 6.8 Hz, 2H), 3.50 - 3.46 (m, 2H), 3.37 - 3.34 (m, 2H), 3.28 - 3.24 (m, 2H), 2.67 - 2.61 (m, 1H), 2.30 - 2.24 (m, 1H), 2.15 - 2.03 (m, 2H), 1.19 (d, *J* = 6.4 Hz, 6H), 1.12 (s, 3H), 0.74 - 0.66 (m, 2H), 0.46 - 0.40 (m, 2H). |
| | | **MS (ESI) M/Z:** 568.0 [M+H]⁺. |
| 080H | 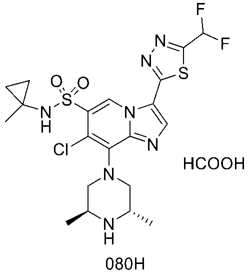 | **¹H NMR** (400 MHz, DMSO-*d₆*) δ 10.01 (s, 1H), 8.69 (s, 2H), 8.30 (s, 1H), 7.71 (t, *J* = 53.2 Hz, 1H), 3.59 - 3.40 (m, 4H), 3.14 (dd, *J* = 12.0, 5.9 Hz, 2H), 1.23 (d, *J* = 6.4 Hz, 6H), 1.22 (s, 3H), 0.81 - 0.69 (m, 2H), 0.52 - 0.41 (m, 2H). |
| | | **MS (ESI) M/Z:** 532.2 [M+H]⁺. |
| 080J | 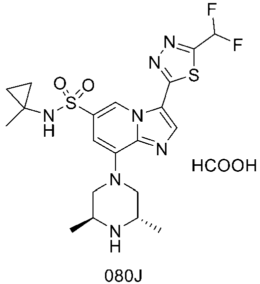 | **¹H NMR** (400 MHz, DMSO-*d₆*) δ 9.62 (d, *J* = 1.5 Hz, 1H), 8.60 (s, 1H), 8.41 (s, 1H), 8.26 (s, 1H), 7.70 (t, *J* = 53.2 Hz, 1H), 6.97 (d, *J* = 1.6 Hz, 1H), 3.59 - 3.35 (m, 6H), 1.22 *(d, J =* 6.3 Hz, 6H), 1.13 (s, 3H), 0.75 - 0.65 (m, 2H), 0.48 - 0.40 (m, 2H). |
| | | **MS (ESI) M/Z:** 498.2 [M+H]⁺. |
| 080K | 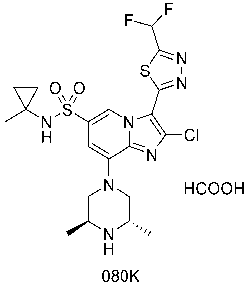 | **¹H NMR** (400 MHz, DMSO-*d₆*+D₂O) δ 9.81 (d, *J* = 1.5 Hz, 1H), 8.34 (s, 2H), 7.73 (t, *J* = 53.2 Hz, 1H), 7.08 (d, *J* = 1.6 Hz, 1H), 3.53 - 3.30 (m, 6H), 1.19 (d, *J* = 6.0 Hz, 6H), 1.13 (s, 3H), 0.77 - 0.66 (m, 2H), 0.52 - 0.34 (m, 2H). |
| | | **MS (ESI) M/Z:** 532.0 [M+H]⁺. |
| 082B | 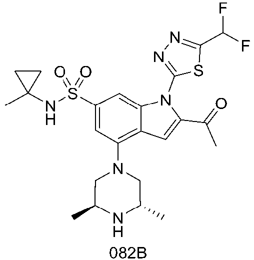 | **¹H NMR** (400 MHz, DMSO-*d₆*) δ 8.12 (s, 1H), 7.79 - 7.52 (m, 2H), 7.26 (s, 1H), 7.06 (s, 1H), 3.38 - 3.33 (m, 2H), 3.27 - 3.24 (m, 2H), 3.03 - 2.93 (m, 2H), 2.65 (s, 3H), 1.22 (d, *J* = 6.4 Hz, 6H), 1.00 (s, 3H), 0.62 - 0.56 (m, 2H), 0.36 - 0.33 (m, 2H). |
| | | **MS (ESI) M/Z:** 539.0 [M+H]⁺. |
| 082C | 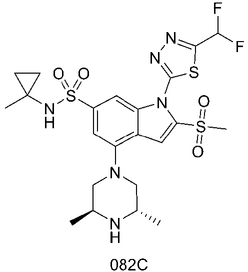 | **¹H NMR** (400 MHz, DMSO-*d₆*+D₂O) δ 7.76 - 7.52 (m, 2H), 7.32 (s, 1H), 7.10 (s, 1H), 3.52 (s, 3H), 3.30 - 3.25 (m, 2H), 3.22 - 3.19 (m, 2H), 3.00 - 2.91 (m, 2H), 1.20 (d, *J* = 6.4 Hz, 6H), 1.00 (s, 3H), 0.61 - 0.55 (m, 2H), 0.37 - 0.35 (m, 2H). |
| | | **MS (ESI) M/Z:** 575.0 [M+H]⁺. |
| 083A | 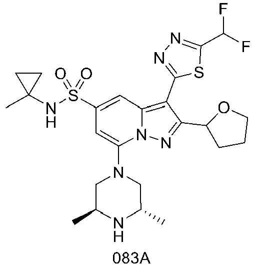 | **¹H NMR** (400 MHz, DMSO-*d₆*) δ 8.48 (d, *J* = 1.6 Hz, 1H), 8.45 (s, 1H), 7.65 (t, *J* = 53.2 Hz, 1H), 6.80 (s, 1H), 5.43 - 5.38 (m, 1H), 3.92 - 3.85 (m, 2H), 3.37 - 3.23 (m, 6H), 2.62 - 2.60 (m, 1H), 2.33 - 2.31 (m, 1H), 2.11 - 2.05 (m, 2H), 1.26 (d, *J* = 4.2 Hz, 6H), 1.11 (s, 3H), 0.74 - 0.65 (m, 2H), 0.48 - 0.44 (m, 2H). |
| | | **MS (ESI) M/Z:** 568.1 [M+H]⁺. |
| 083A1 | 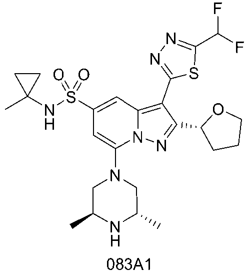 | **¹H NMR** (400 MHz, DMSO-*d₆*) δ 8.48 (d, *J* = 1.6 Hz, 1H), 8.45 (s, 1H), 7.65 (t, *J* = 53.2 Hz, 1H), 6.80 (s, 1H), 5.42 (t, *J* = 6.4 Hz, 1H), 3.93 - 3.85 (m, 2H), 3.42 - 3.40 (m, 4H), 3.28 - 3.25 (m, 2H), 2.64 - 2.62 (m, 1H), 2.35 - 2.29 (m, 1H), 2.09 - 2.05 (m, 2H), 1.27 (d, *J* = 5.6 Hz, 6H), 1.11 (s, 3H), 0.74 - 0.65 (m, 2H), 0.48 - 0.44 (m, 2H). |
| | | **MS (ESI) M/Z:** 568.1 [M+H]⁺. |
| 083A2 | 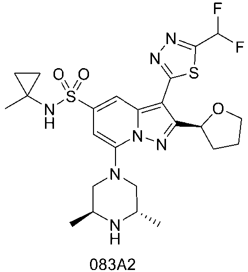 | **¹H NMR** (400 MHz, DMSO-*d₆*) δ 8.49 (d, *J* = 1.6 Hz, 1H), 8.45 (s, 1H), 7.66 (t, *J* = 53.2 Hz, 1H), 6.81 (s, 1H), 5.40 (t, *J* = 6.4 Hz, 1H), 3.93 - 3.89 (m, 2H), 3.46 - 3.40 (m, 4H), 3.28 - 3.25 (m, 2H), 2.59 - 2.50 (m, 1H), 2.33 - 2.30 (m, 1H), 2.11 - 2.04 (m, 2H), 1.29 (d, *J* = 5.2 Hz, 6H), 1.11 (s, 3H), 0.75 - 0.66 (m, 2H), 0.48 - 0.44 (m, 2H). |
| | | **MS (ESI) M/Z:** 568.1 [M+H]⁺. |
| 084H | 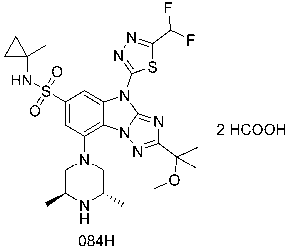 | **¹H NMR** (400 MHz, DMSO-*d₆*+D₂O) δ 8.71 (d, *J* = 1.5 Hz, 1H), 8.41 (s, 2H), 7.66 (t, *J* = 53.0 Hz, 1H), 7.46 (d, *J* = 1.6 Hz, 1H), 3.55 - 3.27 (m, 4H), 3.11 (s, 3H), 2.92 (dd, *J* = 11.1, 5.9 Hz, 2H), 1.66 (s, 6H), 1.23 (d, *J* = 6.4 Hz, 6H), 1.08 (s, 3H), 0.69 - 0.62 (m, 2H), 0.43 - 0.37 (m, 2H). |
| | | **MS (ESI) M/Z:** 610.2 [M+H]⁺. |
| 084J | 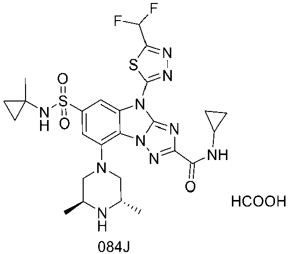 | **¹H NMR** (400 MHz, DMSO-*d₆*) δ 8.93 (s, 1H), 8.78 (d, *J* = 1.4 Hz, 1H), 8.72 (d, *J* = 4.3 Hz, 1H), 8.42 (s, 1H), 7.71 (t, *J* = 52.0 Hz, 1H), 7.55 (d, *J* = 1.5 Hz, 1H), 3.99 - 3.87 (m, 2H), 3.57 - 3.47 (m, 2H), 3.41 - 3.30 (m, 2H), 2.90 (td, *J =* 7.3, 3.7 Hz, 1H), 1.49 (d, *J* = 6.6 Hz, 6H), 1.11 (s, 3H), 0.82 - 3.62 (m, 6H), 0.47 - 0.39 (m, 2H). |
| | | **MS (ESI) M/Z:** 621.2 [M+H]⁺. |
| 084K | 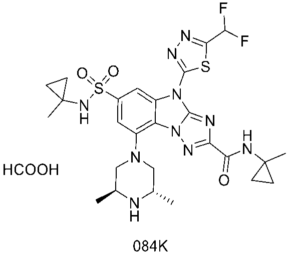 | **¹H NMR** (400 MHz, DMSO-*d₆*) δ 8.92 (s, 1H), 8.78 (d, *J* = 1.4 Hz, 1H), 8.42 (s, 1H), 7.72 (t, *J=* 53.0 Hz, 1H), 7.55 (d, *J* = 1.6 Hz, 1H), 3.96 - 3.86 (m, 2H), 3.56 - 3.46 (m, 2H), 3.34 - 3.38 (m, 2H), 1.48 (d, *J* = 6.6 Hz, 6H), 1.42 (s, 3H), 1.24 (s, 1H), 1.12 (s, 3H), 0.86 - 0.80 (m, 2H), 0.73 - 0.64 (m, 4H), 0.46 - 0.40 (m, 2H). |
| | | **MS (ESI) M/Z:** 635.2 [M+H]⁺. |
| 084L | 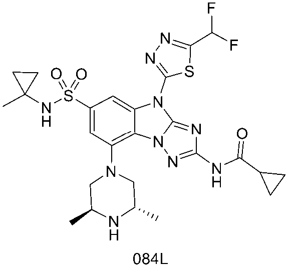 | **¹H NMR** (400 MHz, DMSO-*d₆*) δ 11.11 (s, 1H), 8.68 (s, 1H), 8.29 (s, 1H), 7.69 (t, *J* = 53.1 Hz, 1H), 7.43 (s, 1H), 3.41 - 3.35 (m, 4H), 2.91 - 2.87 (m, 2H), 2.01 - 1.99 (m, 1H), 1.19 (d, *J* = 6.5 Hz, 6H), 1.09 (s, 3H), 0.87 - 0.85 (m, 4H), 0.67 - 0.64 (m, 2H), 0.41 - 0.39 (m, 2H). |
| | | **MS (ESI) M/Z:** 621.2 [M+H]⁺. |
| 084M | 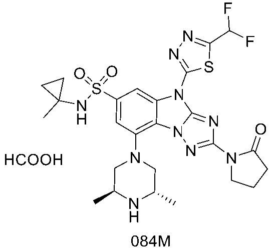 | **¹H NMR** (400 MHz, DMSO-*d₆*) δ 8.70 (d, *J* = 1.5 Hz, 1H), 8.32 (s, 1H), 8.29 (s, 1H), 7.68 (t, *J =* 53.1 Hz, 1H), 7.45 (d, *J* = 1.6 Hz, 1H), 4.07 - 3.95 (m, 2H), 3.51 - 3.47 (m, 2H), 3.38 - 3.36 (m, 2H), 2.96 (dd, *J =* 11.2, 5.9 Hz, 2H), 2.60 - 2.50 (m, 2H), 2.17 (p, *J =* 7.6 Hz, 2H), 1.26 (d, *J* = 6.4 Hz, 6H), 1.10 (s, 3H), 0.66 (q, *J =* 4.4 Hz, 2H), 0.45 - 0.33 (m, 2H). |
| | | **MS (ESI) M/Z:** 621.2 [M+H]⁺. |
| 065Y | 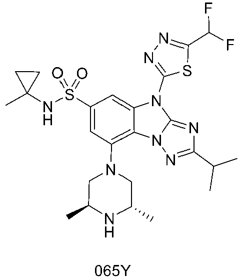 | **¹H NMR** (400 MHz, DMSO-*d₆*+D₂O) δ 8.68 (d, *J* = 1.2 Hz, 1H), 7.70 - 7.43 (m, 2H), 3.49 - 3.47 (m, 2H), 3.36 - 3.33 (m, 2H), 3.20 - 3.14 (m, 1H), 2.99 - 2.96 (m, 2H), 1.37 (d, *J =* 6.8 Hz, 6H), 1.25 (d, *J* = 6.4 Hz, 6H), 1.05 (s, 3H), 0.64 - 0.61 (m, 2H), 0.41 - 0.38 (m, 2H). |
| | | **MS (ESI) M/Z:** 580.3 [M+H]⁺. |
| 079M | 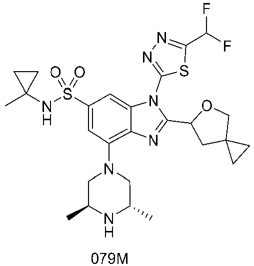 | **¹H NMR** (400 MHz, DMSO-*d₆*) δ 8.06 (s, 1H), 7.81 - 7.54 (m, 2H), 7.09 (s, 1H), 5.59 - 5.55 (m, 1H), 3.67 - 3.60 (m, 2H), 3.50 - 3.35 (m, 4H), 3.29 - 3.26 (m, 2H), 2.82 - 2.75 (m, 1H), 2.35 - 2.32 (m, 1H), 1.17 (d, *J* = 6.4 Hz, 6H), 1.03 (s, 3H), 0.73 - 0.70 (m, 2H), 0.65 - 0.62 (m, 4H), 0.37 - 0.34 (m, 2H). |
| | | **MS (ESI) M/Z:** 594.4 [M+H]⁺. |
| 079M1 | 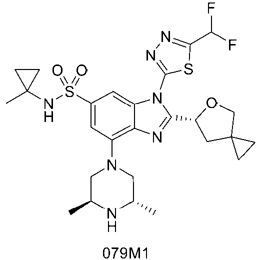 | **¹H NMR** (400 MHz, DMSO-*d₆*) δ 8.06 (s, 1H), 7.81 - 7.54 (m, 2H), 7.09 (d, *J* = 1.2 Hz, 1H), 5.60 - 5.57 (m, 1H), 3.67 - 3.60 (m, 2H), 3.50 - 3.47 (m, 2H), 3.40 - 3.35 (m, 2H), 3.29 - 3.25 (m, 2H), 2.82 - 2.77 (m, 1H), 2.37 - 2.32 (m, 1H), 1.17 (d, *J* = 6.4 Hz, 6H), 1.03 (s, 3H), 0.75 - 0.71 (m, 2H), 0.65 - 0.60 (m, 4H), 0.37 - 0.34 (m, 2H). |
| | | **MS (ESI) M/Z:** 594.4 [M+H]⁺. |
| 079M2 | 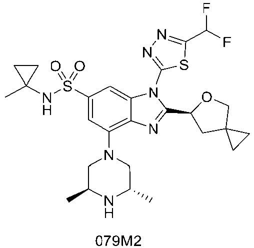 | **¹H NMR** (400 MHz, DMSO-*d₆*) δ 8.06 (s, 1H), 7.81 - 7.54 (m, 2H), 7.09 (d, *J* = 1.2 Hz, 1H), 5.58 - 5.55 (m, 1H), 3.67 - 3.65 (m, 2H), 3.41 - 3.26 (m, 6H), 2.80 - 2.75 (m, 1H), 2.35 - 2.30 (m, 1H), 1.17 (d, *J* = 6.4 Hz, 6H), 1.04 (s, 3H), 0.75 - 0.72 (m, 2H), 0.68 - 0.59 (m, 4H), 0.39 - 0.34 (m, 2H). |
| | | **MS (ESI) M/Z:** 594.4 [M+H]⁺. |
| 079N | 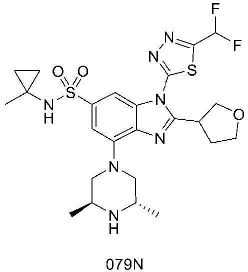 | **¹H NMR** (400 MHz, DMSO-*d₆*) δ 8.03 (s, 1H), 7.68 (t, *J* = 53.2 Hz, 1H), 7.50 (s, 1H), 7.08 (s, 1H), 4.05 - 4.03 (m, 2H), 3.97 - 3.87 (m, 2H), 3.85 - 3.80 (m, 1H), 3.47 - 3.40 (m, 4H), 3.29 - 3.26 (m, 2H), 2.39 - 2.33 (m, 1H), 2.29 - 2.20 (m, 1H), 1.19 (d, *J* = 6.8 Hz, 6H), 1.03 (s, 3H), 0.65 - 0.59 (m, 2H), 0.37 - 0.34 (m, 2H). |
| | | **MS (ESI) M/Z:** 568.4 [M+H]⁺. |
| 082E | 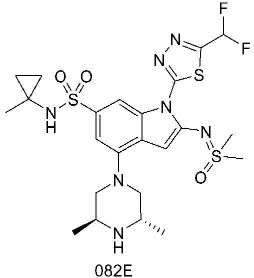 | **¹H NMR** (400 MHz, DMSO-*d₆*+D₂O) δ 8.76 (s, 1H), 7.46 (t, *J* = 53.6 Hz, 1H), 7.11 (d, *J* = 1.6 Hz, 1H), 5.99 (s, 1H), 3.51 - 3.50 (m, 6H), 3.25 - 3.23 (m, 2H), 2.98 - 2.95 (m, 2H), 2.88 - 2.74 (m, 2H), 1.21 (d, *J* = 6.4 Hz, 6H), 1.00 (s, 3H), 0.63 - 0.55 (m, 2H), 0.36 - 0.29 (m, 2H). |
| | | **MS (ESI) M/Z:** 588.2 [M+H]⁺. |

### Biological Evaluation

### Test Example 1: Enzymatic inhibitory activity of the compounds of the present disclosure against PARG

In this experiment, the enzymatic inhibitory activity of the compounds of the present disclosure against PARG was determined using an HTRF (homogeneous time-resolved fluorescence) assay.

### 1. Experimental materials

HTRF assay reagents were purchased from PerkinElmer (Cat. Nos. 61HISTLB and 610SAXLB). The purified protein was prepared in-house.

PARG enzyme reaction buffer: 50 mM Tris (pH 7.4), 0.1 mg/mL BSA, 3 mM EDTA, 0.4 mM EGTA, 1 mM DTT, 50 mM KCl, and 0.01% Tween 20.

### 2. Experimental methods

1) Using an ultra-micro pipette, the compounds of the present disclosure were serially diluted (starting at 10 µM with 3- to 5-fold serial dilutions) and added to a 384-well reaction plate, with each concentration tested in duplicate wells. Control group 1 was added with 10 µM control compound (100% inhibition group). Control group 2 was added with DMSO (0% inhibition group). After compound addition, the 384-well plate was centrifuged at 1000 rpm for 1 minute.
2) 2.5 µL of PARG protein was added to each well and incubated with the compounds of the present disclosure at room temperature for 15 minutes, followed by centrifugation at 1000 rpm for 1 minute.
3) 2.5 µL of PARylated PARP1 protein was added to each well and incubated at room temperature for 60 minutes, followed by centrifugation at 1000 rpm for 1 minute.
4) 5 µL of His-Tb & SA-XL665 HTRF assay reagents were added to each well and incubated at room temperature for 60 minutes, followed by centrifugation at 1000 rpm for 1 minute.
5) Luminescence signals were measured using a PHERAstar FSX microplate reader from BMG.
6) Data analysis was performed using GraphPad Prism 8. The IC₅₀ values of the compounds of the present disclosure were calculated as follows. The average inhibition rate of control group 1 (100% inhibition group) was set as the relative inhibition rate of 100%. The average value of duplicate wells in control group 2 (0% inhibition group) was set as the relative inhibition rate of 0%. The HTRF signal values were converted into relative inhibition rates. The IC₅₀ values of the compounds were calculated by fitting the data to a four-parameter logistic model. The experimental results are shown in Table 15.

**Table 15: Enzymatic inhibitory activity of the compounds of the present disclosure against PARG**

| Example compound | IC₅₀ against PARG enzyme (nM) |
|---|---|
| A002 | 16.4 |
| B002 | 11.6 |
| A003 | 5.4 |
| A004 | 10.7 |
| B003 | 10.2 |
| B001 | 44.7 |
| C002 | 48.2 |
| B004 | 15.6 |
| B005 | 11.5 |
| A006 | 3.7 |
| A005 | 30.4 |
| B011 | 9.9 |
| B010 | 31.4 |
| A013 | 2.9 |
| A018 | 34.1 |
| A019 | 18.1 |
| A023 | 1.5 |
| A027 | 3.8 |
| A029 | 3.0 |
| A030 | 18.2 |
| A005 | 30.4 |
| A014 | 12.3 |
| A028 | 19.0 |
| B013 | 21.3 |
| B014 | 31.4 |
| B015 | 15.8 |
| A032 | 2.4 |
| A031 | 35.2 |
| A033 | 4.0 |
| A036 | 6.8 |
| A037 | 6.8 |
| B016 | 21.2 |
| B017 | 31.5 |
| B019 | 6.3 |
| B020 | 19.8 |
| B021 | 25.1 |
| A038 | 5.2 |
| A039 | 3.5 |
| B022 | 18.7 |
| B023 | 16.7 |
| A040 | 5.0 |
| A041 | 4.4 |
| A042 | 5.1 |
| A043 | 8.0 |
| A044 | 7.0 |
| A045 | 13.9 |
| A046 | 5.5 |
| A047 | 15.0 |
| A048 | 34.8 |
| A049 | 15.7 |
| A050 | 22.5 |
| B024 | 16.5 |
| A051 | 7.5 |
| A052 | 1.6 |
| A053 | 6.7 |
| A054 | 38.6 |
| H001 | 1.1 |
| A055 | 11.9 |
| A056 | 5.2 |
| 008B | 9.6 |
| 057L | 8.6 |
| 057M | 2.8 |
| 057N | 8.3 |
| 057P | 29.3 |
| 057Q | 5.1 |
| 059C | 6.5 |
| 059D | 28.8 |
| 0651 | 1.8 |
| 065J | 2.4 |
| 065K | 6.3 |
| 065L | 5.5 |
| 065M | 1.4 |
| 065N | 7.3 |
| 065P | 5.8 |
| 067B | 6.9 |
| 067G | 8.7 |
| 067J | 7.8 |
| 067JD | 31.6 |
| 067L | 4.9 |
| 067LD | 2.2 |
| 067M | 29.0 |
| 067N | 3.1 |
| 067ND | 2.3 |
| 067P | 5.2 |
| 067Q | 5.0 |
| 067R | 3.4 |
| 067S | 13.3 |
| 067T | 16.0 |
| 067U | 20.0 |
| 067V | 13.4 |
| 067Wl | 19.7 |
| 067W2 | 13.1 |
| 067X | 21.1 |
| 067Y | 21.2 |
| 068A | 3.5 |
| 068B | 15.8 |
| 069D | 23.5 |
| 070A | 15.3 |
| 070B | 20.3 |
| 071C2 | 8.9 |
| 072B | 15.9 |
| 073A | 30.6 |
| 073B | 4.5 |
| 074A | 0.8 |
| 074A-11 | 2.8 |
| 074B | 2.7 |
| 074C | 2.9 |
| 074H | 7.5 |
| 074J | 10.8 |
| 074K | 1.9 |
| 075B | 35.7 |
| 076A | 13.0 |
| 076B | 9.7 |
| 076C | 8.6 |
| 076D | 14.7 |
| 076E | 4.9 |
| 076F1 | 30.6 |
| 076G | 9.0 |
| 076H1 | 14.4 |
| 076H2 | 14.0 |
| 076J | 7.0 |
| 077A | 10.4 |
| 077B | 3.4 |
| 077F | 9.2 |
| 077G | 1.6 |
| 077H | 3.0 |
| 077J | 10.4 |
| 077K | 12.8 |
| 077N | 3.4 |
| 077T | 27.8 |
| 077V | 21.7 |
| 021B | 3.3 |
| 065O | 3.1 |
| 065R | 0.4 |
| 065S | 1.4 |
| 065T | 1.5 |
| 065U | 0.5 |
| 065W | 0.8 |
| 065X | 0.5 |
| 065Z | 1.0 |
| 074G | 3.3 |
| 074L | 15.0 |
| 074M | 6.2 |
| 077B2 | 1.6 |
| 077X | 21.3 |
| 077Y | 10.3 |
| 079J | 1.8 |
| 079J1 | 0.6 |
| 080B | 0.5 |
| 080B1 | 2.2 |
| 080B2 | 0.4 |
| 080J | 2.4 |
| 080K | 1.7 |
| 083A | 2.1 |
| 083A1 | 14.6 |
| 083A2 | 1.0 |
| 084H | 0.7 |
| 084J | 0.7 |
| 084K | 0.7 |
| 084L | 1.1 |
| 084M | 0.8 |
| 065Y | 0.8 |
| 079M | 3.8 |
| 079M2 | 1.9 |
| 079N | 6.2 |
| 082E | 5.5 |
| 081G | 4.9 |

Conclusion: Analysis of the above experimental data indicated that the compounds of the present disclosure exhibited significant inhibitory activity against PARG enzyme.

### Test Example 2: Inhibitory effect of the compounds of the present disclosure on the proliferation of KURAMOCHI cells

In this experiment, the inhibitory effect of the compounds of the present disclosure on the proliferation of the cancer cell line KURAMOCHI was evaluated by measuring intracellular ATP levels using a bioluminescent assay, and the half maximal inhibitory concentration (IC₅₀) was determined.

### 1. Experimental materials

RPMI 1640 medium (Cat. No. 11875-093), fetal bovine serum (10099-141), and 100× Pen/Strep (15070-063) were purchased from GIBCO. CellTiter-Glo Luminescent Cell Viability Assay reagent (Cat. No. G7573) was purchased from Promega.

### 2. Experimental methods

1) Day 1: KURAMOCHI cells were counted using a cell counter and seeded into a 96-well culture plate at a density of 4000 cells per well in 100 µL medium. The plate was incubated overnight in an incubator (37°C, 5% CO₂).
2) Day 2: Using a D300e digital dispenser (TECAN), 500 nL of serially diluted test compounds of the present disclosure (starting at 10 µM, a total of 9 concentrations, 1:4 dilution) were added to the cells in the culture plate. The final concentration of DMSO was 0.5%. The cell culture plate was incubated for 7 days in a cell culture incubator (37°C, 5% CO₂). Control group 1 received only medium and DMSO (100% inhibition group). Control group 2 received only DMSO (0% inhibition group). The final concentration of DMSO in both groups was 0.5%.
3) Day 9: 100 µL of CellTiter-Glo reagent was added to each well. The plate was shaken at 500 rpm for 2 minutes, centrifuged at 1000 rpm for 1 minute, and then incubated at room temperature in the dark for 10 minutes to stabilize the luminescence signal.
4) Luminescence signals were measured using an EnVision microplate reader (PerkinElmer).
5) Data analysis was performed using GraphPad Prism 8. The IC₅₀ values of the compounds of the present disclosure were calculated as follows. The CTG signal values were analyzed. The average inhibition rate of control group 1 (100% inhibition group) was set as the relative inhibition rate of 100%. The average inhibition rate of control group 2 (0% inhibition group) was set as the relative inhibition rate of 0%. The CTG signal values were converted into relative inhibition rates. The inhibition percentage (inhibition%) for each concentration of the compounds of the present disclosure was calculated according to the following formula:
   $Inhibition % = \left(b-x\right) / \left(b-a\right) * 100 % ;$
   a = CTG value (highest concentration), b = CTG value (blank well), x = CTG value (test well).
   IC₅₀ was calculated using GraphPad Prism 8.

(1) The concentration and inhibition rate corresponding to each well were recorded. Statistical calculation was performed using Log10 (compound concentration).
(2) The data were input into GraphPad Prism 8 and Analysis was selected.
(3) Nonlinear regression (curve fit) was selected.
(4) Log (inhibitor) vs. response - Variable slope was selected.
(5) The calculation formula was selected, and the calculation was performed according to the following formula: Y = Bottom + (Top - Bottom) / (1 + 10 ^ ((LogIC₅₀ - X) * HillSlope)); X: log of dose or concentration; Y: response; Top and Bottom: maximum and minimum response.
(6) The IC₅₀ values were calculated by fitting the data. The experimental results are shown in Table 16.

**Table 16: Inhibitory effect of the compounds of the present disclosure on the proliferation of KURAMOCHI cells**

| Example compound | IC₅₀ against KURAMOCHI cells (nM) |
|---|---|
| A002 | 86 |
| B002 | 16 |
| A003 | 13 |
| B003 | 16 |
| C002 | 25 |
| D002 | 85 |
| B001 | 53 |
| A004 | 80 |
| F002 | 49 |
| A008 | 59 |
| A006 | 8 |
| A005 | 45 |
| A018 | 7 |
| A019 | 16 |
| A023 | 4 |
| A027 | 5 |
| A029 | 8 |
| A030 | 10 |
| A013 | 13 |
| A014 | 37 |
| A028 | 48 |
| B010 | 59 |
| B011 | 32 |
| B013 | 53 |
| B014 | 43 |
| A032 | 5 |
| A031 | 63 |
| A033 | 9 |
| A036 | 12 |
| A037 | 14 |
| B016 | 74 |
| B017 | 20 |
| B019 | 19 |
| B020 | 59 |
| B021 | 51 |
| A038 | 7 |
| A039 | 7 |
| A040 | 9 |
| A041 | 11 |
| A042 | 6 |
| A043 | 11 |
| A045 | 16 |
| A046 | 8 |
| A047 | 13 |
| A048 | 27 |
| A049 | 50 |
| A050 | 20 |
| A051 | 12 |
| A052 | 2 |
| A053 | 37 |
| 008B | 9 |
| 057L | 8 |
| 057M | 6 |
| 057N | 9 |
| 057Q | 7 |
| 059C | 20 |
| 059D | 40 |
| 065I | 5 |
| 065J | 7 |
| 065K | 22 |
| 065L | 18 |
| 065M | 3 |
| 065N | 9 |
| 065P | 7 |
| 067B | 15 |
| 067G | 26 |
| 067J | 21 |
| 067L | 4 |
| 067LD | 5 |
| 067N | 3 |
| 067ND | 3 |
| 067P | 74 |
| 067Q | 13 |
| 067R | 6 |
| 067S | 14 |
| 067T | 11 |
| 067U | 21 |
| 067V | 83 |
| 067Wl | 23 |
| 067W2 | 11 |
| 067X | 23 |
| 067Y | 34 |
| 068A | 4 |
| 068B | 17 |
| 069E | 55 |
| 070A | 18 |
| 070B | 25 |
| 071C2 | 50 |
| 072B | 53 |
| 072D | 52 |
| 073A | 34 |
| 073B | 22 |
| 074A | 1 |
| 074A-11 | 4 |
| 074B | 3 |
| 074C | 13 |
| 074H | 12 |
| 074K | 8 |
| 076E | 35 |
| 076H1 | 34 |
| 076H2 | 40 |
| 076J | 9 |
| 077A | 11 |
| 077B | 7 |
| 077F | 9 |
| 077G | 2 |
| 077H | 5 |
| 077N | 8 |
| 077S | 58 |
| 077T | 35 |
| 077V | 37 |
| 078A | 30 |
| 078B | 34 |
| 078C | 15 |
| 021B | 11 |
| 065O | 3 |
| 065R | 2 |
| 065S | 2 |
| 065T | 13 |
| 065U | 2 |
| 065W | 4 |
| 065X | 4 |
| 065Z | 4 |
| 074G | 40 |
| 074M | 16 |
| 077B2 | 3 |
| 077X | 16 |
| 079J | 4 |
| 079J1 | 2 |
| 080B | 1 |
| 080B1 | 4 |
| 080B2 | 0.6 |
| 080J | 5 |
| 080K | 4 |
| 083A | 4 |
| 083A1 | 25 |
| 083A2 | 3 |
| 084H | 3 |
| 084J | 16 |
| 084K | 9 |
| 084L | 29 |
| 084M | 9 |
| 065Y | 6 |
| 079M | 15 |
| 081G | 25 |

Conclusion: Analysis of the above experimental data indicated that the example compounds of the present disclosure exhibited an inhibitory effect on the proliferation of KURAMOCHI cells.

### Test Example 3: In vitro evaluation of PXR activation potential of the compounds of the present disclosure in stably transfected DPX2 cells

### 1. Experimental purpose

The purpose of this study was to evaluate the potential of the compounds of the present disclosure and the comparative compound (WO2023183850A1, compound of formula A) to activate PXR *in vitro,* thereby inducing drug-metabolizing enzymes.

### 2. Experimental materials

DPX2 cells (HepG2 cell line stably transfected with PXR and a CYP3A4 promoter-driven luciferase reporter gene) were purchased from Puracyp Inc (Carlsbad, CA). Fetal bovine serum was purchased from Acantor.

### 3. Experimental methods

1) DPX2 cells were cultured in medium containing 10% fetal bovine serum (FBS).
2) DPX2 cells were cultured in a T-75 flask in a cell culture incubator at 37°C with 5% CO₂ and 95% relative humidity. The cells were subjected to trypsinization when they reached 80% to 90% confluence.
3) The medium was aspirated from the T-75 flask, and the cells were rinsed with 10 mL of PBS. After removing PBS, 3 to 5 mL of trypsin was added. The flask was incubated at 37°C for approximately 5 minutes or until cell detachment and floating were observed. An excess of serum-containing medium was then added to inactivate the trypsin.
4) The detached cells were transferred to a 50 mL centrifuge tube and centrifuged at 150 g for 5 minutes. The cell pellet was resuspended in seeding medium to a density of 3.2 × 10⁵ cells/mL. 25 µL of the cell suspension was then seeded into each well of a 384-well cell culture plate. The 384-well plate was incubated for 24 hours in the cell culture incubator before conducting the PXR activation assay.
5) DMSO stock solutions of test compounds, positive control (rifampicin), and negative control (propranolol) were prepared at 1000× the final incubation concentrations using a Tecan system. The final incubation concentration of positive control (rifampicin) was 1µM, 10 µM, or 20 µM. The final incubation concentration of negative control (propranolol) was 10 µM. The final incubation concentrations of test compounds were provided by the client. The final concentration of DMSO in incubation buffer was 0.1%.
6) The 384-well plate was removed from the cell culture incubator, and 25 nL of DMSO stock solution containing test or control compounds was dispensed into the corresponding wells using Echo, with each experimental condition tested in triplicate. The 384-well plate was returned to the cell culture incubator and incubated for 24 or 48 hours.
7) Prior to the experiment, cell morphology and monolayer integrity were examined to ensure acceptable quality. After 24 or 48 hours of cell treatment, quantification of PXR activation was performed.
8) CellTiter Fluor^{™} Cell Viability Assay Kit and One-Glo Luciferase Assay Kit were equilibrated to room temperature. GF-AFC substrate (10 µL) was transferred to assay buffer (10 mL), followed by 1:1 dilution with 10 mL of PBS to prepare a cell viability assay system. One-Glo luciferase assay buffer was mixed with One-Glo luciferase assay substrate to prepare a luciferase assay system.
9) The 384-well plate was removed from the cell culture incubator, and the medium was aspirated from the plate. 25 µL of the prepared cell viability assay system was gently added to each well, followed by incubation for 30 minutes in the cell culture incubator at 37°C.
10) The 384-well plate was removed from the cell culture incubator, and fluorescence of individual wells was measured using a microplate reader in fluorescence mode with excitation at 400 nm and emission at 505 nm. Subsequently, 25 µL of the prepared luciferase assay system was gently added to each well, followed by vortexing and incubation at room temperature for 5 minutes, and luminescence of individual wells was measured using a microplate reader.

### 4. Data analysis

### 1) Cell viability

Cell viability percentage was calculated using the following formula: Cell viability (%) = Iₛₐₘₚₗₑ / I_{vehicle} × 100, where Iₛₐₘₚₗₑ is the mean fluorescence intensity of treated wells; I_{vehicle} is the mean fluorescence intensity of cells treated with 0.1% DMSO.

### 2) Fold activation calculation

All calculations were performed using Microsoft Excel. Normalized luciferase activity was calculated as RLU/RFU, where RLU represents the average of relative light units of triplicate wells for each test compound at each dose, and RFU represents the average of relative fluorescence units of triplicate wells for each test compound at each dose. RLU and RFU for the vehicle represent the average of triplicate wells for the cell samples treated with 0.1% DMSO. Fold activation at the mRNA level was calculated using the following formula:$Fold activation = \left({RLU}_{sample} / {RFU}_{sample}\right) / \left({RLU}_{vehicle} / {RFU}_{vehicle}\right)$

**Table 17: Fold activation of PXR by the compounds of the present disclosure**

| Example compound | Fold activation |
|---|---|
| Comparative compound | 3.16 |
| 065R | 1.45 |
| 065S | 1.82 |
| 065W | 1.11 |

Conclusion: As shown in Table 17, the compounds of the present disclosure exhibited weaker activation ability compared to the comparative compound.

### Test Example 4: Pharmacokinetic evaluation of the compounds of the present disclosure in mice

Using mice as test animals, the compounds of the present disclosure were administered via intravenous bolus and oral injection. Plasma samples were collected at specific time points. Compound concentrations in plasma were determined by LC-MS/MS, and PK parameters were calculated to characterize the pharmacokinetic behavior of the compounds of the present disclosure in plasma.

### 1. Experimental protocol

### 1.1 Test compounds:

Some compounds of the present disclosure.

### 1.2 Experimental animals

CD-1 mice, male, supplied by Zhejiang Vital River Laboratory Animal Technology Co., Ltd.

BALB/c nude mice, female, supplied by Jiangsu GemPharmatech Co., Ltd.

### 1.3 Dosing regimen

Dosing regimen for CD-1 mice: Both the IV (intravenous) and PO (oral) experimental groups consisted of 3 mice. The IV group was administered at a dose of 1 mg/kg and a volume of 5 mL/kg. The PO group was administered at a dose of 30 mg/kg and a volume of 10 mL/kg. The dosing vehicle was 5% (v/v) DMSO/10% (v/v) Solutol/85% (v/v) saline.

Dosing regimen for BALB/c nude mice: The PO (oral) experimental group consisted of 3 mice, which were administered at a dose of 30 mg/kg and a volume of 10 mL/kg. The dosing vehicle was 5% (v/v) DMSO/10% (v/v) Solutol/85% (v/v) saline.

### 1.4 Experimental equipment

The centrifuge was purchased from Eppendorf. The pipettes were purchased from Eppendorf.

### 1.5 Sample collection

Following administration, 0.025 mL of blood was collected via venipuncture at the following time points: 0.0833 (IV), 0.25, 0.5, 1, 2, 4, 8, and 24 hours. Blood samples were collected into EDTA-K2 tubes and centrifuged at 4°C and 2000 g for 10 minutes to separate plasma, which was stored at -80°C.

### 1.6 Sample processing

Mouse plasma processing:
1) 15 µL of plasma was mixed with 200 µL of acetonitrile for protein precipitation. The mixture was vortexed and centrifuged for 15 minutes.
2) The supernatant after processing was diluted with water and analyzed by LC/MS/MS to determine the concentration of the test compound.

### 2. Experimental results

Pharmacokinetic parameters were calculated using WinNonlin 6.1. The PK parameters following IV and PO administration in CD-1 mice are shown in Table 18. The PK parameters following PO administration in BALB/c nude mice are shown in Table 19. Specifically, Cₘₐₓ represents maximum plasma concentration, CL represents clearance, Vss represents volume of distribution at steady state, T_{1/2} represents terminal elimination half-life, MRT_{Inf} represents mean residence time, AUC represents area under the plasma concentration-time curve, and F represents bioavailability.

**Table 18: Pharmacokinetic parameters of the compounds of the present disclosure in CD-1 mice**

| Compound | Route of administration | Dose (mg/kg) | Cₘₐₓ (*nM*) | CL (mL/hr/kg) | Vss (L/kg) | T_{1/2} (hr) | MRT_{Inf} (hr) | AUC (*hr***nM*) |
|---|---|---|---|---|---|---|---|---|
| 065R | IV | 1 | / | 50 | 6.8 | 2.2 | 2.3 | 555 |
| | PO | 30 | 1609 | / | / | 2.4 | 4.6 | 11246 |
| 065W | IV | 1 | / | 22 | 7.2 | 5.2 | 5.6 | 1256 |
| | PO | 30 | 1981 | / | / | 3.3 | 5.7 | 18280 |

**Table 19: Pharmacokinetic parameters of the compounds of the present disclosure in BALB/c nude mice**

| Compound | Route of administration | Dose (mg/kg) | Cₘₐₓ (*nM*) | T_{1/2} (hr) | MRT_{Inf} (hr) | AUC (*hr***nM*) |
|---|---|---|---|---|---|---|
| 065R | PO | 30 | 1616 | 2.62 | 4.39 | 9520 |
| 065S | PO | 30 | 1345 | 2.69 | 4.80 | 9210 |
| 065W | PO | 30 | 1340 | 5.28 | 7.74 | 16397 |
| 080B2 | PO | 30 | 1868 | 2.45 | 3.27 | 8349 |

| | | | | | | |
|---|---|---|---|---|---|---|
| Note: "/" indicates not determined. | | | | | | |

Conclusion: As shown in Tables 18 and 19, the compounds of the present disclosure exhibited favorable pharmacokinetic properties in both CD-1 mice and BALB/c nude mice.

### Test Example 5: In vivo efficacy study

### 1. Experimental purpose

The purpose of this study was to evaluate the anti-tumor activity and toxic side effects of the compounds of the present disclosure, 065M and 077B2, after 16 consecutive days of oral administration in a KURAMOCHI ovarian cancer xenograft model.

### 2. Experimental materials

NOD SCID mice, female, SPF grade, purchased from Beijing Anikeepter Biotech Co., Ltd.

KURAMOCHI cells, purchased from JCRB.

### 3. Experimental procedures

### 3.1 Cell culture

KURAMOCHI tumor cells (purchased from JCBR) were cultured in RPMI 1640 medium containing 10% inactivated fetal bovine serum, 100 U/mL penicillin, and 100 µg/mL streptomycin in an incubator at 37°C with 5% CO₂. The culture medium was changed weekly. The cells were passaged when they reached 80% to 90% confluence, limiting the number of passages to no more than 4 to 5 times. Tumor cells in the logarithmic growth phase were used for *in vivo* implantation.

### 3.2 Cell inoculation

KURAMOCHI tumor cells (2 × 10⁷ cells) resuspended in serum-free RPMI 1640 medium + Matrigel (1:1) were subcutaneously inoculated into the right flank of each experimental animal at a dose of 200 µL. A total of 35 mice were inoculated: 35 mice for the efficacy study; 15 mice for the PK/PD study; 10 mice remaining after grouping. When the tumor volume reached approximately 150 to 200 mm³, the mice were grouped for dosing to initiate the efficacy study. Specifically, they were divided into 7 groups, with 5 mice per group. The remaining animals were grouped for dosing when the tumor volume reached approximately 250 mm³.

### 3.3 Tumor grouping, treatment, and measurements

a. Groups and dosing regimens are shown in Table 20.

**Table 20: In vivo efficacy study design**

| Group | Number of animals | Treatment | Dose (mg/kg)* | Concentration (mg/mL)* | Route of administration | Regimen |
|---|---|---|---|---|---|---|
| G1 | 5 | Vehicle control | - | - | PO | QD × 16 days |
| G2 | 5 | Comparative compound | 30 | 3 | PO | QD × 16 days |
| G3 | 5 | 065M | 30 | 3 | PO | QD × 16 days |
| G4 | 5 | 077B2 | 30 | 3 | PO | BID × 16 days |
| G5 | 5 | 077B2 | 100 | 10 | PO | QD × 16 days |

| | | | | | | |
|---|---|---|---|---|---|---|
| Note: QD: once daily; BID: twice daily. | | | | | | |

b. Treatment was initiated after grouping, with a dosing volume of 10 µL/g body weight via oral administration (PO). Animals were weighed daily before dosing and administered once daily for 21 consecutive days. Tumor diameters were measured twice weekly.
c. Tumor volume (TV) was measured twice weekly to monitor changes in tumor size and growth rate. Tumor volume was calculated as: V = 1/2 × a × b², where a and b represent tumor length and tumor width, respectively. The growth inhibitory effect of compounds on tumor tissue was evaluated using tumor growth inhibition (TGI (%)). TGI (%) = [1 - (average tumor volume of a given treatment group - average tumor volume of the treatment group on the day of grouping) / (average tumor volume of the negative control group - average tumor volume of the negative control group on the day of grouping)] × 100%. Data for both the treatment group and the negative control group were collected on the same day.
d. The mice were weighed during tumor measurements. The relationship between changes in body weight and time of treatment was recorded. The survival and health status of the mice, including general conditions such as activity level and food intake during the dosing period, were also observed. Treatment was discontinued if the body weight of a single animal decreased by more than 15%. Treatment was resumed when the body weight recovered to within 10% of baseline. If treatment was discontinued for more than 48 hours and the animal was in good condition, treatment could be resumed even if the body weight had not recovered to within 10%. Nutritional gel supplementation was provided during treatment discontinuation.
e. At the study endpoint, the mice were euthanized. Carcasses were frozen in a freezer and transferred to a qualified medical waste disposal unit for disposal.

### 4. Experimental results

**Table 21: Experimental data**

| **Group** | **Dose (mg/kg)** | **Tumor volume^{a} (mm³)** | **TGI (%)** |
|---|---|---|---|
| G1: Vehicle control | - | 567 ± 66 | - |
| G2: Comparative compound QD | 30 | 90 ± 22 | 120 |
| G3: 065M QD | 30 | 62 ± 6 | 127 |
| G4: 077B2 BID | 30 | 61 ± 9 | 127 |
| G5: 077B2 QD | 100 | 57 ± 10 | 127 |

| | | | |
|---|---|---|---|
| ^{a}Mean ± SEM. | | | |

### 5. Experimental conclusion

As shown in Table 21 above, both compound 065M and compound 077B2 significantly inhibited tumor growth. In addition, good tolerability was observed in mice.

## Claims

1. A compound of formula (IA), a pharmaceutically acceptable salt thereof, a stereoisomer thereof, a prodrug thereof, an N-oxide thereof, a solvate thereof, or an isotopic derivative thereof, 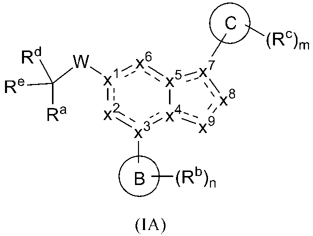 wherein
x¹ is selected from the group consisting of C, CR^{x1}, and N, wherein R^{x1} is selected from the group consisting of H, halogen, hydroxyl, -NRⁿ¹Rⁿ², C₁₋₄ alkyl, and -OC₁₋₄ alkyl;
x² is selected from the group consisting of CR^{x2}, N, C(=O), and C(=CR^{x2}), wherein R^{x2} is selected from the group consisting of H, halogen, hydroxyl, amino, cyano, C₂₋₆ alkenyl, C₂₋₆ alkynyl, C₁₋₆ alkyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x³ is selected from the group consisting of C, CR^{x3}, and N, wherein R^{x3} is selected from the group consisting of H, halogen, hydroxyl, -NRⁿ¹Rⁿ², C₁₋₄ alkyl, and -OC₁₋₄ alkyl;
x⁴ is selected from the group consisting of C, CR^{x4}, and N, wherein R^{x4} is selected from the group consisting of H, halogen, hydroxyl, -NRⁿ¹Rⁿ², C₁₋₄ alkyl, and -OC₁₋₄ alkyl;
x⁵ is selected from the group consisting of C, CR^{x5}, and N, wherein R^{x5} is selected from the group consisting of H, halogen, hydroxyl, -NRⁿ¹Rⁿ², C₁₋₄ alkyl, and -OC₁₋₄ alkyl;
x⁶ is selected from the group consisting of CR^{x6}, N, C(=O), and C(=CR^{x6}), wherein R^{x6} is selected from the group consisting of H, halogen, hydroxyl, cyano, C₂₋₆ alkenyl, C₂₋₆ alkynyl, C₁₋₆ alkyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -C(O)R^{ba}, S(O)ₚR^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁷ is selected from the group consisting of C, CR^{x7}, and N, wherein R^{x7} is selected from the group consisting of H, halogen, hydroxyl, -NRⁿ¹Rⁿ², C₁₋₄ alkyl, and -OC₁₋₄ alkyl;
x⁸ is selected from the group consisting of N, O, CR^{x8}, CR^{x8}R^{x8}, NR^{x8}, S(O)ₚ, C(=O), and C(=CR^{x8}), wherein R^{x8}, at each occurrence, is identical or different, and is independently selected from the group consisting of H, deuterium, halogen, hydroxyl, -NRⁿ¹Rⁿ², cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, -S(O)ₚR^{ba}, -N=S(O)R^{ba}, S(O)ₚNR^{be}R^{bf}, -C(O)OR^{bb}, and -C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x8} attached to the same carbon atom together form a 3- to 8-membered cycloalkyl or 3- to 8-membered heterocyclyl ring; wherein the 3- to 8-membered cycloalkyl or 3- to 8-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, - C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
x⁹ is selected from the group consisting of CR^{x9}, N, O, NR^{x9}, S(O)ₚ, CR^{x9}R^{x9}, and C(=CR^{x8}), wherein R^{x9}, at each occurrence, is identical or different, and is independently selected from the group consisting of H, deuterium, halogen, hydroxyl, -NRⁿ¹Rⁿ², cyano, C₂₋₆ alkenyl, C₂₋₆ alkynyl, C₁₋₆ alkyl, C₁₋₆ alkoxy, C₁₋₆ hydroxyalkyl, -C₁₋₆ alkoxy-C₁₋₆ alkyl, 3- to 8-membered cycloalkyl, 3- to 8-membered heterocyclyl, 6- to 10-membered aryl, 5- to 10-membered heteroaryl, -OR^{f}, -C(O)R^{ba}, S(O)ₚR^{ba}, S(O)ₚNR^{be}R^{bf}, -C(O)OR^{bb}, and - C(O)NR^{bc}R^{bd}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, - C(O)OR^{bb1}, -C(O)NR^{bc1}R^{bd1}, 3- to 8-membered cycloalkyl, 3- to 8-membered heterocyclyl, 6-to 10-membered aryl, and 5- to 10-membered heteroaryl;
alternatively, two R^{x9} attached to the same carbon atom together form a 3- to 8-membered cycloalkyl or 3- to 8-membered heterocyclyl ring; wherein the 3- to 8-membered cycloalkyl and 3- to 8-membered heterocyclyl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl;
alternatively, R^{x8} and R^{x9}, together with the atom to which they are attached, form a 3- to 7-membered ring; wherein the 3- to 7-membered ring is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -OR^{f}, - C(O)R^{ba}, S(O)ₚR^{ba}, S(O)ₚNR^{be}R^{bf}, -C(O)OR^{bb}, -C(O)NR^{bc}R^{bd}, C₁₋₆ deuteroalkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NRⁿ¹Rⁿ², nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
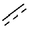 is a single bond or a double bond;
R^{a}, R^{d}, and R^{e} are identical or different, and are each independently selected from the group consisting of hydrogen, deuterium, halogen, C₁₋₆ alkyl, cyano, -NRⁿ¹Rⁿ², hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, -C(O)NR^{bc1}R^{bd1}, 3- to 8-membered cycloalkyl, 3- to 8-membered heterocyclyl, 6- to 10-membered aryl, and 5- to 10-membered heteroaryl;
alternatively, any two of R^{a}, R^{d}, and R^{e}, together with the atom to which they are attached, form a 3- to 8-membered cycloalkyl, 3- to 8-membered heterocyclyl, 6- to 10-membered aryl, or 5- to 10-membered heteroaryl ring; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba}, S(O)ₚR^{ba}, S(O)ₚNR^{be}R^{bf}, -C(O)OR^{bb}, - C(O)NR^{bc}R^{bd}, 3- to 8-membered cycloalkyl, 3- to 8-membered heterocyclyl, 6- to 10-membered aryl, and 5- to 10-membered heteroaryl;
W is selected from the group consisting of 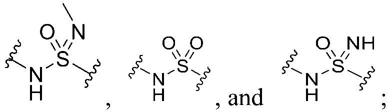
ring B is selected from the group consisting of 3- to 10-membered cycloalkyl, 6- to 10-membered aryl, 5- to 10-membered heteroaryl, 5- to 7-membered monocyclic heterocyclyl, 7-to 12-membered spiro heterocyclyl, 6- to 10-membered bridged heterocyclyl, and 6- to 10-membered fused heterocyclyl;
each R^{b} is identical or different, and is independently selected from the group consisting of hydrogen, deuterium, halogen, C₁₋₆ alkyl, cyano, hydroxyl, C₂₋₆ alkenyl, C₂₋₆ alkynyl, C₁₋₆ alkoxy, C₁₋₆ hydroxyalkyl, -NRⁿ¹Rⁿ², -C₁₋₆ alkoxy-C₁₋₆ alkyl, 3- to 8-membered cycloalkyl, 3-to 8-membered heterocyclyl, 6- to 10-membered aryl, 5- to 10-membered heteroaryl, 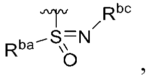 -OR^{f}, -C(O)R^{ba}, -C(O)OR^{bb}, -C(O)NR^{bc}R^{bd}, S(O)ₚNR^{be}R^{bf}, and S(O)ₚR^{ba}; wherein the C₁₋₆ alkyl, C₂₋₆ alkenyl, C₂₋₆ alkynyl, C₁₋₆ alkoxy, -C₁₋₆ alkoxy-C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
Rⁿ¹, Rⁿ², R^{a1}, and R^{a2} are identical or different, and are each independently selected from the group consisting of hydrogen, deuterium, C₁₋₆ alkyl, C₁₋₆ haloalkoxy, C₁₋₆ hydroxyalkyl, - OR^{f}, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, amino, nitro, hydroxyl, and C₁₋₆ hydroxyalkyl;
R^{ba}, R^{bb}, R^{bc}, R^{bd}, R^{be}, and R^{bf} are identical or different, and are each independently selected from the group consisting of hydrogen, deuterium, C₁₋₆ alkyl, C₁₋₆ haloalkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, C₁₋₆ alkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, -OR^{f}, -C(O)R^{ba1}, S(O)ₚR^{ba1}, S(O)ₚNR^{be1}R^{bf1}, -C(O)OR^{bb1}, -C(O)NR^{bc1}R^{bd1}, cyano, -NR^{a1}R^{a2}, nitro, hydroxyl, and C₁₋₆ hydroxyalkyl;
alternatively, Rⁿ¹ and Rⁿ², R^{a1} and R^{a2}, R^{bc} and R^{bd}, or R^{be} and R^{bf}, together with the nitrogen atom to which they are attached, form a 3- to 7-membered heterocyclyl ring; wherein the 3- to 7-membered heterocyclyl is optionally substituted by one or more substituents selected from the group consisting of deuterium, halogen, C₁₋₆ alkyl, C₁₋₆ deuteroalkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, 3- to 8-membered cycloalkyl, 3- to 8-membered heterocyclyl, 6- to 10-membered aryl, and 5- to 10-membered heteroaryl;
ring C is selected from the group consisting of 3- to 10-membered cycloalkyl, 3- to 10-membered heterocyclyl, 6- to 10-membered aryl, and 5- to 10-membered heteroaryl;
each R^{c} is identical or different, and is independently selected from the group consisting of hydrogen, deuterium, halogen, C₁₋₆ alkyl, cyano, -NRⁿ¹Rⁿ², hydroxyl, C₂₋₆ alkenyl, C₂₋₆ alkynyl, C₁₋₆ alkoxy, C₁₋₆ hydroxyalkyl, -C₁₋₆ alkoxy-C₁₋₆ alkyl, -C(O)R^{ba}, S(O)ₚR^{ba}, S(O)ₚNR^{be}R^{bf}, -C(O)OR^{bb}, -C(O)NR^{bc}R^{bd}, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5-to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, C₁₋₆ hydroxyalkyl, -C(O)R^{ba1}, S(O)ₚR^{ba1}, S(O)ₚNR^{be1}R^{bf1}, -C(O)OR^{bb1}, - C(O)NR^{bc1}R^{bd1}, 3- to 8-membered cycloalkyl, 3- to 8-membered heterocyclyl, 6- to 10-membered aryl, and 5- to 10-membered heteroaryl; wherein the 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, cyano, -NR^{a1}R^{a2}, nitro, hydroxyl, - C(O)R^{ba2}, S(O)ₚR^{ba2}, S(O)ₚNR^{be2}R^{bf2}, -C(O)OR^{bb2}, -C(O)NR^{bc2}R^{bd2}, and C₁₋₆ hydroxyalkyl;
R^{ba1}, R^{bb1}, R^{bc1}, R^{bd1}, R^{be1}, R^{bf1}, R^{ba2}, R^{bb2}, R^{bc2}, R^{bd2}, R^{be2}, and R^{bf2} are identical or different, and are each independently selected from the group consisting of hydrogen, deuterium, C₁₋₆ alkyl, C₁₋₆ haloalkoxy, C₁₋₆ alkoxy, C₁₋₆ hydroxyalkyl, C₂₋₆ alkenyl, C₂₋₆ alkynyl, C₂₋₆ haloalkenyl, C₂₋₆ haloalkynyl, C₁₋₆ deuteroalkoxy, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkoxy, C₁₋₆ deuteroalkyl, cyano, -NR^{a1}R^{a2}, -OR^{f}, nitro, hydroxyl, and C₁₋₆ hydroxyalkyl;
R^{f} is selected from the group consisting of hydrogen, C₁₋₆ alkyl, C₁₋₆ hydroxyalkyl, 3- to 8-membered cycloalkyl, 3- to 8-membered heterocyclyl, 6- to 10-membered aryl, and 5- to 10-membered heteroaryl; wherein the C₁₋₆ alkyl, 3- to 8-membered cycloalkyl, 3- to 8-membered heterocyclyl, 6- to 10-membered aryl, and 5- to 10-membered heteroaryl are optionally substituted by one or more substituents selected from the group consisting of deuterium, halogen, C₁₋₆ alkyl, oxo, C₂₋₆ alkenyl, C₂₋₆ alkynyl, C₂₋₆ haloalkenyl, C₂₋₆ haloalkynyl, C₁₋₆ alkoxy, C₁₋₆ haloalkyl, C₁₋₆ haloalkoxy, C₁₋₆ deuteroalkyl, C₁₋₆ deuteroalkoxy, cyano, -NR^{a1}R^{a2}, nitro, hydroxyl, and C₁₋₆ hydroxyalkyl;
n is selected from the group consisting of 0, 1, 2, and 3;
m is selected from the group consisting of 0, 1, 2, and 3;
p is selected from the group consisting of 0, 1, and 2.

2. The compound, the pharmaceutically acceptable salt thereof, the stereoisomer thereof, the prodrug thereof, the N-oxide thereof, the solvate thereof, or the isotopic derivative thereof according to claim 1, wherein R^{d} and R^{e}, together with the atom to which they are attached, form a 3- to 6-membered cycloalkyl or 3- to 6-membered heterocyclyl ring.

3. The compound, the pharmaceutically acceptable salt thereof, the stereoisomer thereof, the prodrug thereof, the N-oxide thereof, the solvate thereof, or the isotopic derivative thereof according to claim 1 or 2, wherein R^{d} and R^{e}, together with the atom to which they are attached, form a cyclopropyl or oxetanyl ring.

4. The compound, the pharmaceutically acceptable salt thereof, the stereoisomer thereof, the prodrug thereof, the N-oxide thereof, the solvate thereof, or the isotopic derivative thereof according to at least one of claims 1 to 3, which is a compound of formula (I), a pharmaceutically acceptable salt thereof, or a stereoisomer thereof, 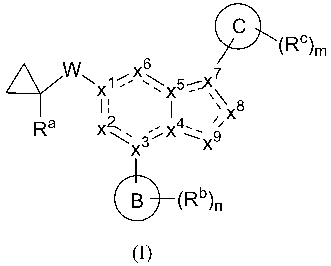 wherein 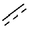 , ring B, ring C, R^{b}, R^{a}, R^{c}, m, n, W, and x¹ to x⁹ are as defined in at least one of claims 1 to 3.

5. The compound, the pharmaceutically acceptable salt thereof, the stereoisomer thereof, the prodrug thereof, the N-oxide thereof, the solvate thereof, or the isotopic derivative thereof according to at least one of claims 1 to 4, wherein ring C is 5-membered heteroaryl; each R^{c} is identical or different, and is independently C₁₋₆ haloalkyl; preferably, ring C is 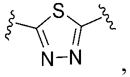 and R^{c} is -CHF₂.

6. The compound, the pharmaceutically acceptable salt thereof, the stereoisomer thereof, the prodrug thereof, the N-oxide thereof, the solvate thereof, or the isotopic derivative thereof according to at least one of claims 1 to 5, which is a compound of formula (II), a pharmaceutically acceptable salt thereof, a stereoisomer thereof, a prodrug thereof, an N-oxide thereof, a solvate thereof, or an isotopic derivative thereof: 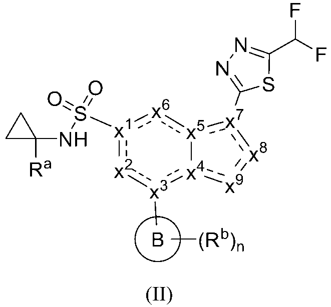 wherein 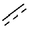 , x¹, x², x³, x⁴, x⁵, x⁶, x⁷, x⁸, x⁹, ring B, R^{b}, R^{a}, and n are as defined in at least one of claims 1 to 5.

7. The compound, the pharmaceutically acceptable salt thereof, the stereoisomer thereof, the prodrug thereof, the N-oxide thereof, the solvate thereof, or the isotopic derivative thereof according to at least one of claims 1 to 6, wherein R^{a} is selected from the group consisting of - CH₃, -CH₂F, -CN, -CH₂=CHF, -CHF₂, -CHF₃, and -CD₃; preferably, R^{a} is selected from the group consisting of -CH₃, -CH₂F, and -CN.

8. The compound, the pharmaceutically acceptable salt thereof, the stereoisomer thereof, the prodrug thereof, the N-oxide thereof, the solvate thereof, or the isotopic derivative thereof according to at least one of claims 1 to 7, wherein ring B is selected from the group consisting of

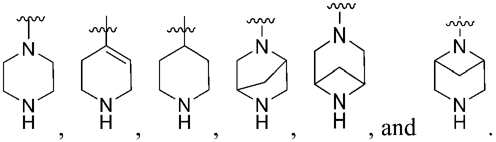

9. The compound, the pharmaceutically acceptable salt thereof, the stereoisomer thereof, the prodrug thereof, the N-oxide thereof, the solvate thereof, or the isotopic derivative thereof according to at least one of claims 1 to 8, wherein 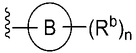 is selected from the group consisting of 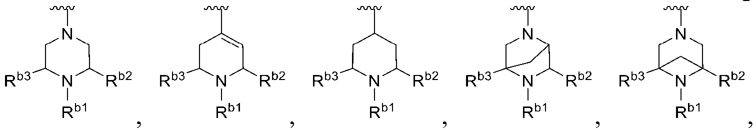 and 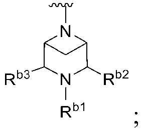 R^{b1}, R^{b2}, and R^{b3} are identical or different, and are each independently R^{b}; R^{b} is as defined in at least one of claims 1 to 8; preferably, 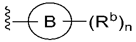 is selected from the group consisting of 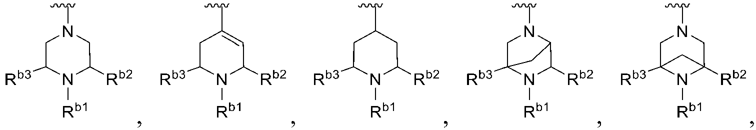 and 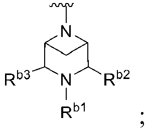 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 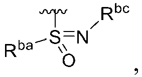 S(O)₂NR^{be}R^{bf}; and -S(O)R^{ba}; R^{b2} and R^{b3} are identical or different, and are each independently selected from the group consisting of hydrogen, C₁₋₆ alkyl, C₂₋₆ alkenyl, and C₂₋₆ alkynyl; R^{ba}, R^{bb}, R^{bc}, R^{bd}, R^{be}, and R^{bf} are as defined in at least one of claims 1 to 8; more preferably, 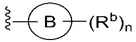 is selected from the group consisting of 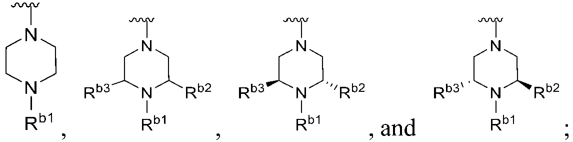 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 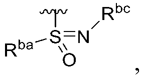 S(O)₂NR^{be}R^{bf}, and -S(O)R^{ba}; R^{b2} and R^{b3} are identical or different, and are each independently selected from the group consisting of hydrogen, C₁₋₆ alkyl, C₂₋₆ alkenyl, and C₂₋₆ alkynyl; R^{ba}, R^{bb}, R^{bc}, R^{bd}, R^{be}, and R^{bf} are as defined in at least one of claims 1 to 8; further preferably, 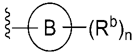 is selected from the group consisting of 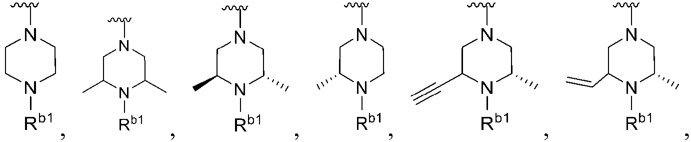 and 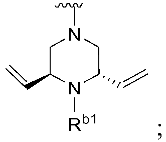 R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 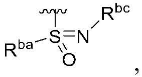 S(O)₂NR^{be}R^{bf}, and -S(O)R^{ba}; R^{ba}, R^{bb}, R^{bc}, R^{bd}, R^{be}, and R^{bf} are as defined in at least one of claims 1 to 8; most preferably, 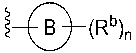 is selected from the group consisting of 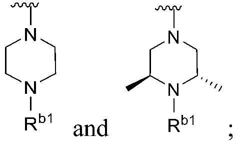 R^{b1} is selected from the group consisting of hydrogen, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 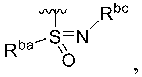 S(O)₂NR^{be}R^{bf}, and -S(O)R^{ba}; R^{ba}, R^{bb}, R^{bc}, R^{bd}, R^{be}, and R^{bf} are identical or different, and are each independently selected from the group consisting of hydrogen, C₁₋₆ alkyl, 3- to 8-membered heterocyclyl, C₁₋₆ haloalkyl, C₁₋₆ hydroxyalkyl, C₁₋₆ deuteroalkyl, 3- to 8-membered cycloalkyl, C₁₋₆ alkoxy, C₂₋₆ alkenyl, and 5-to 6-membered heteroaryl; wherein the C₁₋₆ alkyl, 3- to 8-membered heterocyclyl, 3- to 8-membered cycloalkyl, and 5- to 6-membered heteroaryl are optionally substituted by one or more substituents selected from the group consisting of C₁₋₆ alkyl, halogen, C₁₋₆ deuteroalkyl, C₁₋₆ haloalkyl, hydroxyl, C₁₋₆ alkoxy, C₁₋₆ deuteroalkoxy, -C(O)OR^{bb1}, and -NR^{a1}R^{a2}; R^{bb1}, R^{a1}, and R^{a2} are as defined in at least one of claims 1 to 8.

10. The compound, the pharmaceutically acceptable salt thereof, the stereoisomer thereof, the prodrug thereof, the N-oxide thereof, the solvate thereof, or the isotopic derivative thereof according to at least one of claims 1 to 9, wherein R^{b} and R^{b1} are each identical or different, and are each independently selected from the group consisting of hydrogen, 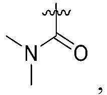 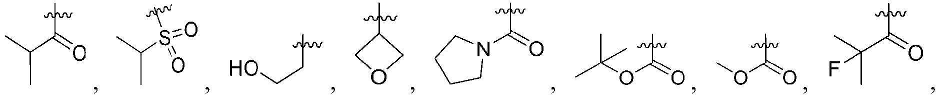 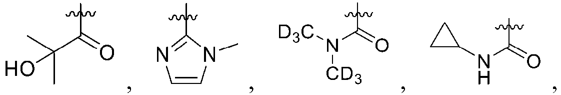 methyl, vinyl, ethynyl,

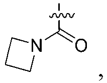

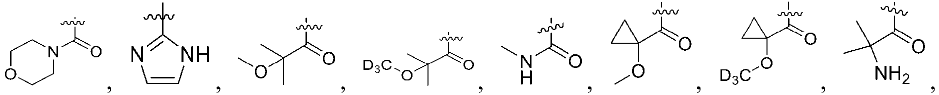

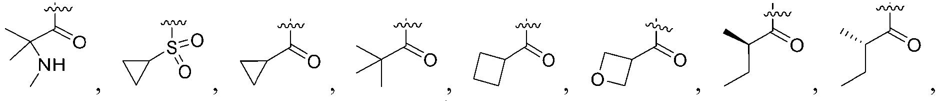

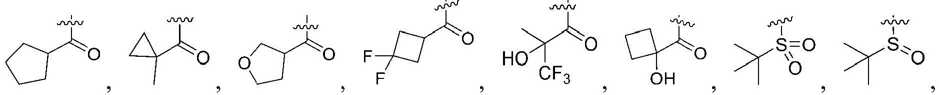

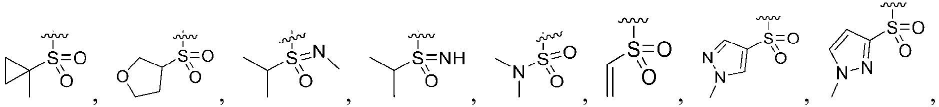

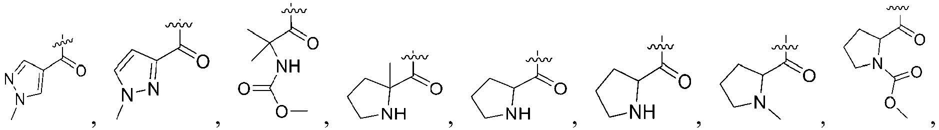

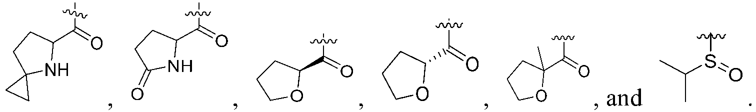

11. The compound, the pharmaceutically acceptable salt thereof, the stereoisomer thereof, the prodrug thereof, the N-oxide thereof, the solvate thereof, or the isotopic derivative thereof according to at least one of claims 1 to 10, wherein 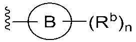 is selected from the group consisting of 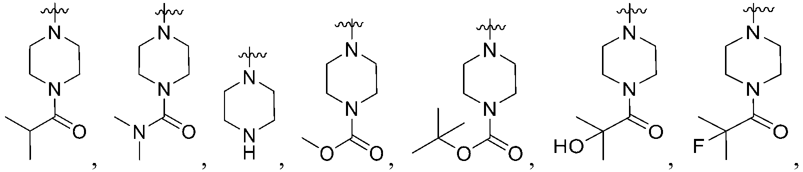 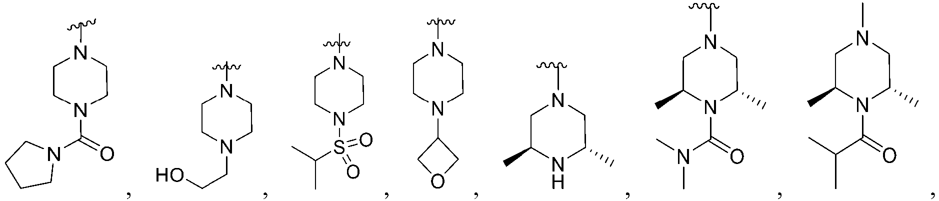 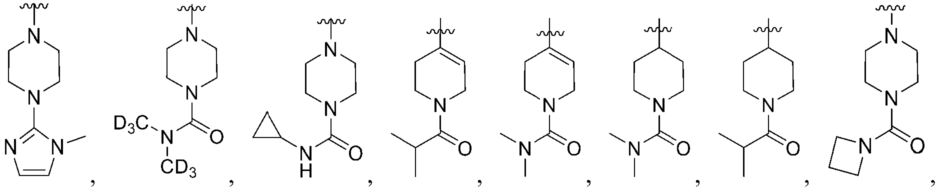 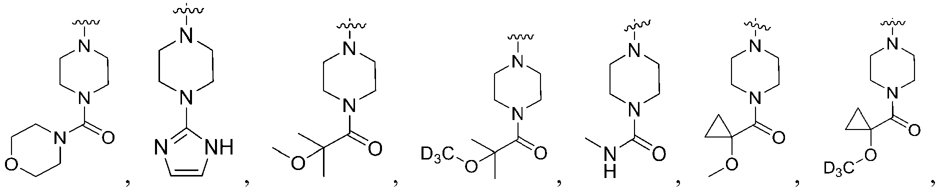 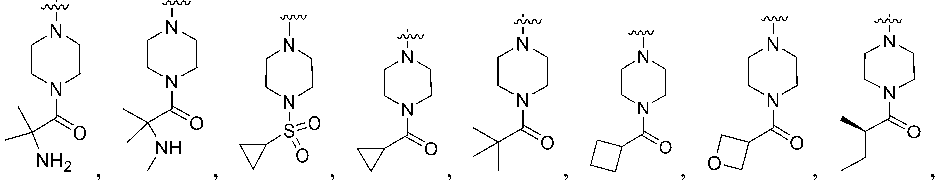 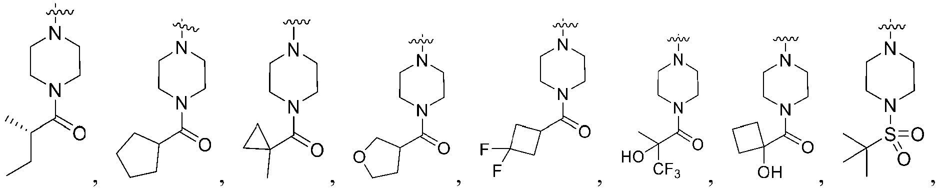 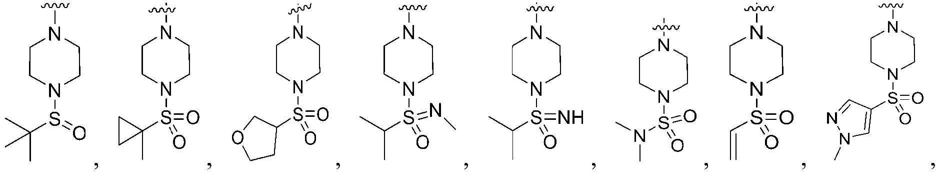 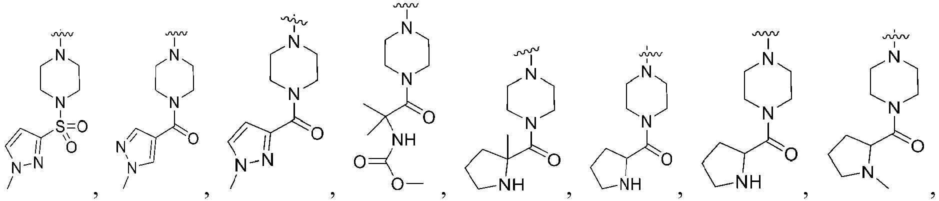 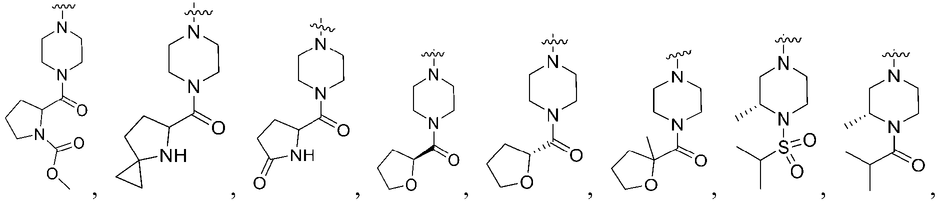 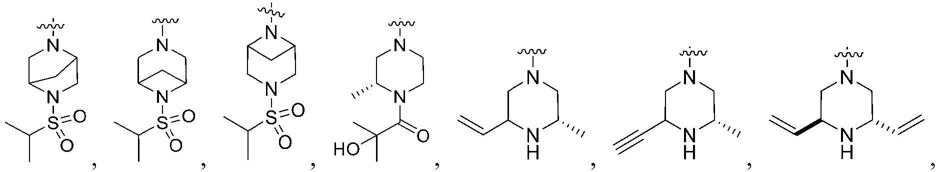 and

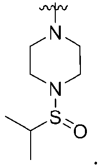

12. The compound, the pharmaceutically acceptable salt thereof, the stereoisomer thereof, the prodrug thereof, the N-oxide thereof, the solvate thereof, or the isotopic derivative thereof according to at least one of claims 1 to 11, wherein the structural moiety 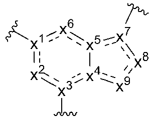 is selected from the group consisting of 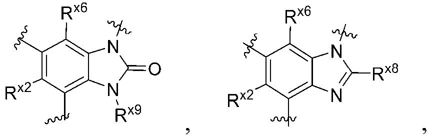 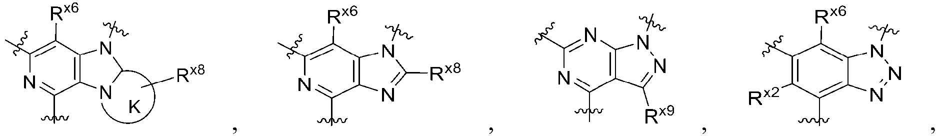 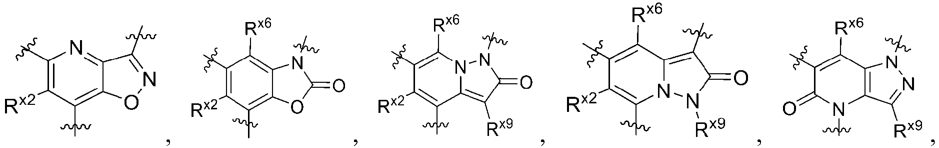 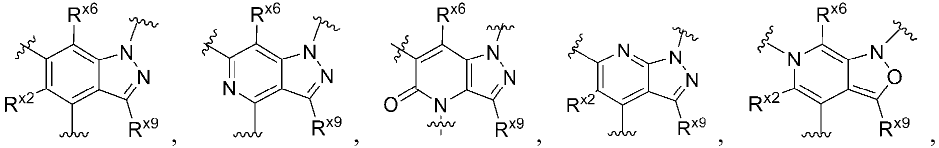 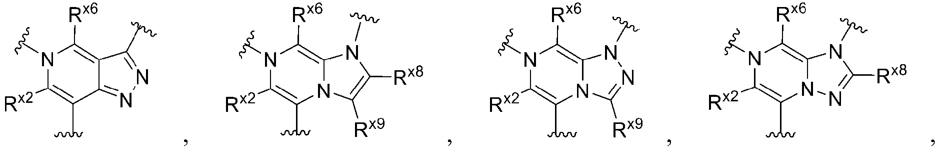 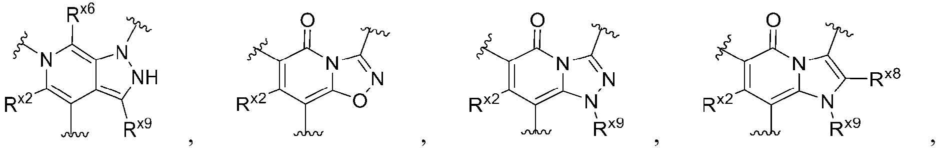 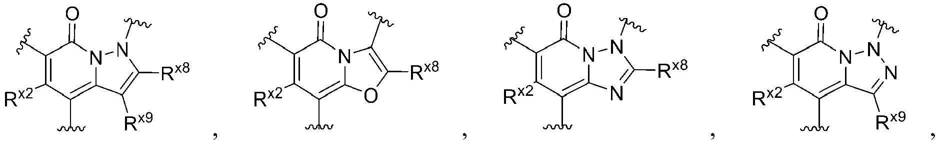 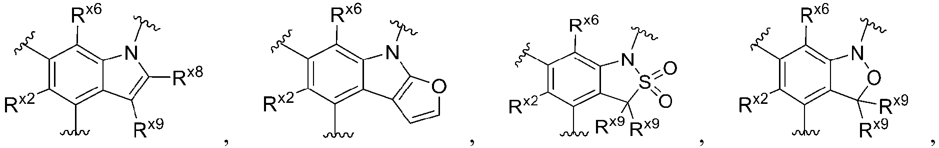 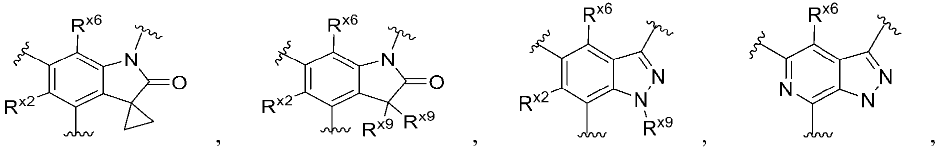 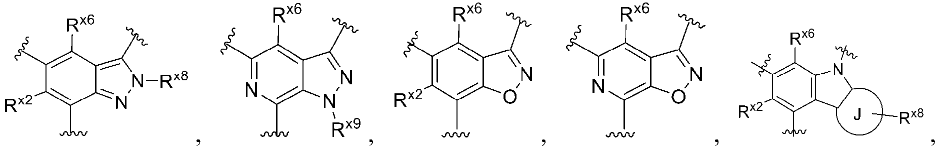 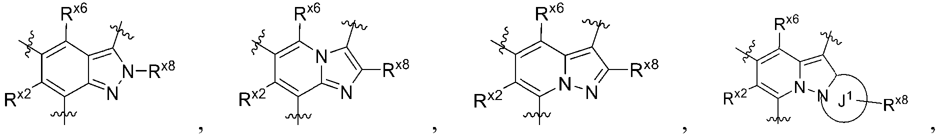 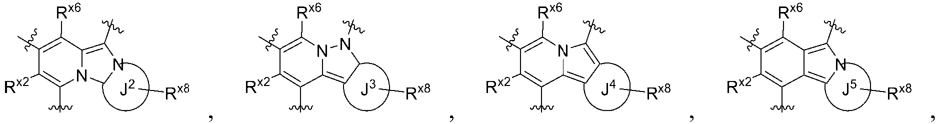 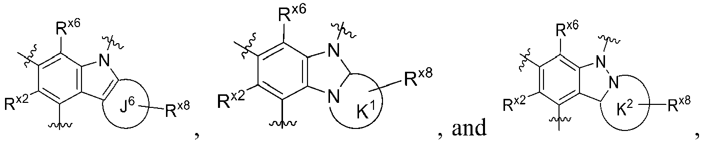 wherein ring J, ring J¹, ring J², ring J³, ring J⁴, ring J³, ring J⁶, ring K¹, ring K², and ring K are identical or different, and are each independently an optionally substituted 3- to 7-membered ring; R^{x2}, R^{x6}, R^{x8}, and R^{x9} are as defined in at least one of claims 1 to 14; preferably, the structural moiety 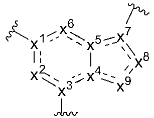 is selected from the group consisting of

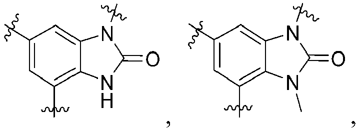

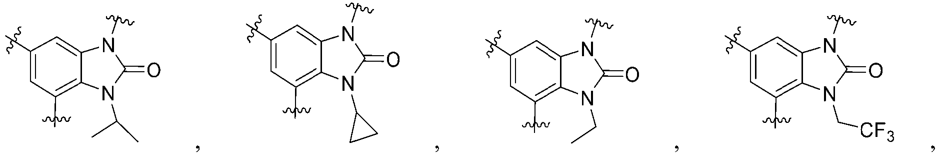

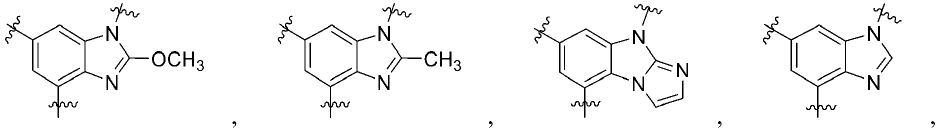

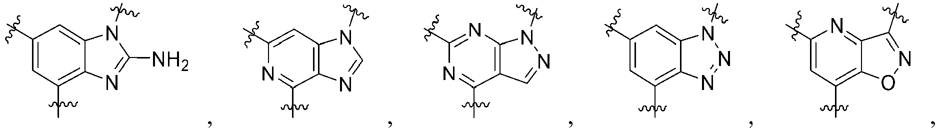

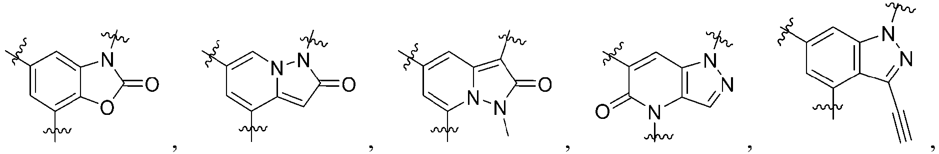

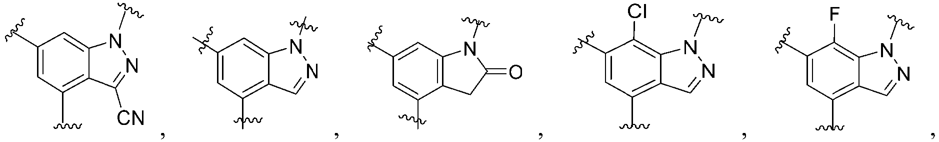

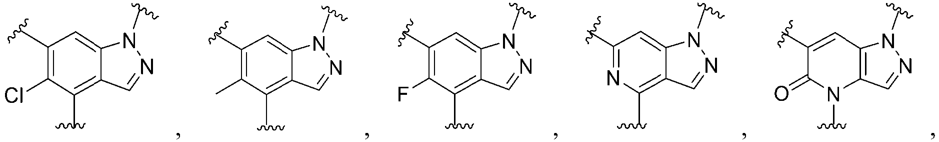

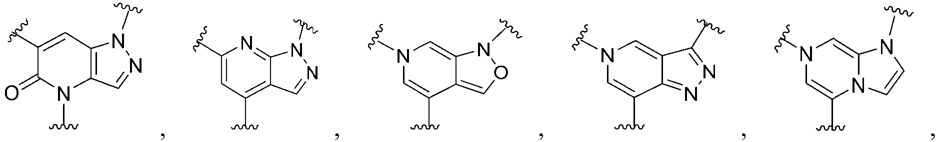

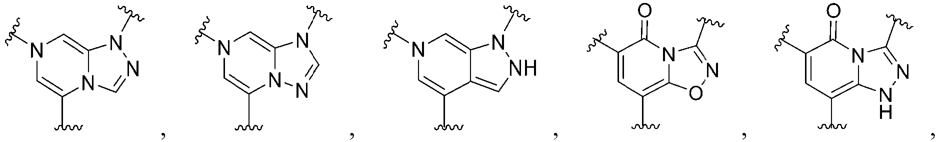

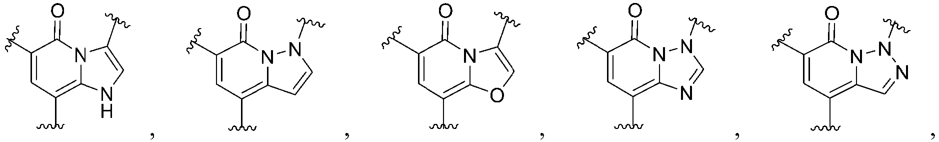

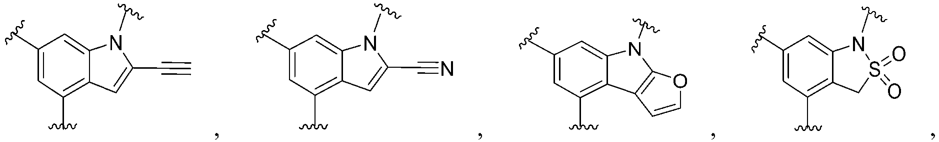

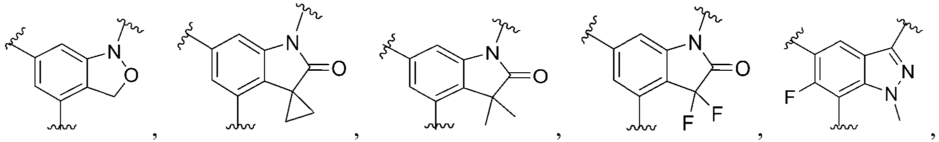

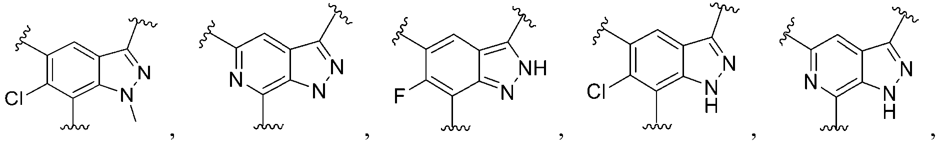

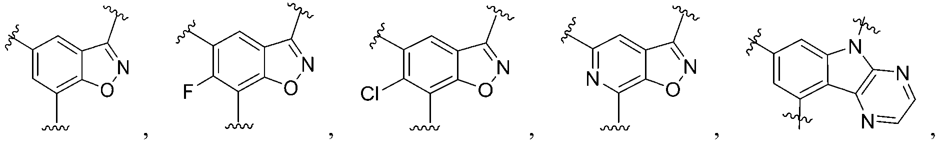

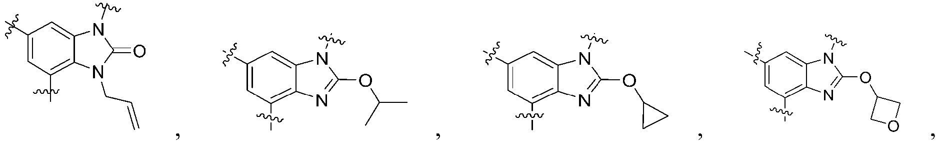

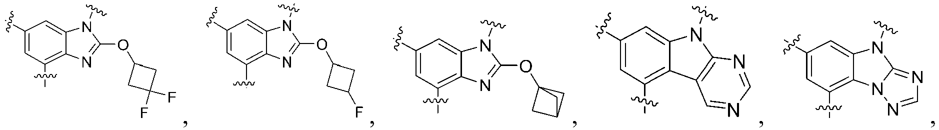

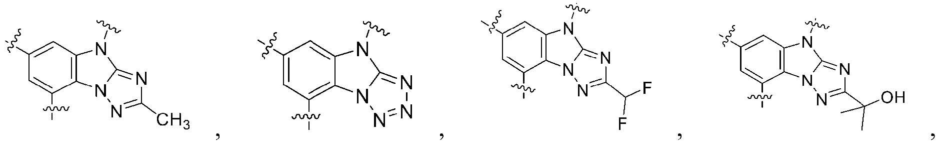

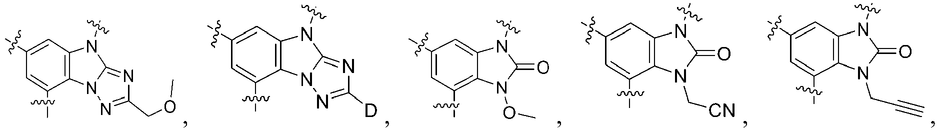

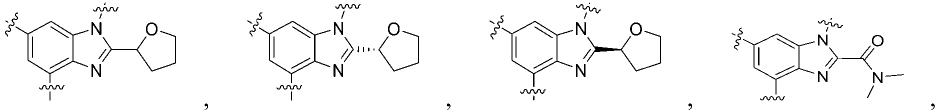

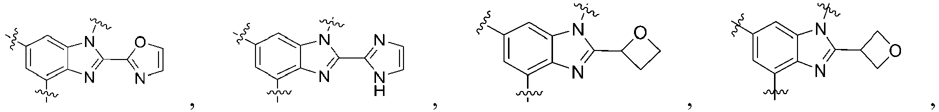

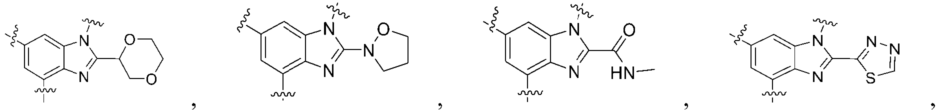

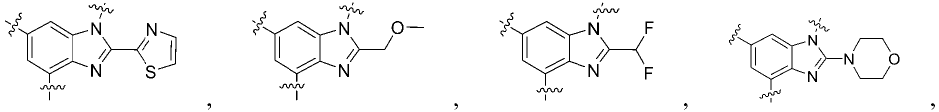

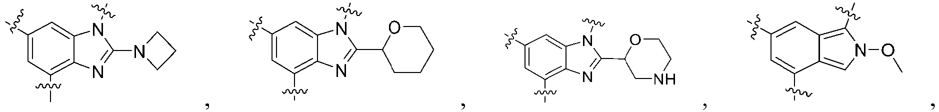

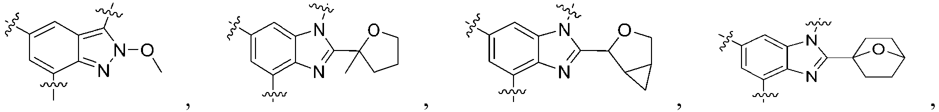

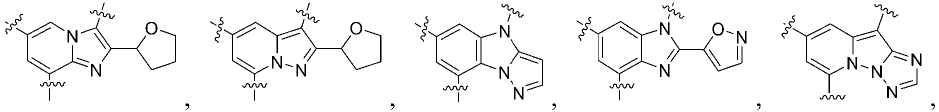

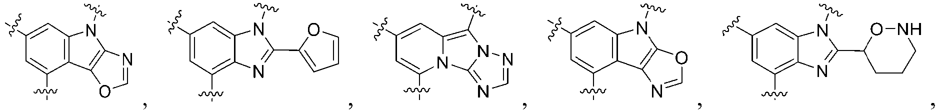

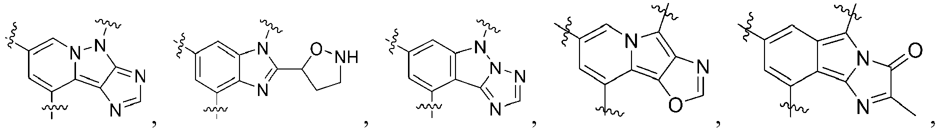

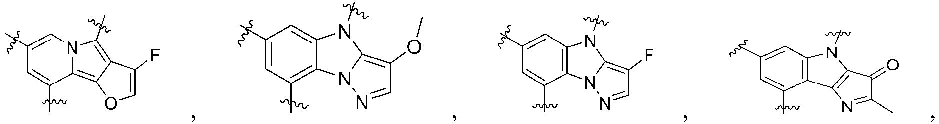

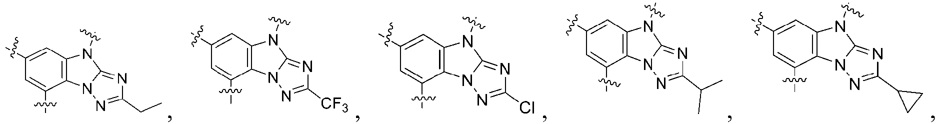

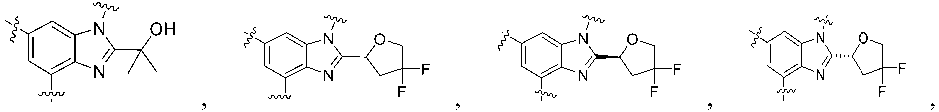

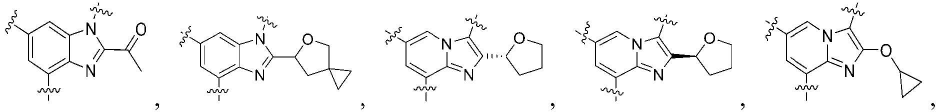

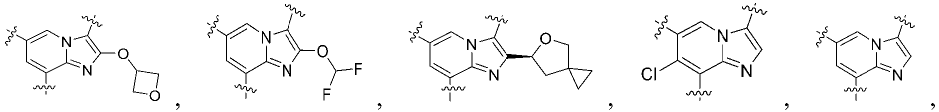

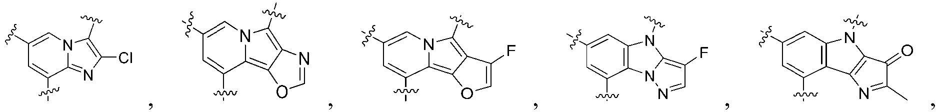

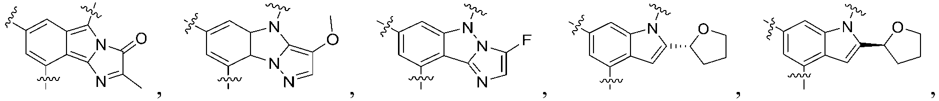

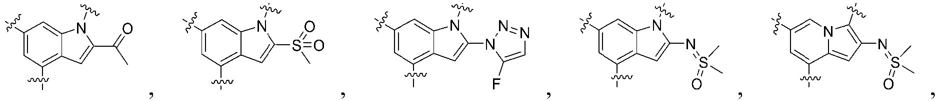

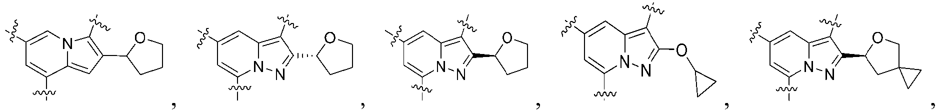

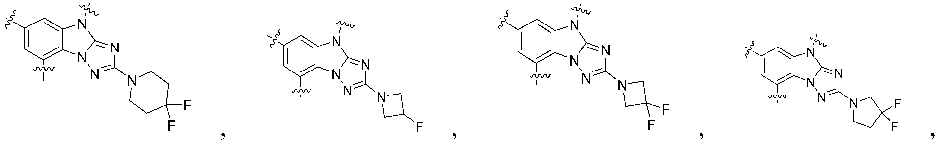

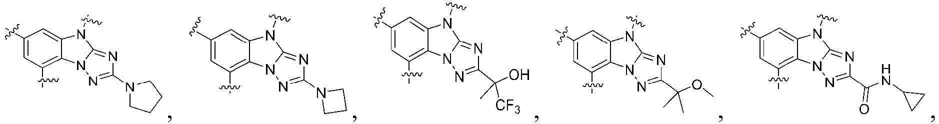

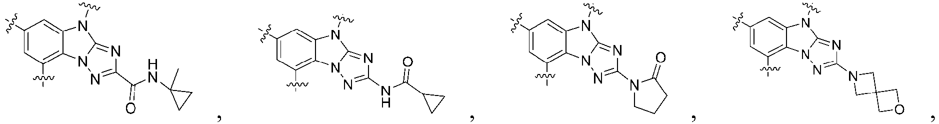

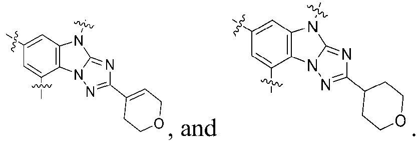

13. The compound, the pharmaceutically acceptable salt thereof, the stereoisomer thereof, the prodrug thereof, the N-oxide thereof, the solvate thereof, or the isotopic derivative thereof according to at least one of claims 1 to 12, which is a compound of formula (II-1B), formula (II-1C), formula (II-2B), or formula (II-2C), a pharmaceutically acceptable salt thereof, a stereoisomer thereof, a prodrug thereof, an N-oxide thereof, a solvate thereof, or an isotopic derivative thereof: 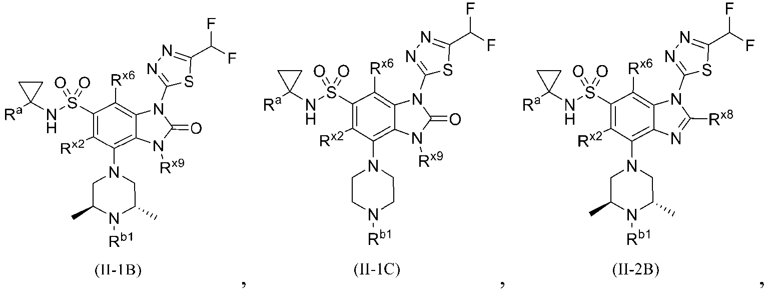 or 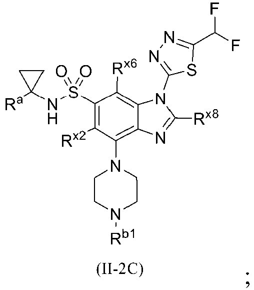 wherein R^{b1} is selected from the group consisting of hydrogen, C₁₋₆ alkyl, C₁₋₆ hydroxyalkyl, 3- to 8-membered heterocyclyl, -S(O)₂R^{ba}, -C(O)R^{ba}, -C(O)OR^{bb}, -C(O)NR^{bc}R^{bd}, 5- to 6-membered heteroaryl optionally substituted by C₁₋₆ alkyl, 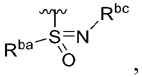 S(O)₂NR^{be}R^{bf}; and -S(O)R^{ba}; R^{a}, R^{x2}, R^{x6}, R^{x8}, R^{x9}, R^{ba}, R^{bb}, R^{bc}, R^{bd}, R^{be}, and R^{bf} are as defined in at least one of claims 1 to 12.

14. The compound, the pharmaceutically acceptable salt thereof, the stereoisomer thereof, the prodrug thereof, the N-oxide thereof, the solvate thereof, or the isotopic derivative thereof according to at least one of claims 1 to 13, which is a compound of formula (II-2K), formula (II-2L), or formula (II-2M), a pharmaceutically acceptable salt thereof, a stereoisomer thereof, a prodrug thereof, an N-oxide thereof, a solvate thereof, or an isotopic derivative thereof: 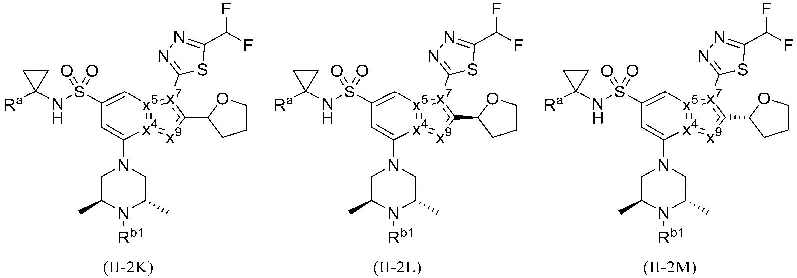 wherein
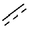 is a single bond or a double bond;
x⁴, x⁵, and x⁷ are identical or different, and are each independently selected from the group consisting of N and C;
x⁹ is selected from the group consisting of N and CH;
and the ring containing x⁴, x⁵, x⁷, and x⁹ is an aromatic ring;
R^{b1} is R^{b}; R^{b} and R^{a} are as defined in at least one of claims 1 to 13.

15. The compound, the pharmaceutically acceptable salt thereof, the stereoisomer thereof, the prodrug thereof, the N-oxide thereof, the solvate thereof, or the isotopic derivative thereof according to at least one of claims 1 to 14, which is a compound of formula (II-3A), a pharmaceutically acceptable salt thereof, a stereoisomer thereof, a prodrug thereof, an N-oxide thereof, a solvate thereof, or an isotopic derivative thereof: 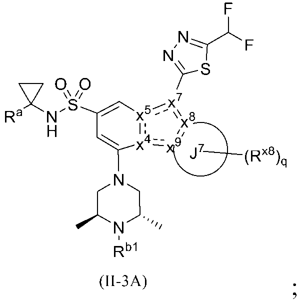 wherein
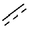 is a single bond or a double bond;
x⁴, x⁵, x⁷, x⁸, and x⁹ are identical or different, and are each independently selected from the group consisting of N and C; and the ring containing x⁴, x⁵, x⁷, x⁸, and x⁹ is an aromatic ring;
ring J⁷ is selected from the group consisting of 5- to 10-membered heteroaryl and 3- to 8-membered heterocyclyl;
q is selected from the group consisting of 0, 1, and 2;
R^{b1} is R^{b}; R^{b}, R^{a}, and R^{x8} are as defined in at least one of claims 1 to 14.

16. The compound, the pharmaceutically acceptable salt thereof, the stereoisomer thereof, the prodrug thereof, the N-oxide thereof, the solvate thereof, or the isotopic derivative thereof according to at least one of claims 1 to 15, wherein ring J, ring J¹, ring J², ring J³, ring J⁴, ring J⁵, ring J⁶, ring J⁷, ring K¹, ring K², and ring K are identical or different, and are each independently a 3- to 7-membered ring, preferably 5- to 10-membered heteroaryl or 3- to 8-membered heterocyclyl, more preferably 5- to 10-membered heteroaryl, further preferably 5-to 6-membered nitrogen-containing heteroaryl, and most preferably 5-membered nitrogen-containing heteroaryl.

17. The compound, the pharmaceutically acceptable salt thereof, the stereoisomer thereof, the prodrug thereof, the N-oxide thereof, the solvate thereof, or the isotopic derivative thereof according to at least one of claims 1 to 16, wherein R^{x8}, at each occurrence, is identical or different, and is independently selected from the group consisting of hydrogen, deuterium, methyl, amino, methoxy, 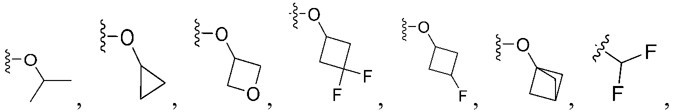 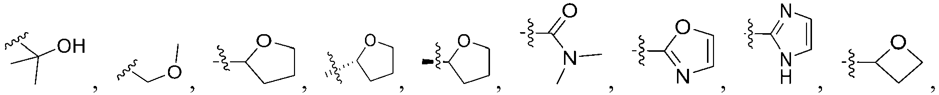 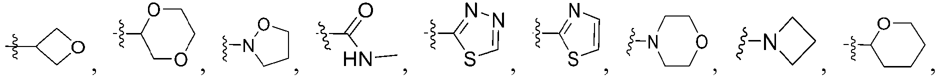 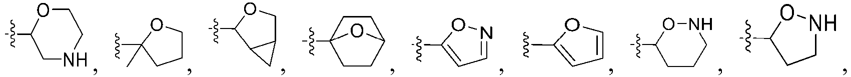 oxo, Cl, F, ethyl, trifluoromethyl, isopropyl, cyclopropyl,

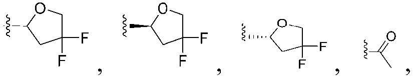

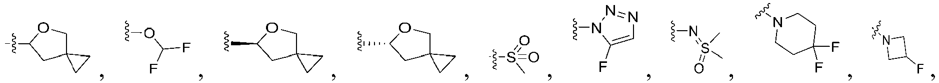

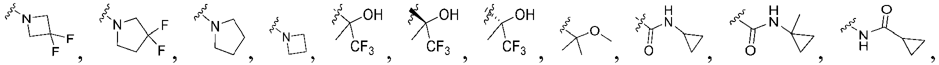

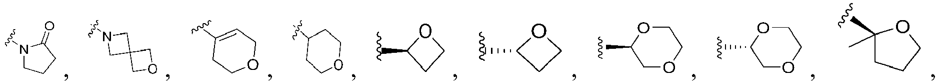

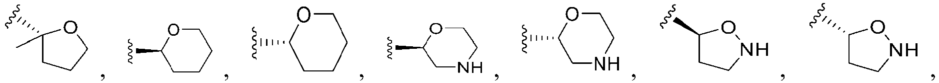

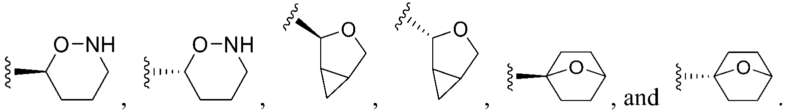

18. The compound, the pharmaceutically acceptable salt thereof, the stereoisomer thereof, the prodrug thereof, the N-oxide thereof, the solvate thereof, or the isotopic derivative thereof according to at least one of claims 1 to 17, wherein R^{x9} is selected from the group consisting of methyl, ethyl, isopropyl, cyclopropyl, -CH₂CF₃, 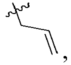 methoxy,

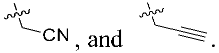

19. The compound, the pharmaceutically acceptable salt thereof, the stereoisomer thereof, the prodrug thereof, the N-oxide thereof, the solvate thereof, or the isotopic derivative thereof according to at least one of claims 1 to 18, wherein R^{ba}, R^{bb}, R^{bc}, R^{bd}, R^{be}, and R^{bf} are identical or different, and are each independently selected from the group consisting of hydrogen, methyl, -CD₃, isopropyl, *tert*-butyl, 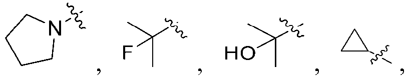 methoxy, *tert*-butoxy,

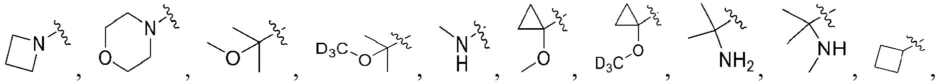

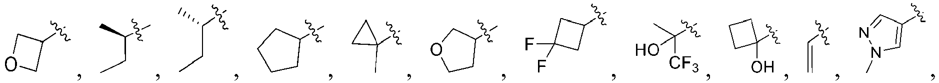

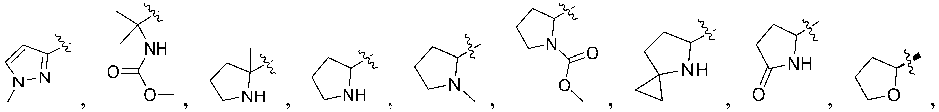

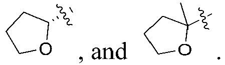

20. The compound, the pharmaceutically acceptable salt thereof, the stereoisomer thereof, the prodrug thereof, the N-oxide thereof, the solvate thereof, or the isotopic derivative thereof according to at least one of claims 1 to 19, wherein R^{b1}, at each occurrence, is identical or different, and is independently hydrogen.

21. A compound, a pharmaceutically acceptable salt thereof, a stereoisomer thereof, a prodrug thereof, an N-oxide thereof, a solvate thereof, or an isotopic derivative thereof, wherein the compound is selected from the group consisting of any one of the following compounds:

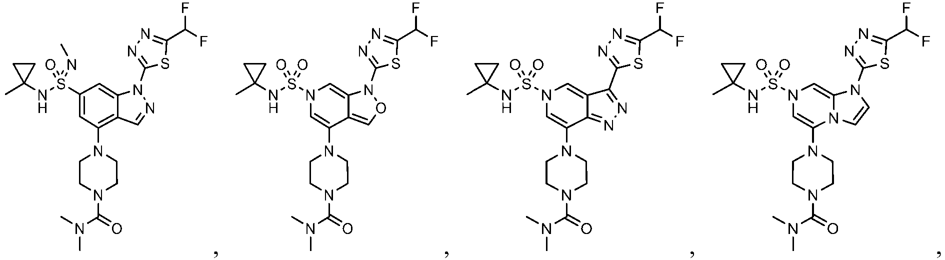

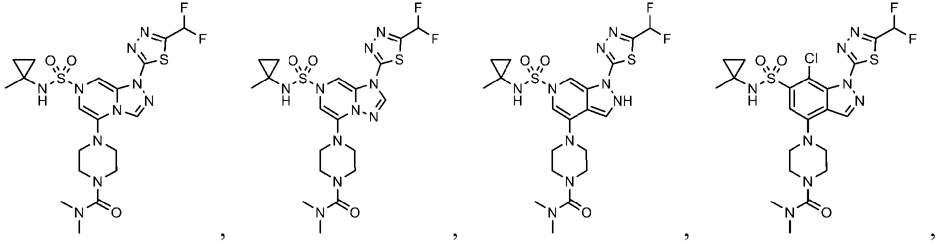

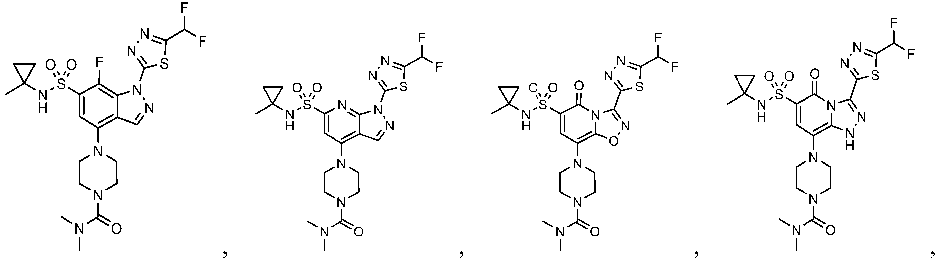

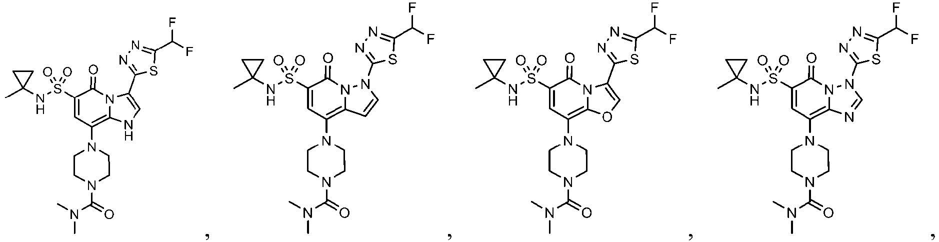

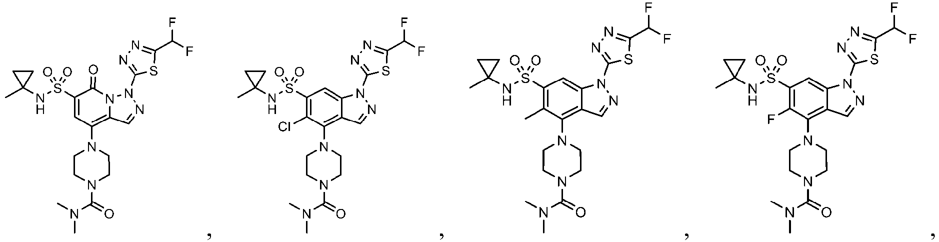

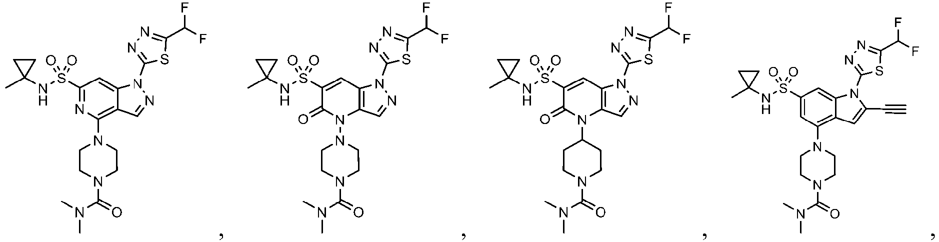

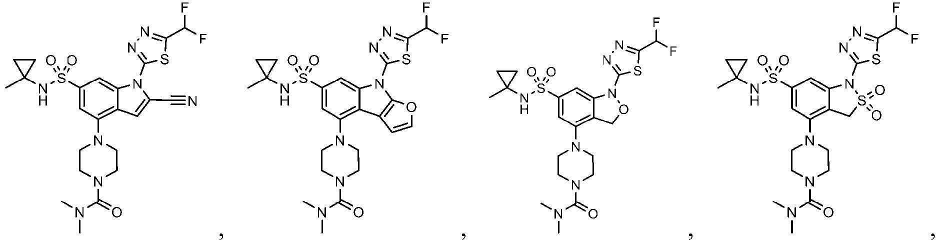

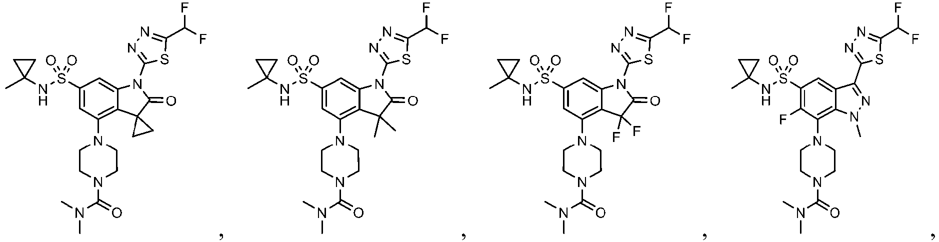

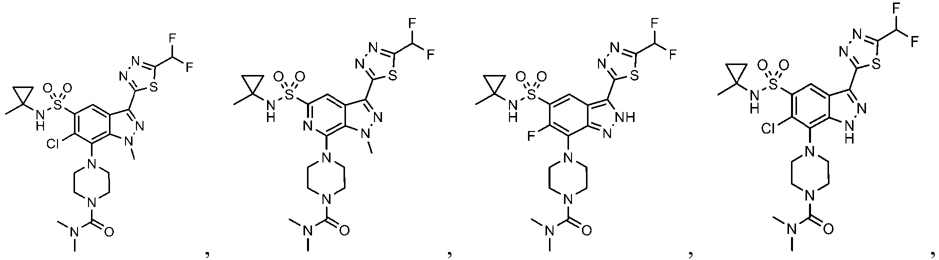

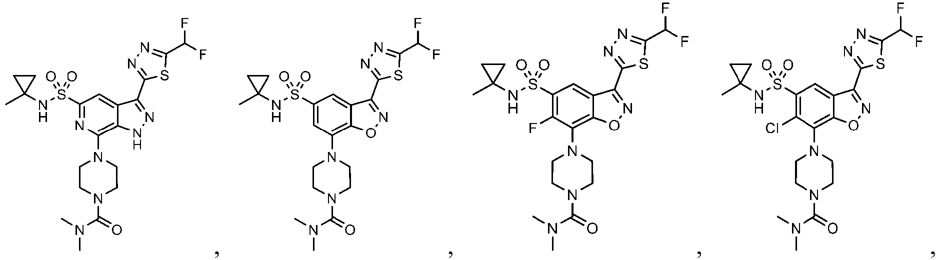

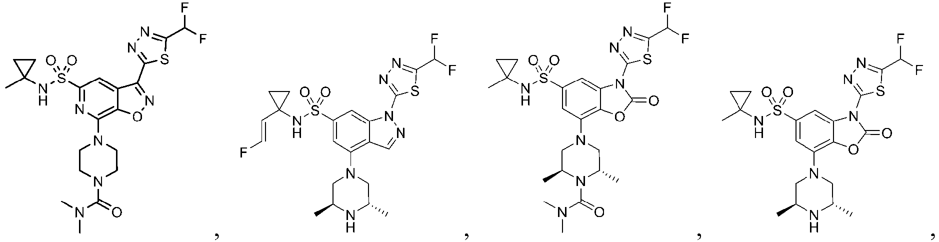

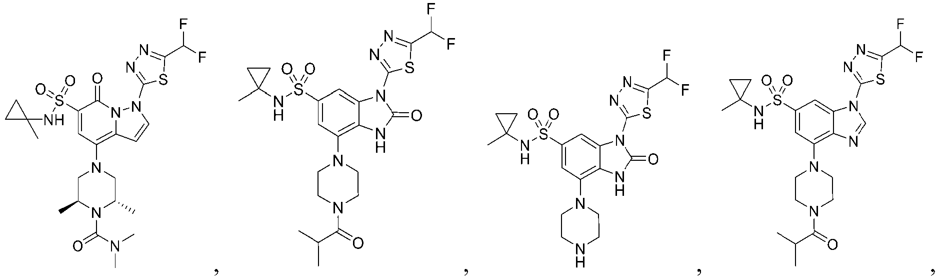

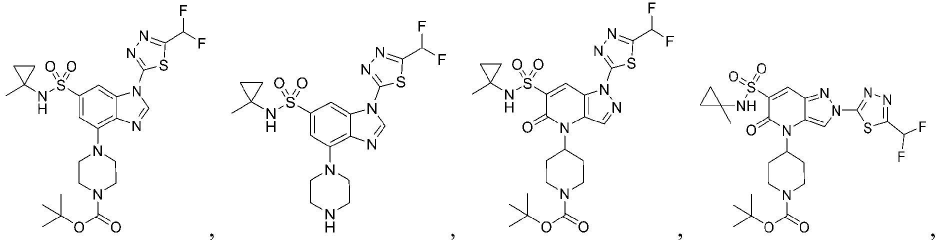

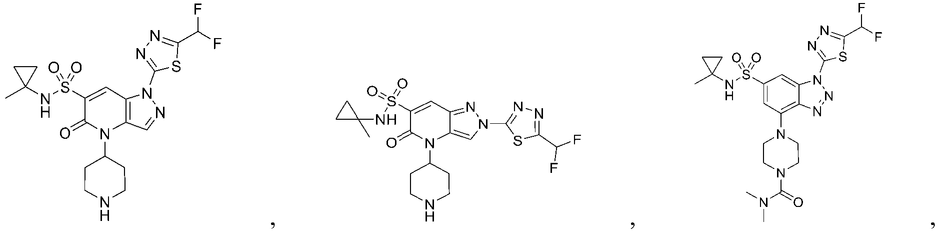

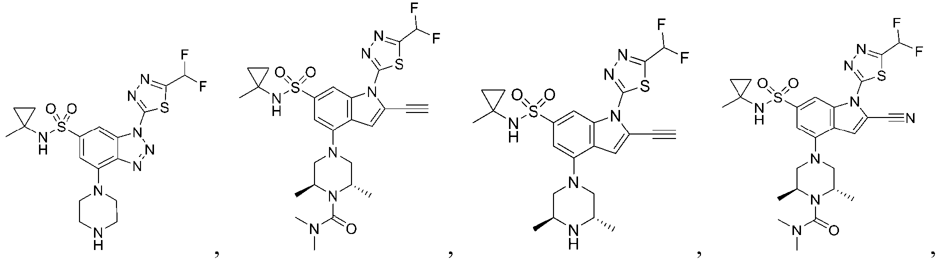

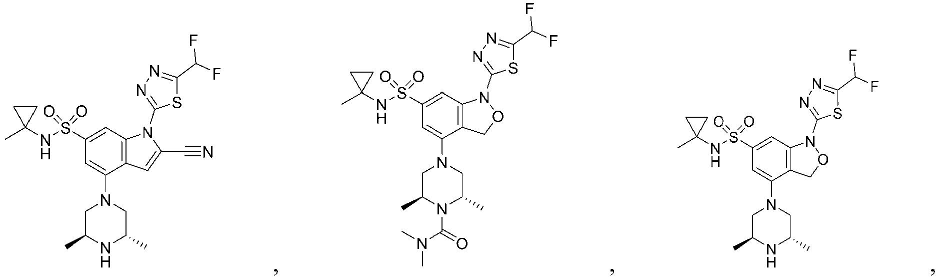

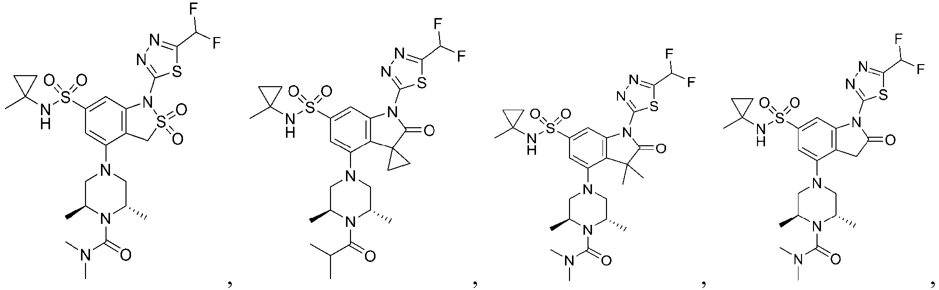

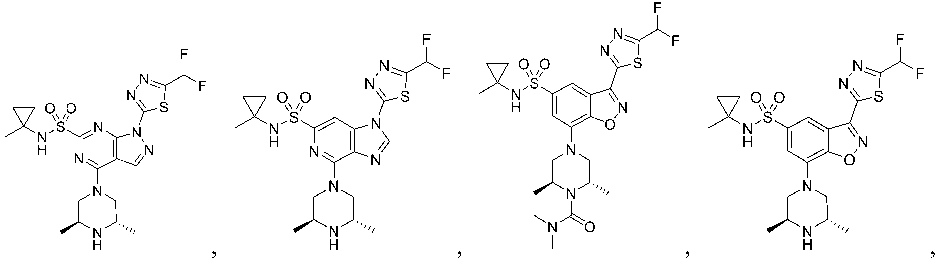

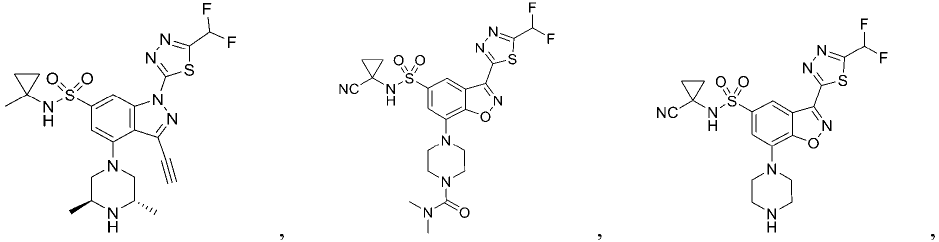

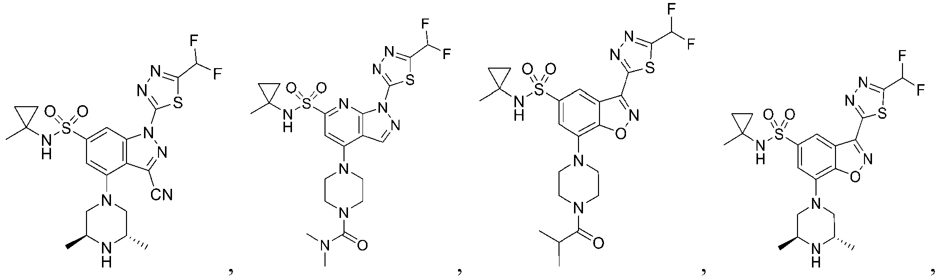

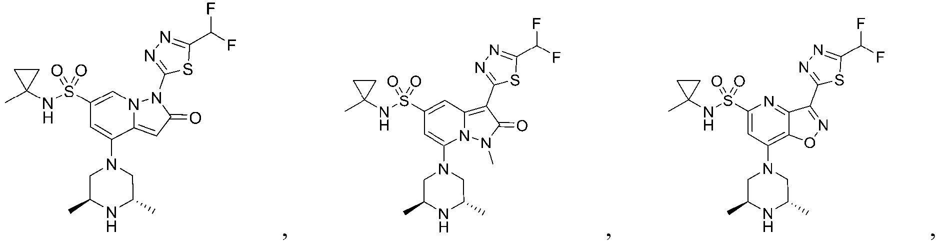

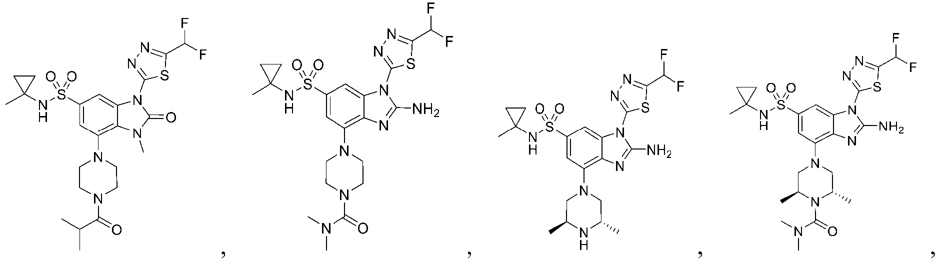

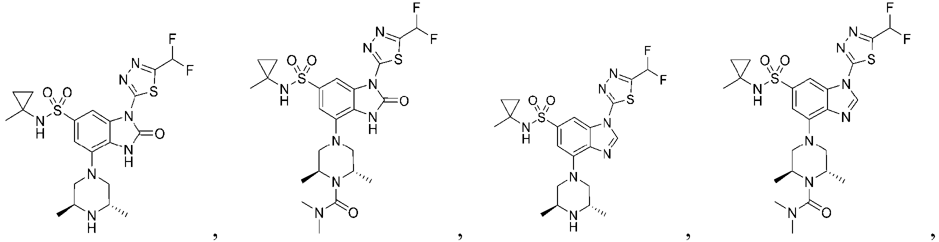

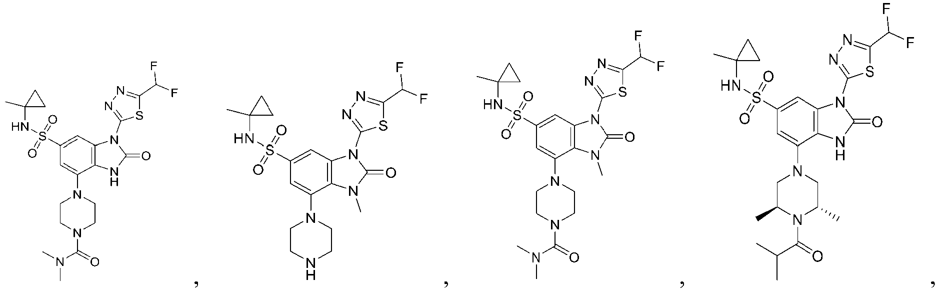

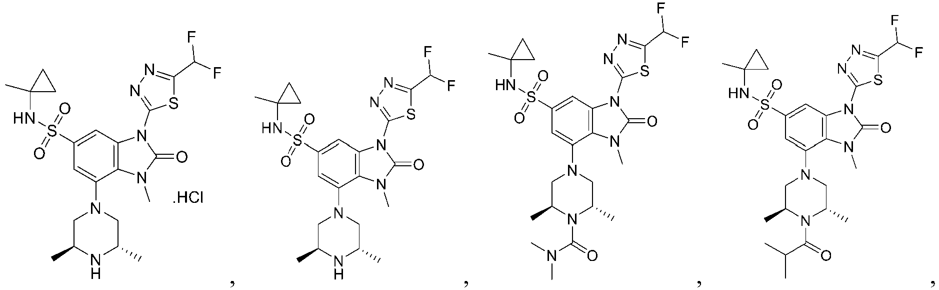

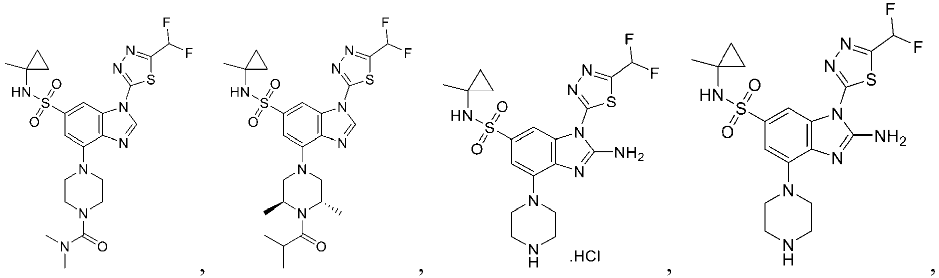

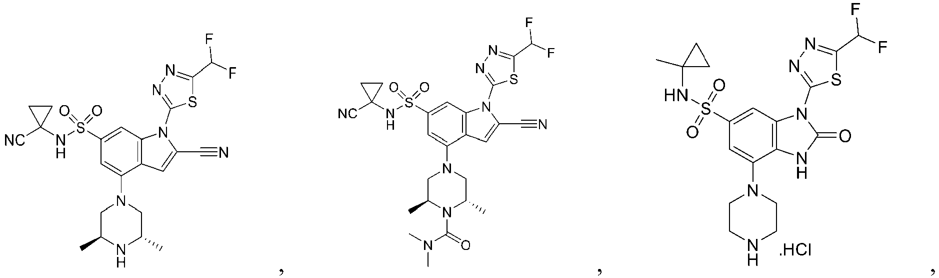

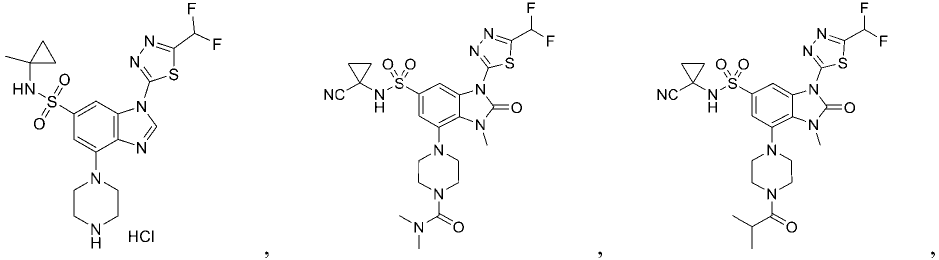

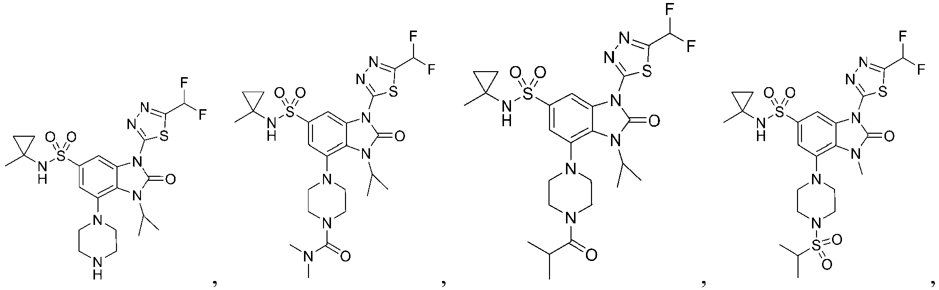

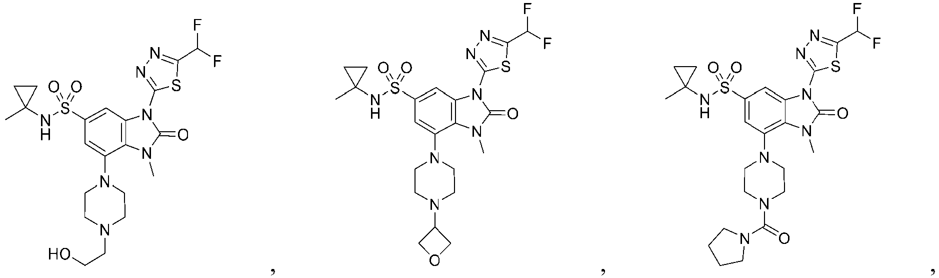

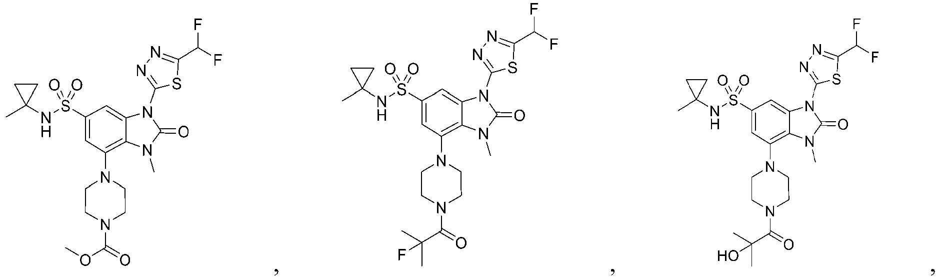

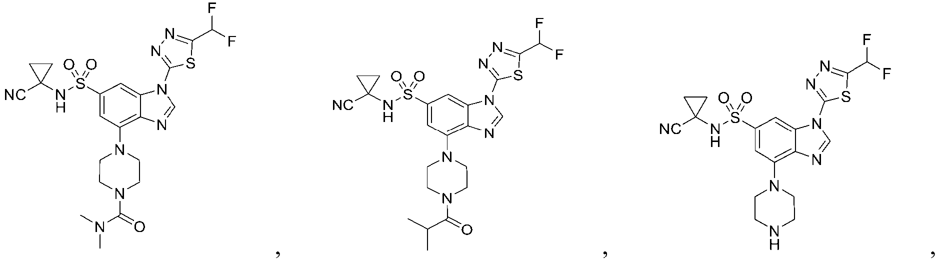

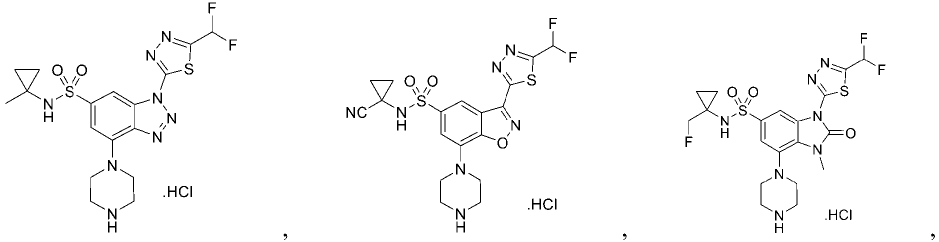

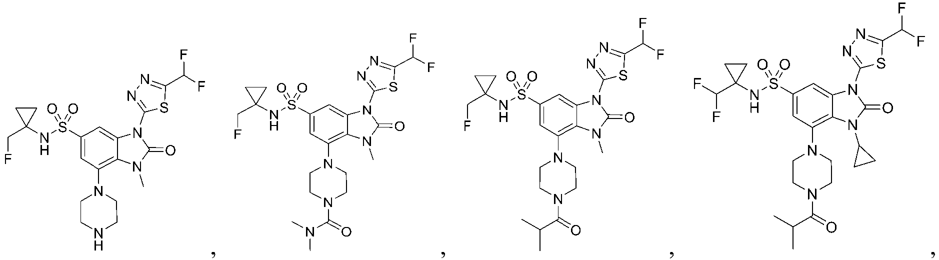

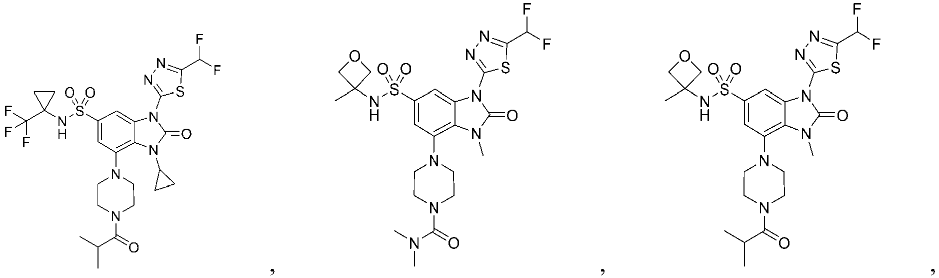

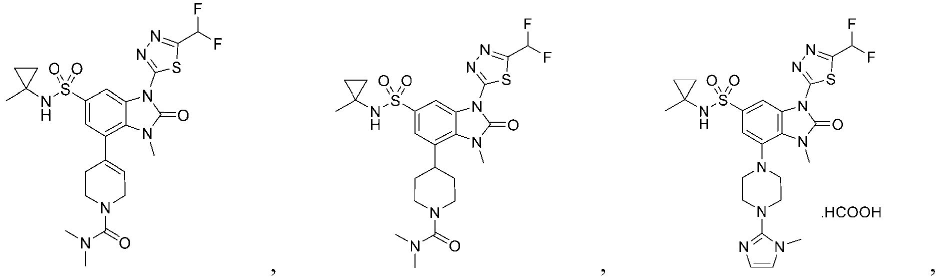

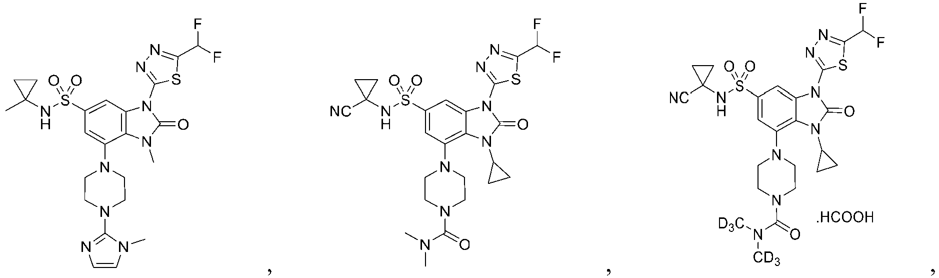

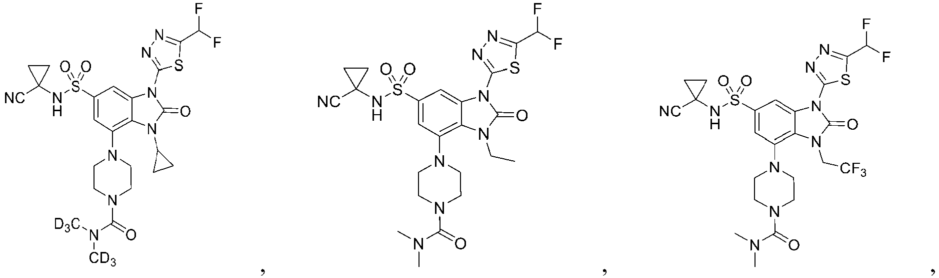

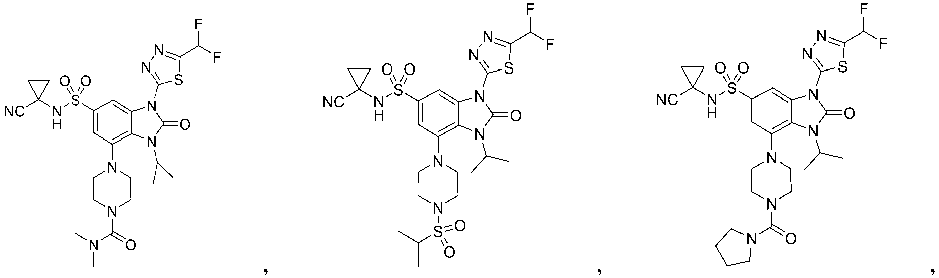

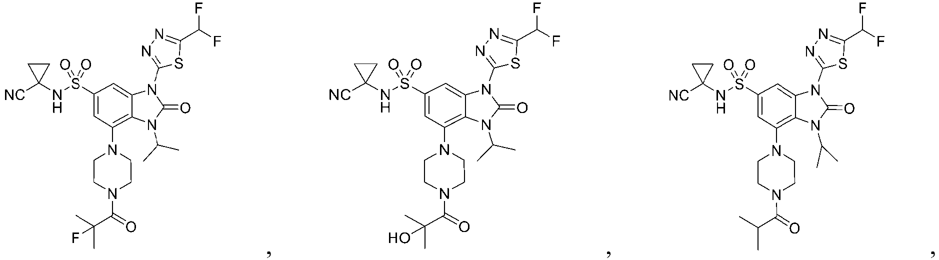

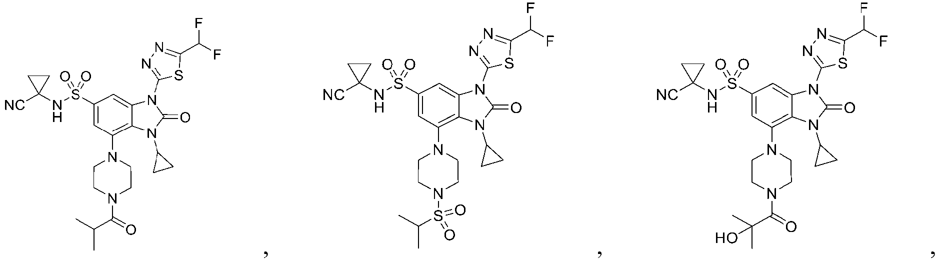

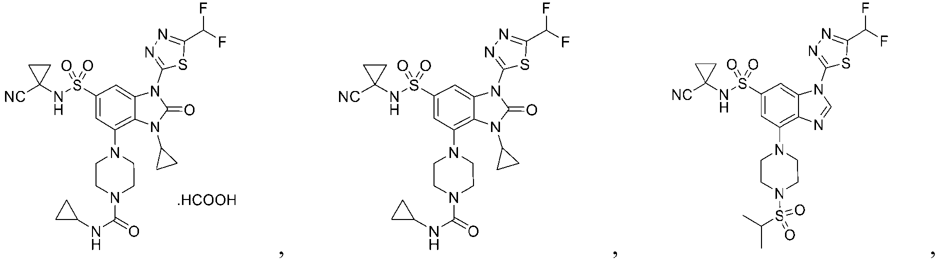

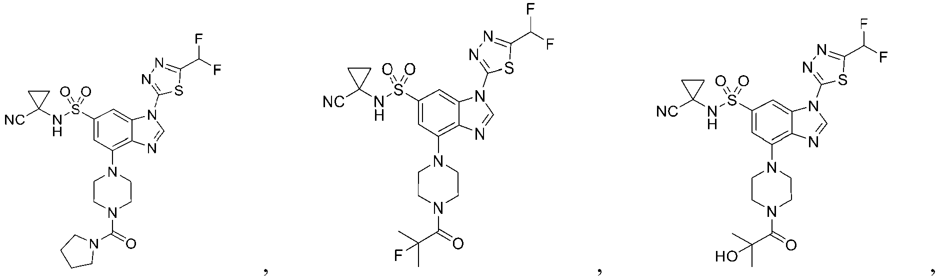

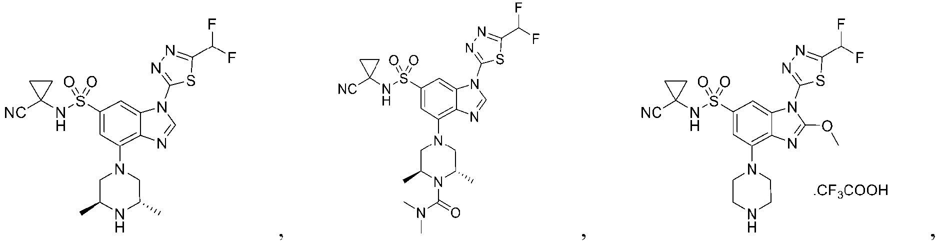

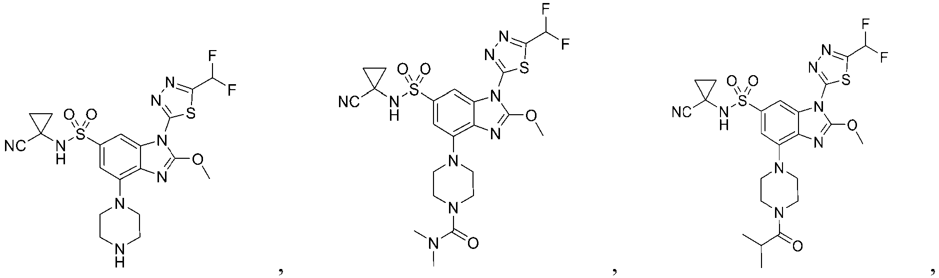

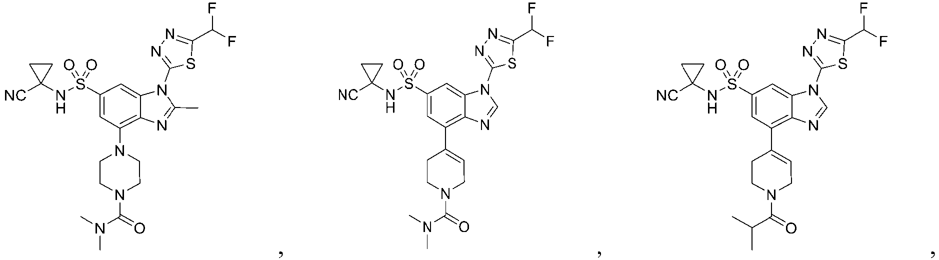

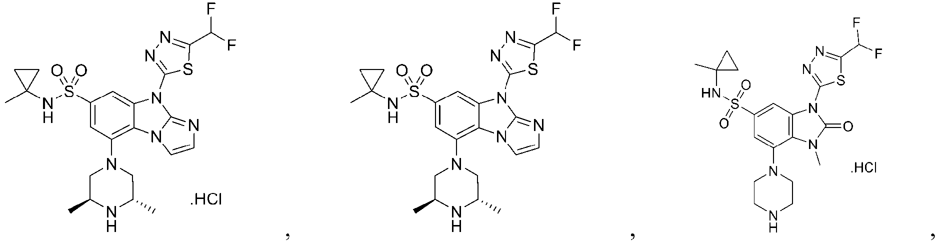

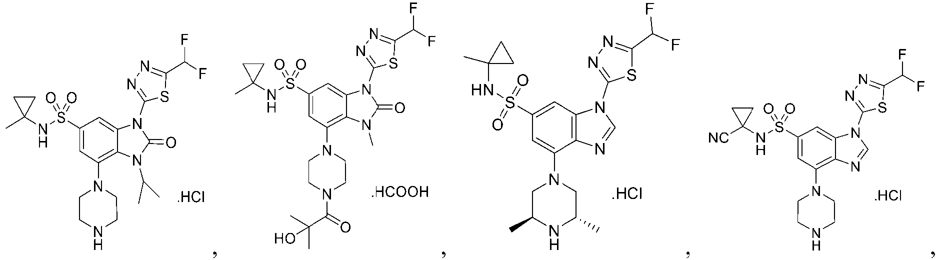

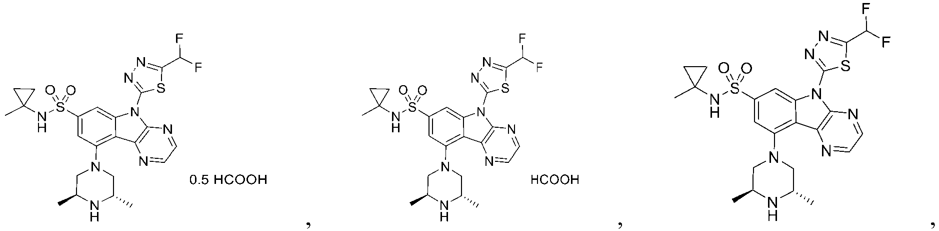

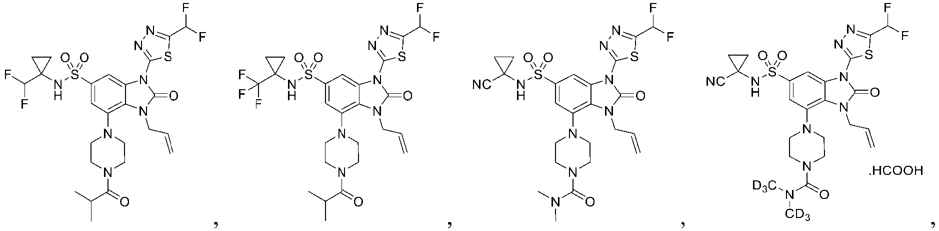

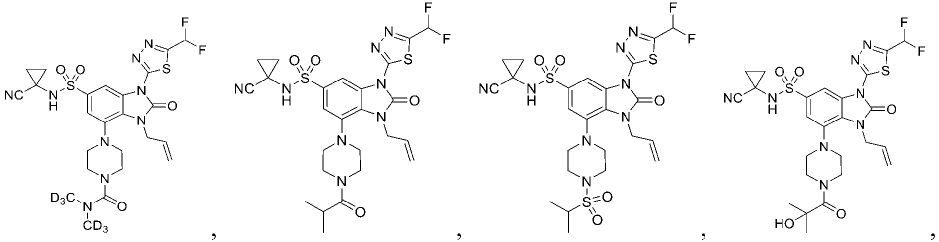

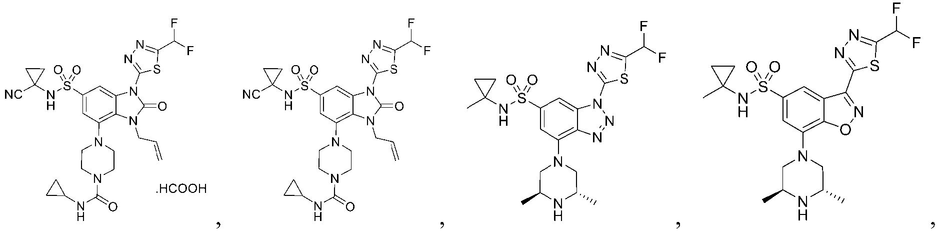

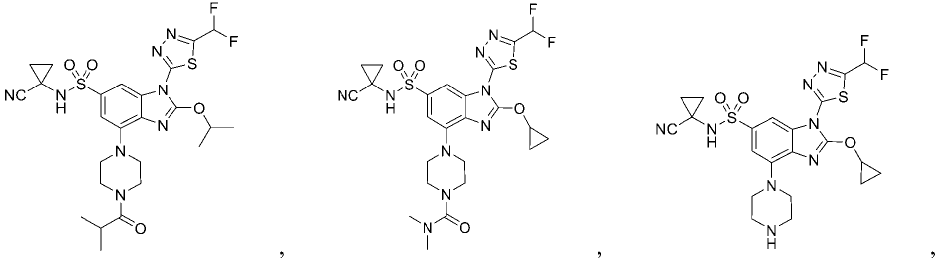

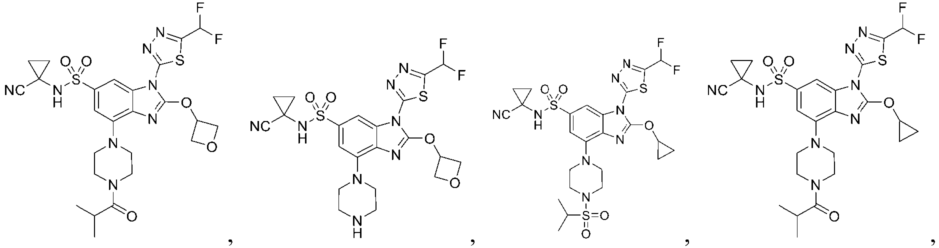

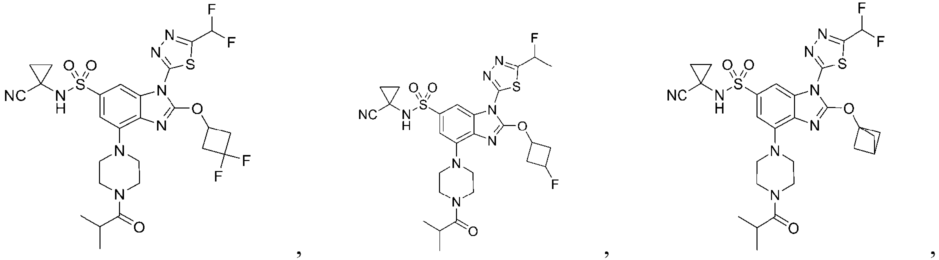

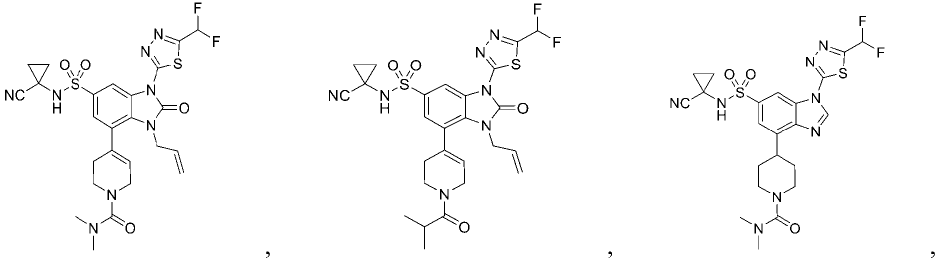

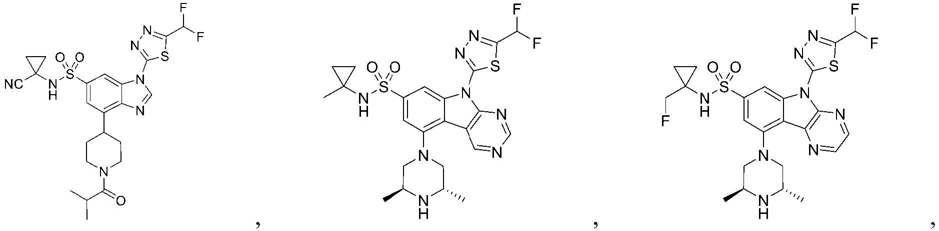

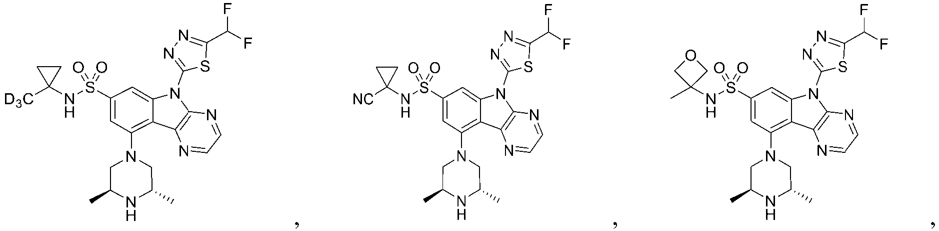

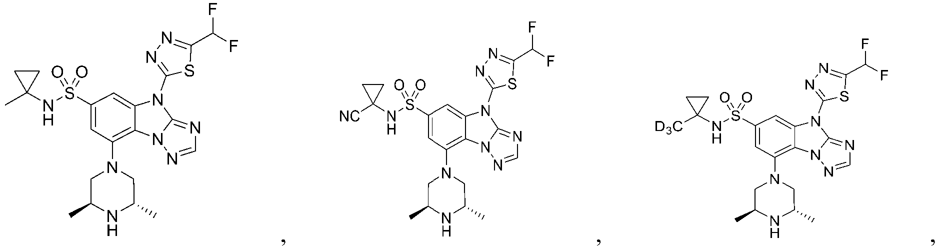

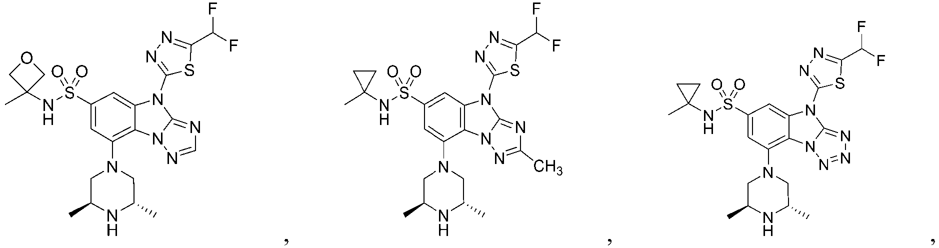

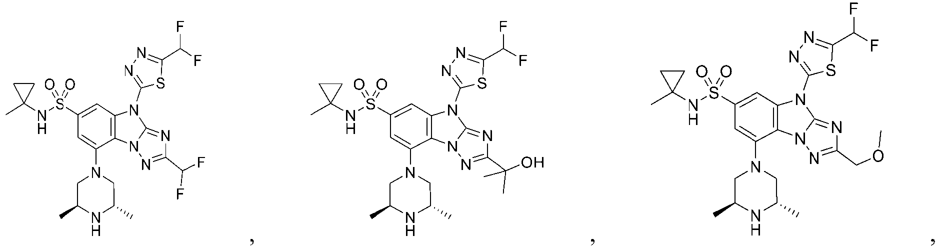

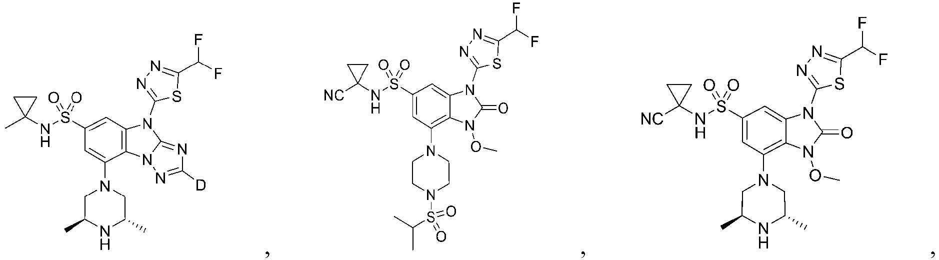

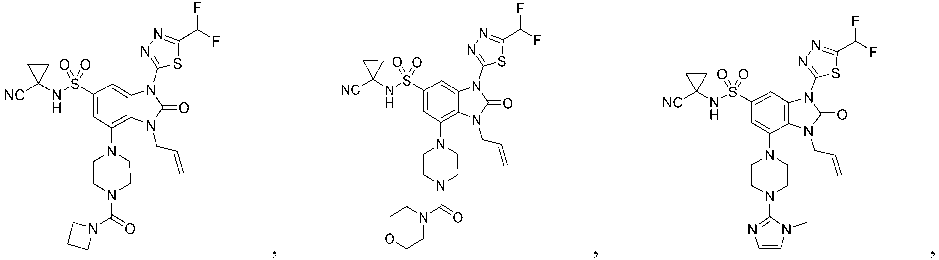

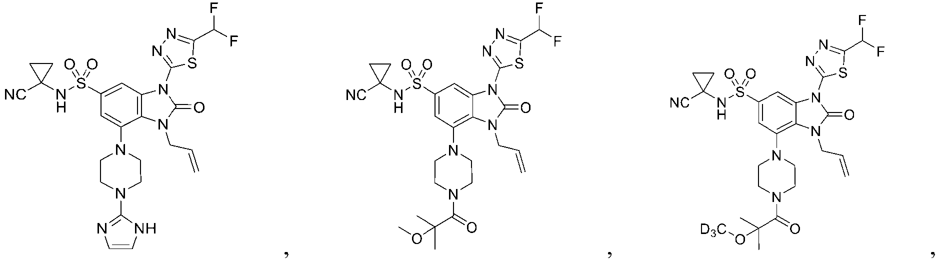

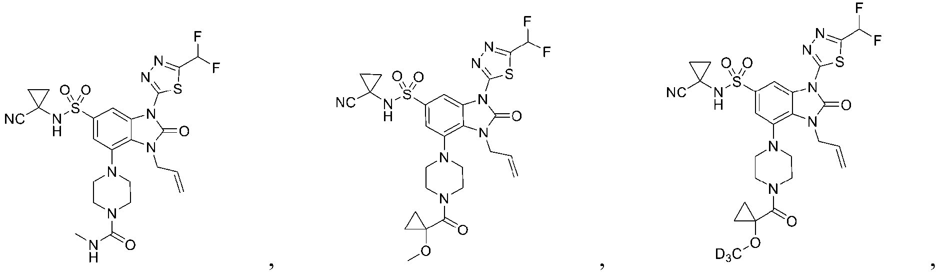

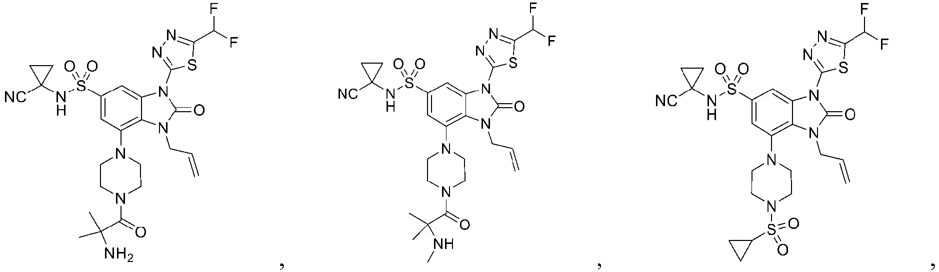

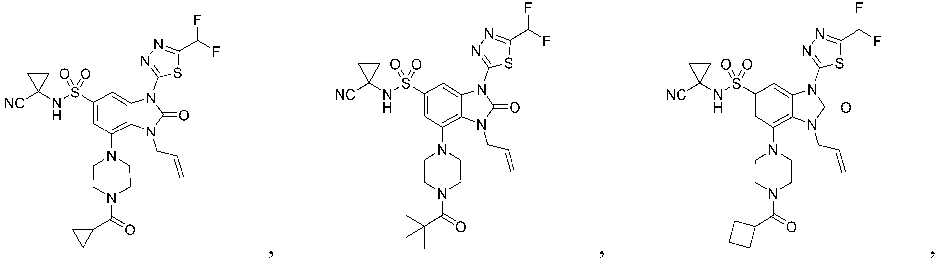

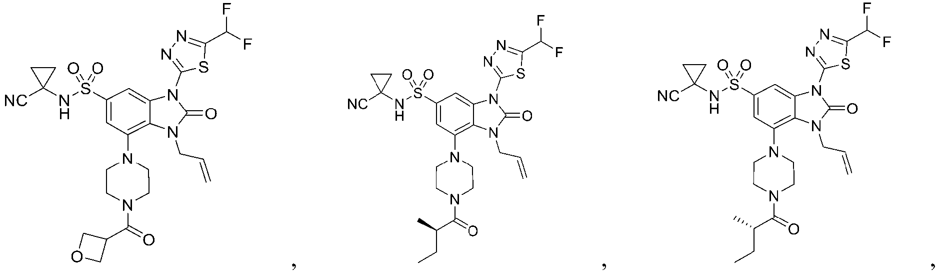

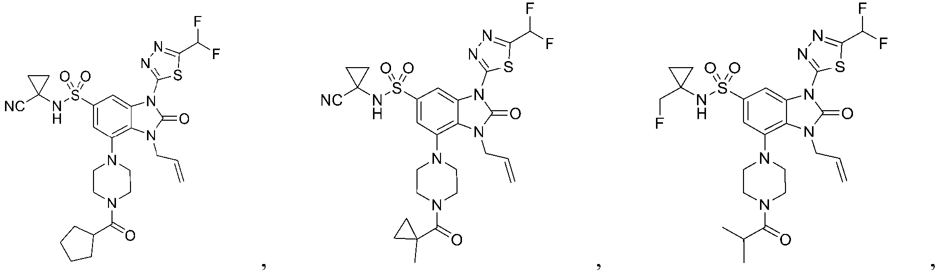

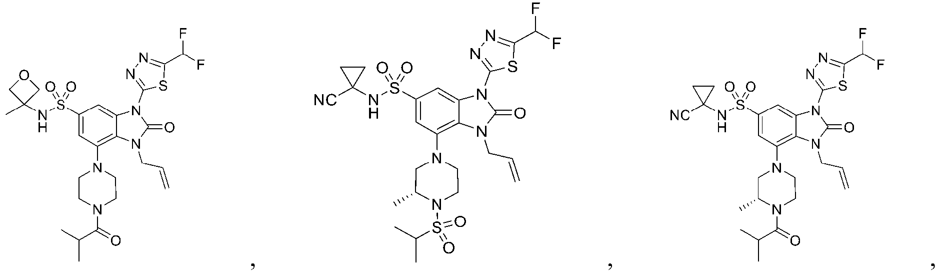

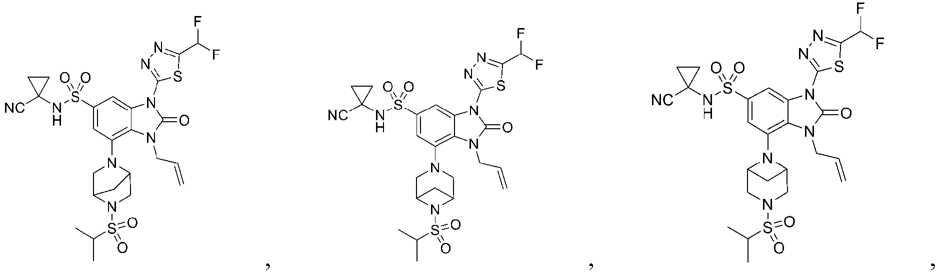

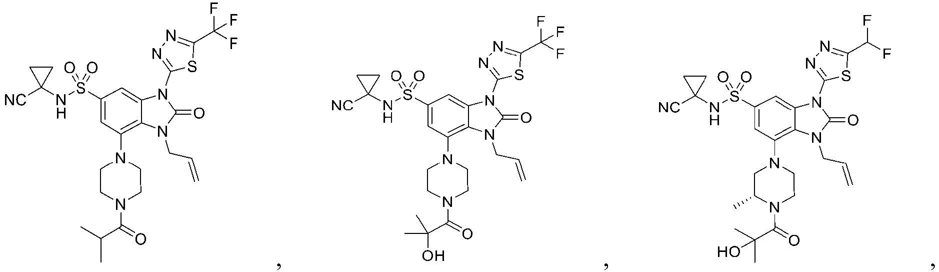

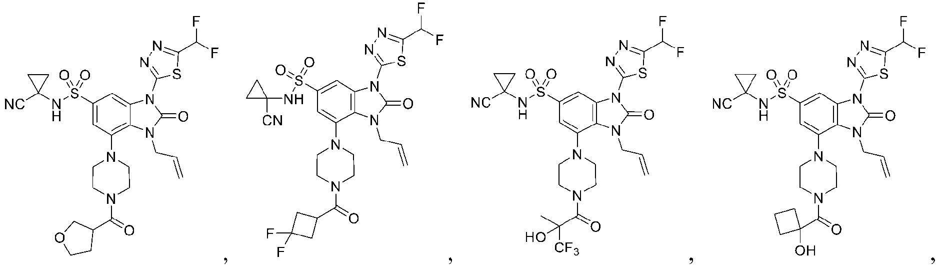

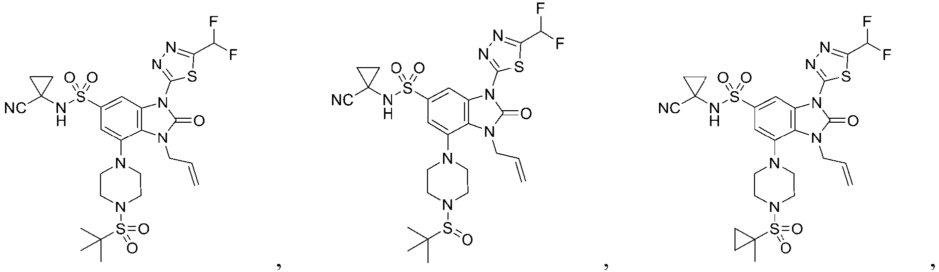

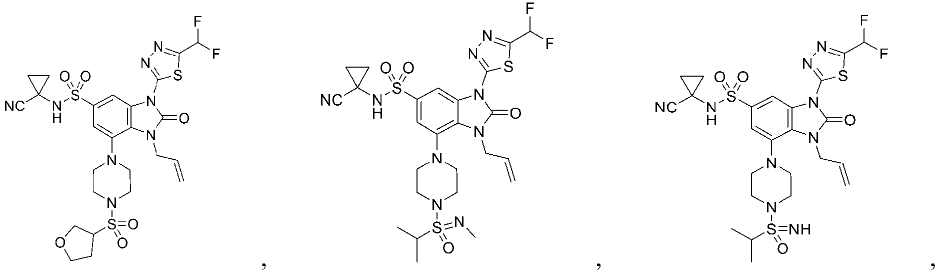

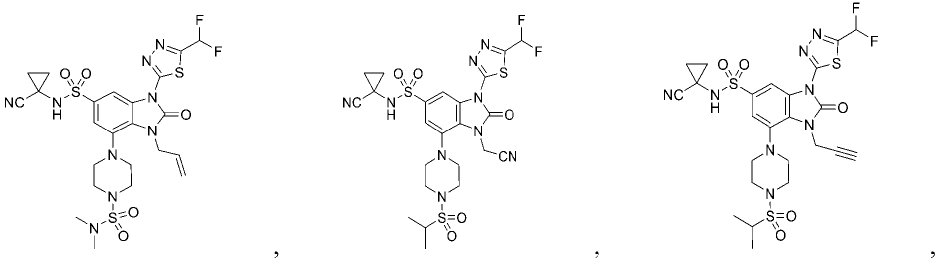

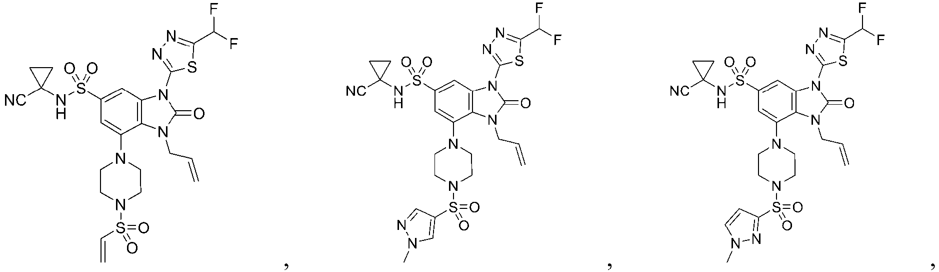

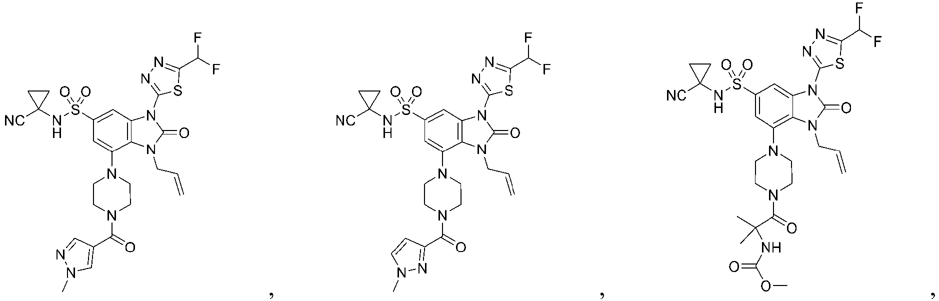

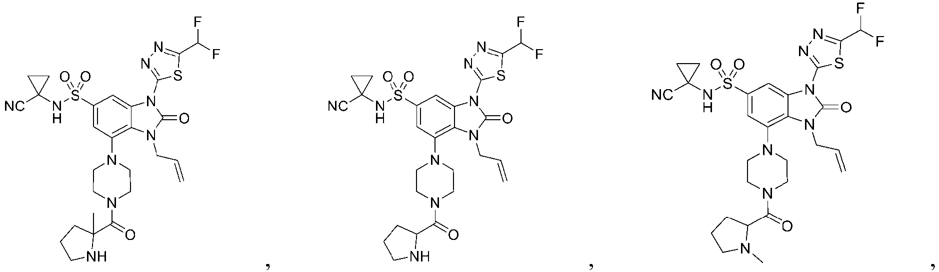

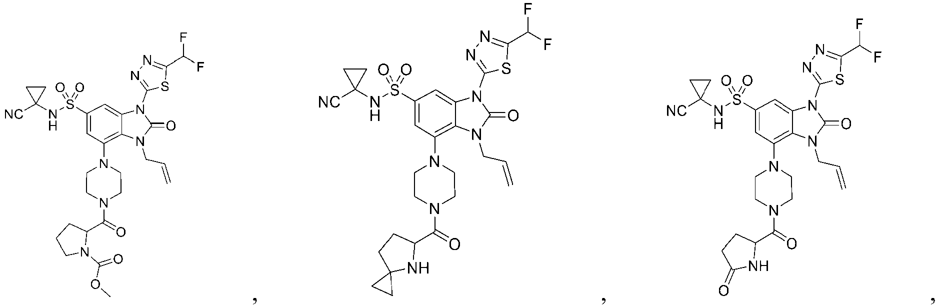

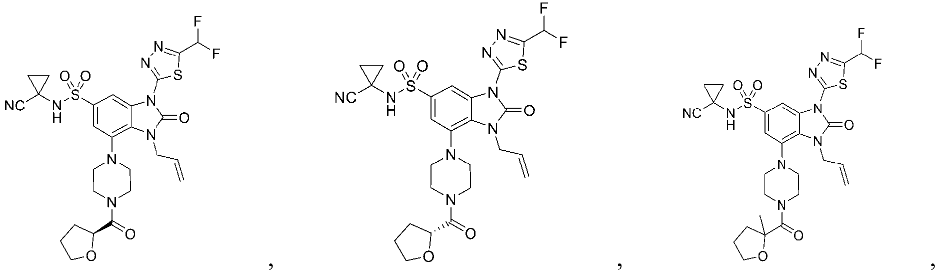

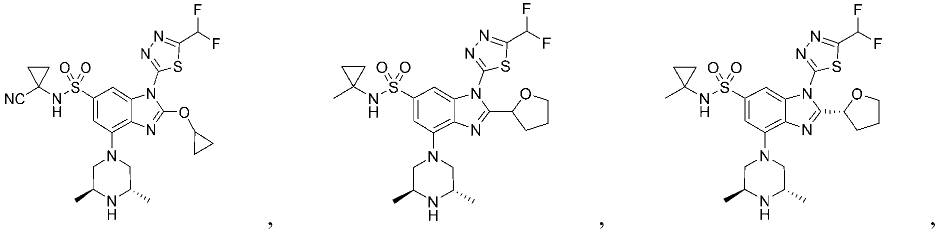

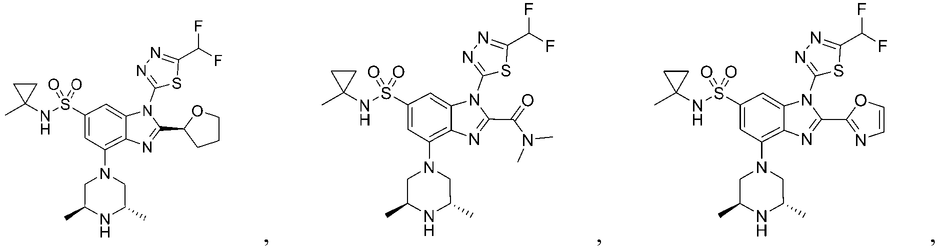

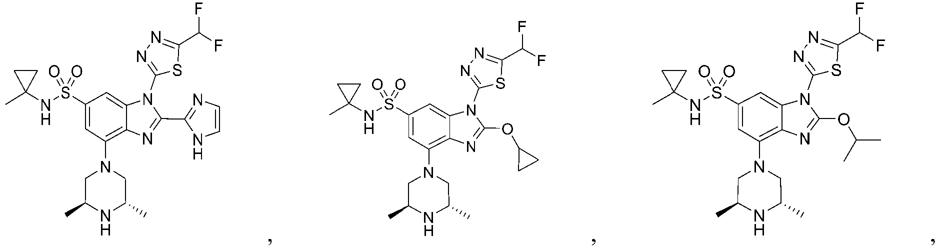

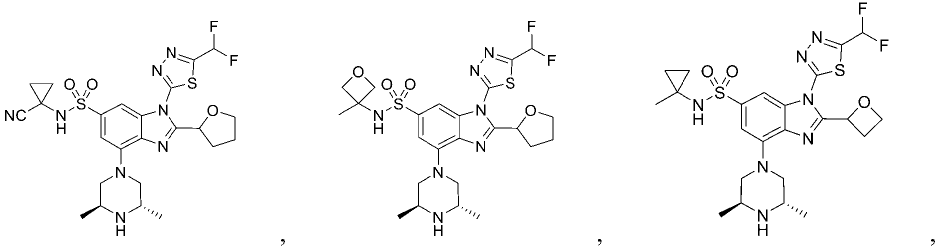

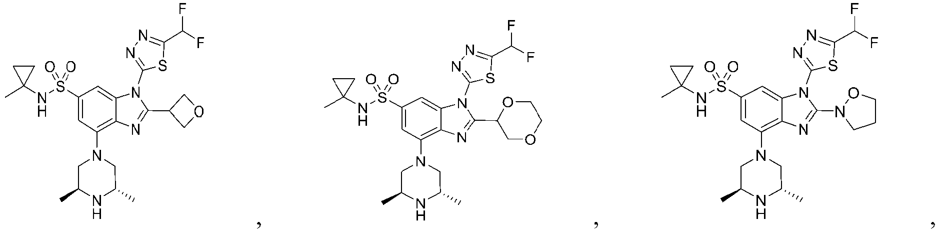

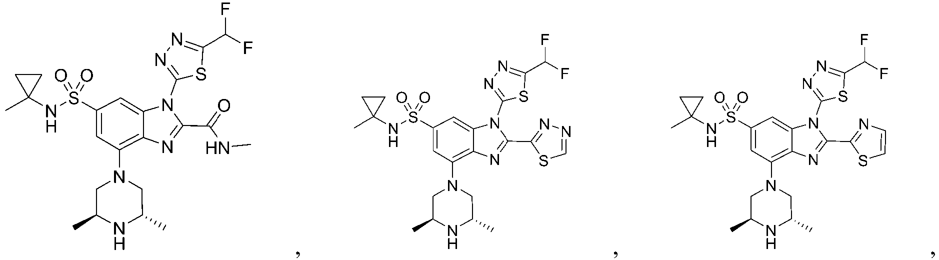

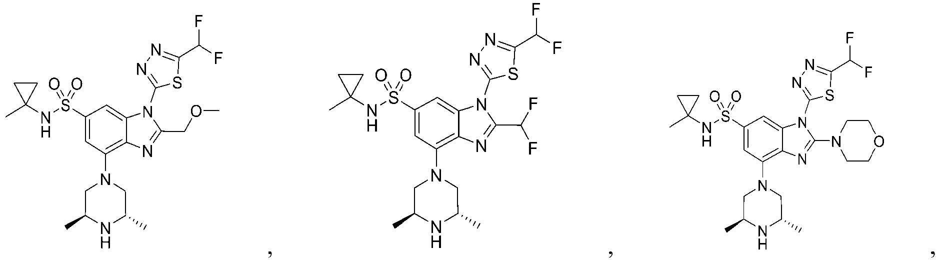

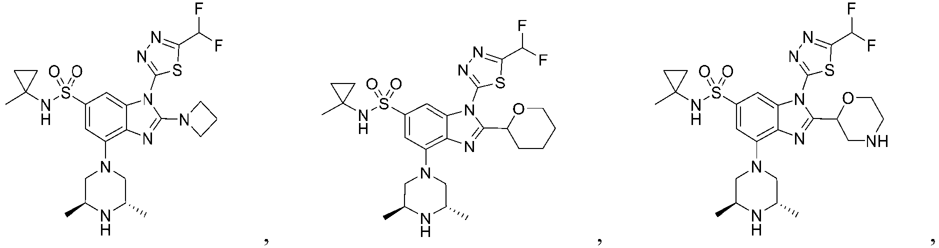

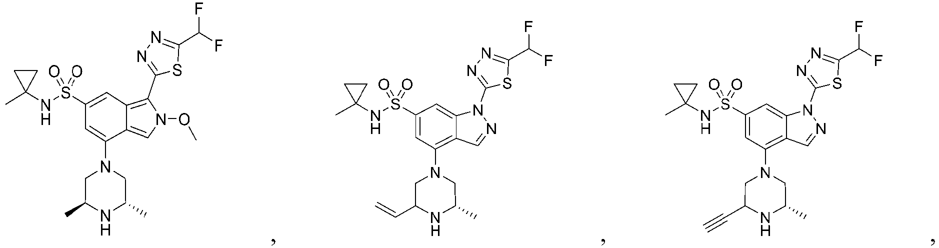

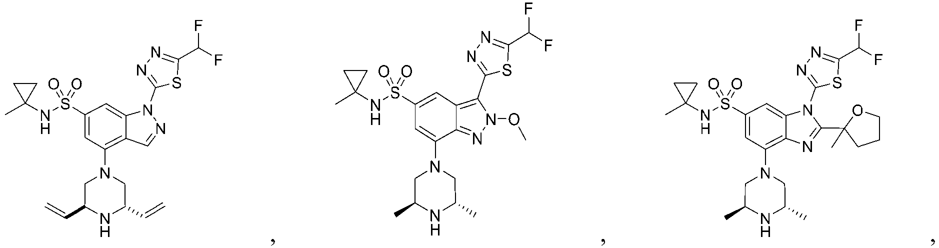

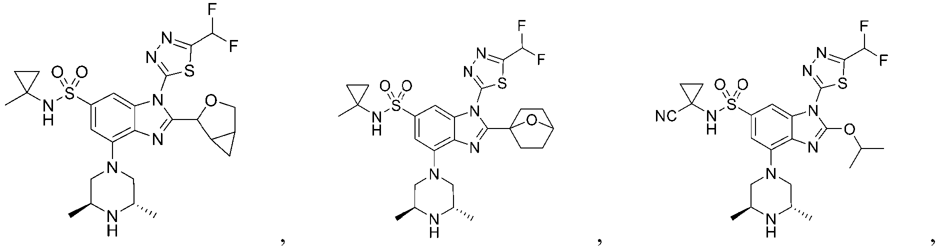

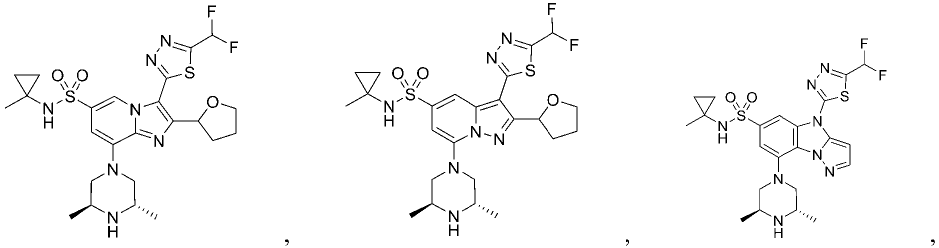

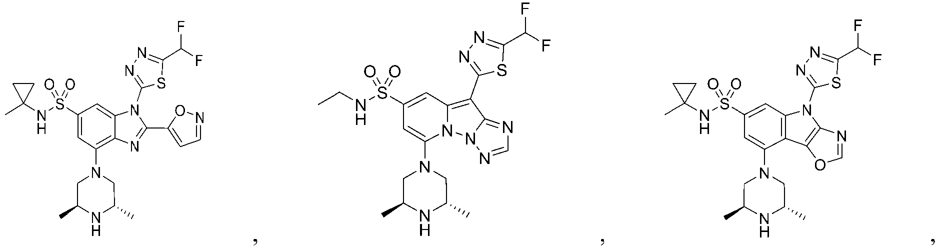

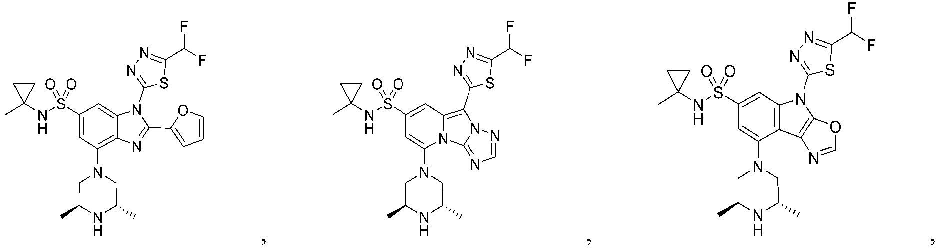

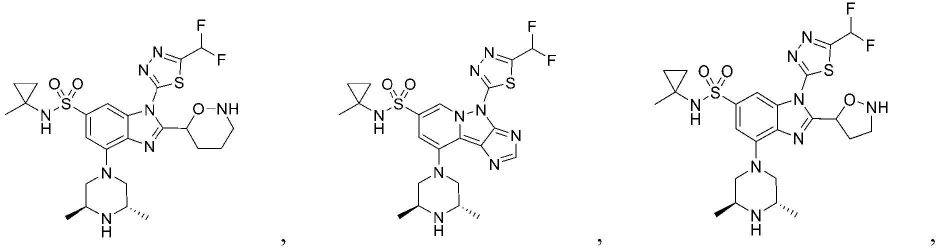

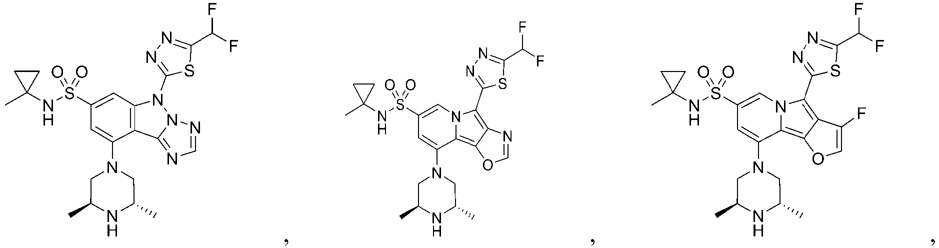

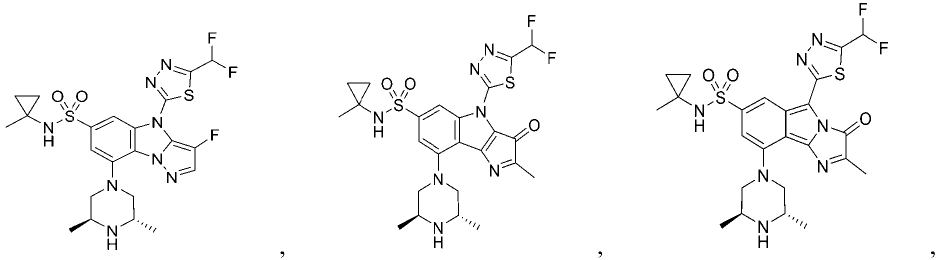

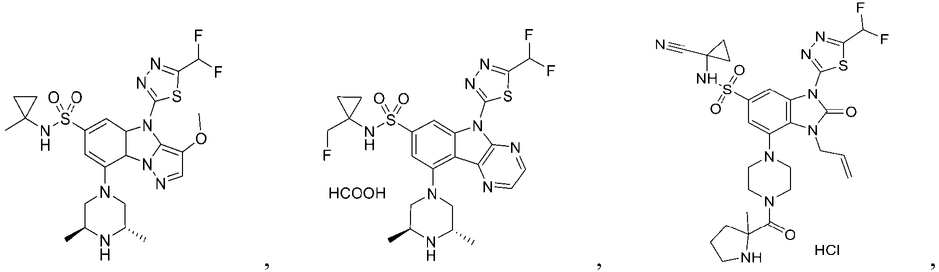

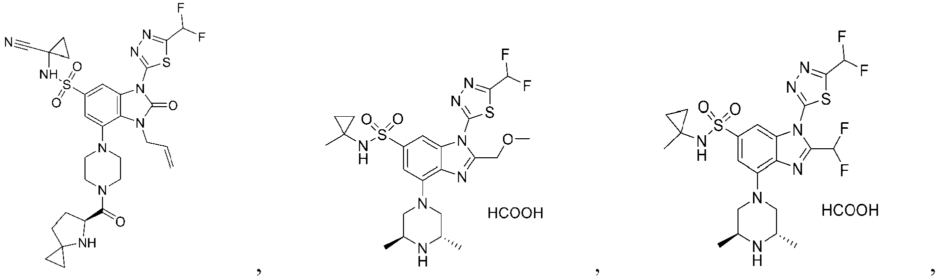

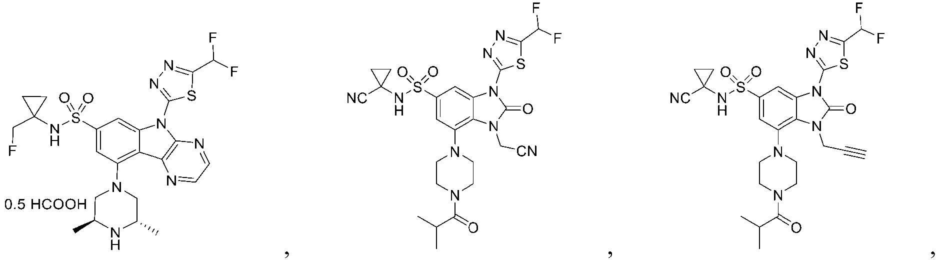

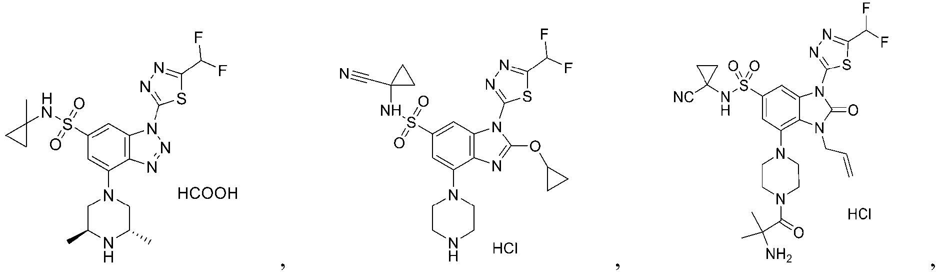

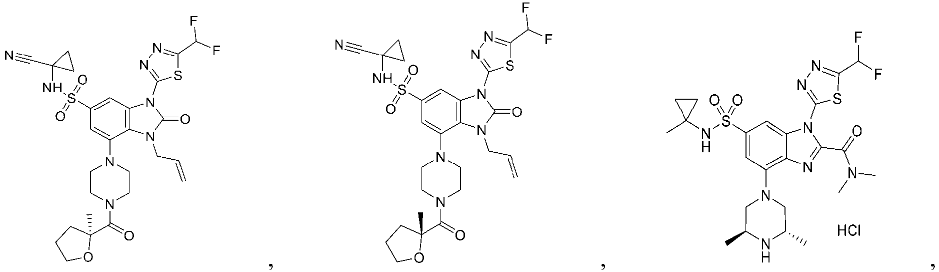

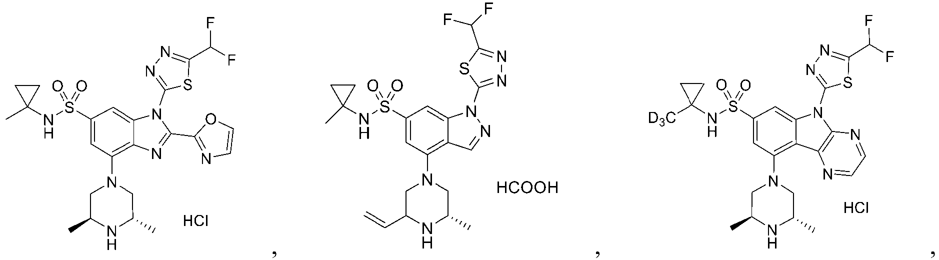

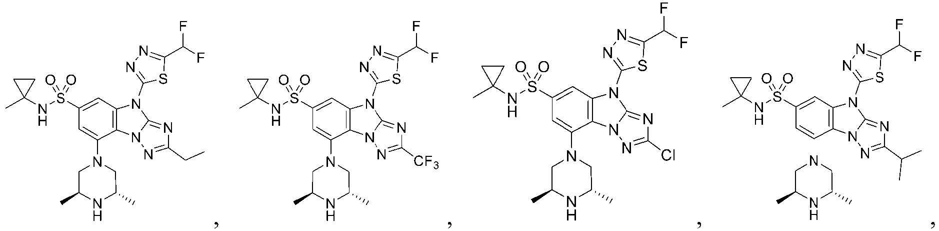

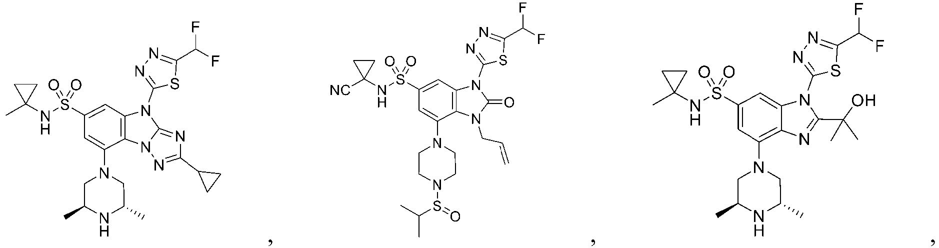

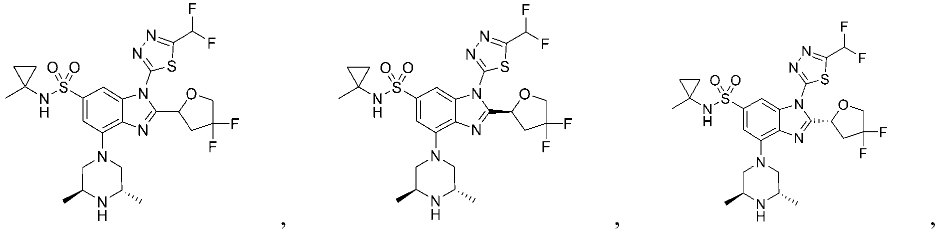

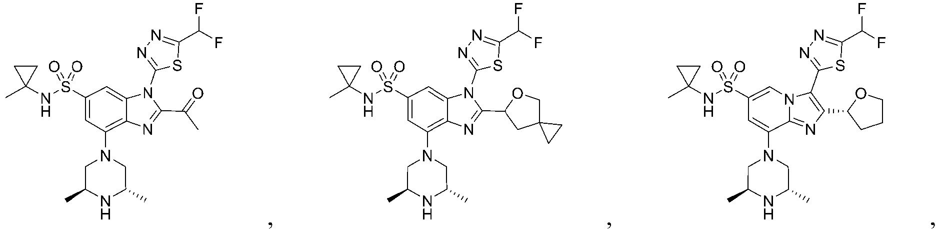

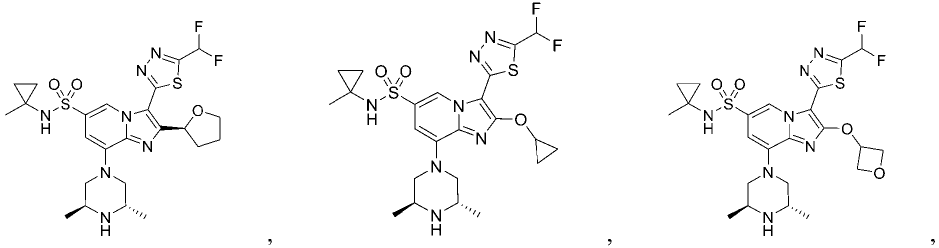

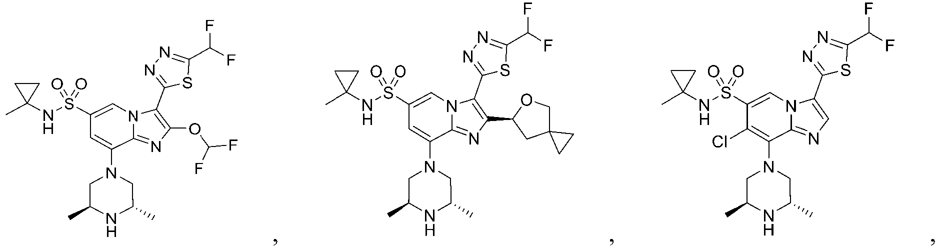

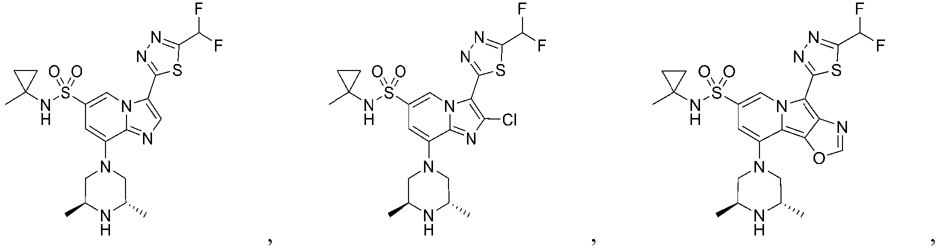

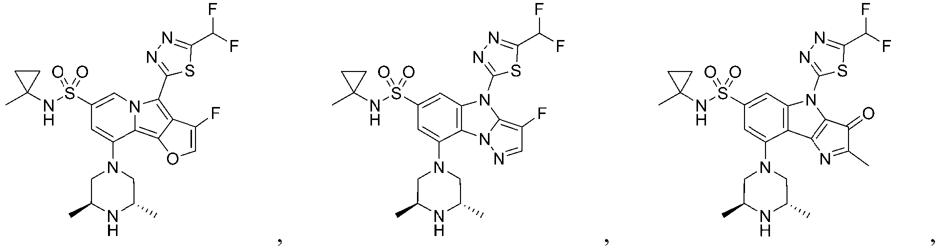

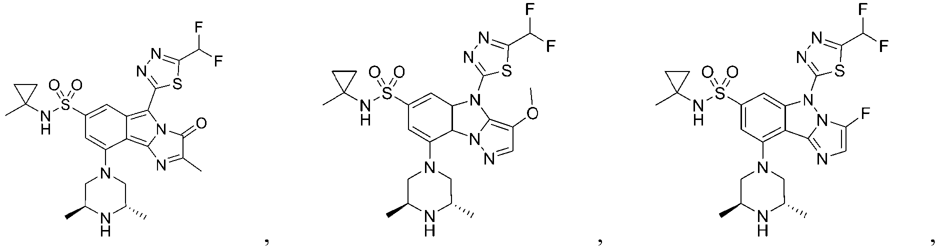

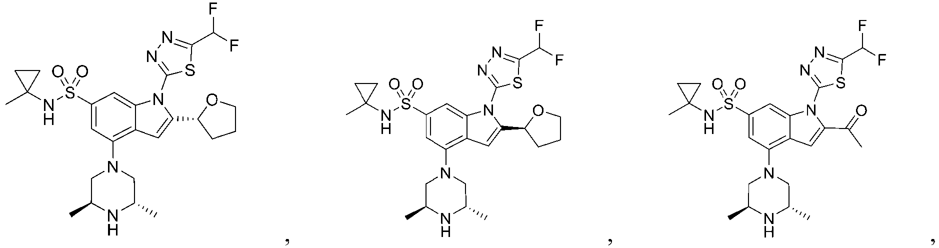

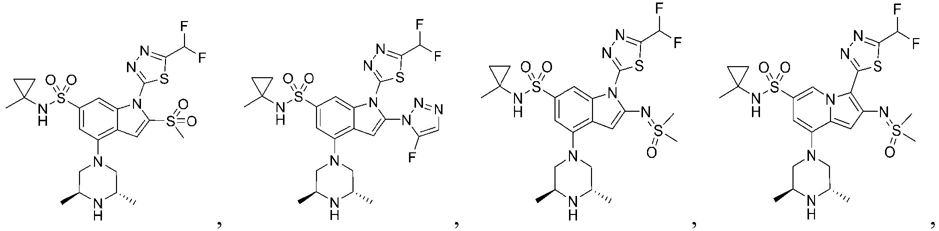

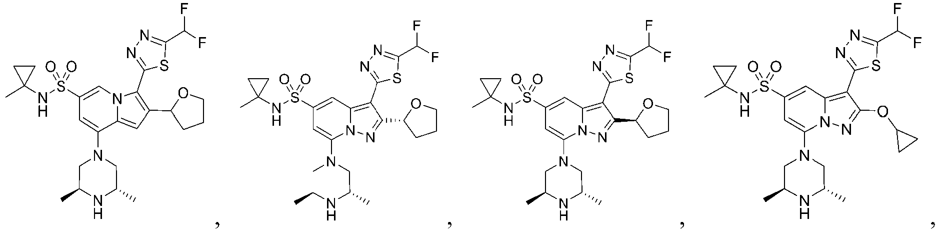

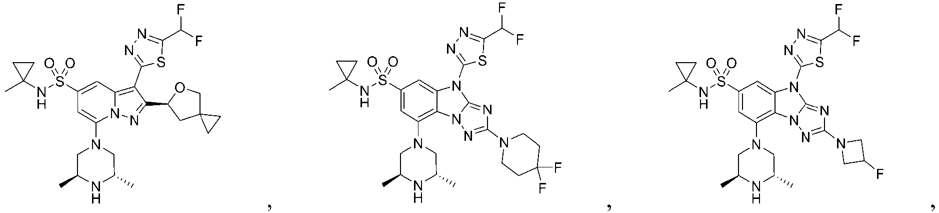

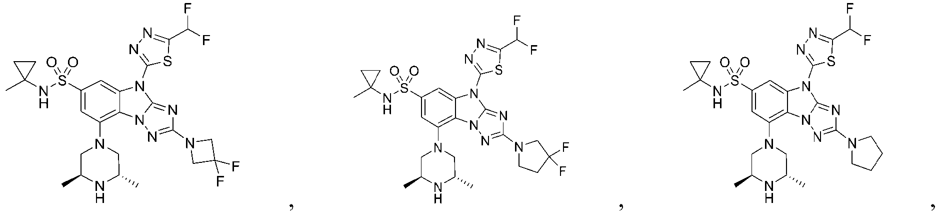

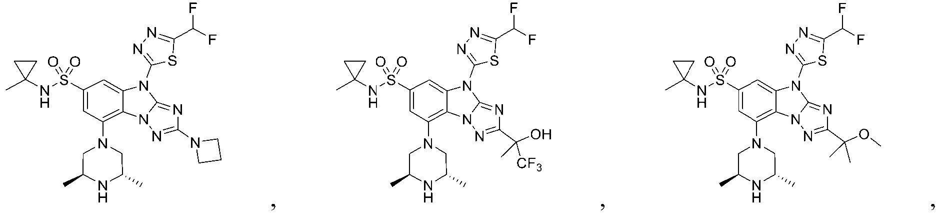

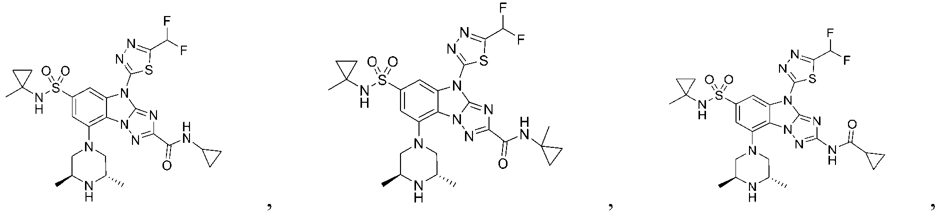

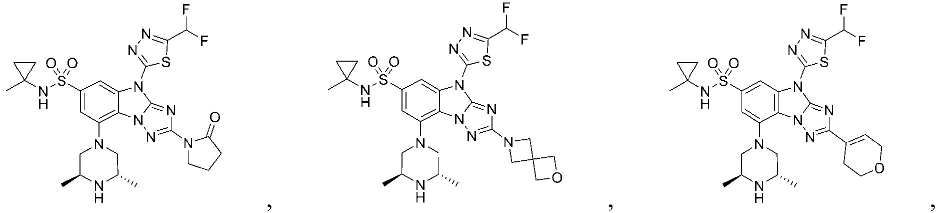

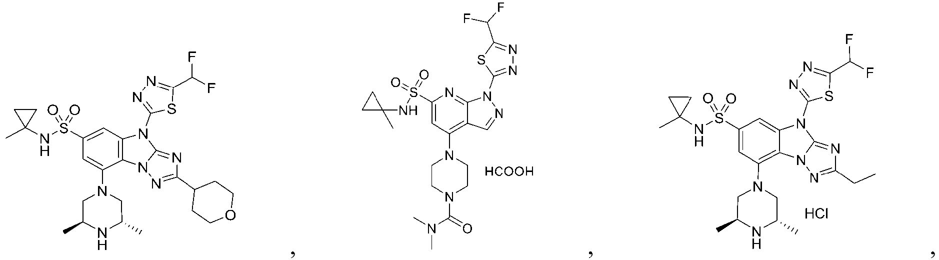

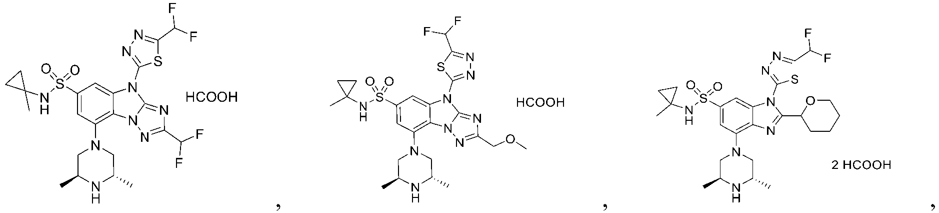

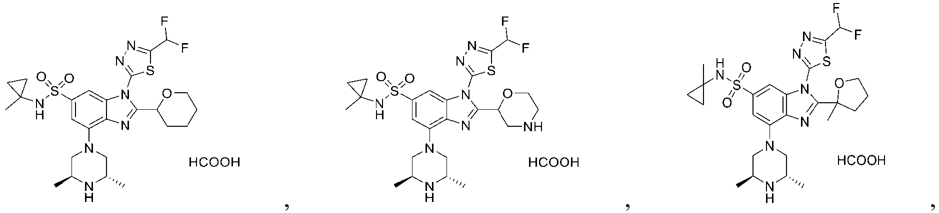

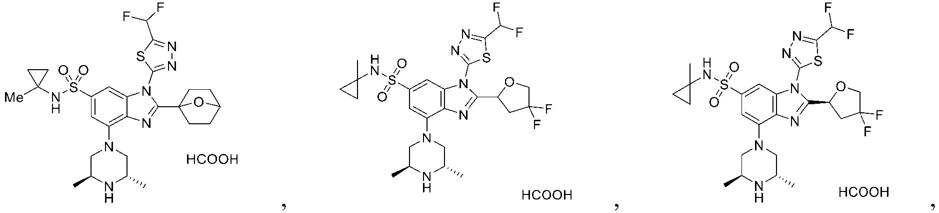

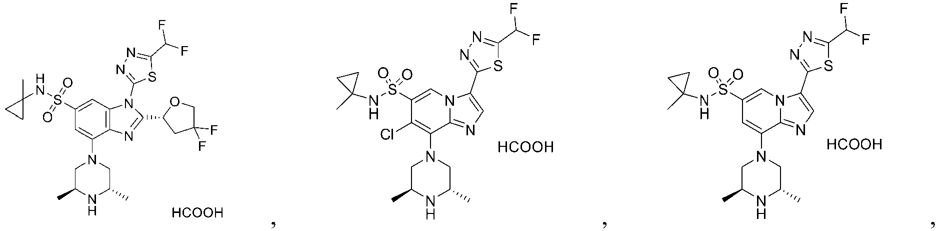

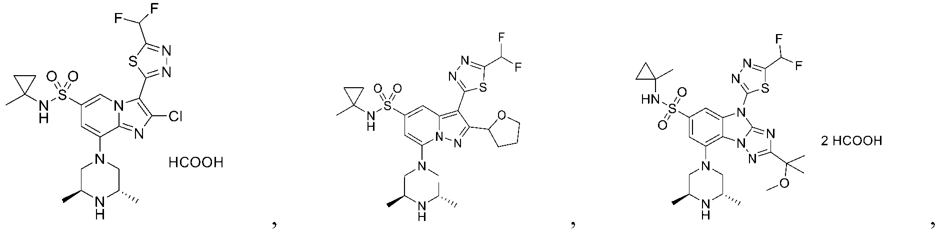

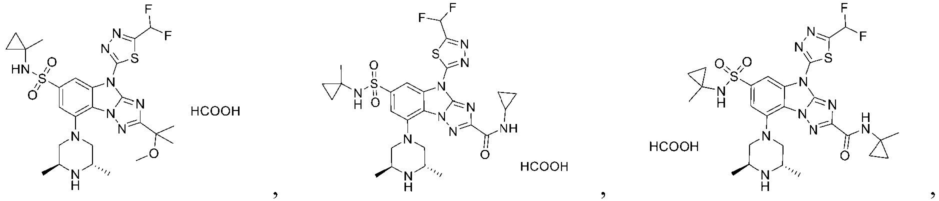

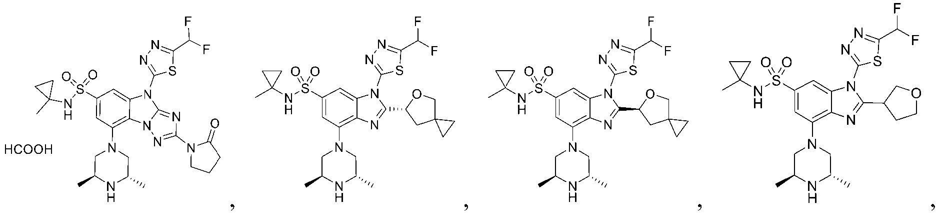

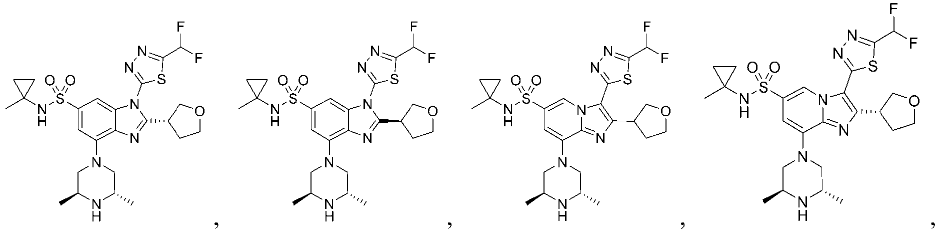

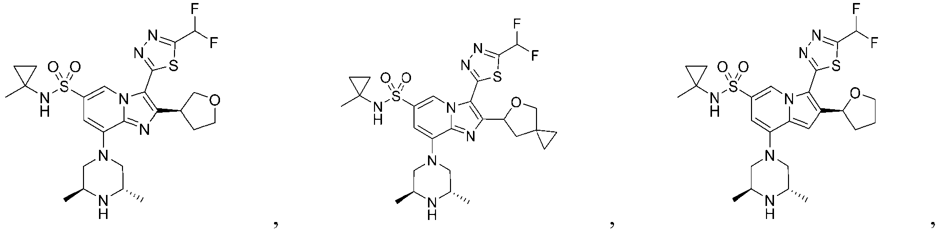

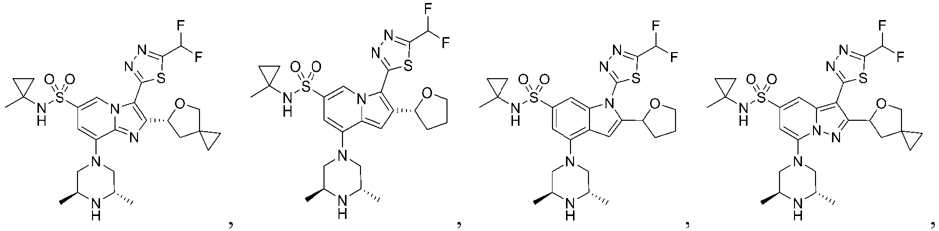

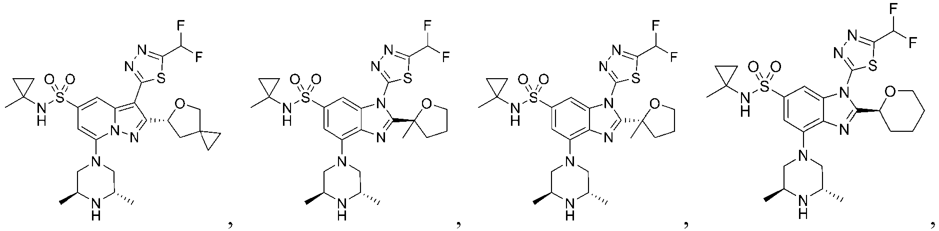

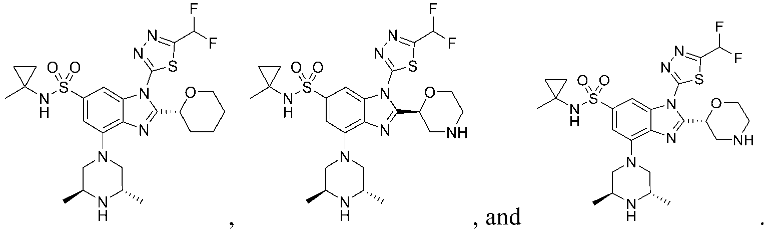

22. A pharmaceutical composition comprising the compound, the pharmaceutically acceptable salt thereof, the stereoisomer thereof, the prodrug thereof, the N-oxide thereof, the solvate thereof, or the isotopic derivative thereof according to at least one of claims 1 to 21, and a pharmaceutically acceptable carrier.

23. Use of the compound, the pharmaceutically acceptable salt thereof, the stereoisomer thereof, the prodrug thereof, the N-oxide thereof, the solvate thereof, or the isotopic derivative thereof according to at least one of claims 1 to 21, or the pharmaceutical composition according to claim 22, in the manufacture of a medicament for the treatment of PARG-mediated cancer.

24. A method of treating PARG-mediated cancer, comprising administering to a patient a therapeutically effective amount of the compound, the pharmaceutically acceptable salt thereof, the stereoisomer thereof, the prodrug thereof, the N-oxide thereof, the solvate thereof, or the isotopic derivative thereof according to at least one of claims 1 to 21, or the pharmaceutical composition according to claim 22.

25. The use according to claim 23 or the method according to claim 24, wherein the cancer is selected from the group consisting of ovarian cancer, pancreatic cancer, breast cancer, and prostate cancer.
